# EUROPÄISCHE PATENTSCHRIFT

(11) **EP 2 268 625 B1**
(45) Veröffentlichungstag und Bekanntmachung des Hinweises auf die Patenterteilung: **16.05.2012**
(21) Anmeldenummer: 09732141.8
(22) Anmeldetag: 06.04.2009
(51) Int. Cl.: C07D 253/08, A61K 31/5395, A61K 31/498, A61P 9/00, C07D 209/46, C07D 209/48, C07D 211/76, C07D 213/64, C07D 231/56, C07D 233/32, C07D 237/14, C07D 237/32, C07D 239/88, C07D 261/20, C07D 263/20, C07D 273/04, C07D 275/06, C07D 277/34, C07D 417/12, C07D 253/075

(54) **OXO-HETEROCYCLISCH SUBSTITUIERTE CARBONSÄURE-DERIVATE UND IHRE VERWENDUNG**
OXO-HETEROCYCLIC SUBSTITUTED CARBOXYLIC ACID DERIVATES AND THE USE THEREOF
DÉRIVÉS D'ACIDE CARBOXYLIQUE À SUBSTITUTION OXO-HÉTÉROCYCLIQUE, ET LEUR UTILISATION

(30) Priorität: 14.04.2008 DE 102008018675
(43) Veröffentlichungstag der Anmeldung: 05.01.2011
(73) Patentinhaber: Bayer Pharma Aktiengesellschaft, 13353 Berlin (DE)
(72) Erfinder: HAHN, Michael, G., 40764 Langenfeld (DE); BECKER, Eva-Maria, 42327 Wuppertal (DE); KNORR, Andreas, 40699 Erkrath (DE); SCHNEIDER, Dirk, 42115 Wuppertal (DE); STASCH, Johannes-Peter, 42651 Solingen (DE); SCHLEMMER, Karl-Heinz, 42113 Wuppertal (DE); WUNDER, Frank, 42117 Wuppertal (DE); STOLL, Frederike, 40215 Düsseldorf (DE); HEITMEIER, Stefan, 42489 Wülfrath (DE); MÜNTER, Klaus, 42489 Wülfrath (DE); GRIEBENOW, Nils, 41541 Dormagen (DE); LAMPE, Thomas, 40545 Düsseldorf (DE); EL SHEIKH, Sherif, 45219 Essen (DE); LI, Volkhart Min-Jian, 42553 Velbert (DE)
(86) Internationale Anmeldenummer: PCT/EP2009/002510
(87) Internationale Veröffentlichungsnummer: WO 2009/127338

(56) Entgegenhaltungen:
- EP-A1- 0 779 279
- EP-A1- 0 802 192
- EVGENOV OLEG V ET AL: "NO-independent stimulators and activators of soluble guanylate cyclase: discovery and therapeutic potential." NATURE REVIEWS. DRUG DISCOVERY SEP 2006, Bd. 5, Nr. 9, September 2006 (2006-09), Seiten 755-768, XP002534424 ISSN: 1474-1776 in der Anmeldung erwähnt

## Beschreibung

Die vorliegende Anmeldung betrifft neue Carbonsäure-Derivate mit einer Oxo-substituierten azaheterocyclischen Partialstruktur, Verfahren zu ihrer Herstellung, ihre Verwendung zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention kardiovaskulärer Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriwetische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Innenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall und Myokardinfarkt führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisen-Zentralatom des Häms. Neben den Nebenwirkungen gehört die Toleranzentwicklung zu den entscheidenden Nachteilen dieser Behandlungsweise [O.V. Evgenov et al., Nature Rev. Drug Disc. 5 (2006), 755].

In den letzten Jahren wurden Substanzen identifiziert, die die lösliche Guanylatcyclase direkt, d.h. ohne vorherige Freisetzung von NO stimulieren. Mit dem Indazolderivat YC-1 wurde erstmals ein NO-unabhängiger, jedoch Häm-abhängiger Stimulator der sGC beschrieben [Evgenov et al., *ibid*.]. Ausgehend von YC-1 wurden weitere Substanzen gefunden, welche eine höhere Potenz als YC-1 besitzen und keine relevante Hemmung von Phosphodiesterasen (PDE) aufweisen. Dies führte zur Identifizierung der Pyrazolopyridin-Derivate BAY 41-2272, BAY 41-8543 und BAY 63-2521. Diese Verbindungen bilden gemeinsam mit den kürzlich publizierten, strukturell diversen Substanzen CMF-1571 und A-350619 die neue Klasse der sGC-Stimulatoren [Evgenov et al., *ibid*.]. Gemeinsames Charakteristikum dieser Substanzklasse ist eine NO-uriabhängige und selektive Aktivierung der hämhaltigen sGC. Darüber hinaus zeigen die sGC-Stimulatoren in Kombination mit NO einen synergistischen Effekt auf die sGC-Aktivierung, welcher auf einer Stabilisierung des Nitrosyl-Häm-Komplexes basiert. Die genaue Bindungsstelle der sGC-Stimulatoren an der sGC ist bis heute Gegenstand der Diskussion. Entfernt man von der löslichen Guanylatcyclase die HämGruppe, zeigt das Enzym immer noch eine nachweisbare katalytische Basalaktivität, d.h. es wird nach wie vor cGMP gebildet. Die verbleibende katalytische Basalaktivität des Häm-freien Enzyms ist durch keinen der vorstehend genannten Stimulatoren stimulierbar [Evgenov et al., *ibid.*].

Darüber hinaus wurden NO- und Häm-unabhängige sGC-Aktivatoren, mit BAY 58-2667 als Prototyp dieser Klasse, identifiziert. Gemeinsame Charakteristika dieser Substanzen sind, dass sie in Kombination mit NO nur einen additiven Effekt auf die Enzymaktivierung ausüben, und dass die Aktivierung des oxidierten oder hämfreien Enzyms im Vergleich zum hämhaltigen Enzym deutlich stärker ist [Evgenov et al., *ibid.*; J.P. Stasch et al., Br. J. Pharmacol. 136 (2002), 773; J.P. Stasch et al., J. Clin. Invest. 116 (2006), 2552]. Spektroskopische Untersuchungen lassen erkennen, dass BAY 58-2667 die oxidierte Hämgruppe verdrängt, die durch Schwächung der Eisen-Histidin-Bindung nur schwach an der sGC gebunden ist. Auch wurde gezeigt, dass das charakteristische sGC-Hämbindungsmotiv Tyr-x-Ser-x-Arg sowohl für die Interaktion der negativ geladenen Propionsäuren der Hämgruppe als auch für die Wirkung von BAY 58-2667 zwingend erforderlich ist. Vor diesem Hintergrund wird angenommen, dass die Bindungsstelle von BAY 58-2667 an der sGC identisch zur Bindungsstelle der Hämgruppe ist [J.P. Stasch et al., J. Clin. Invest. 116 (2006), 2552].

Die in der vorliegenden Erfindung beschriebenen Verbindungen sind nun ebenfalls in der Lage, die Häm-freie Form der löslichen Guanylatcyclase zu aktivieren. Dies wird auch dadurch belegt, dass diese neuartigen Aktivatoren einerseits am Häm-haltigen Enzym keine synergistische Wirkung mit NO zeigen und andererseits sich ihre Wirkung nicht durch den Häm-abhängigen Inhibitor der löslichen Guanylatcyclase, 1*H*-1,2,4-Oxadiazolo[4,3-*a*]chinoxalin-1-on (ODQ), blockieren lässt, sondern durch diesen sogar potenziert wird [vgl. O.V. Evgenov et al., Nature Rev. Drug Disc. 5 (2006), 755; J.P. Stasch et al., J. Clin. Invest. 116 (2006), 2552].

In WO 00/64876 und WO 00/64888 werden Di- und Triaryl-Säurederivate als PPAR-Liganden zur Behandlung von Diabetes, Hyperlipidämie, Atherosklerose und Hypertonie beansprucht. Weitere multicyclische Carbonsäure-Derivate werden in EP 1 357 115-A1, EP 1 394 154-A1 sowie EP 1 541 564-A1 als PPAR- und/oder RXR-Liganden für die Behandlung von Diabetes, Hyperlipidämie, Obesitas und Hypertonie beschrieben. Weiterhin sind multicyclische Säurederivate aus WO 91/19475 als Leukotrien-Antagonisten mit anti-inflammatorischen und anti-allergischen Eigenschaften bekannt. In EP 1 229 010-A1 werden bestimmte Diarylamid-Derivate zur Behandlung von Atherosklerose und Restenose offenbart. Ferner werden in EP 0 779 279-A1 und EP 0 802 192-A1 verschiedene Phenylacetamid-Derivate mit azaheterocyclischer Partialstruktur als Apolipoprotein B-Inhibitoren zur Behandlung von Atherosklerose und koronaren Herzerkrankungen beschrieben, und in EP 0 608 709-A1 werden 2-Oxochinolinylmethyl-substituierte Phenylacetamide als Angiotensin II-Antagonisten zur Behandlung von arterieller Hypertonie und Atherosklerose offenbart.

Aufgabe der vorliegenden Erfindung war die Bereitstellung neuer Verbindungen, welche in der oben beschriebenen Weise als Aktivatoren der löslichen Guanylatcyclase wirken und als solche insbesondere zur Behandlung und zur Prävention kardiovaskulärer Erkrankungen eingesetzt werden können.

Strukturell zeichnen sich die Verbindungen der vorliegenden Erfindung durch eine terminale Carbonsäure-Gruppierung aus, welche auf die im Folgenden dargestellte Weise mit einem Oxo-substituierten Aza-Heterocyclus als Kopfgruppe verknüpft ist.

Gegenstand der vorliegenden Erfindung sind Verbindungen der allgemeinen Formel (I) 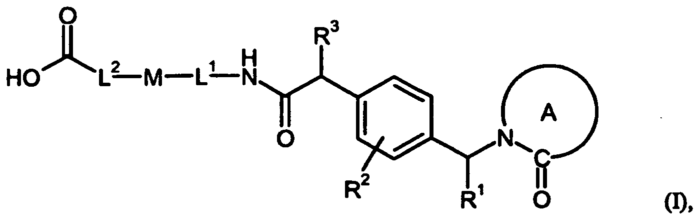 in welcher
der Ring A für einen N-verknüpften 5- bis 7-gliedrigen, gesättigten oder partiell ungesättigten, Oxo-substituierten Azaheterocyclus steht,
der (*i*) ein oder zwei weitere Heteroatome aus der Reihe N, O und/oder S als Ringglieder enthalten kann,
der (*ii*) mit einem Rest ausgewählt aus der Reihe Fluor, Chlor, (C₁-C₆)-Alkyl, Trifluormethyl, (C₃-C₇)-Cycloalkyl, 4- bis 7-gliedriges Heterocyclyl und Phenyl substituiert ist oder der benzo-anelliert ist,
wobei der Phenyl-Substituent und der anellierte Phenylring ihrerseits bis zu zweifach, gleich oder verschieden, mit einem Rest ausgewählt aus der Reihe Halogen, Cyano, (C₁-C₄)-Alkyl, (C₂-C₄)-Alkenyl, Trifluormethyl, (C₁-C₄)-Alkoxy und Trifluormethoxy substituiert sein können,
und
der (*iii*) darüber hinaus bis zu zweifach, gleich oder verschieden, mit weiteren Resten ausgewählt aus der Reihe Fluor, (C₁-C₆)-Alkyl, Trifluormethyl, Oxo, (C₃-C₇)-Cycloalkyl, 4- bis 7-gliedriges Heterocyclyl und Phenyl substituiert sein kann,
wobei Phenyl seinerseits bis zu zweifach, gleich oder verschieden, mit einem Rest ausgewählt aus der Reihe Halogen, Cyano, (C₁-C₄)-Alkyl, (C₂-C₄)-Alkenyl, Trifluormethyl, (C₁-C₄)-Alkoxy und Trifluormethoxy substituiert sein kann,
- R¹: für Wasserstoff, (C₁-C₄)-Alkyl oder Cyclopropyl steht,
- R²: für Wasserstoff, Halogen, Cyano, (C₁-C₄)-Alkyl oder Trifluormethyl steht,
- R³: für (C₃-C₆)-Alkyl oder (C₃-C₆)-Alkenyl, welche jeweils mit Cyano, (C₁-C₄)-Alkoxy oder Trifluormethoxy und bis zu sechsfach mit Fluor substituiert sein können,
oder
für (C₃-C₇)-Cycloalkyl oder (C₃-C₇)-Cycloalkenyl, welche jeweils bis zu zweifach, gleich oder verschieden, mit (C₁-C₄)-Alkyl, Trifluormethyl und (C₁-C₄)-Alkoxy sowie bis zu vierfach mit Fluor substituiert sein können,
oder
für Oxetanyl, Tetrahydrofuranyl oder Tetrahydropyranyl steht,
- L¹: für eine Bindung oder für Methylen, Ethan-1,2-diyl oder Propan-1,3-diyl, welche jeweils bis zu zweifach, gleich oder verschieden, mit (C₁-C₄)-Alkyl substituiert sein können, steht,
- L²: für eine Bindung oder für Methylen, Ethan-1,2-diyl, Propan-1,3-diyl, Ethen-1,2-diyl oder Propen-1,3-diyl, welche jeweils bis zu zweifach, gleich oder verschieden, mit (C₁-C₄)-Alkyl substituiert sein können, steht
oder
für eine Gruppe der Formel 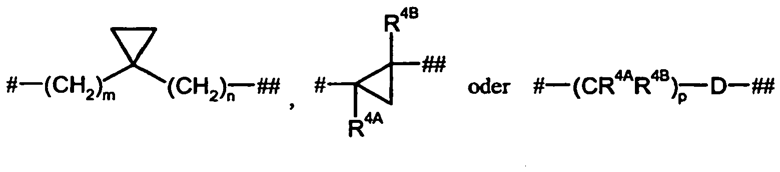 steht,
worin
- #: die Verknüpfungsposition mit der Carbonsäure-Gruppierung kennzeichnet,
- ##: die Verknüpfungsposition mit der Gruppe M kennzeichnet,
- m: die Zahl 0 oder 1 bedeutet,
- n: die Zahl 0, 1 oder 2 bedeutet,
- p: die Zahl 1 oder 2 bedeutet,
- D: O oder S bedeutet
- und R^{4A} und R^{4B}: unabhängig voneinander Wasserstoff oder (C₁-C₄)-Alkyl bedeuten,
wobei im Falle, dass die Gruppe -CR^{4A}R^{4B}- zweifach auftritt, die einzelnen Bedeutungen von R^{4A} und R^{4B} jeweils gleich oder verschieden sein können,
- M: für Phenylen oder 5- oder 6-gliedriges Heteroarylen mit bis zu zwei Ring-Heteroatomen aus der Reihe N, O und/oder S steht, wobei Phenylen und Heteroarylen jeweils bis zu zweifach, gleich oder verschieden, mit einem Rest ausgewählt aus der Reihe Halogen, Cyano, (C₁C₄)-Alkyl, Trifluormethyl, Hydroxy, (C₁-C₄)-Alkoxy und Trifluormethoxy substituiert sein können,
oder
für Cyclopropan-1,2-diyl, Cyclobutan-1,2-diyl, Cyclobutan-1,3-diyl, Cyclopentan-1,2-diyl, Cyclopentan-1,3-diyl, Cyclohexan-1,2-diyl, Cyclohexan-1,3-diyl oder Cyclohexan-1,4-diyl steht, welche jeweils bis zu zweifach, gleich oder verschieden, mit einem Rest ausgewählt aus der Reihe Fluor, (C₁-C₄)-Alkyl, Trifluormethyl und (C₁-C₄)-Alkoxy substituiert sein können,
- oder L² und M: miteinander verknüpft sind und zusammen eine Gruppe der Formel 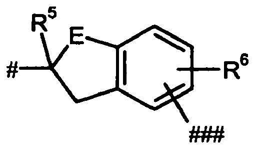 bilden, worin
- #: die Verknüpfungsposition mit der Carbonsäure-Gruppierung kennzeichnet,
- ###: die Verknüpfungsposition mit der Gruppe L¹ kennzeichnet,
- E: O, S, CH₂ oder CH₂CH₂ bedeutet,
- R⁵: Wasserstoff, (C₁-C₄)-Alkyl oder Trifluormethyl bedeutet
- und R⁶: Wasserstoff, Halogen, (C₁-C₄)-Alkyl, Trifluormethyl, (C₁-C₄)-Alkoxy oder Trifluormethoxy bedeutet,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäwe, Toluolsulfonsäwe, Benzolsulfonsäure, Naphthalindisulfonsäwe, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch hydrolysierbare Ester-Derivate der erfindungsgemäßen Carbonsäuren der Formel (I). Hierunter werden Ester verstanden, die in physiologischen Medien, unter den Bedingungen der im weiteren beschriebenen biologischen Tests und insbesondere *in vivo* auf enzymatischem oder chemischem Wege zu den freien Carbonsäuren, als den biologisch hauptsächlich aktiven Verbindungen, hydrolysiert werden können. Als solche Ester werden (C₁-C₄)-Alkylester, in welchen die Alkylgruppe geradkettig oder verzweigt sein kann, bevorzugt. Besonders bevorzugt sind Methyl-, Ethyl- oder *tert*.-Butylester.

Im Rahmen der vorliegenden Erfindung haben die Substituenten, soweit nicht anders spezifiziert, **die folgende Bedeutung:**
(C₁-C₆)-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, *n*-Propyl, Isopropyl, *n*-Butyl, *iso*-Butyl, *sec*.-Butyl, *tert*.-Butyl, *n*-Pentyl, 2-Pentyl, 3-Pentyl, *n*-Hexyl, 2-Hexyl und 3-Hexyl.
(C₁-C₆)-Alkyl steht im Rahmen der Erfindung für einen geradkettigen oder verzweigten Alkylrest mit 3 bis 6 Kohlenstoffatomen. Bevorzugt ist ein verzweigter Alkylrest mit 3 bis 5 Kohlenstoff atomen. Beispielhaft und vorzugsweise seien genannt: *n*-Propyl, Isopropyl, *n*-Butyl, *iso*-Butyl, *sec*.-Butyl, *tert.*-Butyl, *n*-Pentyl, 2-Pentyl, 3-Pentyl, *n*-Hexyl, 2-Hexyl und 3-Hexyl.
(C₃-C₆)-Alkenyl und (C₂-C₄)-Alkenyl stehen im Rahmen der Erfindung für einen geradkettigen oder verzweigten Alkenylrest mit einer Doppelbindung und 3 bis 6 bzw. 2 bis 4 Kohlenstoff atomen. Bevorzugt ist ein verzweigter Alkenylrest mit 3 bis 5 Kohlenstoffatomen bzw. ein geradkettiger Alkenylrest mit 2 oder 3 Kohlenstoffatomen. Beispielhaft und vorzugsweise seien genannt: Vinyl, Allyl, Isopropenyl, *n*-But-2-en-1-yl, 2-Methylprop-2-en-1-yl und *n*-But-3-en-1-yl.
(C₁-C₄)-Alkoxy steht im Rahmen der Erfindung für einen geradkettigen oder verzweigten Alkoxyrest mit 1 bis 4 Kohlenstoffatomen. Beispielhaft und vorzugsweise seien genannt: Methoxy, Ethoxy, *n*-Propoxy, Isopropoxy, n-Butoxy und *tert*.-Butoxy.
(C₃-C₇)-Cycloalkyl und (C₃-C₆)-Cycloalkyl stehen im Rahmen der Erfindung für eine monocyclische, gesättigte Cycloalkylgruppe mit 3 bis 7 bzw. 3 bis 6 Kohlenstoffatomen. Bevorzugt ist ein Cycloalkylrest mit 3 bis 6 Kohlenstoffatomen. Beispielhaft und vorzugsweise seien genannt: Cyclopropyl, Cyclobutyl, Cyclopentyl, Cyclohexyl und Cycloheptyl.
(C₃-C₇)-Cycloalkenyl und (C₄-C₆)-Cycloalkenyl stehen im Rahmen der Erfindung für eine monocyclische Cycloalkylgruppe mit 3 bis 7 bzw. 4 bis 6 Ring-Kohlenstoffatomen und einer Ring-Doppelbindung. Bevorzugt ist ein Cycloalkenylrest mit 4 bis 6, besonders bevorzugt mit 5 oder 6 Kohlenstoffatomen. Beispielhaft und vorzugsweise seien genannt: Cyclopropenyl, Cyclobutenyl, Cyclopentenyl, Cyclohexenyl und Cycloheptenyl.
4- bis 7-gliedriges Heterocyclyl und 4- bis 6-gliedriges Heterocyclyl stehen im Rahmen der Erfindung für einen monocyclischen, gesättigten Heterocyclus mit insgesamt 4 bis 7 bzw. 4 bis 6 Ringatomen, der ein oder zwei Ring-Heteroatome aus der Reihe N, O und/oder S enthält und über ein Ring-Kohlenstoffatom oder gegebenenfalls ein Ring-Stickstoffatom verknüpft ist. Bevorzugt ist 4-bis 6-gliedriges Heterocyclyl mit ein oder zwei Ring-Heteroatomen aus der Reihe N und/oder O. Beispielhaft seien genannt: Azetidinyl, Oxetanyl, Pyrrolidinyl, Pyrazolidinyl, Tetrahydrofuranyl, Piperidinyl, Piperazinyl, Tetrahydropyranyl, Morpholinyl, Thiomorpholinyl, Hexahydroazepinyl und Hexahydro-1,4-diazepinyl. Bevorzugt sind Azetidinyl, Oxetanyl, Pyrrolidinyl, Tetrahydrofuranyl, Piperidinyl, Piperazinyl, Tetrahydropyranyl und Morpholinyl.
5- oder 6-gliedriges Heteroarylen steht im Rahmen der Erfindung für einen divalenten aromatischen Heterocyclus (Heteroaromaten) mit insgesamt 5 oder 6 Ringatomen, der ein oder zwei Ring-Heteroatome aus der Reihe N, O und/oder S enthält und über Ring-Kohlenstoffatome und/oder gegebenenfalls ein Ring-Stickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Pyridyl, Pyrimidinyl, Pyridazinyl und Pyrazinyl. Bevorzugt sind Furyl, Thienyl, Thiazolyl, Oxazolyl, Isoxazolyl, Pyridyl und Pyrimidinyl.
Halogen schließt im Rahmen der Erfindung Fluor, Chlor, Brom und Iod ein. Bevorzugt sind Chlor, Fluor oder Brom, besonders bevorzugt Fluor oder Chlor.

Ein Oxo-Substituent steht im Rahmen der Erfindung für ein Sauerstoffatom, das über eine Doppelbindung an ein Kohlenstoffatom gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Besonders bevorzugt ist die Substitution mit einem oder zwei Substituenten.

Gegenstand der vorliegenden Erfindung sind insbesondere Verbindungen der Formel (I), in welcher
der Ring A für einen Oxo-substituierten Azaheterocyclus der Formel 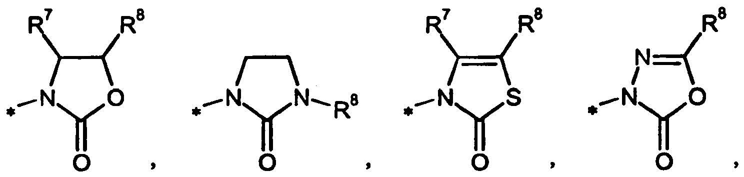 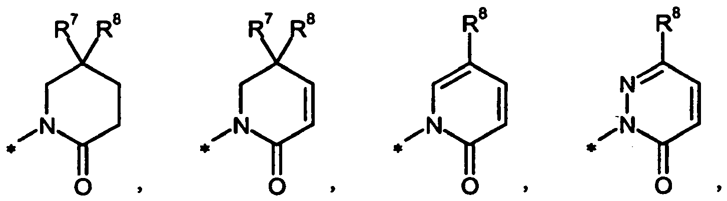 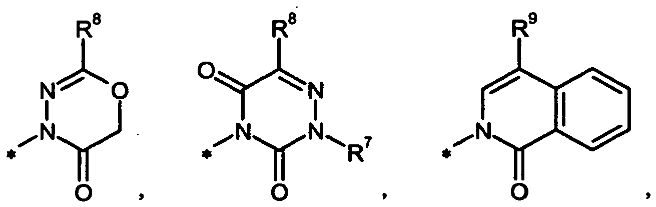 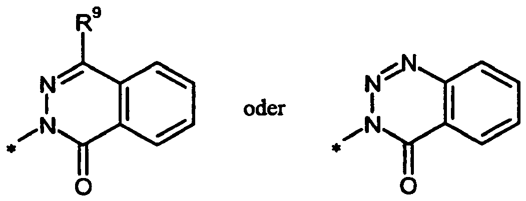 steht, worin
- *: die Verknüpfungsposition mit dem restlichen Teil des Moleküls kennzeichnet,
- R⁷: Wasserstoff, (C₁-C₆)-Alkyl oder (C₃-C₆)-Cycloalkyl bedeutet,
- R⁸: (C₁-C₆)-Alkyl, Trifluormethyl, (C₃-C₆)-Cycloalkyl, 4- bis 6-gliedriges Heterocyclyl oder Phenyl bedeutet, wobei Phenyl seinerseits bis zu zweifach, gleich oder ver- schieden, mit einem Rest ausgewählt aus der Reihe Fluor, Chlor, Brom, Cyano, (C₁-C₄)-Alkyl, Vinyl, Trifluormethyl, (C₁-C₄)-Alkoxy und Trifluormethoxy substituiert sein kann,
- und R⁹: Wasserstoff bedeutet oder die oben genannte Bedeutung von R⁸ hat,
sowie ihre Salze, Solvate und Solvate der Salze.

Gegenstand der vorliegenden Erfindung sind insbesondere auch Verbindungen der Formel (I), in welcher
der Ring A für einen Oxo-substituierten Azaheterocyclus der Formel 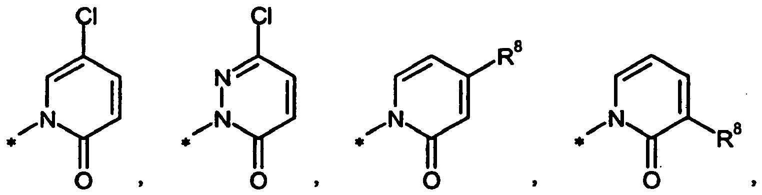 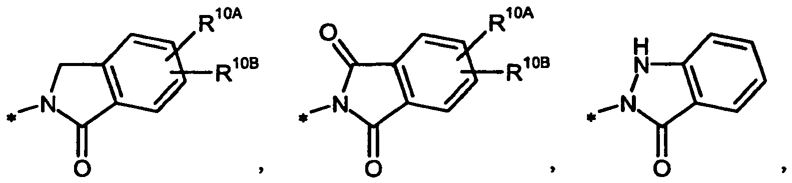 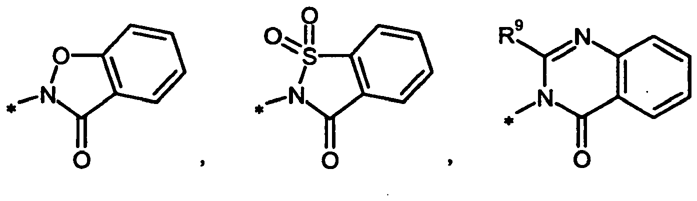 oder 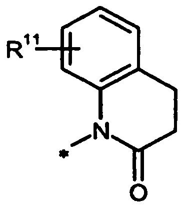 steht, worin
- *: die Verknüpfungsposition mit dem restlichen Teil des Moleküls kennzeichnet,
- R⁸: (C₁-C₆)-Alkyl, Trifluormethyl, (C₃-C₆)-Cycloalkyl, 4- bis 6-gliedriges Heterocyclyl oder Phenyl bedeutet, wobei Phenyl seinerseits bis zu zweifach, gleich oder verschieden, mit einem Rest ausgewählt aus der Reihe Fluor, Chlor, Brom, Cyano, (C₁-C₄)-Alkyl, Vinyl, Trifluormethyl, (C₁-C₄)-Alkoxy und Trifluormethoxy substituiert sein kann,
- R⁹: Wasserstoff bedeutet oder die oben genannte Bedeutung von R⁸ hat,
- R^{10A} und R^{10B}: unabhängig voneinander Wasserstoff, Fluor oder Chlor bedeuten
- und R¹¹: Wasserstoff, Fluor oder Chlor bedeutet,
sowie ihre Salze, Solvate und Solvate der Salze.

Bevorzugt im Rahmen der vorliegenden Erfindung sind Verbindungen der Formel (I), in welcher der Ring A für einen Oxo-substituierten Azaheterocyclus der Formel 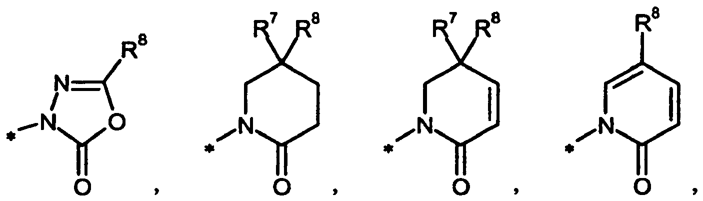 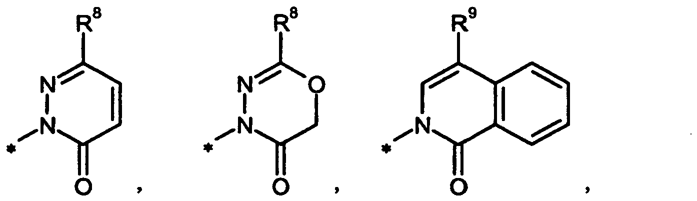 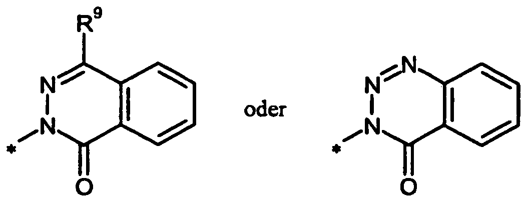 steht, worin
- *: die Verknüpfungsposition mit dem restlichen Teil des Moleküls kennzeichnet,
- R⁷: Wasserstoff oder (C₁-C₄)-Alkyl bedeutet,
- R⁸: (C₁-C₆)-Alkyl, Trifluormethyl, (C₃-C₆)-Cycloalkyl oder Phenyl bedeutet, wobei Phenyl seinerseits bis zu zweifach, gleich oder verschieden, mit einem Rest ausgewählt aus der Reihe Fluor, Chlor, Cyano, (C₁-C₄)-Alkyl, Trifluormethyl, (C₁-C₄)-Alkoxy und Trifluormethoxy substituiert sein kann,
- und R⁹: Wasserstoff bedeutet oder die zuvor genannte Bedeutung von R⁸ hat,

- R¹: für Wasserstoff oder (C₁-C₄)-Alkyl steht,
- R²: für Wasserstoff, Fluor, Chlor oder Trifluormethyl steht,
- R³: für (C₃-C₆)-Alkyl oder (C₃-C₆)-Alkenyl, welche jeweils mit Cyano, Methoxy, Ethoxy oder Trifluormethoxy und bis zu sechsfach mit Fluor substituiert sein können,
oder
für (C₃-C₆)-Cycloalkyl oder (C₄-C₆)-Cycloalkenyl, welche jeweils bis zu zweifach, gleich oder verschieden, mit (C₁-C₄)-Alkyl und Trifluormethyl sowie bis zu vierfach mit Fluor substituiert sein können,
oder
für Oxetanyl steht,
- L¹: für eine Bindung oder für Methylen, Ethan-1,2-diyl oder Propan-1,3-diyl, welche jeweils bis zu zweifach mit Methyl substituiert sein können, steht,
- L²: für eine Bindung oder für Methylen, Ethan-1,2-diyl, Propan-1,3-diyl oder Ethen-1,2-diyl, welche jeweils bis zu zweifach mit Methyl substituiert sein können, steht
oder
für eine Gruppe der Formel 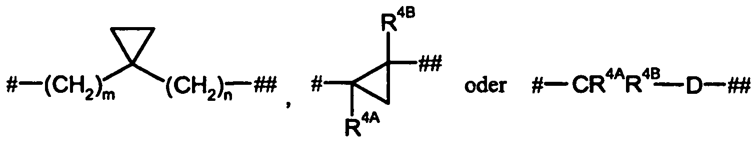 steht,
worin
- #: die Verknüpfungsposition mit der Carbonsäure-Gruppierung kennzeichnet,
- ##: die Verknüpfungsposition mit der Gruppe M kennzeichnet,
- m: die Zahl 0 oder 1 bedeutet,
- n: die Zahl 0, 1 oder 2 bedeutet,
- D: O oder S bedeutet
und
- R^{4A} und R^{4B}: unabhängig voneinander Wasserstoff oder Methyl bedeuten,

- M: für Phenylen oder 5- oder 6-gliedriges Heteroarylen mit bis zu zwei Ring-Heteroatomen aus der Reihe N, O und/oder S steht, wobei Phenylen und Heteroarylen jeweils bis zu zweifach, gleich oder verschieden, mit einem Rest ausgewählt aus der Reihe Fluor, Chlor, (C₁-C₄)-Alkyl, Trifluormethyl, Methoxy und Trifluormethoxy substituiert sein können, oder für Cyclopentan-1,3-diyl, Cyclohexan-1,3-diyl oder Cyclohexan-1,4-diyl steht, welche jeweils bis zu zweifach, gleich oder verschieden, mit einem Rest ausgewählt aus der Reihe Fluor, Methyl und Trifluormethyl substituiert sein können,
- oder L² und M: miteinander verknüpft sind und zusammen eine Gruppe der Formel 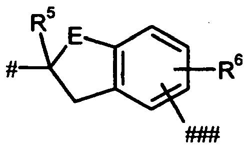 bilden, worin
# die Verknüpfungsposition mit der Carbonsäure-Gruppierung kennzeichnet,
### die Verknüpfungsposition mit der Gruppe L¹ kennzeichnet,
E CH₂ oder CH₂CH₂ bedeutet,
R⁵ Wasserstoff, Methyl oder Trifluormethyl bedeutet
und R⁶ Wasserstoff, Fluor, Chlor, Methyl, Trifluormethyl, Methoxy oder Trifluormethoxy bedeutet,
sowie ihre Salze, Solvate und Solvate der Salze.

Bevorzugt im Rahmen der vorliegenden Erfindung sind auch Verbindungen der Formel (I), in welcher
der Ring A für einen Oxo-substituierten Azaheterocyclus der Formel 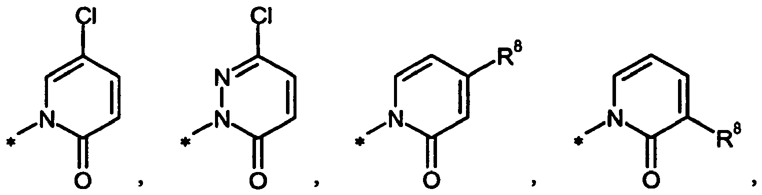 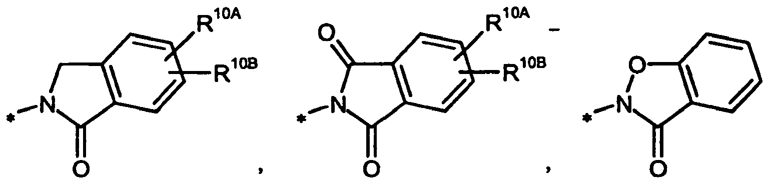 oder 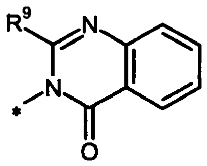 steht, worin
- *: die Verknüpfungsposition mit dem restlichen Teil des Moleküls kennzeichnet,
- R⁸: (C₁-C₆)-Alkyl, Trifluormethyl, (C₃-C₆)-Cycloalkyl oder Phenyl bedeutet, wobei Phenyl seinerseits bis zu zweifach, gleich oder verschieden, mit einem Rest ausge- wählt aus der Reihe Fluor, Chlor, Cyano, (C₁-C₄)-Alkyl, Trifluormethyl, (C₁-C₄)-Alkoxy und Trifluormethoxy substituiert sein kann,
- R⁹: Wasserstoff bedeutet oder die zuvor genannte Bedeutung von R⁸ hat
- und R^{10A} und R^{10B}: unabhängig voneinander Wasserstoff, Fluor oder Chlor bedeuten,

- R¹: für Wasserstoff oder (C₁-C₄)-Alkyl steht,
- R²: für Wasserstoff, Fluor, Chlor oder Trifluormethyl steht,
- R³: für (C₃-C₆)-Alkyl oder (C₃-C₆)-Alkenyl, welche jeweils mit Cyano, Methoxy, Ethoxy oder Trifluormethoxy und bis zu sechsfach mit Fluor substituiert sein können,
oder
für (C₃-C₆)-Cycloalkyl oder (C₄-C₆)-Cycloalkenyl, welche jeweils bis zu zweifach, gleich oder verschieden, mit (C₁-C₄)-Alkyl und Trifluormethyl sowie bis zu vierfach mit Fluor substituiert sein können,
oder
für Oxetanyl steht,
- L¹: für eine Bindung oder für Methylen, Ethan-1,2-diyl oder Propan-1,3-diyl, welche jeweils bis zu zweifach mit Methyl substituiert sein können, steht,
- L²: für eine Bindung oder für Methylen, Ethan-1,2-diyl, Propan-1,3-diyl oder Ethen-1,2-diyl, welche jeweils bis zu zweifach mit Methyl substituiert sein können, steht
oder
für eine Gruppe der Formel 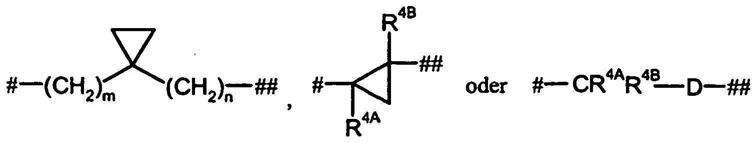 steht,
worin
# die Verknüpfungsposition mit der Carbonsäure-Gruppierung kennzeichnet,
## die Verknüpfungsposition mit der Gruppe M kennzeichnet,
m die Zahl 0 oder 1 bedeutet,
n die Zahl 0, 1 oder 2 bedeutet,
D O oder S bedeutet
und R^{4A} und R^{4B} unabhängig voneinander Wasserstoff oder Methyl bedeuten,
- M: für Phenylen oder 5- oder 6-gliedriges Heteroarylen mit bis zu zwei Ring-Heteroatomen aus der Reihe N, O und/oder S steht, wobei Phenylen und Heteroarylen jeweils bis zu zweifach, gleich oder verschieden, mit einem Rest ausgewählt aus der Reihe Fluor, Chlor, (C₁-C₄)-Alkyl, Trifluormethyl, Methoxy und Trifluormethoxy substituiert sein können,
oder
für Cyclopentan-1,3-diyl, Cyclohexan-1,3-diyl oder Cyclohexan-1,4-diyl steht, welche jeweils bis zu zweifach, gleich oder verschieden, mit einem Rest ausgewählt aus der Reihe Fluor, Methyl und Trifluormethyl substituiert sein können,
- oder L² und M: miteinander verknüpft sind und zusammen eine Gruppe der Formel 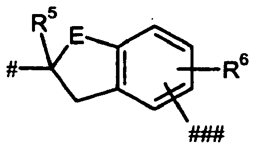 bilden, worin
# die Verknüpfungsposition mit der Carbonsäure-Gruppierung kennzeichnet,
### die Verknüpfungsposition mit der Gruppe L¹ kennzeichnet,
E CH₂ oder CH₂CH₂ bedeutet,
R⁵ und Wasserstoff, Methyl oder Trifluormethyl bedeutet
R⁶ Wasserstoff, Fluor, Chlor, Methyl, Trifluormethyl, Methoxy oder Trifluormethoxy bedeutet,
sowie ihre Salze, Solvate und Solvate der Salze.

Besonders bevorzugt im Rahmen der vorliegenden Erfindung sind Verbindungen der Formel (I), in welcher
der Ring A für einen Oxo-substituierten Azaheterocyclus der Formel 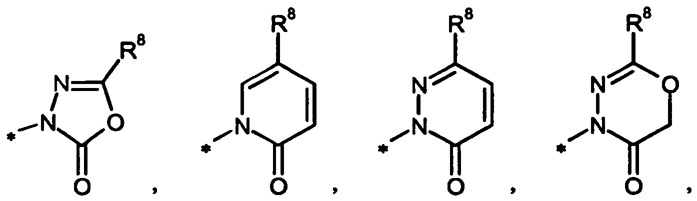 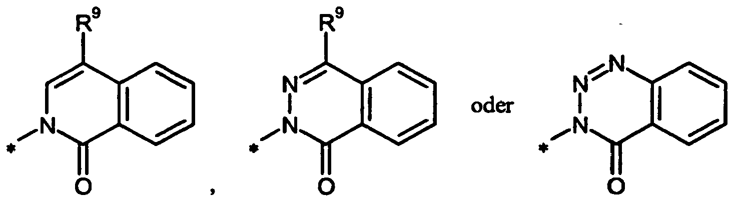 steht, worin
- *: die Verknüpfungsposition mit dem restlichen Teil des Moleküls kennzeichnet,
- R⁸: Trifluormethyl, (C₃-C₆)-Cycloalkyl oder Phenyl bedeutet, wobei Phenyl seinerseits bis zu zweifach, gleich oder verschieden, mit einem Rest ausgewählt aus der Reihe Fluor, Chlor, Cyano, Methyl, Trifluormethyl, Methoxy und Trifluormethoxy substituiert sein kann,
- und R⁹: Wasserstoff bedeutet oder die zuvor genannte Bedeutung von R⁸ hat,

- R¹: für Wasserstoff oder Methyl steht,
- R²: für Wasserstoff, Fluor oder Chlor steht,
- R³: für (C₃-C₆)-Alkyl oder (C₃-C₆)-Alkenyl, welche jeweils bis zu sechsfach mit Fluor substituiert sein können, steht
oder
für (C₃-C₆)-Cycloalkyl, Cyclopentenyl oder Cyclohexenyl, welche jeweils bis zu zweifach, gleich oder verschieden, mit Methyl, Ethyl und Trifluormethyl sowie bis zu vierfach mit Fluor substituiert sein können, steht,
- L¹: für eine Bindung oder für Methylen oder Ethan-1,2-diyl steht,
- L²: für eine Bindung oder für Methylen, Ethan-1,2-diyl, Propan-1,3-diyl oder Ethen-1,2-diyl, welche jeweils bis zu zweifach mit Methyl substituiert sein können, steht oder für eine Gruppe der Formel 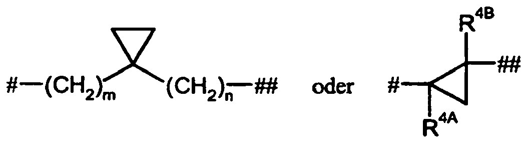 steht,
worin
# die Verknüpfungsposition mit der Carbonsäure-Gruppierung kennzeichnet,
## die Verknüpfungsposition mit der Gruppe M kennzeichnet,
m die Zahl 0 oder 1 bedeutet,
n die Zahl 1 oder 2 bedeutet,
und R^{4A} und R^{4B} unabhängig voneinander Wasserstoff oder Methyl bedeuten,
- M: für Phenylen, Pyridylen, Furylen, Thienylen, Thiazolylen oder Isoxazolylen, welche jeweils bis zu zweifach, gleich oder verschieden, mit einem Rest ausgewählt aus der Reihe Fluor, Chlor, Methyl und Trifluormethyl substituiert sein können, steht
oder
für Cyclopentan-1,3-diyl, Cyclohexan-1,3-diyl oder Cyclohexan-1,4-diyl, welche jeweils bis zu zweifach, gleich oder verschieden, mit Fluor und Methyl substituiert sein können, steht,
- oder L² und M: miteinander verknüpft sind und zusammen eine Gruppe der Formel 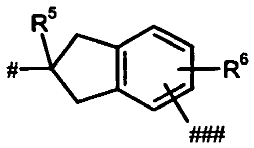 bilden, worin
# die Verknüpfungsposition mit der Carbonsäure-Gruppierung kennzeichnet,
### die Verknüpfungsposition mit der Gruppe L¹ kennzeichnet,
R⁵ Wasserstoff, Methyl oder Trifluormethyl bedeutet
und R⁶ Wasserstoff, Fluor oder Chlor bedeutet,
sowie ihre Salze, Solvate und Solvate der Salze.

Besonders bevorzugt im Rahmen der vorliegenden Erfindung sind auch Verbindungen der Formel (I), in welcher
der Ring A für einen Oxo-substituierten Azaheterocyclus der Formel 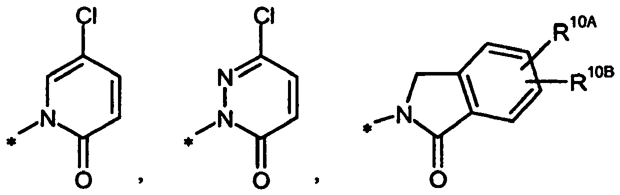 oder 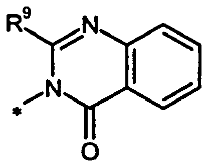 steht, worin
- *: die Verknüpfungsposition mit dem restlichen Teil des Moleküls kennzeichnet,
- R⁹: Wasserstoff, Methyl oder Trifluormethyl bedeutet
und
R^{10A} und R^{10B} unabhängig voneinander Wasserstoff oder Fluor bedeuten,
- R¹: für Wasserstoff oder Methyl steht,
- R²: für Wasserstoff, Fluor oder Chlor steht,
- R³: für (C₃-C₆)-Alkyl oder (C₃-C₆)-Alkenyl, welche jeweils bis zu sechsfach mit Fluor substituiert sein können, steht
oder
für (C₃-C₆)-Cycloalkyl, Cyclopentenyl oder Cyclohexenyl, welche jeweils bis zu zweifach, gleich oder verschieden, mit Methyl, Ethyl und Trifluormethyl sowie bis zu vierfach mit Fluor substituiert sein können, steht,
- L¹: für eine Bindung oder für Methylen oder Ethan-1,2-diyl steht,
- L²: für eine Bindung oder für Methylen, Ethan-1,2-diyl, Propan-1,3-diyl oder Ethen-1,2-diyl, welche jeweils bis zu zweifach mit Methyl substituiert sein können, steht
oder
für eine Gruppe der Formel 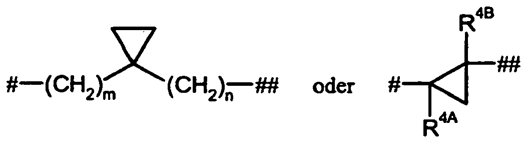 steht,
worin
# die Verknüpfungsposition mit der Carbonsäure-Gruppierung kennzeichnet,
## die Verknüpfungsposition mit der Gruppe M kennzeichnet,
m die Zahl 0 oder 1 bedeutet,
n die Zahl 1 oder 2 bedeutet,
und R^{4A} und R^{4B} unabhängig voneinander Wasserstoff oder Methyl bedeuten,
- M: für Phenylen, Pyridylen, Furylen, Thienylen, Thiazolylen oder Isoxazolylen, welche jeweils bis zu zweifach, gleich oder verschieden, mit einem Rest ausgewählt aus der Reihe Fluor, Chlor, Methyl und Trifluormethyl substituiert sein können, steht oder für Cyclopentan-1,3-diyl, Cyclohexan-1,3-diyl oder Cyclohexan-1,4-diyl, welche jeweils bis zu zweifach, gleich oder verschieden, mit Fluor und Methyl substituiert sein können, steht,
- oder L² und M: miteinander verknüpft sind und zusammen eine Gruppe der Formel 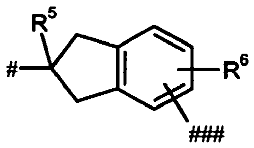 bilden, worin
# die Verknüpfungsposition mit der Carbonsäure-Gruppierung kennzeichnet,
### die Verknüpfungsposition mit der Gruppe L¹ kennzeichnet,
R⁵ Wasserstoff, Methyl oder Trifluormethyl bedeutet
und R⁶ Wasserstoff, Fluor oder Chlor bedeutet,
sowie ihre Salze, Solvate und Solvate der Salze.

Ganz besonders bevorzugt im Rahmen der vorliegenden Erfindung sind Verbindungen der Formel (I), in welcher
der Ring A für einen Oxo-substituierten Azaheterocyclus der Formel 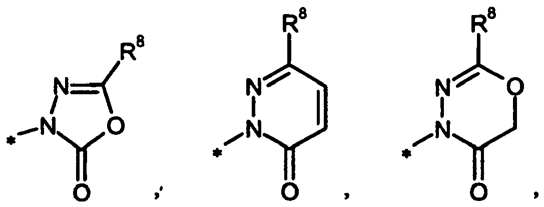 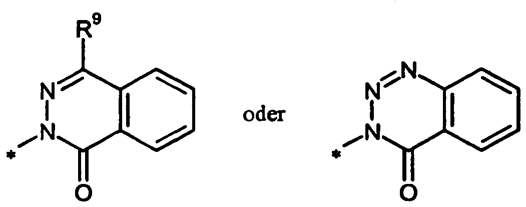 steht, worin
- *: die Verknüpfungsposition mit dem restlichen Teil des Moleküls kennzeichnet,
- R⁸: Trifluormethyl oder Phenyl, welches bis zu zweifach, gleich oder verschieden, mit einem Rest ausgewählt aus der Reihe Fluor, Chlor, Methyl und Trifluormethyl substituiert sein kann, bedeutet
- und R⁹: Wasserstoff bedeutet oder die zuvor genannte Bedeutung von R⁸ hat,

- R¹: für Wasserstoff steht,
- R²: für Wasserstoff steht,
- R³: für Propan-2-yl, Butan-2-yl, Pentan-2-yl, 1,1,1-Trifluorpropan-2-yl, 1,1,1-Trifluorbutan-2-yl, 4,4,4-Trifluorbutan-2-yl, 4,4,4-Trifluor-2-methylbutan-1-yl, Cyclopentyl oder 3,3-Difluorcyclopentyl steht,
- L¹: für eine Bindung oder für Methylen steht,
- L²: für eine Bindung, für Methylen oder Ethan-1,2-diyl, welche jeweils bis zu zweifach mit Methyl substituiert sein können, oder für Ethen-1,2-diyl steht
oder für eine Gruppe der Formel 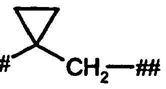 steht, worin
# die Verknüpfungsposition mit der Carbonsäure-Gruppierung
und ## die Verknüpfungsposition mit der Gruppe M kennzeichnen,
- M: für 1,3-Phenylen oder 1,4-Phenylen, welche bis zu zweifach, gleich oder verschieden, mit Fluor, Chlor, Methyl und Trifluormethyl substituiert sein können, oder für Cyclohexan-1,3-diyl oder Cyclohexan-1,4-diyl steht,
- oder L² und M: miteinander verknüpft sind und zusammen eine Gruppe der Formel 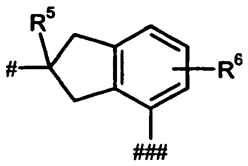 bilden, worin
# die Verknüpfungsposition mit der Carbonsäure-Gruppierung kennzeichnet,
### die Verknüpfungsposition mit der Gruppe L¹ kennzeichnet,
R⁵ Wasserstoff oder Methyl bedeutet
und R⁶ Wasserstoff oder Fluor bedeutet,
sowie ihre Salze, Solvate und Solvate der Salze.

Ganz besonders bevorzugt im Rahmen der vorliegenden Erfindung sind auch Verbindungen der Formel (I), in welcher
der Ring A für einen Oxo-substituierten Azaheterocyclus der Formel 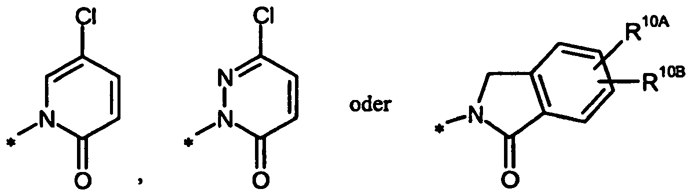 steht, worin
- * und: die Verknüpfungsposition mit dem restlichen Teil des Moleküls kennzeichnet
- R^{10A} und R^{10B}: unabhängig voneinander Wasserstoff oder Fluor bedeuten,

- R¹: für Wasserstoff steht,
- R²: für Wasserstoff steht,
- R³: für Propan-2-yl, Butan-2-yl, Pentan-2-yl, 1,1,1-Trifluorpropan-2-yl, 1,1,1-Trifluorbutan-2-yl, 4,4,4-Trifluorbutan-2-yl, 4,4,4-Trifluor-2-methylbutan-1-yl, Cyclopentyl oder 3,3-Difluorcyclopentyl steht,
- L¹: für eine Bindung steht,
- L²: für Methylen oder Ethan-1,2-diyl, welche jeweils bis zu zweifach mit Methyl substituiert sein können, oder für Ethen-1,2-diyl steht
oder
für eine Gruppe der Formel 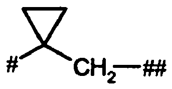 steht, worin
# die Verknüpfungsposition mit der Carbonsäure-Gruppierung
und ## die Verknüpfungsposition mit der Gruppe M kennzeichnen,
- M oder: für 1,3-Phenylen oder 1,4-Phenylen, welche bis zu zweifach, gleich oder verschieden, mit Fluor, Chlor, Methyl und Trifluormethyl substituiert sein können, steht,
- L² und M: miteinander verknüpft sind und zusammen eine Gruppe der Formel 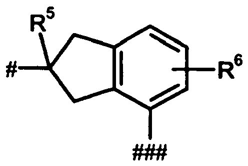 bilden, worin
# die Verknüpfungsposition mit der Carbonsäure-Gruppierung kennzeichnet,
### die Verknüpfungsposition mit der Gruppe L¹ kennzeichnet,
R⁵ Wasserstoff oder Methyl bedeutet
und R⁶ Wasserstoff oder Fluor bedeutet,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichnet, dass man zunächst eine Verbindung der Formel (II) 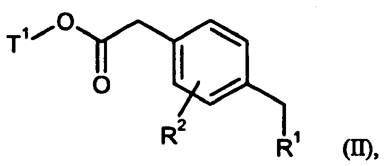 in welcher R¹ und R² die oben angegebenen Bedeutungen haben
und
- T¹: für (C₁-C₄)-Alkyl steht,
in einem inerten Lösungsmittel in Gegenwart einer Base mit einer Verbindung der Formel (III) 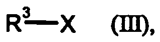 in welcher R³ die oben angegebene Bedeutung hat
und
- X: für eine Abgangsgruppe wie beispielsweise Halogen, Mesylat, Tosylat oder Triflat steht,
in eine Verbindung der Formel (IV) 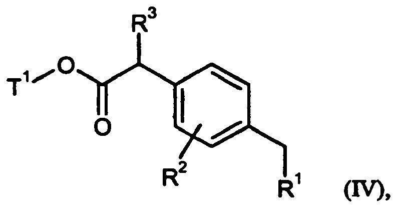 in welcher R¹, R², R³ und T¹ jeweils die oben angegebenen Bedeutungen haben,
überführt, diese danach in einem inerten Lösungsmittel mit elementarem Brom oder mit N-Bromsuccinimid zu einer Verbindung der Formel (V) 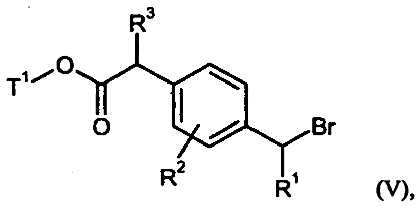 in welcher R¹, R², R³ und T¹ jeweils die oben angegebenen Bedeutungen haben,
bromiert, anschließend in einem inerten Lösungsmittel in Gegenwart einer Base mit einer Verbindung der Formel (VI) 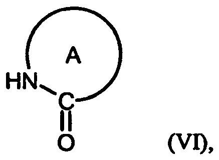 in welcher der Ring A für einen Oxo-substituierten Azaheterocyclus, wie oben definiert, steht,
zu einer Verbindung der Formel (VII) 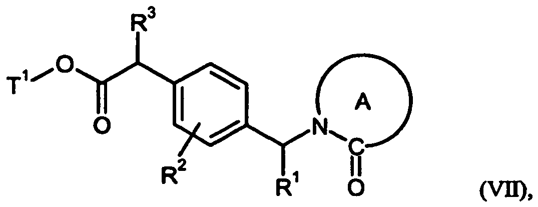 in welcher der Ring A, R¹, R², R³ und T¹ jeweils die oben angegebenen Bedeutungen haben,
umsetzt, nachfolgend den Ester-Rest T¹ in (VII) unter basischen oder sauren Bedingungen abspaltet, die resultierende Carbonsäure der Formel (VIII) 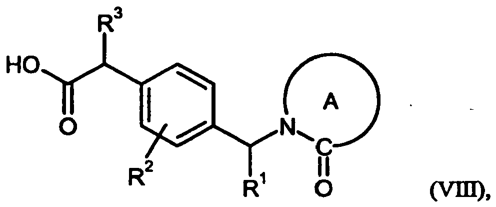 in welcher der Ring A, R¹, R² und R³ jeweils die oben angegebenen Bedeutungen haben,
dann in einem inerten Lösungsmittel in Gegenwart eines Kondensationsmittels oder über die Zwischenstufe des entsprechenden Carbonsäurechlorids in Gegenwart einer Base mit einem Amin der Formel (IX) 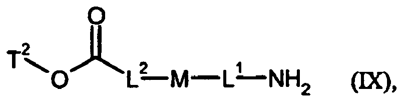 in welcher L¹, L² und M die oben angegebenen Bedeutungen haben
und
- T²: für (C₁-C₄)-Alkyl steht,
zu einer Verbindung der Formel (X) 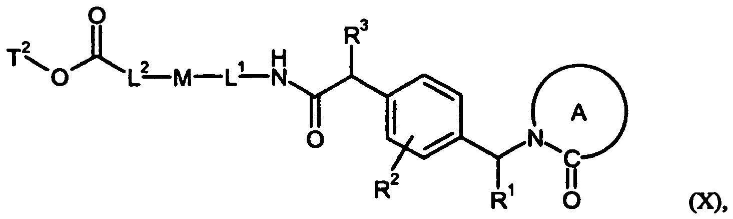 in welcher der Ring A, R¹, R², R³, L¹, L², M und T² jeweils die oben angegebenen Bedeutungen haben,
kuppelt und abschließend den Ester-Rest T² in (X) durch erneute basische oder saure Solvolyse zur Carbonsäure der Formel (I) abspaltet
und die Verbindungen der Formel (I) gegebenenfalls nach dem Fachmann bekannten Methoden in ihre Enantiomere und/oder Diastereomere trennt und/oder gegebenenfalls mit den entsprechenden (i) Lösungsmitteln und/oder (ii) Basen oder Säuren zu ihren Solvaten, Salzen und/oder Solvaten der Salze umsetzt.

Bei der zuvor beschriebenen Reaktionssequenz kann es gegebenenfalls zweckmäßig sein, die Reihenfolge einzelner Transformationen zu vertauschen. So ist es beispielsweise möglich, die Verbindung der Formel (V-A) [T¹in (V) = *tert*.-Butyl] 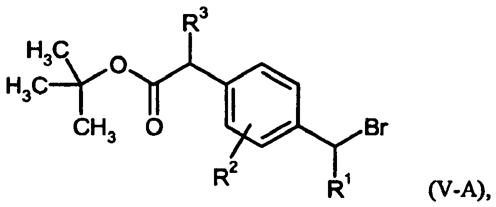 in welcher Erz, R² und R³ die oben angegebenen Bedeutungen haben,
zunächst durch Behandlung mit einer Säure in eine Carbonsäure der Formel (XI) 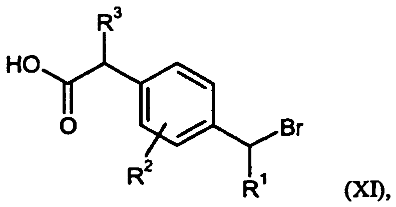 in welcher R¹, R² und R³ die oben angegebenen Bedeutungen haben,
zu überführen und diese anschließend in einem inerten Lösungsmittel in Gegenwart eines Kondensationsmittels oder über die Zwischenstufe des entsprechenden Carbonsäurechlorids in Gegenwart einer Base mit einem Amin der Formel (IX) 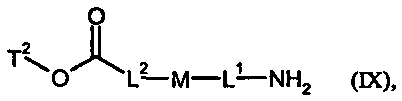 in welcher L¹, L² und M die oben angegebenen Bedeutungen haben
und
- T²: für (C₁-C₄)-Alkyl steht,
zu einer Verbindung der Formel (XII) 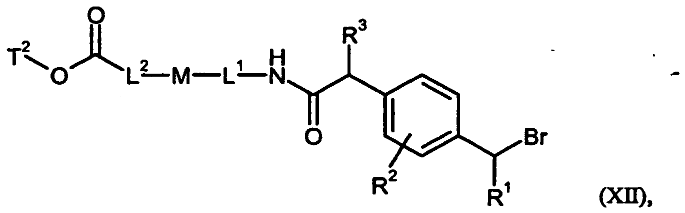 in welcher R¹, R², R³, L¹, L², M und T² jeweils die oben angegebenen Bedeutungen haben,
zu kuppeln, welche dann in einem inerten Lösungsmittel in Gegenwart einer Base mit einer Verbindung der Formel (VI) 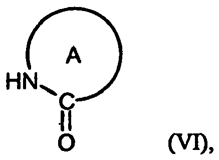 in welcher der Ring A für einen Oxo-substituierten Azaheterocyclus, wie oben beschrieben, steht,
zu der Verbindung der Formel (X) 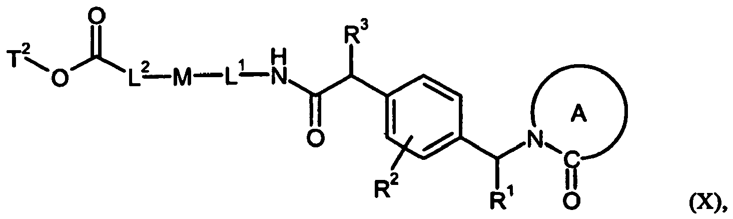 in welcher der Ring A, R¹, R², R³, L¹, L², M und T² jeweils die oben angegebenen Bedeutungen haben,
umgesetzt und durch Abspaltung des Ester-Restes T² in (X) in die Carbonsäure der Formel (I) überführt wird.

Eine Trennung der erfindungsgemäßen Verbindungen in die entsprechenden Enantiomere und/ oder Diastereomere kann gegebenenfalls, je nach Zweckmäßigkeit, auch bereits auf der Stufe der Verbindungen (VII), (VIII) oder (X) erfolgen, welche dann in separierter Form entsprechend den zuvor beschriebenen Verfahrenssequenzen weiter umgesetzt werden. Eine solche Auftrennung der Stereoisomeren läßt sich nach üblichen, dem Fachmann bekannten Methoden durchführen; vorzugsweise werden chromatographische Verfahren oder eine Trennung über diastereomere Salze verwendet.

Inerte Lösungsmittel für den Verfahrensschritt (II) + (III) → (IV) sind beispielsweise Ether wie Diethylether, Methyl-*tert*.-butylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder dipolar-aprotische Lösungsmittel wie Dimethylformamid (DMF), Dimethylsulfoxid (DMSO), *N,N'*-Dimethylpropylenharnstoff (DMPU) oder *N*-Methylpyrrolidon (NMP). Ebenso ist es möglich, Gemische der genannten Lösungsmittel einzusetzen. Bevorzugt werden Tetrahydrofuran, Dimethylformamid oder Gemische hiervon verwendet.

Als Basen für den Verfahrensschritt (II) + (III) → (IV) eignen sich übliche starke anorganische oder organische Basen. Hierzu gehören insbesondere Alkalialkoholate wie Natrium- oder Kaliummethanolat, Natrium- oder Kaliumethanolat oder Natrium- oder Kalium-*tert*.-butylat, Alkalihydride wie Natrium- oder Kaliumhydrid, oder Amide wie Lithium-, Natrium- oder Kalium-bis (trimethylsilyl)amid oder Lithiumdüsopropylamid. Bevorzugt wird Kaüum-*tert*.-butylat, Natriumhydrid oder Lithiumdiisopropylamid verwendet.

Die Umsetzung (II) + (III) → (IV) erfolgt im Allgemeinen in einem Temperaturbereich von -100°C bis +30°C, bevorzugt bei -78°C bis 0°C.

Die Bromierung im Verfahrensschritt (IV) → (V) wird vorzugsweise in einem Halogenkohlenwasserstoff als Lösungsmittel, insbesondere in Dichlormethan oder Tetrachlormethan, in einem Temperaturbereich von +40°C bis +100°C durchgeführt. Als Bromierungsmittel eignen sich elementares Brom in Gegenwart von Licht sowie insbesondere *N*-Bromsuccinimid (NBS) unter Zusatz von α,α'-Azobis(isobutyronitril) (AIBN) oder Dibenzoylperoxid als Initiator [vgl. z.B. R.R. Kurtz, D.J. Houser, J. Org. Chem. 46, 202 (1981); Z.-J. Yao et al., Tetrahedron 55, 2865 (1999)].

Inerte Lösungsmittel für die Verfahrensschritte (V) + (VI) → (VII) und (XII) + (VI) → (X) sind beispielsweise Ether wie Diethylether, Methyl-tert.-butylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Chlorbenzol oder Chlortoluol, oder andere Lösungsmittel wie Dimethylformamid (DMF), Dimethylsulfoxid (DMSO), *N,N'*-Dimethylpropylenharnstoff (DMPU), *N*-Methylpyrrolidon (NMP), Acetonitril oder Pyridin. Ebenso ist es möglich, Gemische der genannten Lösungsmittel einzusetzen. Bevorzugt werden Tetrahydrofuran, Dimethylformamid oder Gemische hiervon verwendet.

Als Basen für diese Umsetzungen eignen sich die üblichen anorganischen oder organischen Basen. Hierzu gehören insbesondere Alkalihydroxide wie beispielsweise Lithium-, Natrium- oder Kaliumhydroxid, Alkalicarbonate wie Lithium-, Natrium-, Kalium- oder Cäsiumcarbonat, Alkalialkoholate wie Natrium- oder Kaliummethanolat, Natrium- oder Kaliumethanolat oder Natrium-oder Kalium-*tert*.-butylat, Alkalihydride wie Natrium- oder Kaliumhydrid, oder Amide wie Lithium-, Natrium- oder Kalium-bis-(trimethylsilyl)amid oder Lithiumdiisopropylamid. Bevorzugt wird Cäsiumcarbonat oder Natriumhydrid eingesetzt.

Die Reaktionen (V) + (VI) → (VII) und (XII) + (VI) → (X) erfolgen im Allgemeinen in einem Temperaturbereich von -20°C bis +120°C, bevorzugt im Bereich von 0°C bis +80°C.

Die Abspaltung der Ester-Gruppen T¹ bzw. T² in den Verfahrensschritten (VII) → (VIII), (X) → (I) und (V-A) → (XI) wird nach üblichen Methoden durchgeführt, indem man die Ester in inerten Lösungsmitteln mit Säuren oder Basen behandelt, wobei bei letzterem die zunächst entstehenden Salze durch Behandeln mit Säure in die freien Carbonsäuren überführt werden. Im Falle der *tert*.-Butylester erfolgt die Esterspaltung bevorzugt mit Säuren.

Als inerte Lösungsmittel eignen sich für diese Reaktionen Wasser oder die für eine Esterspaltung üblichen organischen Lösungsmittel. Hierzu gehören bevorzugt Alkohole wie Methanol, Ethanol, n-Propanol, Isopropanol, n-Butanol oder *tert*.-Butanol, oder Ether wie Diethylether, Tetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten . Lösungsmittel einzusetzen. Im Falle einer basischen Ester-Hydrolyse werden bevorzugt Gemische von Wasser mit Dioxan, Tetrahydrofuran, Methanol und/oder Ethanol eingesetzt. Im Falle der Umsetzung mit Trifluoressigsäure wird bevorzugt Dichlormethan und im Falle der Umsetzung mit Chlorwasserstoff bevorzugt Tetrahydrofuran, Diethylether, Dioxan oder Wasser verwendet.

Als Basen sind die üblichen anorganischen Basen geeignet. Hierzu gehören insbesondere Alkali-oder Erdalkalihydroxide wie beispielsweise Lithium-, Natrium-, Kalium- oder Bariumhydroxid, oder Alkali- oder Erdalkalicarbonate wie Natrium-, Kalium- oder Calciumcarbonat. Bevorzugt sind Lithium-, Natrium- oder Kaliumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der *tert*.-Butylester und Salzsäure im Falle der Methylester.

Die Esterspaltung erfolgt im Allgemeinen in einem Temperaturbereich von -20°C bis +100°C, bevorzugt bei 0°C bis +60°C.

Inerte Lösungsmittel für die Verfahrensschritte (VIII) + (IX) → (X) und (XI) + (IX) → (XII) [Amid-Kupplung] sind beispielsweise Ether wie Diethylether, *tert*.-Butyl-methylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Acetonitril, Ethylacetat, Pyridin, Dimethylsulfoxid (DMSO), Dimethylformamid (DMF), *N,N'*-Dimethylpropylenharnstoff (DMPU) oder *N-*Methylpyrrolidinon (NMP). Ebenso ist es möglich, Gemische der genannten Lösungsmittel zu verwenden. Bevorzugt sind Dichlormethan, Tetrahydrofuran, Dimethylformamid oder Gemische dieser Lösungsmittel.

Als Kondensationsmittel bei diesen Kupplungsreaktionen eignen sich beispielsweise Carbodiimide wie N,N'-Diethyl-, *N,N'*-Dipropyl-, *N,N*-Diisopropyl-, *N,N*-Dicyclohexylcarbodiimid (DCC) oder *N*-(3-Dimethylaminoisopropyl)-*N'*-ethylcarbodiimid-Hydrochlorid (EDC), Phosgen-Derivate wie *N,N'*-Carbonyldiimidazol (CDI), 1,2-Oxazoliumverbindungen wie 2-Ethyl-5-phenyl-1,2-oxazolium-3-sulfat oder 2-*tert*.-Butyl-5-methylisoxazolium-perchlorat, Acylaminoverbindungen wie 2-Ethoxy-1-ethoxycarbonyl-1,2-dihydrochinolin, oder Isobutylchlorformiat, Propanphosphonsäureanhydrid, Cyanophosphonsäurediethylester, Bis-(2-oxo-3-oxazolidinyl)-phosphorylchlorid, Benzotriazol-1-yloxy-tris(dimethylamino)phosphonium-hexafluorophosphat, Benzotriazol-1-yloxy-tris-(pyrrolidino)phosphonium-hexafluorophosphat (PyBOP), O-(Benzotriazol-1-yl)-*N,N,N',N'*-tetra-methyluronium-tetrafluoroborat (TBTU), *O*-(Benzotriazol-1-yl)-N,N,N;N'-tetramethyluronium-hexafluorophosphat (HBTU), 2-(2-Oxo-1-(2*H*)-pyridyl)-1,1,3,3-tetramethyluronium-tetrafluoroborat (TPTU), *O*-(7-Azabenzotriazol-1-yl)-*N,N,N,N'*-tetramethyluronium-hexafluorophosphat (HATU) oder *O*-(1H-6-Chlorbenzotriazol-1-yl)-1,1,3,3-tetramethyluronium-tetrafluoroborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat, oder organische Basen wie Triethylamin, *N*-Methylmorpholin, *N*-Methylpiperidin, *N,N*-Diisopropylethylamin, Pyridin oder 4-*N,N-*Dimethylaminopyridin. Bevorzugt eingesetzt werden *O*-(7-Azabenzotriazol-1-yl)-*N,N,N',N'*-tetramethyluronium-hexafluorophosphat (HATU) oder *O*-(Benzotriazol-1-yl)-*N,N,N',N'*-tetrarnethyluronium-tetrafluoroborat (TBTU), jeweils in Kombination mit Pyridin oder *N,N*-Diisopropylethylamin, oder *N*-(3-Dimethylaminoisopropyl)-*N'-*etbylcarbodiimid-Hydrochlorid (EDC) in Verbindung mit 1-Hydroxybenzotriazol (HOBt) und Triethylamin.

Die Kupplungen (VIII) + (IX) → (X) und (XI) + (IX) → (XII) werden in der Regel in einem Temperaturbereich von 0°C bis +60°C, bevorzugt bei +10°C bis +40°C durchgeführt.

Beim Einsatz eines der Verbindung (VIII) bzw. (XI) entsprechenden Carbonsäurechlorids wird die Kupplung mit der Amin-Komponente (IX) in Gegenwart einer üblichen organischen Hilfsbase wie Triethylamin, *N*-Methylmorpholin, *N*-Methylpiperidin, *N,N*-Diisopropylethylamin, Pyridin, 4-*N,N* Dimethylaminopyridin, 1,8-Diazabicyclo[5.4.0]undec-7-en (DBU) oder 1,5-Diazabicyclo[4.3.0]-non-5-en (DBN) durchgeführt. Bevorzugt wird Triethylamin oder *N,N*-Diisopropylethylamin verwendet.

Die Reaktion von (IX) mit dem Carbonsäurechlorid erfolgt im Allgemeinen in einem Temperaturbereich von -20°C bis +60°C, bevorzugt im Bereich von 0°C bis +40°C.

Die Herstellung der Carbonsäurechloride selbst erfolgt auf übliche Weise durch Behandlung der Carbonsäuren (VIII) bzw. (XI) mit Thionylchlorid.

Die genannten Umsetzungen können bei normalem, bei erhöhtem oder bei erniedrigtem Druck durchgeführt werden (z.B. von 0.5 bis 5 bar). Im Allgemeinen arbeitet man jeweils bei Normaldruck.

Die Intermediate der Formel (IV) können auch auf anderen Wegen - als Alternativen zum oben beschriebenen Alkylierungsverfahren (II) + (III) → (IV) - hergestellt werden. So sind Verbindungen der Formel (IV) beispielsweise auch durch Palladium-katalysierte Arylierung von Carbonsäureestern der Formel (XIII) 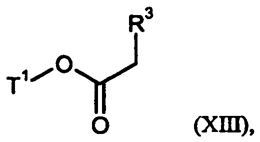 in welcher R³ und T¹ die oben angegebenen Bedeutungen haben,
mit Phenylbromiden oder -iodiden der Formel (XIV) 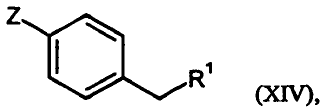 in welcher R¹ die oben angegebene Bedeutung hat
und
- Z: für Brom oder Iod steht,
zugänglich, oder sie lassen sich über eine Friedel-Crafts-Acylierung von Toluol-Derivaten der Formel (XV) 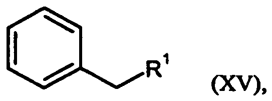 in welcher R¹ die oben angegebene Bedeutung hat,
mit Carbonsäurechloriden der Formel (XVI) 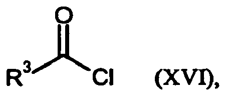 in welcher R³ die oben angegebene Bedeutung hat,
zu Phenylketonen der Formel (XVII) 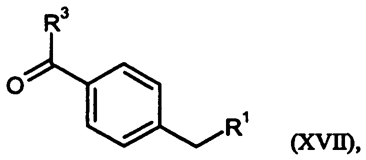 in welcher R¹ und R³ die oben angegebenen Bedeutungen haben,
erhalten; die Verbindungen der Formel (XVII) können dann in einer mehrstufigen, literaturbekannten Reaktionssequenz in die Phenylessigsäure-Derivate der Formel (IV) überführt werden (siehe nachfolgende Reaktionsschemata 3 und 4).

Im Falle, dass R³ für einen gegebenenfalls substituierten Cyclopentyl- oder Cyclohexyl-Rest steht, können entsprechende Verbindungen der Formel (IV) beispielsweise auch über die Michael-Addition eines Phenylessigsäureesters der Formel (XVIII) 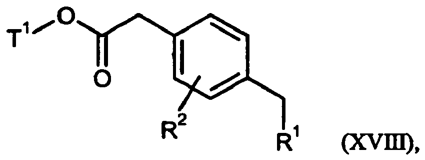 in welcher R¹, R² und T¹ jeweils die oben angegebenen Bedeutungen haben,
an 2-Cyclopenten-1-on bzw. 2-Cyclohexen-1-on und anschließende Transformation der Keto-Gruppe hergestellt werden (siehe nachfolgendes Reaktionsschema 5).

Die Verbindungen der Formeln (II), (III), (VI), (IX), (XIII), (XIV), (XV), (XVI) und (XVIII) sind kommerziell erhältlich, als solche in der Literatur beschrieben oder können in Analogie zu literaturbekannten Verfahren hergestellt werden (vgl. auch nachfolgendes Reaktionsschema 6).

Die Herstellung der erfindungsgemäßen Verbindungen kann durch die folgenden Syntheseschemata beispielhaft veranschaulicht werden: 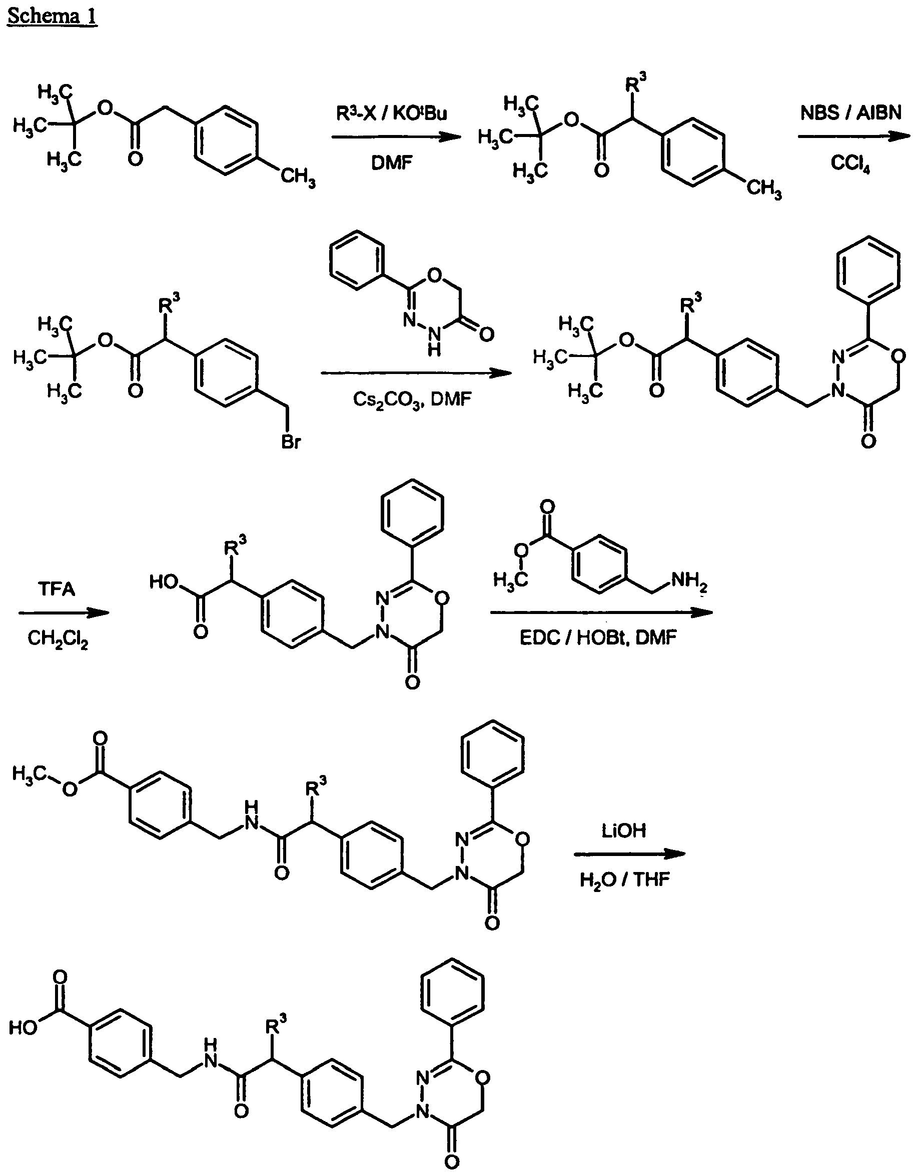 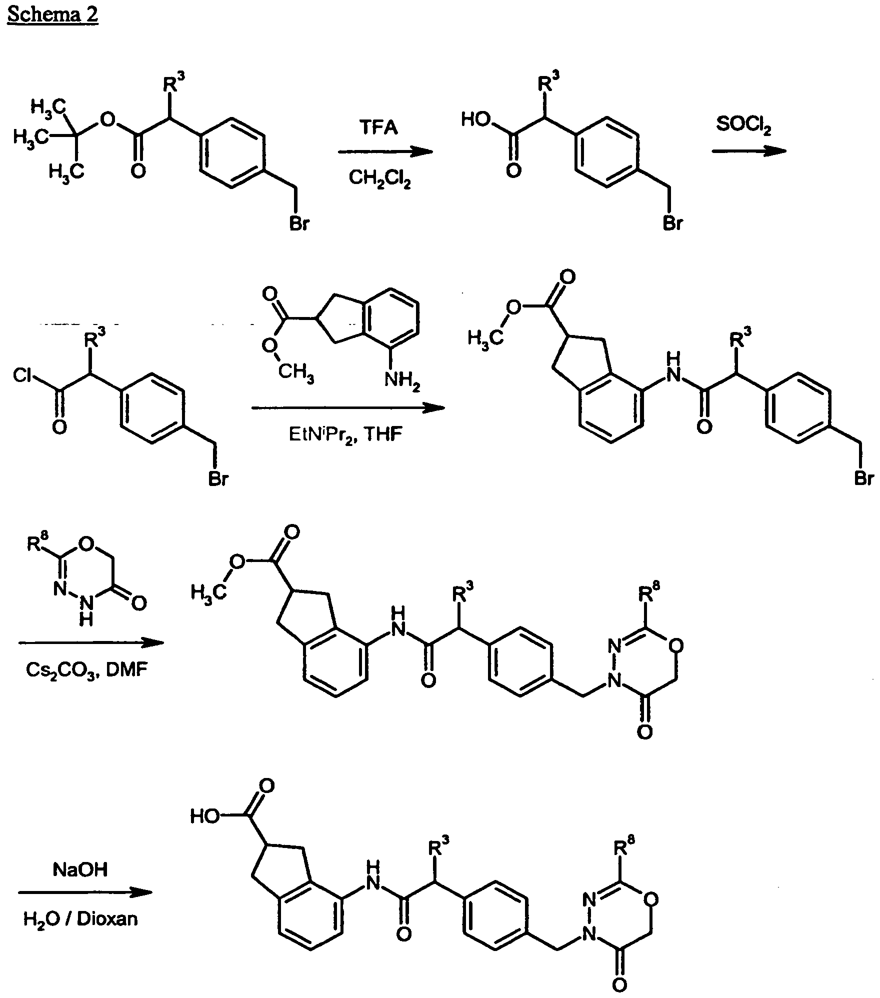 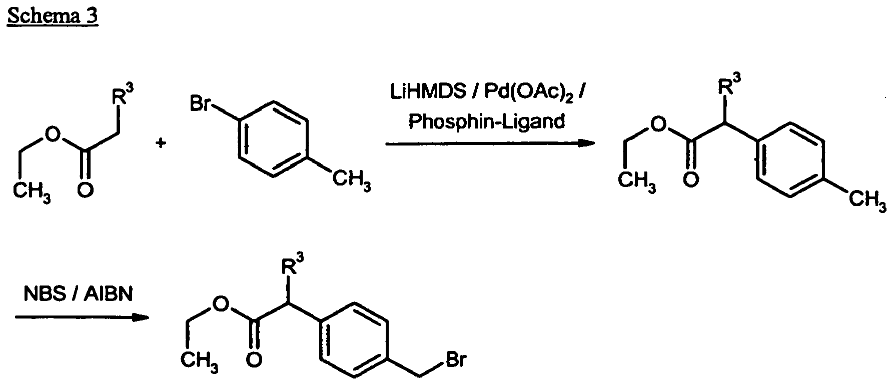 [vgl. z.B. J. Am. Chem. Soc. 2001,123, 7996-8002]. 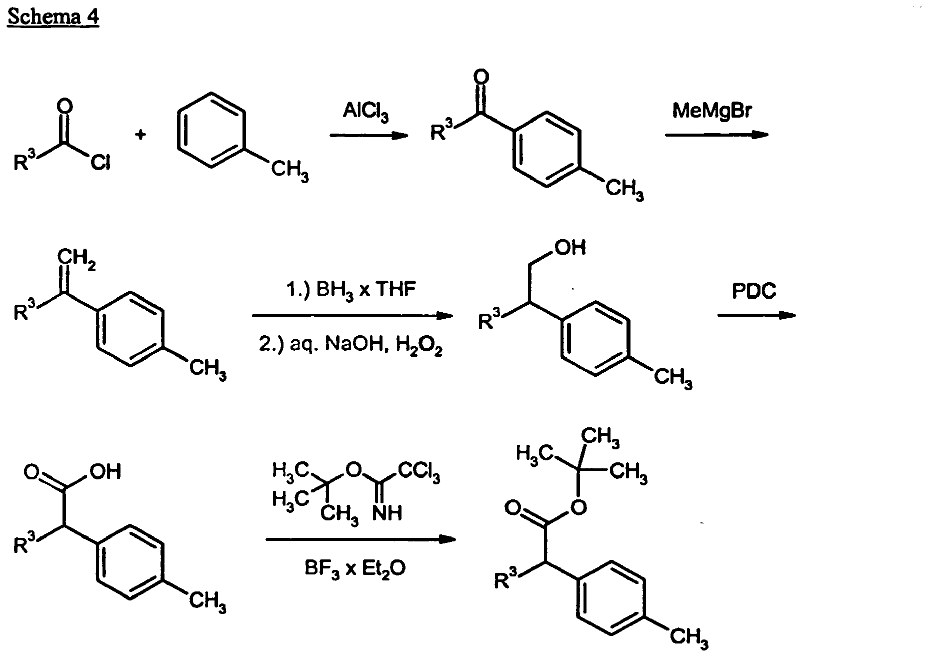 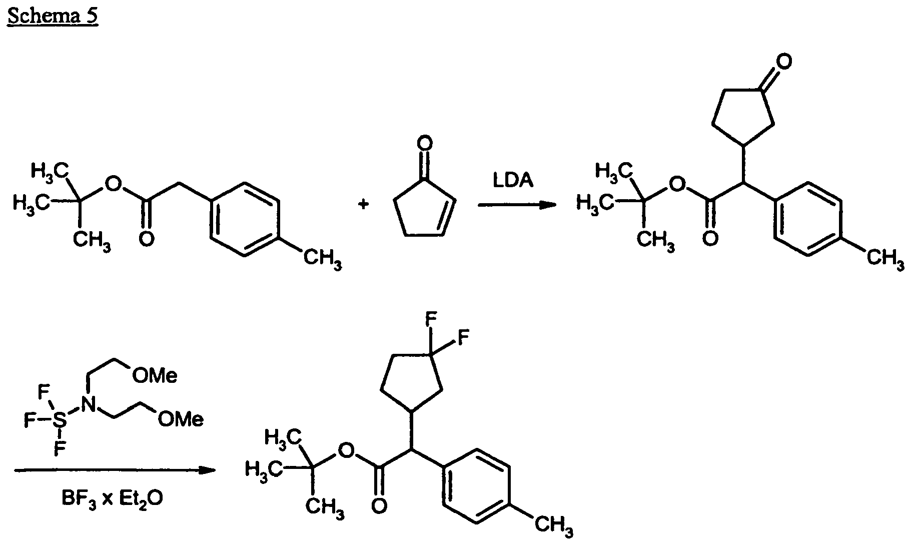 [vgl. z.B. J. Org. Chem. 2001, 66 (20), 6775-6786].

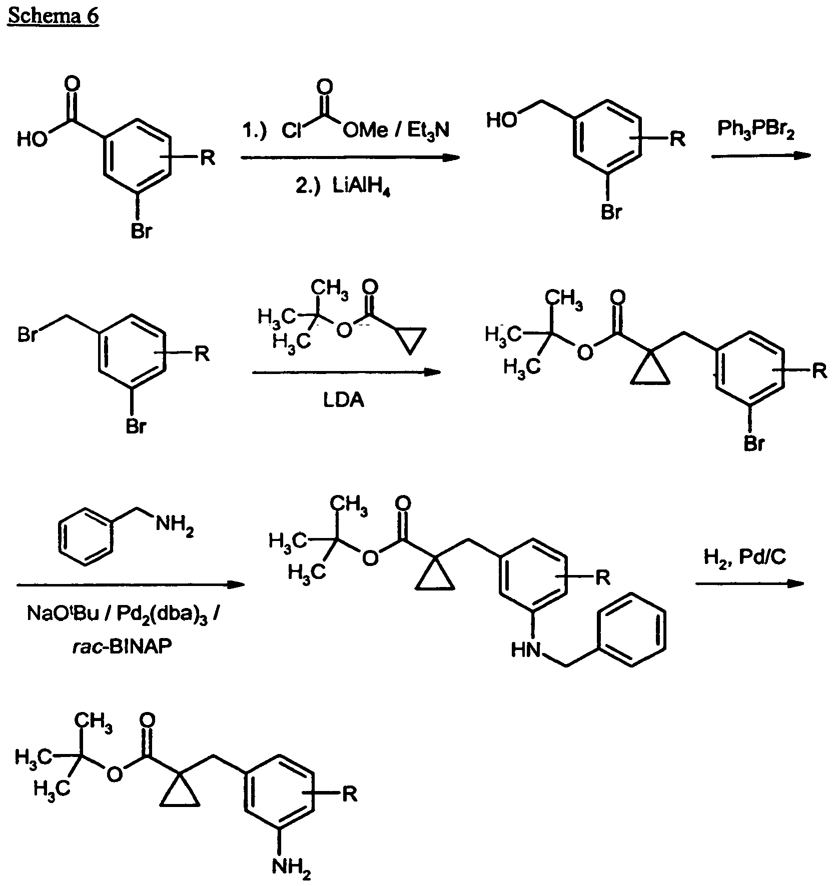

Die erfindungsgemäßen Verbindungen besitzen wertvolle pharmakologische Eigenschaften und können zur Vorbeugung und Behandlung von Erkrankungen bei Menschen und Tieren verwendet werden.

Die erfindungsgemäßen Verbindungen stellen potente Aktivatoren der löslichen Guanylatcyclase dar. Sie führen zu einer Gefäßrelaxation, zu einer Thrombozytenaggregationshemmung und zu einer Blutdrucksenkung sowie zu einer Steigerung des koronaren Blutflusses. Diese Wirkungen sind über eine direkte, Häm-unabhängige Aktivierung der löslichen Guanylatcyclase und einen intrazellulären cGMP-Anstieg vermittelt.

Die erfindungsgemäßen Verbindungen können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, pulmonaler Hypertonie, renaler Hypertonie, peripheren und kardialen Gefäßerkrankungen, Arrhythmien, zur Behandlung von thromboembolischen Erkrankungen und Ischämien wie Myokardinfarkt, Hirnschlag, transistorischen und ischämischen Attacken, peripheren Durchblutungsstörungen, zur Verhinderung von Restenosen wie nach Thrombolysetherapien, percutan-transluminalen Angioplastien (PTA), percutan-transluminalen Koronarangioplastien (PTCA) und Bypass sowie zur Behandlung von Arteriosklerose, asthmatischen Erkrankungen, Krankheiten des Urogenitalsystems wie beispielsweise Prostatahypertrophie, erektile Dysfunktion, weibliche sexuelle Dysfunktion und Inkontinenz, von Osteoporose, Glaukom und Gastroparese eingesetzt werden.

Außerdem können die erfindungsgemäßen Verbindungen zur Behandlung von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, CREST-Syndrom, Erythematose, Onychomykose sowie von rheumatischen Erkrankungen verwendet werden.

Die erfindungsgemäßen Verbindungen können darüber hinaus zur Verhinderung von ischämie-und/oder reperfusionsbedingten Schädigungen von Organen oder Geweben sowie als Zusatzstoffe für Perfusions- und Konservierungslösungen von Organen, Organteilen, Geweben oder Gewebeteilen menschlichen oder tierischen Ursprungs insbesondere bei chirurgischen Eingriffen oder im Bereich der Transplantationsmedizin Verwendung finden.

Ferner eignen sich die erfindungsgemäßen Verbindungen zur Behandlung von Respiratory Distress-Syndromen und chronisch-obstruktiven Atemwegserkrankungen (COPD), von akuter und chronischer Niereninsuffizienz sowie zur Förderung der Wundheilung.

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Him-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Him-Traumas. Ebenso können die erfindungsgemäßen Verbindungen zur Bekämpfung von Schmerzzuständen eingesetzt werden.

Zudem besitzen die erfindungsgemäßen Verbindungen anti-inflammatorische Wirkung und können daher als entzündungshemmende Mittel eingesetzt werden.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- NO-unabhängige, jedoch Häm-abhängige Stimulatoren der Guanylatcyclase, wie insbesondere die in WO 00/06568, WO 00/06569, WO 02/42301 und WO 03/095451 beschriebenen Verbindungen;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, Apixaban, Fidexaban, Razaxaban, Fondaparinux, Idraparinux, DU-176b, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Diuretikum, wie beispielhaft und vorzugsweise Furosemid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- abs.: absolut
- Ac: Acetyl
- AIBN: 2,2'-Azobis-(2-methylpropionitril)
- aq.: wässrig, wässrige Lösung
- ATP: Adenosin-5'-triphosphat
- BINAP: 2,2'-Bis(diphenylphosphino)-1,1'-binaphthyl
- Boc: *tert*.-Butoxycarbonyl
- Brij^{®}: Polyethylenglycol-dodecylether
- BSA: bovines Serumalbumin
- Bsp.: Beispiel
- Bu: Butyl
- c: Konzentration
- CI: chemische Ionisation (bei MS)
- d: Tag(e)
- dba: Dibenzylidenaceton
- DBU: 1,8-Diazabicyclo[5.4.0]undec-7-en
- DC: Dünnschichtchromatographie
- DCI: direkte chemische Ionisation (bei MS)
- de: Diastereomerenüberschuss
- DIBAH: Düsobutylaluminiumhydrid
- DIEA: Diisopropylethylamin
- DMF: Dimethylformamid
- DMSO: Dimethylsulfoxid
- d. Th.: der Theorie (bei Ausbeute)
- DTT: Dithiothreitol
- EDC: *N'*-(3-Dimethylaminopropyl)-*N*-ethylcarbodiimid-Hydrochlorid
- ee: Enantiomerenüberschuss
- EI: Elektronenstoß-Ionisation (bei MS)
- ent: enantiomerenrein, Enantiomer
- eq.: Äquivalent(e)
- ESI: Elektrospray-Ionisation (bei MS)
- Et: Ethyl
- GC: Gaschromatographie
- GTP: Guanosin-5'-triphosphat
- h: Stunde(n)
- HATU: *O*-(7-Azabenzotriazol-1-yl)-*N,N,N',N'-*tetramethyluronium-Hexafluorophosphat
- HOBt: 1-Hydroxy-1*H*-benzotriazol-Hydrat
- HPLC: Hochdruck-, Hochleistungsflüssigchromatographie
- ⁱPr: Isopropyl
- KO^{t}Bu: Kalium-*tert*.-butylat
- LC-MS: Flüssigchromatographie-gekoppelte Massenspektroskopie
- LDA: Lithiumdiisopropylamid
- LiHDMS: Lithiumhexamethyldisilazid [Lithium-bis(trimethylsilyl)amid]
- Me: Methyl
- min: Minute(n)
- MS: Massenspektroskopie
- NBS: *N*-Bromsuccinimid
- NMR: Kernresonanzspektroskopie
- PDC: Pyridiniumdichromat
- Ph: Phenyl
- Pr: Propyl
- rac: racemisch, Racemat
- R_{f}: Retentionsindex (bei DC)
- RP: reverse phase (Umkehrphase, bei HPLC)
- RT: Raumtemperatur
- Rₜ: Retentionszeit (bei HPLC)
- s.o.: siehe oben
- ^{t}Bu: *tert*.-Butyl
- TBTU: *O*-(Benzotriazol-1-yl)-*N,N,N',N'*-tetramethyluronium-Tetrafluoroborat
- TCTU: *O*-(1*H*-6-Chlorbenzotriazol-1-yl)-1,1,3,3-tetramethyluronium-Tetrafluoroborat
- TEA: Triethanolamin
- TFA: Trifluoressigsäure
- THF: Tetrahydrofuran
- UV: Ultraviolett-Spektroskopie
- v/v: Volumen zu Volumen-Verhältnis (einer Lösung)
- zus.: zusammen

### LC/MS-Methoden:

### Methode 1 (LC-MS)

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 2 (LC-MS)

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 3 (LC-MS)

Instrument: Micromass Platform LCZ mit HPLC Agilent Serie 1100; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 4 (LC-MS)

Instrument: Micromass Quattro LCZ mit HPLC Agilent Serie 1100; Säule: Phenomenex Synergi 2µ Hydro-RP Mercury 20 mm x 4 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 5 (LC-MS)

Instrument: Micromass Platform LCZ mit HPLC Agilent Serie 1100; Säule: Thermo Hypersil GOLD 3µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### Methode 6 (LC-MS)

Gerätetyp MS: Micromass ZQ; Gerätetyp HPLC: Waters Alliance 2795; Säule: Merck Chromolith SpeedROD RP-18e 100 mm x 4.6 mm; Eluent A: Wasser + 500 µl 50%-ige Ameisensäure / 1, Eluent B: Acetonitril + 500 µl 50%-ige Ameisensäure / 1; Gradient: 0.0 min 10% B → 7.0 min 95% B → 9.0 min 95% B; Fluss: 0.0 min 1.0 ml/min → 7.0 min 2.0 ml/min → 9.0 min 2.0 ml/min; Ofen: 35°C; UV-Detektion: 210 nm.

### Methode 7 (LC-MS)

Gerätetyp MS: Micromass ZQ; Gerätetyp HPLC: HP 1100 Series; UV DAD; Säule: Phenomenex Gemini 3µ 30 mm x 3.00 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 8 (LC-MS)

Instrument: Micromass Quattro LCZ mit HPLC Agilent Serie 1100; Säule: Phenomenex Onyx Monolithic C18, 100 mm x 3 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 208-400 nm.

### Methode 9 (LC-MS)

Gerätetyp MS: Waters ZQ; Gerätetyp HPLC: Waters Alliance 2795; Säule: Phenomenex Onyx Monolithic C18, 100 mm x 3 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 210 nm.

### Methode 10 (LC-MS)

Gerätetyp MS: Micromass ZQ; Gerätetyp HPLC: Waters Alliance 2795; Säule: Phenomenex Synergi 2.5 µ MAX-RP 100A Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

### Methode 11 (LC-MS)

Instrument: Micromass Quattro Premier mit Waters UPLC Acquity; Säule: Thermo Hypersil GOLD 1.9 µ 50 mm x 1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen:50°C; UV-Detektion: 210 nm.

### Methode 12 (LC-MS)

Gerätetyp MS: Waters Micromass Quattro Micro; Gerätetyp HPLC: Agilent 1100 Serie; Säule: Thermo Hypersil GOLD 3 µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 13 (LC-MS)

Gerätetyp MS: Waters ZQ; Gerätetyp HPLC: Agilent 1100 Series; UV DAD; Säule: Thermo Hypersil GOLD 3 µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A - 3.0 min 10% A → 4.0 min 10% A → 4.1 min 100% A (Fluss 2.5 ml/min); Ofen: 55°C; Fluss: 2 ml/min; UV-Detektion: 210 nm.

### Methode 14 (LC-MS)

Instrument MS: Waters ZQ 2000; Instrument HPLC: Agilent 1100, 2-Säulen-Schaltung; Autosampler: HTC PAL; Säule: YMC-ODS-AQ, 50 mm x 4.6 mm, 3.0 µm; Eluent A: Wasser + 0.1% Ameisensäure, Eluent B: Acetonitril + 0.1 % Ameisensäure; Gradient: 0.0 min 100% A → 0.2 min 95% A → 1.8 min 25% A → 1.9 min 10% A → 2.0 min 5% A → 3.2 min 5% A → 3.21 min 100% A → 3.35 min 100% A; Ofen: 40°C; Fluss: 3.0 ml/min; UV-Detektion: 210 nm.

### Methode 15 (LC-MS)

Instrument: Waters Acquity SQD UPLC System; Säule: Waters Acquity UPLC HSS T3 1.8 µ, 50 mm x 1 mm; Eluent A: 1 1 Wasser + 0.25 ml 99%-ige Ameisensäure, Eluent B: 1 1 Acetonitril + 0.25 ml 99%-ige Ameisensäure; Gradient: 0.0 min 90% A → 1.2 min 5% A → 2.0 min 5% A; Fluss: 0.40 ml/min; Ofen: 50°C; UV-Detektion: 210-400 nm.

### GC/MS-Methoden:

### Methode 1 (GC-MS)

Instrument: Micromass GCT, GC 6890; Säule: Restek RTX-35MS, 30 m x 250 µm x 0.25 µm; konstanter Fluss mit Helium: 0.88 ml/min; Ofen: 60°C; Inlet: 250°C; Gradient: 60°C (0.30 min halten), 50°C/min → 120°C, 16°C/min → 250°C, 30°C/min → 300°C (1.7 min halten).

### Methode 2 (GC-MS)

Instrument: Micromass GCT, GC 6890; Säule: Restek RTX-35MS, 30 m x 250 µm x 0.25 µm; konstanter Fluss mit Helium: 0.88 ml/min; Ofen: 60°C; Inlet: 250°C; Gradient: 60°C (0.30 min halten), 50°C/min → 120°C, 16°C/min → 250°C, 30°C/min → 300°C (8.7 min halten).

### Methode 3 (GC-MS)

Instrument: Micromass GCT, GC 6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### HPLC-Methoden:

### Methode 1 (HPLC)

Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 9 min 90% B → 9.2 min 2% B → 10 min 2% B; Fluss: 0.75 ml/min; Säulentemperatur: 30°C; UV-Detektion: 210 nm.

### Methode 2 (HPLC)

Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 15 min 90% B → 15.2 min 2% B → 16 min 2% B; Fluss: 0.75 ml/min; Säulentemperatur: 30°C; UV-Detektion: 210 nm.

### Ausgangsverbindungen und Intermediate:

### Beispiel 1A

### tert.-Butyl-5,5,5-trifluor-2-(4-methylphenyl)pentanoat

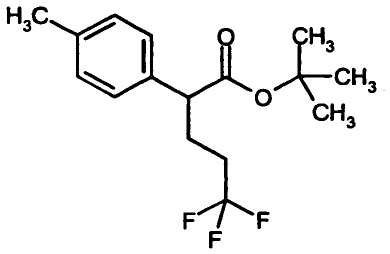

Unter Sauerstoffausschluss wurden 0.88 ml (6.3 mmol) Diisopropylamin in 20 ml THF vorgelegt, auf -78°C abgekühlt und langsam mit 2.52 ml (6.3 mmol) einer 2.5 M Lösung von n-Butyllithium in Hexan versetzt. Anschließend wurde die Reaktionslösung auf-10°C erwärmt und 10 min bei dieser Temperatur nachgerührt. Die Reaktionslösung wurde dann wieder auf -78°C abgekühlt und langsam mit 1 g (4.85 mmol) *tert*.-Butyl-(4-methylphenyl)acetat, gelöst in 10 ml THF, versetzt. Die Reaktionslösung wurde anschließend langsam auf -30°C erwärmt und danach erneut auf -78°C abgekühlt. Nach Erreichen dieser Temperatur wurden 0.62 ml (5.82 mmol) 3-Brom-1,1,1-trifluorpropan langsam zugetropft. Nach beendeter Zugabe wurde die Lösung langsam auf Raumtemperatur erwärmt und über Nacht gerührt. Nach DC-Kontrolle (Laufmittel Cyclohexan/Essigsäureethylester 10:1) wurde der Ansatz mit gesättigter Ammoniumchlorid-Lösung versetzt und in Essigsäureethylester aufgenommen. Die wässrige Phase wurde zweimal mit Essigsäureethylester extrahiert. Die vereinigten organischen Phasen wurden über Natriumsulfat getrocknet. Nach Filtration wurde das Lösungsmittel im Vakuum entfernt. Das Rohprodukt wurde über Kieselgel chromatographisch gereinigt (Laufmittel Cyclohexan/Essigsäureethylester 10:1). Es wurden 542 mg (1.79 mmol, 37% d. Th.) eines gelblichen Öls isoliert.
GC-MS (Methode 3): Rₜ = 4.41 min; m/z = 246 (M-C₄H₉+H)⁺.

### Beispiel 2A

### tert.-Butyl-3-methyl-2-(4-methylphenyl)pentanoat

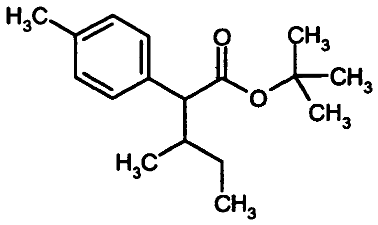

Unter Argon wurden 19.58 g (174.5 mmol) Kalium-*tert*.-butylat in 200 ml DMF vorgelegt, auf 0°C abgekühlt, langsam mit 30 g (145.4 mmol) *tert*.-Butyl-(4-methylphenyl)acetat, gelöst in 50 ml DMF, versetzt und anschließend 30 min bei 0°C gerührt. Nachfolgend wurden 18.95 ml (174.5 mmol) 2-Brombutan langsam zugetropft und die Lösung 4 h bei 0°C nachgerührt. Danach wurde die Reaktionslösung mit 200 ml Wasser und 200 ml Diethylether versetzt. Die wässrige Phase wurde zweimal mit Diethylether extrahiert. Die vereinigten organischen Phasen wurden über Magnesiumsulfat getrocknet. Nach Filtration wurde das Lösungsmittel im Vakuum entfernt. Das Rohprodukt wurde über Kieselgel chromatographisch gereinigt (Laufmittel Cyclohexan/Essigsäureethylester 20:1). Es wurden 15.5 g (59.1 mmol, 40.6% d. Th.) einer farblosen Flüssigkeit isoliert.
¹H-NMR (400 MHz, DMSO-d₆, δ/ppm): 7.17 (2H, d), 7.11 (2H, d), 3.11 (1H, d), 2.27 (3H, s), 2.04-1.90 (1H, m), 1.55-1.42 (1H, m), 1.35 (9H, s), 1.24-1.10 (1H, m), 0.99-0.86 (3H, m), 0.77-0.51 (3H, m).
GC-MS (Methode 3): Rₜ = 5.04 min; m/z = 206 (M-C₄H₉+H)⁺.

Auf zu Beispiel 2A analoge Weise wurden die in der folgenden Tabelle aufgeführten Verbindungen erhalten:

| **Beispiel** | **Name / Struktur** | **Analytische Daten** |
|---|---|---|
| **3A** | *tert*.-Butyl-3-methyl-2-(4-methylphenyl)butanoat | ¹H-NMR (400 MHz, DMSO-d₆, δ/ppm): 7.17 (2H, d), 7.11 (2H, d), 3.00 (1H, d), 2.28 (3H, s), 2.21-2.07 (1H, m), 1.35 (9H, s), 0.97 (3H, d), 0.61 (3H, d). GC-MS (Methode 3): Rₜ = 4.70 min; m/z = 192 (M-C₄H₉+H)⁺. |
| | 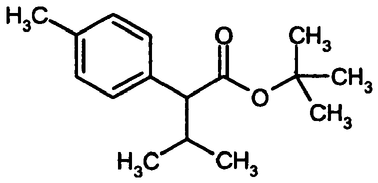 | |
| **4A** | *tert*. -Butyl-cyclopentyl(4-methylphenyl)acetat | ¹H-NMR (400 MHz, DMSO-d₆, δ/ppm): 7.19 (2H, d), 7.11 (2H, d), 3.12 (1H, d), 2.45-2.29 (1H, m), 2.27 (3H, s), 1.89-1.71 (1H, m), 1.67-1.45 (3H, m), 1.44-1.15 (3H, m), 1.36 (9H, s), 1.02-0.84 (1H, m). MS (DCI): m/z = 292 (M+NH₄)⁺; GC-MS (Methode 3): Rₜ = 5.89 min; m/z = 218 (M-C₄H₉+H)⁺. |
| | 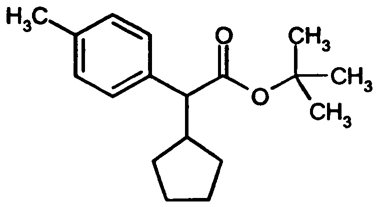 | |
| **5A** | *tert*.-Butyl-4-cyano-3-methyl-2-(4-methylphenyl)-butanoat | ¹H-NMR (400 MHz, DMSO-d₆, δ/ppm): 7.17 (4H, d), 3.31-3.18 (1H, m), 2.69-2.49 (1H, m), 2.48-2.34 (1H, m), 2.29 (3H, s), 2.08-1.98 (1H, m), 1.34 (9H, s), 1.11 (2H, d), 0.75 (1H, d). LC-MS (Methode 10): Rₜ = 2.35 und 2.38 min; m/z = 274 (M+H)⁺. |
| | 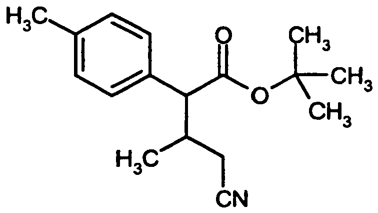 | |
| **6A** | *tert*.-Butyl-3-methyl-2-(4-methylphenyl)hexanoat | MS (DCI): m/z = 294 (M+NH₄)⁺; GC-MS (Methode 3): Rₜ = 5.30 min; m/z = 220 (M-C₄H₉+H)⁺. |
| | 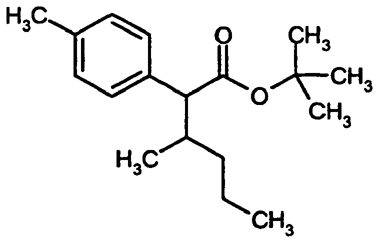 | |
| **7A** | *tert*.-Butyl-4-methoxy-3-methyl-2-(4-methyl-phenyl)butanoat | MS (DCI): m/z = 279 (M+H)⁺ ; GC-MS (Methode 3): **Rₜ** = 5.34 und 5.37 min; m/z = 222 (M-C₄H₉+H)⁺. |
| | 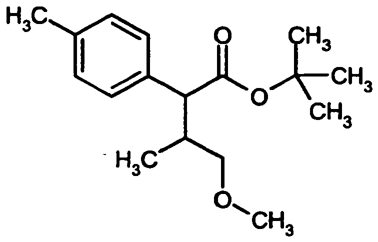 | |

### Beispiel 8A

### tert.-Butyl-2-[4-(brommethyl)phenyl]-5,5,5-trifluorpentanoat

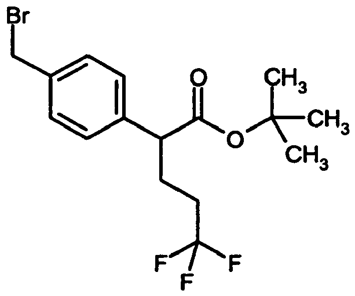

540 mg (1.79 mmol) *tert*.-Butyl-5,5,5-trifluor-2-(4-methylphenyl)pentanoat, 333.8 mg (1.78 mmol) *N*-Bromsuccinimid und 14.7 mg (0.09 mmol) 2,2'-Azobis-2-methylpropannitril in 10 ml Tetrachlonnethan wurden 2 h unter Rückfluss gerührt. Nach vollständiger Umsetzung wurde das Succinimid abfiltriert und der Filterrückstand mit Dichlormethan nachgewaschen. Das Filtrat wurde im Vakuum eingeengt. Das Rohprodukt wurde über Kieselgel chromatographisch gereinigt (Laufmittel Cyclohexan/Essigsäureethylester 10:1). Es wurden 659 mg (1.72 mmol, 97% d. Th.) eines gelblichen Öls isoliert.
GC-MS (Methode 3): **Rₜ** = 5.91 min; m/z = 301 (M-Br)⁺.

### Beispiel 9A

### tert.-Butyl-2-[4-(brommethyl)phenyl]-3-methylpentanoat

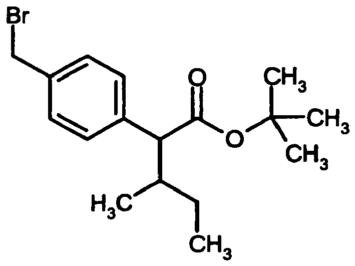

15 g (59.1 mmol) *tert*.-Butyl-3-methyl-2-(4-methylphenyl)pentanoat, 11 g (62 mmol) *N*-Bromsuccinimid und 97 mg (0.59 mmol) 2,2'-Azobis-2-methylpropannitril in 150 ml Dichlormethan wurden 2 h unter Rückfluss gerührt. Nach vollständiger Umsetzung wurde das Lösungsmittel im Vakuum entfernt. Das Rohprodukt wurde über Kieselgel chromatographisch gereinigt (Laufmittel Cyclohexan/Essigsäureethylester 20:1). Es wurden 16.22 g (47.5 mmol, 80% d. Th.) eines farblosen Öls isoliert.
GC-MS (Methode 3): Rₜ = 6.41 min; m/z = 261 (M-Br)⁺.
MS (DCI): m/z = 358/360 (M+NH₄)⁺.

Auf analoge Weise wurden die in der folgenden Tabelle aufgeführten Verbindungen erhalten:

| **Beispiel** | **Name / Struktur** | **Analytische Daten** |
|---|---|---|
| **10A** | *tert*.-Butyl-[4-(brommethyl)phenyl](cyclopentyl)-acetat | ¹H-NMR (400 MHz, DMSO-d₆, δ/ppm): 7.39 (2H, d), 7.30 (2H, d), 4.68 (2H, s), 3.21 (1H, d), 2.45-2.31 (1H, m), 1.89-1.74 (1H, m), 1.69-1.45 (3H, m), 1.44-1.16 (3H, m), 1.35 (9H, s), 1.02-0.88 (1H, m). MS (DCI): m/z = 370/372 (M+NH₄)⁺. |
| | 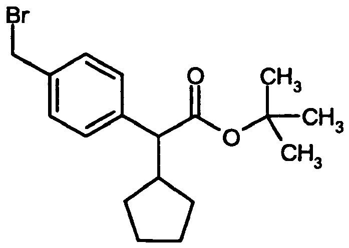 | |
| **11A** | *tert*.-Butyl-2-[4-(brommethyl)phenyl]-3-methyl-butanoat | ¹H-NMR (400 MHz, DMSO-d₆, δ/ppm): 7.40 (2H, d), 7.29 (2H, d), 4.68 (2H, s), 2.99 (1H, d), 2.23-2.08 (1H, m), 1.36 (9H, s), 0.98 (3H, d), 0.60 (3H, d). GC-MS (Methode 3): Rₜ = 6.22 min; m/z = 247 (M-Br)⁺. |
| | 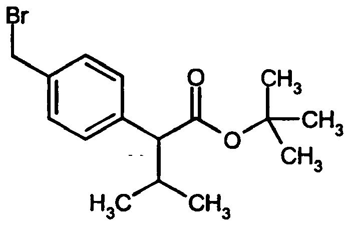 | |
| **12A** | *tert*.-Butyl-2-[4-(brommethyl)phenyl]-4-cyano-3-methylbutanoat | ¹H-NMR (400 MHz, DMSO-d₆, δ/ppm): 7.67-7.26 (4H, m), 4.70 (2H, s), 3.45-3.26 (1H, m), 2.60-2.22 (1H, m), 2.11-1.99 (1H, m), 1.40 und 1.37 (zus. 9H, 2s), 1.15 (2H, d), 0.77 (1H, d). MS (DCI): m/z = 351/353 (M)⁺, 369/371 (M+NH₄)⁺. |
| | 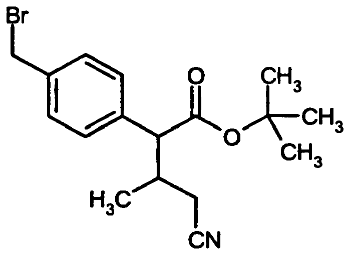 | |
| **13A** | *tert*.-Butyl-2-[4-(brommethyl)phenyl]-3-methyl-hexanoat | MS (DCI): m/z = 372/374 (M+NH₄)⁺. |
| | 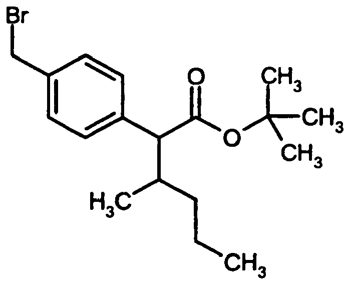 | |
| **14A** | *tert*.-Butyl-2-[4-(brommethyl)phenyl]-4-methoxy-3-methylbutanoat | MS (DCI): m/z = 357/359 (M+H)⁺, 318/320 (M-C₄H₈+NH₄)⁺, 301/303 (M-C₄H₈)⁺. |
| | 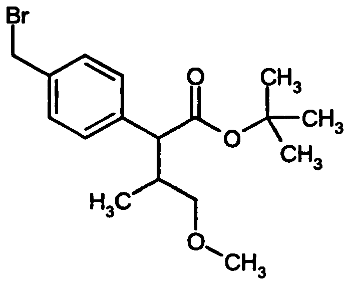 | |

### Beispiel 15A

### N'-(2-Chloracetyl)benzolcarbohydrazid

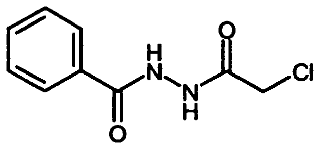

Eine Suspension von 500 g (3.67 mol) Benzolcarbohydrazid in 3.75 Liter THF wurde auf Rückfluss erhitzt, wobei sich das Benzolcarbohydrazid löste. Zu dieser Lösung wurden 497.7 g (4.41 mol) Chloracetylchlorid, gelöst in 125 ml THF, getropft und die Lösung 30 min unter Rückfluss nachgerührt. Nach vollständiger Umsetzung (DC-Kontrolle, (Laufmittel Dichlormethan/Methanol 9:1) wurde der Ansatz mit 22.5 Liter Wasser und 10 Liter Essigsäureethylester versetzt und mit festem Natriumhydrogencarbonat auf pH 7 eingestellt. Die wässrige Phase wurde einmal mit 2.5 Liter Essigsäureethylester extrahiert. Die vereinigten organischen Phasen wurden getrocknet und die Lösung anschließend im Vakuum bis zur Trockene eingeengt. Der erhaltene weiße Feststoff wurde in einem 1:1-Gemisch aus Dichlormethan und Methanol gelöst und auf 3 kg Kieselgel aufgezogen. An zwei Kieselgel-Portionen (jeweils 8 kg) wurde zunächst mit 50 Liter Dichlormethan/Essigsäureethylester 7:3 und dann mit 125 Liter Dichlormethan/Essigsäureethylester 1:1 als Laufmittel chromatographiert. Nach Einengen der Produktfraktionen wurden 424 g (1.99 mol, 54% d. Th.) der Titelverbindung als weißer Feststoff erhalten.
¹H-NMR (400 MHz, DMSO-d₆, δ/ppm): 10.56-10.32 (2H, breit), 7.88 (2H, d), 7.58 (1H, t), 7.50 (2H, t), 4.21 (2H, s).
MS (DCI): m/z = 213 (M+H)⁺, 230 (M+NH₄)⁺.

### Beispiel 16A

### 2-Phenyl-4H-1,3,4-oxadiazin-5(6H)-on

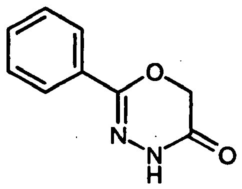

812 g (3.82 mol) *N'*-(2-Chloracetyl)benzolcarbohydrazid wurden in 13 Liter trockenem DMF gelöst und mit 384.95 g (4.58 mol) Natriumhydrogencarbonat versetzt. Anschließend wurde die Reaktionslösung auf 100°C erhitzt und über Nacht bei dieser Temperatur gerührt. Nach vollständiger Umsetzung (DC-Kontrolle, Laufmittel Dichlormethan/Essigsäureethylester 9:1) wurde die Reaktionslösung auf Raumtemperatur abgekühlt, auf 65 Liter Wasser gegossen und dreimal mit jeweils 17.5 Liter Essigsäureethylester extrahiert. Die vereinigten organischen Phasen wurden mit 13.8 Liter gesättigter wässriger Natriumhydrogencarbonat-Lösung gewaschen, getrocknet und im Vakuum bis zur Trockene eingeengt. Der erhaltene Feststoff wurde in einem 9:1-Gemisch aus Dichlormethan und Methanol gelöst und auf 17 kg Kieselgel aufgezogen. An zwei Kieselgel-Portionen (jeweils 8 kg) wurde mit 260 Liter Dichlormethan/Essigsäureethylester 9:1 als Laufmittel chromatographiert. Die vereinigten Produktfraktionen wurden eingeengt, und der erhaltene Feststoff wurde mit 3 Liter Diethylether ausgerührt. Nach Filtration wurden 247 g (1.40 mol, 35% d. Th.) der Titelverbindung als weißer Feststoff erhalten.
¹H-NMR (400 MHz, DMSO-d₆, δ/ppm): 11.04 (1H, s), 7.78 (2H, d), 7.53-7.41 (3H, m), 4.79 (2H, s).
MS (DCI): m/z = 177 (M+H)⁺.

Auf analoge Weise wurden die in der folgenden Tabelle aufgeführten Verbindungen erhalten:

| **Beispiel** | **Name / Struktur** | **Analytische Daten** |
|---|---|---|
| **17A** | 2-(2-Chlorphenyl)-4*H*-1,3,4-oxadiazin-5(6*H*)-on | LC-MS (Methode 2): Rₜ = 1.49 min; m/z = 211 (M+H)⁺. |
| | 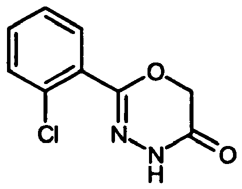 | |
| **18A** | 2-(2-Methoxyphenyl)-4*H*-1,3,4-oxadiazin-5(6*H*)-on | LC-MS (Methode 7): Rₜ = 1.54 min; m/z = 207 (M+H)⁺. |
| | 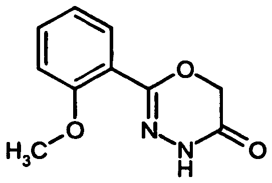 | |

### Beispiel 19A

### N'-(Chloracetyl)-2,2-dimethylpropanhydrazid

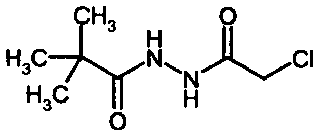

In 600 ml trockenem Essigsäureethylester wurden 5.8 g (49.929 mmol) 2,2-Dimethylpropanhydrazid gelöst und auf Rückfluss erhitzt. Zur Lösung wurden 6.767 g (59.915 mmol) Chloracetylchlorid, gelöst in 45 ml trockenem Essigsäureethylester, getropft. Es wurde für 30 min unter Rückfluss nachgerührt. Nach dem Abkühlen wurde das Reaktionsgemisch am Rotationsverdampfer eingeengt und der Rückstand im Hochvakuum getrocknet. Es wurden 10.14 g der Titelverbindung erhalten. Das Rohprodukt wurde ohne weitere Reinigung weiter umgesetzt.

### Beispiel 20A

### 2-tert.-Butyl-4H-1,3,4-oxadiazin-5(6H)-on

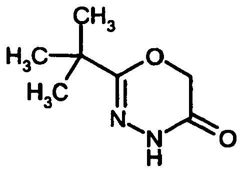

In 800 ml trockenem DMF wurden 8.34 g (43.29 mmol) *N'*-(Chloracetyl)-2,2-dimethylpropanhydrazid gelöst und mit 4.24 g (50.52 mmol) Natriumhydrogencarbonat versetzt. Das Reaktionsgemisch wurde über Nacht auf 100°C erhitzt. Nach dem Abkühlen wurde das Lösungsmittel am Rotationsverdampfer entfernt, der Rückstand mit Wasser versetzt und mit 1 N Salzsäure angesäuert. Das Gemisch wurde dreimal mit Essigsäureethylester extrahiert, die vereinigten organischen Phasen mit Wasser gewaschen, über Natriumsulfat getrocknet und am Rotationsverdampfer eingeengt. Es wurden 5.05 g der Zielverbindung erhalten. Das Rohprodukt wurde ohne weitere Reinigung weiter umgesetzt.
GC-MS (Methode 3): Rₜ = 3.81 min; m/z = 156 (M)⁺.

Auf analoge Weise wurden die in der folgenden Tabelle aufgeführten Verbindungen erhalten:

| **Beispiel** | **Name / Struktur** | **Analytische Daten** |
|---|---|---|
| **21A** | 2-(Propan-2-yl)-4*H*-1,3,4-oxadiazin-5(6*H*)-on | GC-MS (Methode 3): Rₜ = 3.72 min; m/z = 142 (M)⁺. |
| | 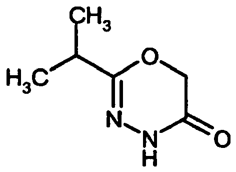 | |
| **22A** | 2-(2-Methylpropyl)-4*H*-1,3,4-oxadiazin-5(6*H*)-on | GC-MS (Methode 3): Rₜ = 4.14 min; m/z = 156 (M)⁺. |
| | 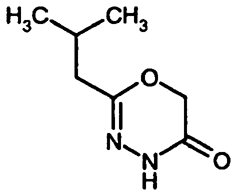 | |

### Beispiel 23A

### 5-Methyl-2-oxohexan-3-ylthiocyanat

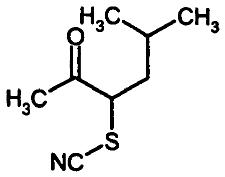

17.91 g (120.5 mmol) 3-Chlor-5-methylhexan-2-on wurden in 120 ml Methylethylketon gelöst, mit 10.0 g (123.4 mmol) Natriumthiocyanat versetzt und die Reaktionsmischung für 1 h unter Rückfluss erhitzt. Nach dem Abkühlen wurde das Reaktionsgemisch filtriert, der Rückstand mit Methylethylketon gewaschen und die vereinigten Filtrate am Rotationsverdampfer eingeengt. Der ölige Rückstand wurde in ca. 400 ml Dichlormethan aufgenommen und dreimal mit je 100 ml Wasser gewaschen. Die organische Phase wurde über Natriumsulfat getrocknet und das Lösungsmittel am Rotationsverdampfer entfernt. Es wurden 20.07 g des Rohprodukts erhalten, welches als solches weiter umgesetzt wurde.
LC-MS (Methode 13): **Rₜ** = 1.99 min; m/z = 171 (M)⁺.

### Beispiel 24A

### 4-Methyl-5-(2-methylpropyl)-1,3-thiazol-2(3H)-on

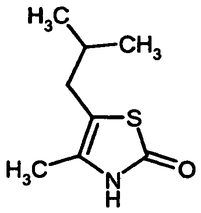

20.07 g (117.26 mmol) 5-Methyl-2-oxohexan-3-ylthiocyanat wurden mit 25 ml 85%-iger Phosphorsäure versetzt und innerhalb 1 h auf 95°C erhitzt. Bei einer Temperatur von 95-100°C wurde 0.5 h nachgerührt. Nach dem Abkühlen wurde das Reaktionsgemisch auf 40 ml Wasser gegeben und mehrmals mit *tert*.-Butylmethylether extrahiert. Die vereinigten organischen Phasen wurden mit Wasser gewaschen, über Natriumsulfat getrocknet und am Rotationsverdampfer eingeengt. Es wurden 18.11 g der Zielverbindung erhalten, welche ohne weitere Reinigung weiter umgesetzt wurde.
GC-MS (Methode 3): Rₜ = 5.80 min; m/z = 171 (M)⁺.

### Beispiel 25A

### tert.-Butyl-cyclopentyl{4-[(5-oxo-2-phenyl-5,6-dihydro-4H-1,3,4-oxadiazin-4-yl)methyl]phenyl}-acetat

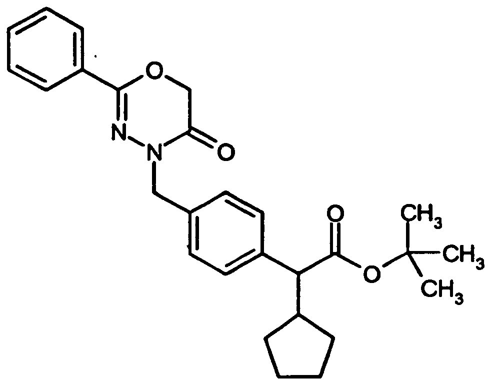

### Darstellungsmethode 1:

9.9 g (28.0 mmol) *tert*.-Butyl-[4-(bronunethyl)phenyl](cyclopentyl)acetat, 5.92 g (33.6 mmol) 2-Phenyl-4*H*-1,3,4-oxadiazin-5(6*H*)-on und 13.70 g (42.03 mmol) Cäsiumcarbonat wurden in 100 ml DMF 12 h lang bei 60°C gerührt. Nach dem Abkühlen wurde der Ansatz auf Eiswasser gegeben und mit Diethylether extrahiert. Die organische Phase wurde über Magnesiumsulfat getrocknet und im Vakuum bis zur Trockene eingeengt. Das Rohprodukt wurde über Kieselgel chromatographisch gereinigt (Laufmittel Cyclohexan/Essigsäureethylester 20:1). Es wurden 6.6 g (14.7 mmol, 52% d. Th.) der Titelverbindung erhalten.

### Darstellungsmethode 2:

8.16 g (23.1 mmol) *tert*.-Butyl-[4-(brommethyl)phenyl](cyclopentyl)acetat, 3.7 g (21 mmol) 2-Phenyl-4*H*-1,3,4-oxadiazin-5(6*H*)-on und 7.53 g (23.1 mmol) Cäsiumcarbonat wurden in 147 ml DMF 12 h lang bei Raumtemperatur gerührt. Anschließend wurde die Reaktionslösung mit gesättigter wässriger Natriumhydrogencarbonat-Lösung verrührt und zweimal mit Essigsäureethylester extrahiert. Die vereinigten organischen Phasen wurden über Magnesiumsulfat getrocknet und im Vakuum bis zur Trockene eingeengt. Das erhaltene Rohprodukt wurde über Kieselgel chromatographisch gereinigt (Laufmittel Cyclohexan/Essigsäureethylester 5:1). Es wurden 6.51 g (14.5 mmol, 69% d. Th.) der Titelverbindung erhalten.
¹H-NMR (400 MHz, DMSO-d₆, δ/ppm): 7.76 (2H, d), 7.55-7.42 (3H, m), 7.31 (4H, s), 4.94 (2H, s), 4.87 (2H, s), 3.19 (1H, d), 2.45-2.31 (1H, m), 1.88-1.74 (1H, m), 1.69-1.46 (3H, m), 1.45-1.15 (3H, m), 1.34 (9H, s), 1.03-0.89 (1H, m).
LC-MS (Methode 7): Rₜ = 3.27 min; m/z = 449 (M+H)⁺.

Auf analoge Weise wurden die in der folgenden Tabelle aufgeführten Verbindungen erhalten:

| **Beispiel** | **Name / Struktur** | **Analytische Daten** |
|---|---|---|
| **26A** | *tert*.-Butyl-(4-{[2-(3-chlorphenyl)-5-oxo-5,6-dihydro-4*H*-1,3,4-oxadiazin-4-yl]methyl}phenyl)(cyclopentyl)acetat | LC-MS (Methode 7): Rₜ = 3.59 min; m/z = 483 (M)⁺. |
| | 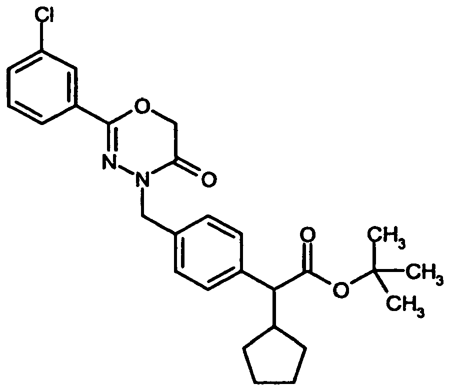 | |
| **27A** | *tert*.-Butyl-(4-{[2-(2-chlorphenyl)-5-oxo-5,6-dihydro-4*H*-1,3,4-oxadiazin-4-yl]methyl}phenyl)(cyclopentyl)acetat | LC-MS (Methode 7): Rₜ = 3.28 min; m/z = 483 (M)⁺. |
| | 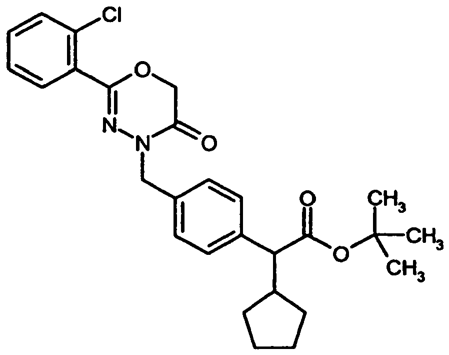 | |
| **28A** | Methyl-(4-([2-(4-chlorphenyl)-5-oxo-5,6-dihydro-4*H*-l,3,4-oxadiazin-4-yl]methyl)phenyl)(cydopentyl)acetat | LC-MS (Methode 7): Rₜ = 3.29 min; m/z = 441 (M+H)⁺. |
| | 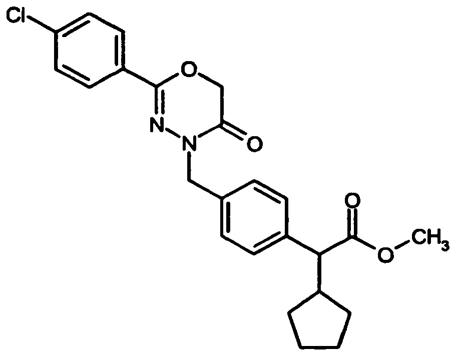 | |
| **29A** | *tert*.-Butyl-cyclopentyl(4-{[2-(2-methoxyphenyl)-5-oxo-5,6-dihydro-4*H*-1,3,4-oxadiazin-4-yl]methyl}phenyl)-acetat | LC-MS (Methode 2): Rₜ = 3.09 min; m/z = 479 (M+H)⁺. |
| | 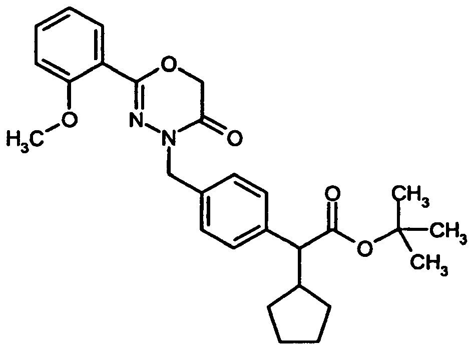 | |
| **30A** | Methyl-cyclopentyl(4-{[2-(4-fluorphenyl)-5-oxo-5,6-dihydro-4*H*-1,3,4-oxadiazin-4-yl]methyl}phenyl)acetat | LC-MS (Methode 7): Rₜ = 3.16 min; m/z = 425 (M+H)⁺. |
| | 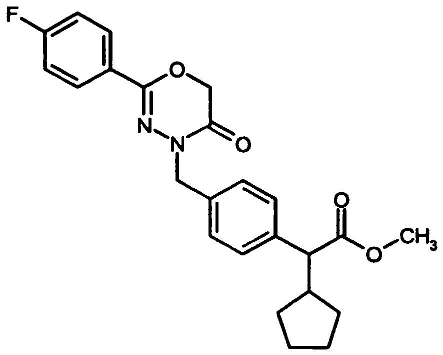 | |
| **31A** | *tert*.-Butyl-cyclopentyl(4-{[2-(2-methylpropyl)-5-oxo-5,6-dihydro-4*H*-1,3,4-oxadiazin-4-yl]methyl}phenyl)-acetat | LC-MS (Methode 10): Rₜ = 2.92 min; m/z = 429 (M+H)⁺. |
| | 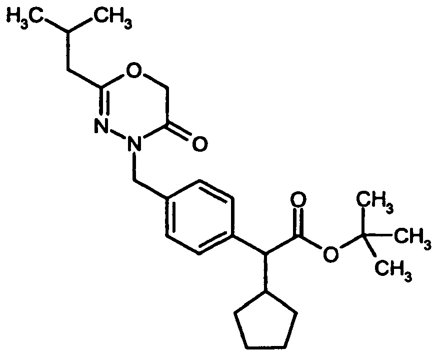 | |
| **32A** | *tert*.-Butyl-cyclopentyl{4-[(3,5-dioxo-6-phenyl-2,5-dihydro-1,2,4-triazin-4(3*H*)-yl)methyl]phenyl}acetat | LC-MS (Methode 2): Rₜ = 3.04 min; m/z = 406 (M-C₄H₈+H)⁺. |
| | 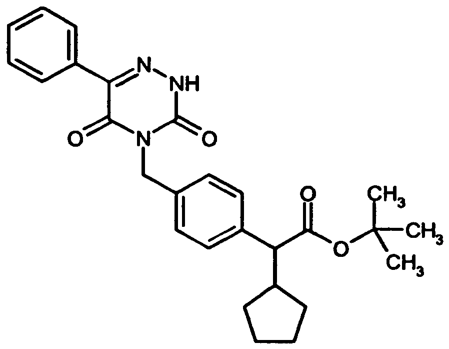 | |
| **33A** | *tert*.-Butyl-cyclopentyl{4-[(4-oxo-1,2,3-benzotriazin-3(4*H*)-yl)methyl]phenyl}acetat | ¹H-NMR (400 MHz, DMSO-d₆, δ/ppm): 8.29-8.11 (2H, m), 8.10 (1H, t), 7.95 (1H, t), 7.37-7.28 (4H, m), 5.58 (2H, s), 3.19 (1H, d), 2.43-2.26 (1H, m), 1.87-1.74 (1H, m), 1.67-1.45 (3H, m), 1.44-1.30 (1H, m), 1.35 (9H, s), 1.30-1.16 (2H, m), 0.99-0.87 (1H, m). LC-MS (Methode 10): Rₜ = 2.73 min; m/z = 420 (M+H)⁺, 442 (M+Na)⁺. |
| | 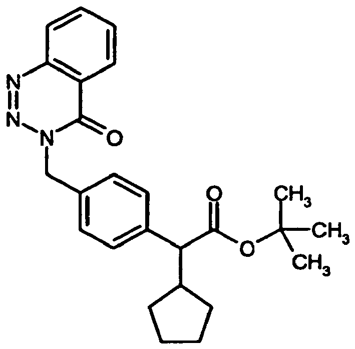 | |
| **34A** | *tert*.-Butyl-cyclopentyl{4-[(2-oxo-5-phenyl-1,3,4-oxadiazol-3(2*H*)-yl)methyl]phenyl} acetat | LC-MS (Methode 7): Rₜ = 3.27 min; m/z = 452 (M+NH₄)⁺. |
| | 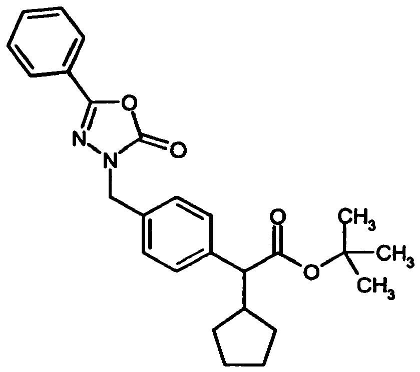 | |
| **35A** | Methyl-cyclopentyl(4-{[4-methyl-5-(2-methylpropyl)-2-oxo-1,3-thiazol-3(2*H*)-yl]methyl)phenyl)acetat | LC-MS (Methode 13): Rₜ = 2.99 min; m/z = 402 (M+H)⁺. |
| | 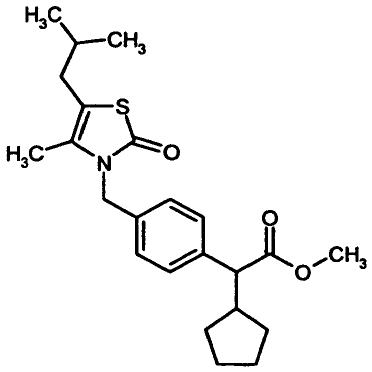 | |
| **36A** | Methyl-cyclopentyl(4-{[5-oxo-2-(propan-2-yl)-5,6-dihydro-4*H*-1,3,4-oxadiazin-4-yl]methyl}phenyl)acetat | LC-MS (Methode 7): Rₜ = 2.86 min; m/z = 373 (M+H)⁺. |
| | 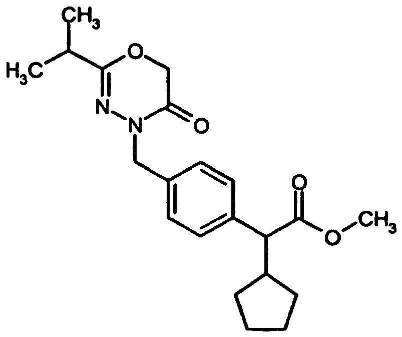 | |
| **37A** | Methyl-{4-[(2-*tert*.-butyl-5-oxo-5,6-dihydro-4H-1,3,4-oxadiazin-4-yl)methyl]phenyl}(cyclopentyl)acetat | LC-MS (Methode 7): Rₜ = 3.02 min; m/z = 387 (M+H)⁺. |
| | 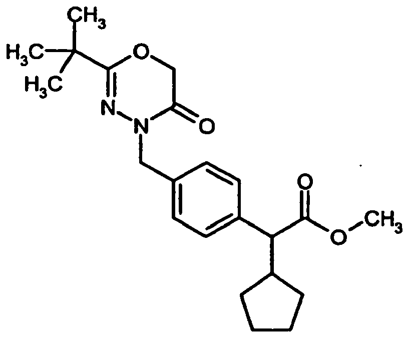 | |
| **38A** | tert.-Butyl-2-{4-[(2-*tert*.-butyl-5-oxo-5,6-dihydro-4H 1,3,4-oxadiazin-4-yl)methyl]phenyl}-3-methylbutanoat | LC-MS (Methode 11): Rₜ = 1.69 min; m/z = 403 (M+H)⁺. |
| | 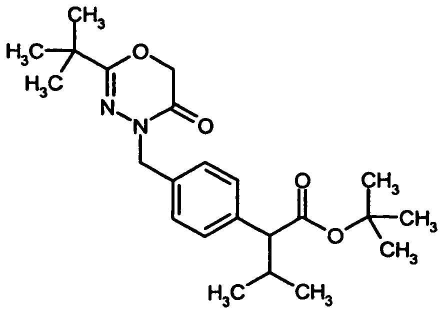 | |
| **39A** | *tert*.-Butyl-3-methyl-2-{4-[(5-oxo-2-phenyl-5,6-dihydro-4*H*-1,3,4-oxadiazin-4-yl)methyl]phenyl}butanoat | LC-MS (Methode 10): Rₜ = 2.76 min; m/z = 423 (M+H)⁺. |
| | 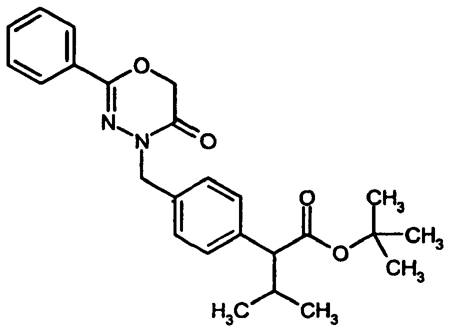 | |
| **40A** | *tert*.-Butyl-5,5,5-trifluor-2-{4-[(5-oxo-2-phenyl-5,6-dihydro-4*H*-1,3,4-oxadiazin-4-yl)methyl]phenyl}-pentanoat | LC-MS (Methode 10): Rₜ = 2.69 min; m/z = 477 (M+H)⁺. |
| | 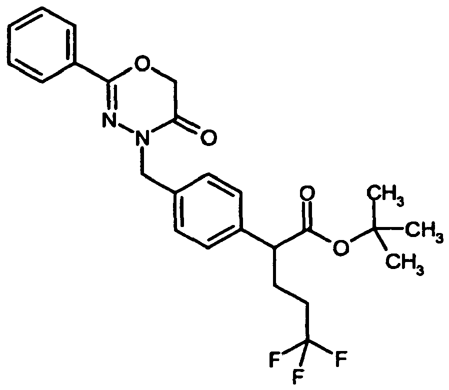 | |
| **41A** | *tert*.-Butyl-3-methyl-2-{4-[(5-oxo-2-phenyl-5,6-dihydro-4*H*-1,3,4-oxadiazin-4-y1)methyl]phenyl}pentanoat | LC-MS (Methode 7): Rₜ = 3.24 min; m/z = 437 (M+H)⁺. |
| | 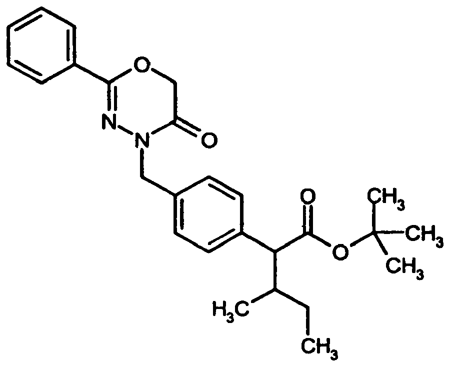 | |
| **42A** | *tert*.-Butyl-3-methyl-2-{4-[(5-oxo-2-phenyl-5,6-dihydro-4*H*-1,3,4-oxadiazin-4-yl)methyl]phenyl}hexanoat | LC-MS (Methode 11): Rₜ = 1.76 min; m/z = 473 (M+Na)⁺. |
| | 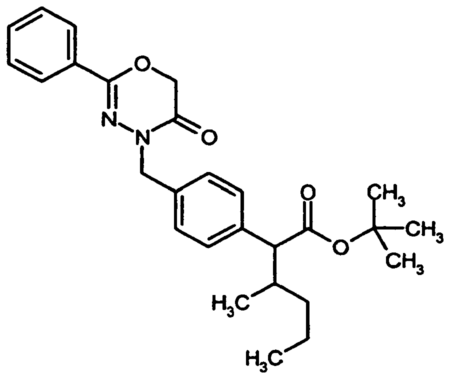 | |
| **43A** | *tert*.-Butyl-4-cyano-3-methyl-2-{4-[(5-oxo-2-phenyl-5,6-dihydro-4*H*-1,3,4-oxadiazin-4-yl)methyl]phenyl}butanoat | LC-MS (Methode 11): Rₜ = 1.46 min; m/z = 448 (M+H)⁺. |
| | 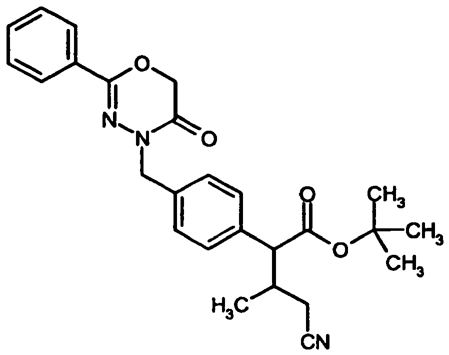 | |
| **44A** | *tert*.-Butyl-4-methoxy-3-methyl-2-{4-[(5-oxo-2-phenyl-5,6-dihydro-4*H*-1,3,4-oxadiazin-4-yl)methyl]phenyl}-butanoat | LC-MS (Methode 10): Rₜ = 2.62 min; m/z = 475 (M+Na)⁺. |
| | 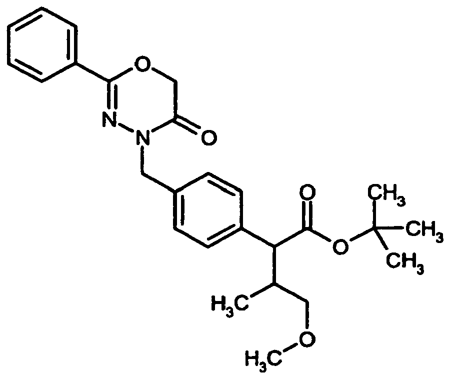 | |

### Beispiel 45A

### tert.-Butyl-(4-{[4-(4-chlorphenyl)-1-oxophthalazin-2(1H)-yl]methyl}phenyl)(cyclopentyl)acetat

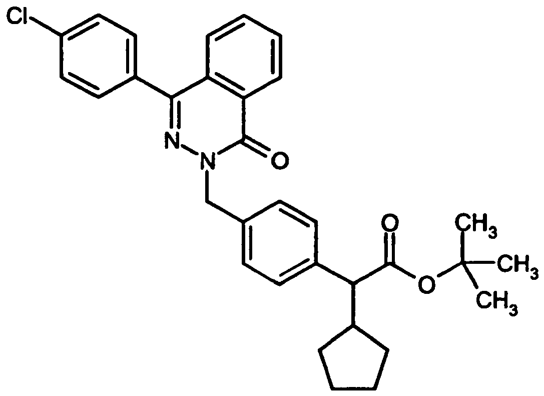

Eine Lösung von 500 mg (1.95 mmol) 4-(4-Chlorphenyl)phthalazin-1(2H)-on [CAS Reg.-Nr. 51334-86-2] in 7.6 ml THF wurde bei 0°C portionsweise mit 54 mg (2.14 mmol) Natriumhydrid versetzt. Nach Beendigung der Gasentwicklung wurden 688 mg (1.95 mmol) *tert.*-Butyl-[4-(brommethyl)phenyl](cyclopentyl)acetat, gelöst in 4.5 ml DMF, langsam bei 0°C zugetropft und die Reaktionslösung dann über Nacht bei Raumtemperatur gerührt. Anschließend wurde das Reaktionsgemisch vorsichtig mit Wasser versetzt. Der Ansatz wurde dreimal mit Essigsäureethylester extrahiert, die vereinigten organischen Phasen mit gesättigter Natriumchlorid-Lösung gewaschen und über Natriumsulfat getrocknet. Nach Filtration wurde das Lösungsmittel im Vakuum bis zur Trockene entfernt. Das erhaltene Rohprodukt wurde durch Flash-Chromatographie an Kieselgel gereinigt (Laufmittel: zunächst Cyclohexan/Essigsäureethylester 3:1, dann Essigsäureethylester). Es wurden 630 mg (93% Reinheit, 1.11 mmol, 57% d. Th.) eines farblosen Feststoffs erhalten.
¹H-NMR (400 MHz, DMSO-d₆, δ/ppm): 8.43-8.35 (1H, m), 7.98-7.88 (2H, m), 7.74-7.68 (1H, m), 7.57-7.49 (4H, m), 7.37-7.25 (4H, m), 5.37 (2H, s), 3.18 (1H, d), 2.42-2.29 (1H, m), 1.86-1.72 (1H, m), 1.68-1.45 (3H, m), 1.44-1.30 (1H, m), 1.38 (9H, s), 1.29-1.15 (2H, m), 1.00-0.86 (1H, m).
LC-MS (Methode 2): Rₜ = 3.45 min; m/z = 529 (M+H)⁺.

Auf analoge Weise wurden die in der folgenden Tabelle aufgeführten Verbindungen erhalten:

| **Beispiel** | **Name / Struktur** | **Analytische Daten** |
|---|---|---|
| **46A** | *tert.*-Butyl-cyclopentyl{4-[(3-methyl-2-oxo-imidazolidin-1-yl)methyl]phenyl} acetat | ¹H-NMR (400 MHz, DMSO-d₆, δ/ppm): 7.28 (2H, d), 7.16 (2H, d), 4.23 (2H, s), 3.24 (2H, t), 3.17 (1H, d), 3.12 (2H, t), 2.44-2.30 (1H, m), 1.87-1.74 (1H, m), 1.67-1.45 (3H, m), 1.44-1.31 (1H, m), 1.35 (9H, s), 1.30-1.17 (2H, m), 1.01-0.90 (1H, m). MS (DCI): m/z = 373 (M+H)⁺. |
| | 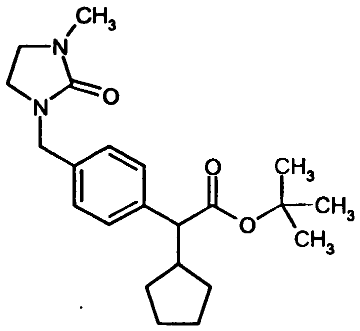 | |
| **47A** | *tert*.-Butyl-(4-{[3-(3-chlorphenyl)-2-oxoimidazolidin-1-yl]methyl}phenyl)(cyclopentyl)acetat | ¹H-NMR (400 MHz, DMSO-d₆, δ/ppm): 7.81 (1H, t), 7.44 (1H, d), 7.35 (1H, d), 7.31 (2H, d), 7.24 (2H, d), 7.05 (1H, d), 4.37 (2H, s), 3.83 (2H, t), 3.37 (2H, t), 3.19 (1H, d), 2.45-2.31 (1H, m), 1.88-1.76 (1H, m), 1.69-1.46 (3H, m), 1.45-1.32 (1H, m), 1.36 (9H, s), 1.31-1.17 (2H, m), 1.01-0.90 (1H, m). LC-MS (Methode 11): Rₜ = 1.75 min; m/z = 413 (M-C₄H₈)⁺. |
| | 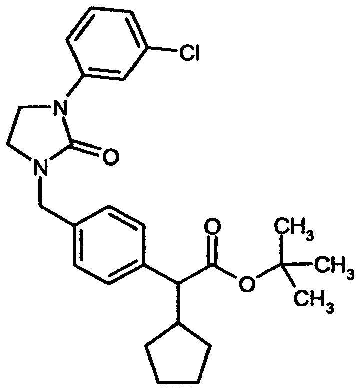 | |
| **48A** | *tert.*-Butyl-cyclopentyl(4-{[(4*S*,5*R*)-4-methyl-2-oxo-5-phenyl-1,3-oxazolidin-3-yl]methyl}phenyl)acetat | LC-MS (Methode 2): Rₜ = 3.14 min; m/z = 899 (2M+H)⁺. |
| | 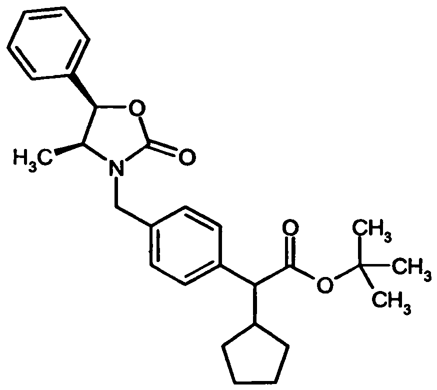 | |
| **49A** | *tert*.-Butyl-cyclopentyl(4-{[(4*R*,5*S*)-4-methyl-2-oxo-5-phenyl-1,3-oxazolidin-3-yl]methyl}phenyl)acetat | LC-MS (Methode 2): Rₜ = 3.14 min; m/z = 899 (2M+H)⁺. |
| | 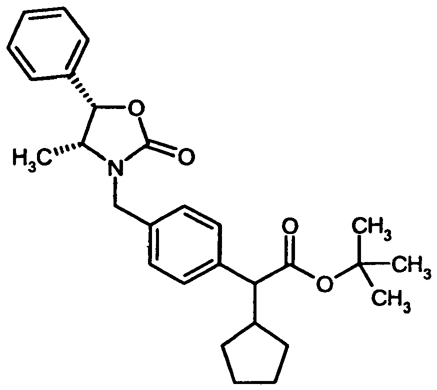 | |
| **50A** | *tert*.-Butyl-cyclopentyl{4-[(5-methyl-2-oxo-5-phenylpiperidin-1-yl)methyl]phenyl}acetat | LC-MS (Methode 7): Rₜ = 3.35 min; m/z = 923 (2M+H)⁺. |
| | 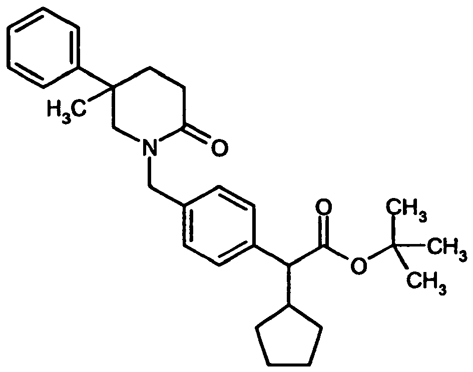 | |

### Beispiel 51A

### rac-Cyclopentyl{4-[(5-oxo-2-phenyl-5,6-dihydro-4H-1,3,4-oxadiazin-4-yl)methyl]phenyl}-essigsäure

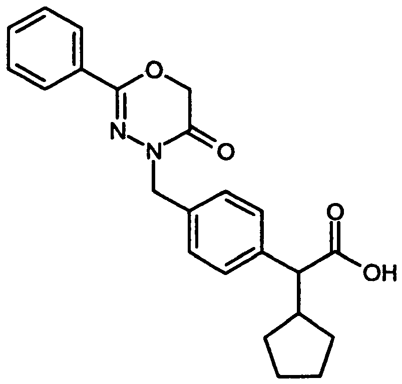

Eine Lösung von 6.6 g (14.7 mmol) *t***ert**.-Butyl-cyclopentyl{4-[(5-oxo-2-phenyl-5,6-dihydro-4*H* 1,3,4-oxadiazin-4-yl)methyl]phenyl}acetat in 90 ml Dichlormethan wurde bei Raumtemperatur langsam mit 22.67 ml (294.3 mmol) Trifluoressigsäure versetzt und die Mischung über Nacht gerührt. Danach wurde das Lösungsmittel im Vakuum entfernt, der Rückstand in 100 ml Essigsäureethylester aufgenommen und mit 50 ml Wasser ausgeschüttelt. Die organische Phase wurde über Magnesiumsulfat getrocknet. Nach Filtration wurde das Lösungsmittel im Vakuum entfernt. Es wurden 4.8 g (12.23 mmol, 83% d. Th.) eines farblosen Feststoffs erhalten.
¹H-NMR (400 MHz, DMSO-d₆, δ/ppm): 12.35-12.15 (1H, breites s), 7.78 (2H, d), 7.54-7.40 (3H, m), 7.29 (4H, s), 4.91 (2H, s), 4.83 (2H, s), 3.22 (1H, d), 2.48-2.35 (1H, m), 1.89-1.76 (1H, m), 1.68-1.46 (3H, m), 1.45-1.32 (1H, m), 1.32-1.14 (2H, m), 1.01-0.89 (1H, m).
LC-MS (Methode 7): Rₜ = 2.75 min; m/z = 393 (M+H)⁺.

Auf analoge Weise wurden die in der folgenden Tabelle aufgeführten Verbindungen erhalten:

| **Beispiel** | **Name / Struktur** | **Analytische Daten** |
|---|---|---|
| **52A** | (4-{[2-(3-Chlorphenyl)-5-oxo-5,6-dihydro-4*H*-1,3,4-oxadiazin-4-yl]methyl}phenyl)(cyclopentyl)essigsäure | LC-MS (Methode 7): Rₜ = 2.78 min; m/z = 427 (M+H)⁺. |
| | 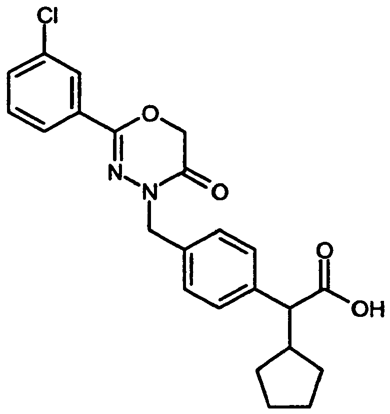 | |
| **53A** | (4-{[2-(2-Chlorphenyl)-5-oxo-5,6-dihydro-4*H*-1,3,4-oxadiazin-4-yl]methyl}phenyl)(cyclopentyl)essigsäure | LC-MS (Methode 7): Rₜ = 2.74 min; m/z = 427 (M+H)⁺. |
| | 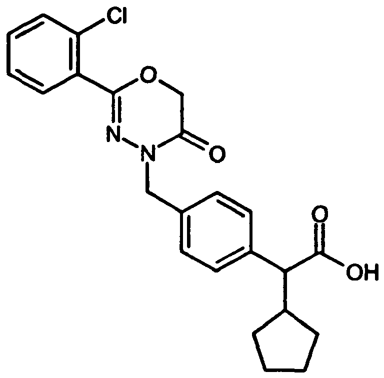 | |
| **54A** | Cyclopentyl(4-{[2-(2-methoxyphenyl)-5-oxo-5,6-dihydro-4*H*-1,3,4-oxadiazin-4-yl]methyl}phenyl)-essigsäure | LC-MS (Methode 7): Rₜ = 2.66 min; m/z = 423 (M+H)⁺. |
| | 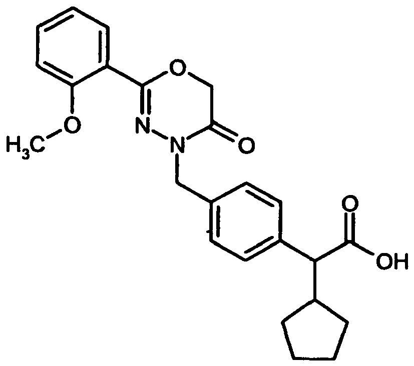 | |
| **55A** | Cyclopentyl {4-[(3,5-dioxo-6-phenyl-2,5-dihydro-1,2,4-triazin-4(3*H*)-yl)methyl]phenyl}essigsäure | LC-MS (Methode 2): Rₜ = 2.28 min; m/z = 811 (2M+H)⁺. |
| | 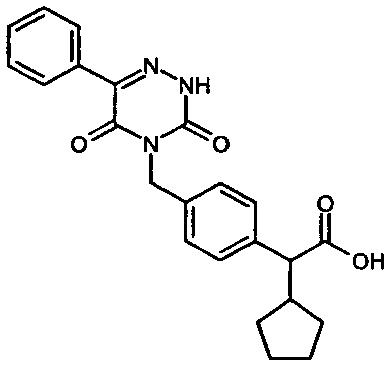 | |
| **56A** | Cyclopentyl {4-[(4-oxo-1,2,3-benzotriazin-3(4*H*)-yl)-methyl]phenyl} essigsäure | ¹H-NMR (400 MHz, DMSO-d₆, δ/ppm): 12.6-11.8 (1H, breites s), 8.28-8.21 (2H, m), 8.10 (1H, t), 7.94 (1H, t), 7.36-7.28 (4H, m), 5.57 (2H, s), 3.21 (1H, d), 2.48-2.35 (1H, m), 1.87-1.77 (1H, m), 1.66-1.45 (3H, m), 1.45-1.33 (1H, m), 1.31-1.16 (2H, m), 0.98-0.86 (1H, m). LC-MS (Methode 7): Rₜ = 2.45 min; m/z = 364 (M+H)⁺. |
| | 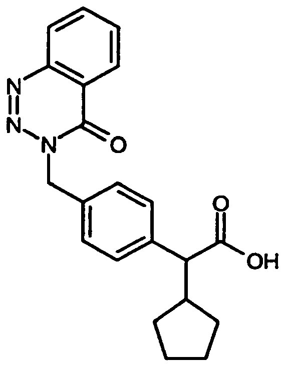 | |
| **57A** | Cyclopentyl {4-[(2-oxo-5-phenyl-1,3,4-oxadiazol-3(2*H*)-yl)methyl]phenyl} essigsäure | ¹H-NMR (400 MHz, DMSO-d₆, δ/ppm): 12.6-11.9 (1H, breites s), 7.78 (2H, d), 7.62-7.48 (3H, m), 7.39-7.25 (4H, m), 4.95 (2H, s), 3.24 (1H, d), 2.48-2.36 (1H, m), 1.90-1.76 (1H, m), 1.67-1.47 (3H, m), 1.47-1.35 (1H, m), 1.33-1.17 (2H, m), 1.02-0.88 (1H, m). LC-MS (Methode 7): Rₜ = 2.64 min; m/z = 379 (M+H)⁺. |
| | 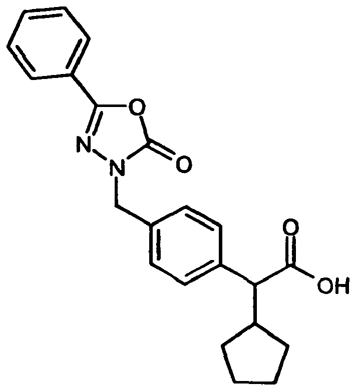 | |
| **58A** | 2-{4-[(2-*tert.*-Butyl-5-oxo-5,6-dihydro-4*H*-1,3,4-oxadiazin-4-yl)methyl]phenyl}-3-methylbutansäure | LC-MS (Methode 7): Rₜ = 2.53 min; m/z = 347 (M+H)⁺. |
| | 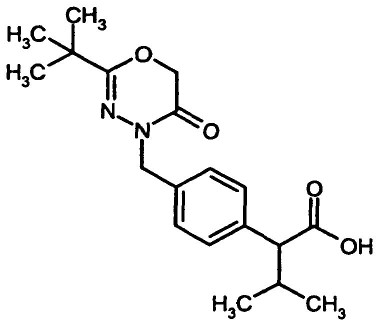 | |
| 59A | 3-Methyl-2-{4-[(5-oxo-2-phenyl-5,6-dihydro-4*H*-1,3,4-oxadiazin-4-yl)methyl]phenyl}butansäure | ¹H-NMR (400 MHz, DMSO-d₆, δ/ppm): 12.28 (1H, s), 7.76 (2H, d), 7.54-7.40 (3H, m), 7.38-7.24 (4H, m), 4.91 (2H, s), 4.86 (2H, s), 3.09 (1H, d), 2.26-2.11 (1H, m), 0.98 (3H, d), 0.61 (3H, d). LC-MS (Methode 7): Rₜ = 2.60 min; m/z = 367 (M+H)⁺. |
| | 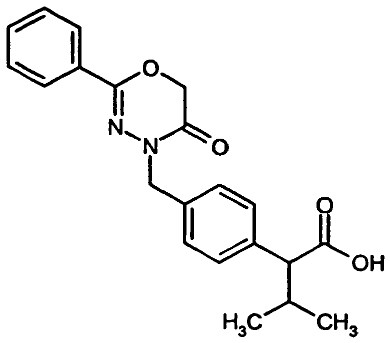 | |
| **60A** | 5,5,5-Trifluor-2-{4-[(5-oxo-2-phenyl-5,6-dihydro-4*H* 1,3,4-oxadiazin-4-yl)methyl]phenyl}pentansäure | ¹H-NMR (400 MHz, DMSO-d₆, δ/ppm): 13.0-12.0 (1H, breites s), 7.77 (2H, d), 7.55-7.41 (3H, m), 7.35 (2H, d), 7.26 (2H, d), 4.92 (2H, s), 4.87 (2H, s), 3.67-3.53 (1H, m), 2.31-1.95 (3H, m), 1.89-1.74 (1H, m). LC-MS (Methode 7): Rₜ = 2.47 min; m/z = 421 (M+H)⁺. |
| | 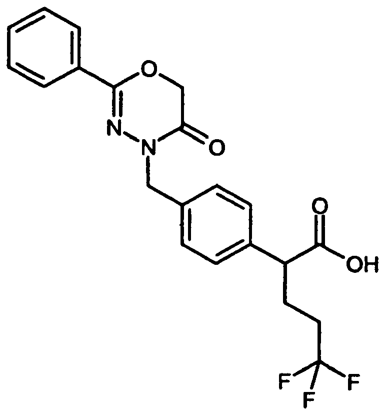 | |
| **61A** | 3-Methyl-2-{4-[(5-oxo-2-phenyl-5,6-dihydro-4*H*-1,3,4-oxadiazin-4-yl)methyl]phenyl}pentansäure | LC-MS (Methode 10): Rₜ = 2.15 min; m/z = 381 (M+H)⁺. |
| | 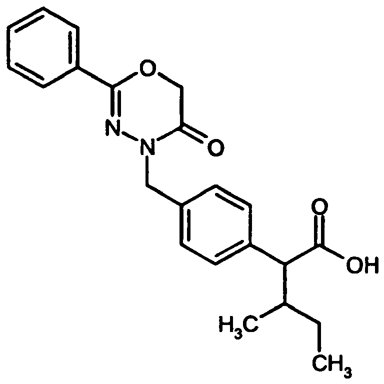 | |
| **62A** | 3-Methyl-2-{4-[(5-oxo-2-phenyl-5,6-dihydro-4*H*-1,3,4-oxadiazin-4-yl)methyl]phenyl}hexansäure | LC-MS (Methode 12): Rₜ = 2.52 min; m/z = 395 (M+H)⁺. |
| | 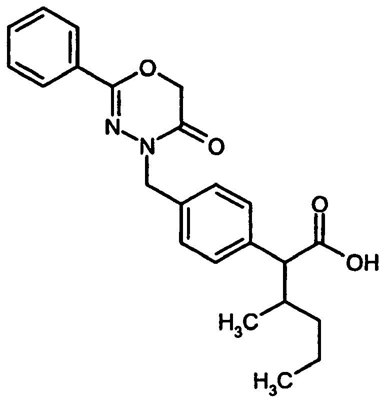 | |
| **63A** | 4-Cyano-3-methyl-2-{4-[(5-oxo-2-phenyl-5,6-dihydro-4*H*-1,3,4-oxadiazin-4-yl)methyl]phenyl}butansäure | LC-MS (Methode 11): Rₜ = 1.13 min; m/z = 392 (M+H)⁺. |
| | 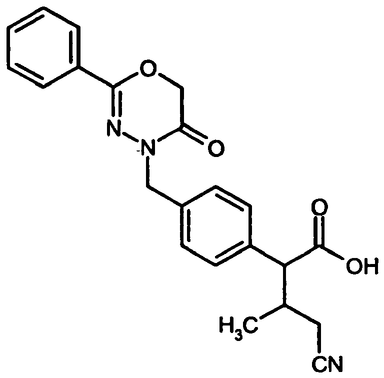 | |
| **64A** | 4-Methoxy-3-methyl-2-{4-[(5-oxo-2-phenyl-5,6-dihydro-4*H*-1,3,4-oxadiazin-4-yl)methyl]phenyl}-butansäure | LC-MS (Methode 7): Rₜ = 2.26 min; m/z = 397 (M+H)⁺. |
| | 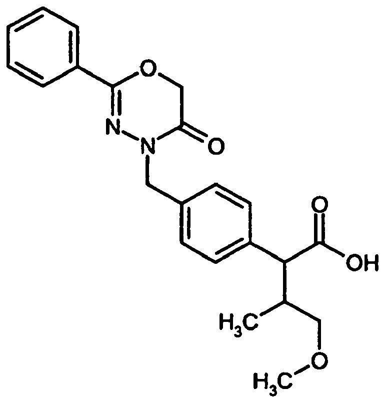 | |
| **65A** | (4-{[4-(4-Chlorphenyl)-1-oxophthalazin-2(1*H*)-yl]-methyl}phenyl)(cyclopentyl)essigsäure | LC-MS (Methode 2): Rₜ = 2.89 min; m/z = 473 (M+H)⁺. |
| | 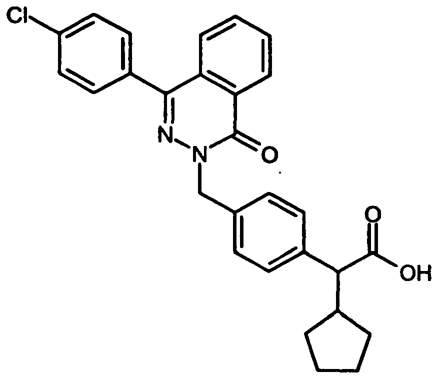 | |
| **66A** | Cyclopentyl{4-[(3-methyl-2-oxoimidazolidin-1-yl)-methyl]phenyl}essigsäure | ¹H-NMR (400 MHz, DMSO-d₆, δ/ppm): 12.23 (1H, s), 7.28 (2H, d), 7.18 (2H, d), 4.22 (2H, s), 3.28-3.17 (3H, m), 3.11 (2H, t), 2.47-2.35 (1H, m), 1.89-1.77 (1H, m), 1.69-1.48 (3H, m), 1.48-1.34 (1H, m), 1.33-1.15 (2H, m), 1.00-0.88 (1H, m). MS (DCI): m/z = 317 (M+H)⁺. |
| | 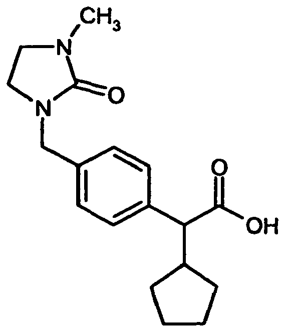 | |
| **67A** | (4-{[3-(3-Chlorphenyl)-2-oxoimidazolidin-1-yl]-methyl}phenyl)(cyclopentyl)essigsäure | ¹H-NMR (400 MHz, DMSO-d₆, δ/ppm): 12.5-11.9 (1H, breites s), 7.81 (1H, s), 7.44 (1H, d), 7.38-7.28 (3H, m), 7.25 (2H, d), 7.04 (1H, d), 4.37 (2H, s), 3.81 (2H, t), 3.37 (2H, t), 3.22 (1H, d), 2.48-2.37 (1H, m), 1.89-1.76 (1H, m), 1.68-1.47 (3H, m), 1.47-1.34 (1H, m), 1.33-1.15 (2H, m), 1.01-0.88 (1H, m). LC-MS (Methode 7): Rₜ = 2.72 min; m/z = 429/ 431 (M+NH₄)⁺. |
| | 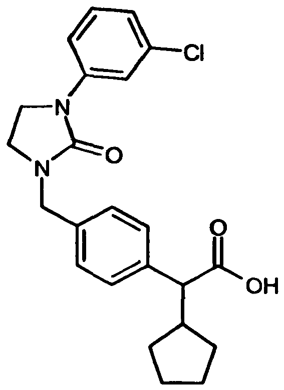 | |
| **68A** | Cyclopentyl(4-{[(4**S**,5*R*)-4-methyl-2-oxo-5-phenyl-1,3-oxazolidin-3-yl]methyl}phenyl)essigsäure | LC-MS (Methode 2): Rₜ = 2.38 min; m/z = 787 (2M+H)⁺. |
| | 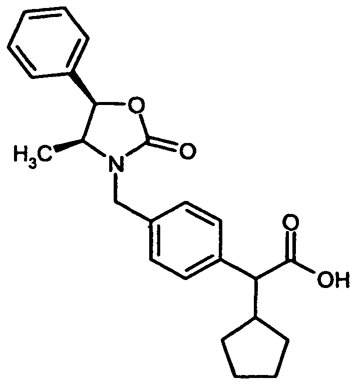 | |
| **69A** | Cyclopentyl(4-{[(4*R*,5*S*)-4-methyl-2-oxo-5-phenyl-1,3-oxazolidin-3-yl]methyl}phenyl)essigsäure | LC-MS (Methode 2): Rₜ = 2.38 min; m/z = 787 (2M+H)⁺. |
| | 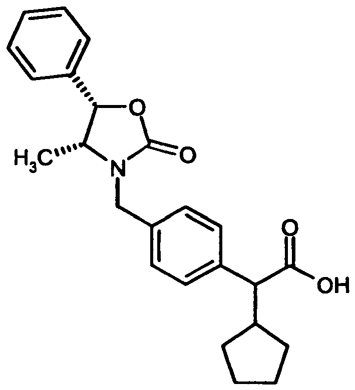 | ) |
| **70A** | Cyclopentyl{4-[(5-methyl-2-oxo-5-phenylpiperidin-1-yl)methyl]phenyl} essigsäure | LC-MS (Methode 7): Rₜ = 2.66 min; m/z = 406 (M+H)⁺. |
| | 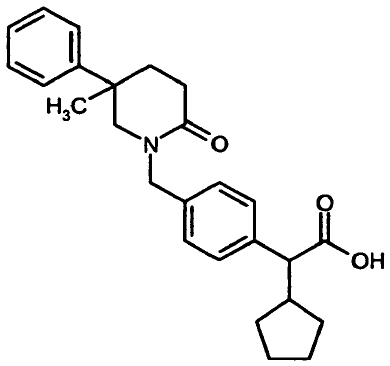 | |

### Beispiel 71A

### Cyclopentyl(4-{[4-methyl-5-(2-methylpropyl)-2-oxo-1,3-thiazol-3(2H)-yl]methyl}phenyl)-essigsäure

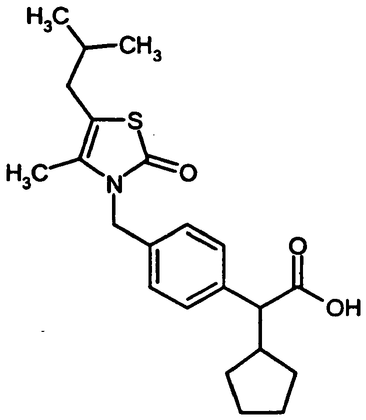

1.01 g (2.515 mmol) Cyclopentyl(4-{[4-methyl-5-(2-methylpropyl)-2-oxo-1,3-thiazol-3(2*H*)-yl]-methyl}phenyl)essigsäuremethylester wurden in 30 ml THF gelöst und mit 10 ml Methanol sowie 10 ml 1 N Natronlauge versetzt. Die Reaktionslösung wurde 2 Tage bei Raumtemperatur gerührt. Es wurden danach weitere 5 ml 1 N Natronlauge hinzugefügt und nochmals 2 Tage bei RT gerührt. Es wurden dann 20 ml 1 N Salzsäure zugegeben, und die Reaktionsmischung wurde dreimal mit Essigsäureethylester extrahiert. Die vereinigten organischen Phasen wurden über Natriumsulfat getrocknet und das Lösungsmittel am Rotationsverdampfer entfernt. Es wurden 956 mg (2.47 mmol, 98% d. Th.) der Zielverbindung erhalten.
LC-MS (Methode 7): Rₜ = 2.95 min; m/z = 775.4 (2M+H)⁺.

### Beispiel 72A

### (4-{[2-(4-Chorphenyl)-5-oxo-5,6-dihydro-4H-1,3,4-oxadiazin-4-yl]methyl}phenyl)(cyclopentyl)-essigsäure

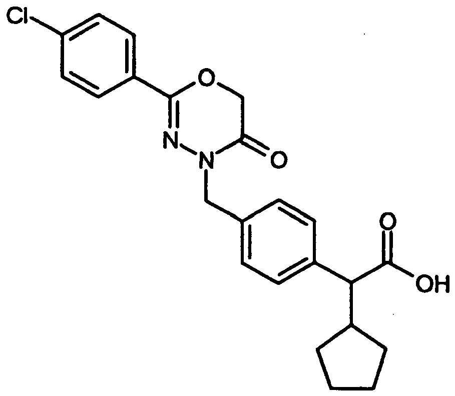

Eine Lösung von 450 mg (1.02 mmol) Methyl-(4-{[2-(4-chlorphenyl)-5-oxo-5,6-dihydro-4*H*-1,3,4-oxadiazin-4-yl]methyl}phenyl)(cyclopentyl)acetat in 10 ml Dioxan und 10 ml Wasser wurde mit 86 mg (2.04 mmol) Lithiumhydroxid-Monohydrat versetzt und 12 h bei 80°C gerührt. Nach Entfernen des Dioxans im Vakuum wurde die wässrige Phase mit 1 M Salzsäure auf pH 2 gestellt, wobei das Produkt flockig ausfiel. Die Lösung wurde filtriert und der Filter-Rückstand im Vakuum getrocknet. Es wurden 373 mg (0.87 mmol, 86% d. Th.) der Titelverbindung als weißer Feststoff erhalten.
LC-MS (Methode 13): Rₜ = 2.67 min; m/z = 427 (M+H)⁺.

Auf zu Beispiel 71A und 72A analoge Weise wurden die in der folgenden Tabelle aufgeführten Verbindungen erhalten:

| **Beispiel** | **Name / Struktur** | **Analytische Daten** |
|---|---|---|
| **73A** | Cyclopentyl(4-{[5-oxo-2-(propan-2-yl)-5,6-dihydro-4*H*-1,3,4-oxadiazin-4-yl]methyl}phenyl)essigsäure | LC-MS (Methode 7): Rₜ = 2.52 min; m/z = 359 (M+H)⁺. |
| | 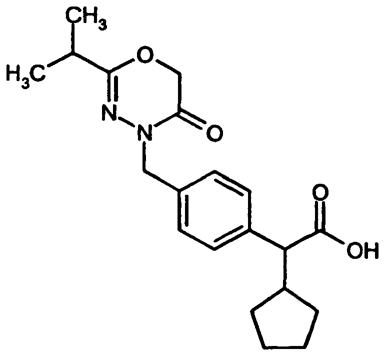 | |
| **74A** | {4-[(2-*tert*.-Butyl-5-oxo-5,6-dihydro-4*H*-1,3,4-oxadiazin-4-yl)methyl]phenyl}(cyclopentyl)essigsäure | LC-MS (Methode 7): Rₜ = 2.70 min; m/z = 373 (M+H)⁺. |
| | 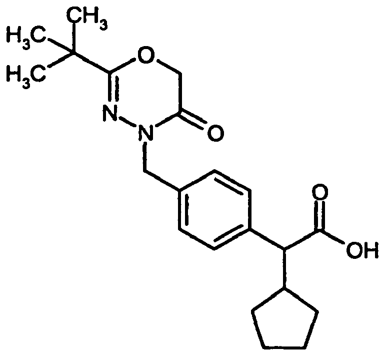 | |
| **75A** | Cyclopentyl(4-{[2-(4-fluorphenyl)-5-oxo-5,6-dihydro-4*H*-1,3,4-oxadiazin-4-yl]methyl}phenyl)essigsäure | LC-MS (Methode 13): Rₜ = 2.53 min; m/z = 411 (M+H)⁺. |
| | 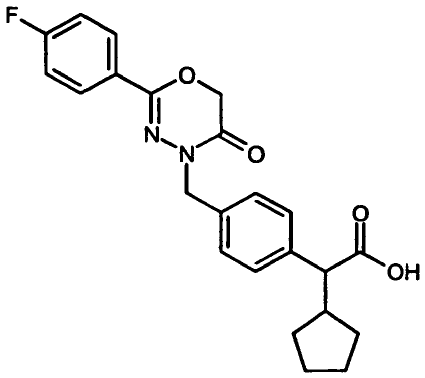 | |

### Beispiel 76A und Beispiel 77A

### ent-Cyclopentyl{4-[(5-oxo-2-phenyl-5,6-dihydro-4H-1,3,4-oxadiazin-4-yl)methyl]phenyl}essigsäure (Enantiomer 1 und 2)

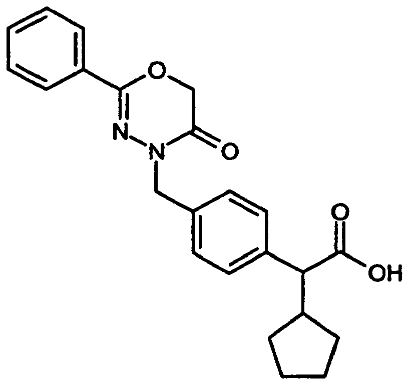

75 g (191.1 mmol) racemische Cyclopentyl{4-[(5-oxo-2-phenyl-5,6-dihydro-4*H*-1,3,4-oxadiazin-4-yl)methyl]phenyl}essigsäure (Beispiel 51A) wurden mittels präparativer HPLC an chiraler Phase in die Enantiomere aufgetrennt [Säule: chirale Kieselgel-Phase basierend auf dem Selektor Poly-(*N*-methacryloyl-L-isoleucin-3-pentylamid), 430 mm x 40 mm; Elutionsmittel: Isohexan/ Ethylacetat 1:1 (v/v); Fluss: 50 ml/min; Temperatur: 24°C; UV-Detektion: 270 nm]:

### Beispiel 76A (Enantiomer 1):

Ausbeute: 35 g
LC-MS (Methode 7): Rₜ = 2.75 min; m/z = 393 (M+H)⁺
Rₜ 5.73 min; Reinheit >99%; >99% ee
[Säule: chirale Kieselgel-Phase basierend auf dem Selektor Poly(*N*-methacryloyl-L-isoleucin-3-pentylamid), 250 mm x 4.6 mm; Elutionsmittel: Isohexan/Ethylacetat 1:1 (v/v); Fluss: 2 ml/min; Temperatur: 24°C; UV-Detektion: 270 nm].

### Beispiel 77A (Enantiomer 2):

Ausbeute: 32 g
¹H-NMR (400 MHz, DMSO-d₆, δ/ppm): 12.35-12.15 (1H, breites s), 7.78 (2H, d), 7.54-7.40 (3H, m), 7.29 (4H, s), 4.91 (2H, s), 4.83 (2H, s), 3.22 (1H, d), 2.48-2.35 (1H, m), 1.89-1.76 (1H, m), 1.68-1.46 (3H, m), 1.45-1.32 (1H, m), 1.32-1.14 (2H, m), 1.01-0.89 (1H, m).
LC-MS (Methode 7): Rₜ = 2.75 min; m/z = 393 (M+H)⁺
Rₜ 6.86 min; Reinheit >99%; >99% ee
[Säule: chirale Kieselgel-Phase basierend auf dem Selektor Poly(*N*-methacryloyl-L-isoleucin-3-pentylamid), 250 mm x 4.6 mm; Elutionsmittel: Isohexan/Ethylacetat 1:1 (v/v); Fluss: 2 ml/min;
Temperatur: 24°C; UV-Detektion: 270 nm].

### Beispiel 78A und Beispiel 79A

### ent-3-Methyl-2-{4-[(5-oxo-2-phenyl-5,6-dihydro-4H-1,3,4-oxadiazin-4-yl)methyl]phenyl}butansäure (Enantiomer 1 und 2)

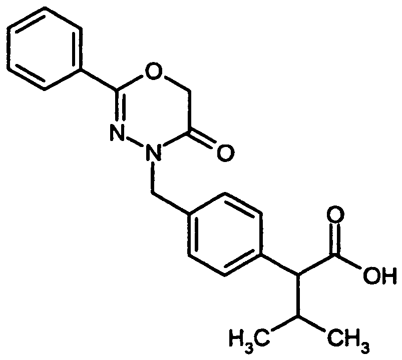

3 g (8.19 mmol) racemische 3-Methyl-2-{4-[(5-oxo-2-phenyl-5,6-dihydro-4*H*-1,3,4-oxadiazin-4-yl)methyl]phenyl}butansäure (Beispiel 59A) wurden mittels präparativer HPLC an chiraler Phase in die Enantiomere aufgetrennt [Säule: Daicel Chiralpak AD-H, 5 µm, 250 mm x 20 mm; Elutionsmittel: Isohexan/(Isopropanol + 0.2% Trifluoressigsäure + 1% Wasser) 50:50 (v/v); Fluss: 15 ml/min; Temperatur: 40°C; UV-Detektion: 220 nm]:

### Beispiel 78A (Enantiomer 1):

Rₜ 4.58 min; Reinheit >99%; >99% ee (Säule s.o.)
Ausbeute: 1.23 g
LC-MS (Methode 11): Rₜ = 1.26 min; m/z = 367 (M+H)⁺.

### Beispiel 79A (Enantiomer 2):

Rₜ 7.34 min; Reinheit >99%; >99% ee (Säule s.o.)
Ausbeute: 1.32 g
¹H-NMR (400 MHz, DMSO-d₆, δ/ppm): 12.5-12.0 (1H, breites s), 7.77 (2H, d), 7.55-7.40 (3H, m), 7.38-7.23 (4H, m), 4.91 (2H, s), 4.85 (2H, s), 3.09 (1H, d), 2.27-2.11 (1H, m), 0.99 (3H, d), 0.61 (3H, d).
LC-MS (Methode 11): Rₜ = 1.25 min; m/z = 367 (M+H)⁺.

### Beispiel 80A

### rac-Cyclopentyl{4-[(5-oxo-2-phenyl-5,6-dihydro-4H-1,3,4-oxadiazin-4-yl)methyl]phenyl}acetylchlorid

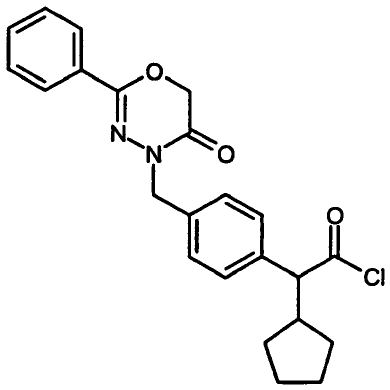

8.9 g (22.68 mmol) *rac*-Cyclopentyl{4-[(5-oxo-2-phenyl-5,6-dihydro-4*H*-1,3,4-oxadiazin-4-yl)-methyl]phenyl}essigsäure wurden mit 63.6 ml (871.5 mmol) Thionylchlorid 2 h bei Raumtemperatur gerührt. Die Reaktionslösung wurde anschließend auf 50°C erwärmt und weitere 2 h nachgerührt. Nach vollständiger Umsetzung wurde die Reaktionslösung im Vakuum vom Thionylchlorid befreit. Es wurden 7.7 g (18.74 mmol, 82% d. Th.) der Titelverbindung als farbloses Öl erhalten, welches ohne weitere Reinigung in Folgereaktionen eingesetzt wurde.
¹H-NMR (400 MHz, DMSO-d₆, δ/ppm): 7.76 (2H, d), 7.58-7.36 (3H, m), 7.30 (4H, s), 4.91 (2H, s), 4.82 (2H, s), 3.21 (1H, d), 2.48-2.29 (1H, m), 1.89-1.76 (1H, m), 1.68-1.45 (3H, m), 1.45-1.32 (1H, m), 1.32-1.15 (2H, m), 1.01-0.87 (1H, m).
MS (ESI): m/z = 411 (M+H)⁺.

Auf analoge Weise wurden die in der folgenden Tabelle aufgeführten Verbindungen erhalten:

| **Beispiel** | **Name / Struktur** | **Analytische Daten** |
|---|---|---|
| **81A** | *ent*-Cyclopentyl{4-[(5-oxo-2-phenyl-5,6-dihydro-4*H*-1,3,4-oxadiazin-4-yl)methyl]phenyl}acetylchlorid (*Enantiomer 1; aus Beispiel 77A*) | siehe Beispiel 80A |
| | 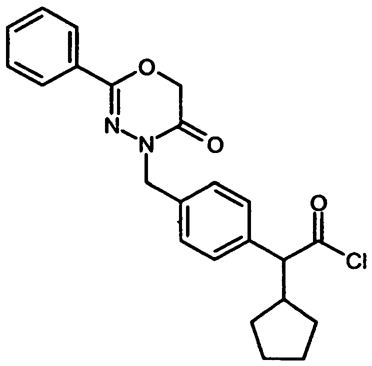 | |
| **82A** | Cyclopentyl(4-{[5-oxo-2-(propan-2-yl)-5,6-dihydro-4*H*-1,3,4-oxadiazin-4-yl]methyl}phenyl)acetylchlorid | |
| | 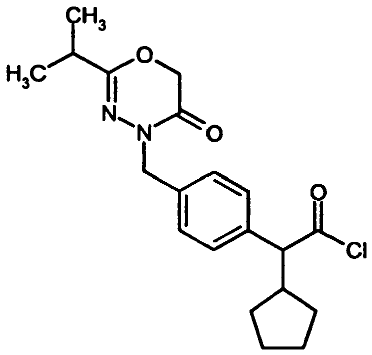 | |
| **83A** | {4-[(2-*tert*.-Butyl-5-oxo-5,6-dihydro-4*H*-1,3,4-oxadiazin-4-yl)methyl]phenyl}(cyclopentyl)acetylchlorid | |
| | 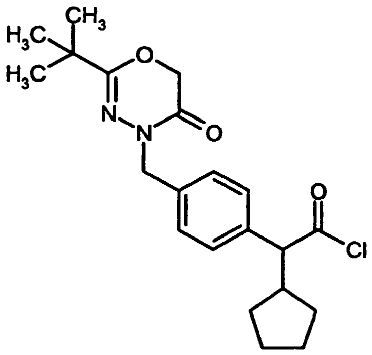 | |
| **84A** | Cyclopentyl(4-{[4-methyl-5-(2-methylpropyl)-2-oxo-1,3-thiazol-3(2*H*)-yl]methyl}phenyl)acetylchlorid | LC-MS (Methode 11): Rₜ = 1.45 min; m/z = 388 (M-Cl+H₂O)⁺. |
| | 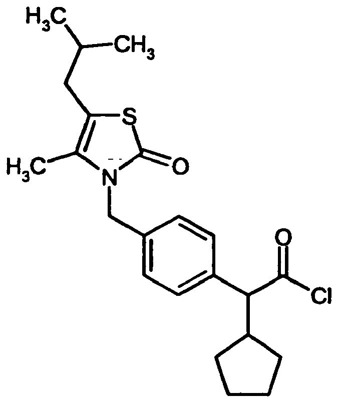 | |

### Beispiel 85A

### Dimethyl-4-nitro-1,3-dihydro-2H-inden-2,2-dicarboxylat

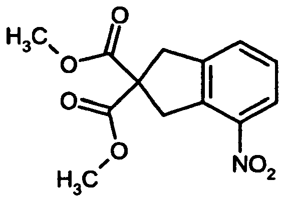

Eine Lösung von 126.79 g (0.96 mol) Malonsäuredimethylester, 296.5 g (0.96 mol) 1,2-Bis(brommethyl)-3-nitrobenzol und 530.5 g (3.84 mol) Kaliumcarbonat in 5.9 Liter Acetonitril wurde über Nacht unter Rückfluss gerührt (DC-Kontrolle: Laufmittel Petrolether/Dichlormethan 7:3 zur Identifizierung des Eduktes 1,2-Bis(brommethyl)-3-nitrobenzol, Dichlormethan/Petrolether 7:3 zur Identifizierung des Produktes). Nach Abkühlen auf Raumtemperatur und Filtration wurde das Lösungsmittel im Vakuum bis zur Trockene entfernt. Der Rückstand wurde in 2 Liter Essigsäureethylester aufgenommen und einmal mit 500 ml gesättigter Natriumchlorid-Lösung gewaschen und über Natriumsulfat getrocknet. Nach Filtration wurde das Lösungsmittel im Vakuum bis zur Trockene entfernt. Das erhaltene Rohprodukt wurde durch Flash-Chromatographie an 10 kg Kieselgel gereinigt (Laufmittel Dichlormethan/Petrolether 7:3 → 9:1). Es wurden 191 g (0.63 mol, Reinheit 93%, 66% d. Th.) eines farblosen Feststoffs erhalten.
¹H-NMR (400 MHz, CDCl₃, δ/ppm): 8.04 (1H, d), 7.51 (1H, d), 7.37 (1H, t), 4.11 (2H, s), 3.78 (6H, s), 3.68 (2H, s).
MS (DCI, NH₃): m/z = 297 (M+NH₄)⁺.

### Beispiel 86A

### 4-Nitro-2,3-dihydro-1H-inden-2-carbonsäure

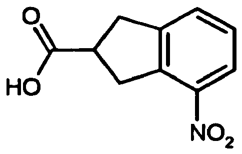

Eine Lösung von 190 g (0.68 mol) Dimethyl-4-nitro-1,3-dihydro-2*H*-inden-2,2-dicarboxylat in 1520 ml Dioxan wurde bei Raumtemperatur mit 1520 ml 2 N Natronlauge versetzt und anschließend zwei Stunden bei 50°C gerührt. Nach vollständigem Umsatz (DC-Kontrolle: Laufmittel Dichlormethan/Petrolether 8:2) wurde die Reaktionslösung langsam mit konzentrierter Salzsäure auf pH 1 eingestellt und über Nacht unter Rückfluss gerührt. Die Reaktionslösung wurde dann mit 3.4 Liter Wasser versetzt und dreimal mit jeweils 3.4 Liter Essigsäureethylester extrahiert. Die vereinigten organischen Phasen wurden mit gesättigter Natriumchlorid-Lösung gewaschen und über Natriumsulfat getrocknet. Nach Filtration wurde das Lösungsmittel im Vakuum bis zur Trockene entfernt. Das erhaltene Rohprodukt wurde durch Flash-Chromatographie an 5 kg Kieselgel gereinigt (Laufmittel Dichlormethan/Methanol 95:5). Es wurden 75 g (0.35 mol, 97% Reinheit, 51% d. Th.) eines farblosen Feststoffs erhalten.
¹H-NMR (400 MHz, DMSO-d₆, δ/ppm): 12.6-12.35 (1H, breites s), 7.99 (1H, d), 7.67 (1H, d), 7.46 (1H, t), 3.68-3.47 (3H, m), 3.44-3.17 (2H, m).
MS (DCI, NH₃): m/z = 225 (M+NH₄)⁺.

### Beispiel 87A

### Methyl-4-nitro-2,3-dihydro-1H-inden-2-carboxylat

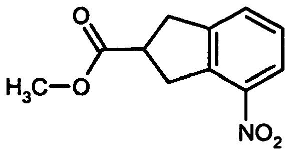

In 538 ml Methanol wurden 52.8 ml (0.72 mol) Thionylchlorid bei -10°C langsam eingetropft und 10 min bei dieser Temperatur nachgerührt. Anschließend wurden 75 g (0.36 mol) 4-Nitro-2,3-di-hydro-1*H*-inden-2-carbonsäure in einer Portion zugegeben und die Mischung drei Stunden bei Raumtemperatur gerührt. Nach Beendigung der Reaktion (DC-Kontrolle: Laufmittel Dichlormethan/Methanol 95:5) wurde das Lösungsmittel im Vakuum abgezogen. Der erhaltene Rückstand wurde in Essigsäureethylester gelöst, einmal mit gesättigter Natriumhydrogencarbonat-Lösung gewaschen und über Natriumsulfat getrocknet. Nach Filtration wurde das Lösungsmittel im Vakuum bis zur Trockene entfernt. Das erhaltene Rohprodukt wurde durch Flash-Chromatographie an 2.2 kg Kieselgel gereinigt (Laufmittel Petrolether/Essigsäureethylester 8:2). Es wurden 75 g (0.34 mol, 94% d. Th.) eines farblosen Feststoffs erhalten.
¹H-NMR (400 MHz, DMSO-d₆, δ/ppm): 7.99 (1H, d), 7.67 (1H, d), 7.47 (1H, t), 3.74-3.43 (3H, m), 3.65 (3H, s), 3.38-3.18 (2H, m).
MS (DCI, NH₃): m/z = 239 (M+NH₄)⁺.

### Beispiel 88A

### rac-Methyl-4-amino-2,3-dihydro-1H-inden-2-carboxylat

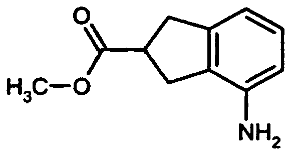

Eine Lösung von 82 g (0.37 mol) Methyl-4-nitro-2,3-dihydro-1*H*-inden-2-carboxylat in 800 ml Essigsäureethylester wurde bei Raumtemperatur mit 3 g Palladium auf Kohle (10%) versetzt und 22 Stunden unter Normaldruck hydriert. Nach vollständiger Umsetzung (DC-Kontrolle: Laufmittel Dichlormethan/Methanol 95:5) wurde die Reaktionslösung über Kieselgur filtriert und das Filtrat im Vakuum eingeengt. Es wurden 67.5 g (0.35 mol, 95% d. Th.) eines farblosen Feststoffs erhalten.
¹H-NMR (400 MHz, DMSO-d₆, δ/ppm): 6.83 (1H, t), 6.39 (2H, t), 4.88 (2H, s), 3.64 (3H, s), 3.38-3.27 (1H, m), 3.12-2.80 (4H, m).
MS (DCI, NH₃): m/z = 209 (M+NH₄)⁺, 192 (M+H)⁺.

### Beispiel 89A und Beispiel 90A

### ent-Methyl-4-amino-2,3-dihydro-1H-inden-2-carboxylat (Enantiomer 1 und 2)

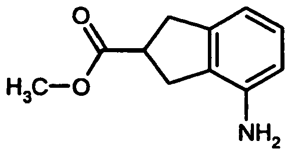

67.5 g (0.35 mmol) racemisches Methyl-4-amino-2,3-dihydro-1*H*-inden-2-carboxylat (Beispiel 88A) wurden mittels präparativer HPLC an chiraler Phase in die Enantiomere aufgetrennt [Säule: Daicel Chiralpak OJ-H, 250 mm x 20 mm; Elutionsmittel: Isohexan/Isopropanol/Methanol 70:18:12 (v/v); Fluss: 25 ml/min; Temperatur: 24°C; UV-Detektion: 260 nm]:

### Beispiel 89A (Enantiomer 1):

Rₜ 11.76 min; Reinheit >99%; >99.5% ee (Säule s.o.)
Ausbeute: 23.1 g
¹H-NMR (400 MHz, DMSO-d₆, δ/ppm): 6.83 (1H, t), 6.39 (2H, t), 4.88 (2H, s), 3.64 (3H, s), 3.38-3.27 (1H, m), 3.12-2.80 (4H, m).
MS (DCI, NH₃): m/z = 209 (M+NH4)⁺, 192 (M+H)⁺.

### Beispiel 90A (Enantiomer 2):

Rₜ 12.65 min; Reinheit >99%; >99% ee (Säule s.o.)
Ausbeute: 30 g.

### Beispiel 91A

### Ethyl-4-methoxy-2-methyl-2,3-dihydro-1H-inden-2-carboxylat

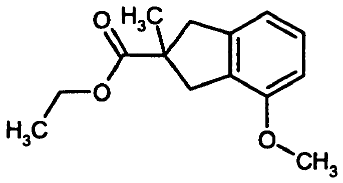

Eine Lösung von 0.95 ml Diisopropylamin (6.75 mmol) in 30 ml THF wurde bei -78°C mit 2.7 ml einer 2.5 M Lösung von n-Butyllithium in Hexan versetzt und anschließend kurz auf -10°C erwärmt. Nach erneutem Abkühlen auf -78°C wurden 1.24 g (5.63 mmol) Ethyl-4-methoxy-2,3-dihydro-1*H*-inden-2-carboxylat [Beilstein Reg.-Nr. 8980172], gelöst in 10 ml THF, zugetropft und die Lösung eine Stunde nachgerührt. Danach wurden 0.46 ml (7.32 mmol) Iodmethan zu der Reaktionslösung getropft und der Ansatz weitere 2 Stunden bei -78°C gerührt. Nach vollständiger Umsetzung wurde die Reaktionsmischung zügig mit gesättigter wässriger Ammoniumchlorid-Lösung versetzt. Nach Trennung der Phasen wurde die wässrige Phase dreimal mit Essigsäureethylester extrahiert und die vereinigten organischen Phasen über Magnesiumsulfat getrocknet. Nach Filtration wurde das Lösungsmittel im Vakuum bis zur Trockene entfernt. Das erhaltene Rohprodukt wurde durch Flash-Chromatographie an Kieselgel gereinigt (Laufmittel Cyclohexan/Essigsäureethylester 10:1). Es wurden 1.19 g (5.08 mmol, 90% d. Th.) eines farblosen Feststoffs erhalten.
LC-MS (Methode 7): Rₜ = 2.60 min; m/z = 235 (M+H)⁺.

### Beispiel 92A

### Methyl-4-hydroxy-2-methyl-2,3-dihydro-1H-inden-2-carboxylat

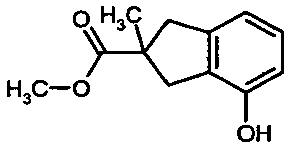

Eine Lösung von 1.19 g (5.08 mmol) Ethyl-4-methoxy-2-methyl-2,3-dihydro-1*H*-inden-2-carboxylat in 20 ml Dichlormethan wurde bei 0°C mit 15.2 ml (15.24 mmol) einer 1 M Lösung von Bortribromid in Dichlormethan versetzt. Man ließ eine Stunde bei 0°C weiter rühren. Dann wurden 16 ml Methanol zugefügt und der Ansatz über Nacht nachgerührt. Anschließend wurden 10 ml Wasser und 10 ml Dichlormethan zugegeben. Nach Trennung der Phasen wurde die wässrige Phase dreimal mit Dichlormethan extrahiert und die vereinigten organischen Phasen über Magnesiumsulfat getrocknet. Nach Filtration wurde das Lösungsmittel im Vakuum bis zur Trockene eingeengt. Es wurden 897 mg (4.35 mmol, 86% d. Th.) eines farblosen Öls erhalten.
LC-MS (Methode 12): Rₜ = 1.83 min; m/z = 207 (M+H)⁺.

### Beispiel 93A

### Methyl-2-methyl-4-{[(trifluormethyl)sulfonyl]oxy}-2,3-dihydro-1H-inden-2-carboxylat

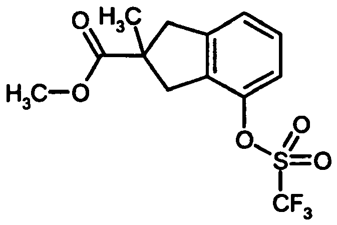

Zu einer Lösung von 897 mg (4.35 mmol) Methyl-4-hydroxy-2-methyl-2,3-dihydro-1*H*-inden-2-carboxylat in 20 ml Pyridin wurden bei -15°C 1.5 ml (8.7 mmol) Trifluormethansulfonsäureanhydrid langsam zugetropft. Nach Erwärmen auf Raumtemperatur wurde der Ansatz 2 h nachgerührt. Danach wurden 20 ml Wasser zugegeben, und nach Trennung der Phasen wurde die wässrige Phase dreimal mit Essigsäureethylester extrahiert. Die vereinigten organischen Phasen wurden mit gesättigter Natriumchlorid-Lösung gewaschen und über Natriumsulfat getrocknet. Nach Filtration wurde das Lösungsmittel im Vakuum bis zur Trockene entfernt. Das erhaltene Rohprodukt wurde durch Flash-Chromatographie an Kieselgel gereinigt (Laufmittel Cyclohexan/Essigsäureethylester 10:1). Es wurden 1100 mg (3.25 mmol, 75% d. Th.) eines farblosen Öls erhalten.
LC-MS (Methode 7): Rₜ = 2.76 min; m/z = 339 (M+H)⁺.

### Beispiel 94A

### Methyl-4-(benzylamino)-2-methyl-2,3-dihydro-1H-inden-2-carboxylat

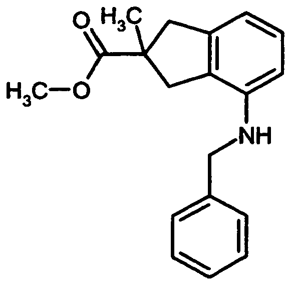

Unter Argon wurde eine Lösung von 1 g (2.96 mmol) Methyl-2-methyl-4-{[(trifluormethyl)sulfonyl]oxy}-2,3-dihydro-1*H*-inden-2-carboxylat, 290 µl (2.66 mmol) Benzylamin, 2.41 g (7.29 mmol) Cäsiumcarbonat, 56 mg (0.12 mmol) 2-Dicyclohexylphosphino-2',4',6'-triisopropylbiphenyl und 54 mg (0.059 mmol) Tris(dibenzylidenaceton)dipalladium in 50 ml Dioxan über Nacht bei 100°C Badtemperatur gerührt. Nach Abkühlen auf Raumtemperatur wurde das Reaktionsgemisch über Kieselgur filtriert und der Rückstand mehrmals mit Dioxan nachgewaschen. Die vereinigten Filtrate wurden im Vakuum bis zur Trockene eingeengt. Das erhaltene Rohprodukt wurde durch Flash-Chromatographie an Kieselgel gereinigt (Laufmittel Cyclohexan/Essigsäureethylester 20:1 → 4:1 → 1:1). Es wurden 769 mg (2.42 mmol, 93% Gehalt, 82% d. Th.) eines farblosen Feststoffs erhalten.
LC-MS (Methode 7): Rₜ = 2.77 min; m/z = 296 (M+H)⁺.

### Beispiel 95A

### Methyl-4-amino-2-methyl-2,3-dihydro-1H-inden-2-carboxylat

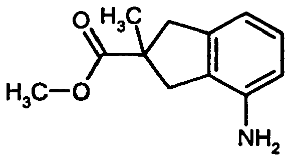

Eine Lösung von 860 mg (2.91 mmol) Methyl-4-(benzylamino)-2-methyl-2,3-dihydro-1*H*-inden-2-carboxylat in 84 ml Methanol wurde bei Raumtemperatur mit 50 mg Palladium auf Kohle (10%) versetzt und über Nacht unter Normaldruck hydriert. Nach vollständiger Umsetzung wurde die Reaktionslösung über Kieselgur filtriert und das Filtrat im Vakuum eingeengt. Das erhaltene Rohprodukt wurde durch Flash-Chromatographie an Kieselgel gereinigt (Laufmittel Cyclohexan/ Essigsäureethylester 10:1 → 1:1). Es wurden 483 mg (2.35 mol, 81% d. Th.) eines farblosen Feststoffs erhalten.
LC-MS (Methode 11): Rₜ = 0.81 min; m/z = 206 (M+H)⁺.

### Beispiel 96A

### tert.-Butyl-3-(3-amino-2-methylphenyl)propanoat

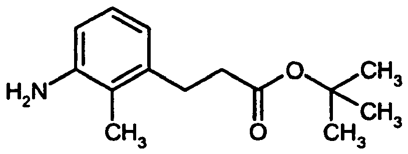

Unter Argon wurden 201 ml (1.39 mol) *tert*.-Butyl-prop-2-enoat zu einer Lösung von 100 g (463 mmol) 1-Brom-2-methyl-3-nitrobenzol, 322 ml (2.31 mol) Triethylamin, 28.18 g (92.58 mmol) Tri-2-tolylphosphin und 10.39 g (46.29 mmol) Palladium(II)acetat in 2 Liter DMF getropft und die Mischung anschließend 36 h bei 125°C gerührt. Nach Abkühlen auf Raumtemperatur wurde das Reaktionsgemisch mit gesättigter wässriger Ammoniumchlorid-Lösung verrührt und die organische Phase abgetrennt. Die wässrige Phase wurde dreimal mit **tert**.-Butylmethylether extrahiert, und die vereinigten organischen Phasen wurden mit gesättigter Natriumchlorid-Lösung gewaschen und über Natriumsulfat getrocknet. Nach Filtration wurde das Lösungsmittel im Vakuum bis zur Trockene entfernt. Der erhaltene Rückstand wurde durch Flash-Chromatographie an Kieselgel gereinigt (Laufmittel Petrolether/Essigsäureethylester 9:1). Es wurden 89 g (338 mmol, 73% d. Th.) des Zwischenprodukts *tert*.-Butyl-(2*E*)-3-(2-methyl-3-nitrophenyl)prop-2-enoat als farbloser Feststoff erhalten. 88 g (334 mmol) dieses Feststoffs wurden in 2 Liter Ethanol gelöst, bei Raumtemperatur mit 7 g Palladium auf Kohle (10%) versetzt und 18 h unter Normaldruck hydriert. Nach vollständiger Umsetzung wurde die Reaktionslösung über Kieselgur filtriert und das erhaltene Filtrat im Vakuum eingeengt. Es wurden 61.3 g (260.5 mmol, 78% d. Th.) der Titelverbindung als farbloser Feststoff erhalten.
¹H-NMR (400 MHz, DMSO-d₆, δ/ppm): 6.77 (1H, t), 6.47 (1H, d), 6.36 (1H, d), 4.72 (2H, s), 2.14 (2H, t), 2.36 (2H, t), 1.95 (3H, s), 1.39 (9H, s).
LC-MS (Methode 7): Rₜ = 1.84 min; m/z = 236 (M+H)⁺.

### Beispiel 97A

### Ethyl-3-(3-amino-2-methylphenyl)propanoat

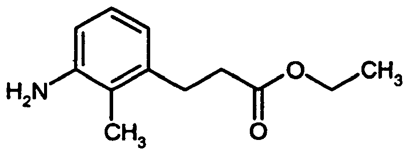

Unter Argon wurden 10.844 g (108 mmol) Ethyl-prop-2-enoat zu einer Lösung von 7.8 g (36.1 mmol) 1-Brom-2-methyl-3-nitrobenzol, 25 ml (180.5 mmol) Triethylamin, 2.197 g (7.22 mmol) Tri-2-tolylphosphin und 810 mg (3.6 mmol) Palladium(II)acetat in 200 ml DMF getropft und die Mischung anschließend 36 h bei 125°C gerührt. Nach Abkühlen auf Raumtemperatur wurde das Reaktionsgemisch mit gesättigter wässriger Ammoniumchlorid-Lösung verrührt und die organische Phase abgetrennt. Die wässrige Phase wurde dreimal mit *tert*.-Butyl-methylether extrahiert, und die vereinigten organischen Phasen wurden mit gesättigter Natriumchlorid-Lösung gewaschen und über Natriumsulfat getrocknet. Nach Filtration wurde das Lösungsmittel im Vakuum bis zur Trockene entfernt. Der erhaltene Rückstand wurde durch Flash-Chromatographie an Kieselgel gereinigt (Laufmittel Petrolether/Essigsäureethylester 3:1). Es wurden 6.6 g (27.2 mmol, Gehalt 97%, 75% d. Th.) des Zwischenprodukts Ethyl-(2*E*)-3-(2-methyl-3-nitrophenyl)prop-2-enoat als farbloser Feststoff erhalten. 6.6 g (27.2 mmol, Gehalt 97%) dieses Feststoffs wurden in 200 ml Ethanol gelöst, bei Raumtemperatur mit 500 mg Palladium auf Kohle (10%) versetzt und über Nacht unter Normaldruck hydriert. Nach vollständiger Umsetzung wurde die Reaktionslösung über Kieselgur filtriert und das erhaltene Filtrat im Vakuum eingeengt. Es wurden 5.47 g (26.38 mmol, Gehalt 97%, 97% d. Th.) der Titelverbindung als farbloser Feststoff erhalten.
LC-MS (Methode 10): Rₜ = 1.07 min; m/z = 208 (M+H)⁺.

### Beispiel 98A

### Methyl-3-(trans-4-aminocyclohexyl)propanoat-Hydrochlorid

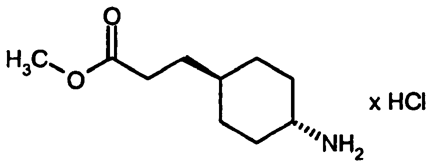

In 100 ml Methanol wurden 2.9 ml (40.5 mmol) Thionylchlorid bei -5°C langsam eingetropft und 10 min bei dieser Temperatur nachgerührt. Anschließend wurden 5 g (18.4 mmol) 3-{*trans*-4-[(*tert*.-Butoxycarbonyl)amino]cyclohexyl}propansäure in einer Portion zugegeben und die Reaktionsmischung über Nacht bei Raumtemperatur gerührt. Nach vollständiger Umsetzung wurde das Lösungsmittel im Vakuum eingedampft. Es wurden 3.97 g (17.9 mmol, 97% d. Th.) eines farblosen Feststoffs erhalten.
¹H-NMR (400 MHz, DMSO-d₆, δ/ppm): 8.18 (3H, breites s), 3.59 (3H, s), 2.96-2.79 (1H, m), 2.31 (2H, t), 2.03-1.89 (2H, m), 1.81-1.68 (2H, m), 1.49-1.21 (4H, m), 1.21-1.08 (1H, m), 1.00-0.85 (2H, m).
MS (ES): m/z = 186 (M+H-HCl)⁺.

### Beispiel 99A

### Methyl-3-(cis-4-aminocyclohexyl)propanoat-Hydrochlorid

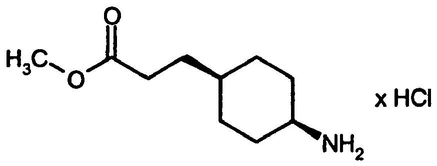

In 100 ml Methanol wurden 2.9 ml (40.5 mmol) Thionylchlorid bei -5°C langsam eingetropft und 10 min bei dieser Temperatur nachgerührt. Anschließend wurden 5 g (18.4 mmol) 3-{cis-4-[(*tert.-*Butoxycarbonyl)amino]cyclohexyl}propansäure in einer Portion zugegeben und die Reaktionsmischung über Nacht bei Raumtemperatur gerührt. Nach vollständiger Umsetzung wurde das Lösungsmittel im Vakuum eingedampft. Es wurden 3.95 g (17.8 mmol, 96.7% d. Th.) eines farblosen Feststoffs erhalten.
¹H-NMR (400 MHz, DMSO-d₆, δ/ppm): 8.05 (3H, breites s), 3.59 (3H, s), 3.21-3.05 (1H, m), 2.39-2.24 (2H, m), 1.74-1.31 (11H, m).
MS (ES): m/z = 186 (M+H-HCl)⁺.

### Beispiel 100A

### Methyl-3-(3-aminocyclohexyl)propanoat-Hydrochlorid

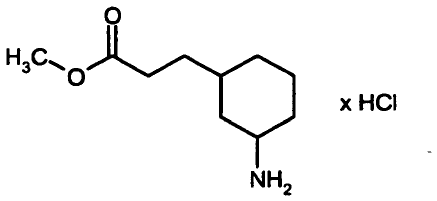

In 50 ml Methanol wurden 1.9 ml (25.7 mmol) Thionylchlorid bei -5°C langsam eingetropft und 10 min bei dieser Temperatur nachgerührt. Anschließend wurden 2 g (11.7 mmol) 3-(3-Aminocyclohexyl)propansäure in einer Portion zugegeben und die Reaktionsmischung über Nacht bei Raumtemperatur gerührt. Nach vollständiger Umsetzung wurde das Lösungsmittel im Vakuum eingedampft. Es wurden 2.46 g (11.1 mmol, 95% d. Th.) eines farblosen Feststoffs erhalten.
¹H-NMR (400 MHz, DMSO-d₆, δ/ppm): 8.05 (3H, breites s), 3.59 (3H, s), 3.01-2.84 (1H, m), 2.39-2.22 (2H, m), 2.01-1.82 (1H, m), 1.80-1.59 (2H, m), 1.59-1.40 (3H, m), 1.40-1.08 (3H, m), 1.01-0.85 (1H, m), 0.85-0.65 (1H, m).
GC-MS (Methode 3): Rₜ = 4.75 min; m/z = 185 (M-HCl)⁺.

### Beispiel 101A

### Ethyl-5-(aminomethyl)isoxazol-3-carboxylat-Hydrochlorid

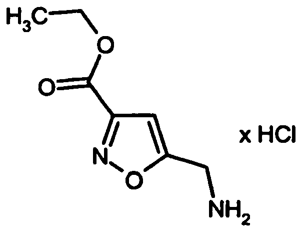

360 mg (1.3 mmol) Ethyl-5-{[(*tert*.-butoxycarbonyl)amino]methyl}isoxazol-3-carboxylat wurden über Nacht in 3.3 ml einer 4 M Lösung von Chlorwasserstoff in Dioxan bei Raumtemperatur gerührt. Nach Beendigung der Reaktion wurde das Lösungsmittel im Vakuum eingedampft. Es wurden 258 mg (1.25 mmol, 94% d. Th.) eines gelblichen Feststoffs erhalten.
LC-MS (Methode 12): Rₜ = 0.49 min; m/z = 171 (M+H-HCl)⁺.

### Beispiel 102A

### Methyl-2-[(3-aminophenyl)sulfanyl]-2-methylpropanoat

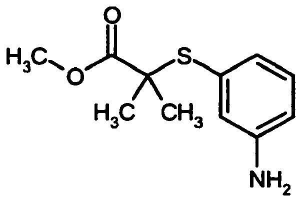

Eine Lösung von 1.2 g (9.6 mmol) 3-Aminobenzolthiol und 1.046 g (12.5 mmol) Natriumhydrogencarbonat in 6 ml DMF wurde bei Raumtemperatur langsam mit 1.36 ml (10.5 mmol) Methyl-2-brom-2-methylpropanoat versetzt und über Nacht gerührt. Nach Beendigung der Reaktion wurde das Reaktionsgemisch auf Wasser gegeben. Der Ansatz wurde dreimal mit Diethylether extrahiert, und die vereinigten organischen Phasen wurden mit gesättigter Natriumchlorid-Lösung gewaschen und über Magnesiumsulfat getrocknet. Nach Filtration wurde das Lösungsmittel im Vakuum bis zur Trockene entfernt. Es wurden 1.45 g (6.4 mmol, 67% d. Th.) eines farblosen Feststoffs erhalten.
LC-MS (Methode 11): Rₜ = 0.96 min; m/z = 226 (M+H)⁺.

### Beispiel 103A

### Methyl-2-methyl-2-(2-methyl-3-nitrophenoxy)propanoat

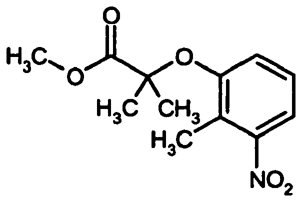

2.00 g (13.06 mmol) 2-Methyl-3-nitrophenol wurden in 40 ml DMF gelöst und mit 3.55 g (19.59 mmol) Methyl-2-brom-2-methylpropanoat sowie 4.68 g (14.37 mmol) Cäsiumcarbonat versetzt. Das Reaktionsgemisch wurde 2 d bei 100°C gerührt und dann mit weiteren 1.77 g (9.80 mmol) Methyl-2-brom-2-methylpropanoat versetzt. Es wurde erneut 1 d bei 100°C gerührt, danach weitere 1.77 g (9.80 mmol) Methyl-2-brom-2-methylpropanoat zugegeben und nochmals 1 d bei 100°C gerührt. Nach dem Abkühlen wurde gesättigte wässrige Natriumhydrogencarbonat-Lösung zugesetzt und die Mischung mehrfach mit Essigsäureethylester extrahiert. Die vereinigten organischen Phasen wurden mit gesättigter wässriger Natriumhydrogencarbonat-Lösung gewaschen, über Natriumsulfat getrocknet und am Rotationsverdampfer eingeengt. Der Rückstand wurde mittels präparativer HPLC gereinigt. Es wurden 1.33 g (39% d. Th.) der Zielverbindung erhalten.
¹H-NMR (300 MHz, CDCl₃, δ/ppm): 1.58 (s, 6H), 2.27 (s, 3H), 3.75 (s, 3H), 6.95 (d, 1H), 7.35 (t, 1H), 7.60 (d, 1H).

### Beispiel 104A

### Methyl-2-(3-amino-2-methylphenoxy)-2-methylpropanoat-Hydrochlorid

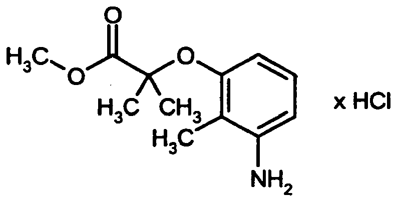

1.33 g (5.25 mmol) Methyl-2-methyl-2-(2-methyl-3-nitrophenoxy)propanoat wurden in 60 ml Ethanol gelöst und mit 1 ml konzentrierter Salzsäure sowie 0.30 g Palladium auf Kohle (10%) versetzt. Unter einer Wasserstoffatmosphäre wurde 3 h bei Normaldruck und Raumtemperatur hydriert. Die Reaktionsmischung wurde danach über Tonsil filtriert. Es wurde mit Ethanol nachgewaschen und die gesammelten Filtrate im Vakuum eingeengt. Es wurden 1.39 g (89% d. Th.) der Zielverbindung erhalten.
LC-MS (Methode 11): Rₜ = 0.80 min; m/z = 225 (M-Cl+H)⁺.

### Beispiel 105

### Methyl-4-[(cyclopentyl{4-[(5-oxo-2-phenyl-5,6-dihydro-4H-1,3,4-oxadiazin-4-yl)methyl]phenyl}-acetyl)amino]-2,3-dihydro-1H-inden-2-carboxylat

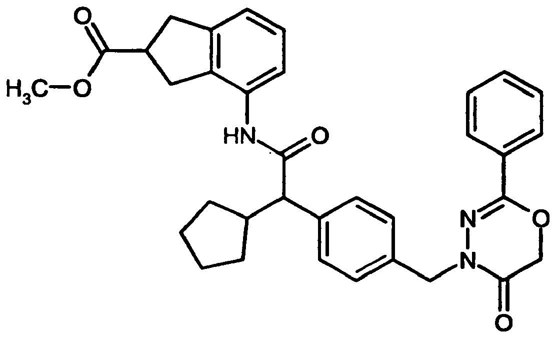

337 mg (0.821 mmol) Cyclopentyl{4-[(5-oxo-2-phenyl-5,6-dihydro-4*H*-1,3,4-oxadiazin-4-yl)-methyl]phenyl}acetylchlorid (Enantiomer 1; Beispiel 81A) wurden in 8 ml Dichlormethan gelöst und mit 83 mg (0.821 mmol) Triethylamin versetzt. Zum Gemisch wurden 157 mg (0.821 mmol) Methyl-4-amino-2,3-dihydro-1*H*-inden-2-carboxylat (Enantiomer 1; Beispiel 89A), gelöst in 2 ml Dichlormethan, hinzugefügt. Es wurde über Nacht bei Raumtemperatur gerührt. Die Reaktionslösung wurde danach mit Wasser versetzt und mit Dichlormethan extrahiert. Die vereinigten organischen Phasen wurden über Natriumsulfat getrocknet und am Rotationsverdampfer eingeengt. Der Rückstand wurde durch Chromatographie an Kieselgel gereinigt (Laufmittel Cyclohexan/ Essigsäureethylester 3:1 → 1:1). Es wurden 182 mg (0.32 mmol, 39% d. Th.) der Titelverbindung erhalten.
LC-MS (Methode 10): Rₜ = 2.50 min; m/z = 566 (M+H)⁺.

Auf analoge Weise wurden die in der folgenden Tabelle aufgeführten Verbindungen erhalten:

| **Beispiel** | **Name / Struktur / Edukte** | **Analytische Daten** |
|---|---|---|
| **106A** | Methyl-4-{[cyclopentyl(4-{[4-methyl-5-(2-methylpropyl)-2-oxo-1,3-thiazol-3(2*H*)-yl]methyl}phenyl)acetyl]amino}-2,3-dihydro-1*H*-inden-2-carboxylat | LC-MS (Methode 10): Rₜ = 2.68 min; m/z = 561 (M+H)⁺. |
| | 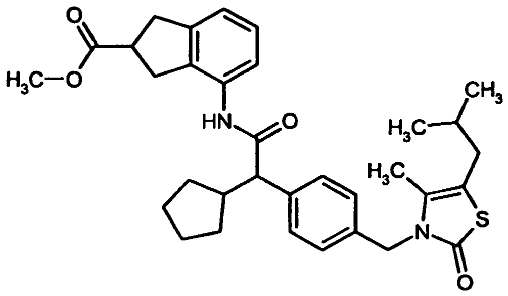 | |
| | (aus Bsp. 84A und Bsp. 88A) | |
| **107A** | Methyl-4-{[cyclopentyl(4-{[5-oxo-2-(propan-2-yl)-5,6-dihydro-4*H*-1,3,4-oxadiazin-4-yl]methyl}phenyl)-acetyl]amino}-2,3-dihydro-1*H*-inden-2-carboxylat | LC-MS (Methode 10): Rₜ = 2.42 und 2.47 min; m/z = 532 (M+H)⁺ (*Isomer 1 und 2*). |
| | 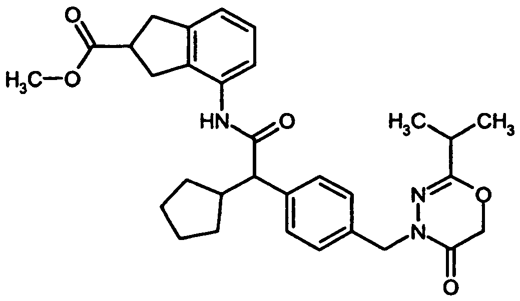 | |
| | (aus Bsp. 82A und Bsp. 89A) | |
| **108A** | Methyl-4-{[{4-[(2-*tert*.-butyl-5-oxo-5,6-dihydro-4*H*-1,3,4-oxadiazin-4-yl)methyl]phenyl}(cyclopentyl)acetyl]amino}-2,3-dihydro-1*H*-inden-2-carboxylat | LC-MS (Methode 11): Rₜ = 1.53 min; m/z = 546 (M+H)⁺. |
| | 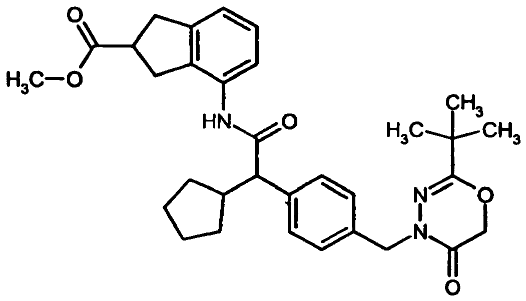 | |
| | (aus Bsp. 83A und Bsp. 89A) | |
| **109A** | Methyl-(2*E*)-3-{2-[(cyclopentyl{4-[(5-oxo-2-phenyl-5,6-dihydro-4*H*-1,3,4-oxadiazin-4-yl)methyl]phenyl}acetyl)-amino]phenyl}prop-2-enoat | LC-MS (Methode 10): Rₜ = 2.41 min; m/z = 552 (M+H)⁺. |
| | 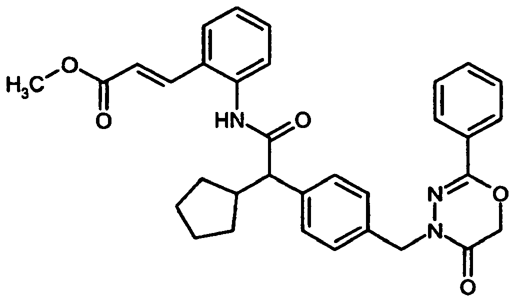 | |
| | (aus Bsp. 81A und Methyl-(2*E*)-3-(2-aminophenyl)-prop-2-enoat [CAS Reg.-Nr. 1664-62-6]) | |
| **110A** | Methyl-{3-[(cyclopentyl{4-[(5-oxo-2-phenyl-5,6-dihydro-4*H*-1,3,4-oxadiazin-4-yl)methyl]phenyl}acetyl)amino]-phenyl}acetat | LC-MS (Methode 10): Rₜ = 2.43 min; m/z = 540 (M+H)⁺. |
| | 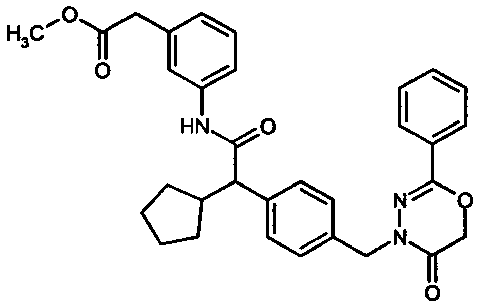 | |
| | (aus Bsp. 81A und Methyl-(3-aminophenyl)acetat-Hydrochlorid [CAS Reg.-Nr. 150319-83-8]) | |
| **111A** | Ethyl-(2*E*)-3-{4-[(cyclopentyl{4-[(5-oxo-2-phenyl-5,6-dihydro-4*H*-1,3,4-oxadiazin-4-yl)methyl]phenyl}acetyl)-amino]phenyl}prop-2-enoat | LC-MS (Methode 12): Rₜ = 2.89 min; m/z = 566 (M+H)⁺. |
| | 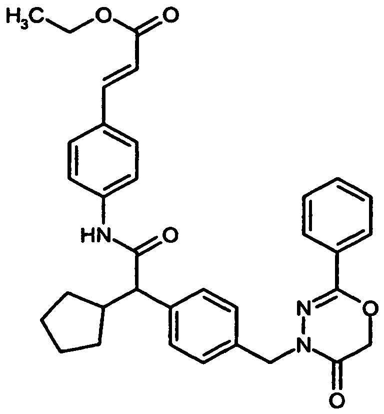 | |
| | (aus Bsp. 81 A und Ethyl-(2E)-3-(4-aminophenyl)-prop-2-enoat [CAS Reg.-Nr. 5048-82-8]) | |

### Beispiel 112A

### Methyl-4-({[(4-{[4-(4-chlorphenyl)-1-oxophthalazin-2(1H)-yl]methyl}phenyl)(cyclopentyl)-acetyl]amino}methyl)benzolcarboxylat

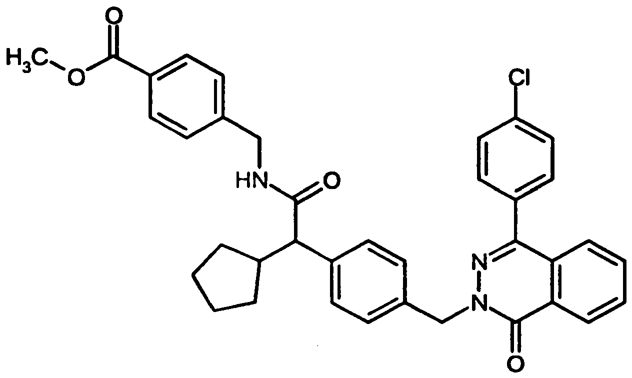

60 mg (0.127 mmol) (4-{[4-(4-Chlorphenyl)-1-oxophthalazin-2(1*H*)-yl]methyl}phenyl)(cyclopentyl)essigsäure (Beispiel 65A) wurden in 0.6 ml Dichlormethan gelöst und nacheinander mit 26 mg (0.127 mmol) Methyl-4-(aminomethyl)benzolcarboxylat-Hydrochlorid, 19 mg (0.140 mmol) 1-Hydroxy-1*H*-benzotriazol-Hydrat, 28 mg (0.146 mmol) 1-(3-Dimethylaminopropyl)-3-ethyl-carbodiimid-Hydrochlorid sowie 28 mg (0.273 mmol) Triethylamin versetzt. Das Reaktionsgemisch wurde über Nacht bei Raumtemperatur gerührt. Danach wurde 1 N Salzsäure zugesetzt und das Gemisch mit Essigsäureethylester extrahiert. Die vereinigten organischen Phasen wurden über Natriumsulfat getrocknet und am Rotationsverdampfer eingeengt. Der Rückstand wurde über präparative HPLC gereinigt. Es wurden 36 mg (46% d. Th.) der Titelverbindung erhalten.
LC-MS (Methode 7): Rₜ = 3.27 min; m/z = 620 (M+H)⁺.

Auf analoge Weise wurden die in der folgenden Tabelle aufgeführten Verbindungen erhalten:

| **Beispiel** | **Name / Struktur / Edukte** | **Analytische Daten** |
|---|---|---|
| **113A** | Methyl-4-{[(cyclopentyl{4-[(5-oxo-2-phenyl-5,6-dihydro-4*H*-1,3,4-oxadiazin-4-yl)methyl]phenyl}acetyl)amino]-methyl}benzolcarboxylat | LC-MS (Methode 2): Rₜ = 2.84 min; m/z = 540 (M+H)⁺. |
| | 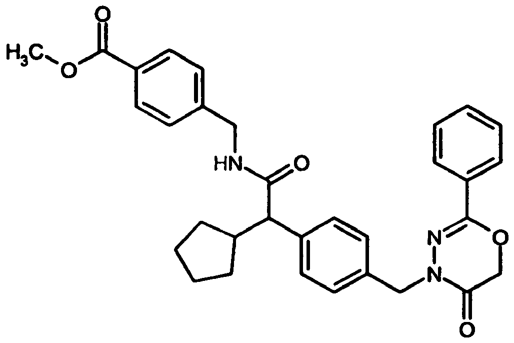 | |
| | (aus Bsp. 51A und Methyl-4-(aminomethyl)-benzolcarboxylat-Hydrochlorid) | |
| **114A** | Methyl-4-{[(cyclopentyl{4-[(2-oxo-5-phenyl-1,3,4-oxadiazol-3(2*H*)-yl)methyl]phenyl}acetyl)amino]-methyl}benzolcarboxylat | LC-MS (Methode 2): Rₜ = 2.71 min; m/z = 526 (M+H)⁺. |
| | 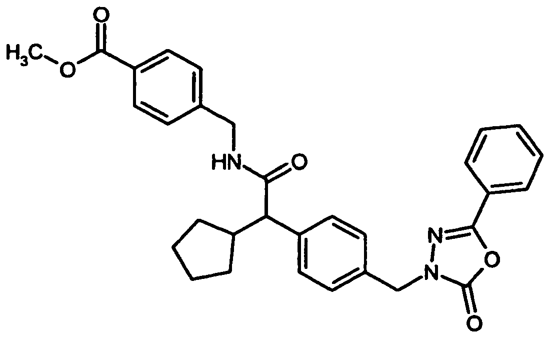 | |
| | (aus Bsp. 57A und Methyl-4-(aminomethyl)-benzolcarboxylat-Hydrochlorid) | |
| **115A** | Methyl-4-({[cyclopentyl(4-{[(4*R*,5*S*)-4-methyl-2-oxo-5-phenyl-1,3-oxazolidin-3-yl]methyl}phenyl)acetyl]amino}-methyl)benzolcarboxylat | LC-MS (Methode 2): Rₜ = 2.76 min; m/z = 541 (M+H)⁺. |
| | 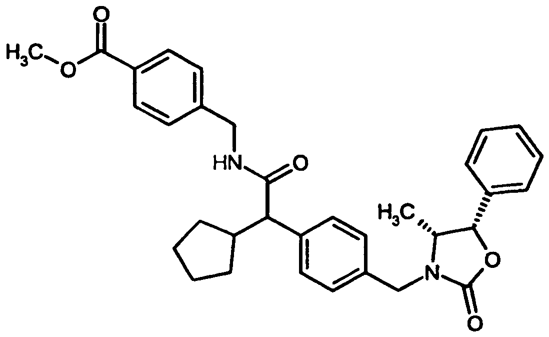 | |
| | (aus Bsp. 69A und Methyl-4-(aminomethyl)-benzolcarboxylat-Hydrochlorid) | |
| **116A** | Methyl-4-({[cyclopentyl(4-{[(4*S*,5*R*)-4-methyl-2-oxo-5-phenyl-1,3-oxazolidin-3-yl]methyl}phenyl)acetyl]amino}-methyl)benzolcarboxylat | LC-MS (Methode 2): Rₜ = 2.76 min; m/z = 541 (M+H)⁺. |
| | 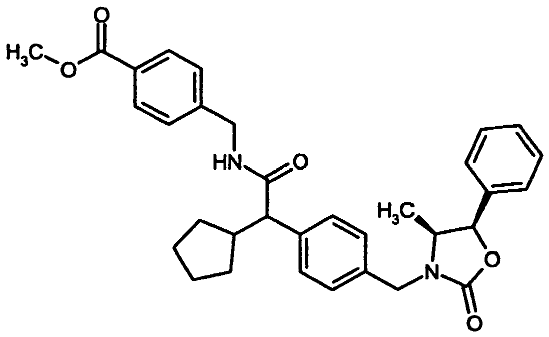 | |
| | (aus Bsp. 68A und Methyl-4-aminomethyl)-benzolcarboxylat-Hydrochlorid) | |
| **117A** | Methyl-4-{[(cyclopentyl{4-[(3,5-dioxo-6-phenyl-2,5-dihydro-1,2,4-triazin-4(3*H*)-yl)methyl]phenyl}acetyl)-amino]methyl}benzolcarboxylat | LC-MS (Methode 2): Rₜ = 2.67 min; m/z = 553 (M+H)⁺. |
| | 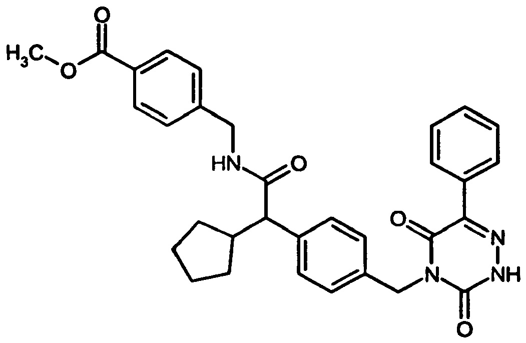 | |
| | (aus Bsp. 55A und Methyl-4-(aminomethyl)-benzolcarboxylat-Hydrochlorid) | |
| **118A** | Methyl-4-{[(cyclopentyl{4-[(5-methyl-2-oxo-5-phenyl-piperidin-1-yl)methyl]phenyl}acetyl)amino]methyl}-benzolcarboxylat | LC-MS (Methode 2): Rₜ = 2.69 min; m/z = 553 (M+H)⁺. |
| | 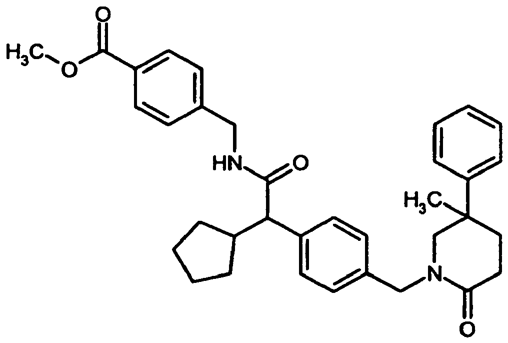 | |
| | (aus Bsp. 70A und Methyl-4-(aminomethyl)-benzolcarboxylat-Hydrochlorid) | |
| **119A** | Methyl-(4-{[(cyclopentyl{4-[(5-oxo-2-phenyl-5,6-dihydro-4*H*-1,3,4-oxadiazin-4-yl)methyl]phenyl}acetyl)amino]-methyl}phenyl)acetat | LC-MS (Methode 2): Rₜ = 2.77 min; m/z = 554 (M+H)⁺. |
| | 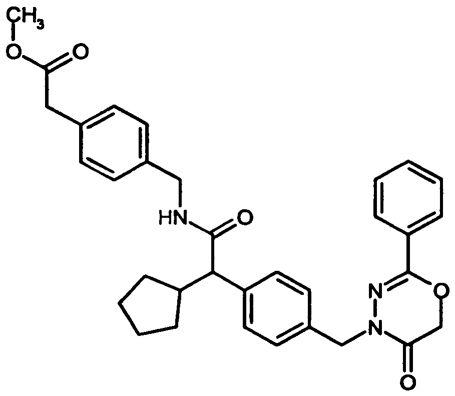 | |
| | (aus Bsp. 51A und Methyl-[4-(aminomethyl)phenyl]acetat-Hydrochlorid [CAS Reg.-Nr. 99075-26-9]) | |
| **120A** | Methyl-4-({[(4-{[2-(3-chlorphenyl)-5-oxo-5,6-dihydro-4*H*-1,3,4-oxadiazin-4-yl]methyl}phenyl)cyclopentyl)acetyl]-amino}methyl)benzolcarboxylat | LC-MS (Methode 2): Rₜ = 2.87 min; m/z = 574 (M+H)⁺. |
| | 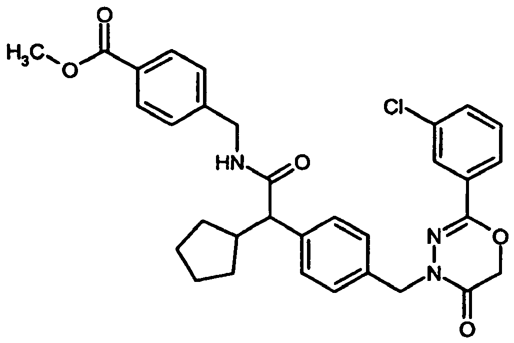 | |
| | (aus Bsp. 52A und Methyl-4-(aminomethyl)-benzolcarboxylat-Hydrochlorid) | |
| **121A** | Methyl-6-({[(4-{([4-(4-chlorphenyl)-1-oxophthalazin-2(1*H*)-yl]methyl}phenyl)(cyclopentyl)acetyl]amino}-methyl)pyridin-3-carboxylat | LC-MS (Methode 2): Rₜ = 2.97 min; m/z = 621 (M+H)⁺. |
| | 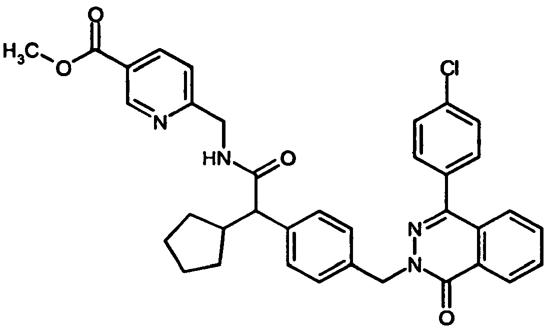 | |
| | (aus Bsp. 65A und Methyl-6-(aminomethyl)pyridin-3-carboxylat-Hydrochlorid [Beilstein Reg.-Nr. 7701946]) | |
| **122A** | Methyl-4({[(4-{[2-(2-chlorphenyl)-5-oxo-5,6-dihydro-4*H*-1,3,4-oxadiazin-4-yl]methyl}phenyl)(cyclopentyl)acetyl]-amino}methyl)benzolcarboxylat | LC-MS (Methode 7): Rₜ = 2.95 min; m/z = 574 (M+H)⁺. |
| | 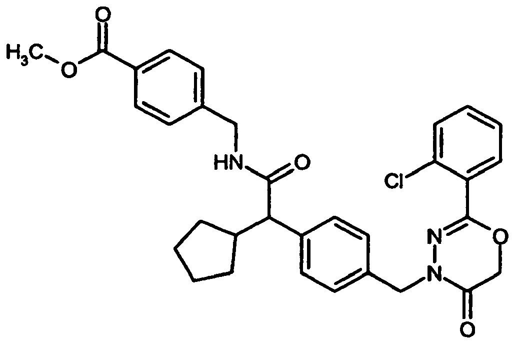 | |
| | (aus Bsp. 53A und Methyl-4-(aminomethyl)-benzolcarboxylat-Hydrochlorid) | |
| **123A** | Ethyl-5-({[(4-{[4-(4-chlorphenyl)-1-oxophthalazin-2(1*H*)-yl]methyl} phenyl)(cyclopentyl)acetyl]amino} methyl)-furan-2-carboxylat | LC-MS (Methode 9): Rₜ = 4.74 min; m/z = 624 (M+H)⁺. |
| | 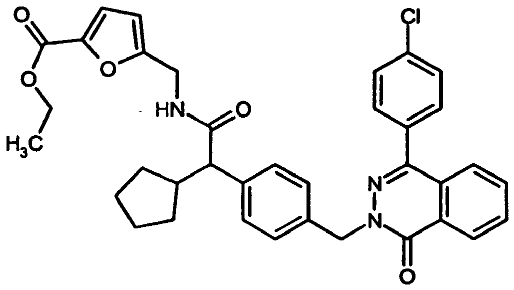 | |
| | (aus Bsp. 65A und Ethyl-5-(aminomethyl)furan-2-carboxylat [CAS Reg.-Nr. 18707-63-6]) | |
| **124A** | Methyl-4-({[(4-{[2-(4-chlorphenyl)-5-oxo-5,6-dihydro-4*H* 1,3,4-oxadiazin-4-yl]methyl}phenyl)(cyclopentyl)acetyl]-amino}methyl)benzolcarboxylat | LC-MS (Methode 9): Rₜ=4.29 min; m/z = 574 (M+H)⁺. |
| | 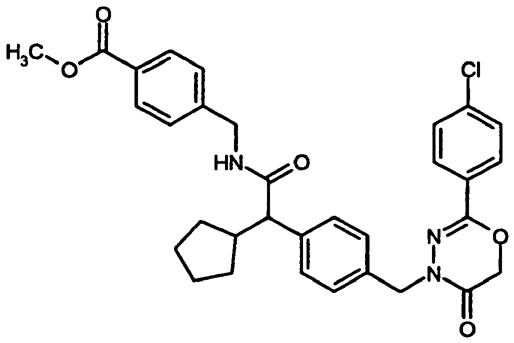 | |
| | (aus Bsp. 72A und Methyl-4-(aminomethyl)-benzolcarboxylat-Hydrochlorid) | |
| **125A** | Methyl-4-({[cyclopentyl(4-{([2-(2-methoxyphenyl)-5-oxo-5,6-dihydro-4*H*-1,3,4-oxadiazin-4-yl]methyl}phenyl)-acetyl]amino}methyl)benzolcarboxylat | LC-MS (Methode 7): Rₜ = 2.78 min; m/z = 570 (M+H)⁺. |
| | 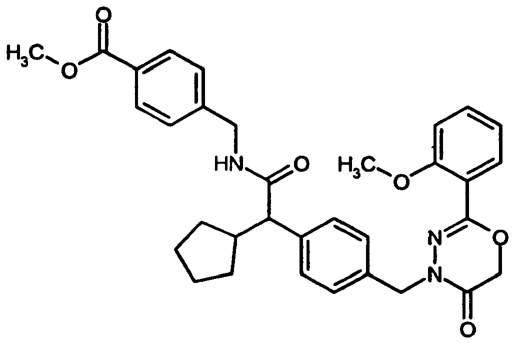 | |
| | (aus Bsp. 54A und Methyl-4-(aminomethyl)-benzolcarboxylat-Hydrochlorid) | |
| **126A** | Ethyl-4-{[(cyclopentyl{4-[(5-oxo-2-phenyl-5,6-dihydro-4*H*-1,3,4-oxadiazin-4-yl)methyl]phenyl}acetyl)amino]-methyl}cyclohexancarboxylat | LC-MS (Methode 11): Rₜ = 1.49 min; m/z = 560 (M+H)⁺. |
| | 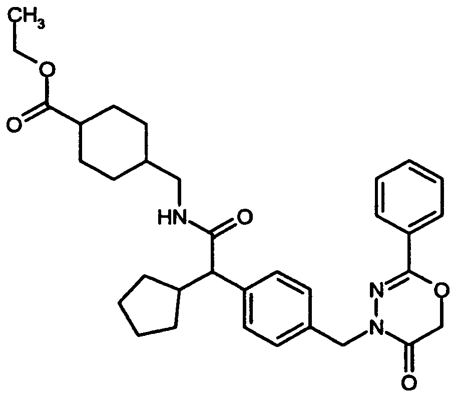 | |
| | (aus Bsp. 51A und Ethyl-4-(aminomethyl)-cyclohexancarboxylat-Hydrochlorid (*cis*/*trans*-Gemisch) [Beilstein Reg.-Nr. 10814930]) | |

### Beispiele 127A -130A

### Ethyl-4-{[(cyclopentyl{4-[(5-oxo-2-phenyl-5,6-dihydro-4H-1,3,4-oxadiazin-4-yl)methyl]phenyl}-acetyl)amino]methyl}cyclohexancarboxylat (Isomer 1 - 4)

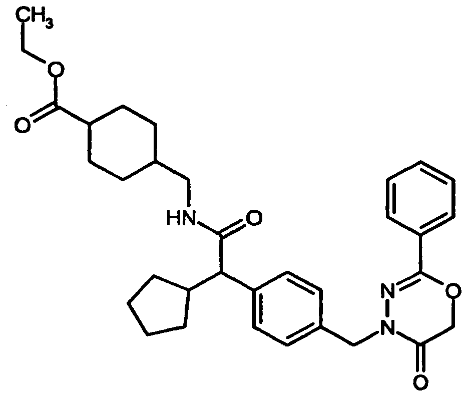

297.5 mg der Verbindung aus Beispiel 126A wurden zunächst mittels präparativer HPLC in die beiden racemischen *cis-* und *trans*-Diastereomere getrennt [Ausbeute: 63 mg Diastereomer 1, 171 mg Diastereomer 2; Säule: Sunfire C18 OBD, 5 µm, 250 mm x 20 mm; Elutionsmittel: Wasser/Acetonitril 30:70 (v/v); Fluss: 25 ml/min; Temperatur: 24°C; UV-Detektion: 210 nm]. Diese wurden dann nachfolgend durch präparative HPLC an chiraler Phase in die jeweiligen Enantiomere aufgetrennt [Säule: Daicel Chiralpak AD-H, 250 mm x 20 mm; Elutionsmittel: Ethanol/Isohexan 30:70 (v/v); Fluss: 1.0 ml/min; Temperatur: 30°C; UV-Detektion: 220 nm]:

### Beispiel 127A (Diastereomer 1, Enantiomer 1):

Ausbeute: 31 mg
Rₜ 5.63 min; Reinheit >98.5%; >99.0% ee (Daicel-Säule s.o.)

### Beispiel 128A Diastereomer 1, Enantiomer 2):

Ausbeute: 25 mg
Rₜ 6.00 min; Reinheit >99.0%; >99.0% ee (Daicel-Säule s.o.)

### Beispiel 129A (Diastereomer 2. Enantiomer 1):

Ausbeute: 77 mg
Rₜ 5.81 min; Reinheit >99.0%; >99.0% ee (Daicel-Säule s.o.)

### Beispiel 130A (Diastereomer 2, Enantiomer 2):

Ausbeute: 91 mg
Rₜ 7.01 min; Reinheit >99.8%; >99.0% ee (Daicel-Säule s.o.).

### Beispiel 131A

### Methyl-4-[(cyclopentyl {4-[(5-oxo-2-phenyl-5,6-dihydro-4H-1,3,4-oxadiazin-4-yl)methyl]phenyl}-acetyl)amino]-2-methyl-2,3-dihydro-1H-inden-2-carboxylat

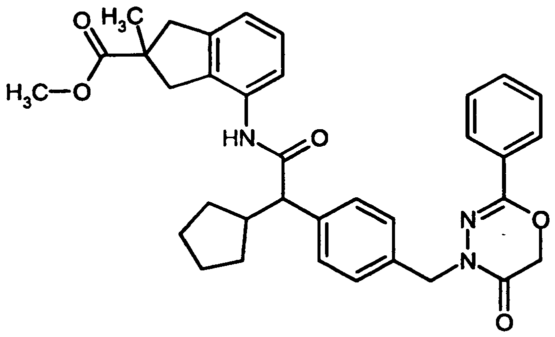

Eine Lösung von 200 mg (0.97 mmol) Cyclopentyl{4-[(5-oxo-2-phenyl-5,6-dihydro-4*H*-1,3,4-oxadiazin-4-yl)methyl]phenyl}essigsäure (Enantiomer 2, Beispiel 77A), 459 mg (1.2 mmol) Methyl-4-amino-2-methyl-2,3-dihydro-1*H*-inden-2-carboxylat (Beispiel 95A), 556 mg (1.46 mmol) *O*-(7-Azabenzotriazol-1-yl)-*N*,*N*,*N*',*N*'-tetramethyluronium-Hexafluorophosphat (HATU) und 5 ml Pyridin in 20 ml DMF wurde über Nacht bei Raumtemperatur gerührt. Nach Beendigung der Reaktion wurde der Ansatz auf Eiswasser gegossen, die Phasen getrennt und die wässrige Phase dreimal mit *tert*.-Butylmethylether extrahiert. Die vereinigten organischen Phasen wurden über Natriumsulfat getrocknet, und nach Filtration wurde das Lösungsmittel im Vakuum bis zur Trockene entfernt. Das erhaltene Rohprodukt wurde über präparative HPLC gereinigt. Es wurden 448 mg (0.77 mmol, 79% d. Th.) eines farblosen Öls erhalten.
LC-MS (Methode 11): Rₜ = 1.54 min; m/z = 580 (M+H)⁺.

Auf analoge Weise wurden die in der folgenden Tabelle aufgeführten Verbindungen erhalten:

| **Beispiel** | **Name / Struktur / Edukte** | **Analytische Daten** |
|---|---|---|
| **132A** | Methyl-4-{[(4-{[2-(4-chlorphenyl)-5-oxo-5,6-dihydro-4*H*-1,3,4-oxadiazin-4-yl]methyl}phenyl)(cyclopentyl)-acetyl]amino}-2,3-dihydro-1*H*-inden-2-carboxylat | ¹H-NMR (400 MHz, DMSO-d₆, δ/ppm): 9.47 (1H, s), 7.76 (2H, d), 7.51 (2H, d), 7.40 (2H, d), 7.35-7.26 (3H, m), 7.05 (1H, t), 6.95 (1H, d), 4.91 (2H, s), 4.85 (2H, s), 3.62 (3H, s), 3.51 (1H, d), 3.39-3.25 (1H, m), 3.20-2.92 (4H, m), 2.61-2.41 (1H, m), 1.88-1.71 (1H, m), 1.71-1.25 (6H, m), 1.04-0.90 (1H, m). LC-MS (Methode 10): Rₜ = 2.65 min; m/z = 600 (M)⁺. |
| | 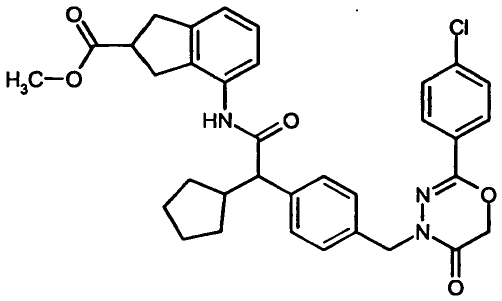 | |
| | (aus Bsp. 72A und Bsp. 88A) | |
| **133A** | Methyl-4-{[(4-{[2-(4-fluorphenyl)-5-oxo-5,6-dihydro-4*H*-1,3,4-oxadiazin-4-yl]methyl}phenyl)(cyclopentyl)-acetyl]amino}-2,3-dihydro-1*H*-inden-2-carboxylat | LC-MS (Methode 13): Rₜ = 2.79 min; m/z = 584 (M+H)⁺. |
| | 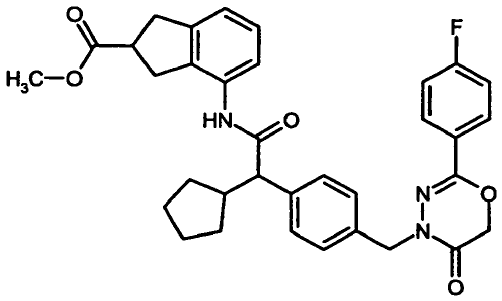 | |
| | (aus Bsp. 75A und Bsp. 88A) | |
| **134A** | Methyl-4-[(3-methyl-2-{4-[(5-oxo-2-phenyl-5,6-dihydro-4*H*-1,3,4-oxadiazin-4-yl)methyl]phenyl}-butanoyl)amino]-2,3-dihydro-1*H*-inden-2-carboxylat | ¹H-NMR (400 MHz, DMSO-d₆, δ/ppm): 9.49 (1H, s), 7.76 (2H, d), 7.53-7.26 (8H, m), 7.05 (1H, t), 6.95 (1H, d), 4.91 (2H, s), 4.86 (2H, s), 3.62 (3H, s), 3.42-3.27 (2H, m), 3.21-2.92 (4H, m), 2.39-2.21 (1H, m), 1.02 (3H, d), 0.65 (3H, d). LC-MS (Methode 11): Rₜ = 1.45 min; m/z = 540 (M+H)⁺. |
| | 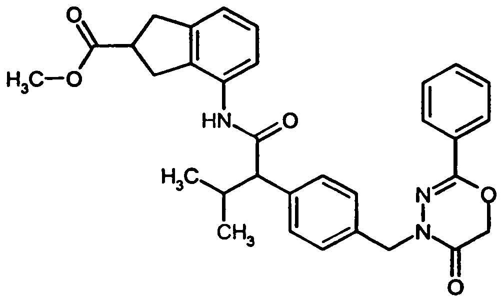 | |
| | (aus Bsp. 59A und Bsp. 88A) | |
| **135A** | Ethyl-(2*E*)-3-{3-[(3-methyl-2-{4-[(5-oxo-2-phenyl-5,6-dihydro-4*H*-1,3,4-oxadiazin-4-yl)methyl]phenyl}-butanoyl)amino]phenyl}prop-2-enoat | LC-MS (Methode 11): Rₜ = 1.51 min; m/z = 540 (M+H)⁺. |
| | 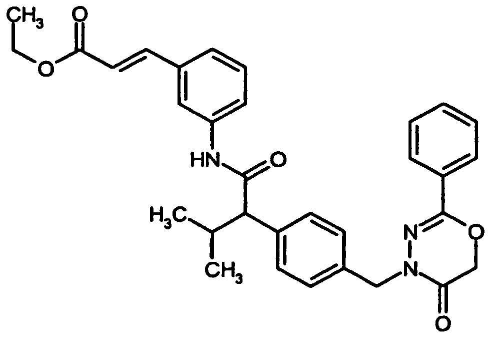 | |
| | (aus Bsp. 79A und Ethyl-(2*E*)-3-(3-aminophenyl)-prop-2-enoat [CAS Reg.-Nr. 6328-01-4]) | |
| **136A** | Methyl-2-methyl-4-[(3-methyl-2-{4-[(5-oxo-2-phenyl-5,6-dihydro-4*H*-1,3,4-oxadiazin-4-yl)methyl]phenyl}-butanoyl)amino]-2,3-dihydro-1*H*-inden-2-carboxylat | LC-MS (Methode 7): Rₜ = 2.97 min; m/z = 554 (M+H)⁺. |
| | 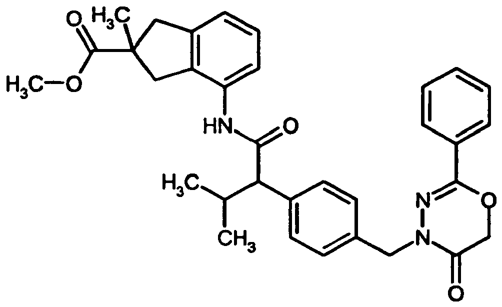 | |
| | (aus Bsp. 79A und Bsp. 95A) | |
| **137A** | Methyl-({3-[(3-methyl-2-{4-[(5-oxo-2-phenyl-5,6-dihydro-4*H*-1,3,4-oxadiazin-4-yl)methyl]phenyl}-butanoyl)amino]phenyl}sulfanyl)acetat | ¹H-NMR (400 MHz, DMSO-d₆, δ/ppm): 10.12 (1H, s), 7.75 (2H, d), 7.63 (1H, s), 7.54-7.28 (8H, m), 7.20 (1H, t), 6.99 (1H, d), 4.89 (2H, s), 4.84 (2H, s), 3.84 (2H, s), 3.59 (3H, s), 3.21 (1H, d), 2.40-2.24 (1H, m), 1.00 (3H, d), 0.68 (3H, d). LC-MS (Methode 7): Rₜ = 2.88 min; m/z = 546 (M+H)⁺. |
| | 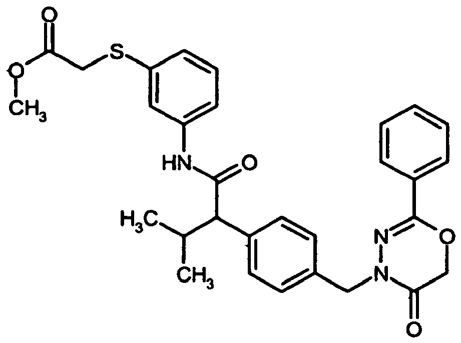 | |
| | (aus Bsp. 79A und Methyl-[(3-aminophenyl)-sulfanyl]acetat [CAS Reg.-Nr. 933371-82-5]) | |
| **138A** | Methyl-({3-[(cyclopentyl{4-[(5-oxo-2-phenyl-5,6-dihydro-4*H*-1,3,4-oxadiazin-4-yl)methyl]phenyl}-acetyl)amino]phenyl} sulfanyl)acetat | ¹H-NMR (400 MHz, DMSO-d₆, δ/ppm): 10.08 (1H, s), 7.74 (2H, d), 7.61 (1H, s), 7.52-7.26 (8H, m), 7.19 (1H, t), 6.98 (1H, d), 4.88 (2H, s), 4.83 (2H, s), 3.83 (2H, s), 3.58 (3H, s), 3.31 (1H, d), 2.61-2.45 (1H, m), 1.83-1.69 (1H, m), 1.68-1.15 (6H, m), 1.03-0.85 (1H, m). LC-MS (Methode 7): Rₜ = 3.02 min; m/z = 572 (M+H)⁺. |
| | 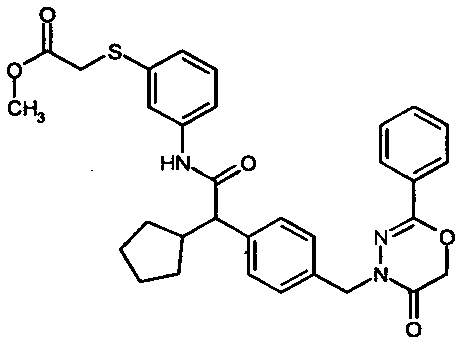 | |
| | (aus Bsp. 77A und Methyl-[(3-aminophenyl)-sulfanyl]acetat [CAS Reg.-Nr. 933371-82-5]) | |
| **139A** | Ethyl-5-{[(cyclopentyl{4-[(5-oxo-2-phenyl-5,6-dihydro-4*H*-1,3,4-oxadiazin-4-yl)methyl]phenyl}acetyl)-amino]methyl}isoxazol-3-carboxylat | LC-MS (Methode 7): Rₜ = 2.81 min; m/z = 545 (M+H)⁺. |
| | 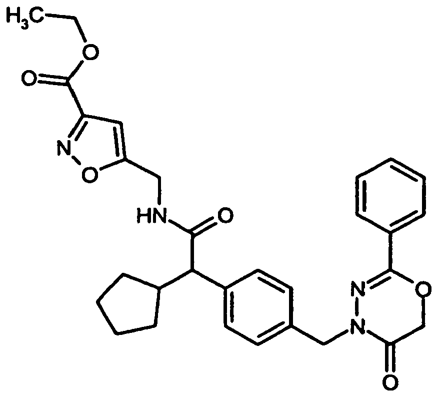 | |
| | (aus Bsp. 77A und Bsp. 101A) | |
| **140A** | Methyl-2-methyl-2-({3-[(3-methyl-2-{4-[(5-oxo-2-phenyl-5,6-dihydro-4*H*-1,3,4-oxadiazin-4-yl)methyl]-phenyl}butanoyl)amino]phenyl}sulfanyl)propanoat | LC-MS (Methode 7): Rₜ = 3.01 min; m/z = 574 (M+H)⁺. |
| | 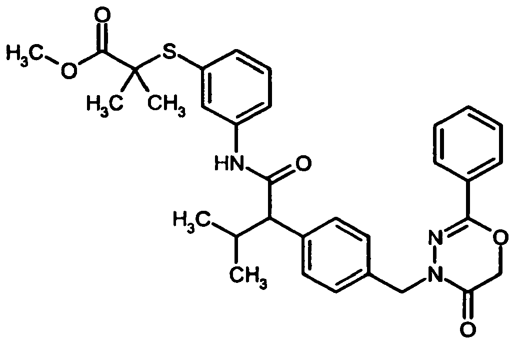 | |
| | (aus Bsp. 79A und Bsp. 102A) | |
| **141A** | Methyl-2-({3-[(cyclopentyl{4-[(5-oxo-2-phenyl-5,6-dihydro-4*H*-1,3,4-oxadiazin-4-yl)methyl]phenyl}-acetyl)amino]phenyl} sulfanyl)-2-methylpropanoat | LC-MS (Methode 7): Rₜ = 3.12 min; m/z = 600 (M+H)⁺. |
| | 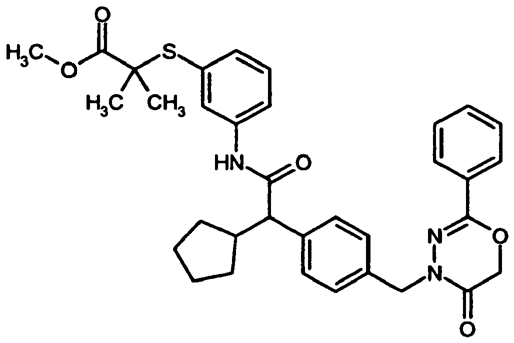 | |
| | (aus Bsp. 77A und Bsp. 102A) | |
| **142A** | Methyl-3-{3-[(3-methyl-2-{4-[(5-oxo-2-phenyl-5,6-dihydro-4*H*-1,3,4-oxadiazin-4-yl)methyl]phenyl}-butanoyl)amino]cyclohexyl}propanoat | LC-MS (Methode 7): Rₜ = 2.78 min; m/z = 534 (M+H)⁺. |
| | 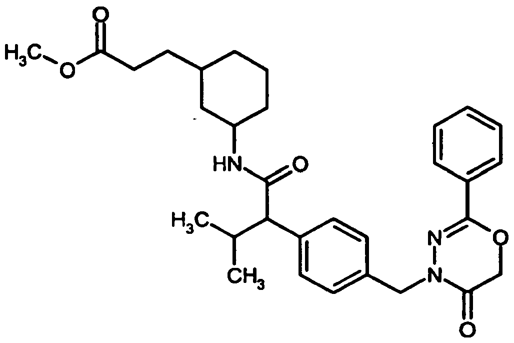 | |
| | (aus Bsp. 79A und Bsp. 100A) | |
| **143A** | Methyl-4-[(5,5,5-trifluor-2-{4-[(5-oxo-2-phenyl-5,6-dihydro-4*H*-1,3,4-oxadiazin-4-yl)methyl]phenyl}-pentanoyl)amino]-2,3-dihydro-1*H*-inden-2-carboxylat | LC-MS (Methode 10): Rₜ = 2.44 min; m/z = 594 (M+H)⁺. |
| | 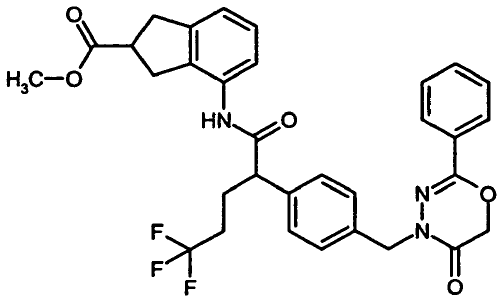 | |
| | (aus Bsp. 60A und Bsp. 89A) | |
| **144A** | Methyl-3-{*trans*-4-[(3-methyl-2-{4-[(5-oxo-2-phenyl-5,6-dihydro-4*H*-1,3,4-oxadiazin-4-yl)methyl]-phenyl}butanoyl)amino]cyclohexyl}propanoat | ¹H-NMR (400 MHz, DMSO-d₆, δ/ppm): 7.83 (1H, d), 7.76 (2H, d), 7.53-7.40 (3H, m), 7.32-7.22 (4H, m), 4.90 (2H, s), 4.84 (2H, s), 3.56 (3H, s), 3.41-3.28 (1H, m), 2.93 (1H, d), 2.27 (2H, t), 2.22-2.11 (1H, m), 1.87-1.75 (1H, m), 1.72-1.49 (2H, m), 1.44-1.31 (2H, m), 1.31-1.18 (2H, m), 1.18-0.95 (2H, m), 0.95-0.77 (2H, m), 0.91 (3H, d), 0.59 (3H, d). LC-MS (Methode 11): Rₜ = 1.41 min; m/z = 534 (M+H)⁺. |
| | 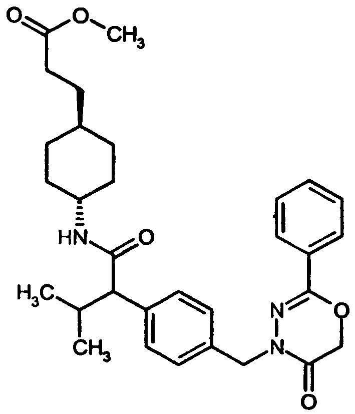 | |
| | (aus Bsp. 79A und Bsp. 98A) | |
| **145A** | Methyl-3-{*cis*-4-[(3-methyl-2-{4-[(5-oxo-2-phenyl-5,6-dihydro-4*H*-1,3,4-oxadiazin-4-yl)methyl]phenyl}-butanoyl)amino]cyclohexyl}propanoat | ¹H-NMR (400 MHz, DMSO-d₆, δ/ppm): 7.95 (1H, s), 7.75 (2H, d), 7.54-7.40 (3H, m), 7.35-7.21 (4H, m), 4.91 (2H, s), 4.84 (2H, s), 3.74-3.62 (1H, m), 3.57 (3H, s), 3.11 (1H, d), 2.28 (2H, t), 2.22-2.11 (1H, m), 1.61-1.19 (11H, m), 0.91 (3H, d), 0.61 (3H, d). LC-MS (Methode 7): Rₜ =2.78 min; m/z = 534 (M+H)⁺. |
| | 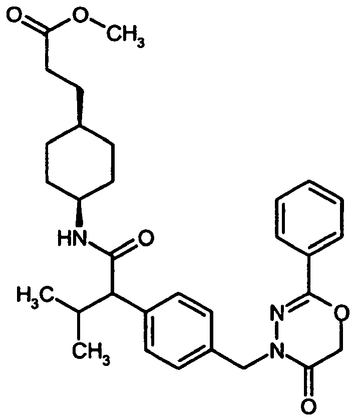 | |
| | (aus Bsp. 79A und Bsp. 99A) | |
| **146A** | Ethyl-3-{3-[(3-methyl-2-{4-[(5-oxo-2-phenyl-5,6-dihydro-4*H*-1,3,4-oxadiazin-4-yl)methyl]phenyl}-pentanoyl)amino]phenyl}propanoat | LC-MS (Methode 10): Rₜ = 2.54 min; m/z = 556 (M+H)⁺. |
| | 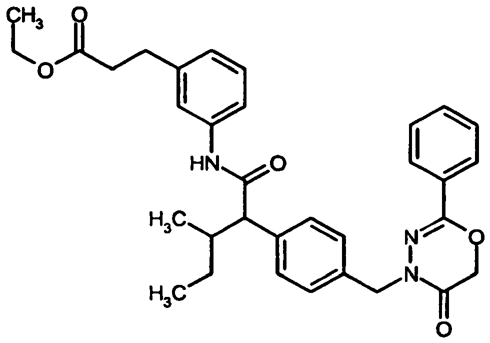 | |
| | (aus Bsp. 61A und Ethyl-3-(3-aminophenyl)propanoat) | |
| **147A** | Methyl-4-[(3-methyl-2-{4-[(5-oxo-2-phenyl-5,6-dihydro-4*H*-1,3,4-oxadiazin-4-yl)methyl]phenyl}-pentanoyl)amino]-2,3-dihydro-1*H*-inden-2-carboxylat | LC-MS (Methode 10): Rₜ = 2.45 min; m/z = 554 (M+H)⁺. |
| | 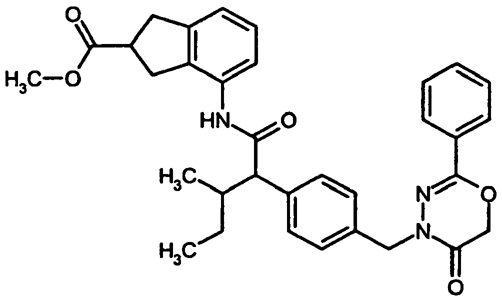 | |
| | (aus Bsp. 61A und Bsp. 89A) | |
| **148A** | Methyl-3-{*trans*-4-[(cyclopentyl{4-[(5-oxo-2-phenyl-5,6-dihydro-4*H*-1,3,4-oxadiazin-4-yl)methyl]phenyl}-acetyl)amino]cyclohexyl}propanoat | ¹H-NMR (400 MHz, DMSO-d₆, δ/ppm): 7.80 (1H, d), 7.76 (2H, d), 7.54-7.41 (3H, m), 7.28 (4H, q), 4.91 (2H, s), 4.83 (2H, s), 3.56 (3H, s), 3.41-3.31 (1H, m), 3.08 (1H, d), 2.55-2.39 (1H, m), 2.27 (2H, t), 1.86-1.75 (1H, m), 1.75-0.78 (18H, m). LC-MS (Methode 7): Rₜ = 2.90 min; m/z = 560 (M+H)⁺. |
| | 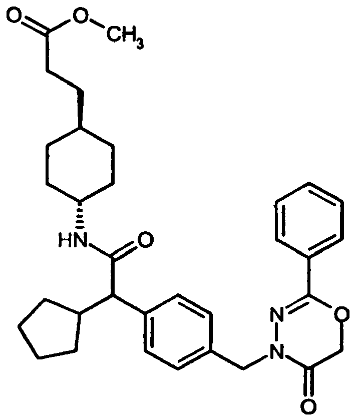 | |
| | (aus Bsp. 77A und Bsp. 98A) | |
| **149A** | Methyl-3-{*cis*-4-[(cyclopentyl{4-[(5-oxo-2-phenyl-5,6-dihydro-4*H*-1,3,4-oxadiazin-4-yl)methyl]phenyl}-acetyl)amino]cyclohexyl}propanoat | ¹H-NMR (400 MHz, DMSO-d₆, δ/ppm): 7.82-7.69 (3H, m), 7.54-7.40 (3H, m), 7.33 (2H, d), 7.26 (2H, d), 4.91 (2H, s), 4.84 (2H, s), 3.74-3.61 (1H, m), 3.57 (3H, s), 3.26 (1H, d), 2.55-2.39 (1H, m), 2.28 (2H, t), 1.73-1.10 (16H, m), 0.99-0.78 (3H, m). LC-MS (Methode 7): Rₜ = 2.90 min; m/z = 560 (M+H)⁺. |
| | 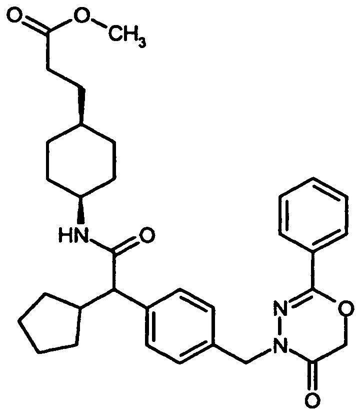 | |
| | (aus Bsp. 77A und Bsp. 99A) | |
| **150A** | Ethyl-4-{[(3-methyl-2-{4-[(5-oxo-2-phenyl-5,6-dihydro-4*H*-1,3,4-oxadiazin-4-yl)methyl]phenyl}pentanoyl)-amino]methyl} cyclohexancarboxylat | LC-MS (Methode 10): Rₜ = 2.45 min; m/z = 548 (M+H)⁺. |
| | 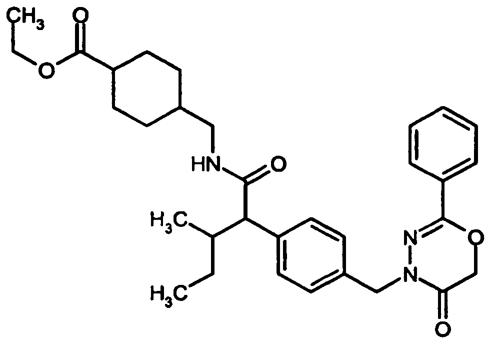 | |
| | (aus Bsp. 61A und Ethyl-4-(aminomethyl)-cyclohexancarboxylat (Isomerengemisch; Beilstein Reg.-Nr. 10777142)) | |
| **151A** | Ethyl-3-{2-methyl-3-[(3-methyl-2-{4-[(5-oxo-2-phenyl-5,6-dihydro-4*H*-1,3,4-oxadiazin-4-yl)methyl]phenyl}-pentanoyl)amino]phenyl}propanoat | LC-MS (Methode 10): Rₜ = 2.49 min; m/z = 570 (M+H)⁺. |
| | 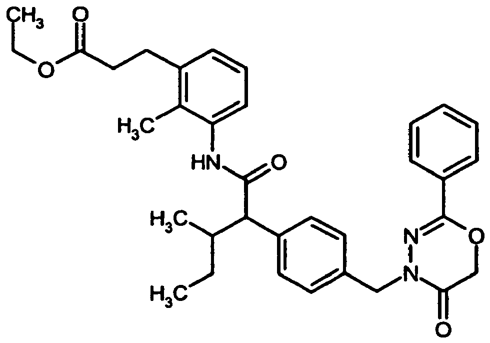 | |
| | (aus Bsp. 61A und Bsp. 97A) | |
| **152A** | Methyl-(1*S*,3*S*)-3-[(cyclopentyl{4-[(5-oxo-2-phenyl-5,6-dihydro-4*H*-1,3,4-oxadiazin-4-yl)methyl]phenyl}-acetyl)amino]cyclopentancarboxylat | ¹H-NMR (400 MHz, DMSO-d₆, δ/ppm): 8.00 (1H, d), 7.78 (2H, d), 7.54-7.39 (3H, m), 7.37-7.20 (4H, m), 4.91 (2H, s), 4.84 (2H, s), 4.05-3.91 (1H, m), 3.55 (3H, s), 3.08 (1H, d), 2.94-2.81 (1H, m), 2.60-2.39 (1H, m), 1.99-1.78 (3H, m), 1.75-1.10 (10H, m), 0.96-0.81 (1H, m). LC-MS (Methode 7): Rₜ = 2.68 min; m/z =518 (M+H)⁺. |
| | 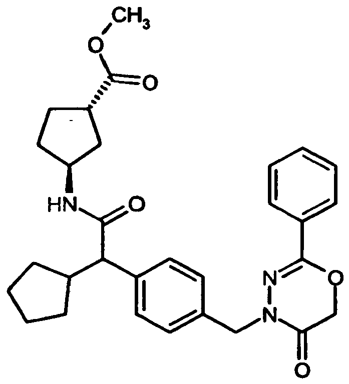 | |
| | (aus Bsp. 77A und Methyl-(1*S*,3*S*)-3-amino-cyclopentancarboxylat-Hydrochlorid [CAS Reg.-Nr. 222530-45-2]) | |
| **153A** | Methyl-(1*S*,3*R*)-3-[(cyclopentyl{4-[(5-oxo-2-phenyl-5,6-dihydro-4*H*-1,3,4-oxadiazin-4-yl)methyl]phenyl}-acetyl)amino]cyclopentancarboxylat | ¹H-NMR (400 MHz, DMSO-d₆, δ/ppm): 8.01 (1H, d), 7.77 (2H, d), 7.54-7.41 (3H, m), 7.29 (4H, q), 4.91 (2H, s), 4.83 (2H, s), 3.98-3.85 (1H, m), 3.59 (3H, s), 3.10 (1H, d), 2.84-2.70 (1H, m), 2.58-2.39 (1H, m), 2.23-2.10 (1H, m), 1.84-1.11 (12H, m), 0.96-0.81 (1H, m). LC-MS (Methode 7): Rₜ =2.69 min; m/z = 518 (M+H)⁺. |
| | 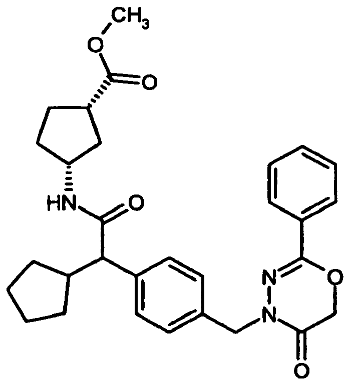 | |
| | (aus Bsp. 77A und Methyl-(1*S*,3*R*)-3-amino-cyclopentancarboxylat-Hydrochlorid [CAS Reg.-Nr. 180323-49-3]) | |
| **154A** | Methyl-(1*R*,3*R*)-3-[(cyclopentyl{4-[(5-oxo-2-phenyl-5,6-dihydro-4*H*-1,3,4-oxadiazin-4-yl)methyl]phenyl}-acetyl)amino]cyclopentancarboxylat | ¹H-NMR (400 MHz, DMSO-d₆, δ/ppm): 7.99 (1H, d), 7.76 (2H, d), 7.55-7.41 (3H, m), 7.28 (4H, q), 4.91 (2H, s), 4.84 (2H, s), 4.04-3.92 (1H, m), 3.58 (3H, s), 3.09 (1H, d), 2.95-2.82 (1H, m), 2.58-2.39 (1H, m), 2.01-1.85 (2H, m), 1.84-1.12 (11H, m), 0.95-0.82 (1H, m). LC-MS (Methode 7): Rₜ = 2.66 min; m/z =518 (M+H)⁺. |
| | 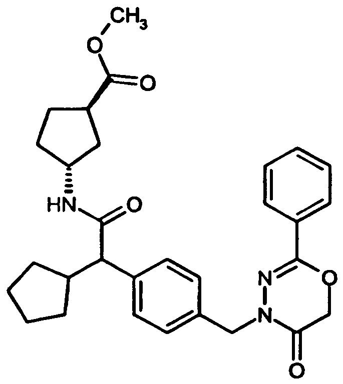 | |
| | (aus Bsp. 77A und Methyl-(1*R*,3*R*)-3-amino-cyclopentancarboxylat-Hydrochlorid [CAS Reg.-Nr. 489446-79-9]) | |
| **155A** | Methyl-(1*R*,3*S*)-3-[(cyclopentyl{4-[(5-oxo-2-phenyl-5,6-dihydro-4*H*-1,3,4-oxadiazin-4-yl)methyl]phenyl}-acetyl)amino]cyclopentancarboxylat | LC-MS (Methode 11): Rₜ = 1.39 min; m/z =518 (M+H)⁺. |
| | 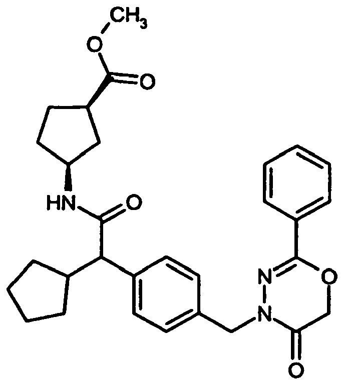 | |
| | (aus Bsp. 77A und Methyl-(1*R*,3*S*)-3-amino-cyclopentancarboxylat-Hydrochlorid [CAS Reg.-Nr. 180196-56-9]) | |
| **156A** | *tert*.-Butyl-3-{3-[(cyclopentyl{4-[(4-oxo-1,2,3-benzotriazin-3(4*H*)-yl)methyl]phenyl}acetyl)amino]-phenyl}propanoat | LC-MS (Methode 7): Rₜ = 3.14 min; m/z = 565 (M-H)⁻. |
| | 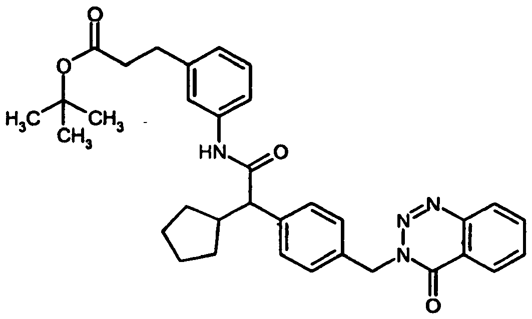 | |
| | (aus Bsp. 56A und *tert*.-Butyl-3-(3-aminophenyl)-propanoat [Beilstein Reg.-Nr. 10341419]) | |
| **157A** | *tert*.-Butyl-3-{3-[(cyclopentyl{4-[(2-oxo-5-phenyl-1,3,4-oxadiazol-3(2*H*)-yl)methyl]phenyl}acetyl)amino]-phenyl}propanoat | LC-MS (Methode 7): Rₜ = 3.24 min; m/z = 580 (M-H)⁻. |
| | 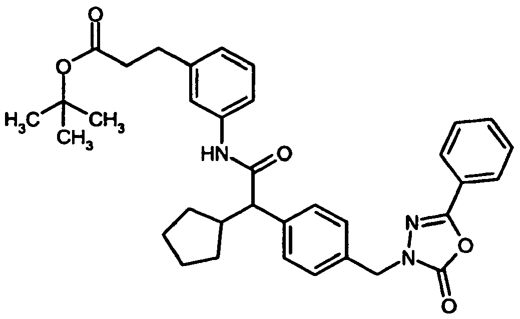 | |
| | (aus Bsp. 57A und *tert*.-Butyl-3-(3-aminophenyl)-propanoat [Beilstein Reg.-Nr. 10341419]) | |
| **158A** | Methyl-*trans*-4-[(cyclopentyl {4-[(5-oxo-2-phenyl-5,6-dihydro-4*H*-1,3,4-oxadiazin-4-yl)methyl]phenyl}-acetyl)amino]cyclohexancarboxylat | ¹H-NMR (400 MHz, DMSO-d₆, δ/ppm): 7.85 (1H, d), 7.76 (2H, d), 7.53-7.40 (3H, m), 7.29 (4H, q), 4.91 (2H, s), 4.83 (2H, s), 3.68-3.55 (1H, m), 3.59 (3H, s), 3.20 (1H, d), 2.48-2.39 (1H, m), 1.88-1.73 (2H, m), 1.73-1.35 (10H, m), 1.35-1.12 (4H, m), 0.99-0.82 (1H, m). LC-MS (Methode 7): Rₜ = 2.76 min; m/z = 532 (M+H)⁺. |
| | 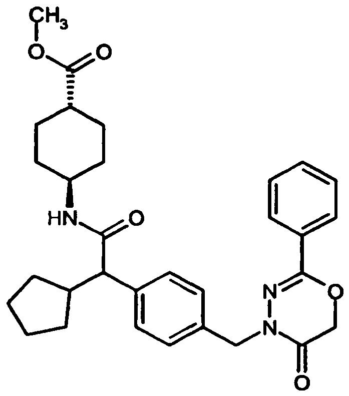 | |
| | (aus Bsp. 77A und Methyl-*trans*-4-aminocyclohexan-carboxylat-Hydrochlorid [CAS Reg.-Nr. 61367-07-5]) | |
| **159A** | Methyl-*cis*-4-[(cyclopentyl{4-[(5-oxo-2-phenyl-5,6-dihydro-4*H*-1,3,4-oxadiazin-4-yl)methyl]phenyl}-acetyl)amino]cyclohexancarboxylat | ¹H-NMR (400 MHz, DMSO-d₆, δ/ppm): 7.85 (1H, d), 7.76 (2H, d), 7.54-7.41 (3H, m), 7.28 (4H, q), 4.91 (2H, s), 4.83 (2H, s), 3.56 (3H, s), 3.42-3.55 (1H, m), 3.09 (1H, d), 2.57-2.39 (1H, m), 2.29-2.16 (1H, m), 1.94-1.78 (3H, m), 1.77-1.01 (12H, m), 0.95-0.82 (1H, m). LC-MS (Methode 7): Rₜ = 2.73 min; m/z = 532 (M+H)⁺. |
| | 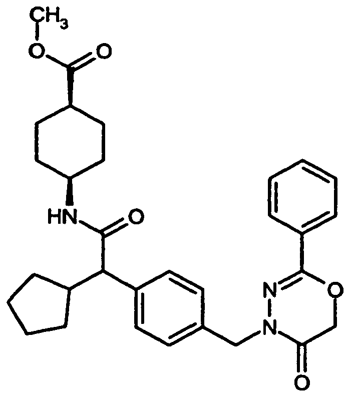 | |
| | (aus Bsp. 77A und Methyl-*cis*-aminocyclohexan-carboxylat-Hydrochlorid [CAS Reg.-Nr. 61367-16-6]) | |
| **160A** | Methyl-2-methyl-4-[(3-methyl-2-{4-[(5-oxo-2-phenyl-5,6-dihydro-4*H*-1,3,4-oxadiazin-4-yl)methyl]phenyl}-pentanoyl)amino]-2,3-dihydro-1*H*-inden-2-carboxylat | LC-MS (Methode 11): Rₜ = 1.53 min; m/z = 568 (M+H)⁺. |
| | 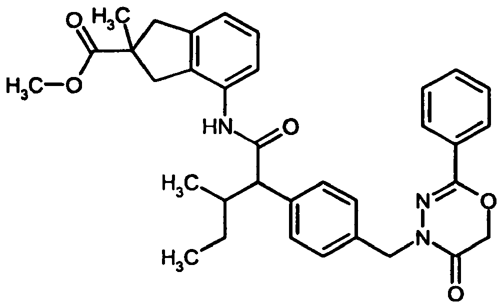 | |
| | (aus Bsp. 61A und Bsp. 95A) | |
| **161A** | *tert.*-Butyl-3-(3- {[(4-{[3-(3-chlorphenyl)-2-oxo-imidazolidin-1-yl]methyl}phenyl)(cyclopentyl)-acetyl]amino}-2-methylphenyl)propanoat | LC-MS (Methode 10): Rₜ = 2.83 min; m/z = 648 (M+NH₄)⁺. |
| | 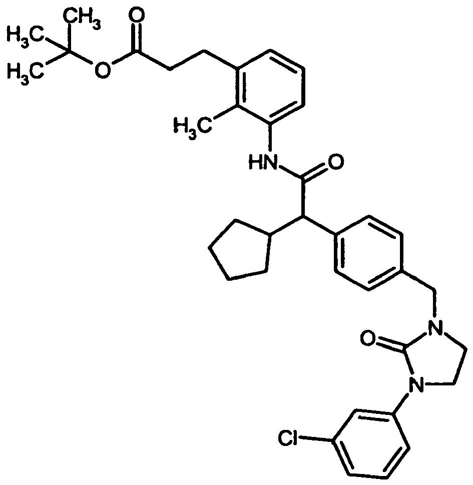 | |
| | (aus Bsp. 67A und Bsp. 96A) | |
| **162A** | *tert*.-Butyl-3-{3-[(cyclopentyl{4-[(3-methyl-2-oxo-imidazolidin-1-yl)methyl]phenyl}acetyl)amino]-2-methylphenyl}propanoat | LC-MS (Methode 11): Rₜ = 1.43 min; m/z =551 (M+NH₄)⁺. |
| | 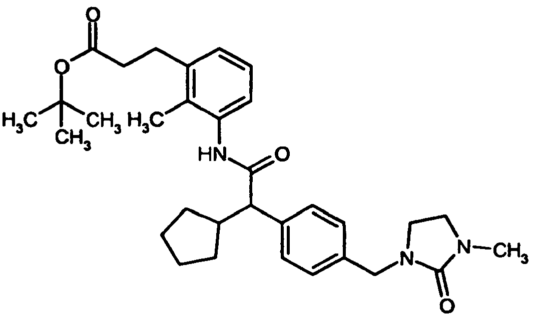 | |
| | (aus Bsp. 66A und Bsp. 96A) | |
| **163A** | tert.-Butyl-3-{2-methyl-3-[(3-methyl-2-{4-[(5-oxo-2-phenyl-5,6-dihydro-4*H*-1,3,4-oxadiazin-4-yl)methyl]-phenyl}hexanoyl)amino]phenyl}propanoat | LC-MS (Methode 11): Rₜ = 1.68 min; m/z = 634 (M+Na)⁺, 610 (M-H)⁻. |
| | 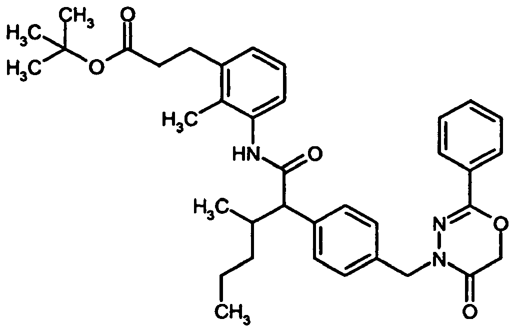 | |
| | (aus Bsp. 62A und Bsp. 96A) | |
| **164A** | Ethyl-3-{3-[(3-methyl-2-{4-[(5-oxo-2-phenyl-5,6-dihydro-4*H*-1,3,4-oxadiazin-4-yl)methyl]phenyl}-hexanoyl)amino]phenyl}propanoat | LC-MS (Methode 7): Rₜ = 3.04 min; m/z = 570 (M+H)⁺. |
| | 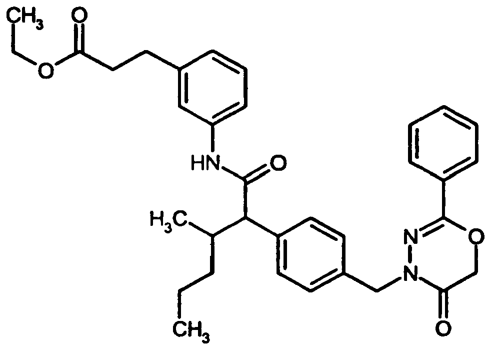 | |
| | (aus Bsp. 62A und Ethyl-3-(3-aminophenyl)propanoat) | |
| **165A** | Methyl-4-[(3-methyl-2-{4-[(5-oxo-2-phenyl-5,6-dihydro-4*H*-1,3,4-oxadiazin-4-yl)methyl]phenyl}-hexanoyl)amino]-2,3-dihydro-1*H*-inden-2-carboxylat | LC-MS (Methode 7): Rₜ = 2.96 min; m/z = 568 (M+H)⁺. |
| | 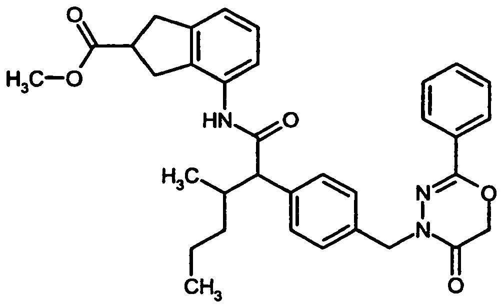 | |
| | (aus Bsp. 62A und Bsp. 89A) | |
| **166A** | *tert*.-Butyl-3-{3-[(cyclopentyl{4-[(4-oxo-1,2,3-benzotriazin-3(4*H*)-yl)methyl]phenyl}acetyl)amino]-2-methylphenyl}propanoat | ¹H-NMR (400 MHz, DMSO-d₆, δ/ppm): 9.46 (1H, d), 8.28-8.21 (2H, m), 8.10 (1H, t), 7.95 (1H, t), 7.40 (2H, d), 7.33 (2H, d), 7.04-6.92 (3H, m), 5.57 (2H, s), 3.45 (1H, d), 2.77 (2H, t), 2.61-2.48 (1H, m), 2.41 (2H, t), 1.98 (3H, s), 1.89-1.75 (1H, m), 1.74-1.40 (4H, m), 1.40-1.28 (2H, m), 1.36 (9H, s), 1.02-0.88 (1H, m). LC-MS (Methode 11): Rₜ = 1.57 min; m/z = 603 (M+Na)⁺. |
| | 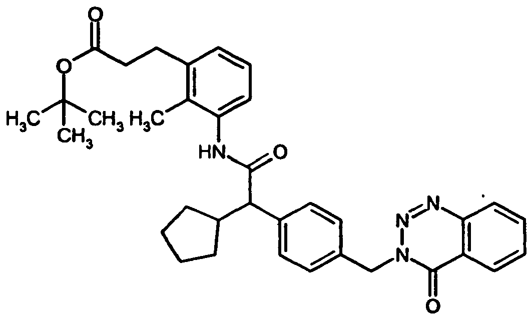 | |
| | (aus Bsp. 56A und Bsp. 96A) | |
| **167A** | *tert*.-Butyl-3-{3-[(cyclopentyl{4-[(2-oxo-5-phenyl-1,3,4-oxadiazol-3(2*H*)-yl)methyl]phenyl}acetyl)amino]-2-methylphenyl}propanoat | LC-MS (Methode 10): Rₜ = 2.77 min; m/z = 613 (M+NH₄)⁺. |
| | 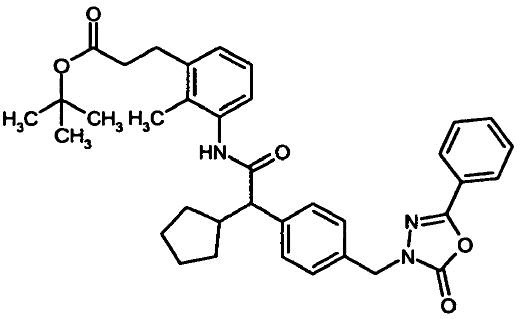 | |
| | (aus Bsp. 57A und Bsp. 96A) | |
| **168A** | *tert*.-Butyl-3-(3-{[(4-{[3-(3-chlorphenyl)-2-oxo-imidazolidin-1-yl]methyl}phenyl)(cyclopentyl)acetyl]-amino}phenyl)propanoat | LC-MS (Methode 10): Rₜ = 2.86 min; m/z = 634 (M+NH₄)⁺. |
| | 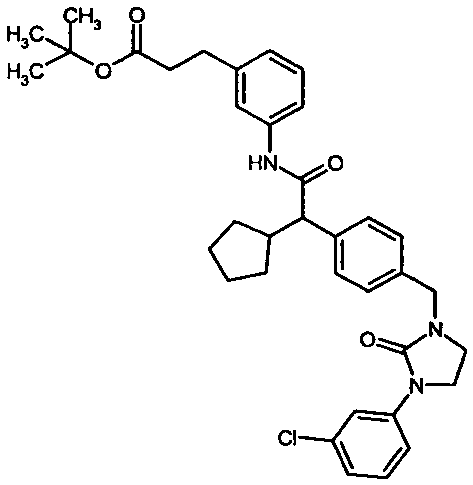 | |
| | (aus Bsp. 67A und *tert*.-Butyl-3-(3-aminophenyl)-propanoat) | |
| **169A** | *tert*.-Butyl-3-{3-[(cyclopentyl{4-[(3-methyl-2-oxo-imidazolidin-1-yl)methyl]phenyl}acetyl)amino]-phenyl}propanoat | LC-MS (Methode 11): Rₜ = 1.44 min; m/z =518 (M-H)⁻. |
| | 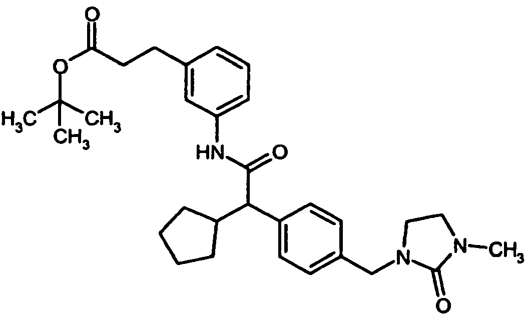 | |
| | (aus Bsp. 66A und *tert*.-Butyl-3-(3-aminophenyl)-propanoat) | |
| **170A** | *tert*.-Butyl-3-{3-[(4-methoxy-3-methyl-2-{4-[(5-oxo-2-phenyl-5,6-dihydro-4*H*-1,3,4-oxadiazin-4-yl)methyl]-phenyl}butanoyl)amino]-2-methylphenyl}propanoat | LC-MS (Methode 7): Rₜ = 2.94 min; m/z = 614 (M+H)⁺. |
| | 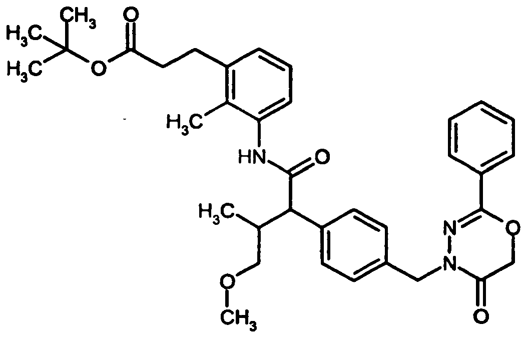 | |
| | (aus Bsp. 64A und Bsp. 96A) | |
| **171A** | *tert*.-Butyl-3-{3-[(4-cyano-3-methyl-2-{4-[(5-oxo-2-phenyl-5,6-dihydro-4*H*-1,3,4-oxadiazin-4-yl)methyl]-phenyl}butanoyl)amino]-2-methylphenyl}propanoat | LC-MS (Methode 7): Rₜ = 2.47 min; m/z = 626 (M+NH₄)⁺. |
| | 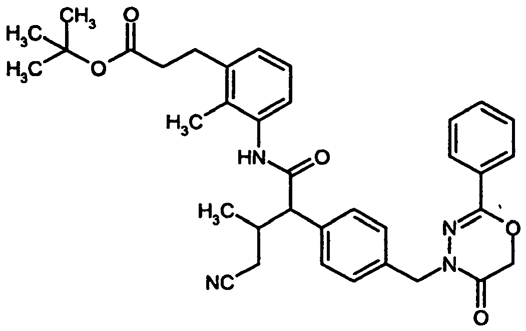 | |
| | (aus Bsp. 63A und Bsp. 96A) | |
| **172A** | Methyl-3-{3-[(4-cyano-3-methyl-2-{4-[(5-oxo-2-phenyl-5,6-dihydro-4*H*-1,3,4-oxadiazin-4-yl)methyl]-phenyl}butanoyl)amino]phenyl}propanoat | LC-MS (Methode 7): Rₜ = 2.58 min; m/z = 553 (M+H)⁺. |
| | 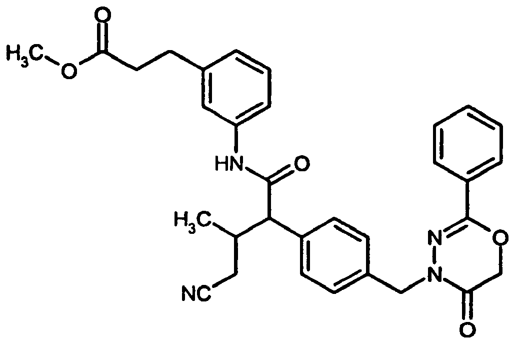 | |
| | (aus Bsp. 63A und Methyl-3-(3-aminophenyl)-propanoat) | |
| **173A** | Methyl-4-[(4-cyano-3-methyl-2-{4-[(5-oxo-2-phenyl-5,6-dihydro-4*H*-1,3,4-oxadiazin-4-yl)methyl]phenyl}-butanoyl)amino]-2,3-dihydro-1*H*-inden-2-carboxylat | LC-MS (Methode 7): Rₜ = 2.59 min; m/z = 565 (M+H)⁺. |
| | 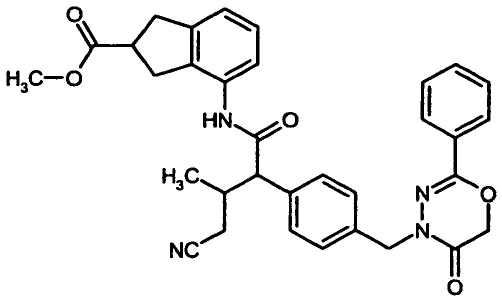 | |
| | (aus Bsp. 63A und Bsp. 89A) | |
| **174A** | Ethyl-(2*E*)-3-{3-[(cyclopentyl{4-[(5-oxo-2-phenyl-5,6-dihydro-4*H*-1,3,4-oxadiazin-4-yl)methyl]phenyl}-acetyl)amino]phenyl}prop-2-enoat | LC-MS (Methode 11): Rₜ = 1.58 min; m/z = 566 (M+H)⁺. |
| | 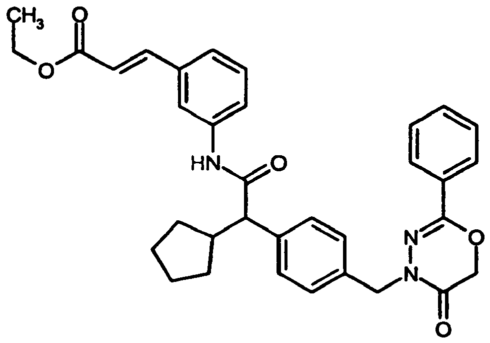 | |
| | (aus Bsp. 77A und Ethyl-(2*E*)-3-(3-aminophenyl)-prop-2-enoat) | |
| **175A** | *tert*.-Butyl-3-{3-[(cyclopentyl{4-[(5-oxo-2-phenyl-5,6-dihydro-4*H*-1,3,4-oxadiazin-4-yl)methyl]phenyl}-acetyl)amino]-2-methylphenyl}propanoat | ¹H-NMR (400 MHz, DMSO-d₆, δ/ppm): 9.46 (1H, d), 7.78 (2H, d), 7.55-7.38 (5H, m), 7.31 (2H, d), 7.05-6.91 (3H, m), 4.92 (2H, s), 4.86 (2H, s), 3.45 (1H, d), 2.76 (2H, t), 2.61-2.46 (1H, m), 2.40 (2H, t), 1.96 (3H, s), 1.89-1.76 (1H, m), 1.75-1.28 (6H, m), 1.35 (9H, s), 1.06-0.81 (1H, m). LC-MS (Methode 10): Rₜ = 2.79 min; m/z = 632 (M+Na)⁺. |
| | 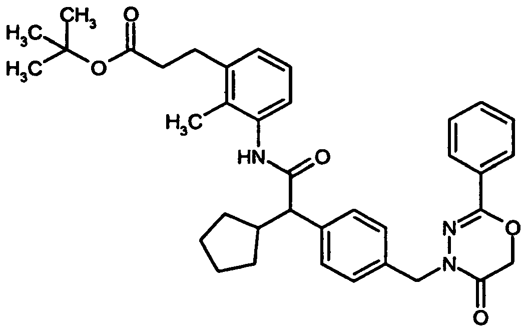 | |
| | (aus Bsp. 77A und Bsp. 96A) | |
| **176A** | Ethyl-(2*E*)-3-{4-[(3-methyl-2-{4-[(5-oxo-2-phenyl-5,6-dihydro-4*H*-1,3,4-oxadiazin-4-yl)methyl]phenyl}-butanoyl)amino]phenyl}prop-2-enoat | LC-MS (Methode 11): Rₜ = 1.50 min; m/z = 540 (M+H)⁺. |
| | 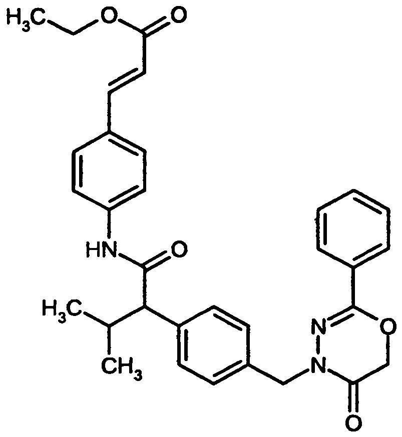 | |
| | (aus Bsp. 79A und Ethyl-(2*E*)-3-(4-aminophenyl)-prop-2-enoat) | |
| **177A** | Methyl-2-{3-[(cyclopentyl{4-[(5-oxo-2-phenyl-5,6-dihydro-4*H*-1,3,4-oxadiazin-4-yl)methyl]phenyl}acetyl)-amino]-2-methylphenoxy} -2-methylpropanoat | LC-MS (Methode 7): Rₜ = 3.05 min; m/z = 598 (M+H)⁺. |
| | 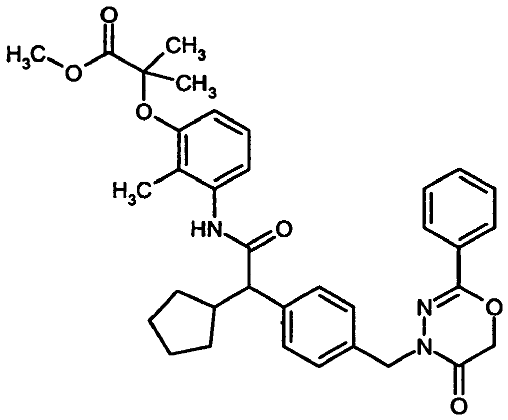 | |
| | (aus Bsp. 77A und Bsp. 104A) | |
| **178A** | Ethyl-(2*E*)-3-{3-[(2-{4-[(2-tert.-butyl-5-oxo-5,6-dihydro-4*H*-1,3,4-oxadiazin-4-yl)methyl]phenyl}-3-methylbutanoyl)amino]phenyl}prop-2-enoat | LC-MS (Methode 7): Rₜ = 3.08 min; m/z = 520 (M+H)⁺. |
| | 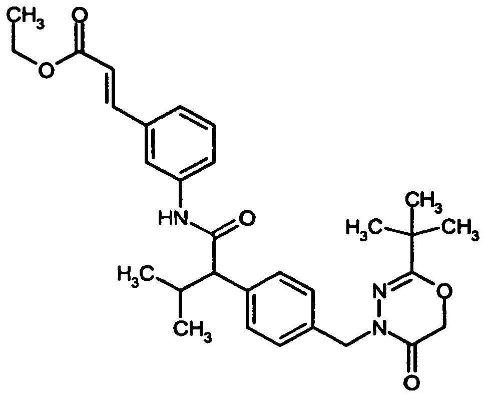 | |
| | (aus Bsp. 58A und Ethyl-(2*E*)-3-(3-aminophenyl)-prop-2-enoat) | |
| **179A** | Ethyl-4-{3-[(cyclopentyl{4-[(5-oxo-2-phenyl-5,6-dihydro-4*H*-1,3,4-oxadiazin-4-yl)methyl]phenyl}-acetyl)amino]phenyl}butanoat | LC-MS (Methode 7): Rₜ = 3.16 min; m/z = 582 (M+H)⁺. |
| | 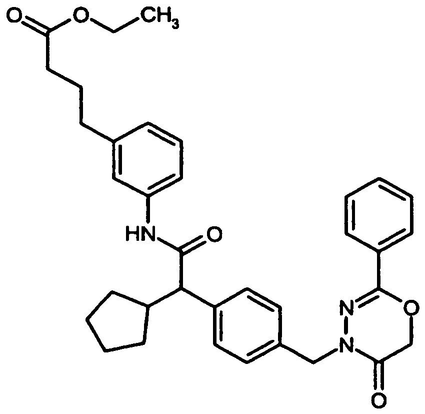 | |
| | (aus Bsp. 77A und Ethyl-4-(3-aminophenyl)butanoat [Beilstein Reg.-Nr. 10431434]) | |
| **180A** | Ethyl-3-{3-[(cyclopentyl{4-[(5-oxo-2-phenyl-5,6-dihydro-4*H*-1,3,4-oxadiazin-4-yl)methyl]phenyl}-acetyl)amino]phenyl}propanoat | LC-MS (Methode 7): Rₜ = 3.03 min; m/z = 568 (M+H)⁺. |
| | 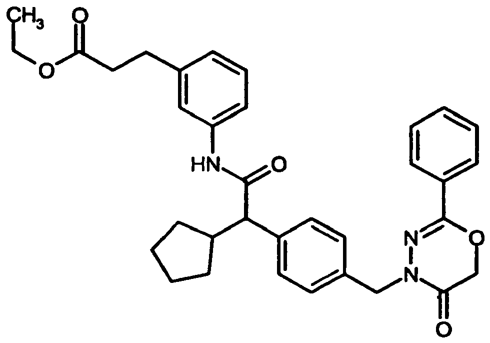 | |
| | (aus Bsp. 77A und Ethyl-3-(3-aminophenyl)propanoat) | |
| **181A** | Methyl-2-{3-[(cyclopentyl {4-[(5-oxo-2-phenyl-5,6-dihydro-4*H*-1,3,4-oxadiazin-4-yl)methyl]phenyl}-acetyl)amino]phenyl}-2-methylpropanoat | LC-MS (Methode 11): Rₜ = 1.55 min; m/z = 568 (M+H)⁺. |
| | 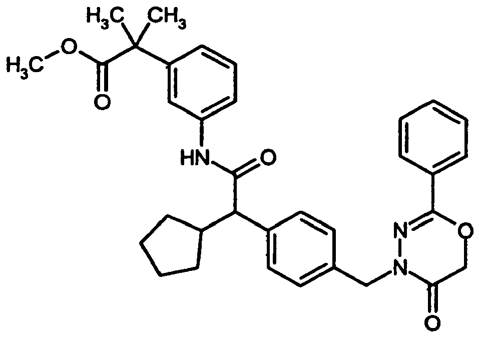 | |
| | (aus Bsp. 77A und Methyl-2-(3-aminophenyl)-2-methylpropanoat) | |

### Beispiel 182A

### [4-(Brommethyl)phenyl](cyclopentyl)essigsäure

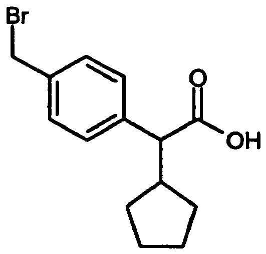

Eine Lösung von 500 mg (1.415 mmol) *tert*.-Butyl-[4-(brommethyl)phenyl](cyclopentyl)acetat (Beispiel 10A) in 5 ml Dichlormethan wurde mit 1.635 ml (2.420 g, 21.228 mmol) Trifluoressigsäure versetzt. Es wurde 2 h bei Raumtemperatur gerührt. Danach wurde gesättigte wässrige Natriumhydrogencarbonat-Lösung zugesetzt und das Gemisch mit Essigsäureethylester extrahiert. Die vereinigten organischen Phasen wurden über Natriumsulfat getrocknet und im Vakuum eingeengt. Es wurden 364 mg (87% d. Th.) der Titelverbindung erhalten.
LC-MS (Methode 10): Rₜ = 2.09 min; m/z = 295 (M-H)⁻.

### Beispiel 183A

### [4-(Brommethyl)phenyl](cyclopentyl)acetylchlorid

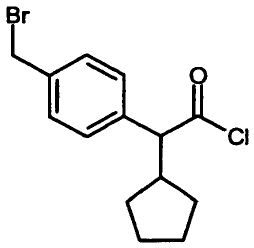

246 mg (0.766 mmol) [4-(Brommethyl)phenyl](cyclopentyl)essigsäure wurden in 5 ml Thionylchlorid gelöst und 1 h bei Raumtemperatur und anschließend 1 h bei 50°C gerührt. Das Gemisch wurde danach im Vakuum eingeengt. Es wurden 253 mg der Titelverbindung erhalten, welche ohne weitere Aufreinigung in der Folgestufe umgesetzt wurde.

### Beispiele 184A

### Methyl-4-({[4-(brommethyl)phenyl](cyclopentyl)acetyl}amino)-2,3-dihydro-1H-inden-2-carboxylat

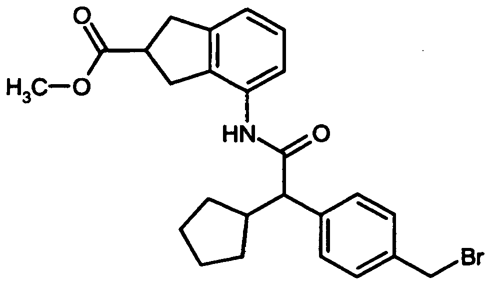

235 mg (1.228 mmol) Methyl-4-amino-2,3-dihydro-1H-inden-2-carboxylat (racemisch; Beispiel 88A) wurden in 10 ml THF, gelöst und bei 0°C mit 428 mg (4.911 mmol) *N,N*-Diisopropylethylamin sowie 388 mg (1.228 mmol) [4-(Brommethyl)phenyl](cyclopentyl)acetylchlorid versetzt. Es wurde 30 min bei 0°C gerührt. Danach wurde gesättigte wässrige Natriumhydrogencarbonat-Lösung zugesetzt und das Gemisch mit Essigsäureethylester extrahiert. Die vereinigten organischen Phasen wurden über Natriumsulfat getrocknet und am Rotationsverdampfer eingeengt. Es wurden 577 mg der Titelverbindung erhalten.
LC-MS (Methode 13): Rₜ = 2.72 min; m/z = 470 (M+H)⁺.

### Beispiel 185A

### Methyl-4-{[cyclopentyl(4-{[2-(2-methylpropyl)-5-oxo-5,6-dihydro-4H-1,3,4-oxadiazin-4-yl]-methyl}phenyl)acetyl]amino}-2,3-dihydro-1H-inden-2-carboxylat

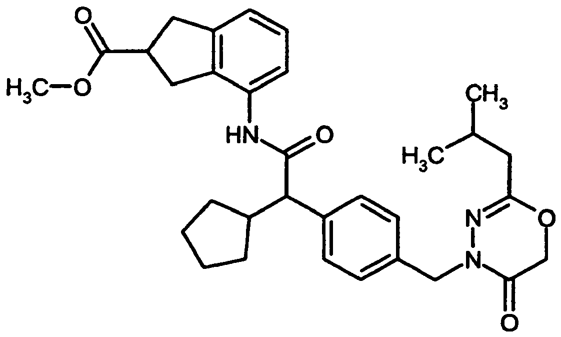

100 mg (0.213 mmol) Methyl-4-({[4-(brommethyl)phenyl](cyclopentyl)acetyl}amino)-2,3-dihydro-1*H*-inden-2-carboxylat wurden in 3.5 ml DMF gelöst und mit 55 mg (0.213 mmol) 2-(2-Methylpropyl)-4*H*-1,3,4-oxadiazin-5(6H)-on (Beispiel 22A) sowie 76 mg (0.234 mmol) Cäsiumcarbonat versetzt. Das Reaktionsgemisch wurde 3 d bei Raumtemperatur gerührt. Der Ansatz wurde dann im Vakuum eingeengt und der Rückstand mittels präparativer HPLC gereinigt. Es wurden 8 mg (8% d. Th.) der Titelverbindung erhalten.
LC-MS (Methode 7): Rₜ = 3.07 min; m/z = 546 (M-H)⁻.

### Beispiel 186A

### Ethyl-3-{4-[(3-methyl-2-{4-[(5-oxo-2-phenyl-5,6-dihydro-4H-1,3,4-oxadiazin-4-yl)methyl]-phenyl }butanoyl)amino]phenyl}propanoat

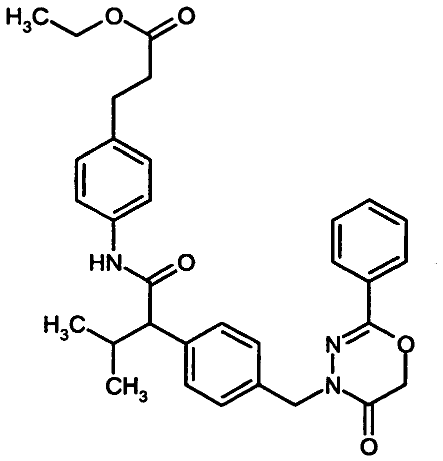

41 mg (0.076 mmol) Ethyl-(2*E*)-3-{4-[(3-methyl-2-{4-[(5-oxo-2-phenyl-5,6-dihydro-4*H*-1,3,4-oxadiazin-4-yl)methyl]phenyl}butanoyl)amino]phenyl}prop-2-enoat (Beispiel 176A) wurden in 17 ml Ethanol gelöst und mit 75 mg Palladium auf Kohle (10%) versetzt. Unter Wasserstoffatmosphäre wurde 2 h bei Normaldruck hydriert. Das Reaktionsgemisch wurde danach über Celite filtriert und das Filtrat im Vakuum eingeengt. Es wurden 39 mg (95% d. Th.) der Titelverbindung erhalten.
LC-MS (Methode 10): Rₜ = 2.46 min; m/z = 542 (M+H)⁺.

### Beispiel 187A

### Methyl-3-{3-[(cyclopentyl{4-[(5-oxo-2-phenyl-5,6-dihydro-4H-1,3,4-oxadiazin-4-yl)methyl]-phenyl}acetyl)amino]cyclohexyl}propanoat

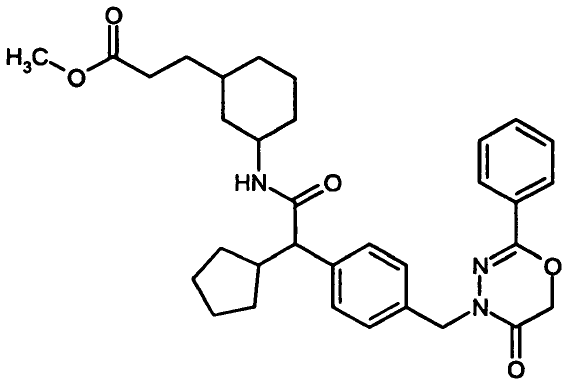

Eine Lösung von 590 mg (1.5 mmol) Cyclopentyl{4-[(5-oxo-2-phenyl-5,6-dihydro-4*H*-1,3,4-oxadiazin-4-yl)methyl]phenyl}essigsäure (Enantiomer 2; Beispiel 77A), 400 mg (1.8 mmol) Methyl-3-(3-aminocyclohexyl)propanoat-Hydrochlorid (Isomerengemisch), 1.05 ml (6.0 mmol) *N,N*-Diisopropylethylamin und 857 mg (2.26 mmol) *O*-(7-Azabenzotriazol-1-yl)-*N,N,N',N*'-tetramethyluronium-Hexafluorophosphat (HATU) in 13 ml DMF wurde über Nacht bei Raumtemperatur gerührt. Nach Beendigung der Reaktion wurde der Ansatz auf Eiswasser gegossen, wobei die Zielverbindung in Form weißer Kristalle ausfiel. Nach Filtration wurden die erhaltenen Kristalle dreimal mit Wasser gewaschen und anschließend über Nacht bei 40°C im Vakuumtrockenschrank getrocknet. Es wurden 830 mg (1.58 mol, 98% d. Th.) der Titelverbindung als Diastereomerengemisch erhalten.
LC-MS (Methode 7): Rₜ = 2.90 und 2.92 min; m/z = 560 (M+H)⁺.

### Beispiele 188A - 191A

### Methyl-3-{3-[(cyclopentyl{4-[(5-oxo-2-phenyl-5,6-dihydro-4H-1,3,4-oxadiaän-4-yl)methyl]-phenyl}acetyl)amino]cyclohexyl}propanoat (Isomer 1 - 4)

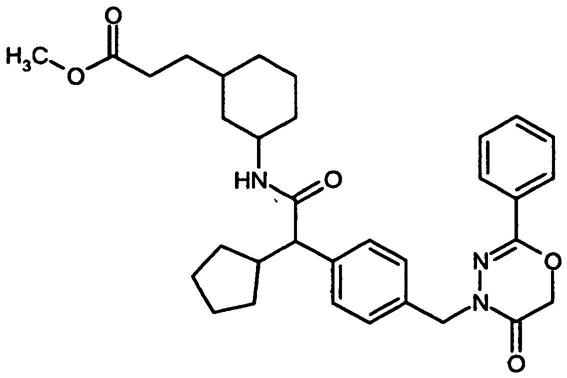

830 mg (1.58 mmol) des als Diastereomerengemisch erhaltenen Methyl-3-{3-[(cyclopentyl{4-[(5-oxo-2-phenyl-5,6-dihydro-4*H*-1,3,4-oxadiazin-4-yl)methyl]phenyl}acetyl)amino]cyclohexyl}propanoats (Beispiel 187A) wurden mittels präparativer HPLC an chiraler Phase weiter aufgetrennt [Säule: Daicel Chiralpak AD-H, 5 µm, 250 mm x 20 mm; Elutionsmittel: Isohexan/Ethanol 60:40 (v/v); Fluss: 15 ml/min; Temperatur: 40°C; UV-Detektion: 220 nm]. Es wurden vier Diastereomere in isomerenreiner Form als farblose Feststoffe erhalten (siehe Beispiele 188A - 191A).

### Beispiel 188A (Diastereomer 1):

Rₜ 6.63 min; Reinheit >95%; >99% ee (Säule s.o.)
Ausbeute: 36 mg
LC-MS (Methode 11): Rₜ = 1.50 min; m/z = 560 (M+H)⁺.

### Beispiel 189A (Diastereomer 2):

Rₜ 6.98 min; Reinheit >99%; >96% ee (Säule s.o.)
Ausbeute: 36 mg
LC-MS (Methode 11): Rₜ =1.49 min; m/z = 560 (M+H)⁺.

### Beispiel 190A (Diastereomer 3):

Rₜ 7.39 min; Reinheit >99%; >99% ee (Säule s.o.)
Ausbeute: 295 mg
LC-MS (Methode 7): Rₜ = 2.89 min; m/z = 560 (M+H)⁺.

### Beispiel 191A (Diastereomer 4):

Rₜ 8.48 min; Reinheit >99%; >99% ee
Ausbeute: 307 mg
LC-MS (Methode 7): Rₜ = 2.92 min; m/z = 560 (M+H)⁺.

### Beispiele 192A

### Methyl-(2E)-3-{2-[(3-methyl-2-{4-[(5-oxo-2-phenyl-5,6-dihydro-4H-1,3,4-oxadiazin-4-yl)methyl]-phenyl}butanoyl)amino]phenyl}prop-2-enoat

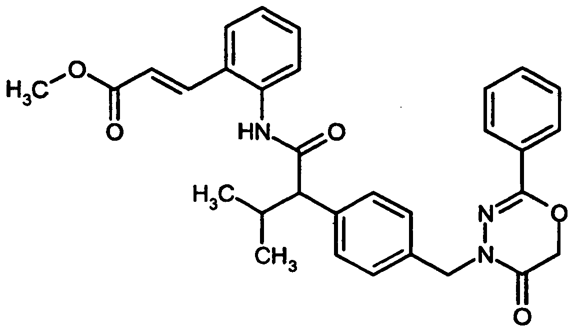

Eine Lösung von 50 mg (0.14 mmol) 3-Methyl-2-{4-[(5-oxo-2-phenyl-5,6-dihydro-4*H*-1,3,4-oxadiazin-4-yl)methyl]phenyl}butansäure (Enantiomer 2; Beispiel 79A), 20 mg (0.11 mmol) Methyl-(2*E*)-3-(2-aminophenyl)prop-2-enoat, 1.5 ml Pyridin und 65 mg (0.17 mmol) *O*-(7-Azabenzotriazol-1-yl)-*N,N,N',N*'-tetramethyluronium-Hexafluorophosphat (HATU) in 5 ml DMF wurde über Nacht bei Raumtemperatur gerührt. Nach Beendigung der Reaktion wurde der Ansatz auf Eiswasser gegossen, die Phasen getrennt und die wässrige Phase dreimal mit *tert*.-Butylmethylether extrahiert. Die vereinigten organischen Phasen wurden über Natriumsulfat getrocknet, und nach Filtration wurde das Lösungsmittel im Vakuum bis zur Trockene entfernt. Das erhaltene Rohprodukt wurde über präparative HPLC gereinigt. Es wurden 7 mg (0.01 mmol, 9.8% d. Th.) eines farblosen Öls erhalten.
LC-MS (Methode 11): Rₜ = 1.38 min; m/z = 526 (M+H)⁺.

### Beispiel 193A

### Ethyl-3-{3-[(3-methyl-2-{4-[(5-oxo-2-phenyl-5,6-dihydro-4H-1,3,4-oxadiazin-4-yl)methyl]-phenyl}butanoyl)amino]phenyl}propanoat

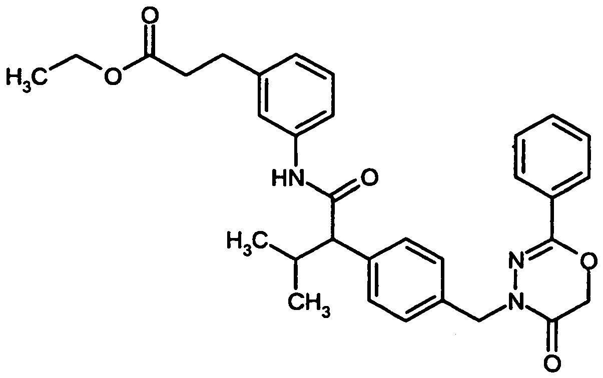

130 mg (0.24 mmol) Ethyl-(2*E*)-3-{3-[(3-methyl-2-{4-[(5-oxo-2-phenyl-5,6-dihydro-4H-1,3,4-oxadiazin-4-yl)methyl]phenyl}butanoyl)amino]phenyl}prop-2-enoat (Beispiel 135A) wurden in 10 ml Ethanol gelöst und mit 10 mg Palladium auf Kohle (10%) versetzt. Unter Wasserstoffatmosphäre wurde über Nacht bei Normaldruck hydriert. Das Reaktionsgemisch wurde dann über Celite filtriert und das Filtrat im Vakuum eingeengt. Es wurden 118 mg (0.22 mmol, 90% d. Th.) der Titelverbindung erhalten.
LC-MS (Methode 11): Rₜ = 1.48 min; m/z = 542 (M+H)⁺.

### Beispiel 194A

### (+/-)-{4-[(Acetyloxy)methyl]phenyl}(cyclopentyl)essigsäure-tert.-butylester

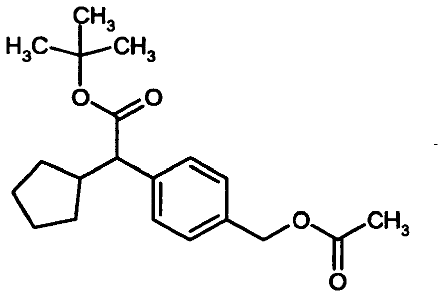

Zu einer Suspension von 16.3 g (84.9 mmol) Cäsiumacetat in 80 ml DMF wurden 20.0 g (75%-ig, 42.5 mmol) (+/-)-[4-(Brommethyl)phenyl](cyclopentyl)essigsäure-*tert*.-butylester gegeben. Die resultierende Mischung wurde für 1.5 h auf 50°C erhitzt. Nach Abkühlen wurde die Mischung mit Ethylacetat verdünnt, nacheinander mit Wasser und gesättigter Natriumchlorid-Lösung gewaschen, über Magnesiumsulfat getrocknet und im Vakuum eingeengt. Das Rohprodukt wurde durch Chromatographie an Silicagel gereinigt (Laufmittel Cyclohexan/Ethylacetat 40:1 → ca. 10:1). Es wurden 11.7 g (76.4% d. Th.) der Titelverbindung erhalten.
GC-MS (Methode 3): Rₜ = 7.16 min; keine Ionisation.
MS (DCI): m/z = 350 (M+NH₄)⁺.
¹H-NMR (400 MHz, DMSO-*d*₆): δ = 7.35-7.29 (m, 4H), 5.07 (s, 2H), 3.21 (d, 1H), 2.45-2.37 (m, 1H), 2.07 (s, 3H), 1.98-1.88 (m, 1H), 1.65-1.38 (m, ca. 4H), 1.37 (s, 9H), 1.30-1.18 (m, 2H), 1.01-0.92 (m, 1H).

### Beispiel 195A

### (+/-)-{4-[(Acetyloxy)methyl]phenyl}(cyclopentyl)essigsäure

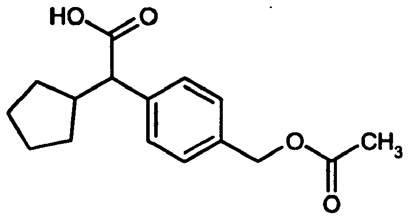

13.27 g (37.2 mmol, 95%-ig) (+/-)-{4-[(Acetyloxy)methyl]phenyl}(cyclopentyl)essigsäure-*tert*.-butylester wurden in 117 ml Dichlormethan gelöst, auf 0°C abgekühlt und mit 58.4 ml Trifluoressigsäure versetzt. Die Reaktionsmischung wurde zunächst 1.5 h bei 0°C und dann weitere 1.5 h bei RT gerührt. Die Mischung wurde anschließend im Vakuum eingeengt und der Rückstand im Hochvakuum getrocknet. Der Rückstand wurde in 50 ml Dichlormethan aufgenommen und die Lösung viermal mit Wasser gewaschen. Die organische Phase wurde über Magnesiumsulfat getrocknet und im Vakuum eingeengt. Nach Trocknen im Hochvakuum wurden 11.41 g der Titelverbindung erhalten, welche ohne weitere Reinigung in der Folgereaktion eingesetzt wurde.
LC-MS (Methode 10): Rₜ = 1.88 min; m/z = 275 (M-H)⁻.
¹H-NMR (400 MHz, DMSO-*d*₆): δ = 7.35-7.28 (m, 4H), 5.03 (s, 2H), 3.24 (d, 1H), 2.48-2.40 (m, 1H), 2.05 (s, 3H), 1.90-1.80 (m, 1H), 1.65-1.20 (m, ca. 6H), 0.98-0.90 (m, 1H).

### Beispiele 196A

### (+/-)-3-(3-{[{4-[(Acetyloxy)methyl]phenyl}(cyclopentyl)acetyl]amino}-2-methylphenyl)propansäure-tert.-butylester

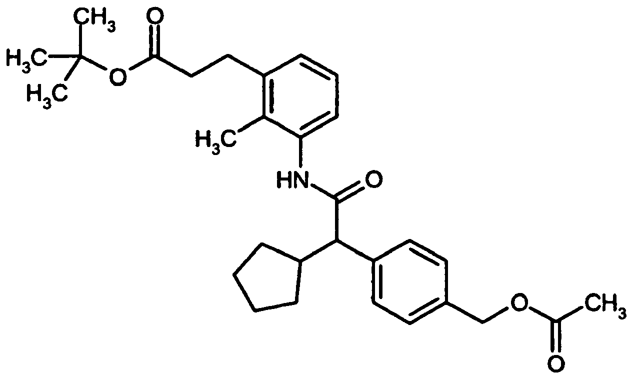

11.41 g (90%-ig, 37.16 mmol) (+/-)-{4-[(Acetyloxy)methyl]phenyl}(cyclopentyl)essigsäure wurden in einer Mischung aus 71.8 ml DMF und 22.5 ml Pyridin gelöst und mit 15.54 g (40.88 mmol) 1-[Bis-(dimethylamino)methylen]-5-chlor-3-oxy-1*H*-benzotriazol-1-ium-Tetrafluoroborat sowie 8.75 g (37.16 mmol) 3-(3-Amino-2-methylphenyl)propansäure-*tert*.butylester versetzt. Die Reaktionsmischung wurde über Nacht bei RT gerührt, bevor im Hochvakuum eingeengt wurde. Der Rückstand wurde in 100 ml Ethylacetat aufgenommen und nacheinander mit 10%-iger wässriger Zitronensäure, gesättigter Natriumhydrogensulfat-Lösung und gesättigter Natriumchlorid-Lösung gewaschen. Die organische Phase wurde über Magnesiumsulfat getrocknet und im Vakuum eingeengt. Das Rohprodukt wurde durch Chromatographie an Silicagel gereinigt (Laufmittel Cyclohexan/Ethylacetat 10:1 → 3:1). Es wurden 16.36 g (89.2% d. Th.) der Titelverbindung erhalten.
LC-MS (Methode 11): Rₜ = 1.52 min; m/z = 511 (M+NH₄)⁺.
¹H-NMR (400 MHz, DMSO-*d*₆): δ = 9.98 (s, 1H), 7.42 (d, 2H), 7.32 (d, 2H), 7.05-6.96 (m, 3H), 5.05 (s, 2H), 3.48 (d, 1H), 2.70 (t, 2H), 2.64-2.55 (m, 1H), 2.42 (t, 2H), 2.08 (s, 3H), 2.02 (s, 3H), 1.90-1.80 (m, 1H), 1.72-1.45 (m, ca. 5H), 1.38 (s, 9H), 1.02-0.95 (m, 1H).

### Beispiel 197A

### (+/-)-3-[3-({Cyclopentyl[4-(hydroxymethyl)phenyl]acetyl}amino)-2-methylphenyl]propansäure-tert.-butylester

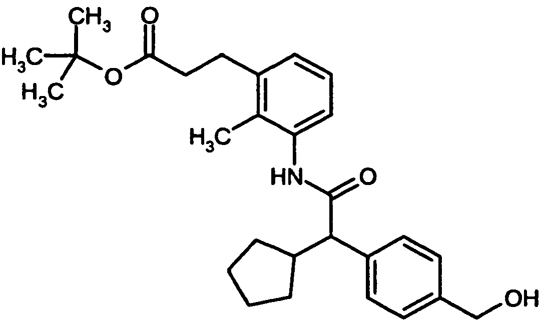

16.0 g (32.41 mmol) (+/-)-3-(3-{[{4-[(Acetyloxy)methyl]phenyl}(cyclopentyl)acetyl]amino}-2-methylphenyl)propansäure-*tert*.-butylester wurden in 300 ml einer 2 M Lösung von Ammoniak in Methanol gelöst und zunächst 2 h bei 30-40°C und dann über Nacht bei RT gerührt. Die Lösung wurde danach im Vakuum eingeengt und der Rückstand durch Chromatographie an Silicagel gereinigt (Laufmittel Dichlormethan/Methanol 100:1). Es wurden 13.6 g (93.1 % d. Th.) der Titelverbindung erhalten.
LC-MS (Methode 11): Rₜ = 1.37 min; m/z = 452 (M+H)⁺.
¹H-NMR (400 MHz, DMSO-*d*₆): δ = 9.95 (s, 1H), 7.37 (d, 2H), 7.27 (d, 2H), 7.05-6.96 (m, 3H), 5.13 (t, 1H), 4.47 (d, 2H), 3.45 (d, 1H), 2.79 (t, 2H), 2.62-2.52 (m, 1H), 2.42 (t, 2H), 2.01 (s, 3H), 1.90-1.80 (m, 1H), 1.73-1.40 (m, 4H), 1.38 (s, 9H), 1.02-0.95 (m, 1H).

### Beispiel 198A

### (+/-)-3-[3-({[4-(Brommethyl)phenyl](cyclopentyl)acetyl} amino)-2-methylphenyl]propansäure-tert.-butylester

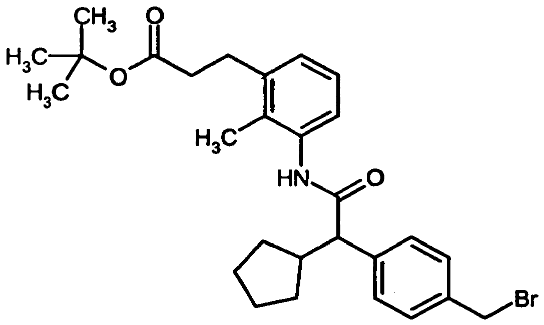

6.0 g (13.29 mmol) (+/-)-3-[3-({Cyclopentyl[4-(hydroxymethyl)phenyl]acetyl}amino)-2-methylphenyl]propansäure-*tert*.-butylester wurden in 375 ml trockenem THF gelöst und mit 9.25 g (27.9 mmol) Tetrabromkohlenstoff versetzt. Anschließend wurden über 1.5 h in kleineren Portionen 9.06 g (34.54 mmol) Triphenylphosphin hinzugefügt. Die Reaktionsmischung wurde 5 h bei RT gerührt, bevor vom Feststoff abfiltriert und das Filtrat eingeengt wurde. Der Rückstand wurde durch Chromatographie an Silicagel gereinigt (Laufmittel Cyclohexan/Ethylacetat 5:1 → 1:1). Es wurden 7.22 g (ca. 100% d. Th. bei ca. 95% Reinheit) der Titelverbindung erhalten.
LC-MS (Methode 7): Rₜ = 3.07 min; m/z= 514/516 (M+H)⁺.
¹H-NMR (400 MHz, DMSO-*d*₆): δ = 9.99 (s, 1H), 7.42-7.38 (m, 4H), 7.05-6.95 (m, 3H), 4.70 (d, 2H), 3.48 (d, 1H), 3.30-3.25 (m, ca. 1H), 2.70 (t, 2H), 2.64-2.53 (m, 1H), 2.41 (t, 2H), 2.01 (s, 3H), 1.90-1.80 (m, 1H), 1.73-1.40 (m, 4H), 1.35 (s, 9H), 1.02-0.93 (m, 1H).

### Beispiel 199A

### (1R,2S,SR)-5-Methyl-2-(propan-2-yl)cyclohexyl-(4-methylphenyl)acetat

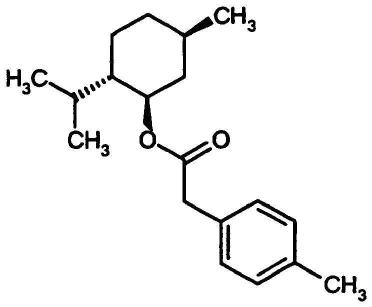

303.7 g (2022.46 mmol) (4-Methylphenyl)essigsäure und 301 g (1926.2 mmol) (1*R*,2*S*,5*R*)-5-Methyl-2-(propan-2-yl)cyclohexanol wurden in 933 ml Toluol vorgelegt, mit 2.5 ml (38.5 mmol) Methansulfonsäure versetzt und über Nacht unter Rückfluss an einem Wasserabscheider erhitzt. Man ließ danach abkühlen und versetzte die Reaktionslösung mit einer Mischung aus 30 ml 45%-iger Natronlauge und 400 ml Wasser. Nach 30 min wurden die Phasen getrennt. Die organische Phase wurde über Natriumsulfat getrocknet, filtriert und am Rotationsverdampfer eingeengt. Es wurden 569.5 g (97% d. Th.) der Titelverbindung erhalten.
¹H-NMR (500 MHz, DMSO-*d*₆): δ = 7.12 (s, 4H), 4.56 (td, 1H), 3.57 (s, 2H), 2.50 (br. s, 1H), 2.27 (s, 3H), 1.84 (d, 1H), 1.77-1.70 (m, 1H), 1.66-1.57 (m, 2H), 1.48-1.37 (m, 1H), 1.32 (t, 1H), 1.10-0.89 (m, 2H), 0.86 (d, 3H), 0.81 (d, 3H), 0.65 (d, 3H).

### Beispiel 200A

### (1R,2S,5R)-5-Methyl-2-(propan-2-yl)cyclohexyl-(2S)-cyclopentyl(4-methylphenyl)ethanoat

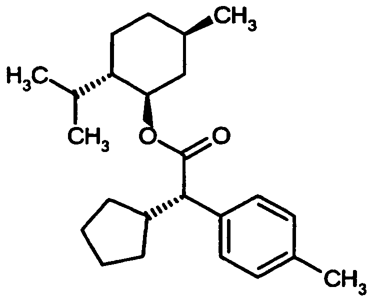

Unter Argon wurden 442.73 g (3945.5 mmol) Kalium-*tert*: butylat in 1230 ml DMF bei -10°C vorgelegt und portionsweise mit 569 g (1972.7 mmol) (1*R*,2*S*,5*R*)-5-Methyl-2-(propan-2-yl)cyclohexyl-(4-methylphenyl)acetat versetzt. Anschließend wurden 352.81 g (2367.8 mmol) Bromcyclopentan zugetropft, wobei die Reaktionstemperatur zwischen -5°C und -10°C gehalten wurde. Nach 90 min bei -10°C wurde mit 1.6 Liter Wasser versetzt und 15 min bei RT gerührt. Es wurden dann 1.2 Liter Essigsäureethylester zugegeben, weitere 15 min gerührt und anschließend die Phasen getrennt. Die organische Phase wurde über Natriumsulfat getrocknet, filtriert und am Rotationsverdampfer eingeengt. Das Rohprodukt wurde aus 2 Liter Methanol bei 50°C umkristallisiert. Es wurden 423.0 g (60% d. Th.) der Titelverbindung erhalten.
¹H-NMR (400 MHz, DMSO-*d*₆): δ = 7.19 (d, 2H), 7.11 (d, 2H), 4.55 (td, 1H), 3.26 (d, 1H), 2.27 (s, 3H), 1.83-1.73 (m, 2H), 1.68-1.24 (m, 11H), 1.23-1.13 (m, 1H), 1.04-0.94 (m, 2H), 0.88-0.77 (m, 8H), 0.66 (d, 3H).

### Beispiel 201A

### (1R,2S,5R)-5-Methyl-2-(propan-2-yl)cyclohexyl-(2S)-[4-(brommethyl)phenyl](cyclopentyl)-ethanoat

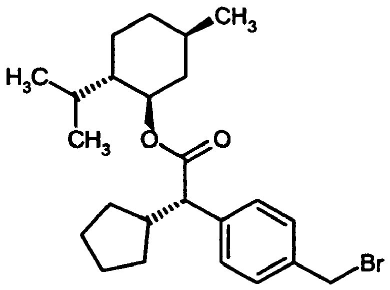

Die Titelverbindung kann gemäß US-Patent 5,714,494 durch Bromierung von (1*R*,2*S*,5*R*)-5-Methyl-2-(propan-2-yl)cyclohexyl-(2*S*)-cyclopentyl(4-methylphenyl)ethanoat in siedendem Tetrachlorkohlenstoff mit *N*-Bromsuccinimid in Gegenwart von 2,2'-Azobis-(2-methylpropionitril) hergestellt werden.
GC-MS (Methode 3): Rₜ = 9.15 min; keine Ionisation.
LC-MS (Methode 12): Rₜ = 3.54 min; keine Ionisation.
MS (DCI): m/z = 452/454 (M+NH₄)⁺.

### Beispiel 202A

### (2S)-Cyclopentyl-(4-methylphenyl)essigsäure

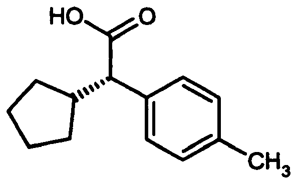

50.0 g (0.140 mol) (1*R*,2*S*,5*R*)-5-Methyl-2-(propan-2-yl)cyclohexyl-(2*S*)-cyclopentyl(4-methylphenyl)ethanoat wurden 16 h lang in 500 ml Trifluoressigsäure unter Rückfluss gerührt. Die Trifluoressigsäure wurde danach am Rotationsverdampfer entfernt und der Rückstand in je 500 ml Wasser und Essigsäureethylester aufgenommen. Nach Extraktion wurde die organische Phase mit gesättigter Natriumchlorid-Lösung gewaschen, über Natriumsulfat getrocknet und eingeengt. Der Rückstand wurde in 1.5 Liter Wasser aufgenommen, mit Natronlauge auf pH 10 eingestellt und zweimal mit je 300 ml *tert*.-Butylmethylether gewaschen. Die wässrige Phase wurde dann mit konzentrierter Salzsäure auf pH 4 eingestellt und zweimal mit je 300 ml Essigsäureethylester extrahiert. Die vereinigten Ethylacetat-Extrakte wurden über Natriumsulfat getrocknet, filtriert und am Rotationsverdampfer eingeengt. Es wurden 27.4 g (89% d. Th.) der Titelverbindung erhalten.
LC-MS (Methode 11): Rₜ = 1.28 min; m/z = 217 (M-H)⁻.
¹H-NMR (400 MHz, DMSO-*d*₆): δ = 7.19 (d, 2H), 7.13-7.08 (m, 2H), 3.16 (d, 1H), 2.41 (dt, 1H), 2.26 (s, 3H), 1.87-1.76 (m, 1H), 1.66-1.14 (m, 6H), 1.00-0.87 (m, 1H).

### Beispiel 203A

### (2S)-Cyclopentyl-(4-methylphenyl)essigsäure-tert-.-butylester

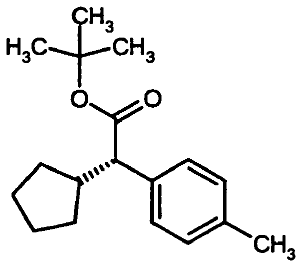

160 g (732.9 mmol) (2*S*)-Cyclopentyl-(4-methylphenyl)essigsäure wurden in 480 ml Dichlormethan vorgelegt und bei 10°C mit 2.1 ml Schwefelsäure und 172 ml (1722.4 mmol) einkondensiertem 2-Methylprop-1-en versetzt. Die Mischung wurde über Nacht bei RT gerührt. Falls erforderlich, wurde die Zugabe von Schwefelsäure und 2-Methylprop-1-en wiederholt, bis kein Edukt mehr vorhanden war. Nach Beendigung der Reaktion wurden 21 g Kaliumcarbonat zugesetzt und die Mischung 2-3 h nachgerührt (Gasentwicklung). Man verdünnte dann mit 700 ml Wasser und extrahierte mit Essigsäureethylester. Die organische Phase wurde über Natriumsulfat getrocknet, filtriert und am Rotationsverdampfer eingeengt. Das Rohprodukt wurde durch Chromatographie an Silicagel gereinigt (Laufmittel Cyclohexan/Essigsäureethylester 4:1). Es wurden 172 g (85% d. Th.) der Titelverbindung erhalten.
¹H-NMR (400 MHz, DMSO-*d*₆): δ = 7.19 (d, 2H), 7.11 (d, 2H), 3.13 (d, 1H), 2.44-2.31 (m, 1H), 2.27 (s, 3H), 1.86-1.76 (m, 1H), 1.68-1.36 (m, 4H), 1.34 (s, 9H), 1.31-1.17 (m, 2H), 1.00-0.89 (m, 1H).

### Beispiel 204A

### (2S)-[4-(Brommethyl)phenyl](cyclopentyl)essigsäure-tert.-butylester

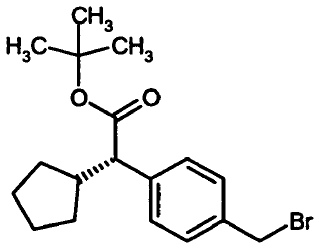

3.0 g (10.93 mmol) (2*S*)-Cyclopentyl-(4-methylphenyl)essigsäure-*tert*.-butylester wurden in 20 ml Chloroform auf Rückfluss erhitzt und dann mit 90 mg (0.55 mmol) 2,2'-Azobis-(2-methylpropannitril) sowie 2.72 g (15.31 mmol) N-Bromsuccinimid, in 5 Portionen alle 30 min, versetzt. Es wurde 6 h unter Rückfluss gerührt, dann auf RT abgekühlt und am Rotationsverdampfer eingeengt. Der Rückstand wurde durch Chromatographie an 120 ml Silicagel gereinigt (Laufmittel Cyclohexan/Essigsäureethylester 4:1). Es wurden 2.8 g (72% d. Th.) der Titelverbindung erhalten.
¹H-NMR (400 MHz, DMSO-*d*₆): δ = 7.39 (d, 2H), 7.31 (d, 2H), 4.68 (s, 2H), 2.45-2.32 (m, 1H), 1.89-1.77 (m, 1H), 1.69-1.38 (m, 5H), 1.37-1.32 (m, 9H), 1.30-1.16 (m, 2H), 1.00-0.92 (m, 1H).

### Beispiel 205A

### Ethyl-4,4,4-trifluor-3-methyl-2-(4-methylphenyl)butanoat

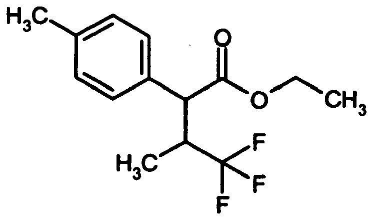

Unter Argon wurden 196.9 mg (0.88 mmol) Palladium(II)acetat und 724.8 mg (1.84 mmol) 2-Dicyclohexylphosphino-2'-(*N,N*-dimethylamino)biphenyl in 50 ml wasserfreiem Toluol vorgelegt. Die Reaktionslösung wurde anschließend langsam mit 43.8 ml (43.8 mmol) einer 1 M Lösung von Lithiumhexamethyldisilazid in THF versetzt und 10 min bei Raumtemperatur gerührt. Nachfolgend wurde die Reaktionslösung auf -10°C abgekühlt, langsam mit 7 g (38.0 mmol) Ethyl-4,4,4-trifluor-3-methylbutanoat versetzt und 10 min bei -10°C nachgerührt. Danach wurden 5 g (29.2 mmol) 4-Bromtoluol, gelöst in 50 ml Toluol, zugetropft und die Reaktionslösung erst auf Raumtemperatur und dann auf 80°C erwärmt. Das Gemisch wurde 2 h bei dieser Temperatur gerührt, dann auf Raumtemperatur abgekühlt und über Nacht nachgerührt. Nach erfolgter Umsetzung (DC-Kontrolle; Laufmittel Cyclohexan/Dichlormethan 2: 1) wurde die Reaktionsmischung über Kieselgur filtriert, der Rückstand mehrfach mit Essigsäureethylester und Dichlormethan gewaschen und die vereinigten Filtrate im Vakuum eingeengt. Das erhaltene Rohprodukt wurde über Kieselgel chromatographisch gereinigt (Laufmittel Petrolether/Dichlormethan 4:1 → 3:1). Es wurden 3.91 g (14.3 mmol, 48.8% d. Th.) der Titelverbindung als farblose Flüssigkeit isoliert.
¹H-NMR (400 MHz, DMSO-d₆, δ/ppm): 7.26 (2H, d), 7.20-7.12 (2H, m), 4.17-3.95 (2H, m), 3.74 (0.25H, d), 3.66 (0.75H, d), 3.35-3.07 (1H, m), 2.29 (2.25H, s), 2.28 (0.75H, s), 1.17 (0.75H, d), 1.11 (3H, t), 0.76 (2.25H, d) (Diastereomerengemisch).
GC-MS (Methode 3): Rₜ = 4.20 min; m/z = 275 (M+H)⁺ (Diastereomer 1); Rₜ = 4.23 min; m/z = 275 (M+H)⁺ (Diastereomer 2).

Auf analoge Weise wurde die in der folgenden Tabelle aufgeführte Verbindung erhalten:

| **Beispiel** | **Name / Struktur** | **Analytische Daten** |
|---|---|---|
| **206A** | Ethyl-2-(4-methylphenyl)-3-(trifluormethyl)pentanoat | GC-MS (Methode 3): Rₜ = 4.57 min; m/z = 288 (M+H)⁺. |
| | 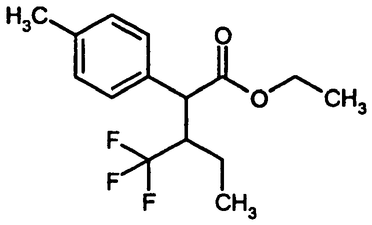 | |
| | (aus 4-Bromtoluol und Ethyl-3-(trifluormethyl)pentanoat [CAS Reg.-Nr. 400-25-9]) | |

### Beispiel 207A

### tert.-Butyl-6,6,6-trifluor-4-methyl-2-(4-methylphenyl)hexanoat

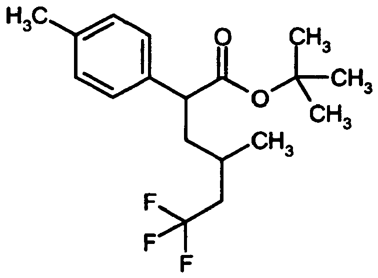

Unter Sauerstoffausschluss wurden 0.44 ml (3.2 mmol) Diisopropylamin in 4 ml THF vorgelegt, auf -78°C abgekühlt und langsam mit 1.28 ml (3.2 mmol) einer 2.5 M Lösung von n-Butyllithium in Hexan versetzt. Anschließend wurde die Reaktionslösung auf -10°C erwärmt und 10 min bei dieser Temperatur nachgerührt. Die Reaktionslösung wurde dann wieder auf -78°C abgekühlt und langsam mit 500 mg (2.32 mmol) *tert*.-Butyl-(4-methylphenyl)acetat, gelöst in 6 ml THF, versetzt. Die Reaktionslösung wurde anschließend langsam auf -30°C erwärmt und danach erneut auf -78°C abgekühlt. Nach Erreichen dieser Temperatur wurden 596 mg (2.91 mmol) 5-Brom-1,1,1-trifluor-3-methylpentan langsam zugetropft. Nach beendeter Zugabe wurde die Lösung langsam auf Raumtemperatur erwärmt und über Nacht gerührt. Nach DC-Kontrolle (Laufmittel Cyclohexan/ Essigsäureethylester 10:1) wurde der Ansatz mit gesättigter Ammoniumchlorid-Lösung versetzt und in Essigsäureethylester aufgenommen. Die wässrige Phase wurde zweimal mit Essigsäureethylester extrahiert. Die vereinigten organischen Phasen wurden über Natriumsulfat getrocknet. Nach Filtration wurde das Lösungsmittel im Vakuum entfernt. Das Rohprodukt wurde über Kieselgel chromatographisch gereinigt (Laufmittel Cyclohexan/Essigsäureethylester 20:1). Es wurden 490 mg (1.48 mmol, 61% d. Th.) eines gelblichen Öls isoliert.
GC-MS (Methode 3): Rₜ = 4.89 min; m/z = 274 (M+H)⁺.

### Beispiel 208A

### 4,4,4-Trifluor-2-methyl-1-(4-methylphenyl)butan-1-on

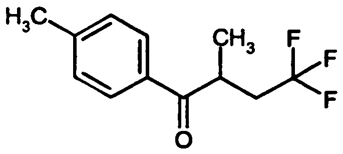

12.6 ml (118.6 mmol) Toluol und 20.9 g (119.7 mmol) 4,4,4-Trifluor-2-methylbutanoylchlorid in 300 ml 1,2-Dichlorethan wurden bei 0°C portionsweise mit 16.44 g (123.3 mmol) Aluminiumchlorid versetzt und eine Stunde bei 0°C gerührt. Anschließend wurde der Reaktionsansatz langsam auf Raumtemperatur erwärmt und drei Stunden bei dieser Temperatur nachgerührt. Die Reaktionsmischung wurde danach langsam in 300 ml eisgekühlte 18.5%-ige Salzsäure gegeben und die organische Phase anschließend abgetrennt. Die wässrige Phase wurde noch dreimal mit Dichlormethan extrahiert. Die vereinigten organischen Phasen wurden über Natriumsulfat getrocknet, filtriert und das Lösungsmittel im Vakuum entfernt. Das Rohprodukt wurde über Kieselgel chromatographisch gereinigt (Laufmittel Cyclohexan/Essigsäureethylester 4:1). Es wurden 19.96 g (18.7 mmol, 73% d. Th.) der Titelverbindung erhalten.
LC-MS (Methode 11): Rₜ = 1.35 min; m/z = 228 (M-H)⁻.
¹H-NMR (400 MHz, DMSO-d₆): δ = 7.92 (d, 2H), 7.36 (d, 2H), 3.82-3.98 (m, 1H), 2.70-2.92 (m, 1H), 2.40-2.49 (m, 1H), 2.36-2.40 (m, 3H), 1.18 (d, 3H).

### Beispiel 209A

### 1-Methyl-4-(5,5,5-trifluor-3-methylpent-1-en-2-yl)benzol

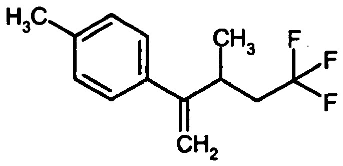

Unter Argon wurden 1.45 ml (4.34 mmol) einer 3 M Lösung von Methylmagnesiumiodid in Diethylether in 10 ml Diethylether vorgelegt und bei 0°C langsam mit 1000 mg (4.34 mmol) 4,4,4-Trifluor-2-methyl-1-(4-methylphenyl)butan-1-on, gelöst in 5 ml Diethylether, versetzt. Anschlie-βend wurde das Reaktionsgemisch langsam auf Raumtemperatur erwärmt und über Nacht nachgerührt. Die Reaktionslösung wurde danach langsam in 15 ml 1 M Salzsäure gegeben und mit Diethylether verdünnt. Nach Abtrennung der organischen Phase wurde die wässrige Phase noch dreimal mit Diethylether extrahiert. Die vereinigten organischen Phasen wurden über Magnesiumsulfat getrocknet, filtriert und das Lösungsmittel im Vakuum entfernt. Das Rohprodukt wurde über Kieselgel chromatographisch gereinigt (Laufmittel Cyclohexan/Essigsäureethylester 50:1). Es wurden 710 mg (3.11 mmol, 71% d. Th.) der Titelverbindung als farbloses Öl erhalten.
GC-MS (Methode 3): Rₜ = 3.23 min; m/z = 228 (M)⁺.

### Beispiel 210A

### 5,5,5-Trifluor-3-methyl-2-(4-methylphenyl)pentan-1-ol

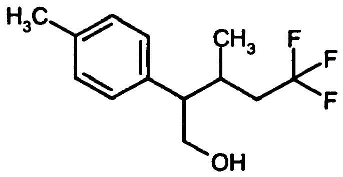

Unter Argon wurden 9.4 g (41.2 mmol) 1-Methyl-4-(5,5,5-trifluor-3-methylpent-1-en-2-yl)benzol in 130 ml THF gelöst, mit 57.6 ml einer 1 M Boran-THF-Komplex-Lösung versetzt und 24 h bei Raumtemperatur gerührt. Anschließend wurden unter kräftigen Rühren zunächst 79 ml Wasser, dann 24 ml 6 M Natronlauge und schließlich 14.5 ml (475 mmol) 30%-ige wässrige Wasserstoffperoxid-Lösung zugegeben. Darauf folgend wurde die Reaktionslösung in 200 ml gesättigte Natriumchlorid-Lösung gegeben, die organische Phase abgetrennt und die wässrige Phase noch dreimal mit Diethylether extrahiert. Die vereinigten organischen Phasen wurden über Magnesiumsulfat getrocknet, filtriert und das Lösungsmittel im Vakuum entfernt. Das Rohprodukt wurde über Kieselgel chromatographisch gereinigt (Laufmittel Cyclohexan/Essigsäureethylester 4:1 → 2:1). Es wurden 5 g (20.3 mmol, 49% d. Th.) der Titelverbindung als farbloses Öl erhalten.
GC-MS (Methode 3): Rₜ = 4.45 min; m/z = 246 (M)⁺ (Diastereomer 1); Rₜ = 4.54 min; m/z = 246 (M)⁺ (Diastereomer 2).

### Beispiel 211A

### 5,5,5-Trifluor-3-methyl-2-(4-methylphenyl)pentansäure

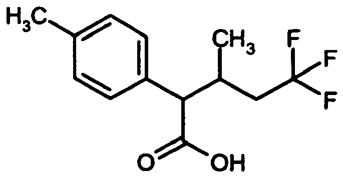

5 g (20.3 mmol) 5,5,5-Trifluor-3-methyl-2-(4-methylphenyl)pentan-1-ol in 83 ml DMF wurden mit 26.7 g (71.1 mmol) Pyridiniumdichromat versetzt und 12 Stunden bei Raumtemperatur gerührt. Darauf folgend wurde die Reaktionsmischung in 100 ml Wasser gegeben, die organische Phase abgetrennt und die wässrige Phase noch sechsmal mit Diethylether extrahiert. Die vereinigten organischen Phasen wurden fünfmal mit 0.5 M Natronlauge extrahiert. Anschließend wurden die vereinigten wässrig-basischen Phasen mit 3 M Salzsäure auf ca. pH 3 angesäuert und sechsmal mit Essigsäureethylester extrahiert. Die vereinigten Essigester-Phasen wurden über Magnesiumsulfat getrocknet, filtriert und das Lösungsmittel im Vakuum entfernt. Es wurden 3.79 g (14.56 mmol, 71 % d. Th.) der Titelverbindung als farbloses Öl erhalten.
LC-MS (Methode 15): Rₜ = 1.09 min; m/z = 259 (M-H)⁻ (Diastereomer 1); Rₜ = 1.12 min; m/z = 259 (M-H)⁻ (Diastereomer 2).

### Beispiele 212A

### tert.-Butyl-5,5,5-trifluor-3-methyl-2-(4-methylphenyl)pentanoat

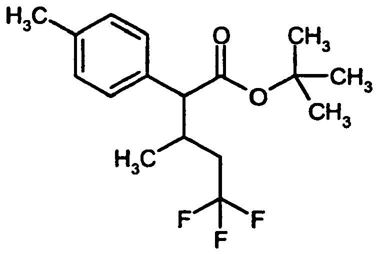

Unter Argon wurden bei Raumtemperatur 3.79 g (14.5 mmol) 5,5,5-Trifluor-3-methyl-2-(4-methylphenyl)pentansäure in 60 ml THF gelöst und mit 27 µl (0.22 mmol) einer 1 M Bortrifluorid-Diethylether-Komplex-Lösung versetzt. Anschließend wurden portionsweise 3.828 g (17.48 mmol) *tert*.-Butyl-2,2,2-trichlorethanimidoat zudosiert und die Mischung über Nacht bei Raumtemperatur gerührt. Nach vollständiger Umsetzung (DC-Kontrolle; Laufmittel Cyclohexan/ Essigsäureethylester 2:1) wurden unter kräftigern Rühren der Reaktionslösung 2 g festes Natriumhydrogencarbonat zugegeben. Die Reaktionsmischung wurde danach filtriert und das Lösungsmittel im Vakuum entfernt. Das Rohprodukt wurde über Kieselgel chromatographisch gereinigt (Laufmittel Cyclohexan/Essigsäureethylester 5:1). Es wurden 3.25 g (10.27 mmol, 71% d. Th.) der Titelverbindung als farbloses Öl erhalten.
MS (DCI): m/z = 334 (M+NH₄)⁺.
GC-MS (Methode 3): Rₜ = 4.49 min; m/z = 260 (M-C₄H₈)⁺ (Diastereomer 1); Rₜ = 4.52 min; m/z = 260 (M-C₄H₈)⁺ (Diastereomer 2).

### Beispiel 213A

### tert.-Butyl-(4-methylphenyl)(3-oxocyclopentyl)acetat

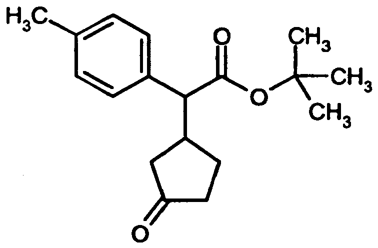

Unter Argon wurden 4.4 ml (31.5 mmol) Diisopropylamin in 50 ml THF vorgelegt, auf -30°C abgekühlt und langsam mit 13.7 ml (31.5 mmol) einer 2.3 M Lösung von n-Butyllithium in Hexan versetzt. Anschließend wurde die Reaktionslösung auf -20°C erwärmt, langsam mit 5 g (24.3 mmol) *tert*.-Butyl-(4-methylphenyl)acetat, gelöst in 30 ml THF, versetzt und 2 Stunden bei dieser Temperatur nachgerührt. Die Reaktionslösung wurde anschließend auf -78°C abgekühlt und langsam mit 2.2 ml (25.7 mmol) 2-Cyclopenten-1-on, gelöst in 20 ml THF, versetzt. Nach beendeter Zugabe wurde die Lösung eine Stunde bei dieser Temperatur nachgerührt. Nach DC-Kontrolle (Laufmittel Cyclohexan/Essigsäureethylester 9:1) wurde der Ansatz mit gesättigter Ammoniumchlorid-Lösung versetzt und in Essigsäureethylester aufgenommen. Die wässrige Phase wurde zweimal mit Essigsäureethylester extrahiert. Die vereinigten organischen Phasen wurden über Natriumsulfat getrocknet. Nach Filtration wurde das Lösungsmittel im Vakuum entfernt. Das Rohprodukt wurde über Kieselgel chromatographisch gereinigt (Laufmittel Cyclohexan/Essigsäureethylester 9:1). Es wurden 3020 mg (10.47 mmol, 43% d. Th.) der Titelverbindung als farbloses Öl isoliert.
MS (DCI): m/z = 306 (M+NH₄)⁺.
¹H-NMR (500 MHz, DMSO-d₆): δ = 7.20 (t, 2H), 7.14 (t, 2H), 3.38 (t, 1H), 2.66-2.80 (m, 1H), 2.30-2.39 (m, 1H), 2.28 (d, 3H), 1.92-2.23 (m, 3H), 1.72-1.79 (m, 1H), 1.51-1.66 (m, 1H), 1.35 (d, 9H).

### Beispiel 214A

### tert.-Butyl-(3,3-difluorcyclopentyl)(4-methylphenyl)acetat

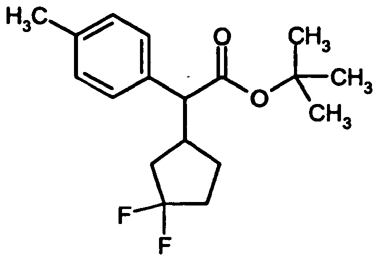

Unter Argon wurden 4.8 ml (4.85 mmol) einer 50%-igen Lösung von 1,1'-[(Trifluor-λ⁴-sulfanyl)-imino]bis(2-methoxyethan) (Desoxofluor) in THF, gelöst in 5 ml Toluol, vorgelegt, auf 5°C abgekühlt, langsam mit 22 µl (0.17 mmol) einer 1 M Bortrifluorid-Diethylether-Komplex-Lösung versetzt und zwei Stunden bei 5°C nachgerührt. Anschließend wurde die Reaktionslösung langsam mit 1 g (3.45 mmol) *tert*.-Butyl-(4-methylphenyl)(3-oxocyclopentyl)acetat, gelöst in 5 ml Toluol, versetzt, dann auf 55°C erwärmt und 24 Stunden bei dieser Temperatur nachgerührt. Die Reaktionsmischung wurde danach in ein auf 0°C gekühltes Gemisch bestehend aus 5 ml Toluol und 10 ml 2 M Natronlauge gegeben. Die organische Phase wurde abgetrennt, und die wässrige Phase wurde noch zweimal mit Essigsäureethylester extrahiert. Die vereinigten organischen Phasen wurden über Natriumsulfat getrocknet. Nach Filtration wurde das Lösungsmittel im Vakuum entfernt. Das Rohprodukt wurde über Kieselgel chromatographisch gereinigt (Laufmittel Cyclohexan/ Essigsäureethylester 7:1). Es wurden 701 mg (2.26 mmol, 65% d. Th.) der Titelverbindung als farbloses Öl isoliert.
MS (DCI): m/z = 328 (M+NH₄)⁺.
GC-MS (Methode 3): Rₜ = 5.64 min; m/z = 254 (M-C₄H₈)⁺ (Diastereomer 1); Rₜ = 5.66 min; m/z = 254 (M-C₄H₈)⁺ (Diastereomer 2).

### Beispiel 215A

### Ethyl-2-[4-(brommethyl)phenyl]-4,4,4-trifluor-3-methylbutanoat

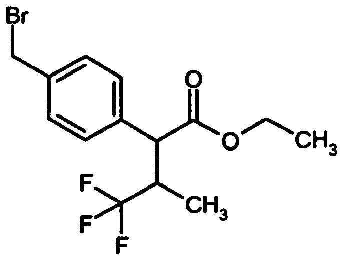

2.25 g (8.2 mmol) Ethyl-4,4,4-trifluor-3-methyl-2-(4-methylphenyl)butanoat, 1.53 g (8.6 mmol) *N-*Bromsuccinimid und 67 mg (0.41 mmol) 2,2'-Azobis-(2-methylpropannitril) in 36 ml Trichlormethan wurden über Nacht unter Rückfluss gerührt. Nach vollständiger Umsetzung wurde das Succinimid abfiltriert und der Filterrückstand mit Dichlormethan nachgewaschen. Das Filtrat wurde im Vakuum eingeengt. Das Rohprodukt wurde über Kieselgel chromatographisch gereinigt (Laufmittel Cyclohexan/Essigsäureethylester 40:1). Es wurden 2.667 g (7.5 mmol, 92% d. Th.) eines gelblichen Öls isoliert.
GC-MS (Methode 3): Rₜ = 5.72 min; m/z = 373 (M-Br)⁺ (Diastereomer 1); Rₜ = 5.74 min; m/z = 373 (M-Br)⁺ (Diastereomer 2).

Auf analoge Weise wurden die in der folgender Tabelle aufgeführten Verbindungen erhalten:

| **Beispiel** | **Name / Struktur** | **Analytische Daten** |
|---|---|---|
| **216A** | *tert*.-Butyl-2-[4-(brommethyl)phenyl]-6,6,6-trifluor-4-methylhexanoat | GC-MS (Methode 3): Diastereomer 1 Rₜ = 6.27 min, m/z = 274 (M-Br-C₄H₈)⁺; Diastereomer 2 Rₜ = 6.30 min, m/z = 274 (M-Br-C₄H₈)⁺. |
| | 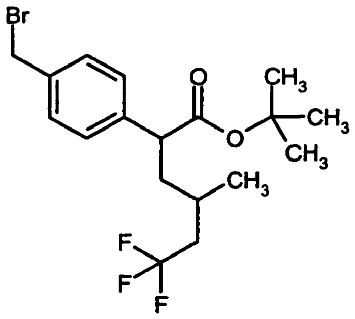 | |
| **217A** | *tert*.-Butyl-2-[4-(brommethyl)phenyl]-5,5,5-trifluor-3-methylpentanoat | GC-MS (Methode 3): Diastereomer 1 Rₜ = 5.96 min, m/z = 259 (M-Br-C₄H₈)⁺; Diastereomer 2 Rₜ = 6.00 min, m/z = 259 (M-Br-C₄H₈)⁺. MS (DCI): m/z = 412/414 (M+NH₄)⁺. |
| | 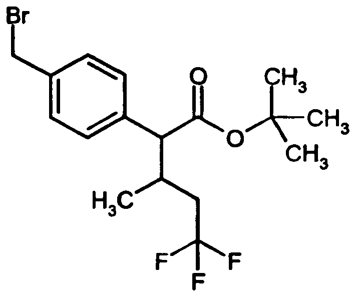 | |
| **218A** | Ethyl-2-[4-(brommethyl)phenyl]-3-(trifluormethyl)-pentanoat | GC-MS (Methode 3): Diastereomer 1 Rₜ = 6.01 min, m/z = 287 (M-Br)⁺; Diastereomer 2 Rₜ = 6.04 min, m/z = 287 (M-Br)⁺. |
| | 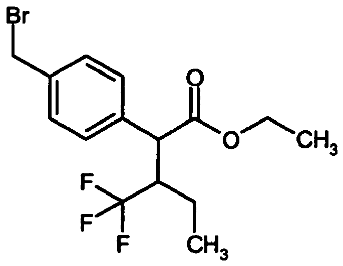 | |
| **219A** | *tert*.-Butyl-[4-(brommethyl)phenyl](3,3-difluor-cyclopentyl)acetat | GC-MS (Methode 3): Diastereomer 1 Rₜ = 5.64 min, m/z = 331/333 (M-C₄H₉)⁺, 254 (M-Br-C₄H₈+H)⁺; Diastereomer 2 Rₜ = 5.66 min, m/z = 331/333 (M-C₄H₉)⁺, 254 (M-Br-C₄H₈+H)⁺. |
| | 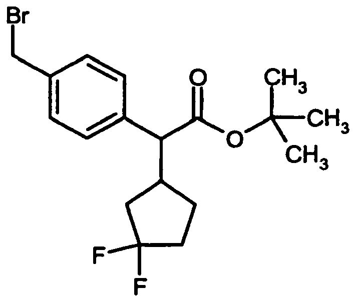 | |

### Beispiel 220A

### 1-(3-Brombenzyl)cyclopropancarbonsäure-tert-butylester

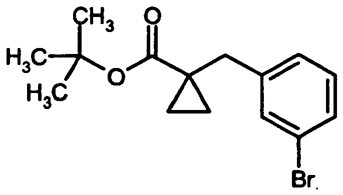

14.8 ml (105.48 mmol) Diisopropylamin wurden unter Argon in 66 ml trockenem THF vorgelegt und auf -40°C abgekühlt. Es wurden langsam 42.2 ml (105.48 mmol) n-Butyllithium-Lösung (2.5 M in Hexan) zugetropft und das Gemisch 30 min nachgerührt. Die Reaktionslösung wurde danach auf -78°C gekühlt und mit einer Lösung von 10.0 g (70.32 mmol) Cyclopropancarbonsäure-tert.-butylester in 17 ml THF versetzt. Nach 4 h Rühren bei -78°C wurde eine Lösung von 19.34 g (77.36 mmol) 3-Brombenzylbromid in 17 ml THF zugegeben. Das Reaktionsgemisch wurde langsam über Nacht auf RT erwärmt, bevor vorsichtig mit Ammoniumchlorid-Lösung versetzt wurde und dreimal mit Ethylacetat extrahiert wurde. Die vereinigten organischen Phasen wurden mit gesättigter Natriumchlorid-Lösung gewaschen, über Magnesiumsulfat getrocknet und im Vakuum eingeengt. Das Rohprodukt wurde durch Chromatographie an 750 g Silicagel gereinigt (Laufmittel Cyclohexan/Dichlormethan 50:1, dann 5:1). Es wurden 13.3 g (60.7% d. Th.) der Titelverbindung erhalten.
GC-MS (Methode 3): Rₜ = 5.94 min; m/z = 256 (M-C₄H₈)⁺.
¹H-NMR (400 MHz, DMSO-*d*₆): δ = 7.46 (s, 1H), 7.38 (m, 1H), 7.25 (m, 2H), 2.82 (s, 2H), 1.28 (s, 9H), 1.08 (q, 2H), 0.87 (q, 2H).

### Beispiel 221A

### 1-[3-(Benzylamino)benzyl]cyclopropancarbonsäure-tert.-butylester

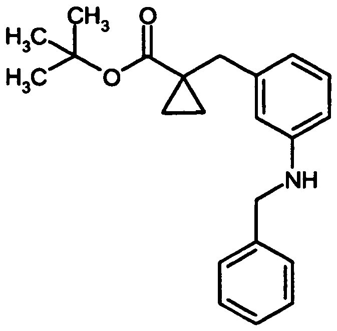

Unter Argon und trockenen Bedingungen wurden 13.3 g (42.73 mmol) 1-(3-Brombenzyl)cyclopropancarbonsäure-*tert*.-butylester, 5.6 ml (51.28 mmol) Benzylamin, 1.96 g (2.14 mmol) Tris-(dibenzylidenaceton)dipalladium, 4.93 g (51.28 mmol) Natrium-*tert*.-butylat und 1.06 g (1.71 mmol) (+/-)-2,2'-Bis(diphenylphosphino)-1,1'-binaphthyl in 50 ml Toluol suspendiert. Die Reaktionsmischung wurde für 1.5 h bei 110°C gerührt. Das Gemisch wurde dann über Kieselgur abgesaugt, der Rückstand mit Toluol nachgewaschen und das Filtrat eingeengt. Der Filtrat-Rückstand wurde in Ethylacetat aufgenommen und zweimal mit Ammoniumchlorid-Lösung extrahiert. Die organische Phase wurde über Magnesiumsulfat getrocknet und im Vakuum eingeengt. Das Rohprodukt wurde durch Chromatographie an Silicagel gereinigt (Laufmittel Cyclohexan/Ethylacetat 20:1). Es wurden 6.98 g (48.4% d. Th.) der Titelverbindung erhalten.
LC-MS (Methode 12): Rₜ = 2.75 min; m/z = 338 (M+H)⁺.
¹H-NMR (400 MHz, DMSO-*d*₆): δ = 7.35-7.26 (m, 4H), 7.20 (t, 1H), 6.91 (t, 1H), 6.45 (s, 1H), 6.38 (m, 2H), 6.12 (t, 1H), 4.23 (d, 2H), 2.69 (s, 2H), 1.28 (s, 9H), 0.99 (q, 2H), 0.69 (q, 2H).

### Beispiel 222A

### 1-(3-Aminobenzyl)cyclopropancarbonsäure-tert.-butylester

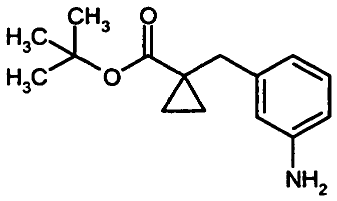

6.98 g (22.43 mmol) 1-[3-(Benzylanmino)benzyl]cyclopropancarbonsäure-*tert*.-butylester wurden in 50 ml Ethanol und 50 ml THF gelöst und mit 0.48 g (0.45 mmol) Palladium (10%-ig auf Kohle) versetzt. Bei RT wurde 2 h lang unter einer Wasserstoffatmosphäre bei Normaldruck gerührt. Das Reaktionsgemisch wurde danach über Kieselgur abgesaugt, der Rückstand mit THF nachgewaschen und das Filtrat eingeengt. Das Rohprodukt wurde durch Chromatographie an Silicagel gereinigt (Laufmittel Cyclohexan/Ethylacetat 10:1). Es wurden 3.66 g (65.9% d. Th.) der Titelverbindung erhalten.
LC-MS (Methode 7): Rₜ = 1.84 min; m/z = 192 (M+H)⁺.
¹H-NMR (400 MHz, DMSO-*d*₆): δ = 6.88 (t, 1 H), 6.42 (s, 1 H), 6.37 (dd, 2 H), 4.89 (d, 2 H), 2.69 (s, 2 H), 1.31 (s, 9 H), 1.03 (q, 2 H), 0.75 (q, 2 H).

### Beispiel 223A

### 1-(2-Methyl-3-nitrobenzyl)cyclopropancarbonsäure-tert.-butylester

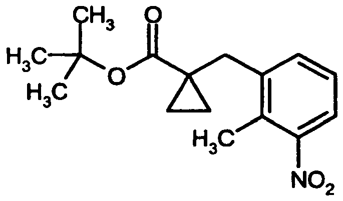

1.28 ml (9.15 mmol) Diisopropylamin wurden unter Argon in 6 ml trockenem THF vorgelegt und auf -40°C abgekühlt. Es wurden langsam 4.26 ml (9.15 mmol) n-Butyllithium-Lösung (2.5 M in Hexan) zugetropft und das Gemisch 30 min nachgerührt. Die Reaktionslösung wurde dann auf -78°C gekühlt und mit einer Lösung von 1.30 g (9.15 mmol) Cyclopropancarbonsäure-*tert*.-butylester in 2 ml THF versetzt. Nach 4 h Rühren bei -78°C wurde eine Lösung von 2.00 g (8.69 mmol) 1-(Brommethyl)-2-methyl-3-nitrobenzol in 2 ml THF zugegeben. Das Reaktionsgemisch wurde langsam über Nacht auf RT erwärmt. Es wurde dann vorsichtig mit Ammoniumchlorid-Lösung versetzt und dreimal mit Ethylacetat extrahiert. Die vereinigten organischen Phasen wurden mit gesättigter Natriumchlorid-Lösung gewaschen, über Magnesiumsulfat getrocknet und im Vakuum eingeengt. Das Rohprodukt wurde durch Chromatographie an Silicagel gereinigt (Laufmittel Cyclohexan/Dichlormethan 50:1, dann 5:1). Es wurden 0.78 g (29.3% d. Th.) der Titelverbindung erhalten.
¹H-NMR (400 MHz, DMSO-d₆): δ = 7.61 (d, 1 H), 7.54 (d, 1 H), 7.39 (t, 1 H), 2.96 (s, 2 H), 2.26 (s, 3 H), 1.26 (s, 9 H), 1.20 (q, 2 H), 0.79 (q, 2 H).

### Beispiel 224A

### 1-(3-Amino-2-methylbenzyl)cyclopropancarbonsäure-tert.-butylester

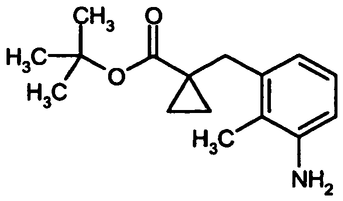

765 mg (2.63 mmol) 1-(2-Methyl-3-nitrobenzyl)cyclopropancarbonsäure-*tert*.-butylester wurden in 5 ml Ethanol gelöst und mit 139.7 mg (0.13 mmol) Palladium (10%-ig auf Kohle) versetzt. Bei RT wurde unter Normaldruck für 2 h hydriert. Das Reaktionsgemisch wurde über Kieselgur abgesaugt, der Rückstand mit THF nachgewaschen und das Filtrat eingeengt. Es wurden 680 mg (99.1% d. Th.) der Titelverbindung erhalten.
LC-MS (Methode 7): Rₜ = 1.96 min; m/z = 262 (M+H)⁺.
¹H-NMR (400 MHz, DMSO-d₆): δ = 6.77 (t, 1 H), 6.47 (d, 1 H), 6.40 (d, 2 H), 4.69 (s, 2 H), 2.88 (s, 2 H), 1.91 (s, 3 H), 1.31 (s, 9 H), 1.03 (q, 2 H), 0.55 (q, 2 H).

### Beispiel 225A

### (2E)-3-(4-Fluor-3-nitrophenyl)prop-2-ensäure-tert.-butylester

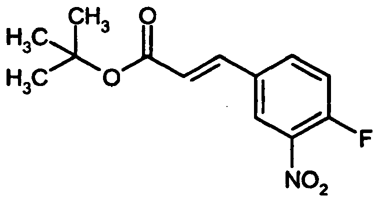

Unter Argon wurden 130.1 mg (3.25 mmol) Natriumhydrid in 2 ml Toluol und 2 ml THF vorgelegt. Die Mischung wurde auf 0°C abgekühlt und langsam mit 857.7 mg (3.40 mmol) *tert*.-Butyl-(diethoxyphosphoryl)acetat versetzt. Es wurde für 30 min nachgerührt und dann 500 mg (2.96 mmol) 4-Fluor-3-nitrobenzaldehyd zugegeben. Der Ansatz wurde langsam auf RT erwärmt und 3 h gerührt. Das Reaktionsgemisch wurde danach auf Wasser gegeben und dreimal mit Essigsäureethylester extrahiert. Die organische Phase wurde im Vakuum eingeengt und der Rückstand durch Chromatographie an Silicagel gereinigt (Laufmittel Cyclohexan/Ethylacetat 30:1 → 10:1). Es wurden 543 mg (69% d. Th.) der Titelverbindung erhalten.
LC-MS (Methode 12): Rₜ = 2.41 min; m/z = 212 (M-C₄H₈+H)⁺.
¹H-NMR (400 MHz, DMSO-*d*₆): δ = 8.51 (dd, 1 H), 8.19 (ddd, 1 H), 7.69-7.59 (m, 2 H), 6.70 (d, 1 H), 1.49 (s, 9 H).

### Beispiel 226A

### 3-(3-Amino-4-fluorphenyl)propansäure-tert.-butylester

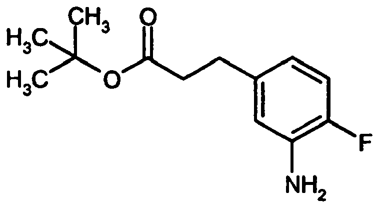

535 mg (2.00 mmol) (2E)-3-(4-Fluor-3-nitrophenyl)prop-2-ensäure-*tert*.-butylester wurden in 1 ml Ethanol und 1 ml THF gelöst und mit 21.3 mg Palladium (10%-ig auf Kohle) versetzt. Bei RT wurde über Nacht unter einer Wasserstoffatmosphäre bei Normaldruck hydriert. Das Reaktionsgemisch wurde über Kieselgur abgesaugt, der Rückstand mit THF nachgewaschen und das Filtrat eingeengt. Es wurden 479 mg (100% d. Th.) der Titelverbindung erhalten.
LC-MS (Methode 15): Rₜ = 1.06 min; m/z = 184 (M-C₄H₈)⁺.
¹H-NMR (400 MHz, DMSO-*d*₆): δ = 6.84 (dd, 1 H), 6.58 (dd, 1 H), 6.36-6.29 (m, 1 H), 5.00 (s, 2 H), 2.64 (t, 2 H), 2.42 (t, 2 H), 1.36 (s, 9 H).

### Beispiel 227A

### 3-Brom-2-fluoranilin

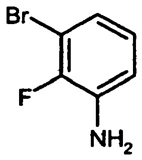

1.0 g (4.54 mmol) 3-Brom-2-fluornitrobenzol wurden unter Argon in 5 ml Dioxan vorgelegt und mit 4.3 g (22.72 mmol) Zinn(II)chlorid sowie ein paar Tropfen 1 N Salzsäure versetzt. Nach 2 h Rühren wurde der Ansatz am Rotationsverdampfer eingeengt und der Rückstand in Essigsäureethylester aufgenommen. Die organische Phase wurde mit 1 N Natronlauge, Wasser und gesättigter Natriumchlorid-Lösung gewaschen, dann über Magnesiumsulfat getrocknet und eingeengt. Es wurden 826 mg (95% d. Th.) der Titelverbindung erhalten.
LC-MS (Methode 15): Rₜ = 0.89 min; m/z = 190 (M+H)⁺.
¹H-NMR (400 MHz, DMSO-*d*₆): δ = 6.83-6.70 (m, 3 H), 5.43 (s, 2 H).

### Beispiel 228A

### (2E)-3-(3-Amino-2-fluorphenyl)prop-2-ensäure-tert.-butylester

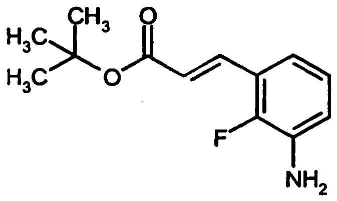

In 2.4 ml DMF wurden 400 mg (2.10 mmol) 3-Brom-2-fluoranilin, 809.42 mg (6.32 mmol) Acrylsäure-*tert*.-butylester und 1.47 ml (10.53 mmol) Triethylamin vorgelegt. Das Reaktiongefäß wurde dreimal evakuiert und jeweils wieder mit Argon belüftet. Anschließend wurden 47.26 mg (0.21 mmol) Palladium(II)acetat sowie 128.14 mg (0.42 mmol) Tri-2-tolylphosphin zugegeben und das Reaktionsgefäß erneut zweimal evakuiert und wieder mit Argon belüftet. Die Mischung wurde über Nacht bei 145°C gerührt. Der Ansatz wurde danach abgekühlt, auf gesättigte Ammoniumchlorid-Lösung gegeben und dreimal mit Essigsäureethylester extrahiert. Die vereinten organischen Phasen wurden über Magnesiumsulfat getrocknet und eingeengt. Das Rohprodukt wurde durch präparative RP-HPLC gereinigt (Eluent: Acetonitril/Wasser-Gradient). Es wurden 210 mg (42% d. Th.) der Titelverbindung erhalten.
LC-MS (Methode 11): Rₜ = 1.27 min; m/z = 238 (M+H)⁺.
¹H-NMR (400 MHz, DMSO-*d*₆): δ = 7.59 (d, 1 H), 6.94-6.87 (m, 2 H), 6.85-6.77 (m, 1 H), 6.45 (d, 1 H), 5.27 (s, 2 H), 1.52-1.43 (m, 9 H).

### Beispiel 229A

### 3-(3-Amino-2-fluorphenyl)propansäure-tert.-butylester

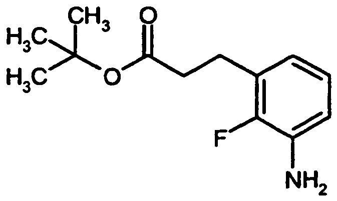

210 mg (0.88 mmol) (2*E*)-3-(3-Amino-2-fluorphenyl)prop-2-ensäure-*tert*.-butylester wurden in 0.44 ml Ethanol und 0.20 ml THF, gelöst und mit 9.4 mg Palladium (10%-ig auf Kohle) versetzt. Bei RT wurde über Nacht unter einer Wasserstoffatmosphäre bei Normaldruck hydriert. Das Reaktionsgemisch wurde über Kieselgur abgesaugt, der Rückstand mit THF nachgewaschen und das Filtrat eingeengt. Das Rohprodukt wurde über eine Biotage-Säule gereinigt (Laufmittel Cyclohexan/Ethylacetat 10:1). Es wurden 116 mg (54% d. Th.) der Titelverbindung erhalten.
LC-MS (Methode 15): Rₜ = 1.07 min; m/z = 184 (M-C₄H₈)⁺.
¹H-NMR (400 MHz, DMSO-*d*₆): δ = 6.75 (t, 1 H), 6.60 (t, 1 H), 6.38 (t, 1 H), 5.01 (br. s, 2 H), 2.74 (t, 2 H), 2.48-2.42 (m, 2 H), 1.36 (s, 9 H).

### Allgemeine Vorschrift 1: Herstellung von Benzylalkoholen aus Benzoesäuren

Eine 0.5 M Lösung der betreffenden Benzoesäure in Toluol wurde bei RT mit 1.3 eq. Triethylamin und anschließend mit 1.2 eq. Chlorameisensäuremethylester versetzt und das Gemisch über Nacht bei RT gerührt. Die entstandene Suspension wurde danach über Celite filtriert und der Rückstand mit Toluol nachgewaschen. Das Filtrat wurde eingeengt, der Filtrat-Rückstand in THF (1.5 ml/ mmol) gelöst und dann zu einer auf -78°C gekühlten Suspension von 1.2 eq. Lithiumaluminiumhydrid in THF (1 ml/mmol) getropft. Nach 1.5 h bei -78°C wurde die Reaktionsmischung auf RT erwärmt und über Nacht weitergerührt. Die entstandene Suspension wurde in 5%-ige Natronlauge (5 ml/mmol) gegossen und das Gemisch über Celite filtriert und mit Ethylacetat nachgewaschen. Das Filtrat wurde mehrfach mit Ethylacetat extrahiert. Die vereinigten organischen Phasen wurden mit gesättigter Kochsalz-Lösung gewaschen, über Magnesiumsulfat getrocknet und im Vakuum eingeengt.

Nach dieser Vorschrift wurden die folgenden Verbindungen hergestellt:

| **Beispiel** | **Name / Struktur** | **Analytische Daten** |
|---|---|---|
| **230A** | (3-Brom-5-fluorphenyl)methanol | LC-MS (Methode 11): Rₜ = 0.96 min; m/z = 187 [M-H₂O]⁺. |
| | 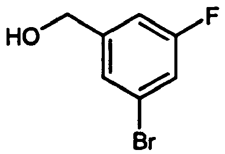 | |
| **231A** | (5-Brom-2-fluorphenyl)methanol | LC-MS (Methode 12): Rₜ = 1.61 min; m/z = 187 [M-H₂O]⁺. |
| | 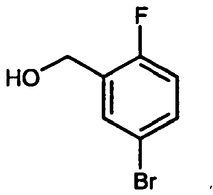 | |
| **232A** | (3-Brom-2-fluorphenyl)methanol | LC-MS (Methode 15): Rₜ = 0.81 min; m/z = 187 [M-H₂O]⁺. ¹H-NMR (400 MHz, DMSO-*d*₆): δ = 7.54-7.66 (m, 1H), 7.47 (t, 1H), 7.16 (t, 1H), 5.39 (t, 1H), 4.56 (d, 2H). |
| | 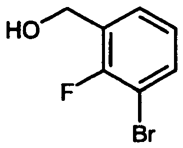 | |

### Allgemeine Vorschrift 2: Herstellung von Benzylbromiden aus Benzylalkoholen

Methode 2A: Der betreffende Benzylalkohol wurde in DMF (2 ml/mmol) vorgelegt und mit 2 eq. Tetrabromkohlenstoff versetzt. Anschließend wurden portionsweise innerhalb von 30 min 2 eq. Triphenylphosphin zugegeben und die Mischung über Nacht bei RT gerührt. Das Reaktionsgemisch wurde dann in Wasser gegossen und mit *tert.-*Butylmethylether extrahiert. Die organischen Phasen wurden über Magnesiumsulfat getrocknet und eingeengt. Das Rohprodukt wurde anschließend über Flash-Chromatographie an Kieselgel gereinigt (Laufmittel Cyclohexan).

Methode 2B: Der betreffende Benzylalkohol wurde in Dichlormethan (2 ml/mmol) vorgelegt, mit 1.2 eq. Triphenylphosphindibromid versetzt und über Nacht bei RT gerührt. Anschließend wurde die Reaktionsmischung mit Wasser gewaschen, und die organische Phase wurde über Magnesiumsulfat getrocknet und eingeengt. Das Rohprodukt wurde über Flash-Chromatographie an Kieselgel gereinigt (Laufmittel Cyclohexan).

Nach der Allgemeinen Vorschrift 2A bzw. 2B wurden die folgenden Verbindungen hergestellt:

| **Beispiel** | **Name / Struktur** | **Methode** | **Analytische Daten** |
|---|---|---|---|
| **233A** | 1-Brom-3-(brommethyl)-5-fluorbenzol | 2A | LC-MS (Methode 15): Rₜ = 1.17 min; m/z = 289 [M+NH₄]⁺. |
| | 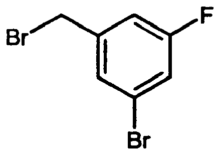 | | |
| **234A** | 2-Brom-4-(brommethyl)-1-fluorbenzol | 2B | GC-MS (Methode 3): Rₜ = 4.53 min; m/z = 268 [M]⁺. ¹H-NMR (400 MHz, DMSO-*d*₆): δ = 7.84 (dd, 1H), 7.42-7.59 (m, 1H), 7.33-7.42 (m, 1H), 4.71 (s, 2H). |
| | 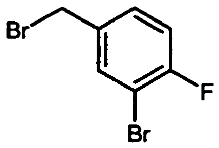 | | |
| **235A** | 4-Brom-2-(brommethyl)-1-fluorbenzol | 2B | GC-MS (Methode 3): Rₜ = 4.32 min; m/z = 268 [M]⁺. ¹H-NMR (400 MHz, DMSO-*d*₆): δ = 7.80 (dd, 1H), 7.60 (ddd, 1H), 7.19-7.30 (m, 1H), 4.62-4.73 (m, 2H). |
| | 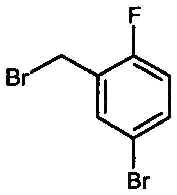 | | |
| **236A** | 1-Brom-3-(brommethyl)-2-fluorbenzol | 2B | GC-MS (Methode 3): Rₜ = 4.32 min; m/z = 268 [M]⁺. ¹H-NMR (400 MHz, DMSO-*d*₆): δ = 7.66-7.78 (m, 1H), 7.49-7.63 (m, 1H), 7.18 (t, 1H), 4.73 (s, 2H). |
| | 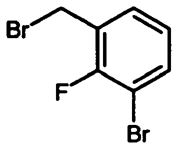 | | |

### Allgemeine Vorschrift 3: Alkylierung von Benzylbromiden mit Ester-Enolaten

Eine 0.3 M Lösung von Diisopropylamin in THF wurde unter Argon auf -40°C abgekühlt und mit 1 eq. *n*-Butyllithium versetzt. Nach 30 min wurde die Lösung auf -78°C abgekühlt und mit 0.8 eq. einer Lösung des betreffenden Carbonsäureesters in THF (0.7 M) versetzt. Die Reaktionsmischung wurde 4 h bei -78°C gerührt und anschließend mit 0.75 eq. des betreffenden Benzylbromids in THF (0.6 M) versetzt. Das Reaktionsgemisch wurde über Nacht unter Erwärmung auf RT gerührt. Danach wurde gesättigte Ammoniumchlorid-Lösung zugegeben. Das Gemisch wurde mit Ethylacetat extrahiert, und die organische Phase wurde über Magnesiumsulfat getrocknet und eingeengt. Das so erhaltene Rohprodukt wurde über Flash-Chromatographie an Kieselgel gereinigt (typisches Laufmittel-Gemisch: Cyclohexan/Ethylacetat 15:1 → 10:1).

Nach der Allgemeinen Vorschrift 3 wurden die folgenden Verbindungen hergestellt:

| **Beispiel** | **Name / Struktur** | **Analytische Daten** |
|---|---|---|
| **237A** | *tert*.-Butyl-1-(3-brom-5-fluorbenzyl)-cyclopropancarboxylat | GC-MS (Methode 3): Rₜ = 5.64 min; m/z = 272/274 [M-C₄H₉]⁺. ¹H-NMR (400 MHz, DMSO-*d*₆): δ = 7.37 (dd, 1H), 7.33 (s, 1H), 7.13 (d, 1H), 2.84 (s, 2H), 1.25-1.32 (m, 9H), 1.06-1.14 (m, 2H), 0.89-0.93 (m, 2H). |
| | 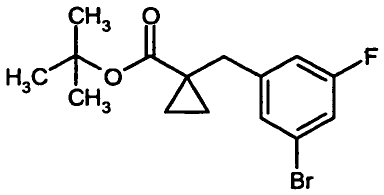 | |
| **238A** | *tert*.-Butyl-3-(3-brom-5-fluorphenyl)propanoat | GC-MS (Methode 3): Rₜ = 5.24 min; m/z = 246/248 [M-C₄H₉]⁺. ¹H-NMR (400 MHz, DMSO-*d*₆): δ = 7.33-7.40 (m, 1H), 7.32 (s, 1H), 7.15 (d, 1H), 2.82 (t, 2H), 2.52-2.58 (m, 2H), 1.35 (s, 9H). |
| | 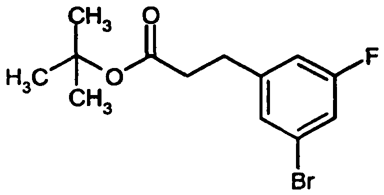 | |
| **239A** | *tert*.-Butyl-1-(3-brom-4-fluorbenzyl)-cyclopropancarboxylat | GC-MS (Methode 3): Rₜ = 5.85 min; m/z = 272/274 [M-C₄H₉]⁺. ¹H-NMR (400 MHz, DMSO-*d*₆): δ = 7.52-7.62 (m, 1H), 7.22-7.36 (m, 2H), 2.81 (s, 2H), 1.29 (s, 9H), 1.05-1.10 (m, 2H), 0.86-0.91 (m, 2H). |
| | 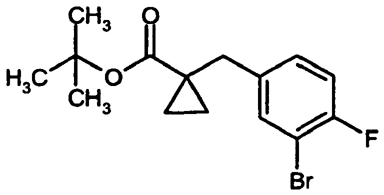 | |
| **240A** | *tert*.-Butyl-1-(5-brom-2-fluorbenzyl)-cyclopropancarboxylat | GC-MS (Methode 3): Rₜ = 5.72 min; m/z = 272/274 [M-C₄H₉]⁺. ¹H-NMR (400 MHz, DMSO-*d*₆): δ = 7.52 (dd, 1H), 7.39-7.49 (m, 1H), 7.07-7.21 (m, 1H), 2.86 (s, 2H), 1.29 (s, 9H), 1.10-1.15 (m, 2H), 0.84-0.91 (m, 2H). |
| | 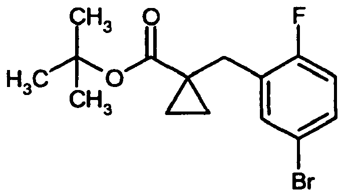 | |
| **241A** | *tert*.-Butyl-3-(5-brom-2-fluorphenyl)propanoat | GC-MS (Methode 3): Rₜ = 5.32 min; m/z = 246/248 [M-C₄H₉]⁺. |
| | 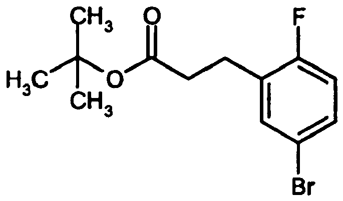 | |
| **242A** | *tert*.-Butyl-1-(3-brom-2-fluorbenzyl)-cyclopropancarboxylat | ¹H-NMR (400 MHz, DMSO-*d*₆): δ = 7.50-7.59 (m, 1H), 7.39 (t, 1H), 7.11 (t, 1H), 2.91 (s, 2H), 1.22-1.29 (m, 9H), 1.10-1.16 (m, 2H), 0.84-0.91 (m, 2H). |
| | 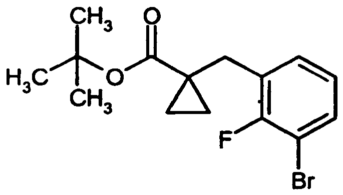 | |

### Allgemeine Vorschrift 4: Buchwald-Hartwig-Reaktion von Phenylbromiden zu N-Benzylphenylaminen

Unter Argonatmosphäre wurden 1.2 eq. Natrium-*tert*.-butylat in Toluol (1.5 ml/mmol) suspendiert, mit 1 eq. des betreffenden Phenylbromids, 1.2 eq. Benzylamin, 0.05 eq. Tris(dibenzylidenaceton)-dipalladium sowie 0.04 eq. *rac*-2,2'-Bis(diphenylphosphino)-1,1'-binaphthyl versetzt und das Gemisch für 2 h auf 110°C erhitzt. Nach Abkühlen auf RT wurde der Ansatz mit gesättigter Anunoniumchlorid-Lösung und Ethylacetat versetzt und über Celite filtriert. Die organische Phase wurde je einmal mit gesättigter Ammoniumchlorid-Lösung und gesättigter Natriumchlorid-Lösung gewaschen, dann über Magnesiumsulfat getrocknet und eingeengt. Das so erhaltene Rohprodukt wurde über Flash-Chromatographie an Kieselgel gereinigt (typisches Laufmittel-Gemisch: Cyclohexan/ Ethylacetat 50:1).

Nach der Allgemeinen Vorschrift 4 wurden die folgenden Verbindungen hergestellt:

| **Beispiel** | **Name / Struktur** | **Analytische Daten** |
|---|---|---|
| **243A** | *tert*.-Butyl-1-[3-(benzylamino)-5-fluorbenzyl]-cyclopropancarboxylat | LC-MS (Methode 11): Rₜ = 1.60 min; m/z = 356 [M+H]⁺. ¹H-NMR (400 MHz, DMSO-*d*₆): δ = 7.28-7.37 (m, 4H), 7.16-7.28 (m, 1H), 6.50 (t, 1H), 6.31 (s, 1H), 6.04-6.20 (m, 2H), 4.23 (d, 2H), 2.68 (s, 2H), 1.22-1.32 (m, 9H), 0.97-1.06 (m, 2H), 0.67-0.79 (m, 2H). |
| | 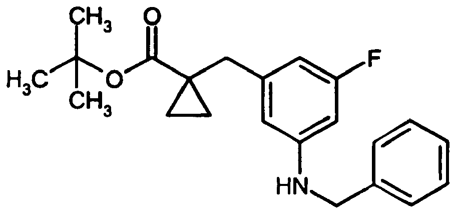 | |
| **244A** | *tert*.-Butyl-3-[3-(benzylamino)-5-fluorphenyl]-propanoat | LC-MS (Methode 15): Rₜ = 1.35 min; m/z = 330 [M+H]⁺. ¹H-NMR (400 MHz, DMSO-*d*₆): δ = 7.26-7.40 (m, 4H), 7.18-7.26 (m, 1H), 6.48 (t, 1H), 6.29 (s, 1H), 6.07-6.20 (m, 2H), 4.24 (d, 2H), 2.64 (t, 2H), 2.43 (t, 2H), 1.36 (s, 9H). |
| | 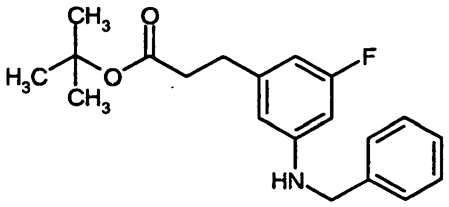 | |
| **245A** | *tert*.-Butyl-1-[3-(benzylamino)-4-fluorbenzyl]-cyclopropancarboxylat | LC-MS (Methode 11): Rₜ = 1.63 min; m/z = 356 [M+H]⁺. ¹H-NMR (400 MHz, DMSO-*d*₆): δ = 7.27-7.39 (m, 4H), 7.22 (d, 1H), 6.88 (dd, 1H), 6.43 (dd, 1H), 6.31-6.38 (m, 1H), 6.02-6.13 (m, 1H), 4.30 (d, 2H), 2.64 (s, 2H), 1.25 (s, 9H), 0.89-0.96 (m, 2H), 0.55-0.63 (m, 2H). |
| | 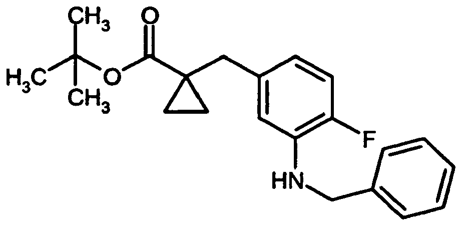 | |
| **246A** | *tert*.-Butyl-3-[5-(benzylamino)-2-fluorphenyl]-propanoat | LC-MS (Methode 15): Rₜ = 1.34 min; m/z = 330 [M+H]⁺. ¹H-NMR (400 MHz, DMSO-*d*₆): δ = 7.26-7.46 (m, 4H), 7.16-7.26 (m, 1H), 6.80 (t, 1H), 6.47 (dd, 1H), 6.37 (dt, 1H), 6.04 (t, 1 H), 4.21 (d, 2H), 2.69 (t, 2H), 2.42 (t, 2H), 1.36 (s, 9H). |
| | 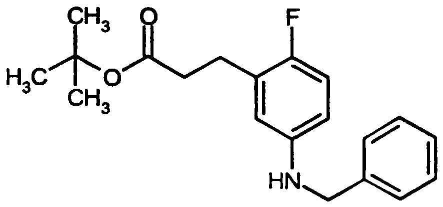 | |
| **247A** | tert.-Butyl-1-[5-(benzylamino)-2-fluorbenzyl]-cyclopropancarboxylat | LC-MS (Methode 11): Rₜ = 1.60 min; m/z = 356 [M+H]⁺. ¹H-NMR (400 MHz, DMSO-*d*₆): δ = 7.18-7.35 (m, 5H), 6.75-6.87 (m, 1H), 6.52 (dd, 1H), 6.37 (dt, 1H), 6.11 (t, 1H), 4.19 (d, 2H), 2.76 (s, 2H), 1.29 (s, 9H), 0.92-1.04 (m, 2H), 0.60-0.72 (m, 2H). |
| | 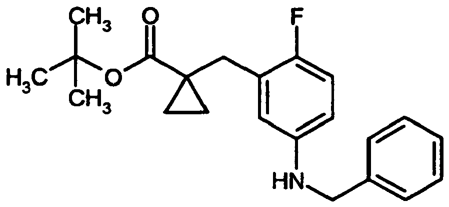 | |
| **248A** | *tert*.-Butyl-1-[3-(benzylamino)-2-fluorbenzyl]-cyclopropancarboxylat | LC-MS (Methode 11): Rₜ = 1.66 min; m/z = 356 [M+H]⁺. ¹H NMR (400 MHz, DMSO-*d*₆): δ = 7.25-7.41 (m, 4H), 7.13-7.25 (m, 1H), 6.75 (t, 1H), 6.48 (t, 1H), 6.39 (t, 1H), 6.02-6.16 (m, 1H), 4.32 (d, 2H), 2.85 (s, 2H), 1.29 (s, 9H), 1.03-1.12 (m, 2H), 0.70-0.83 (m, 2H). |
| | 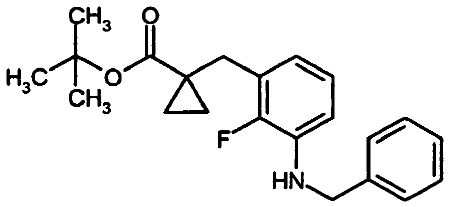 | |

### Allgemeine Vorschrift 5: Hydrierung von N-Benzylphenylaminen zu Phenylaminen zu Phenylaminen

Das betreffende *N*-Benzylphenylamin wurde in einem 1:1-Gemisch aus Ethanol und THF (5 ml/ mmol) gelöst, mit 10% Palladium auf Aktivkohle (35 mg/mmol) versetzt und das Gemisch bei RT und 1 bar Wasserstoffatmosphäre über Nacht gerührt. Anschließend wurde das Reaktionsgemisch über Celite filtriert, der Rückstand mit Ethanol nachgewaschen und das Filtrat eingeengt. Das so erhaltene Rohprodukt wurde über Flash-Chromatographie an Kieselgel gereinigt (typisches Laufmittel-Gemisch: Cyclohexan/Ethylacetat 3:1).

Nach der Allgemeinen Vorschrift 5 wurden die folgenden Verbindungen hergestellt:

| **Beispiel** | **Name / Struktur** | **Analytische Daten** |
|---|---|---|
| **249A** | *tert.*-Butyl-1-(3-amino-5-fluorbenzyl)-cyclopropancarboxylat | LC-MS (Methode 15): Rₜ = 1.14 min; m/z = 266 [M+H]⁺. ¹H-NMR (400 MHz, DMSO-*d₆*): δ = 6.25 (s, 1H), 6.11 (d, 1H), 6.14 (d, 1H), 5.28 (s, 2H), 2.68 (s, 2H), 1.30 (s, 9H), 1.01-1.11 (m, 2H), 0.71-0.85 (m, 2H). |
| | 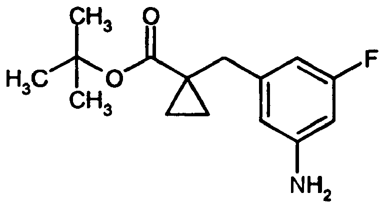 | |
| **250A** | *tert*.-Butyl-3-(3-amino-5-fluorphenyl)propanoat | GC-MS (Methode 3): Rₜ = 5.89 min; m/z = 239 [M]⁺. ¹H-NMR (400 MHz, DMSO-*d₆*): δ = 6.20 (s, 1H), 6.05-6.17 (m, 2H), 5.30 (s, 2H), 2.63 (t, 2H), 2.43 (t, 2H), 1.37 (s, 9H). |
| | 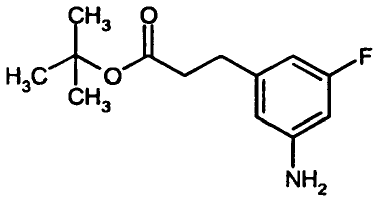 | |
| **251A** | *tert.*-Butyl-1-(3-amino-4-fluorbenzyl)-cyclopropancarboxylat | LC-MS (Methode 15): Rₜ = 1.14 min; m/z = 266 [M+H]⁺. ¹H-NMR (400 MHz, DMSO-*d₆*): δ = 6.84 (dd, 1H), 6.63 (dd, 1H), 6.36 (ddd, 1H), 4.98 (s, 2H), 2.68 (s, 2H), 1.25-1.35 (m, 9H), 0.96-1.09 (m, 2H), 0.69-0.83 (m, 2H). |
| | 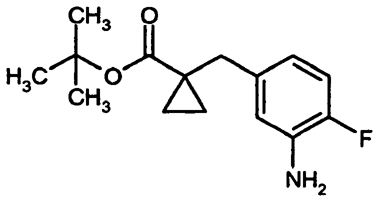 | |
| **252A** | *tert.*-Butyl-3-(5-amino-2-fluorphenyl)propanoat | LC-MS (Methode 15): Rₜ = 1.14 min; m/z = 240 [M+H]⁺. ¹H-NMR (400 MHz, DMSO-*d₆*): δ = 6.76 (dd, 1H), 6.29-6.50 (m, 2H), 4.85 (br. s, 2H), 2.68 (t, 2H), 2.42 (t, 2H), 1.36 (s, 9H). |
| | 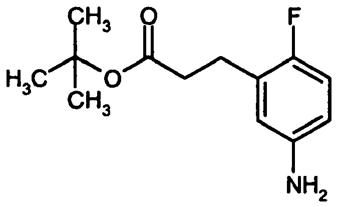 | |
| **253A** | *tert*.-Butyl-1-(3-amino-2-fluorbenzyl)-cyclopropancarboxylat | LC-MS (Methode 15): Rₜ = 1.14 min; m/z = 266 [M+H]⁺. ¹H-NMR (400 MHz, DMSO-*d*₆): δ = 6.68-6.87 (m, 1H), 6.51-6.66 (m, 1H), 6.39-6.51 (m, 1H), 4.98 (s, 2H), 2.83 (s, 2H), 1.25-1.38 (m, 9H), 1.01-1.13 (m, 2H), 0.68-0.84 (m, 2H). |
| | 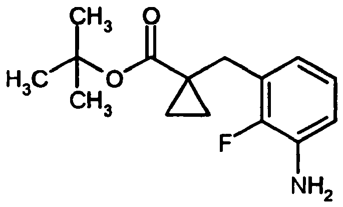 | |
| **254A** | *tert.* -Butyl-1-(5-amino-2-fluorbenzyl)-cyclopropancarboxylat | GC-MS (Methode 3): Rₜ = 6.16 min; m/z = 265 [M]⁺. |
| | 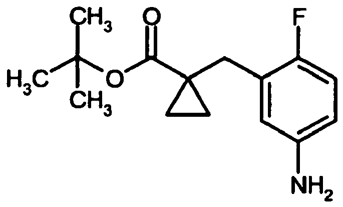 | |

### Beispiel 255A

### Methyl-2,2-dimethyl-3-(3-nitrophenyl)propanoat

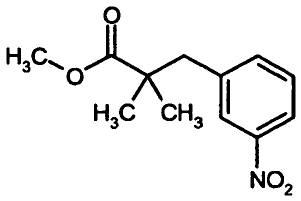

Unter Argon wurden 11.7 ml (83.32 mmol) Diisopropylamin in 200 ml THF gelöst und auf -78°C abgekühlt. Zu dieser Lösung wurden 33.3 ml (83.32 mmol) einer 2.5 M Lösung von n-Butyllithium in Hexan zugetropft. Anschließend wurde die Reaktionslösung auf -10°C erwärmt und 10 min bei dieser Temperatur nachgerührt. Die Reaktionslösung wurde dann wieder auf -78°C abgekühlt und langsam mit 8.4 ml (72.9 mmol) Methyl-2-methylpropanoat versetzt. Anschließend wurde 30 min bei -78°C nachgerührt. Danach wurden 15 g (69.4 mmol) 1-(Brommethyl)-3-nitrobenzol, gelöst in 150 ml THF, langsam zugetropft. Nach beendeter Zugabe wurde die Lösung langsam auf Raumtemperatur erwärmt und über Nacht gerührt. Nach DC-Kontrolle (Laufmittel: Toluol/Essigsäureethylester 10:1) wurde der Ansatz mit gesättigter Ammoniumchlorid-Lösung versetzt und in Essigsäureethylester aufgenommen. Die wässrige Phase wurde dreimal mit Essigsäureethylester extrahiert. Die vereinigten organischen Phasen wurden über Natriumsulfat getrocknet. Nach Filtration wurde das Lösungsmittel im Vakuum entfernt. Das Rohprodukt wurde über Kieselgel chromatographisch gereinigt (Laufmittel Toluol/Essigsäureethylester 20:1). Es wurden 12.2 g (51.4 mmol, 74% d. Th.) der Titelverbindung erhalten.
¹H-NMR (400 MHz, DMSO-d₆): δ = 8.11 (d, 1H), 7.96 (s, 1H), 7.54-7.63 (m, 2H), 2.97 (s, 3H), 2.54 (s, 2H), 1.14 (s, 6H).

### Beispiel 256A

### Methyl-3-(3-aminophenyl)-2,2-dimethylpropanoat

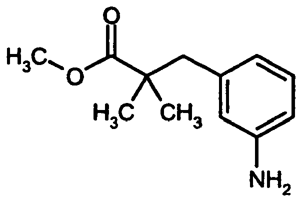

Eine Lösung von 12 g (51.42 mmol) Methyl-2,2-dimethyl-3-(3-nitrophenyl)propanoat in 200 ml Ethanol wurde bei Raumtemperatur mit 1 g Palladium auf Kohle (10%) versetzt und 12 Stunden unter Normaldruck hydriert. Nach vollständiger Umsetzung (DC-Kontrolle; Laufmittel Cyclohexan/Essigsäureethylester 1:1) wurde die Reaktionslösung über Kieselgur filtriert und das Filtrat im Vakuum eingeengt. Das Rohprodukt wurde über Kieselgel chromatographisch gereinigt (Laufmittel Cyclohexan/Essigsäureethylester 4:1 → 1:1). Es wurden 9.2 g (44.39 mmol, 86% d. Th.) der Titelverbindung erhalten.
LC-MS (Methode 10): Rₜ = 1.10 min; m/z = 208 (M+H)⁺.
¹H-NMR (400 MHz, DMSO-d₆): δ = 6.88 (t, 1 H), 6.39 (d, 1 H), 6.27 (s, 1 H), 6.20 (d, 1 H), 4.93 (s, 2 H), 2.62 (s, 3 H), 1.99 (s, 2 H), 1.09 (s, 6 H).

### Beispiel 257A

### 2-Chlor-3-nitrobenzaldehyd

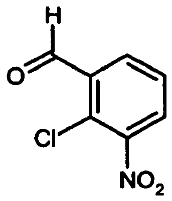

Eine Lösung von 6.11 g (32.57 mmol) (2-Chlor-3-nitrophenyl)methanol in 120 ml Dichlormethan wurde mit 8.43 g (39.09 mmol) Pyridiniumchlorochromat (PCC) versetzt und 12 Stunden bei Raumtemperatur gerührt. Nach vollständigem Umsatz wurde das Lösungsmittel im Vakuum bis zur Trockene eingeengt. Der erhaltene Rückstand wurde über Kieselgel chromatographisch gereinigt (Laufmittel Dichlormethan/Methanol 20:1). Es wurden 4.82 g (25.97 mmol, 79.7% d. Th.) der Titelverbindung erhalten.
GC-MS (Methode 3): Rₜ = 5.09 min; m/z = 186 (M+H)⁺.
¹H-NMR (400 MHz, DMSO-d₆): δ = 10.35 (s, 1 H), 8.33 (dd, 1 H), 8.13 (dd, 1 H), 7.77 (t, 1 H).

### Beispiel 258A

### tert.-Butyl-(2E)-3-(2-chlor-3-nitrophenyl)prop-2-enoat

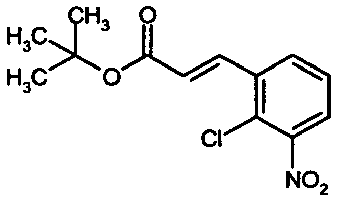

Unter Argon wurden 98 mg (2.46 mmol, 60%-ig) Natriumhydrid in 2 ml Toluol und 2 ml THF suspendiert und auf 0°C abgekühlt. Anschließend wurden 0.6 ml (2.57 mmol) tert.-Butyl-(diethoxyphosphoryl)acetat langsam zugetropft und die Mischung 30 min bei 0°C gerührt. Danach wurden 415 mg (2.24 mmol) 2-Chlor-3-nitrobenzaldehyd zur Reaktionsmischung gegeben und der Ansatz dann auf Raumtemperatur erwärmt. Das Gemisch wurde 2 Stunden bei Raumtemperatur gerührt und anschließend mit 5 ml Wasser versetzt. Nach Abtrennung der organischen Phase wurde die wässrige Phase noch dreimal mit Essigsäureethylester extrahiert. Die vereinigten organischen Phasen wurden über Natriumsulfat getrocknet. Nach Filtration wurde das Lösungsmittel im Vakuum entfernt. Das Rohprodukt wurde über Kieselgel chromatographisch gereinigt (Laufmittel Cyclohexan/Essigsäureethylester 10:1). Es wurden 429 mg (1.51 mmol, 59% d. Th.) der Titelverbindung erhalten.
MS (DCI): m/z = 301 (M+NH₄)⁺.
¹H-NMR (400 MHz, DMSO-d₆): δ = 8.23 (d, 1 H), 8.07 (d, 1 H), 7.84 (d, 1 H), 7.62 (t, 1 H), 6.73 (d, 1 H), 1.50 (s, 9 H).

### Beispiel 259A

### tert.-Butyl-3-(3-amino-2-chlorphenyl)propanoat

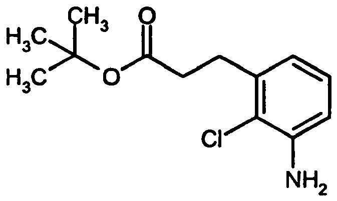

Eine Lösung von 200 mg (0.71 mmol) *tert*.-Butyl-(2*E*)-3-(2-chlor-3-nitrophenyl)prop-2-enoat in 10 ml Essigsäureethylester wurde bei Raumtemperatur mit 50 mg Platin auf Kohle (5%) versetzt und 12 Stunden unter Normaldruck hydriert. Nach vollständiger Umsetzung (DC-Kontrolle; Laufmittel Cyclohexan/Essigsäureethylester 1:1) wurde die Reaktionslösung über Kieselgur filtriert und das Filtrat im Vakuum eingeengt. Das Rohprodukt wurde mittels präparativer HPLC gereinigt. Es wurden 115 mg (0.62 mmol, 64% d. Th.) der Titelverbindung erhalten.
GC-MS (Methode 3): Rₜ = 6.42 min; m/z = 256 (M+H)⁺.

### Beispiel 260A

### tert.-Butyl-(2E)-3-(4-chlor-3-nitrophenyl)prop-2-enoat

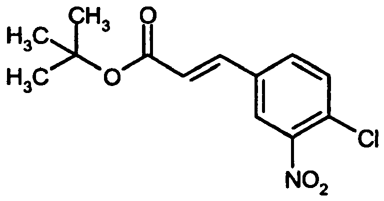

Unter Argon wurden 1.19 g (29.64 mmol, 60%-ig) Natriumhydrid in 25 ml Toluol und 25 ml THF suspendiert und auf 0°C abgekühlt. Anschließend wurden 7.28 ml (30.99 mmol) *tert*.-Butyl-(diethoxyphosphoryl)acetat langsam zugetropft und das Gemisch 30 min bei 0°C gerührt. Danach wurden 5 g (26.94 mmol) 4-Chlor-3-nitrobenzaldehyd zur Reaktionsmischung gegeben und der Ansatz dann auf Raumtemperatur erwärmt. Das Gemisch wurde 2 Stunden bei Raumtemperatur gerührt und anschließend mit 50 ml Wasser versetzt. Nach Abtrennung der organischen Phase wurde die wässrige Phase noch dreimal mit Essigsäureethylester extrahiert. Die vereinigten organischen Phasen wurden über Natriumsulfat getrocknet. Nach Filtration wurde das Lösungsmittel im Vakuum entfernt. Das Rohprodukt wurde über Kieselgel chromatographisch gereinigt (Laufmittel Cyclohexan/Essigsäureethylester 9:1). Es wurden 6.77 g (23.86 mmol, 77% d. Th.) der Titelverbindung erhalten.
MS (DCI): m/z = 301 (M+NH₄)⁺.
¹H-NMR (400 MHz, DMSO-d₆): δ = 8.46 (d, 1 H), 8.07 (dd, 1 H), 7.71 (d, 1 H), 7.51 (d, 1H), 6.75 (d, 1 H), 1.49 (s, 9 H).

### Beispiel 261A

### tert.-Butyl-3-(3-amino-4-chlorphenyl)propanoat

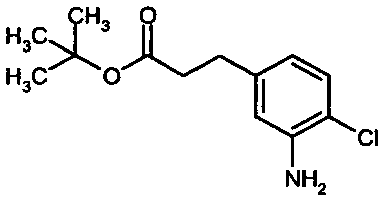

Eine Lösung von 6.74 g (23.76 mmol) *tert*.-Butyl-(2*E*)-3-(4-chlor-3-nitrophenyl)prop-2-enoat in 200 ml Ethanol und 20 ml THF wurde bei Raumtemperatur mit 500 mg Palladium auf Kohle (10%) versetzt und 12 Stunden unter Normaldruck hydriert. Nach vollständiger Umsetzung (DC-Kontrolle; Laufmittel Cyclohexan/Essigsäureethylester 1:1) wurde die Reaktionslösung über Kieselgur filtriert und das Filtrat im Vakuum eingeengt. Das Rohprodukt wurde über Kieselgel chromatographisch gereinigt (Laufmittel Cyclohexan/Essigsäureethylester 4:1 → 2:1). Es wurden 1.40 g (5.47 mmol, 23% d. Th.) der Titelverbindung erhalten.
LC-MS (Methode 15): Rₜ = 1.14 min; m/z = 256 (M+H)⁺.
¹H-NMR (400 MHz, DMSO-d₆): δ = 7.08 (d, 1 H), 6.62 (s, 1 H), 6.39 (dd, 1 H), 5.22 (s, 2 H), 2.66 (t, 2 H), 2.45 (t, 2 H), 1.37 (s, 9 H).

### Beispiel 262A

### 6-Phenylpyridazin-3(2H)-on

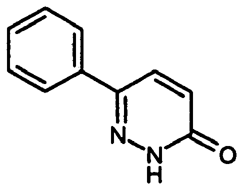

19.6 g (162.95 mmol) 1-Phenylethanon und 5 g (54.32 mmol) Oxoessigsäure-Monohydrat wurden 2 Stunden bei 100°C gerührt. Die Reaktionslösung wurde anschließend auf 40°C abgekühlt und mit 20 ml Wasser und 4 ml Ammoniak versetzt. Nachfolgend wurde das Gemisch zweimal mit 50 ml Dichlormethan extrahiert. Die erhaltene wässrige Phase wurde dann mit 2.64 ml (53.32 mmol) Hydrazin-Monohydrat versetzt und 2 Stunden bei 100°C gerührt. Nach Umsetzung wurde die Reaktionslösung auf Raumtemperatur abgekühlt. Die ausgefallenen Kristalle wurden abgesaugt, mit Wasser gewaschen und über Nacht im Vakuumtrockenschrank bei 50°C getrocknet. Es wurden 4.3 g (24.97 mmol, 15% d. Th.) der Titelverbindung als farblose Kristalle erhalten.
LC-MS (Methode 7): Rₜ = 1.39 min; m/z = 173 (M+H)⁺.
¹H-NMR (400 MHz, DMSO-d₆, δ/ppm): 13.2 (s, 1 H), 8.04 (d, 1 H), 7.86 (d, 2 H), 7.53-7.41 (m, 3 H), 7.00 (d, 1 H).

### Beispiel 263A

### 6-(Trifluormethyl)-4,5-dihydropyridazin-3(2H)-on

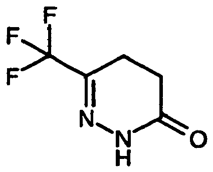

8.44 g (26.1 mmol) *tert*.-Butyl-3-[5-oxo-4-(propan-2-yl)-2-(trifluormethyl)-2,5-dihydro-1,3-oxazol-2-yl]propanoat [CAS Reg.-Nr. 87341-14-8] und 13.44 g (130.5 mmol) Hydrazin-Hydrochlorid wurden in 100 ml Eisessig 2 Stunden bei 118°C gerührt. Die Reaktionslösung wurde anschließend im Vakuum bis zur Trockene eingedampft. Der erhaltene Rückstand wurde in 100 ml Wasser aufgenommen und mit Kaliumcarbonat basisch gestellt. Darauf folgend wurde die basische Lösung dreimal mit Dichlormethan extrahiert. Die vereinigten organischen Phasen wurden über Magnesiumsulfat getrocknet, filtriert und bis zur Trockene eingeengt. Das Rohprodukt wurde über Kieselgel chromatographisch gereinigt (Laufmittel Dichlormethan/Methanol 50:1 → 10:1). Es wurden 2.65 g (15.95 mmol, 6 1 % d. Th.) der Titelverbindung erhalten.
¹H-NMR (400 MHz, DMSO-d₆): δ = 11.33 (br. s, 1 H), 2.76 (t, 2 H), 2.52 (t, 2 H).
GC-MS (Methode 3): Rₜ = 2.76 min; m/z = 166 (M)⁺.

### Beispiel 264A

### 6-(Trifluormethyl)pyridazin-3(2H)-on

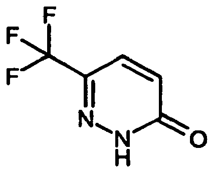

2.6 g (15.65 mmol) 6-(Trifluormethyl)-4,5-dihydropyridazin-3(2*H*)-on wurden in 20 ml Eisessig gelöst und auf 100°C erhitzt. Anschließend wurden zur Lösung langsam 0.81 ml (15.65 mmol) Brom hinzugetropft und 4 Stunden bei 100°C nachgerührt. Nach Abkühlen der Reaktionslösung wurde das Lösungsmittel im Vakuum entfernt und der erhaltene Rückstand über Kieselgel chromatographisch gereinigt (Laufmittel Dichlormethan/Methanol 50:1 → 20:1). Es wurden 1.34 g (8.15 mmol, 52% d. Th.) der Titelverbindung erhalten.
¹H-NMR (400 MHz, DMSO-d₆): δ = 13.73 (br. s, 1 H), 7.81 (d, 1 H), 7.11 (d, 1 H).
GC-MS (Methode 3): Rₜ = 2.92 min; m/z = 164 (M)⁺.
MS (DCI): m/z = 182 (M+NH₄)⁺.

Analog zu Beispiel 25A wurden die in der folgenden Tabelle aufgeführten Verbindungen erhalten:

| **Beispiel** | **Name / Struktur / Edukte** | **Analytische Daten** |
|---|---|---|
| **265A** | Ethyl-4,4,4-trifluor-3-methyl-2-{4-[(5-oxo-2-phenyl-5,6-dihydro-4*H*-1,3,4-oxadiazin-4-yl)methyl]phenyl}butanoat | GC-MS (Methode 3): Rₜ = 9.98 min; m/z = 449 (M+H)⁺. |
| | 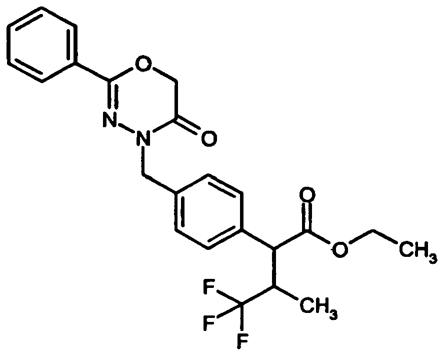 | |
| | (aus Ethyl-2-[4-(brommethyl)phenyl]-4,4,4-trifluor-3-methylbutanoat und 2-Phenyl-4*H*-1,3,4-oxadiazin-5(6*H*)-on) | |
| **266A** | Ethyl-4,4,4-trifluor-3-methyl-2-{4-[(6-oxo-3-phenylpyridazin-1 (6*H*)-yl)methyl]phenyl}butanoat | LC-MS (Methode 7): Rₜ = 2.78 min; m/z = 445 (M+H)⁺. |
| | 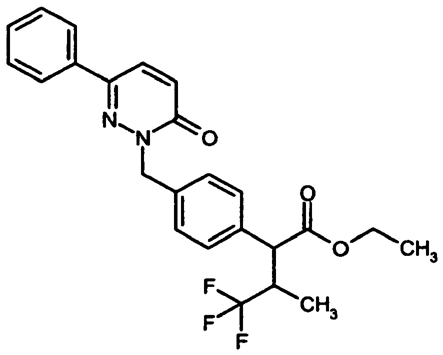 | |
| | (aus Ethyl-2-[4-(brommethyl)phenyl]-4,4,4-trifluor-3-methylbutanoat und 6-Phenylpyridazin-3(2*H*)-on) | |
| **267A** | *tert.*-Butyl-6,6,6-trifluor-4-methyl-2-{4-[(5-oxo-2-phenyl-5,6-dihydro-4*H*-1,3,4-oxadiazin-4-yl)methyl]phenyl}-hexanoat | LC-MS (Methode 10): Rₜ = 2.85 min; m/z = 505 (M+H)⁺. |
| | 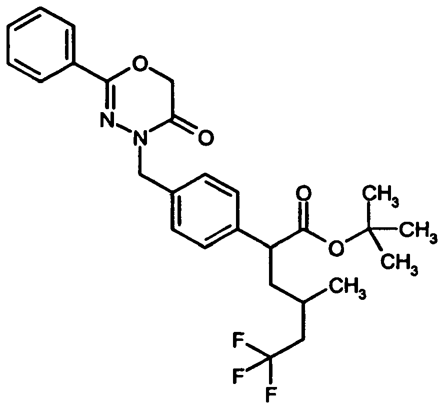 | |
| | (aus *tert.*-Butyl-2-[4-(brommethyl)phenyl]-6,6,6-trifluor-4-methylhexanoat und 2-Phenyl-4*H*-1,3,4-oxadiazin-5(6*H*)-on) | |
| **268A** | *tert.*-Butyl-cyclopentyl {4-[(6-oxo-3-phenylpyridazin-1(6*H*)-yl)methyl]phenyl}acetat | LC-MS (Methode 11): Rₜ = 1.70 min; m/z = 445 (M+H)⁺. |
| | 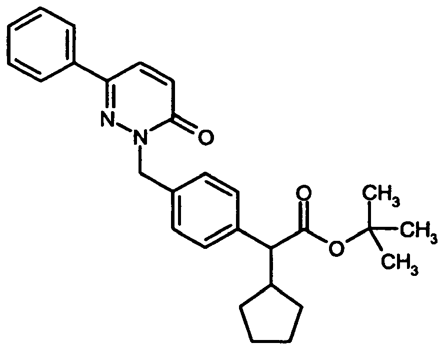 | |
| | (aus Beispiel 10A und 6-Phenylpyridazin-3(2*H*)-on) | |
| **269A** | *tert.*-Butyl-5,5,5-trifluor-3-methyl-2-{4-[(5-oxo-2-phenyl-5,6-dihydro-4*H*-1,3,4-oxadiazin-4-yl)methyl]phenyl}-pentanoat | LC-MS (Methode 11): Rₜ = 1.63 min; m/z = 491 (M+H)⁺. |
| | 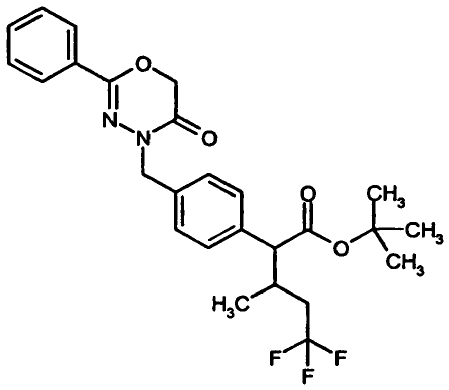 | |
| | (aus *tert.*-Butyl-2-[4-(brommethyl)phenyl]-5,5,5-trifluor-3-methylpentanoat und 2-Phenyl-4*H*-1,3,4-oxadiazin-5(6*H*)-on) | |
| **270A** | Ethyl-2-{4-[(5-oxo-2-phenyl-5,6-dihydro-4*H*-1,3,4-oxadiazin-4-yl)methyl]phenyl}-3-(trifluormethyl)-pentanoat | LC-MS (Methode 12): Rₜ = 2.77 min; m/z = 463 (M+H)⁺. |
| | 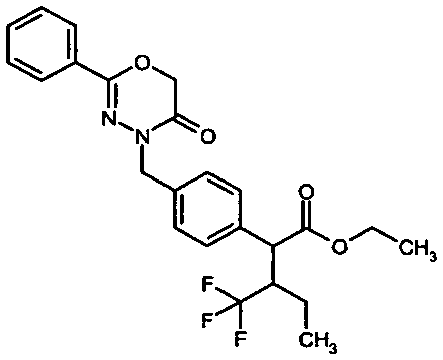 | |
| | (aus Ethyl-2-[4-(brommethyl)phenyl]-3-(trifluormethyl)-pentanoat und 2-Phenyl-4*H*-1,3,4-oxadiazin-5(6*H*)-on) | |
| **271A** | *tert.*-Butyl-cyclopentyl{4-[(3-methyl-6-oxopyridazin-1(6*H*)-yl)methyl]phenyl} acetat | LC-MS (Methode 12): Rₜ = 2.65 min; m/z = 327 (M-C₄H₈+H)⁺. |
| | 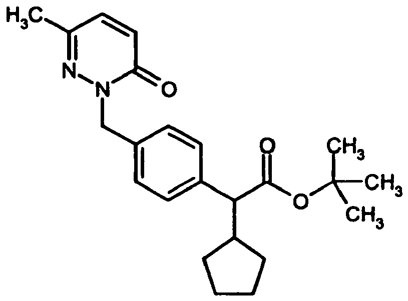 | |
| | (aus Beispiel 10A und 6-Methylpyridazin-3(2*H*)-on) | |
| **272A** | *tert*.-Butyl-cyclopentyl(4-{[6-oxo-3-(trifluormethyl)-pyridazin-l(6*H*)-yl]methyl}phenyl)acetat | LC-MS (Methode 15): Rₜ = 1.45 min; m/z = 381 (M-C₄H₈+H)⁺. ¹H-NMR (400 MHz, DMSO-d₆): δ = 7.88 (d, 1H), 7.31 (d, 2H), 7.18-7.27 (m, 3H), 5.29 (s, 2H), 3.19 (d, 1H), 2.30-2.43 (m, 1H), 1.75-1.87 (m, 1H), 1.57-1.64 (m, 1H), 1.46-1.56 (m, 2H), 1.36-1.45 (m, 1H), 1.34 (s, 9H), 1.18-1.29 (m, 2H), 0.84-1.02 (m, 1H). |
| | 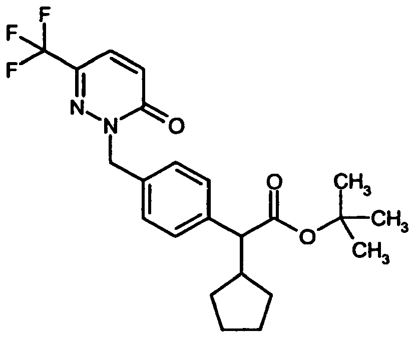 | |
| | (aus Beispiel 10A und 6-(Trifluormethyl)pyridazin-3(2*H*)-on) | |
| **273A** | *tert.*-Butyl-cyclopentyl{4-[(5-methyl-6-oxopyridazin-1 (6*H*)-yl)methyl]phenyl} acetat | LC-MS (Methode 15): Rₜ = 1.42 min; m/z = 382 (M+H)⁺. ¹H-NMR (500 MHz, DMSO-d₆): δ = 7.65 (d, 1H), 7.25-7.34 (m, 3H), 7.17-7.24 (m, 2H), 6.16 (t, 1H), 5.08 (s, 2H), 3.17 (d, 1H), 2.33-2.42 (m, 1H), 2.00 (s, 3H), 1.76-1.86 (m, 1H), 1.60 (d, 1H), 1.46-1.56 (m, 2H), 1.37-1.46 (m, 1H), 1.34 (s, 9H), 1.18-1.29 (m, 2H), 0.89-0.99 (m, 1H). |
| | 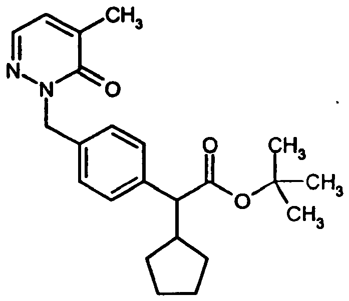 | |
| | (aus Beispiel 10A und 4-Methylpyridazin-3(2*H*)-on) | |
| **274A** | *tert.*-Butyl-cyclopentyl(4-{[6-oxo-4-(trifluormethyl)-pyridazin-1(6*H*)-yl]methyl}phenyl)acetat | LC-MS (Methode 15): Rₜ = 1.39 min; m/z = 380 (M-C₄H₈+H)⁺. ¹H-NMR (500 MHz, DMSO-d₆): δ = 8.10 (d, 1H), 7.30 (d, 2H), 7.24 (d, 2H), 6.82 (s, 1H), 6.53 (d, 1H), 5.13 (s, 2H), 3.18 (d, 1H), 2.29-2.42 (m, 1H), 1.77-1.85 (m, 1H), 1.60 (d, 1H), 1.48-1.56 (m, 2H), 1.37-1.44 (m, 1H), 1.34 (s, 9H), 1.24 (d, 2H), 0.93 (dd, 1H). |
| | 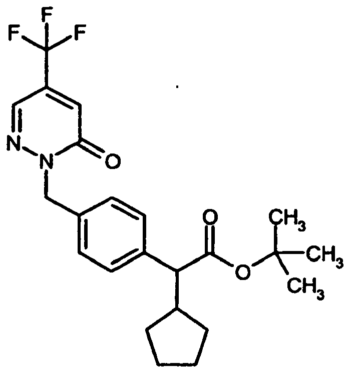 | |
| | (aus Beispiel 10A und 5-(Trifluormethyl)pyridazin-3(2*H*)-on) | |
| **275A** | *tert*.-Butyl-(3,3-difluorcyclopentyl){4-[(5-oxo-2-phenyl-5,6-dihydro-4*H*-1,3,4-oxadiazin-4-yl)methyl]phenyl}-acetat | LC-MS (Methode 15): Rₜ = 1.40 min; m/z = 485 (M+H)⁺. |
| | 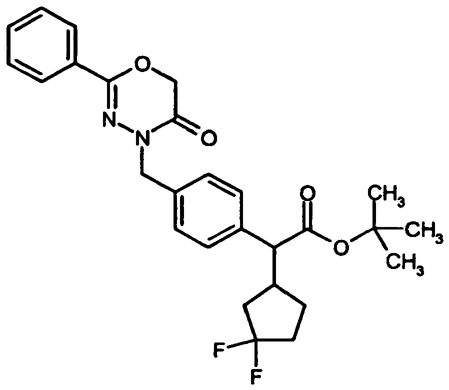 | |
| | (aus *tert.*-Butyl-[4-(brommethyl)phenyl](3,3-difluor-cyclopentyl)acetat und 2-Phenyl-4*H*-1,3,4-oxadiazin-5(6*H*)-on) | |
| **276A** | *tert.*-Butyl-cyclopentyl {4-[(4-methyl-6-oxopyridazin-1(6*H*)-yl)methyl]phenyl} acetat | LC-MS (Methode 15): Rₜ = 1.30 min; m/z = 326 (M-C₄H₈+H)⁺. ¹H-NMR (500 MHz, DMSO-d₆): δ = 7.66 (d, 1H), 7.28 (d, 2H), 7.18 (d, 2H), 6.23 (s, 1H), 6.10 (d, 1H), 5.02 (s, 2H), 3.16 (d, 1H), 2.33-2.41 (m, 1H), 2.12 (s, 3H), 1.75-1.84 (m, 1H), 1.45-1.67 (m, 3H), 1.36-1.44 (m, 1H), 1.34 (s, 9H), 1.17-1.28 (m, 2H), 0.85-0.97 (m, 1H). |
| | 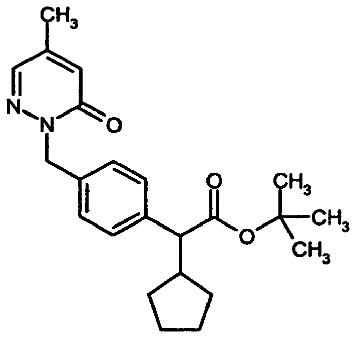 | |
| | (aus Beispiel 10A und 5-Methylpyridazin-3(2*H*)-on) | |
| **277A** | Ethyl-4,4,4-trifluor-3-methyl-2-(4-{[6-oxo-3-(trifluor-methyl)pyridazin-1(6*H*)-yl]methyl}phenyl)butanoat | LC-MS (Methode 11): Rₜ = 1.45 min; m/z = 437 (M+H)⁺. |
| | 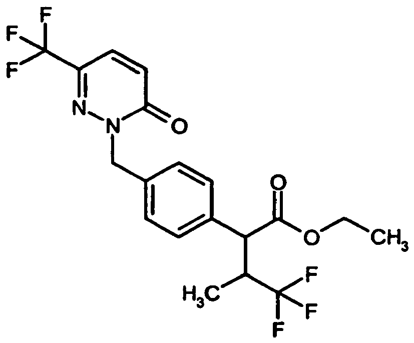 | |
| | (aus Ethyl-2-[4-(brommethyl)phenyl]-3-(trifluormethyl)-pentanoat und 6-(Trifluormethyl)pyridazin-3(2*H*)-on) | |
| **278A** | *tert*.-Butyl-cyclopentyl(4-{[6-oxo-5-(trifluormethyl)-pyridazin-1 (6*H*)-yl]methyl} phenyl)acetat | LC-MS (Methode 15): Rₜ = 1.37 min; m/z = 380 (M-C₄H₈+H)⁺. |
| | 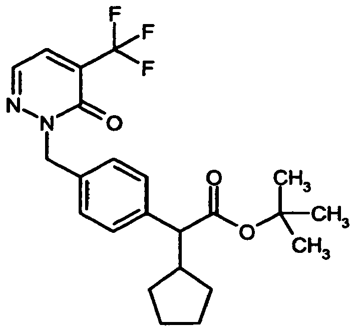 | |
| | (aus Beispiel 10A und 4-(Trifluormethyl)pyridazin-3(2*H*)-on) | |
| **279A** | *tert*.-Butyl-{4-[(3-chlor-6-oxopyridazin-1(6*H*)-yl)-methyl]phenyl}(cyclopentyl)acetat | LC-MS (Methode 11): Rₜ = 1.56 min; m/z = 347 (M-C₄H₈+H)⁺. |
| | 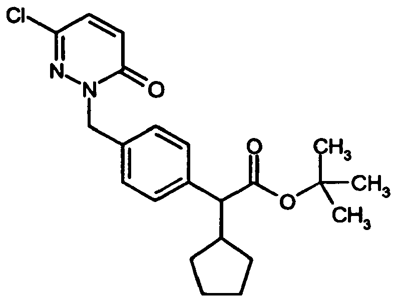 | |
| | (aus Beispiel 10A und 6-Chlorpyridazin-3(2*H*)-on) | |

### Beispiel 280A und Beispiel 281A

### tert.-Butyl-cyclopentyl {4-[(6-oxo-3-phenylpyridazin-1(6H)-yl)methyl]phenyl}acetat (Enantiomer 1 und 2)

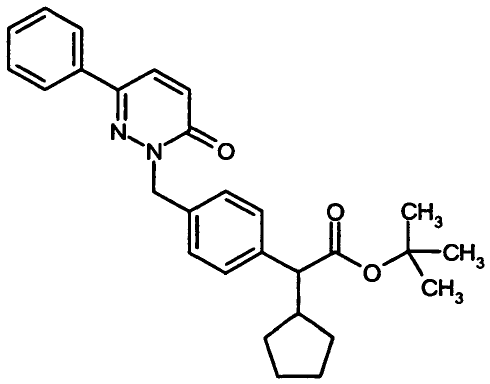

7.42 g (16.69 mmol) des racemischen *tert*.-Butyl-cyclopentyl{4-[(6-oxo-3-phenylpyridazin-1(6*H*)-yl)methyl]phenyl}acetats wurden mittels präparativer HPLC an chiraler Phase in die Enantiomere aufgetrennt [Säule: Daicel Chiralpak AS-H, 5 µm, 250 mm x 20 mm; Elutionsmittel: Isohexan/Isopropanol 75:25 (v/v); Fluss: 15 ml/min; UV-Detektion: 220 nm; Temperatur: 30°C]:

### Beispiel 280A (Enantiomer 1):

Ausbeute: 4.1 g
Rₜ 5.28 min; Reinheit >99%; >99% ee
[Säule: Daicel Chiralpak AS-H, 5 µm, 250 mm x 4.6 mm; Elutionsmittel: Isohexan/Isopropanol 75:25 (v/v); Fluss: 1 ml/min; UV-Detektion: 220 nm; Temperatur: 40°C].

### Beispiel 281A (Enantiomer 2):

Ausbeute: 2.8 g
R, 5.84 min; Reinheit >98%; >96% ee
[Säule: Daicel Chiralpak AS-H, 5 µm, 250 mm x 4.6 mm; Elutionsmittel: Isohexan/Isopropanol 75:25 (v/v); Fluss: 1 ml/min; UV-Detektion: 220 nm; Temperatur: 40°C].

### Beispiel 282A

### tert.-Butyl-cyclopentyl {4-[(1,3-dioxo-1,3-dihydro-2H-isoindol-2-yl)methyl]phenyl} acetat

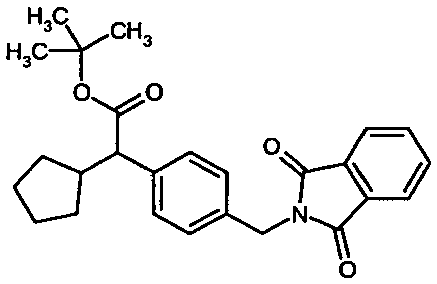

Unter Argon wurden 6.49 g (35 mmol) Kaliumphthalimid in 60 ml DMF vorgelegt, mit 15 g (31.8 mmol) (+/-)-2-(4-(Brommethyl)phenyl)-2-cyclopentylessigsäure-*tert*.-butylester versetzt und über Nacht bei 80°C gerührt. Anschließend wurden weitere 0.5 Äquivalente Kaliumphthalimid zugegeben und 6 h bei 80°C nachgerührt. Der Ansatz wurde dann filtriert und das Filtrat im Vakuum eingeengt. Der Rückstand wurde mit Ethanol verrührt und der Feststoff abgesaugt, mit Ethanol gewaschen und im Hochvakuum getrocknet. Es wurden 8.5 g (ca. 96% Reinheit, 6 1 % d. Th.) der Zielverbindung erhalten.
LC-MS (Methode 10): Rₜ = 2.87 min; m/z = 364 (M-C₄H₈+H)⁺.
¹H-NMR (400 MHz, DMSO-d₆): δ = 7.9-7.85 (m, 4 H), 7.29-7.24 (m, 4 H), 4.75 (s, 1 H), 3.17 (d, 1 H), 2.4-2.3 (m, 1 H), 1.80 (ddd, 1 H), 1.66-1.37 (m, 4 H), 1.34 (s, 9 H), 1.28-1.17 (m, 2 H), 1.06 (t, 1 H), 0.93 (m, 1 H).

### Beispiel 283A

### (+/-)-2-Cyclopentyl-2-(4-((1,3-dioxoisoindolin-2-yl)methyl)phenyl)essigsäure

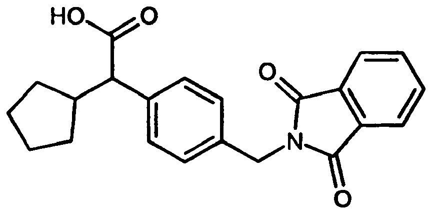

8.5 g (20.26 mmol) (+/-)-2-Cyclopentyl-2-(4-((1,3-dioxoisoindolin-2-yl)methyl)phenyl)essigsäure-*tert.*-butylester wurden in 95 ml Dichlormethan vorgelegt, mit 31.2 ml (405.2 mmol) Trifluoressigsäure versetzt und 2 h bei Raumtemperatur gerührt. Anschließend wurde der Ansatz mit Wasser (250 ml) versetzt und mit Dichlormethan extrahiert. Die organische Phase wurde mit Wasser gewaschen, über Magnesiumsulfat getrocknet und eingeengt. Es wurden 6.98 g (95% d. Th.) der Zielverbindung erhalten.
LC-MS (Methode 11): Rₜ = 1.28 min; m/z = 364 (M+H)⁺.
¹H-NMR (400 MHz, DMSO-d₆): δ = 7.88 (m, 4 H), 7.29-7.24 (m, 4 H), 4.74 (s, 2 H), 3.20 (d, 1 H), 2.46-2.33 (m, 1 H), 1.87-1.76 (m, 1 H), 1.65-1.12 (m, 6 H), 0.97-0.87 (m, 1 H).

### Beispiel 284A

### (+/-)-3-(3-(2-Cyclopentyl-2-(4-((1,3-dioxoisoindolin-2-yl)methyl)phenyl)acetamido)-2-methylphenyl)propansäure-tert.-butylester

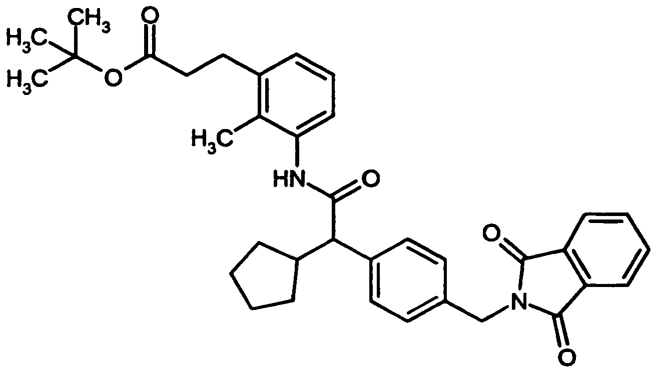

7.5 g (20.6 mmol) (+/-)-2-Cyclopentyl-2-(4-((1,3-dioxoisoindolin-2-yl)methyl)phenyl)essigsäure wurden in einem Gemisch aus 170 ml DMF und 68 ml Pyridin gelöst und mit 10.2 g (26.8 mmol) TCTU versetzt. Nach 30 min Rühren bei Raumtemperatur wurden 4.86 g (20.6 mmol) 3-(3-Amino-2-methylphenyl)propansäure-*tert.*-butylester zugegeben. Nach Rühren über Nacht bei Raumtemperatur wurde das Reaktionsgemisch eingeengt und der Rückstand durch Flash-Chromatogaphie an Kieselgel vorgereinigt (Laufmittel Dichlormethan/Methanol 100:1). Das so erhaltene Produkt wurde nacheinander mit Ethanol und Methanol verrührt, der Feststoff abgesaugt und im Hochvakuum getrocknet. Es wurden 8.5 g (71% d. Th.) der Zielverbindung erhalten.
LC-MS (Methode 10): Rₜ = 2.71 min; m/z = 581 (M+H)⁺.

### Beispiel 285A

### (+/-)-tert.-Butyl-3-[3-({[4-(aminomethyl)phenyl](cyclopentyl)acetyl}amino)-2-methylphenyl]-propanoat

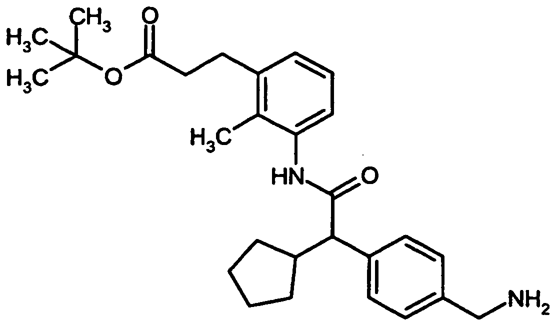

8.5 g (14.6 mmol) (+/-)-3-(3-(2-Cyclopentyl-2-(4-((1,3-dioxoisoindolin-2-yl)methyl)phenyl)acet-amido)-2-methylphenyl)propansäure-*tert.*-butylester wurden in 85 ml Ethanol vorgelegt und mit 2.35 ml (48.3 mmol) Hydrazinhydrat versetzt. Das Reaktionsgemisch wurde über Nacht unter Rückfluss erhitzt. Nach Abkühlen auf Raumtemperatur wurde der ausgefallene Feststoff abfiltriert und mit Ethanol nachgewaschen. Das Filtrat wurde eingeengt, der Rückstand mit Acetonitril verrührt und die ausgefallenen Kristalle abgesaugt und verworfen. Die Mutterlauge wurde eingeengt und der Rückstand durch Flash-Chromatographie an Kieselgel gereinigt (Laufmittel Dichlormethan/Methanol/aq. Ammoniak 100:5:1). Es wurden 4.51 g (68% d. Th.) der Zielverbindung erhalten.
LC-MS (Methode 7): Rₜ = 1.70 min; m/z = 451 (M+H)⁺.
¹H-NMR (400 MHz, DMSO-*d*₆): δ = 9.44 (s, 1 H), 7.31-7.39 (m, 2 H), 7.24-7.30 (m, 2 H), 6.93-7.07 (m, 3 H), 3.68 (s, 2 H), 3.43 (dd, 1 H), 3.12-3.19 (m, 1 H), 2.79 (t, 2 H), 2.38-2.46 (m, 2 H), 2.00 (s, 3 H), 1.21-1.92 (m, 16 H), 0.91-1.06 (m, 1 H).

### Beispiel 286A

### (+/-)-tert.-Butyl-3-{3-[(cyclopentyl{4-[(1-oxo-1,3-dihydro-2H-isoindol-2-yl)methyl]phenyl}-acetyl)amino]-2-methylphenyl}propanoat

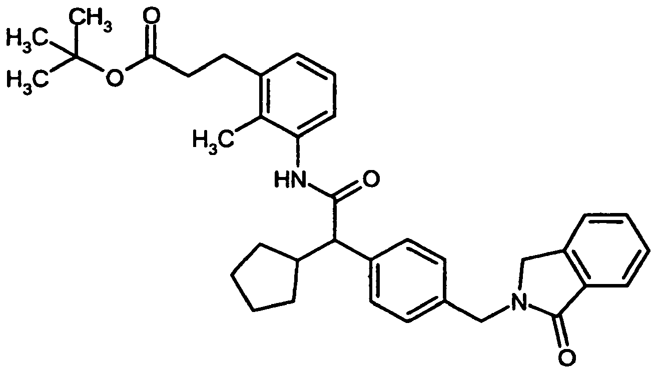

50 mg (0.11 mmol) (+/-)-*tert*.-Butyl-3-[3-({[4-(aminomethyl)phenyl](cyclopentyl)acetyl}amino)-2-methylphenyl]propanoat und 16.4 mg (0.12 mmol) Phthaldialdehyd wurden bei RT in 2.5 ml Eisessig gelöst und 1 h stehengelassen. Anschließend wurde das Reaktionsgemisch in Wasser gegossen und mit Ethylacetat extrahiert. Die organische Phase wurde über Magnesiumsulfat getrocknet und eingeengt. Der Rückstand wurde über präparative RP-HPLC gereinigt. Es wurden 53 mg (85% d. Th.) der Zielverbindung erhalten.
LC-MS (Methode 10): Rₜ = 2.54 min; m/z = 567 (M+H)⁺.
¹H-NMR (400 MHz, DMSO-*d*₆): δ = 9.45 (s, 1 H), 7.73 (d, 1 H), 7.54-7.63 (m, 2 H), 7.47-7.53 (m, 1 H), 7.40 (d, 2 H), 7.23 (d, 2 H), 6.94-7.06 (m, 3 H), 4.71 (s, 2 H), 4.37 (s, 2 H), 3.45 (d, 1 H), 2.78 (t, 2 H), 2.41 (t, 2 H), 1.97 (s, 3 H), 1.27-1.89 (m, 17 H), 0.90-1.05 (m, 1 H).

### Beispiel 287A

### (+/-)-Cyclopentyl{4-[(1-oxo-1,3-dihydro-2H-isoindol-2-yl)methyl]phenyl}essigsäure-tert.-butylester

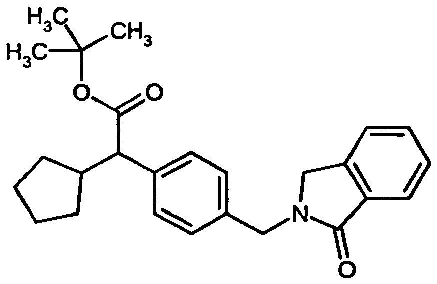

2.035 g (15.3 mmol) 1-Oxoindolin in 12 ml DMF wurden bei 0°C mit 611.3 mg (15.3 mmol, 60%-ig) Natriumhydrid versetzt. Es wurde 30 min gerührt und dann bei 0°C 6.0 g (12.7 mmol) (+/-)-[4-(Brommethyl)phenyl](cyclopentyl)essigsäure-tert.-butylester zugegeben. Man rührte das Reaktionsgemisch weitere 4 h, versetzte dann mit Wasser und extrahierte zweimal mit Dichlormethan. Die vereinigten organischen Phasen wurden mit gesättigter Natriumchlorid-Lösung gewaschen, über Magnesiumsulfat getrocknet und eingeengt. Das Rohprodukt wurde im Ultraschallbad mit Diethylether behandelt und der Feststoff durch Filtration isoliert. Es wurden 3.40 g (60.2% d. Th.) der Zielverbindung erhalten.
LC-MS (Methode 11): Rₜ = 1.57 min; m/z = 350 (M-C₄H₈+H)⁺.
¹H-NMR (400 MHz, DMSO-*d*₆): δ = 7.72 (d, 1 H), 7.60-7.50 (m, 3 H), 7.30 (d, 2 H), 7.21 (d, 2 H), 4.71 (s, 2 H), 4.38 (s, 2 H), 3.18 (d, 1 H), 2.35 (m, 1 H), 1.85-1.37 (m, 5 H), 1.34 (s, 9 H), 1.20 (m, 2 H), 0.91 (m, 1 H).

### Beispiel 288A

### (+)-(2S)-Cyclopentyl{4-[(1-oxo-1,3-dihydro-2H-isoindol-2-yl)methyl]phenyl}essigsäure-tert.-butylester

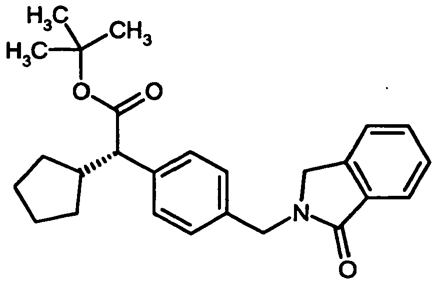

Das in Beispiel 287A erhaltene Racemat wurde durch präparative HPLC an chiraler Phase in die Enantiomere getrennt [Säule: Daicel Chiralpak IA-H, 5 µm, 250 mm x 20 mm; Fluss: 15 ml/min; UV-Detektion: 220 nm; Injektionsvolumen: 0.25 ml; Temperatur: 30°C; Eluent: 20% Acetonitril / 80% Methyl-tert.-butylether]. Ausgehend von 3.40 g Racemat wurden 1.50 g des (+)-Enantiomeren erhalten (das andere Enantiomer wurde nicht rein isoliert).
LC-MS (Methode 11): Rₜ = 1.58 min; m/z = 350 (M-C₄H₈+H)⁺.
¹H-NMR (400 MHz, DMSO-*d*₆): δ = 7.73 (d, 1 H), 7.58 (m, 2 H), 7.50 (t, 1 H), 7.30 (d, 2 H), 7.22 (d, 2 H), 4.71 (s, 2 H), 4.38 (s, 2 H), 3.18 (d, 1 H), 2.36 (m, 1 H), 1.80 (m, 1 H), 1.66-1.36 (m, 4 H), 1.34 (s, 9 H), 1.24 (m, 2 H), 0.95 (m, 1 H).
[α]_{D}²⁰ = + 8.2°, c = 0.38, Chloroform.

### Beispiel 289A

### (+/-)-Cyclopentyl {4-[(1-oxo-1,3-dihydro-2H-isoindol-2-yl)methyl]phenyl}essigsäure

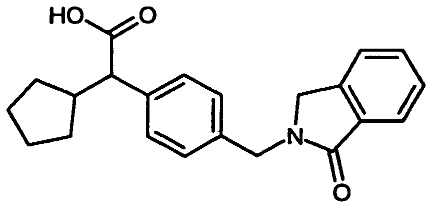

695 mg (1.71 mmol) (+/-)-Cyclopentyl{4-[(1-oxo-1,3-dihydro-2*H*-isoindol-2-yl)methyl]phenyl}-essigsäure-*tert*.-butylester wurden bei RT in 11 ml einer 4 N Lösung von Chlorwasserstoff in Dioxan gelöst und 12 h gerührt. Das Reaktionsgemisch wurde danach eingefroren und im Hochvakuum gefriergetrocknet. Es wurden 700 mg an Produkt erhalten, welches nicht weiter aufgereinigt wurde.
LC-MS (Methode 11): Rₜ = 1.17 min; m/z = 350 (M+H)⁺.
¹H-NMR (400 MHz, DMSO-*d*₆): δ = 12.21 (s, 1 H), 7.73 (d, 1 H), 7.62-7.47 (m, 3 H), 7.30 (d, 2 H), 7.22 (d, 2 H), 4.70 (s, 2 H), 4.37 (s, 2 H), 3.21 (d, 1 H), 2.42 (m, 1 H), 1.89-1.17 (m, 7 H), 0.94 (m, 1 H).

### Beispiel 290A

### (+)-(2S)-Cyclopentyl{4-[(1-oxo-1,3-dihydro-2H-isoindol-2-yl)methyl]phenyl}essigsäure

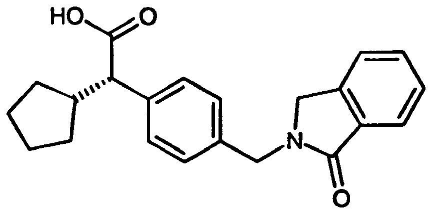

500 mg (1.23 mmol) (+)-(2*S*)-Cyclopentyl{4-[(1-oxo-1,3-dihydro-2*H-*isoindol-2-yl)methyl]-phenyl}essigsäure-tert.-butylester wurden bei RT in 11 ml einer 4 N Lösung von Chlorwasserstoff in Dioxan gelöst und 12 h gerührt. Das Reaktionsgemisch wurde danach eingefroren und im Hochvakuum gefriergetrocknet. Es wurden 450 mg an Produkt erhalten, welches nicht weiter aufgereinigt wurde.
LC-MS (Methode 11): Rₜ = 1.17 min; m/z = 350 (M+H)⁺.
¹H-NMR (400 MHz, DMSO-*d*₆): δ = 7.72 (d, 1 H), 7.57 (m, 2 H), 7.50 (t, 1 H), 7.30 (d, 2 H), 7.22 (d, 2 H), 4.71 (s, 2 H), 4.37 (s, 2 H), 3.21 (d, 1 H), 2.42 (m, 1 H), 1.82 (m, 1 H), 1.66-1.16 (m, 6 H), 0.93 (m, 1 H).
[α]_{D}²⁰ = +38.1°, c = 0.585, Chloroform.

### Beispiel 291A

### 5,6-Difluor-1H-isoindol-1,3(2H)-dion

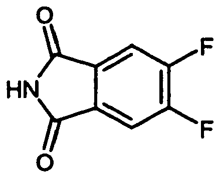

1.0 g (5.4 mmol) 4,5-Difluorphthalsäureanhydrid wurde in 6 ml Formamid 2 h bei 130°C gerührt. Die Reaktionsmischung wurde dann nach Abkühlen auf eisgekühltes Wasser gegeben. Der ausgefallene gelbe Feststoff wurde abgesaugt, gründlich mit kaltem Wasser gewaschen und im Hochvakuum getrocknet. Erhalten wurden 744 mg eines weißen Feststoffs (74.8% d. Th.).
LC-MS (Methode 10): Rₜ = 1.10 min; m/z = 182 (M-H)⁻.
¹H-NMR (400 MHz, DMSO-d₆): δ = 11.58 (s, 1 H), 8.01 (t, 2 H).

### Beispiel 292A

### 5,6-Difluor-3-hydroxy-2,3-dihydro-1H-isoindol-1-on

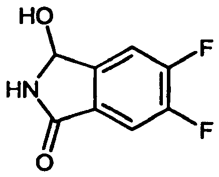

740 mg (4.04 mmol) 5,6-Difluor-1*H*-isoindol-1,3(2*H*)-dion wurden in 6.6 ml Ethanol gelöst, auf 0°C gekühlt und portionsweise mit 76.4 mg (2.02 mmol) Natriumborhydrid versetzt. Nach Ende der Zugabe wurde die Reaktionsmischung langsam auf RT erwärmt und noch 30 min bei RT nachgerührt, bevor das Reaktionsgemisch mit 1 N Salzsäure neutral gestellt wurde. Das Gemisch wurde im Vakuum bis zur Trockene eingeengt und der Rückstand im Hochvakuum weiter getrocknet. Erhalten wurden 800.8 mg Rohprodukt, welches ohne weitere Reinigung in der Folgereaktion eingesetzt wurde.
LC-MS (Methode 15): Rₜ = 0.51 min; m/z = 184 (M-H)⁻.

### Beispiel 293A

### 5,6-Difluor-2,3-dihydro-1H-isoindol-1-on

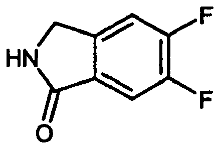

748 mg 5,6-Difluor-3-hydroxy-2,3-dihydro-1*H*-isoindol-1-on (Rohprodukt, ca. 4.04 mmol) wurden in 50 ml Dichlormethan vorgelegt, mit 3.8 ml (48.5 mmol) Trifluoressigsäure und 1.3 ml (8.08 mmol) Triethylsilan versetzt und über Nacht bei RT gerührt. Das Reaktionsgemisch wurde danach eingeengt und der Rückstand in Dichlormethan gelöst und mit Natriumhydrogencarbonat-Lösung gewaschen. Das Rohprodukt wurde durch Chromatographie an Silicagel gereinigt (Laufmittel Cyclohexan/Essigsäureethylester 1:1 bis 0:1). Es wurden 72 mg (10.6% d. Th.) der Zielverbindung erhalten.
LC-MS (Methode 15): Rₜ = 0.61 min; m/z = 170 (M+H)⁺.
¹H-NMR (400 MHz, DMSO-*d*₆): δ = 8.74 (br. s, 1 H), 7.76-7.64 (m, 2 H), 4.36 (s, 2 H).

### Beispiel 294A

### 4-Fluor-1H-isoindol-1,3(2H)-dion

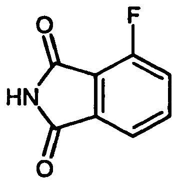

10.0 g (60.2 mmol) 4-Fluor-2-benzofuran-1,3-dion wurden unter Argon in 50 ml Formamid 1 h bei 130°C gerührt. Das abgekühlte Reaktionsgemisch wurde dann auf Eiswasser gegeben. Es fiel ein Feststoff aus, der abgesaugt und mit Wasser gewaschen wurde. Das Produkt wurde im Hochvakuum getrocknet. Es wurden 8.3 g (83% d. Th.) der Zielverbindung erhalten.
LC-MS (Methode 11): Rₜ = 0.57 min; m/z = 166 (M+H)⁺.
¹H-NMR (400 MHz, DMSO-*d*₆): δ = 11.47 (br. s, 1 H), 7.87 (td, 1 H), 7.69-7.61 (m, 2 H).

### Beispiel 295A

### 4-Fluor-2,3-dihydro-1H-isoindol-1-on

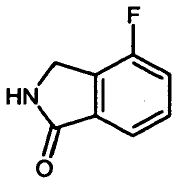

Zu einer auf 0°C gekühlten Suspension von 4.0 g (24.2 mmol) 4-Fluor-1*H*-isoindol-1,3(2*H*)-dion in 40 ml Ethanol wurden portionsweise 458 mg (12.1 mmol) Natriumborhydrid gegeben. Nach Ende der Zugabe wurde die Kühlung entfernt und die Mischung auf RT erwärmt. Nach 20 min Rühren wurden bei RT weitere 458 mg (12.1 mmol) Natriumborhydrid zugesetzt. Nach weiteren 30 min bei RT wurde die Reaktionsmischung vorsichtig mit 1 N Salzsäure versetzt und der pH-Wert neutral eingestellt. Das Gemisch wurde im Vakuum eingeengt und der Rückstand in Dichlormethan aufgenommen und über Celite filtriert. Das aufgefangene Filtrat wurde im Vakuum eingeengt. Erhalten wurden 4.05 g 4-Fluor-3-hydroxy-2,3-dihydro-1*H*-isoindol-1-on, welches als Rohprodukt direkt weiter umgesetzt wurde.

4.05 g (ca. 24.2 mmol) 4-Fluor-3-hydroxy-2,3-dihydro-1*H-*isoindol-1-on wurden in 10 ml Dichlormethan vorgelegt, mit 22.4 ml (290.8 mmol) Trifluoressigsäure und 7.7 ml (48.5 mmol) Triethylsilan versetzt und über Nacht bei RT gerührt. Das Reaktionsgemisch wurde danach eingeengt und der Rückstand in Dichlormethan gelöst und mit Natriumhydrogencarbonat-Lösung gewaschen. Das Rohprodukt wurde durch Chromatographie an Silicagel gereinigt (Laufmittel Cyclohexan/ Essigsäureethylester 3:1 bis 1:1). Es wurden 890 mg (24.2% d. Th.) der Zielverbindung erhalten.
LC-MS (Methode 12): Rₜ = 1.18 min; m/z = 152 (M+M)⁺.
¹H-NMR (400 MHz, DMSO-d₆): δ = 8.77 (br. s, 1 H), 7.58-7.51 (m, 2 H), 7.49-7.40 (m, 1 H), 4.46 (s, 2 H).

### Beispiel 296A

### 4,7-Difluor-1H-isoindol-1,3(2H)-dion

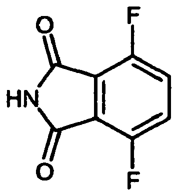

2.15 g (11.68 mmol) 4,7-Difluor-2-benzofuran-1,3-dion wurden unter Argon in 15 ml Formamid 5 h bei 130°C gerührt. Das abgekühlte Reaktionsgemisch wurde dann auf Eiswasser gegeben. Es fiel ein Feststoff aus, der abgesaugt und mit Wasser gewaschen wurde. Das Produkt wurde im Hochvakuum getrocknet. Es wurden 0.45 g (21 % d. Th.) der Zielverbindung erhalten.
LC-MS (Methode 15): Rₜ = 0.56 min; m/z = 184 (M+H)⁺.
¹H-NMR (400 MHz, DMSO-d₆): δ = 11.58 (br. s, 1 H), 7.71 (t, 2 H).

### Beispiel 297A

### (+/-)-Cyclopentyl{4-[(3-oxo-1,3-dihydro-2H-indazol-2-yl)methyl]phenyl}essigsäure-tert.-butylester

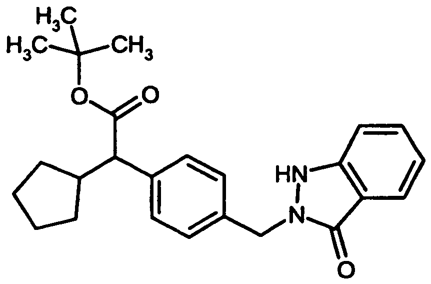

50.9 mg (1.27 mmol, 60%-ig) Natriumhydrid in 1.8 ml DMF wurden bei 0°C mit 170.9 mg (1.27 mmol) 1,2-Dihydro-3*H*-indazol-3-on versetzt. Es wurde 30 min gerührt und dann bei 0°C 500 mg (1.06 mmol) (+/-)-[4-(Brommethyl)phenyl](cyclopentyl)essigsäure-*tert*.-butylester zugegeben. Man rührte das Reaktionsgemisch weitere 30 min und versetzte dann mit Wasser und Essigsäureethylester. Die organische Phase wurde mit gesättigter Natriumchlorid-Lösung gewaschen und über Magnesiumsulfat getrocknet. Das Rohprodukt wurde durch präparative RP-HPLC gereinigt (Eluent: Acetonitril/Wasser-Gradient). Es wurden 217 mg (50.3% d. Th.) der Zielverbindung erhalten.
LC-MS (Methode 11): Rₜ = 1.53 min; m/z = 407 (M+H)⁺.
¹H-NMR (400 MHz, DMSO-*d*₆): δ = 10.67 (br. s, 1 H), 7.62 (d, 1 H), 7.53 (d, 1 H), 7.32 (t, 1 H), 7.24 (d, 2 H), 7.12 (d, 2 H), 6.99 (t, 1 H), 5.33 (s, 2 H), 3.14 (d, 1 H), 2.40-2.29 (m, 1 H), 1.84-1.72 (m, 1 H), 1.65-1.35 (m, 5 H), 1.33 (s, 9 H), 1.26-1.15 (m, 2 H).

### Beispiel 298A

### (+/-)-Cyclopentyl{4-[(3-oxo-1,3-dihydro-2H-indazol-2-yl)methyl]phenyl}essigsäure

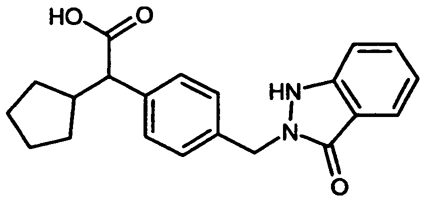

71.0 mg (0.17 mmol) (+/-)-Cyclopentyl{4-[(3-oxo-1,3-dihydro-2*H*-indazol-2-yl)methyl]phenyl}-essigsäure-*tert*.-butylester wurden in 400 µl Dichlormethan vorgelegt und mit 134 µl Trifluoressigsäure versetzt. Es wurde 2 h bei RT gerührt. Das Reaktionsgemisch wurde danach am Rotationsverdampfer eingeengt und der Rückstand im Hochvakuum getrocknet. Es wurden 82 mg Rohprodukt erhalten, welches ohne weitere Aufreinigung eingesetzt wurde.
LC-MS (Methode 15): Rₜ = 1.01 min; m/z = 350 (M+H)⁺.
¹H-NMR (400 MHz, DMSO-*d*₆): δ = 7.62 (d, 1 H), 7.53 (d, 1 H), 7.32 (t, 1 H), 7.24 (d, 2 H), 7.12 (d, 2 H), 6.99 (t, 1 H), 5.33 (s, 2 H), 3.17 (d, 1 H), 2.45-2.35 (m, 1 H), 1.86-1.75 (m, 1 H), 1.63-1.46 (m, 4 H), 1.44-1.33 (m, 1 H), 1.29-1.15 (m, 2 H), 0.96-0.85 (m, 1 H).

### Beispiel 299A

### 4,4,4-Trifluor-3-methyl-2-{4-[(1-oxo-1,3-dihydro-2H-isoindol-2-yl)methyl]phenyl}butansäure-ethylester

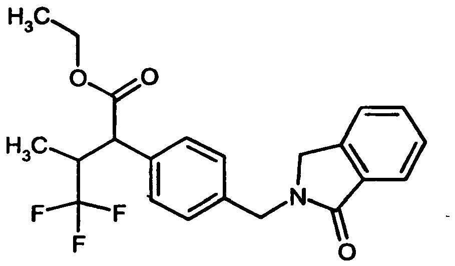

66.9 mg (1.67 mol, 60%-ig) Natriumhydrid in 2 ml DMF wurden bei 0°C mit 222.6 mg (1.67 mmol) 2,3-Dihydro-1*H*-isoindol-1-on versetzt. Es wurde 30 min gerührt und dann bei 0°C 600 mg (1.39 mmol) Ethyl-2-[4-(brommethyl)phenyl]-4,4,4-trifluor-3-methylbutanoat zugegeben. Man rührte das Reaktionsgemisch weitere 2 h, bevor mit Wasser und Essigsäureethylester versetzt wurde. Die organische Phase wurde mit gesättigter Natriumchlorid-Lösung gewaschen und über Magnesiumsulfat getrocknet. Das Rohprodukt wurde durch präparative RP-HPLC gereinigt (Eluent: Acetonitril/Wasser-Gradient). Es wurden 108 mg (19% d. Th.) der Zielverbindung erhalten.
LC-MS (Methode 12): Rₜ = 2.42 min; m/z = 406 (M+H)⁺.
¹H-NMR (400 MHz, DMSO-d₆): δ = 7.73 (d, 1 H), 7.62-7.47 (m, 3 H), 7.40-7.35 (m, 2 H), 7.30-7.22 (m, 2 H), 4.75-4.70 (m, 2 H), 4.41-4.36 (m, 2 H), 4.15-3.96 (m, 2 H), 3.82-3.68 (m, 1 H), 1.19-1.14 (m, 1 H), 1.13-1.08 (m, 3 H), 0.76 (d, 2 H).

### Beispiel 300A

### 4,4,4-Trifluor-3-methyl-2-{4-[(1-oxo-1,3-dihydro-2H-isoindol-2-yl)methyl]phenyl}butansäure

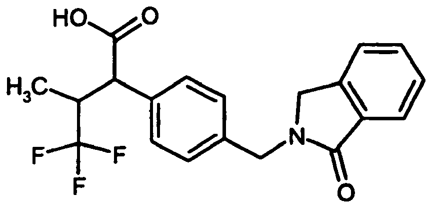

Unter Argon wurden 270 mg (0.66 mmol) 4,4,4-Trifluor-3-methyl-2-{4-[(1-oxo-1,3-dihydro-2*H-*isoindol-2-yl)methyl]phenyl}butansäureethylester in je 1 ml THF, Methanol und Wasser gelöst und mit 69.9 mg (1.66 mmol) Lithiumhydroxid versetzt. Es wurde über Nacht bei RT gerührt. Der Ansatz wurde mit Wasser verdünnt, mit 1 N Salzsäure sauer gestellt und der ausgefallene Niederschlag abgesaugt. Der Feststoff wurde mit Wasser nachgewaschen, bis das Waschwasser neutral blieb. Es wurden 222 mg (88.3% d. Th.) der Zielverbindung erhalten.
LC-MS (Methode 12): Rₜ = 2.05 min; m/z = 378 (M+H)⁺.
¹H-NMR (400 MHz, DMSO-*d*₆): δ = 12.72 (br. s, 1 H), 7.73 (d, 1 H), 7.63-7.54 (m, 2 H), 7.50 (t, 1 H), 7.35 (d, 2 H), 7.27 (d, 2 H), 4.73 (s, 2 H), 4.38 (s, 2 H), 3.63-3.57 (m, 1 H), 3.27-3.18 (m, 1 H), 0.75 (d, 3 H).

### Beispiel 301A

### (+/-)-Cyclopentyl {4-[(5,6-difluor-1-oxo-1,3-dihydro-2H-isoindol-2-yl)methyl]phenyl} essigsäure-tert.-butylester

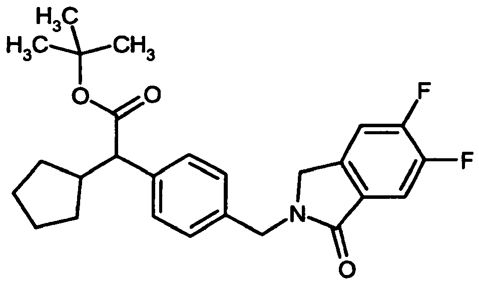

21.3 mg (0.53 mmol, 60%-ig) Natriumhydrid in 1 ml DMF wurden bei 0°C mit 60 mg (0.355 mmol) 5,6-Difluor-2,3-dihydro-1*H*-isoindol-1-on versetzt. Es wurde 30 min gerührt und dann bei 0°C 125.3 mg (0.35 mmol) (+/-)-[4-(Brommethyl)phenyl](cyclopentyl)essigsäure-*tert*.-butylester zugegeben. Man rührte weitere 2 h und versetzte das Reaktionsgemisch dann mit Wasser und Essigsäureethylester. Die organische Phase wurde mit gesättigter Natriumchlorid-Lösung gewaschen und über Magnesiumsulfat getrocknet. Das Rohprodukt wurde durch Chromatographie an Silicagel gereinigt (Laufmittel Cyclohexan/Essigsäureethylester 10:1 bis 2:1). Es wurden 30.0 mg (19.2% d. Th.) der Zielverbindung erhalten.
LC-MS (Methode 10): Rₜ = 2.75 min; m/z = 442 (M+H)⁺.

### Beispiel 302A

### (+/-)-Cyclopentyl {4-[(5,6-difluor-1-oxo-1,3-dihydro-2H-isoindol-2-yl)methyl]phenyl}essigsäure

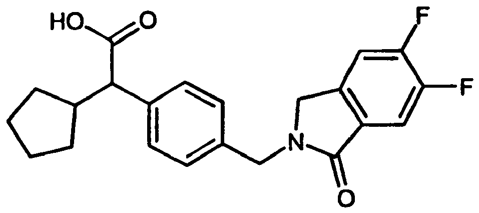

27.0 mg (0.06 mmol) (+/-)-Cyclopentyl{4-[(5,6-difluor-1-oxo-1,3-dihydro-2*H*-isoindol-2-yl)-methyl]phenyl}essigsäure-*tert.*-butylester wurden in 200 µl Dichlormethan vorgelegt und mit 94 µl Trifluoressigsäure versetzt. Es wurde 2 h bei RT gerührt. Das Reaktionsgemisch wurde dann am Rotationsverdampfer eingeengt und der Rückstand im Hochvakuum getrocknet. Es wurden 23.5 mg (99.7% d. Th.) der Zielverbindung erhalten.
LC-MS (Methode 15): Rₜ = 1.09 min; m/z = 386 (M+H)⁺.

### Beispiel 303A

### (+/-)-Cyclopentyl {4-[(4,7-difluor-1,3-dioxo-1,3-dihydro-2H-isoindol-2-yl)-methyl]phenyl}-essigsäure-tert.-butylester

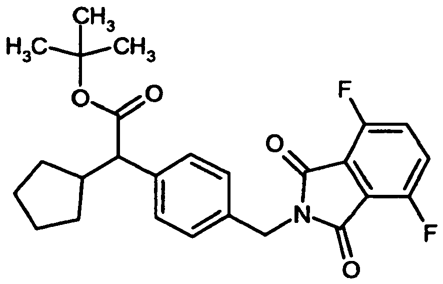

Zu einer Lösung von 964.7 mg (2.71 mmol) (+/-)-[4-(Brommethyl)phenyl](cyclopentyl)essigsäure-*tert*.-butylester in 2 ml DMF wurden 500 mg (2.71 mmol) 4,7-Difluor-1*H*-isoindol-1,3(2*H*)-dion und 889.7 mg (2.71 mmol) Cäsiumcarbonat hinzugefügt. Es wurde für 2 h bei 60°C gerührt und das Reaktionsgemisch dann auf Eiswasser gegeben. Man extrahierte dreimal mit Essigsäureethylester. Die vereinigten organischen Phasen wurden mit gesättigter Natriumchlorid-Lösung gewaschen und über Magnesiumsulfat getrocknet. Das Rohprodukt wurde durch Chromatographie an Silicagel gereinigt (Laufmittel Cyclohexan/Essigsäureethylester 10:1 bis 5:1). Es wurden 424 mg (34.1 % d. Th.) der Zielverbindung erhalten.
LC-MS (Methode 15): Rₜ = 1.47 min; m/z = 478 (M+Na)⁺.
¹H-NMR (400 MHz, DMSO-*d*₆): δ = 7.76 (t, 2 H), 7.31-7.24 (m, 4 H), 4.70 (s, 2 H), 3.18 (d, 1 H), 2.42-2.26 (m, 1H), 1.85-1.75 (m, 1H), 1.66-1.37 (m, 5 H), 1.34 (s, 9 H), 1.30-1.18 (m, 2 H).

### Beispiel 304A

### Cyclopentyl {4-[(4,7-difluor-1-hydroxy-3-oxo-1,3-dihydro-2H-isoindol-2-yl)methyl]phenyl}-essigsäure-tert.-butylester

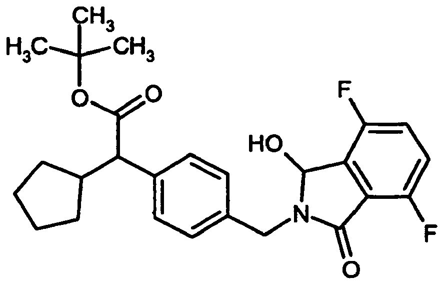

Eine Lösung von 200.0 mg (0.44 mmol) (+/-)-Cyclopentyl{4-[(4,7-difluor-1,3-dioxo-1,3-dihydro-2H-isoindol-2-yl)methyl]phenyl}essigsäure-*tert.-*butylester in 1 ml Ethanol wurde auf 0°C gekühlt und mit 18.3 mg (0.48 mmol) Natriumborhydrid versetzt. Es wurden 0.2 ml Dichlormethan zugegeben und das Reaktionsgemisch auf RT erwärmt. Nach 1 h Rühren wurde das Gemisch eingeengt und der Rückstand durch Chromatographie an Silicagel gereinigt (Laufmittel Cyclohexan/Essigsäureethylester 6:1 bis 4:1). Es wurden 182 mg (90.6% d. Th.) der Zielverbindung erhalten.
LC-MS (Methode 11): Rₜ = 1.49 min; m/z = 458 (M+H)⁺.
¹H-NMR (400 MHz, DMSO-*d₆*): δ = 7.55-7.49 (m, 1 H), 7.41 (td, 1 H), 7.32-7.20 (m, 4 H), 7.03 (dd, 1 H), 5.91-5.82 (m, 1 H), 4.80 (d, 1 H), 4.34 (d, 1 H), 3.18 (d, 1 H), 2.43-2.32 (m, 1 H), 1.86-1.76 (m, 1 H), 1.67-1.46 (m, 3 H), 1.45-1.37 (m, 1 H), 1.35 (s, 9 H), 1.31-1.18 (m, 2 H), 0.95 (dd, 1 H).

### Beispiel 305A

### (+/-)-Cyclopentyl{4-[(4,7-difluor-1-oxo-1,3-dihydro-2H-isoindol-2-yl)methyl]phenyl}essigsäure

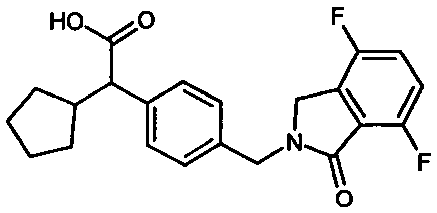

90.0 mg (0.20 mmol) Cyclopentyl{4-[(4,7-difluor-1-hydroxy-3-oxo-1,3-dihydro-2*H*-isoindol-2-yl)-methyl]phenyl}essigsäure-*tert*.-butylester wurden in 1 ml Dichlormethan vorgelegt und mit 300 µl Trifluoressigsäure sowie 62 µl (0.39 mmol) Triethylsilan versetzt. Es wurde 2 h bei RT gerührt. Das Reaktionsgemisch wurde danach am Rotationsverdampfer eingeengt, der Rückstand in Essigsäureethylester aufgenommen und die Lösung mit Wasser und gesättigter Natriumchlorid-Lösung gewaschen. Die organische Phase wurde über Magnesiumsulfat getrocknet und im Vakuum eingeengt. Es wurden 77.0 mg der Zielverbindung erhalten.
LC-MS (Methode 11): Rₜ = 1.21 min; m/z = 386 (M+H)⁺.
¹H-NMR (400 MHz, DMSO-*d*₆): δ = 7.53-7.45 (m, 1 H), 7.38-7.32 (m, 1 H), 7.31 (d, 2 H), 7.25 (d, 2 H), 4.66 (s, 2 H), 4.48 (s, 2 H), 2.47-2.31 (m, 2 H), 1.87-1.77 (m, 1 H), 1.65-1.17 (m, 7 H).

### Beispiel 306A

### (+/-)-Cyclopentyl{4-[(4-fluor-1-oxo-1,3-dihydro-2H-isoindol-2-yl)methyl]phenyl}essigsäure-tert.-butylester

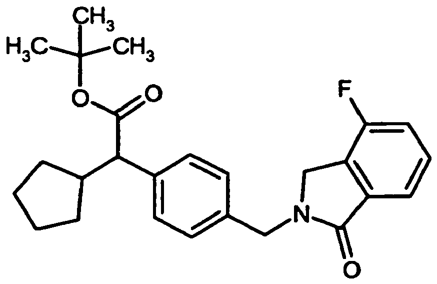

Eine Suspension von 96.3 mg (2.41 mmol, 60%-ig) Natriumhydrid in 1 ml DMF wurde bei 0°C mit 280 mg (1.85 mmol) 4-Fluor-2,3-dihydro-1*H*-isoindol-1-on versetzt. Es wurde 30 min gerührt und dann bei 0°C 654.5 mg (1.85 mmol) (+/-)-[4-(Brommethyl)phenyl](cyclopentyl)essigsäure-*tert*.-butylester zugegeben. Man rührte weitere 2 h, bevor das Reaktionsgemisch mit Wasser und Essigsäureethylester versetzt wurde. Die organische Phase wurde mit gesättigter Natriumchlorid-Lösung gewaschen und über Magnesiumsulfat getrocknet. Das Rohprodukt wurde durch Chromatographie an Silicagel gereinigt (Laufmittel Cyclohexan/Essigsäureethylester 6:1 bis 5:1). Es wurden 394 mg (50.2% d. Th.) der Zielverbindung erhalten.
LC-MS (Methode 15): Rₜ = 1.44 min; m/z = 446 (M+Na)⁺.
¹H-NMR (400 MHz, DMSO-*d*₆): δ = 7.62-7.52 (m, 2 H), 7.45 (dt, 1 H), 7.30 (d, 2 H), 7.24 (d, 2 H), 4.71 (s, 2 H), 4.49 (s, 2 H), 3.18 (d, 1 H), 2.42-2.32 (m, 1 H), 1.80 (dd, 1 In, 1.66-1.36 (m, 5 H), 1.34 (s, 9 H), 1.30-1.14 (m, 2 H).

### Beispiel 307A

### (+/-)-Cyclopentyl{4-[(4-fluor-1-oxo-1,3-dihydro-2H-isoindol-2-yl)methyl]phenyl}essigsäure

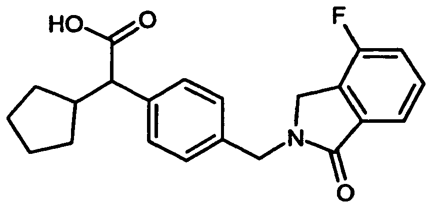

150.0 mg (0.35 mmol) (+/-)-Cyclopentyl{4-[(4-fluor-1-oxo-1,3-dihydro-2*H*-isoindol-2-yl)methyl]-phenyl}essigsäure-*tert*.-butylester wurden in 300 µl Dichlormethan vorgelegt und mit 515 µl Trifluoressigsäure versetzt. Es wurde 1 h bei RT gerührt. Das Reaktionsgemisch wurde dann am Rotationsverdampfer eingeengt und der Rückstand im Hochvakuum getrocknet. Das Produkt wurde mit Acetonitril versetzt und der ausgefallene Feststoff verrührt und dann abgesaugt. Es wurden 112.0 mg (86. 1 % d. Th.) der Zielverbindung erhalten.
LC-MS (Methode 15): Rₜ = 1.08 min; m/z = 368 (M+H)⁺.
¹H-NMR (400 MHz, DMSO-*d*₆): δ = 12.25 (br. s, 1 H) 7.62-7.54 (m, 2 H), 7.44 (t, 1 H), 7.30 (d, 2 H), 7.25 (d, 2 H), 4.71 (s, 2 H), 4.51 (s, 2 H), 3.21 (d, 1 H), 2.48-2.40 (m, 1 H), 1.98-1.80 (m, 1 H), 1.66-1.48 (m, 3 H), 1.45-1.38 (m, 1 H), 1.32-1.18 (m, 2 H), 0.99-0.90 (m, 1H).

### Beispiel 308A

### (-)-(1R,2S,5R)-5-Methyl-2-(propan-2-yl)cyclohexyl-(2S)-{4-[(5-chlor-2-oxopyridin-1(2H)-yl)-methyl]phenyl} (cyclopentyl)ethanoat

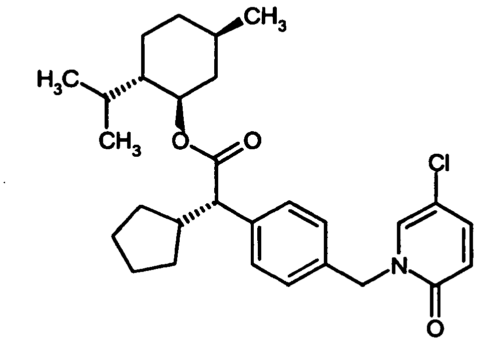

Eine Suspension von 125.4 mg (3.14 mmol, 60%-ig) Natriumhydrid in 5 ml DMF wurde bei RT mit 406.1 mg (3.14 mmol) 5-Chlorpyridin-2(1*H*)-on versetzt. Es wurde 30 min gerührt und dann bei RT 1050 mg (2.41 mmol) (1*R*,2*S*,5*R*)-5-Methyl-2-(propan-2-yl)cyclohexyl-(2*S*)-[4-(brommethyl)phenyl](cyclopentyl)ethanoat zugegeben. Man rührte weitere 2 h bei RT und gab das Reaktionsgemisch dann auf Wasser. Der ausgefallene Feststoff wurde abgesaugt und mit Wasser und Isohexan gewaschen. Es wurden 830 mg (71% d. Th.) der Zielverbindung-erhalten.
LC-MS (Methode 15): Rₜ =1.60 min; m/z = 484 (M+H)⁺.
¹H-NMR (400 MHz, DMSO-*d*₆): δ = 8.10 (d, 1 H), 7.49 (dd, 1 H), 7.30 (d, 2 H), 7.25 (d, 2 H), 6.46 (d, 1 H), 5.04 (s, 2 H), 4.53 (td, 1 H), 2.47-2.38 (m, 1 H), 1.84-1.69 (m, 2 H), 1.68-1.13 (m, 12 H), 1.03-0.91 (m, 2 H), 0.84-0.75 (m, 8 H), 0.61 (d, 3 H).
[α]_{D}²⁰ = -27°, c = 0.540, Chloroform.

### Beispiel 309A

### (+)-(2S)-{4-[(5-Chlor-2-oxopyridin-1(2H)-yl)methyl]phenyl}(cyclopentyl)essigsäure

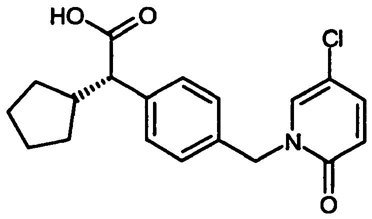

800.0 mg (1.65 mmol) (1*R*,2*S*,5*R*)-5-Methyl-2-(propan-2-yl)cyclohexyl-(2*S*)-{4-[(5-chlor-2-oxo-pyridin-1(2*H*)-yl)methyl]phenyl}(cyclopentyl)ethanoat wurden mit 50 ml Trifluoressigsäure versetzt. Es wurde 48 h unter Rückfluss gerührt. Das Reaktionsgemisch wurde dann am Rotationsverdampfer eingeengt und der Rückstand im Hochvakuum getrocknet. Das Rohprodukt wurde durch Chromatographie an Silicagel gereinigt (Laufmittel Cyclohexan/Essigsäureethylester 4:1 → 2:1 → 1:1). Es wurden 400 mg (70.0% d. Th.) der Zielverbindung erhalten.
LC-MS (Methode 15): Rₜ = 1.01 min; m/z = 346 (M+H)⁺.
¹H-NMR (400 MHz, DMSO-*d*₆): δ = 12.25 (s, 1 H), 8.11 (d, 1 H), 7.49 (dd, 1 H), 7.29 (d, 2 H), 7.24 (d, 2 H), 6.46 (d, 1 H), 5.04 (s, 2 H), 3.21 (d, 1 H), 2.47-2.35 (m, 1 H), 1.82 (dd, 1 H), 1.66-1.17 (m, 6 H), 1.00-0.88 (m, 1 H).
[α]_{D}²⁰ = + 39.5°, c = 0.505, Chloroform.

### Beispiel 310A

### (+/-)-Cyclopentyl(4- {[2-oxo-5-(trifluormethyl)pyridin-1(2H)-yl]methyl}phenyl)essigsäure-tert.-butylester

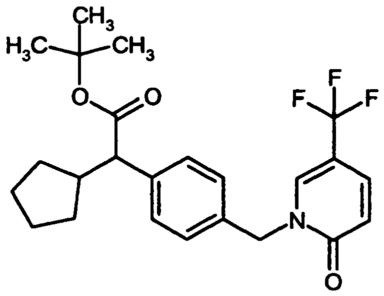

300 mg (1.84 mmol) 5-(Trifluormethyl)pyridin-2(1*H*)-on in 2.5 ml DMF wurden mit 73.6 mg (1.84 mmol, 60%-ig) Natriumhydrid versetzt. Nach 30 min Rühren wurden 500 mg (1.42 mmol) (+/-)-[4-(Brommethyl)phenyl](cyclopentyl)essigsäure-*tert*.-butylester zugegeben. Nach weiteren 2 h Rühren bei RT wurde das Reaktionsgemisch für 1 h auf 40°C erwärmt. Das Gemisch wurde dann mit Wasser und Essigsäureethylester versetzt und die Phasen getrennt. Die organische Phase wurde mit gesättigter Natriumchlorid-Lösung gewaschen und über Magnesiumsulfat getrocknet. Das Rohprodukt wurde durch Chromatographie an Silicagel gereinigt (Laufmittel Cyclohexan/ Essigsäureethylester 6:1 bis 4:1). Es wurden 522.0 mg (84.7% d. Th.) der Zielverbindung erhalten.
LC-MS (Methode 11): Rₜ = 1.62 min; m/z = 380 (M+H-C₄H₈)⁺.
¹H-NMR (400 MHz, DMSO-*d*₆): δ = 8.54 (s, 1 H), 7.70 (dd, 1 H), 7.30 (d, 2 H), 7.24 (d, 2 H), 6.57 (d, 1 H), 5.18-5.07 (m, 2 H), 3.18 (d, 1 H), 2.44-2.30 (m, 1 H), 1.88-1.74 (m, 1 H), 1.67-1.38 (m, 4 H), 1.37-1.33 (m, 9 H), 1.30-1.16 (m, 2 H), 1.00-0.86 (m, 1 H).

### Beispiel 311A

### Cyclopentyl(4-{[2-oxo-5-(trifluormethyl)pyridin-1(2H)-yl]methyl}phenyl)essigsäure

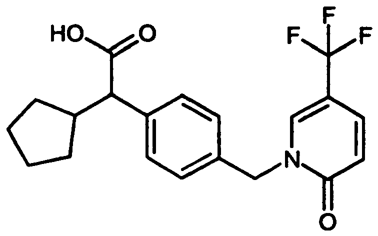

390 mg (0.90 mmol) (+/-)-Cyclopentyl(4-{[2-oxo-5-(trifluormethyl)pyridin-1(2*H*)-yl]methyl}-phenyl)ethansäure-*tert*.-butylester wurden in 2.7 ml Dichlormethan vorgelegt und mit 690 µl Trifluoressigsäure versetzt. Das Reaktionsgemisch wurde 2 h bei RT gerührt, dann im Vakuum eingeengt und der Rückstand im Hochvakuum getrocknet. Es wurden 523 mg an Produkt erhalten, welches ohne weitere Aufreinigung eingesetzt wurde.
LC-MS (Methode 11): Rₜ = 1.23 min; m/z = 380 (M+H)⁺.
¹H-NMR (400 MHz, DMSO-*d*₆): δ = 8.56 (s, 1 H), 7.69 (dd, 1 H), 7.30 (d, 2 H), 7.25 (d, 2 H), 6.57 (d, 1 H), 5.13 (s, 2 H), 3.21 (d, 1 H), 2.47-2.36 (m, 1 H), 1.91-1.76 (m, 1 H), 1.68-1.13 (m, 6 H), 0.92 (dq, 1 H).

### Beispiel 312A

### (-)-(1R,2S,5R)-5-Methyl-2-(propan-2-yl)cyclohexyl-(2S)-cyclopentyl(4-{[2-oxo-5-(trifluormethyl)-pyridin-1(2H)-yl]methyl}phenyl)ethanoat

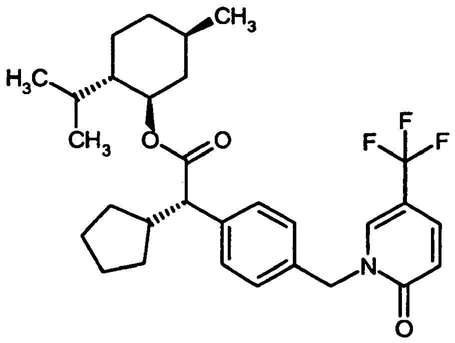

238.8 mg (5.97 mmol, 60%-ig) Natriumhydrid in 5 ml DMF wurden bei RT mit 973.8 mg (5.97 mmol) 5-(Trifluormethyl)pyridin-2(1*H*)-on versetzt. Es wurde 30 min gerührt und dann bei RT 2.0 g (4.59 mmol) (1*R*,2*S*,S*R*)-5-Methyl-2-(propan-2-yl)cyclohexyl-(2*S*)-[4-(brommethyl)phenyl]-(cyclopentyl)ethanoat zugegeben. Man rührte weitere 2 h bei RT. Das Reaktionsgemisch wurde dann mit Wasser und Essigsäureethylester versetzt. Nach Phasentrennung wurde die organische Phase mit gesättigter Natriumchlorid-Lösung gewaschen und über Magnesiumsulfat getrocknet. Das Rohprodukt wurde durch Chromatographie an Silicagel gereinigt (Laufmittel Cyclohexan/ Essigsäureethylester 5:1). Es wurden 2.0 g (85.0% d. Th.) der Zielverbindung erhalten.
LC-MS (Methode 15): Rₜ = 1.65 min; m/z = 518 (M+H)⁺.
¹H-NMR (400 MHz, DMSO-*d*₆): δ = 8.52 (s, 1 H), 7.69 (dd, 1 H), 7.31 (d, 2 H), 7.26 (d, 2 H), 6.57 (d, 1 H), 5.13 (s, 2 H), 4.52 (td, 1 H), 2.48-2.42 (m, 2 H), 1.82-1.13 (m, 13 H), 1.00-0.90 (m, 2 H), 0.83-0.74 (m, 8 H), 0.59 (d, 3 H).
[α]_{D}²⁰ = -21.2°, c = 0.515, Chloroform.

### Beispiel 313A

### (+)-(2S)-Cyclopentyl-(4-{[2-oxo-5-(trifluormethyl)pyridin-1(2H)-yl]methyl}phenyl)essigsäure

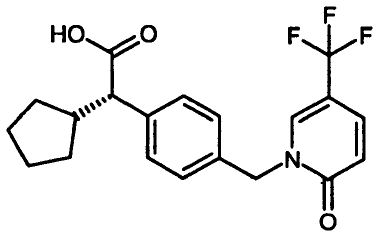

1.0 g (1.93 mmol) (1*R*,2*S*,5*R*)-5-Methyl-2-(propan-2-yl)cyclohexyl-(2*S*)-cyclopentyl(4-{[2-oxo-5-(trifluormethyl)pyridin-1(2*H*)-yl]methyl}phenyl)ethanoat wurden mit 50 ml Trifluoressigsäure versetzt. Es wurde 48 h unter Rückfluss gerührt. Das Reaktionsgemisch wurde dann im Vakuum eingeengt und der Rückstand im Hochvakuum getrocknet. Das Rohprodukt wurde durch Chromatographie an Silicagel gereinigt (Laufmittel Cyclohexan/Essigsäureethylester 4:1 → 2:1 → 1:1). Es wurden 670 mg (91.4% d. Th.) der Zielverbindung erhalten.
LC-MS (Methode 15): Rₜ = 1.07 min; m/z = 380 (M+H)⁺.
¹H-NMR (400 MHz, DMSO-*d*₆): δ = 8.56 (s, 1 H), 7.69 (dd, 1 H), 7.30 (d, 2 H), 7.25 (d, 2 H), 6.57 (d, 1 H), 5.13 (s, 2 H), 3.21 (d, 1 H), 2.47-2.36 (m, 1 H), 1.87-1.77 (m, 1 H), 1.67-1.15 (m, 7 H), 0.99-0.88 (m, 1 H).
[α]_{D}²⁰ = + 19.9°, c = 0.530, Chloroform.

### Beispiel 314A

### 4,4,4-Trifluor-2-{4-[(4-fluor-1-oxo-1,3-dihydro-2H-isoindol-2-yl)methyl]phenyl}-3-methylbutansäureethylester

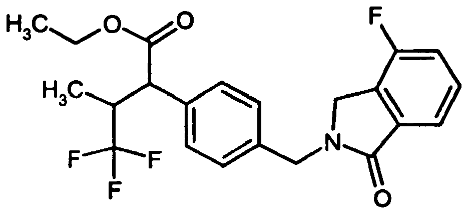

170.7 mg (4.27 mmol, 60%-ig) Natriumhydrid in 4 ml DMF wurden bei 0°C mit 645 mg (4.27 mmol) 4-Fluor-2,3-dihydro-1*H*-isoindol-1-on versetzt. Es wurde 30 min gerührt und dann bei 0°C 2.11 g (5.98 mmol) Ethyl-2-[4-(brommethyl)phenyl]-4,4,4-trifluor-3-methylbutanoat zugegeben. Man rührte weitere 2 h und versetzte das Reaktionsgemisch dann mit Wasser und Essigsäureethylester. Die organische Phase wurde mit gesättigter Natriumchlorid-Lösung gewaschen und über Magnesiumsulfat getrocknet. Das Rohprodukt wurde durch Chromatographie an Silicagel gereinigt (Laufmittel Cyclohexan/Essigsäureethylester 40:1 bis 3:1). Es wurden 856 mg (33.9% d. Th.) der Zielverbindung erhalten.
LC-MS (Methode 15): Rₜ = 1.24 min; m/z = 424 (M+H)⁺.
¹H-NMR (400 MHz, DMSO-*d*₆): δ = 7.62-7.53 (m, 2 H), 7.45 (t, 1 H), 7.38 (d, 2 H), 7.29 (d, 2 H), 4.73 (s, 2 H), 4.50 (s, 2 H), 4.14-3.95 (m, 2 H), 3.71 (d, 1 H), 1.14-1.08 (m, 4 H), 0.76 (d, 3 H).

### Beispiel 315A

### 4,4,4-Trifluor-2-{4-[(4-fluor-1-oxo-1,3-dihydro-2H-isoindol-2-yl)methyl]phenyl}-3-methylbutansäure

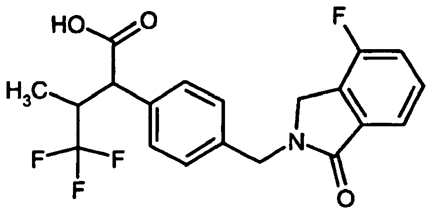

855 mg (2.02 mmol) 4,4,4-Trifluor-2-{4-[(4-fluor-1-oxo-1,3-dihydro-2*H*-isoindol-2-yl)methyl]-phenyl}-3-methylbutansäureethylester wurden in je 5.7 ml THF, Methanol und Wasser gelöst und mit 16.2 ml (40.39 mmol) 10%-iger Natronlauge versetzt. Es wurde über Nacht bei RT gerührt.

Der Ansatz wurde dann mit Wasser verdünnt, mit 1 N Salzsäure sauer gestellt und mit Essigsäureethylester extrahiert. Die organische Phase wurde über Natriumsulfat getrocknet und eingeengt. Das Rohprodukt wurde durch präparative RP-HPLC gereinigt (Eluent: Acetonitril/Wasser-Gradient). Es wurden 457.2 mg (58.2% d. Th.) der Zielverbindung erhalten.
LC-MS (Methode 15): Rₜ = 1.01 min; m/z = 396 (M+H)⁺.
¹H-NMR (400 MHz, DMSO-*d*₆): δ = 12.72 (br. s, 1 H), 7.62-7.54 (m, 2 H), 7.48-7.41 (m, 1 H), 7.36 (d, 2 H), 7.29 (d, 2 H), 4.73 (s, 2 H), 4.50 (s, 2 H), 3.61 (d, 1 H), 3.28-3.19 (m, 1 H), 0.76 (d, 3 H).

### Beispiel 316A

### 4,4,4-Trifluor-3-methyl-2-(4-{[2-oxo-5-(trifluormethyl)pyridin-1(2H)-yl]methyl}phenyl)butansäureethylester (Isomerengemisch)

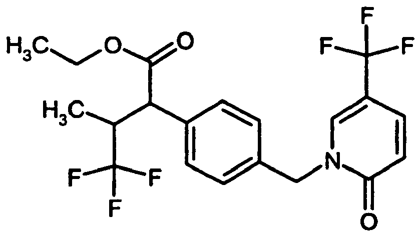

1.80 g (11.04 mmol) 5-(Trifluormethyl)pyridin-2(1*H*)-on wurden in 2.5 ml DMF vorgelegt und mit 0.44 g (11.04 mmol, 60%-ig) Natriumhydrid versetzt. Es wurde 30 min gerührt und dann mit 3.0 g (8.49 mmol) Ethyl-2-[4-(brommethyl)phenyl]-4,4,4-trifluor-3-methylbutanoat versetzt. Nach weiteren 2 h bei RT wurde das Reaktionsgemisch mit Wasser versetzt und mit Essigsäureethylester extrahiert. Die organische Phase wurde mit gesättigter Natriumchlorid-Lösung gewaschen und über Magnesiumsulfat getrocknet. Das Rohprodukt wurde durch Chromatographie an Silicagel gereinigt (Laufmittel Cyclohexan/Essigsäureethylester 10:1 bis 4:1). Es wurden 2.89 g (78.2% d. Th.) der Zielverbindung erhalten.
LC-MS (Methode 15): Rₜ = 1.19 min; m/z = 436 (M+H)⁺.
¹H-NMR (400 MHz, DMSO-*d*₆): δ = 8.61-8.54 (m, 1 H), 7.70 (dd, 1 H), 7.38 (d, 2 H), 7.29 (d, 2 H), 6.57 (d, 1 H), 5.18-5.07 (m, 2 H), 4.17-3.94 (m, 2 H), 3.71 (d, 1 H), 3.31-3.16 (m, 1 H), 1.14-1.05 (m, 3 H), 0.75 (d, 3 H).

### Beispiel 317A

### 4,4,4-Trifluor-3-methyl-2-(4-{[2-oxo-5-(trifluormethyl)pyridin-1(2H)-yl]methyl}phenyl)butansäure (Isomerengemisch)

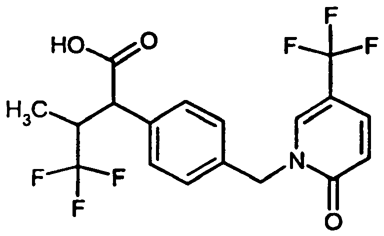

2.80 g (6.43 mmol) 4,4,4-Trifluor-3-methyl-2-(4-{[2-oxo-5-(trifluormethyl)pyridin-1(2*H*)-yl]-methyl}phenyl)butansäureethylester wurden in je 18.2 ml THF, Methanol und Wasser gelöst und mit 51.5 ml (128.6 mmol) 10%-iger Natronlauge versetzt. Es wurde über Nacht bei RT gerührt. Die Reaktionsmischung wurde dann mit Wasser verdünnt, mit 1 N Salzsäure sauer gestellt und mit Essigsäureethylester extrahiert. Die organische Phase wurde über Natriumsulfat getrocknet und eingeengt. Es wurden 2.82 g an Produkt erhalten, welches ohne weitere Aufreinigung eingesetzt wurde.
LC-MS (Methode 15): Rₜ = 1.02 min; m/z = 408 (M+H)⁺.
¹H-NMR (400 MHz, DMSO-d₆): δ = 12.70 (br. s, 1 H), 8.61-8.54 (m, 1 H), 7.70 (dd, 1 H), 7.35 (d, 2 H), 7.28 (d, 2 H), 6.57 (d, 1 H), 5.15 (s, 2 H), 3.61 (d, 1 H), 3.29-3.18 (m, 1 H), 0.75 (d, 3 H).

Das oben erhaltene Isomerengemisch wurde durch präparative HPLC an chiraler Phase aufgetrennt [Säule: Daicel Chiralpak AD-H, 5 µm, 250 mm x 20 mm; Fluss: 15 ml/min; UV-Detektion: 220 nm; Injektionsvolumen: 0.2 ml; Temperatur: 27°C; Eluent: 75% Isohexan / 25% Ethanol (mit 0.2% TFA und 1% Wasser)]. Ausgehend von 2810 mg Isomerengemisch wurden 867 mg von Enantiomer 1 und 776 mg von Enantiomer 2 isoliert *(siehe Beispiele 318A und 319A).*

### Beispiel 318A

### (-)-(2R,3S)-4,4,4-Trifluor-3-methyl-2-(4-{[2-oxo-5-(trifluormethyl)pyridin-1(2H)-yl]methyl}-phenyl)butansäure (Enantiomer 1)

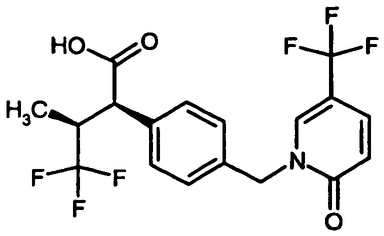

¹H-NMR (400 MHz, DMSO-*d*₆): δ = 8.57 (s, 1 H), 7.70 (dd, 1 H), 7.36 (d, 2 H), 7.29 (d, 2 H), 6.57 (d, 1 H), 5.15 (s, 2 H), 3.60 (d, 1 H), 3.30-3.17 (m, 1 H), 0.75 (d, 3 H).
[α]_{D}²⁰ = -45.9°, c = 0.335, Chloroform.

### Beispiel 319A

### (+)-(2S,3R)-4,4,4-Trifluor-3-methyl-2-(4-{[2-oxo-5-(trifluormethyl)pyridin-1(2H)-yl]methyl}-phenyl)butansäure (Enantiomer 2)

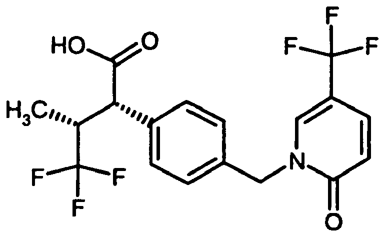

LC-MS (Methode 15): Rₜ = 1.02 min; m/z = 408 (M+M)⁺.
¹H-NMR (400 MHz, DMSO-*d*₆): δ = 8.57 (s, 1 H), 7.70 (dd, 1 H), 7.35 (d, 2 H), 7.29 (d, 2 H), 6.57 (d, 1 H), 5.15 (s, 2 H), 3.60 (d, 1 H), 3.31-3.18 (m, 1 H), 0.75 (d, 3 H).
[α]_{D}²⁰ = +44.8°, c = 0.400, Chloroform.

### Beispiel 320A

### 4,4,4-Trifluor-3-methyl-2-{4-[(5-oxo-2-phenyl-5,6-dihydro-4H-1,3,4-oxadiazin-4-yl)methyl]-phenyl}butansäure

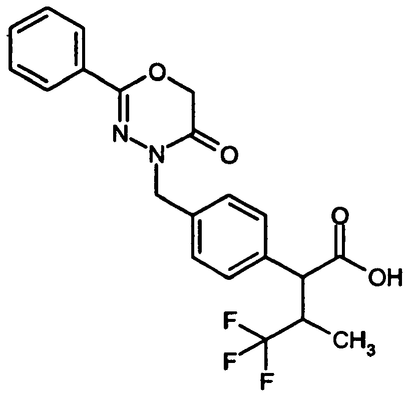

Eine Lösung von 1283 mg (2.86 mmol) Ethyl-4,4,4-trifluor-3-methyl-2-{4-[(5-oxo-2-phenyl-5,6-dihydro-4*H*-1,3,4-oxadiazin-4-yl)methyl]phenyl}butanoat in 10 ml Dioxan wurde mit 11.4 ml (11.4 mmol) 1 N Natronlauge versetzt und über Nacht bei 80°C gerührt. Nach vollständiger Umsetzung wurde das Dioxan im Vakuum abgezogen, die verbleibende Lösung mit Wasser verdünnt und anschließend mit 1 M Salzsäure auf pH 2 gestellt. Der ausgefallene Feststoff wurde abfiltriert, mit Wasser gewaschen und über Nacht im Vakuum bei 45°C getrocknet. Es wurden 1058 mg (2.52 mmol, 88% d. Th.) der Titelverbindung als Isomerengemisch erhalten.
LC-MS (Methode 15): Rₜ = 1.12 min; m/z = 421 (M+H)⁺ (Diastereomer 1); Rₜ = 1.13 min; m/z = 421 (M+H)⁺ (Diastereomer 2).

Analog zu den Beispielen 51A, 72A bzw. 320A wurden die in der folgenden Tabelle aufgeführten Verbindungen hergestellt:

| **Beispiel** | **Name / Struktur / Edukt** | **Analytische Daten** |
|---|---|---|
| **321A** | 6,6,6-Trifluor-4-methyl-2-{4-[(5-oxo-2-phenyl-5,6-dihydro-4*H*-1,3,4-oxadiazin-4-yl)methyl]phenyl}-hexansäure | LC-MS (Methode 7): Rₜ = 2.57 min; m/z = 449 (M+H)⁺. |
| | 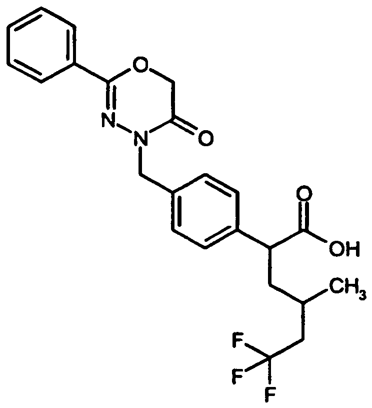 | |
| | (aus *tert*.-Butyl-6,6,6-trifluor-4-methyl-2-{4-[(5-oxo-2-phenyl-5,6-dihydro-4*H*-1,3,4-oxadiazin-4-yl)methyl]-phenyl}hexanoat) | |
| **322A** | 5,5,5-Trifluor-3-methyl-2-{4-[(5-oxo-2-phenyl-5,6-dihydro-4*H*-1,3,4-oxadiazin-4-yl)methyl]phenyl}-pentansäure | LC-MS (Methode 15): Rₜ = 1.15 min; m/z = 435 (M+H)⁺. |
| | 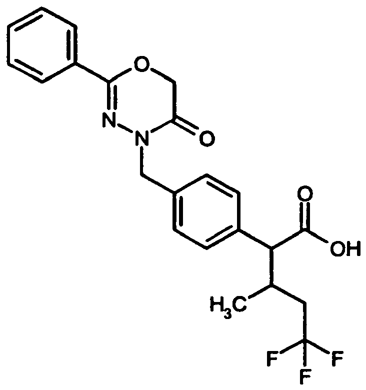 | |
| | (aus *tert*.-Butyl-5,5,5-trifluor-3-methyl-2-{4-[(5-oxo-2-phenyl-5,6-dihydro-4*H*-1,3,4-oxadiazin-4-yl)methyl]-phenyl}pentanoat) | |
| **323A** | {4-[(3-Chlor-6-oxopyridazin-1(6*H*)-yl)methyl]phenyl}-(cyclopentyl)essigsäure | LC-MS (Methode 11): Rₜ = 1.41 min; m/z = 347 (M+H)⁺. |
| | 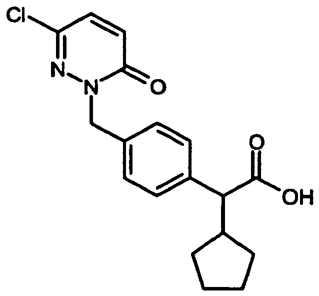 | |
| | (aus *tert*.-Butyl-{4-[(3-chlor-6-oxopyridazin-1(6*H*)-yl)-methyl]phenyl} (cyclopentyl)acetat) | |
| **324A** | 2-{4-[(5-Oxo-2-phenyl-5,6-dihydro-4*H*-1,3,4-oxadiazin-4-yl)methyl]phenyl}-3-(trifluormethyl)pentansäure | LC-MS (Methode 15): Rₜ = 1.18 min; m/z = 435 (M+H)⁺. |
| | 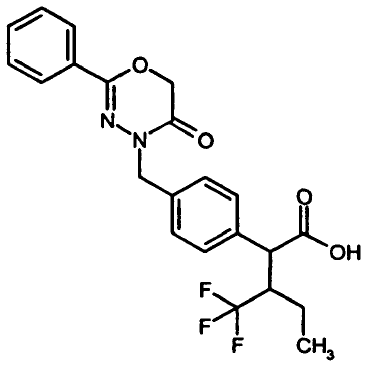 | |
| | (aus Ethyl-2-{4-[(5-oxo-2-phenyl-5,6-dihydro-4*H*-1,3,4-oxadiazin-4-yl)methyl]phenyl}-3(trifluormethyl)-pentanoat) | |
| **325A** | Cyclopentyl{4-[(3-methyl-6-oxopyridazin-1(6*H*)-yl)-methyl]phenyl} essigsäure | LC-MS (Methode 12): Rₜ = 1.96 min; m/z = 327 (M+H)⁺. |
| | 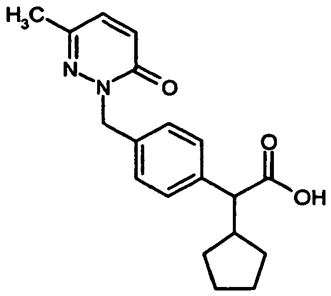 | |
| | (aus t*ert*.-Butyl-cyclopentyl {4-[(3-methyl-6-oxo-pyridazin-1(6*H*)-yl)methyl]phenyl}acetat) | |
| **326A** | Cyclopentyl(4-{[6-oxo-3-(trifluormethyl)pyridazin-1(6*H*)-yl]methyl}phenyl)essigsäure | LC-MS (Methode 12): Rₜ = 2.27 min; m/z = 381 (M+H)⁺. ¹H-NMR (400 MHz, DMSO-d₆): δ = 12.24 (br. s, 1H), 7.86 (d, 1H), 7.31 (d, 2H), 7.25 (d, 2H), 7.20 (d, 1H), 5.28 (s, 2H), 3.22 (d, 1H), 2.3 8-2.46 (m, 1H), 1.77-1.89 (m, 1H), 1.56-1.67 (m, 1H), 1.48-1.56 (m, 2H), 1.34-1.46 (m, 1H), 1.14-1.32 (m, 2H), 0.86-0.99 (m, 1H). |
| | 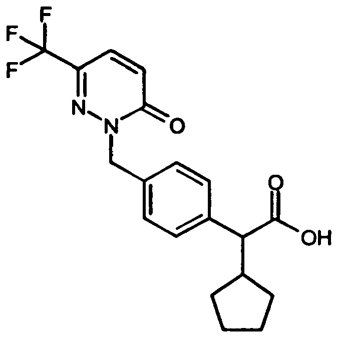 | |
| | (aus *tert*.-Butyl-cyclopentyl(4-{[6-oxo-3-(trifluor-methyl)pyridazin-1(6*H*)-yl)methyl}phenyl)acetat) | |
| **327A** | Cyclopentyl {4-[(5-methyl-6-oxopyridazin-1(6*H*)-yl)-methyl]phenyl}essigsäure | LC-MS (Methode 15): Rₜ = 0.98 min; m/z = 326 (M+H)⁺. ¹H-NMR (400 MHz, DMSO-d₆): δ = 12.24 (s, 1H), 7.64 (d, 1H), 7.26-7.35 (m, 3H), 7.21 (d, 2H), 6.15 (t, 1H), 5.07 (s, 2H), 3.20 (d, 1H), 2.35-2.47 (m, 1H), 2.00 (s, 3H), 1.82 (dd, 1H), 1.47-1.65 (m, 3H), 1.34-1.46 (m, 1H), 1.16-1.31 (m, 2i-n, 0.87-0.99 (m, 1H). |
| | 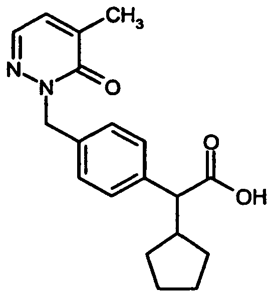 | |
| | (aus *tert*.-Butyl-cyclopentyl{4-[(5-methyl-6-oxo-pyridazin-1(6*H*)-yl)methyl]phenyl}acetat) | |
| **328A** | Cyclopentyl(4-{[6-oxo-4-(trifluormethyl)pyridazin-1(6*H*)-yl]methyl}phenyl)essigsäure | LC-MS (Methode 11): Rₜ = 1.22 min; m/z = 380 (M+H)⁺. ¹H-NMR (400 MHz, DMSO-d₆): δ = 12.24 (s, 1H), 8.08 (d, 1H), 7.30 (d, 2H), 7.25 (d, 2H), 6.81 (s, 1H), 6.51 (dd, 1H), 5.13 (s, 2H), 3.21 (d, 1H), 2.36-2.47 (m, 1H), 1.82 (dd, 1H), 1.47-1.65 (m, 3H), 1.35-1.46 (m, 1H), 1.15-1.31 (m, 2H), 0.87-0.98 (m, 1H). |
| | 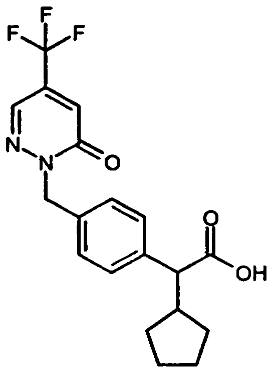 | |
| | (aus *tert*.-Butyl-cyclopentyl(4-{[6-oxo-4-(trifluor-methyl)pyridazin-1(6*H*)-yl]methyl}phenyl)acetat) | |
| **329A** | (3,3-Difluorcyclopentyl) {4-[(5-oxo-2-phenyl-5,6-dihydro-4*H*-1,3,4-oxadiazin-4-yl)methyl]phenyl}-essigsäure | LC-MS (Methode 11): Rₜ = 1.28 min; m/z = 429 (M+H)⁺. |
| | 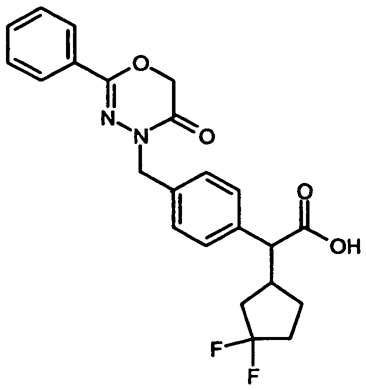 | |
| | (aus *tert*.-Butyl-(3,3-difluorcyclopentyl) {4-[(5-oxo-2-phenyl-5,6-dihydro-4*H*-1,3,4-oxadiazin-4-yl)methyl]-phenyl}acetat) | |
| **330A** | Cyclopentyl{4-[(4-methyl-6-oxopyridazin-1(6*H*)-yl)-methyl]phenyl}essigsäure | LC-MS (Methode 11): Rₜ = 1.07 min; m/z = 326 (M+H)⁺. ¹H-NMR (400 MHz, DMSO-d₆): δ = 7.64 (d, 1H), 7.27 (d, 2H), 7.19 (d, 2H), 6.23 (s, 1H), 6.09 (dd, 1H), 5.02 (s, 2H), 3.20 (d, 1H), 2.36-2.46 (m, 1H), 2.12 (s, 3H), 1.82 (dd, 1H), 1.57-1.70 (m, 1H), 1.47-1.56 (m, 2H), 1.35-1.45 (m, 1H), 1.19-1.30 (m, 2H), 0.92 (dd, 1H). |
| | 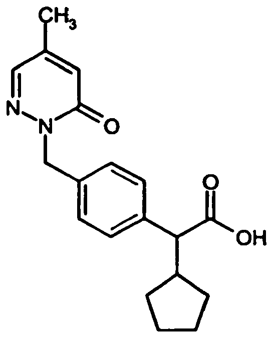 | |
| | (aus *tert*.-Butyl-cyclopentyl{4-[(4-methyl-6-oxo-pyridazin-1(6*H*)-yl)methyl]phenyl}acetat) | |
| **331A** | 4,4,4-Trifluor-3-methyl-2-(4-{[6-oxo-3-(trifluormethyl)-pyridazin-1(6*H*)-yl]methyl}phenyl)butansäure | LC-MS (Methode 11): Rₜ = 1.21 min; m/z = 409 (M+H)⁺. ¹H-NMR (400 MHz, DMSO-d₆): δ = 12.74 (br. s, 1H), 7.87 (d, 1H), 7.36 (d, 2H), 7.29 (d, 2H), 7.21 (d, 1H), 5.30 (s, 2H), 3.61 (d, 1H), 3.17-3.29 (m, 1H), 0.75 (d, 3H). |
| | 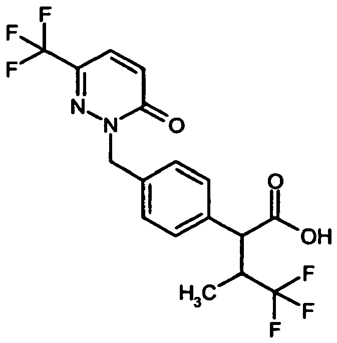 | |
| | (aus Ethyl-4,4,4-trifluor-3-methyl-2-(4-{[6-oxo-3-(trifluormethyl)pyridazin-1(6*H*)-yl]methyl}phenyl)-butanoat) | |
| **332A** | Cyclopentyl(4-{[6-oxo-5-(trifluormethyl)pyridazin-1(6*H*)-yl]methyl}phenyl)essigsäure | LC-MS (Methode 11): Rₜ = 1.39 min; m/z = 380 (M+H)⁺. ¹H-NMR (400 MHz, DMSO-d₆): δ = 12.24 (br. s, 1H), 8.16 (d, 1H), 7.94 (d, 1H), 7.31 (d, 2H), 7.25 (d, 2H), 6.40 (t, 1H), 5.14 (s, 2H), 3.22 (d, 1H), 2.35-2.47 (m, 1H), 1.75-1.89 (m, 1H), 1.48-1.66 (m, 3H), 1.35-1.45 (m, 1H), 1.13-1.32 (m, 2H), 0.87-1.00 (m, 1H). |
| | 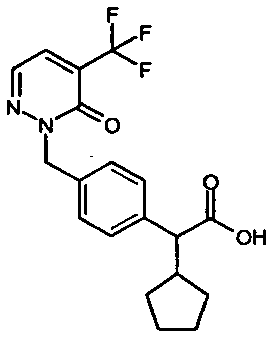 | |
| | (aus *tert*.-Butyl-cyclopentyl(4-{[6-oxo-5-(trifluor-methyl)pyridazin-1(6*H*)-yl]methyl}phenyl)acetat) | |
| **333A** | Cyclopentyl{4-[(6-oxo-3-phenylpyridazin-1(6*H*)-yl)-methyl]phenyl}essigsäure *(Enantiomer 2)* | LC-MS (Methode 10): Rₜ=2.11 min; m/z=389 (M+H)⁺. [α]_{D}²⁰ = -21.0°, c = 0.265, Methanol. |
| | 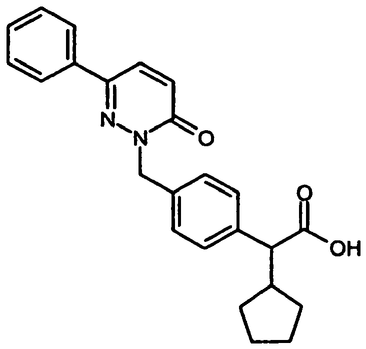 | |
| | (aus *tert*.-Butyl-cyclopentyl{4-[(6-oxo-3-phenylpyri-dazin-1(6*H*)-yl)methyl]phenyl} acetat *(Enantiomer 2))* | |
| **334A** | Cyclopentyl{4-[(6-oxo-3-phenylpyridazin-1(6*H*)-yl)-methyl]phenyl}essigsäure *(Enantiomer 1)* | LC-MS (Methode 10): Rₜ = 2.11 min; m/z = 389 (M+H)⁺. [α]_{D}²⁰ = +37.3°, c = 0.315, Methanol. ¹H-NMR (400 MHz, DMSO-d₆): δ = 12.15-12.31 (br. s, 1H), 8.09 (d, 1H), 7.89 (d, 2H), 7.43-7.53 (m, 3H), 7.25-7.34 (m, 4H), 7.09 (d, 1H), 5.30 (s, 2H), 3.21 (d, 1H), 2.34-2.47 (m, 1H), 1.75-1.89 (m, 1H), 1.32-1.66 (m, 4H), 1.15-1.31 (m, 2H), 0.87-0.99 (m, 1H). |
| | 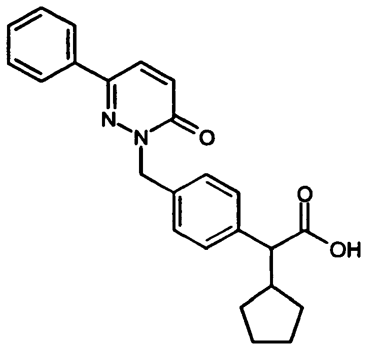 | |
| | (aus *tert*.-Butyl-cyclopentyl{4-[(6-oxo-3-phenylpyri-dazin-1(6*H*)-yl)methyl]phenyl}acetat *(Enantiomer 1))* | |
| **335A** | 4,4,4-Trifluor-3-methyl-2-{4-{(6-oxo-3-phenylpyridazin-1(6*H*)-yl)methyl]phenyl}butansäure | LC-MS (Methode 11): Rₜ = 1.27 min; m/z = 417 (M+H)⁺. |
| | 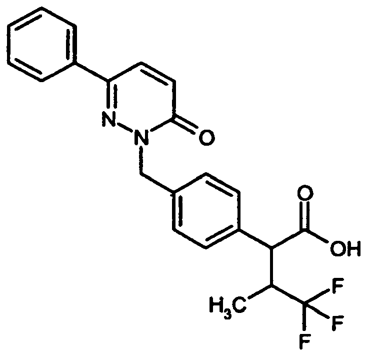 | |
| | (aus Ethyl-4,4,4-trifluor-3-methyl-2-{4-[(6-oxo-3-phenylpyridazin-1(6*H*)-yl)methyl]phenyl}butanoat) | |

### Beispiele 336A - 339A

### 4,4,4-Trifluor-3-methyl-2-{4-[(5-oxo-2-phenyl-5,6-dihydro-4H-1,3,4-oxadiazin-4-yl)methyl]-phenyl}butansäure (Isomer 1 - 4)

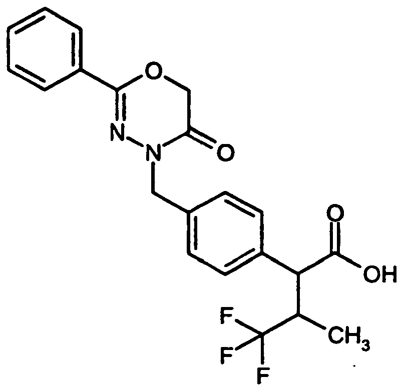

630 mg (1.50 mmol) des Isomerengemisches von 4,4,4-Trifluor-3-methyl-2-{4-[(5-oxo-2-phenyl-5,6-dihydro-4*H*-1,3,4-oxadiazin-4-yl)methyl]phenyl}butansäure wurden mittels präparativer HPLC an chiraler Phase in die Isomere aufgetrennt [Säule: Daicel Chiralpak AD-H, 5 µm, 250 mm x 20 mm; Elutionsmittel: Isohexan/(Isopropanol + 0.2% Trifluoressigsäure + 1% Wasser) 75:25 (v/v); Fluss: 15 ml/min; UV-Detektion: 220 nm; Temperatur: 30°C]:

### Beispiel 336A (Isomer 1):

Ausbeute: 26 mg
Rₜ 6.17 min; Reinheit >99%; >99% ee
[Säule: Daicel Chiralpak AD-H, 5 µm, 250 mm x 4.6 mm; Elutionsmittel: Isohexan/(Isopropanol + 0.2% Trifluoressigsäure + 1% Wasser) 75:25 (v/v); Fluss: 1 ml/min; UV-Detektion: 220 nm; Temperatur: 25°C].

### Beispiel 337A (Isomer 2):

Ausbeute: 35 mg
Rₜ 6.57 min; Reinheit >98%; >99% ee
[Säule: Daicel Chiralpak AD-H, 5 µm, 250 mm x 4.6 mm; Elutionsmittel: Isohexan/(Isopropanol + 0.2% Trifluoressigsäure + 1% Wasser) 75:25 (v/v); Fluss: 1 ml/min; UV-Detektion: 220 nm; Temperatur: 25°C].

### Beispiel 338A (Isomer 3):

**Ausbeute: 236 mg**
Rₜ 8.03 min; Reinheit >99%; >99% ee
[Säule: Daicel Chiralpak AD-H, 5 µm, 250 mm x 4.6 mm; Elutionsmittel: Isohexan/(Isopropanol + 0.2% Trifluoressigsäure + 1% Wasser) 75:25 (v/v); Fluss: 1 ml/min; UV-Detektion: 220 nm; Temperatur: 25°C].
LC-MS (Methode 15): Rₜ = 1.12 min; m/z = 421 (M+H)⁺.
¹H-NMR (400 MHz, DMSO-d₆, δ/ppm): 12.60-12.81 (1H, br. s), 7.78 (2H, d), 7.41-7.53 (3H, m), 7.37 (4H, s), 4.93 (2H, s), 4.89 (2H, s), 3.61 (1H, d), 3.18-3.32 (1H, m), 0.77 (3H, d).
[α]_{D}²⁰ = +45.6°, c = 0.565, Methanol.

### Beispiel 339A (Isomer 4):

Ausbeute: 247 mg
Rₜ 9.17 min; Reinheit >99%; >98% ee
[Säule: Daicel Chiralpak AD-H, 5 µm, 250 mm x 4.6 mm; Elutionsmittel: Isohexan/(Isopropanol + 0.2% Trifluoressigsäure + 1% Wasser) 75:25 (v/v); Fluss: 1 ml/min; UV-Detektion: 220 nm; Temperatur: 25°C].
[α]_{D}²⁰ = -45.8°, c = 0.305, Methanol.

### Beispiele 340A - 343A

### 3-Methyl-2-{4-[(5-oxo-2-phenyl-5,6-dihydro-4H-1,3,4-oxadiazin-4-yl)methyl]phenyl}pentansäure (Isomer 1-4)

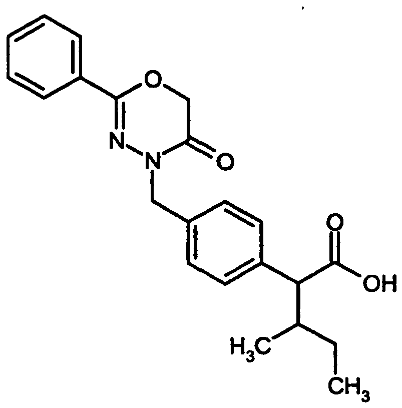

11.8 g (31.02 mmol) des Isomerengemisches von 3-Methyl-2-{4-[(5-oxo-2-phenyl-5,6-dihydro-4*H-*1,3,4-oxadiazin-4-yl)methyl]phenyl}pentansäure wurden mittels präparativer HPLC an chiraler Phase zunächst in die Diastereomere aufgetrennt [Säule: chirale Kieselgel-Phase basierend auf dem Selektor Poly(*N*-methacryloyl-D-valin-3-pentylamid), 500 mm x 75 mm; Elutionsmittel: Isohexan/Essigsäureethylester 30:70 (v/v); Fluss: 200 ml/min; UV-Detektion: 290 nm; Temperatur: 25°C]. Es wurden 4.11 g bzw. 5.2 g der beiden Diastereomere erhalten.

### Trennung von Diastereomer 1:

4.11 g des Diastereomers 1 wurden mittels präparativer HPLC an chiraler Phase in die Enantiomere *(Isomere 1 und 2)* aufgetrennt [Säule: Daicel Chiralpak AD-H, 5 µm, 250 mm x 20 mm; Elutionsmittel: Isohexan/Isopropanol 95:5 (v/v); Fluss: 25 ml/min; UV-Detektion: 230 nm; Temperatur: 24°C]:

### Beispiel 340A (Isomer 1):

Ausbeute: 865 mg
Rₜ 7.36 min; Reinheit >91%; >93% ee
[Säule: Daicel Chiralpak AD-H, 5 µm, 250 mm x 4 mm; Elutionsmittel: Isohexan/Isopropanol 80:20 (v/v); Fluss: 1 ml/min; UV-Detektion: 230 nm; Temperatur: 25°C].
LC-MS (Methode 10): Rₜ = 2.16 min; m/z = 381 (M+H)⁺.

### Beispiel 341A (Isomer 2):

Ausbeute: 1662 mg
Rₜ 7.91 min; Reinheit >99%; >97% ee
[Säule: Daicel Chiralpak AD-H, 5 µm, 250 mm x 4 mm; Elutionsmittel: Isohexan/Isopropanol 80:20 (v/v); Fluss: 1 ml/min; UV-Detektion: 230 nm; Temperatur: 25°C].
LC-MS (Methode 7): Rₜ = 2.53 min; m/z = 381 (M+H)⁺.
¹H-NMR (400 MHz, DMSO-d₆, δ/ppm): 12.35-12.15 (1H, br. s), 7.78 (2H, d), 7.54-7.40 (3H, m), 7.31 (4H, q), 4.92 (2H, s), 4.86 (2H, s), 3.19 (1H, d), 2.09-1.95 (1H, m), 1.59-1.43 (1H, m), 1.25-1.09 (1H, m), 0.89 (3H, t), 0.58 (3H, d).
[α]_{D}²⁰ = +21.7°, c = 0.525, Methanol.

### Trennung von Diastereomer 2:

5.2 g des Diastereomers 2 wurden mittels präparativer HPLC an chiraler Phase in die Enantiomere *(Isomere 3 und 4)* aufgetrennt [Säule: Daicel Chiralcel OJ-H, 5 µm, 250 mm x 20 mm; Elutionsmittel: Isohexan/Isopropanol 95:5 (v/v); Fluss: 25 ml/min; UV-Detektion: 230 nm; Temperatur: 24°C]:

### Beispiel 342A (Isomer 3):

Ausbeute: 2970 mg
Rₜ 7.21 min; Reinheit >94%; >99% ee
[Säule: Daicel Chiralcel OJ-H, 5 µm, 250 mm x 4 mm; Elutionsmittel: Isohexan/Isopropanol 80:20 (v/v); Fluss: 1 ml/min; UV-Detektion: 230 nm; Temperatur: 25°C].
LC-MS (Methode 7): Rₜ = 2.53 min; m/z = 381 (M+H)⁺.

### Beispiel 343A (Isomere 4):

Ausbeute: 1350 mg
Rₜ 7.77 min; Reinheit >90%; >84% ee
[Säule: Daicel Chiralcel OJ-H, 5 µm, 250 mm x 4 mm; Elutionsmittel: Isohexan/Isopropanol 80:20 (v/v); Fluss: 1 ml/min; UV-Detektion: 230 nm; Temperatur: 25°C].
LC-MS (Methode 10): Rₜ = 2.17 min; m/z = 381 (M+H)⁺.

### Beispiel 344A und Beispiel 345A

### 4,4,4-Trifluor-3-methyl-2-{4-[(6-oxo-3-phenylpyridazin-1(6H)-yl)methyl]phenyl}butansäure (Isomer 1 und 2)

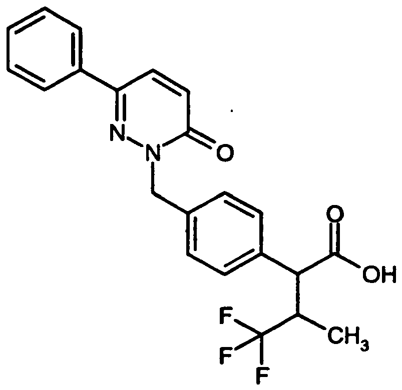

715 mg (1.72 mmol) des Isomerengemisches von 4,4,4-Trifluor-3-methyl-2-{4-[(6-oxo-3-phenylpyridazin-1(6*H*)-yl)methyl]phenyl}butansäure wurden mittels präparativer HPLC an chiraler Phase in die Isomere aufgetrennt [Säule: chirale Kieselgel-Phase basierend auf dem Selektor Poly(N-methacryloyl-L-isoleucin-3-pentylamid), 430 mm x 40 mm; Elutionsmittel: Essigsäureethylester; Fluss: 80 ml/min; UV-Detektion: 265 nm; Temperatur: 26°C]. Dabei wurden nur die beiden Enantiomere *(Isomere 1 und 2)* eines Diastereomers isoliert.

### Beispiel 344A (Isomer 1):

Ausbeute: 284 mg
Rₜ 7.71 min; Reinheit >99%; >99.5% ee; >99% de
[Säule: chirale Kieselgel-Phase basierend auf dem Selektor Poly(*N*-methacryloyl-L-isoleucin-3-pentylamid), 250 mm x 4.6 mm; Elutionsmittel: Isohexan/Essigsäureethylester 20:80 (v/v); Fluss: 2 ml/min; UV-Detektion: 265 nm; Temperatur: 25°C].
LC-MS (Methode 15): Rₜ = 1.10 min; m/z = 417 (M+H)⁺.
[α]_{D}²⁰ = -45.9°, c = 0.48, Methanol.

### Beispiel 345A (Isomer 2):

Ausbeute: 293 mg
Rₜ 13.19 min; Reinheit >99%; >99.5% ee; >88% de
[Säule: chirale Kieselgel-Phase basierend auf dem Selektor Poly(*N*-methacryloyl-L-isoleucin-3-pentylamid), 250 mm x 4.6 mm; Elutionsmittel: Isohexan/Essigsäureethylester 20:80 (v/v); Fluss: 2 ml/min; UV-Detektion: 265 nm; Temperatur: 25°C].
LC-MS (Methode 15): Rₜ = 1.10 min; m/z = 417 (M+H)⁺.
¹H-NMR (400 MHz, DMSO-d₆, δ/ppm): 12.72 (1H, br. s), 8.09 (1H, d), 7.79 (2H, d), 7.54-7.42 (3H, m), 7.36 (4H, s), 7.09 (1H, d), 5.33 (2H, s), 3.60 (1H, d), 3.30-3.16 (1H, m), 0.75 (3H, d).
[α]_{D}²⁰ = +48.0°, c = 0.49, Methanol.

Analog zu Beispiel 112A wurden die in der folgenden Tabelle aufgeführten Verbindungen hergestellt:

| **Beispiel** | **Name / Struktur / Edukte** | **Analytische Daten** |
|---|---|---|
| **346A** | *tert*.-Butyl-3-{2-methyl-3-[(3-methyl-2-{4-[(5-oxo-2-phenyl-5,6-dihydro-4*H*-1,3,4-oxadiazin-4-yl)methyl]-phenyl}butanoyl)amino]phenyl}propanoat | Rₜ = 2.33 min [Säule: Onxy Monolithic C18; Eluent: Acetonitril / (Wasser + 0.05% TFA) 1:1 (v/v); Fluss: 5 ml/min; UV-Detektion: 214/254 nm; Temperatur: 40°C]. |
| | 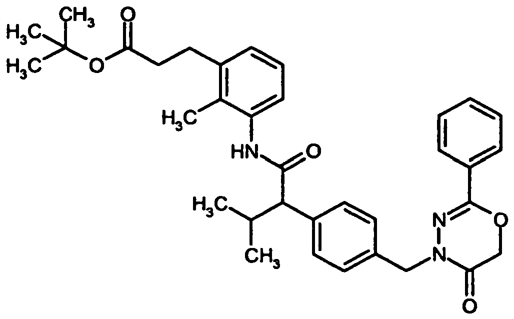 | |
| | (aus Bsp. 79A und Bsp. 96A) | |
| **347A** | Methyl-3-{3-[(cyclopentyl{4-[(5-oxo-2-phenyl-5,6-dihydro-4*H*-1,3,4-oxadiazin-4-yl)methyl]phenyl}-acetyl)amino]phenyl}-2,2-dimethylpropanoat | LC-MS (Methode 7): Rₜ = 3.06 min; m/z = 582 (M+H)⁺. ¹H-NMR (400 MHz, DMSO-d₆): δ = 9.97 (s, 1H), 7.76 (d, 2H), 7.28-7.52 (m, 9H), 7.13 (t, 1H), 6.72 (d, 1H), 4.90 (s, 2H), 4.84 (s, 2H), 3.37 (d, 1H), 3.32 (s, 2H), 2.71 (s, 2H), 1.72-1.83 (m, 1H), 1.13-1.67 (m, 7H), 1.08 (s, 6H), 0.90-1.02 (m, 1H). |
| | 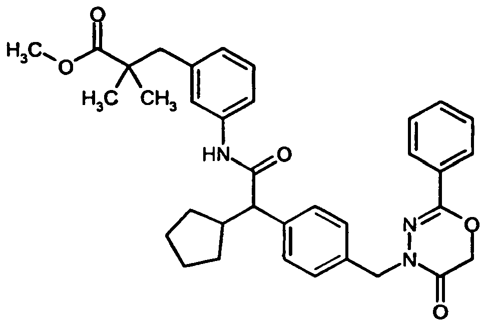 | |
| | (aus Bsp. 77A und Methyl-3-(3-aminophenyl)-2,2-dimethylpropanoat) | |
| **348A** | Methyl-3-{3-[(cyclopentyl{4-[(6-oxo-3-phenylpyridazin-1(6*H*)-yl)methyl]phenyl}acetyl)amino]phenyl}-2,2-dimethylpropanoat | LC-MS (Methode 7): Rₜ = 2.98 min; m/z = 578 (M+H)⁺. ¹H-NMR (400 MHz, DMSO-d₆): δ = 9.96 (s, 1H), 8.06 (d, 1H), 7.87 (d, 2H), 7.42-7.52 (m, 3H), 7.38 (d, 2H), 7.28-7.35 (m, 4H), 7.13 (t, 1H), 7.07 (d, 1H), 6.72 (d, 1H), 5.29 (s, 2H), 3.54 (s, 3H), 3.36 (d, 1H), 2.71 (s, 2H), 1.72-1.82 (m, 1H), 1.17-1.67 (m, 6H), 1.08 (s, 6H), 0.88-1.01 (m, 1H). |
| | 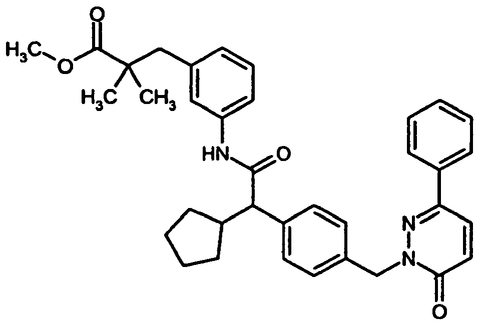 | |
| | (aus Cyclopentyl-{4-[(6-oxo-3-phenylpyridazin-1(6*H*)-yl)methyl]phenyl}essigsäure und Methyl-3-(3-aminophenyl)-2,2-dimethylpropanoat) | |
| **349A** | Methyl-2,2-dimethyl-3-{3-[(3-methyl-2-{4-[(5-oxo-2-phenyl-5,6-dihydro-4*H*-1,3,4-oxadiazin-4-yl)methyl]-phenyl}butanoyl)amino]phenyl}propanoat | LC-MS (Methode 7): Rₜ = 2.93 min; m/z = 556 (M+H)⁺. ¹H-NMR (400 MHz, DMSO-d₆): δ = 9.99 (s, 1H), 7.76 (d, 2H), 7.40-7.53 (m, 3H), 7.26-7.39 (m, 6H), 7.13 (t, 1H), 6.72 (d, 1H), 4.90 (s, 2H), 4.84 (s, 2H), 3.55 (s, 3H), 3.22 (d, 1H), 2.71 (s, 2H), 2.26-2.38 (m, 1H), 1.07 (s, 6H), 0.99 (d, 3H), 0.65 (d, 3H). |
| | 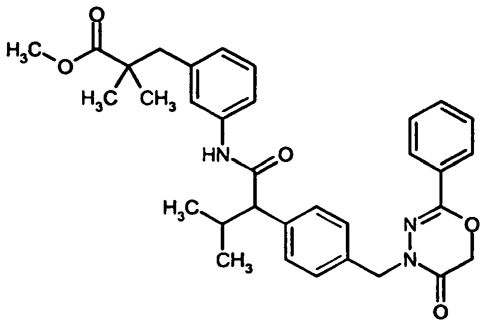 | |
| | (aus Bsp. 79A und Methyl-3-(3-aminophenyl)-2,2-dimethylpropanoat) | |
| **350A** | *tert*.-Butyl-3-{3-[(cyclopentyl{4-[(6-oxo-3-phenyl-pyridazin-1(6*H*)-yl)methyl]phenyl}acetyl)amino]-2-methylphenyl}propanoat | LC-MS (Methode 7): Rₜ = 3.07 min; m/z = 606 (M+H)⁺. ¹H-NMR (400 MHz, DMSO-d₆): δ = 9.45 (s, 1H), 8.07 (d, 1H), 7.88 (d, 2H), 7.43-7.53 (m, 3H), 7.41 (d, 2H), 7.32 (d, 2H), 7.09 (d, 1H), 6.92-7.03 (m, 3H), 5.32 (s, 2H), 3.45 (d, 1H), 2.77 (t, 2H), 2.56-2.64 (m, 1H), 2.40 (t, 2H), 1.94-1.99 (m, 3H), 1.41-1.89 (m. 7H), 1.31-1.40 (m, 9H), 0.88-1.06 (m, 1H). |
| | 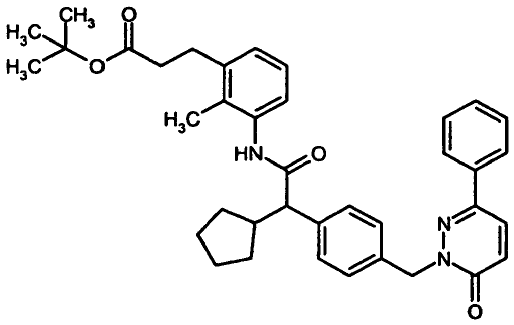 | |
| | (aus Cyclopentyl-{4-[(6-oxo-3-phenylpyridazin-1(6*H*)-yl)methyl]phenyl}essigsäure und Bsp. 96A) | |
| **351A** | Methyl-3-{2-methyl-3-[(6,6,6-trifluor-4-methyl-2-{4-[(6-oxo-3-phenylpyridazin-1(6*H*)-yl)methyl]phenyl}-hexanoyl)amino]phenyl}propanoat | LC-MS (Methode 10): Rₜ = 2.49 min; m/z = 624 (M+1)⁺. |
| | 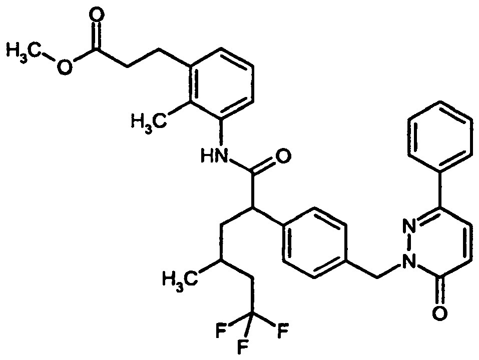 | |
| | (aus 6,6,6-Trifluor-4-methyl-2-{4-[(6-oxo-3-phenyl-pyridazin-1(6*H*)-yl)methyl]phenyl}hexansäure und Bsp. 97A) | |
| **352A** | Methyl-2,2-dimethyl-3-{3-[(3-methyl-2-{4-[(5-oxo-2-phenyl-5,6-dihydro-4*H*-1,3,4-oxadiazin-4-yl)methyl]-phenyl}pentanoyl)amino]phenyl}propanoat | LC-MS (Methode 7): Rₜ = 3.02 min; m/z = 570 (M+H)⁺. ¹H-NMR (400 MHz, DMSO-d₆): δ = 10.00 (s, 1H), 7.76 (d, 2H), 7.41-7.52 (m, 3H), 7.29-7.38 (m, 6H), 7.13 (t, 1H), 6.72 (d, 1H), 4.90 (s, 2H), 4.84 (s, 2H), 3.54 (s, 3H), 3.34 (d, 1H), 2.71 (s, 2H), 2.12-2.23 (m, 1H), 1.46-1.59 (m, 1H), 1.13-1.25 (m, 1H), 1.07 (s, 6H), 0.85-0.93 (m, 3H), 0.61 (d, 3H). |
| | 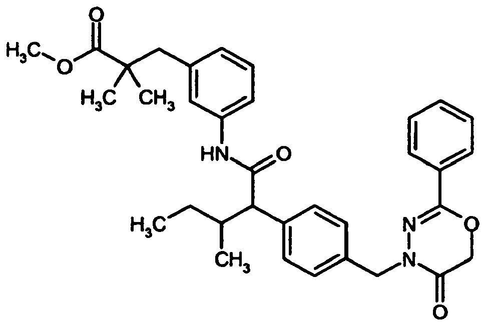 | |
| | (aus 3-Methyl-2-{4-[(5-oxo-2-phenyl-5,6-dihydro-4*H*-1,3,4-oxadiazin-4-yl)methyl]phenyl}pentansäure (*Isomer 2*) und Methyl-3-(3-aminophenyl)-2,2-dimethylpropanoat) | |
| **353A** | Methyl-4-[(cyclopentyl{4-[(6-oxo-3-phenylpyridazin-1(6*H*)-yl)methyl]phenyl}acetyl)aminol-2,3-dihydro-1*H* inden-2-carboxylat | LC-MS (Methode 11): Rₜ = 1.45 min; m/z = 562 (M+H)⁺. |
| | 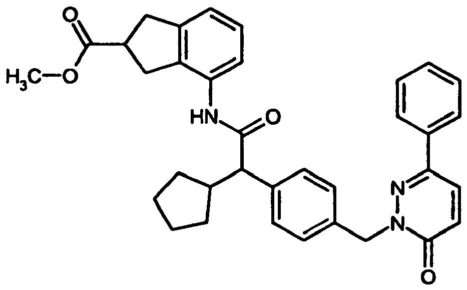 | |
| | (aus Cyclopentyl-{4-[(6-oxo-3-phenylpyridazin-1(6*H*)-yl)methyl]phenyl}essigsäure und Bsp. 89A) | |
| **354A** | *tert*.-Butyl-3-{2-methyl-3-[(4,4,4-trifluor-3-methyl-2-{4-[(6-oxo-3-phenylpyridazin-1 (6*H*)-yl)methyl]-phenyl}butanoyl)amino]phenyl}propanoat | LC-MS (Methode 10): Rₜ = 2.62 min; m/z = 634 (M+H)⁺. |
| | 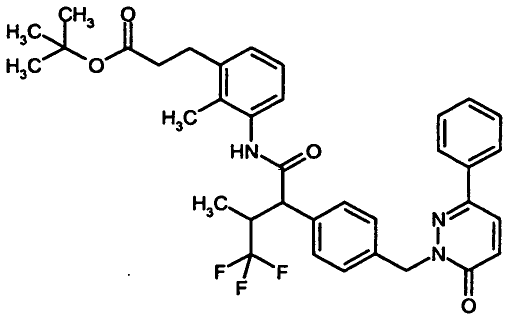 | |
| | (aus 4,4,4-Trifluor-3-methyl-2-{4-[(6-oxo-3-phenyl-pyridazin-1(6*H*)-yl)methyl]phenyl}butansäure und Bsp. 96A) | |
| **355A** | *tert*.-Butyl-1-{3-[(cyclopentyl{4-[(6-oxo-3-phenyl-pyridazin-1(6*H*)-yl)methyl]phenyl}acetyl)amino]-benzyl}cyclopropancarboxylat | LC-MS (Methode 7): Rₜ = 3.18 min; m/z = 616 (M-H)⁻. ¹H-NMR (400 MHz, DMSO-d₆): δ = 9.93 (s, 1H), 8.06 (d, 1H), 7.87 (d, 2H), 7.47 (d, 4H), 7.38 (d, 2H), 7.27-7.35 (m, 3H), 7.13 (t, 1H), 7.07 (d, 1H), 6.87 (d, 1H), 5.29 (s, 2H), 3.37 (d, 1H), 2.75 (s, 2H), 1.74-1.82 (m, 1H), 1.58-1.68 (m, 1H), 1.50-1.58 (m, 2H), 1.41-1.49 (m, 1H), 1.33-1.39 (m, 1H), 1.17-1.30 (m, 11H), 1.01-1.06 (m, 2H), 0.92-1.00 (m, 1H), 0.76-0.81 (m, 2H). |
| | 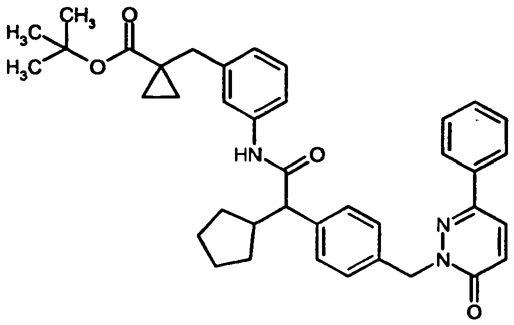 | |
| | (aus Cyclopentyl-{4-[(6-oxo-3-phenylpyridazin-1(6*H*)-yl)methyl]phenyl}essigsäure (*Enantiomer 1*) und 1-(3-Aminobenzyl)cyclopropancarbonsäure-*tert*.-butylester) | |
| **356A** | *tert*.-Butyl-1-{3-[(4,4,4-trifluor-3-methyl-2-{4-[(6-oxo-3-phenylpyridazin-1(6*H*)-yl)methyl]phenyl}butanoyl)-amino]benzyl}cyclopropancarboxylat (*Isomer 1*) | LC-MS (Methode 11): Rₜ = 1.63 min; m/z = 644 (M-H)⁻. |
| | 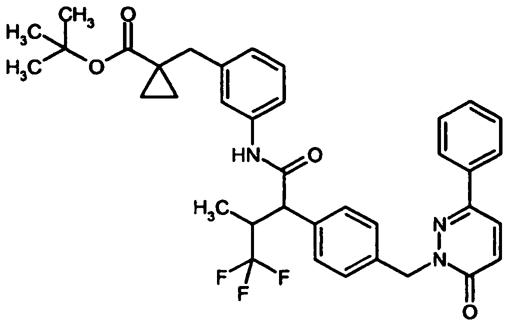 | |
| | (aus 4,4,4-Trifluor-3-methyl-2-{4-[(6-oxo-3-phenyl-pyridazin-1(6*H*)-yl)methyl]phenyl}butansäure (*Isomer 1*) und 1-(3-Aminobenzyl)cyclopropancarbonsäure-*tert*.-butylester) | |
| **357A** | *tert*.-Butyl-1-{3-[(4,4,4-trifluor-3-methyl-2-{4-[(6-oxo-3-phenylpyridazin-1(6*H*)-yl)methyl]phenyl}butanoyl)-amino]benzyl} cyclopropancarboxylat *(Isomer* 2) | LC-MS (Methode 11): Rₜ = 1.63 min; m/z = 644 (M-H)⁻. |
| | 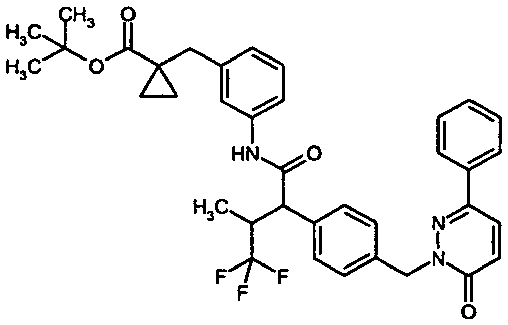 | |
| | (aus 4,4,4-Trifluor-3-methyl-2-{4-[(6-oxo-3-phenyl-pyridazin-1(6*H*)-yl)methyl]phenyl} butansäure *(Isomer 2)* und 1-(3-Aminobenzyl)cyclopropancarbonsäure-*tert*.-butylester) | |
| **358A** | *tert*.-Butyl-1-{3-[(5,5,5-trifluor-3-methyl-2-{4-[(5-oxo-2-phenyl-5,6-dihydro-4*H*-1,3,4-oxadiazin-4-yl)methyl]-phenyl}pentanoyl)amino]benzyl}cyclopropancarboxylat | LC-MS (Methode 15): Rₜ = 1.49 min; m/z = 662 (M-H)⁻. |
| | 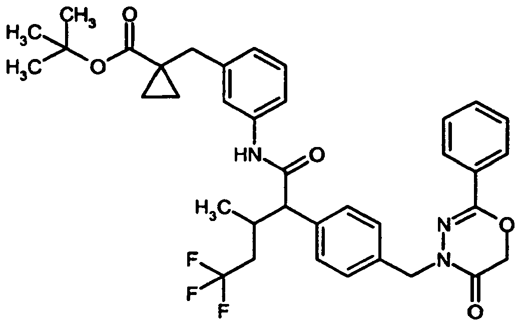 | |
| | (aus 5,5,5-Trifluor-3-methyl-2-{4-[(5-oxo-2-phenyl-5,6-dihydro-4*H*-1,3,4-oxadiazin-4-yl)methyl]phenyl} -pentansäure und 1-(3-Aminobenzyl)cyclopropan-carbonsäure-*tert*.-butylester) | |
| **359A** | *tert*.-Butyl-1-(3-{[{4-[(3-chlor-6-oxopyridazin-1(6*H*)-yl)-methyl]phenyl}(cyclopentyl)acetyl]amino}benzyl)-cyclopropancarboxylat | LC-MS (Methode 15): Rₜ =1.41 min; m/z = 520 (M-C₄H₈)⁺. |
| | 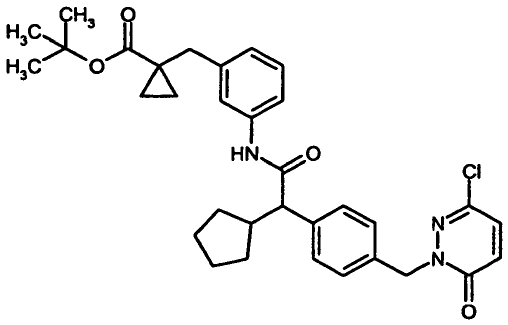 | |
| | (aus {4-[(3-Chlor-6-oxopyridazin-1(6*H*)-yl)methyl]-phenyl}(cyclopentyl)essigsäure und 1-(3-Aminobenzyl)-cyclopropancarbonsäure-*tert*.-butylester) | |
| **360A** | *tert*.-Butyl-1-{3-[(4,4,4-trifluor-3-methyl-2-{4-[(5-oxo-2-phenyl-5,6-dihydro-4*H*-1,3,4-oxadiazin-4-yl)methyl]-phenyl}butanoyl)amino]benzyl}cyclopropancarboxylat (*Isomer 1)* | LC-MS (Methode 15): Rₜ = 1.46 min; m/z = 594 (M-C₄H₈+H)⁺. |
| | 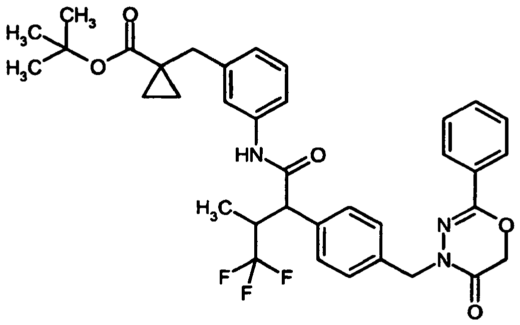 | |
| | (aus 4,4,4-Trifluor-3-methyl-2-{4-[(5-oxo-2-phenyl-5,6-dihydro-4*H*-1,3,4-oxadiazin-4-yl)methyl]phenyl}-butansäure (*Isomer 1*) und 1-(3-Aminobenzyl)-cyclopropancarbonsäure-*tert*.-butylester) | |
| **361A** | *tert*.-Butyl-1-{3-[(4,4,4-trifluor-3-methyl-2-{4-[(5-oxo-2-phenyl-5,6-dihydro-4*H*-1,3,4-oxadiazin-4-yl)methyl]-phenyl}butanoyl)amino]benzyl}cyclopropancarboxylat (*Isomer 2*) | LC-MS (Methode 15): Rₜ = 1.46 min; m/z = 594 (M-C₄H₈+H)⁺. |
| | 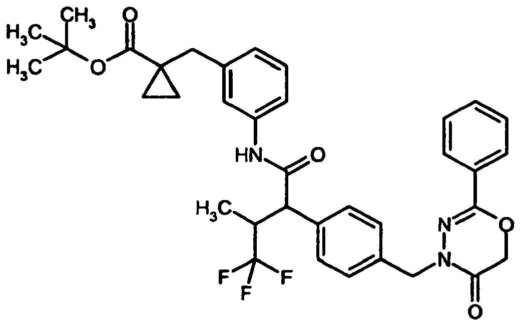 | |
| | (aus 4,4,4-Trifluor-3-methyl-2-{4-[(5-oxo-2-phenyl-5,6-dihydro-4*H*-1,3,4-oxadiazin-4-yl)methyl]phenyl}-butansäure *(Isomer* 2) und 1-(3-Aminobenzyl)-cyclopropancarbonsäure-*tert*.-butylester) | |
| **362A** | *tert*:-Butyl-1-{3-[(4,4,4-trifluor-3-methyl-2-{4-[(5-oxo-2-phenyl-5 ,6-dihydro-4*H*-l ,3,4-oxadiazin-4-yl)metbyl]-phenyl}butanoyl)amino]benzyl}cyclopropancarboxylat (*Isomer 3*) | LC-MS (Methode 15): Rₜ = 1.46 min; m/z = 648 (M-H)⁻. |
| | 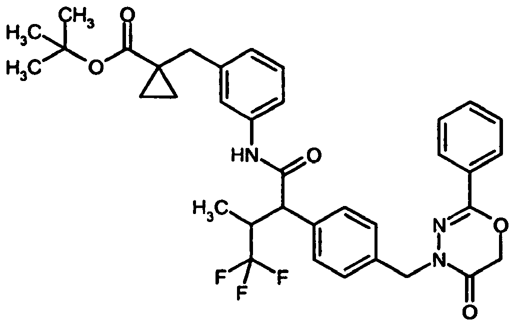 | |
| | (aus 4,4,4-Trifluor-3-methyl-2-{4-[(5-oxo-2-phenyl-5,6-dihydro-4*H*-1,3,4-oxadiazin-4-yl)methyl]phenyl}-butansäure *(Isomer* 3) und 1-(3-Aminobenzyl)-cyclopropancarbonsäure-*tert*.-butylester) | |
| **363A** | *tert*.-Butyl-1-{3-[(4,4,4-trifluor-3-methyl-2-{4-[(5-oxo-2-phenyl-5,6-dihydro-4*H*-1,3,4-oxadiazin-4-yl)methyl]-phenyl}butanoyl)amino]benzyl}cyclopropancarboxylat (*Isomer 4*) | LC-MS (Methode 15): Rₜ = 1.46 min; m/z = 648 (M-H)⁻. |
| | 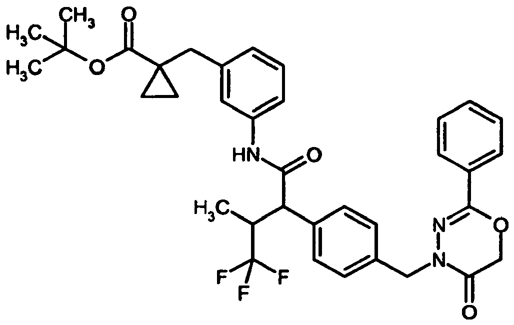 | |
| | (aus 4,4,4-Trifluor-3-methyl-2-{4-[(5-oxo-2-phenyl-5,6-dihydro-4*H*-1,3,4-oxadiazin-4-yl)methyl]phenyl}-butansäure (*Isomer 4*) und 1-(3-Aminobenzyl)-cyclopropancarbonsäure-*tert*.-butylester) | |
| **364A** | *tert*.-Butyl-3-{2-methyl-3-[(4,4,4-trifluor-3-methyl-2-{4-[(5-oxo-2-phenyl-5,6-dihydro-4*H*-1,3,4-oxadiazin-4-yl)-methyl]phenyl}butanoyl)amino]phenyl}propanoat | LC-MS (Methode 11): Rₜ= 1.61 min; m/z = 636 (M-H)⁻. |
| | 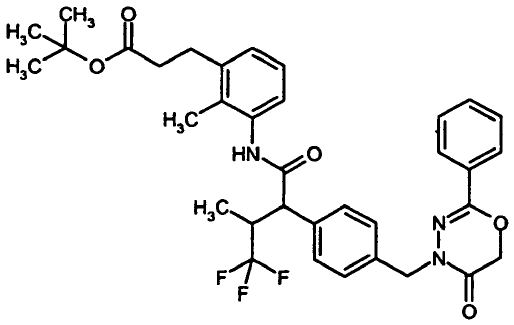 | |
| | (aus 4,4,4-Trifluor-3-methyl-2-(4-[(5-oxo-2-phenyl-5,6-dihydro-4*H*-1,3,4-oxadiazin-4-yl)methyl]phenyl)-butansäure und Bsp. 96A) | |
| **365A** | Methyl-3-{3-[(4,4,4-trifluor-3-methyl-2-{4-[(5-oxo-2-phenyl-5,6-dihydro-4*H*-1,3,4-oxadiazin-4-yl)methyl]-phenyl}butanoyl)amino]phenyl}propanoat | LC-MS (Methode 11): Rₜ = 1.47 min; m/z = 582 (M+H)⁺. |
| | 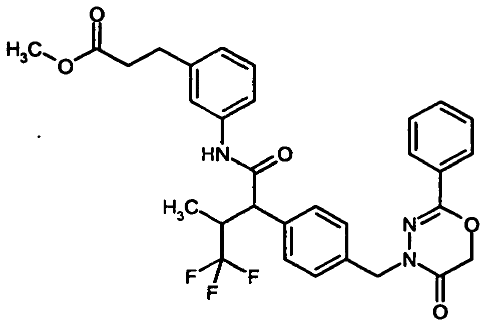 | |
| | (aus 4,4,4-Trifluor-3-methyl-2-{4-[(5-oxo-2-phenyl-5,6-dihydro-4*H*-1,3,4-oxadiazin-4-yl)methyl]phenyl}-butansäure und Methyl-3-(3-aminophenyl)propanoat) | |
| **366A** | *tert*.-Butyl-1-(3-([2-{4-[(5-oxo-2-phenyl-5,6-dihydro-4*H*-1,3,4-oxadiazin-4-yl)methyl]phenyl}-3-(trifluormethyl)-pentanoyl]an-üno)benzyl)cyclopropancarboxylat | LC-MS (Methode 15): Rₜ = 1.49 min; m/z = 662 (M-H)⁻. |
| | 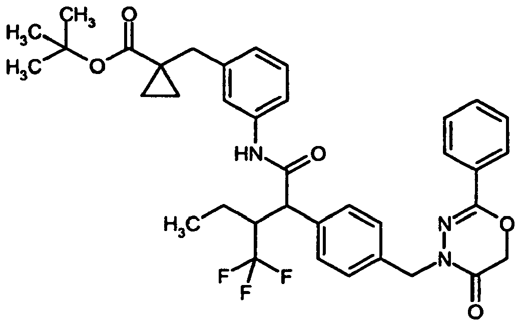 | |
| | (aus 2-{4-[(5-0xo-2-phenyl-5,6-dihydro-4*H*-1,3,4-oxa-diazin-4-yl)methyl]phenyl}-3-(trifluormethyl)pentan-säure und 1-(3-Aminobenzyl)cyclopropancarbonsäure-*tert*.-butylester) | |
| **367A** | *tert*.-Butyl-1-{3-[(cyclopentyl{4-[(3-methyl-6-oxo-pyridazin-1(6*H*)-yl)methyl]phenyl}acetyl)amino]-benzyl}cyclopropancarboxylat | LC-MS (Methode 11): Rₜ =1.54 min; m/z = 500 (M-C₄H₈+H)⁺. ¹H-NMR (400 MHz, DMSO-d₆): δ = 9.94 (s, 1H), 7.49 (s, 1H), 7.30-7.39 (m, 4H), 7.21 (d, 2H), 7.14 (t, 1H), 6.86-6.92 (m, 2H), 5.14 (s, 2H), 3.36 (d, 1H), 2.77 (s, 2H), 2.56-2.62 (m, 1H), 2.24 (s, 3H), 1.77 (dd, 1H), 1.30-1.67 (m, 5H), 1.16-1.29 (m, 1H), 1.24 (s, 9H), 1.03-1.07 (m, 2H), 0.96 (dt, 1H), 0.76-0.82 (m, 2H). |
| | 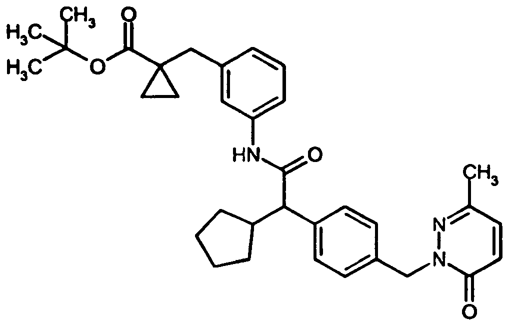 | |
| | (aus Cyclopentyl {4-[(3-methyl-6-oxopyridazin-1(6*H*)-yl)methyl]phenyl}essigsäure und 1-(3-Aminobenzyl)cyclopropancarbonsäure-*tert*.-butylester) | |
| **368A** | *tert*.-Butyl-1-(3-{[cyclopentyl(4-{[6-oxo-3-(trifluor-methyl)pyridazin-1(6*H*)-yl]methyl}phenyl)acetyl]-amino}benzyl)cyclopropancarboxylat | LC-MS (Methode 15): Rₜ = 1.45 min; m/z = 608 (M-H)⁻. ¹H-NMR (400 MHz, DMSO-d₆): δ = 9.95 (s, 1H), 7.85 (d, 1H), 7.49 (s, 1H), 7.39 (d, 2H), 7.34 (d, 1H), 7.25 (d, 2H), 7.18 (d, 1H), 7.14 (t, 1H), 6.88 (d, 1H), 5.27 (s, 2H), 3.37 (d, 1H), 2.77 (s, 2H), 2.56-2.64 (m, 1H), 1.71-1.84 (m, 1H), 1.29-1.69 (m, 6H), 1.23 (s, 9H), 1.02-1.07 (m, 2H), 0.89-1.01 (m, 1H), 0.76-0.82 (m, 2H). |
| | 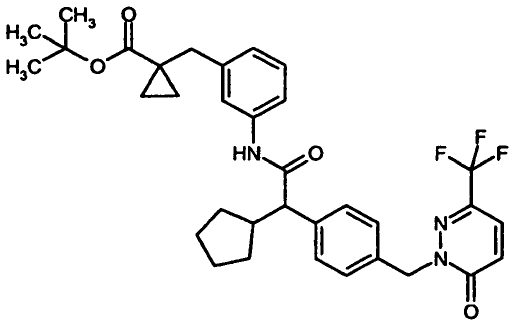 | |
| | (aus Cyclopentyl(4-{[6-oxo-3-(trifluoromethyl)pyridazin-1(6*H*)-yl]methyl}phenyl)essigsäure und 1-(3-Aminobenzyl)cyclopropancarbonsäure-*tert*.-butylester) | |
| **369A** | Methyl-3-(3-{[cyclopentyl(4-{[6-oxo-3-(trifluormethyl)-pyridazin-1(6*H*)-yl]methyl}phenyl)acetyl]amino}-phenyl)propanoat | LC-MS (Methode 11): Rₜ = 1.45 min; m/z = 542 (m+H)⁺. ¹H-NMR (400 MHz, DMSO-d₆): δ = 9.98 (s, 1H), 7.84 (d, 1H), 7.36-7.44 (m, 4H), 7.26 (d, 2H), 7.12-7.21 (m, 2H), 6.86 (d, 1H), 5.27 (s, 2H), 3.56 (s, 3H), 3.37 (d, 1H), 2.74-2.82 (m, 2H), 2.54-2.62 (m, 3H), 1.72-1.82 (m, 1H), 1.60-1.70 (m, 1H), 1.50-1.59 (m, 2H), 1.41-1.50 (m, 1H), 1.31-1.38 (m, 1H), 1.22-1.28 (m, 1H), 0.90-1.00 (m, 1H). |
| | 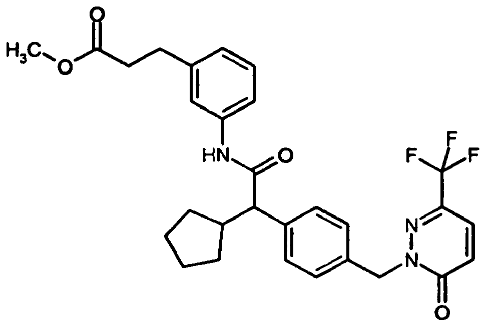 | |
| | (aus Cyclopentyl(4-{[6-oxo-3-(trifluormethyl)pyridazin-1(6*H*)-yl]methyl}phenyl)essigsäure und Methyl-3-(3-aminophenyl)propanoat) | |
| **370A** | *tert*.-Butyl-1-{3-[(cyclopentyl{4-[(3-methyl-2-oxo-pyridin-1(2*H*)-yl)methyl]phenyl}acetyl)amino]benzyl}-cyclopropancarboxylat | LC-MS (Methode 11): Rₜ = 1.54 min; m/z = 553 (M-H)⁻. |
| | 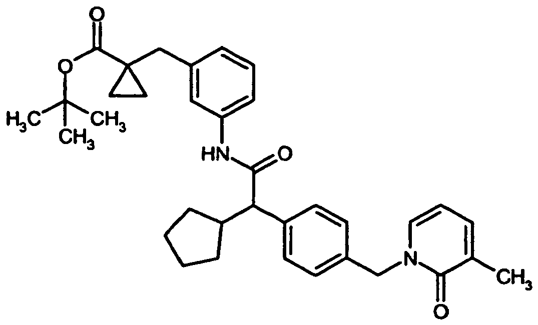 | |
| | (aus Cyclopentyl {4-[(3-methyl-2-oxopyridin-1(2*H*)-yl)-methyl]phenyl} essigsäure und 1-(3-Aminobenzyl)-cyclopropancarbonsäure-*tert*.-butylester) | |
| **371A** | *tert*.-Butyl-1-(3-{[cyclopentyl(4-{[2-oxo-4-(trifluor-methyl)pyridin-1(2*H*)-yl]methyl}phenyl)acetyl]amino}-benzyl)cyclopropancarboxylat | LC-MS (Methode 11): Rₜ = 1.62 min; m/z = 607 (M-H)⁻. |
| | 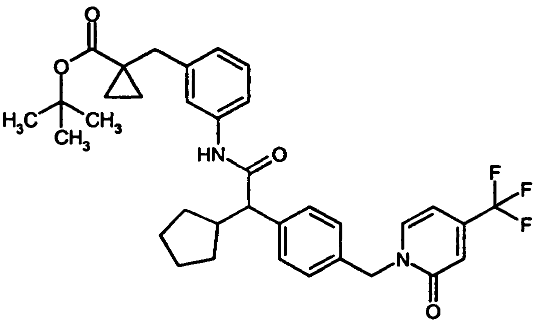 | |
| | (aus Cyclopentyl(4-{[2-oxo-4-(trifluormethyl)pyridin-1(2*H*)-yl]methyl}phenyl)essigsäure und 1-(3-Amino-benzyl)cyclopropancarbonsäure-*tert*.-butylester) | |
| **372A** | Methyl-3-(3-{[(3,3-difluorcyclopentyl){4-[(5-oxo-2-phenyl-5,6-dihydro-4*H*-1,3,4-oxadiazin-4-yl)methyl]-phenyl}acetyl]amino}phenyl)propanoat | LC-MS (Methode 11): Rₜ = 1.46 min; m/z = 590 (M+H)⁺. |
| | 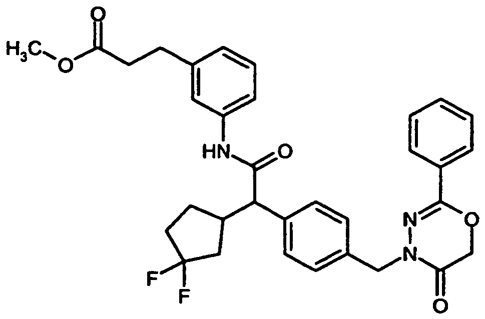 | |
| | (aus (3,3-Difluorcyclopentyl) {4-[(5-oxo-2-phenyl-5,6-dihydro-4*H*-1,3,4-oxadiazin-4-yl)methyl]phenyl}-essigsäure und Methyl-3-(3-aminophenyl)propanoat) | |
| **373A** | *tert*.-Butyl-1-(3-{[(3,3-difluorcyclopentyl){4-[(5-oxo-2-phenyl-5,6-dihydro-4*H*-1,3,4-oxadiazin-4-yl)methyl]-phenyl} acetyl]amino}benzyl)cyclopropancarboxylat | LC-MS (Methode 11): Rₜ = 1.66 min; m/z = 602 (M-C₄+H)⁺. |
| | 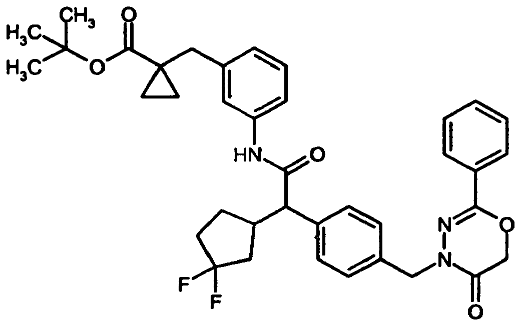 | |
| | (aus (3,3-Difluorcyclopentyl) {4-[(5-oxo-2-phenyl-5,6-dihydro-4*H*-1,3,4-oxadiazin-4-yl)methyl]phenyl}essig-säure und 1-(3-Aminobenzyl)cyclopropancarbonsäure-*tert*.-butylester) | |
| **374A** | *tert*.-Butyl-1-{3-[(cyclopentyl{4-[(4-methyl-2-oxo-pyridin-1(2*H*)-yl)methyl]phenyl}acetyl)amino]benzyl}-cyclopropancarboxylat | LC-MS (Methode 15): Rₜ = 1.34 min; m/z = 555 (M+H)⁺. |
| | 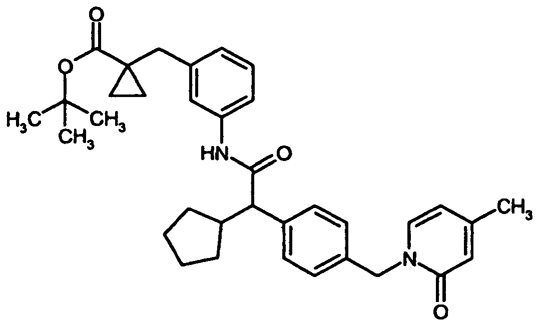 | |
| | (aus Cyclopentyl {4-[(4-methyl-2-oxopyridin-1(2*H*)-yl)-methyl]phenyl}essigsäure und 1-(3-Aminobenzyl)-cyclopropancarbonsäure-*tert*.-butylester) | |
| **375A** | Methyl-3-(3-{[4,4,4-trifluor-3-methyl-2-(4-{[6-oxo-3-(trifluormethyl)pyridazin-1(6*H*)-yl]methyl}phenyl)-butanoyl]amino} phenyl)propanoat | LC-MS (Methode 11): Rₜ = 1.40 min; m/z = 570 (M+H)⁺. |
| | 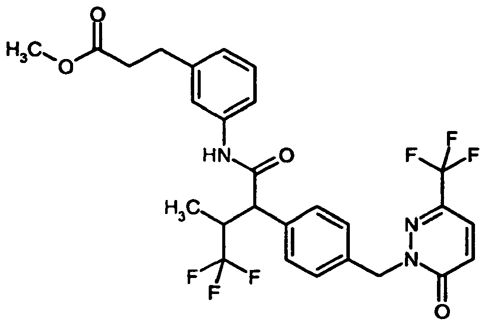 | |
| | (aus 4,4,4-Trifluor-3-methyl-2-(4-{[6-oxo-3-(trifluor-methyl)pyridazin-1(6*H*)-yl]methyl}phenyl)butansäure und Methyl-3-(3-aminophenyl)propanoat) | |
| **376A** | *tert*.-Butyl-1-(3-{[4,4,4-trifluor-3-methyl-2-(4-{[6-oxo-3-(trifluormethyl)pyridazin-(6*H*)-yl]methyl}phenyl)-butanoyl]amino}benzyl)cyclopropancarboxylat | LC-MS (Methode 11): Rₜ = 1.61 min; m/z = 636 (M-H)⁻. ¹H-NMR (400 MHz, DMSO-d₆): δ = 10.13 (s, 1H), 7.85 (d, 1H), 7.46 (s, 1H), 7.40 (d, 2H), 7.27-7.32 (m, 3H), 7.18 (d, 1H), 7.14 (t, 1H), 6.89 (d, 1H), 5.28 (s, 2H), 3.81 (d, 1H), 3.34-3.42 (m, 1H), 2.76 (s, 2H), 1.21 (s, 9H), 1.04 (q, 2H), 0.80 (q, 2H), 0.77 (d, 3H). |
| | 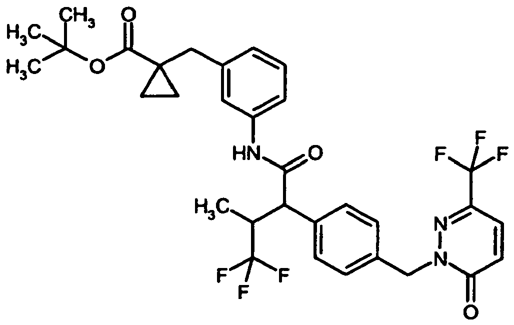 | |
| | (aus 4,4,4-Trifluor-3-methyl-2-(4-{[6-oxo-3-(trifluor-methyl)pyridazin-1(6*H*)-yl]methyl}phenyl)butansäure und 1-(3-Aminobenzyl)cyclopropancarbonsäure-*tert*.-butylester) | |
| **377A** | *tert*.-Butyl-1-(3-{[cyclopentyl(4-{[2-oxo-3-(trifluor-methyl)pyridin-1(2*H*)-yl]methyl}phenyl)acetyl]-amino}benzyl)cyclopropancarboxylat | LC-MS (Methode 15): Rₜ = 1.39 min; m/z = 553 (M-C₄H₈+H)⁺. |
| | 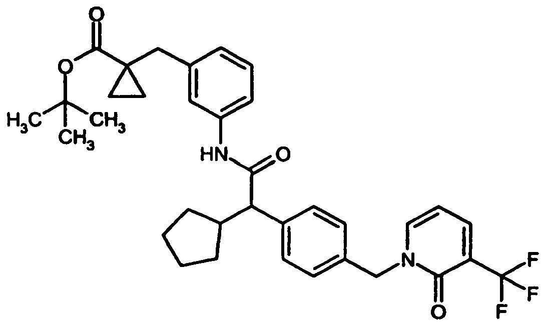 | |
| | (aus Cyclopentyl(4-{[2-oxo-3-(trifluormethyl)pyridin-1(2*H*)-yl]methyl}phenyl)essigsäure und 1-(3-Amino-benzyl)cyclopropancarbonsäure-*tert*.-butylester) | |
| **378A** | *tert*.-Butyl-3-{2-chlor-3-[(4,4,4-trifluor-3-methyl-2-{4-[(5-oxo-2-phenyl-5,6-dihydro-4*H*-1,3,4-oxadiazin-4-yl)-methyl]phenyl}butanoyl)amino]phenyl}propanoat | LC-MS (Methode 11): Rₜ = 1.67 min; m/z = 657 (M-H)⁻. |
| | 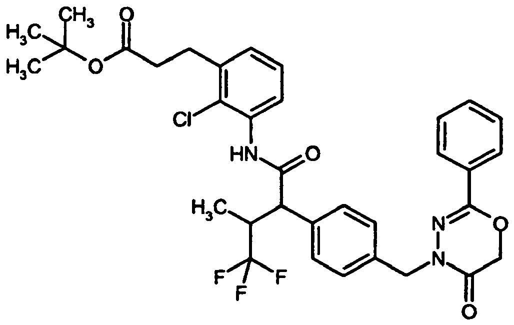 | |
| | (aus 4,4,4-Trifluor-3-methyl-2-{4-[(5-oxo-2-phenyl-5,6-dihydro-4*H*-1,3,4-oxadiazin-4-yl)methyl]phenyl}butan-säure und *tert*.-Butyl-3-(3-amino-2-chlorphenyl)-propanoat) | |
| **379A** | *tert*.-Butyl-3-{4-fluor-3-[(4,4,4-trifluor-3-methyl-2-{4-[(5-oxo-2-phenyl-5,6-dihydro-4*H*-1,3,4-oxadiazin-4-yl)-methyl]phenyl}butanoyl)amino]phenyl}propanoat | LC-MS (Methode 12): Rₜ = 2.94 min; m/z = 640 (M-H)⁻. |
| | 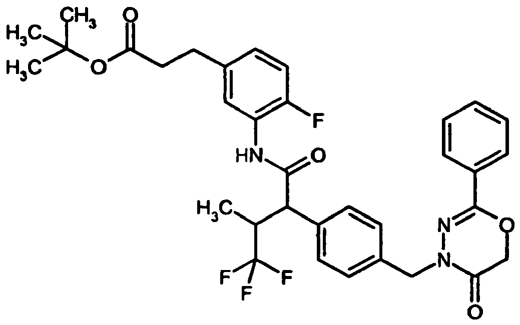 | |
| | (aus 4,4,4-Trifluor-3-methyl-2-{4-[(5-oxo-2-phenyl-5,6-dihydro-4*H*-1,3,4-oxadiazin-4-yl)methyl]phenyl}butan-säure *(Isomer* 3) und *tert*.-Butyl-3-(3-amino-4-fluor-phenyl)propanoat) | |
| **380A** | *tert*.-Butyl-1-{4-fluor-3-[(4,4,4-trifluor-3-methyl-2-{4-[(5-oxo-2-phenyl-5,6-dihydro-4*H*-1,3,4-oxadiazin-4-yl)-methyl]phenyl}butanoyl)amino]benzyl}cyclopropan-carboxylat | LC-MS (Methode 12): Rₜ = 3.03 min; m/z = 666 (M-H)⁻. |
| | 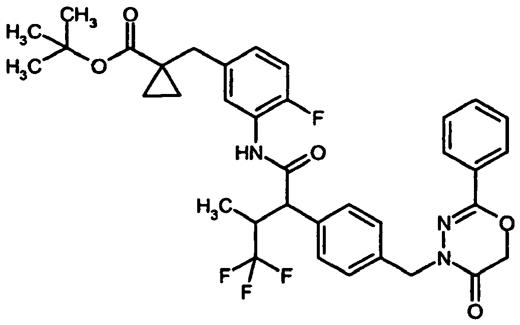 | |
| | (aus 4,4,4-Trifluor-3-methyl-2-{4-[(5-oxo-2-phenyl-5,6-dihydro-4*H*-1,3,4-oxadiazin-4-yl)methyl]phenyl}butan-säure *(Isomer* 3) und *tert*.-Butyl-1-(3-amino-4-fluor-benzyl)cyclopropancarboxylat) | |

Analog zu Beispiel 105A wurde die in der folgenden Tabelle aufgeführte Verbindung erhalten:

| | | |
|---|---|---|
| **381A** | *tert*.-Butyl-3-{4-chlor-3-[(cyclopentyl(4-[(5-oxo-2-phenyl-5,6-dihydro-4*H*-1,3,4-oxadiazin-4-yl)methyl]-phenyl) acetyl)amino]phenyl} propanoat | LC-MS (Methode 11): Rₜ =1.73 min; m/z = 629 (M-H)⁻. |
| | 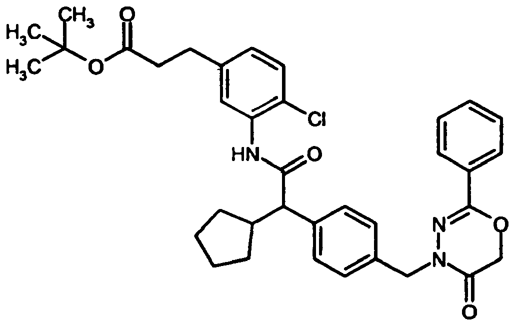 | |
| | (aus Bsp. 80A und *tert*.-Butyl-3-(3-amino-4-chlor-phenyl)propanoat) | |

### Beispiel 382A und Beispiel 383A

### Methyl-3-{3-[(cyclopentyl{4-[(6-oxo-3-phenylpyridazin-1(6H)-yl)methyl]phenyl}acetyl)amino]-phenyl}-2,2-dimethylpropanoat (Enantiomer 1 und 2)

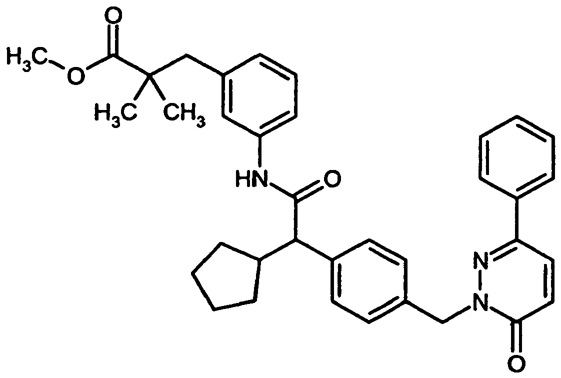

100 mg (0.17 mmol) des Racemats von Methyl-3-{3-[(cyclopentyl{4-[(6-oxo-3-phenylpyridazin-1(6*H*)-yl)methyl]phenyl}acetyl)amino]phenyl}-2,2-dimethylpropanoat wurden mittels präparativer HPLC an chiraler Phase in die Enantiomere aufgetrennt [Säule: Daicel Chiralpak AS-H, 5 µm, 250 mm x 20 mm; Elutionsmittel: Isohexan/Isopropanol 75:25 (v/v); Fluss: 15 ml/min; UV-Detektion: 220 nm; Temperatur: 40°C]:

### Beispiel 382A (Enantiomer 1):

Ausbeute: 58 mg
Rₜ 6.81 min; Reinheit >99%; >99% ee
[Säule: Daicel Chiralpak AD-H, 5 µm, 250 mm x 4.6 mm; Elutionsmittel: Isohexan/Isopropanol 75:25 (v/v); Fluss: 1 ml/min; UV-Detektion: 220 nm; Temperatur: 40°C].

### Beispiel 383A (Enantiomer 2):

Ausbeute: 41 mg
Rₜ 8.45 min; Reinheit >98%; >98.5% ee
[Säule: Daicel Chiralpak AD-H, 5 µm, 250 mm x 4.6 mm; Elutionsmittel: Isohexan/Isopropanol 75:25 (v/v); Fluss: 1 ml/min; UV-Detektion: 220 nm; Temperatur: 40°C].

### Beispiel 384A und Beispiel 385A

### tert.-Butyl-3-{3-[(cyclopentyl {4-[(6-oxo-3-phenylpyridazin-1(6H)-yl)methyl]phenyl} acetyl)-amino]-2-methylphenyl}propanoat (Enantiomer 1 und 2)

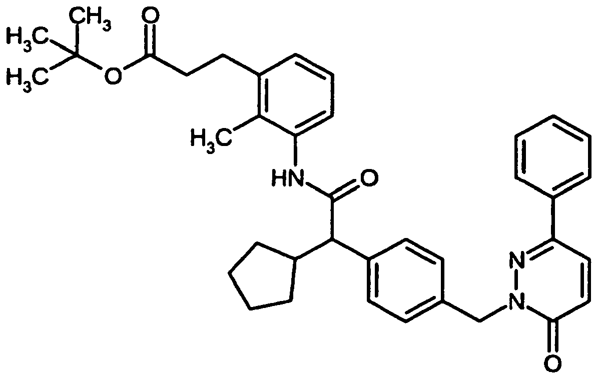

920 mg (1.52 mmol) des Racemats von *tert*.-Butyl-3-{3-[(cyclopentyl{4-[(6-oxo-3-phenylpyridazin-1(6*H*)-yl)methyl]phenyl}acetyl)amino]-2-methylphenyl}propanoat wurden mittels präparativer HPLC an chiraler Phase in die Enantiomere aufgetrennt [Säule: Daicel Chiralpak IA, 5 µm, 250 mm x 20 mm; Elutionsmittel: *tert*.-Butylmethylether/Acetonitril/Methanol 75:20:10 (v/v); Fluss: 15 ml/min; UV-Detektion: 220 nm; Temperatur: 30°C]:

### Beispiel 384A (Enantiomer 1):

Ausbeute: 345 mg
R, 4.76 min; Reinheit >99%; >99% ee
[Säule: Daicel Chiralpak IA, 5 µm, 250 mm x 4.6 mm; Elutionsmittel: *tert*.-Butylmethylether/ Acetonitrivmethanol 75:20:10 (v/v); Fluss: 1 ml/min; UV-Detektion: 220 nm; Temperatur: 25°C].

### Beispiel 385A (Enantiomer 2):

Ausbeute: 478 mg
R, 6.43 min; Reinheit >98%; >98% ee
[Säule: Daicel Chiralpak IA, 5 µm, 250 mm x 4.6 mm; Elutionsmittel: *tert*.-Butylmethylether/ Acetonitril/Methanol 75:20:10 (v/v); Fluss: 1 ml/min; UV-Detektion: 220 nm; Temperatur: 25°C].

### Beispiel 386A und Beispiel 387A

### tert.-Butyl-3-{3-[(cyclopentyl {4-[(2-oxo-5-phenyl-1,3,4-oxadiazol-3(2H)-yl)methyl]phenyl}-acetyl)amino]-2-methylphenyl}propanoat (Enantiomer 1 und 2)

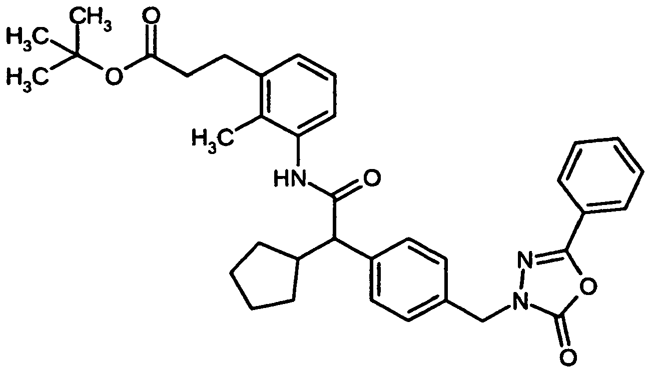

194 mg (0.32 mmol) des Racemats von *tert*.-Butyl-3-{3-[(cyclopentyl {4-[(2-oxo-5-phenyl-1,3,4-oxadiazol-3(2*H*)-yl)methyl]phenyl}acetyl)amino]-2-methylphenyl}propanoat (Beispiel 167A) wurden mittels präparativer HPLC an chiraler Phase in die Enantiomere aufgetrennt [Säule: Daicel Chiralpak IA, 5 µm, 250 mm x 20 mm; Elutionsmittel: *tert*.-Butylmethylether/Acetonitril/ Methanol 75:20:10 (v/v); Fluss: 15 ml/min; UV-Detektion: 220 nm; Temperatur: 27°C]:

### Beispiel 386A (Enantiomer 1):

Ausbeute: 70 mg
Rₜ 4.15 min; Reinheit >99%; >99% ee
[Säule: Daicel Chiralpak IA, 5 µm, 250 mm x 4.6 mm; Elutionsmittel: *tert*.-Butylmethylether/ Acetonitril/Methanol 75:20:10 (v/v); Fluss: 1 ml/min; UV-Detektion: 220 nm; Temperatur: 25°C].

### Beispiel 387A (Enantiomer 2):

Ausbeute: 61 mg
Rₜ 5.24 min; Reinheit >99%; >97.5% ee
[Säule: Daicel Chiralpak IA, 5 µm, 250 mm x 4.6 mm; Elutionsmittel: *tert*.-Butylmethyletherl Acetonitril/Methanol 75:20:10 (v/v); Fluss: 1 ml/min; UV-Detektion: 220 nm; Temperatur: 25°C].

### Beispiel 388A und Beispiel 389A

### tert.-Butyl-1-(3-{[4,4,4-trifluor-3-methyl-2-(4-{[6-oxo-3-(trifluormethyl)pyridazin-1(6H)-yl]-methyl}phenyl)butanoyl]amino}benzyl)cyclopropancarboxylat (Enantiomer 1 und 2)

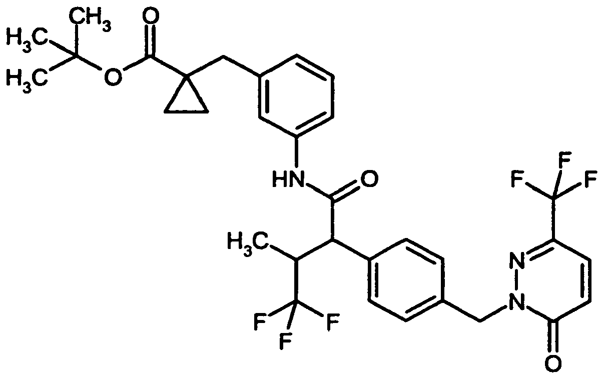

150 mg (0.24 mmol) des Isomerengemisches von *tert*.-Butyl-1-(3-{[4,4,4-trifluor-3-methyl-2-(4-{[6-oxo-3-(trifluormethyl)pyridazin-1(6*H*)-yl]methyl}phenyl)butanoyl]amino}benzyl)cyclopropancarboxylat wurden mittels präparativer HPLC an chiraler Phase weiter aufgetrennt [Säule: Daicel Chiralpak AD-H, 5 µm, 250 mm x 20 mm; Elutionsmittel: Isohexan/Ethanol 80:20 (v/v); Fluss: 15 ml/min; UV-Detektion: 220 nm; Temperatur: 30°C]. Dabei wurden nur die Enantiomere des Hauptdiastereomers isoliert.

### Beispiel 388A (Enantiomer 1):

Ausbeute: 68 mg
Rₜ 3.94 min; Reinheit >95%; >99% ee
[Säule: Daicel Chiralpak AD-H, 5 µm, 250 mm x 4.6 mm; Elutionsmittel: Isohexan/(Ethanol + 0.2% Trifluoressigsäure + 1% Wasser) 80:20 (v/v); Fluss: 1 ml/min; UV-Detektion: 220 nm; Temperatur: 40°C].

### Beispiel 389A (Enantiomer 2):

Ausbeute: 72 mg
Rₜ 4.59 min; Reinheit >96%; >98.8% ee
[Säule: Daicel Chiralpak AD-H, 5 µm, 250 mm x 4.6 mm; Elutionsmittel: Isohexan/(Ethanol + 0.2% Trifluoressigsäure + 1% Wasser) 80:20 (v/v); Fluss: 1 ml/min; UV-Detektion: 220 nm; Temperatur: 40°C].

### Beispiel 390A

### tert.-Butyl-3-{3-[(cyclopentyl{4-[(4-oxochinazolin-3(4H)-yl)methyl]phenyl}acetyl)amino]-2-methylphenyl}propanoat

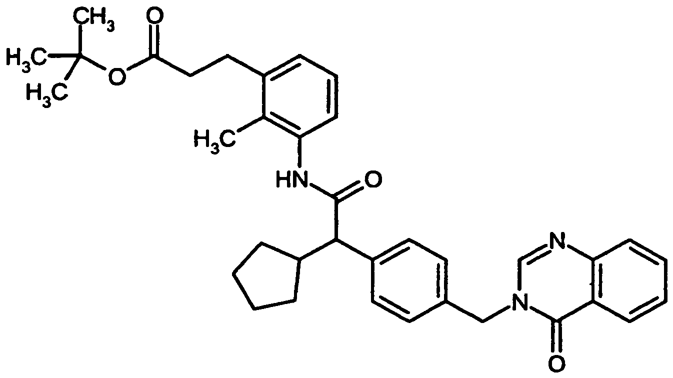

### Darsteltunizsmethode 8:

Eine Lösung von 34 mg (0.23 mmol) Chinazolin-4(3*H*)-on in 5 ml DMF wurde mit 15 mg (0.39 mmol, Gehalt 60%) Natriumhydrid versetzt und 30 min bei 0°C unter einer Argonatmosphäre gerührt. Anschließend wurden 100 mg (0.19 mmol) *tert*.-Butyl-3-[3-{[4erommethyl)phenyl](cyclopentyl)acetyl}amino)-2-methylphenyl]propanoat, gelöst in 1 ml DMF, zu der Reaktionslösung gegeben und diese langsam auf Raumtemperatur erwärmt. Nach vollständiger Umsetzung (DC-Kontrolle; Cyclohexan/Essigsäureethylester 2:1) wurde das Reaktionsgemisch mit 1 ml gesättigter Ammoniumchlorid-Lösung versetzt und direkt mittels präparativer HPLC aufgereinigt. Es wurden 28 mg (0.05 mmol, 25% d. Th.) der racemischen Titelverbindung erhalten.
LC-MS (Methode 10): Rₜ = 2.51 min; m/z = 580 (M+H)⁺.
¹H-NMR (400 MHz, DMSO-d₆): δ = 9.45 (s, 1H), 8.57 (s, 1H), 8.16 (d, 1H), 7.80-7.88 (m, 1H), 7.70 (d, 1H), 7.56 (t, 1H), 7.40 (d, 2H), 7.31 (d, 2H), 6.87-7.06 (m, 3H), 5.19 (s, 2H), 3.45 (d, 1H), 2.77 (t, 2H), 2.41 (t, 2H), 1.97 (s, 3H), 1.82 (dd, 1H), 1.29-1.72 (m, 14H), 0.79-1.03 (m, 1H).

### Beispiel 391A

### tert.-Butyl-3-{3-[(cyclopentyl {4-[(4-methyl-1-oxophthalazin-2(1H)-yl)methyl)phenyl}acetyl)-amino]-2-methylphenyl}propanoat

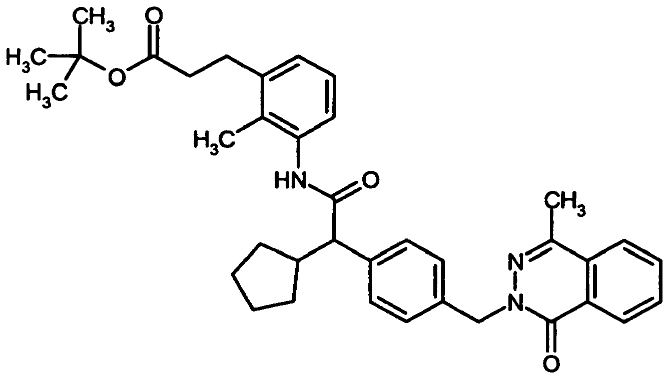

### Darstellungsmethode 9:

50 mg (0.097 mmol) *tert*.-Butyl-3-[3-({[4-(brommethyl)phenyl](cyclopentyl)acetyl}amino)-2-methylphenyl]propanoat, 19 mg (0.12 mmol) 4-Methylphthalazin-1(2*H*)-on sowie 47 mg (0.15 mmol) Cäsiumcarbonat wurden in 5 ml DMF 12 h lang bei 60°C gerührt. Nach dem Abkühlen wurde der Ansatz direkt mittels präparativer HPLC aufgereinigt. Es wurden 43 mg (0.07 mmol, 74% d. Th.) der racemischen Titelverbindung erhalten.
LC-MS (Methode 10): Rₜ = 2.70 min; m/z = 594 (M+H)⁺.

Nach den Darstellungsmethoden 8 und 9 wurden auf analoge Weise die in der folgenden Tabelle aufgeführten Verbindungen hergestellt:

| **Beispiel** | **Name / Struktur / Edukte** | **Analytische Daten** |
|---|---|---|
| **392A** | *tert*.-Butyl-3-{3-[(cyclopentyl{4-[(1-oxoisochinolin-2(1*H*)-yl)methyl]phenyl}acetyl)amino]-2-methylphenyl}-propanoat | LC-MS (Methode 10): Rₜ = 2.64 min; m/z = 579 (M+H)⁺. |
| | 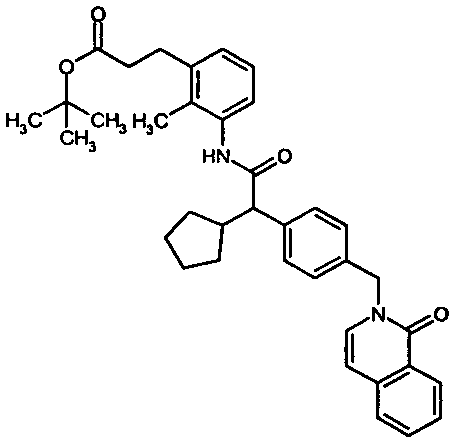 | |
| | (aus *tert*.-Butyl-3-[3-({[4-(brommethyl)phenyl]-(cyclopentyl)acetyl}amino)-2-methylphenyl]propanoat und Isochinolin-1(2*H*)-on) | |
| **393A** | *tert*.-Butyl-3-{3-[(cyclopentyl{4-[(2-oxo-3,4-dihydro-chinolin-1(2*H*)-yl)methyl]phenyl}acetyl)amino]-2-methylphenyl}propanoat | LC-MS (Methode 7): Rₜ = 3.02 min; m/z = 598 (M+NH₄)⁺. |
| | 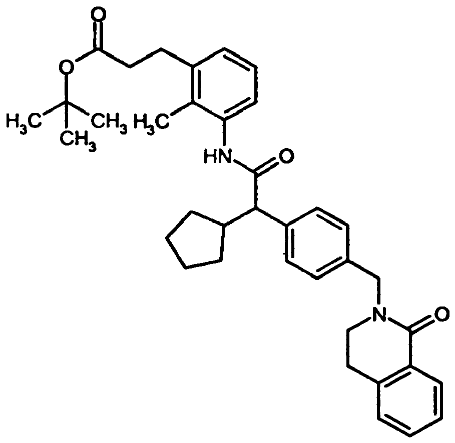 | |
| | (aus *tert*.-Butyl-3-[3-({[4-(brommethyl)phenyl]-(cyclopentyl)acetyl}amino)-2-methytphenyl]propanoat und 3,4-Dihydrochinolin-2(1*H*)-on) | |
| **394A** | *tert*.-Butyl-3-{3-[(cyclopentyl{4-[(1-oxophthalazin-2(1*H*)-yl)methyl]phenyl}acetyl)amino]-2-methyl-phenyl}propanoat | LC-MS (Methode 10): Rₜ = 2.63 min; m/z = 578 (M-H)⁻. ¹H-NMR (400 MHz, DMSO-d₆): δ = 9.45 (s, 1H), 8.47 (s, 1H), 8.29 (d, 1H), 7.93-8.01 (m, 2H), 7.84-7.92 (m, 1H), 7.38 (d, 2H), 7.28 (d, 2H), 6.92-7.05 (m, 3H), 5.32 (s, 2H), 3.45 (d, 1H), 2.78 (t, 2H), 2.34-2.42 (m, 3H), 1.98 (s, 3H), 1.77-1.88 (m, 1H), 1.42-1.71 (m, 4H), 1.30-1.40 (m, 11H), 0.89-1.05 (m, 1H). |
| | 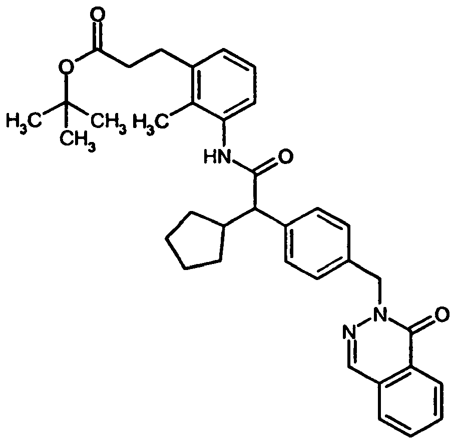 | |
| | (aus *tert*.-Butyl-3-[3-({[4-(bronunethyl)phenyl]-(cyclopentyl)acetyl}amino)-2-methylphenyl]propanoat und Phthalazin-1 (2*H*)-on) | |
| **395A** | *tert*.-Butyl-3-(3-{[{4-[(6-chlor-2-oxo-3,4-dihydro-chinolin-1(2*H*)-yl)methyl]phenyl}(cyclopentyl)-acetyl]amino}-2-methylphenyl)propanoat | LC-MS (Methode 11): Rₜ = 1.64 min; m/z = 614 (M-H)⁻. |
| | 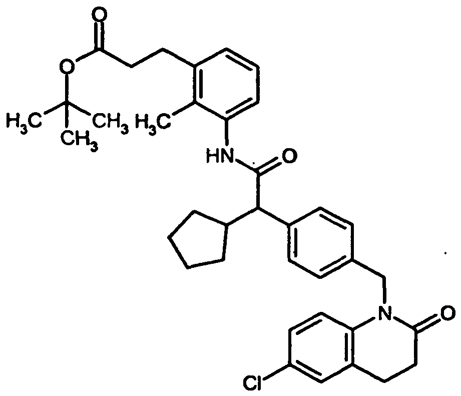 | |
| | (aus *tert*.-Butyl-3-[3-({[4-(bronmethyl)phenyl]-(cyclopentyl)acetyl}amino)-2-methylphenyl]propanoat-und 6-Chlor-3,4-dihydrochinolin-2(1*H*)-on) | |
| **396A** | *tert.-* Butyl-3-(3-{[{4-[(3-chlor-6-oxopyridazin-1(6*H*)-yl)methyl]phenyl}(cyclopentyl)acetyl]amino}-2-methylphenyl)propanoat | LC-MS (Methode 10): Rₜ = 2.53 min; m/z = 562 (M-H)⁻. ¹H-NMR (400 MHz, DMSO-d₆): δ = 9.47 (s, 1H), 7.57 (d, 1H), 7.40 (d, 2H), 7.24 (d, 2H), 7.09 (d, 1H), 6.85-7.05 (m, 3H), 5.18 (s, 2H), 3.46 (d, 1H), 2.79 (t, 2H), 2.41 (t, 2H), 1.99 (s, 3H), 1.81-1.88 (m, 1H), 1.61-1.74 (m, 1H), 1.50-1.60 (m, 2H), 1.46 (d, 1H), 1.33-1.42 (m, 12H), 0.96 (dd, 1H). |
| | 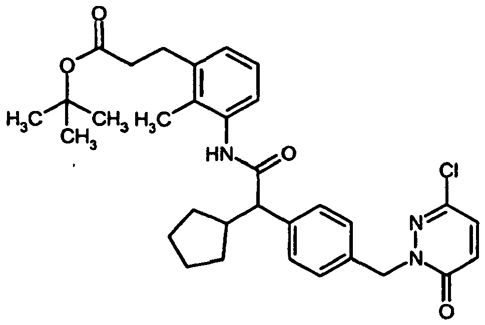 | |
| | (aus *tert*.-Butyl-3-[3-({[4-(brommethyl)phenyl]-(cyclopentyl)acetyl}amino)-2-methylphenyl]propanoat und 6-Chlorpyridazin-3(2*H*)-on) | |
| **397A** | *tert*.-Butyl-3-{3-[(cyclopentyl{4-[(1,1-dioxido-3-oxo-1,2-benzisothiazol-2(3*H*)-yl)methyl]phenyl}acetyl)amino]-2-methylphenyl}propanoat | LC-MS (Methode 10): Rₜ = 2.65 min; m/z = 615 (M-H)⁻. |
| | 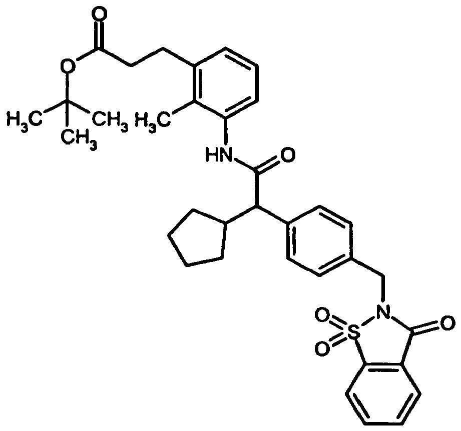 | |
| | (aus *tert*.-Butyl-3-[3-({[4-(brommethyl)phenyl]-(cyclopentyl)acetyl}amino)-2-methylphenyl]propanoat und 1,2-Benzisothiazol-3(2*H*)-on-1,1-dioxid) | |
| **398A** | *tert*.-Butyl-3-{3-[(cyclopentyl{4-[(3-methyl-6-oxo-pyridazin-1 (6H)-yl)methyl]phenyl}acetyl)amino]-2-methylphenyl}propanoat | LC-MS (Methode 11): Rₜ = 1.46 min; m/z = 542 (M-H)⁻. ¹H-NMR (400 MHz, DMSO-d₆): δ = 9.46 (s, 1H), 7.36 (dd, 3H), 7.22 (d, 2H), 6.95-7.04 (m, 3H), 6.91 (d, 1H), 5.16 (s, 2H), 3.48-3.53 (m, 1H), 3.45 (d, 1H), 2.79 (t, 2H), 2.41 (t, 2H), 2.26 (s, 3H), 1.99 (s, 3H), 1.83 (dd, 1H), 1.62-1.71 (m, 1H), 1.52-1.61 (m, 2H), 1.41-1.50 (m, 1H), 1.36 (m, 11H), 0.96 (dd, 1H). |
| | 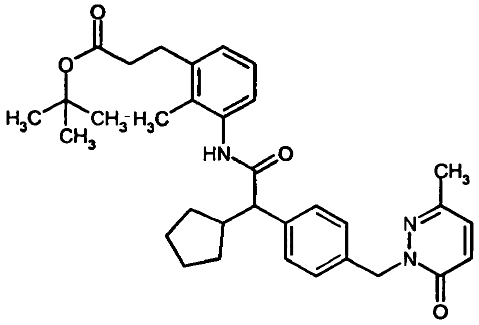 | |
| | (aus *tert*.-Butyl-3-[3-({[4-(brommethyl)phenyl]-(cyclopentyl)acetyl}amino)-2-methylphenyl]propanoat und 6-Methylpyridazin-3(2*H*)-on) | |
| **399A** | *tert*.-Butyl-3-{3-[(cyclopentyl{4-[(5-methyl-2-oxo-pyridin-1(2*H*)-yl)methyl]phenyl}acetyl)amino]-2-methylphenyl}propanoat | LC-MS (Methode 15): Rₜ = 1.27 min; m/z = 487 (M-C₄H₈+H)⁺. ¹H-NMR (400 MHz, DMSO-d₆): δ = 9.47 (s, 1H), 7.56 (s, 1H), 7.38 (d, 2H), 7.29 (dd, 1H), 7.22 (d, 2H), 6.94-7.05 (m, 3H), 6.37 (d, 1H), 5.02 (s, 2H), 3.45 (d, 1H), 2.79 (t, 2H), 2.56-2.65 (m, 1H), 2.41 (t, 2H), 2.00 (s, 3H), 1.99 (s, 3H), 1.78-1.89 (m, 1H), 1.62-1.74 (m, 1H), 1.52-1.61 (m, 2H), 1.42-1.52 (m, 1H), 1.30-1.40 (m, 11H), 0.91-1.02 (m, 1H). |
| | 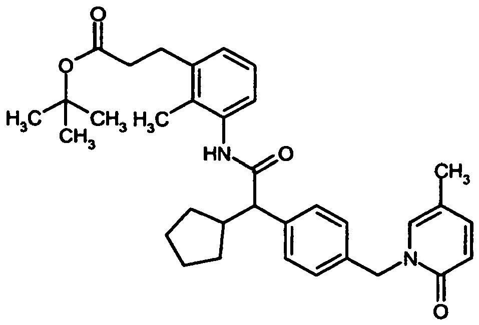 | |
| | (aus *tert*.-Butyl-3-[3-({[4-(brommethyl)phenyl]-(cyclopentyl)acetyl}amino)-2-methylphenyl]propanoat und 5-Methylpyridin-2(1*H*)-on) | |
| **400A** | *tert*.-Butyl-3-{3-[(cyclopentyl{4-[(3-oxo-1,2-benz-isoxazol-2(3*H*)-yl)methyl]phenyl}acetyl)amino]-2-methylphenyl}propanoat | LC-MS (Methode 11): Rₜ = 1.54 min; m/z = 513 (M-C₄H₈+H)⁺. |
| | 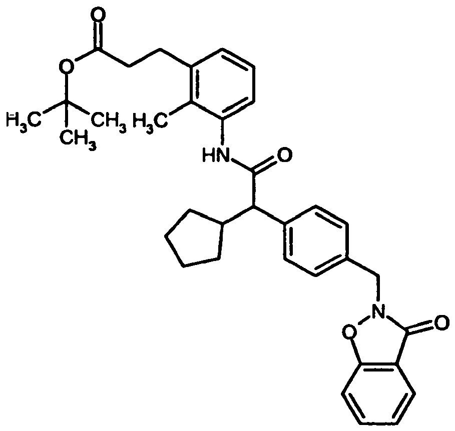 | |
| | (aus *tert*.-Butyl-3-[3-({[4-(brommethyl)phenyl](cyclo-pentyl)acetyl}amino)-2-methylphenyl]propanoat und 1,2-Benzisoxazol-3(2*H*)-on [CAS Reg.-Nr. 21725-69-9]) | |
| **401A** | *tert*.-Butyl-3-(3-{[cyclopentyl(4-{[6-oxo-3-(trifluor-methyl)pyridazin-1(6*H*)-yl]methyl}phenyl)acetyl]-amino} -2-methylphenyl)propanoat | LC-MS (Methode 15): Rₜ = 1.39 min; m/z = 542 (M-C₄+H₈+H)⁺. ¹H-NMR (400 MHz, DMSO-d₆): δ = 9.45 (s, 1H), 7.86 (d, 1H), 7.41 (d, 2H), 7.26 (d, 2H), 7.21 (d, 1H), 6.93-7.05 (m, 3H), 5.75 (s, 2H), 3.46 (d, 1H), 2.78 (t, 2H), 2.53-2.62 (m, 1H), 2.41 (t, 2H), 1.97 (s, 3H), 1.79-1.89 (m, 1H), 1.42-1.72 (m, 4H), 1.33-1.39 (m, 11H), 0.90-1.02 (m, 1H). |
| | 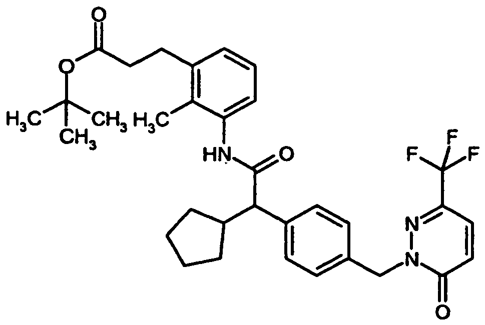 | |
| | (aus *tert*.-Butyl-3-[3-({[4-(brommethyl)phenyl]-(cyclopentyl)acetyl}amino)-2-methylphenyl]propanoat und 6-(Trifluormethyl)pyridazin-3(2*H*)-on) | |

### Beispiel 402A

### (+)-1-(3-{[(2S)-2-Cyclopentyl-2-{4-[(5-oxo-2-phenyl-5,6-dihydro-4H-1,3,4-oxadiazin-4-yl)-methyl]phenyl}acetyl]arnino}benzyl)cyclopropancarbonsäure-tert.-butylester

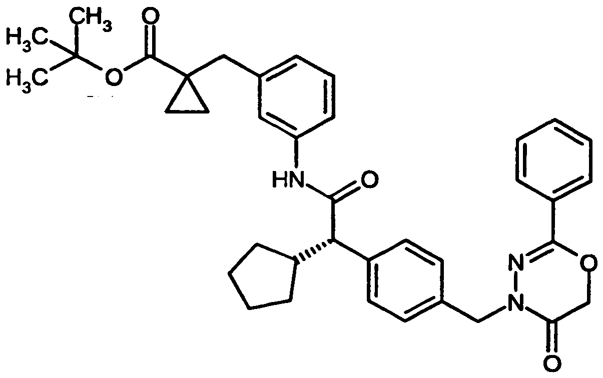

Zu einer Lösung von 786.8 mg (2.0 mmol) (2*S*)-Cyclopentyl{4-[(5-oxo-2-phenyl-5,6-dihydro-4*H-*1,3,4-oxadiazin-4-yl)methyl]phenyl}ethansäure und 325.1 mg (2.41 mmol) 1-Hydroxy-1*H*-benzotriazol-Hydrat in 4 ml DMF wurden bei 0°C 873 µl (5.01 mmol) DIEA und 595.0 mg (2.41 mmol) 1-(3-Aminobenzyl)cyclopropancarbonsäure-*tert*.-butylester gegeben. Die resultierende Mischung wurde bei 0°C portionsweise mit 838.5 mg (2.21 mmol) HATU versetzt. Das Reaktionsgemisch wurde dann langsam auf RT erwärmt und 3 h bei RT gerührt. Man gab die Mischung danach auf Wasser und extrahierte dreimal mit Ethylacetat. Die vereinigten organischen Phasen wurden über Magnesiumsulfat getrocknet und im Vakuum eingeengt. Das Produkt wurde durch Chromatographie an Silicagel gereinigt (Laufmittel Cyclohexan/Ethylacetat 4:1 bis 3:1). Es wurden 827 mg (66.4% d. Th.) der Titelverbindung erhalten.
LC-MS (Methode 10): Rₜ = 2.92 min; m/z = 566 (M-C₄H₈)⁺.
¹H-NMR (400 MHz, DMSO-d₆): δ = 9.95 (s, 1 H), 7.77 (d, 2 H), 7.54-7.36 (m, 6 H), 7.37-7.26 (m, 3 H), 7.14 (t, 1 H), 6.88 (d, 1 H), 4.90 (s, 2 H), 4.84 (s, 2 H), 3.38 (d, 1 H), 2.76 (s, 2 H), 2.65-2.56 (m, 1H), 1.85-1.71 (m, 1 H), 1.70-1.30 (m, 5 H), 1.34 (s, 9 H), 1.05 (d, 2 H), 1.03-0.91 (m, 1 H), 0.91-0.81 (m, 1 H), 0.79 (d, 2 H).
[α]_{D}²⁰ = +27.2°, c = 0.455, Chloroform.

### Beispiel 403A

### (+)-1-(3-{[(2S)-2-Cyclopentyl-2-{4-[(5-oxo-2-phenyl-5,6-dihydro-4H-1,3,4-oxadiazin-4-yl)-methyl]phenyl}acetyl]amino}-2-methylbenzyl)cyclopropancarbonsäure-tert.-butylester

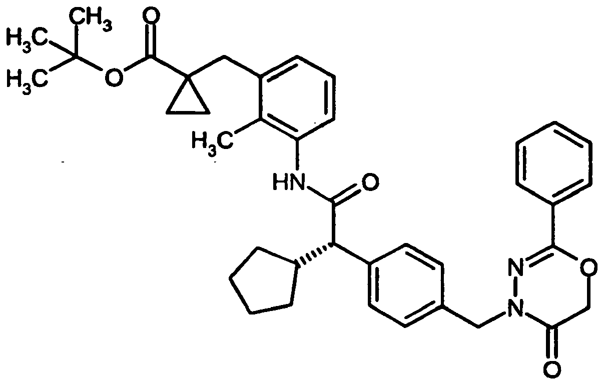

Zu einer Lösung von 150.0 mg (0.38 mmol) (2S)-Cyclopentyl{4-[(5-oxo-2-phenyl-5,6-dihydro-4*H-*1,3,4-oxadiazin-4-yl)methyl]phenyl}ethansäure und 62 mg (0.46 mmol) 1-Hydroxy-1*H-*benzotriazol-Hydrat in 500 µl DMF wurden bei 0°C 200 µl (1.15 mmol) DIEA und 149.8 mg (0.57 mmol) 1-(3-Amino-2-methylbenzyl)cyclopropancarbonsäure-tert.-butylester gegeben. Die resultierende Mischung wurde bei 0°C portionsweise mit 174.4 mg (0.46 mmol) HATU versetzt. Das Reaktionsgemisch wurde dann langsam auf RT erwärmt und 3 h bei RT gerührt. Die Mischung wurde danach auf Wasser gegeben und dreimal mit Ethylacetat extrahiert. Die vereinigten organischen Phasen wurden über Magnesiumsulfat getrocknet und im Vakuum eingeengt. Das Rohprodukt wurde durch präparative RP-HPLC gereinigt (Eluent: Acetonitril/Wasser-Gradient). Es wurden 175 mg (72.0% d. Th.) der Titelverbindung erhalten.
LC-MS (Methode 7): Rₜ = 3.23 min; m/z = 580 (M-C₄H₈)⁺.
¹H-NMR (400 MHz, DMSO-*d*₆): δ = 9.48 (s, 1 H), 7.76 (d, 2 H), 7.54-7.37 (m, 5 H), 7.31 (d, 2 H), 7.06-6.93 (m, 3 H), 4.92 (s, 2 H), 4.86 (s, 2 H), 3.44 (d, 1 H), 2.85 (s, 2 H), 2.63-2.55 (m, 1 H), 1.90 (s, 3 H), 1.88-1.79 (m, 1 H), 1.71-1.41 (m, 4 H), 1.39 (s, 2 H), 1.27 (s, 9 H), 1.10 (d, 2 H), 1.06-0.92 (m, 1 H), 0.62 (q, 2 H).
[α]_{D}²⁰ = +15.6°, c = 0.500, Chloroform.

### Beispiel 404A

### (+1-)-1-{3-[(Cyclopentyl (4-[(1-oxo-1,3-dihydro-2H-isoindol-2-yl)methyl]phenyl)acetyl)amino]-benzyl}cyclopropancarbonsäure-tert.-butylester

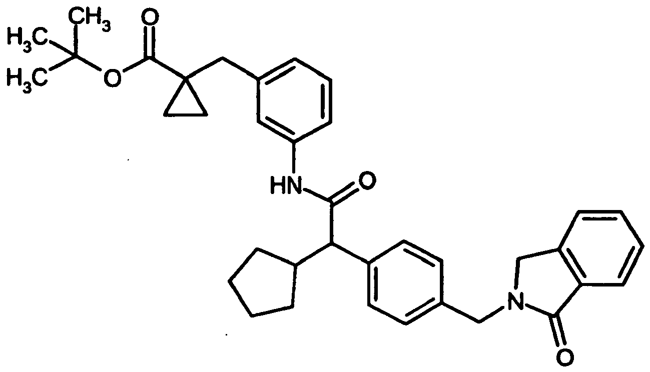

107.4 mg (0.31 mmol) (+/-)-Cyclopentyl{4-[(1-oxo-1,3-dihydro-2*H*-isoindol-2-yl)methyl]phenyl}-essigsäure wurden in 1 ml DMF und 0.17 ml (2.15 mmol) Pyridin vorgelegt, mit 128.6 mg (0.34 mmol) 1-[Bis-(dimethylamino)methylen]-5-chlor-3-oxy-1*H-*benzotriazol-1-ium-Tetrafluoroborat sowie 80 mg (0.31 mmol) 1-(3-Aminobenzyl)cyclopropancarbonsäre-tert.-butylester versetzt und über Nacht bei RT gerührt. Das Reaktionsgemisch wurde dann mit wenig Acetonitril verdünnt und direkt mittels präparativer RP-HPLC aufgereinigt (Eluent: Acetonitril/Wasser-Gradient). Es wurden 112 mg (50.4% d. Th.) der Titelverbindung erhalten.
LC-MS (Methode 7): Rₜ = 3.03 min; m/z = 523 (M-C₄H₈+H)⁺.
¹H-NMR (400 MHz, DMSO-*d*₆): δ = 9.95 (s, 1 H), 7.71 (d, 1 H), 7.62-7.47 (m, 5 H), 7.39 (d, 2 H), 7.22 (d, 2 H), 7.14 (t, 1 H), 6.88 (d, 1 H), 4.73-4.65 (s, 2 H), 4.39-4.31 (s, 2 H), 3.39 (d, 1 H), 2.76 (s, 2 H), 2.64-2.56 (m, 1 H), 1.85-1.70 (m, 1 H), 1.69-1.39 (m, 4 H), 1.40-1.29 (m, 1 H), 1.30-1.24 (m, 1 H), 1.22 (s, 9 H), 1.05 (q, 2 H), 1.02-0.89 (m, 1 H), 0.78 (q, 2 H).

### Allgemeine Vorschrift 6: Kupplung einer Carbonsäure mit einem Amin unter HATU-Aktivierung

Zu einer Lösung von 1 eq. der betreffenden Carbonsäure und 1.0 bis 1.5 eq. des betreffenden Amins in DMF (ca. 0.03 bis 0.5 mol/l) wurden bei RT 1.2 bis 2.5 eq. DIEA gegeben. Die resultierende Mischung wurde auf 0°C gekühlt und portionsweise mit ca. 1.2 eq. HATU versetzt. Das Reaktionsgemisch wurde dann langsam auf RT erwärmt und 1 h bis 24 h bei RT gerührt. Das Zielprodukt konnte durch präparative RP-HPLC (Eluent: Acetonitril/Wasser-Gradient) direkt aus der Reaktionsmischung oder nach wässriger Aufarbeitung und einem weiteren Reinigungsschritt gewonnen werden. Dazu wurde die Reaktionsmischung auf Wasser gegeben und dreimal mit Ethylacetat extrahiert. Die vereinigten organischen Phasen wurden über Magnesiumsulfat getrocknet und im Vakuum eingeengt. Das Rohprodukt wurde durch präparative RP-HPLC (Eluent: Acetonitril/Wasser-Gradient) oder durch Chromatographie an Silicagel (Laufmittel-Gemische aus Cyclohexan/Ethylacetat oder Dichlormethan/Methanol) gereinigt.

Die folgenden Verbindungen wurden gemäß der Allgemeinen Vorschrift 6 hergestellt:

| **Beispiel** | **Name / Struktur** | **Analytische Daten** |
|---|---|---|
| **405A** | 1-(3-{[(2*S*)-2-Cyclopentyl-2-{4-[(1-oxo-1,3-dihydro-2*H*-isoindol-2-yl)methyl]phenyl}acetyl]amino}-benzyl)cyclopropancarbonsäure-*tert*.-butylester | LC-MS (Methode 11): Rₜ = 1.58 min; m/z = 523 (M-C₄H₈+H)⁺. ¹H-NMR (400 MHz, DMSO-d₆): δ = 9.95 (s, 1H), 7.72 (d, 1H), 7.62-7.44 (m, 4H), 7.39 (d, 2H), 7.35 (d, 1H), 7.22 (d, 2H), 7.14 (t, 1H), 6.88 (d, 1H), 4.69 (s, 2H), 4.35 (s, 2H), 3.40-3.35 (m, 1H), 2.76 (s, 2H), 2.63-2.55 (m, 1H), 1.81-1.71 (m, 1H), 1.68-1.41 (m, 4H), 1.40-1.31 (m, 1H), 1.30-1.24 (m, 1H), 1.22 (s, 9H), 1.05 (q, 2H), 1.01-0.90 (m, 1H), 0.79 (q, 2H). |
| | 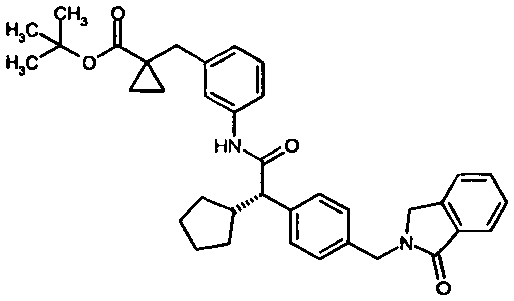 | |
| **406A** | 1-{3-[(Cyclopentyl{4-[(3-oxo-1,3-dihydro-2*H*-indazol-2-yl)methyl]phenyl}acetyl)amino]benzyl}-cyclopropancarbonsäure-*tert.*-butylester | LC-MS (Methode 11): Rₜ =1.55 min; m/z = 524 (M-C₄H₈)⁺. ¹H-NMR (400 MHz, CDCl₃): δ = 7.73 (d, 1H), 7.40-7.33 (m, 1H), 7.32-7.26 (m, 4H), 7.18 (dd, 3H), 7.11 (t, 1H), 7.05 (t, 1H), 6.93 (d, 1H), 5.29 (s, 2H), 5.24 (s, 2H), 3.08 (d, 1H), 2.84 (s, 2H), 2.65-2.54 (m, 1H), 2.04-1.93 (m, 1H), 1.64-1.34 (m, 7H), 1.30 (s, 9H), 1.18-1.14 (m, 2H), 0.71-0.66 (m, 2H). |
| | 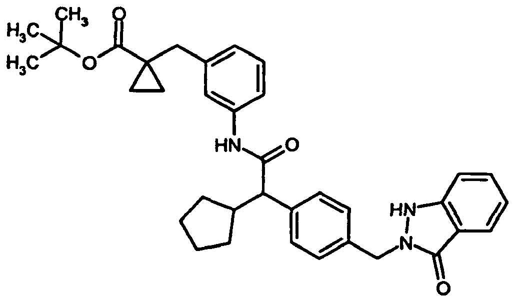 | |
| **407A** | 1-{3-[(Cyclopentyl{4-[(5,6-difluor-1-oxo-1,3-dihydro-2*H*-isoindol-2-yl)methyl]phenyl}acetyl)amino]-benzyl}cyclopropancarbonsäure-*tert.*-butylester | LC-MS (Methode 15): Rₜ = 1.44 min; m/z = 559 (M-C₄H₈)⁺. ¹H-NMR (400 MHz, DMSO-d₆): δ = 9.94 (s, 1H), 7.75 (dd, 1H), 7.65 (dd, 1H), 7.49 (s, 1H), 7.39 (d, 2H), 7.35 (d, 1H), 7.22 (d, 2H), 7.14 (t, 1H), 6.88 (d, 1H), 4.68 (s, 2H), 4.32 (s, 2H), 3.37 (d, 1H), 2.77 (s, 1H), 2.64-2.54 (m, 2H), 1.83-1.72 (m, 1H), 1.68-1.31 (m, 4H), 1.23 (s, 9H), 1.07-1.02 (m, 1H), 1.01-0.90 (m, 1H), 0.82-0.75 (m,1H). |
| | 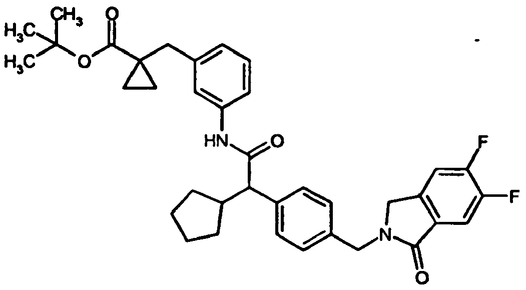 | |
| **408A** | 1-{3-[(Cyclopentyl{4-[(4,7-difluor-1-oxo-1,3-dihydro-2*H*-isoindol-2-yl)methyl]phenyl}acetyl)amino]-benzyl}cyclopropancarbonsäure-*tert.*-butylester | LC-MS (Methode 11): Rₜ = 1.62 min; m/z = 559 (M-C₄H₈)⁺. ¹H-NMR (400 MHz, DMSO-d₆): δ = 9.95 (s, 1H), 7.51-7.44 (m, 2H), 7.40 (d, 2H), 7.36-7.31 (m, 2H), 7.25 (d, 2H), 7.14 (t, 1H), 6.88 (d, 1H), 4.65 (s, 2H), 4.46 (s, 2H), 3.38 (d, 1H), 2.76 (s, 2H), 2.63-2.56 (m, 1H), 1.82-1.73 (m, 1H), 1.67-1.28 (m, 7H), 1.22 (s, 9H), 1.07-1.03 (m, 2H), 0.81-0.77 (m, 2H). |
| | 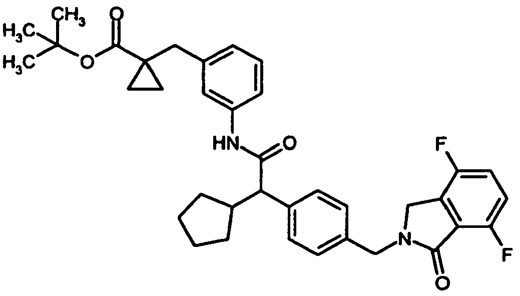 | |
| **409A** | 1-(3-{[Cyclopentyl(4-{[2-oxo-5-(trifluormethyl)-pyridin-1(2*H*)-yl]methyl}phenyl)acetyl]amino}-benzyl)cyclopropancarbonsäure-*tert.*-butylester | LC-MS (Methode 15): Rₜ = 1.41 min; m/z = 553 (M-C₄H₈)⁺. ¹H-NMR (400 MHz, DMSO-d₆): δ = 9.94 (s, 1H), 8.54 (br. s, 1H), 7.66 (s, 1H), 7.49 (s, 1H), 7.38 (d, 2H), 7.33 (d, 1H), 7.27 (d, 2H), 7.14 (t, 1H), 6.89 (d, 1H), 6.56 (d, 1H), 5.76 (s, 1H), 5.12 (s, 2H), 3.35 (d, 1H), 2.76 (s, 2H), 2.37 (s, 1H), 2.35-2.31 (m, 1H), 1.78 (br. s, 1H), 1.68-1.40 (m, 4H), 1.40-1.28 (m, 1H), 1.23 (s, 9H), 1.05 (br. s, 2H), 0.80 (br. s, 2H). |
| | 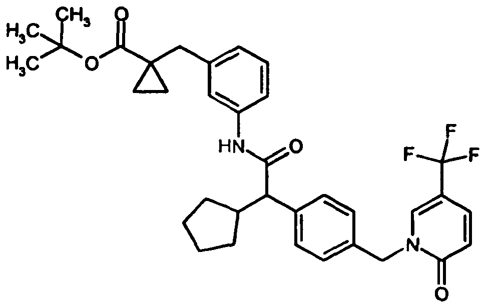 | |
| **410A** | (+)-1-(3-{[(2*S*)-2-{4-[(5-Chlor-2-oxopyridin-1(2,*H*)-yl)-methyl]phenyl}-2-cyclopentylacetyl]amino}benzyl)-cyclopropancarbonsäure-*tert.*-butylester | LC-MS (Methode 12): Rₜ = 2.72 min; m/z = 519 (M-C₄H₈)⁺. ¹H-NMR (400 MHz, DMSO-d₆): δ = 9.94 (s, 1H), 8.09 (d, 1H), 7.51-7.44 (m, 2H), 7.37 (d, 2H), 7.34 (d, 1H), 7.25 (d, 2H), 7.14 (t, 1H), 6.88 (d, 1H), 6.44 (d, 1H), 5.02 (s, 2H), 3.37 (d, 1H), 2.77 (s, 1H), 1.82-1.72 (m, 1H), 1.69-1.27 (m, 8H), 1.24 (s, 9H), 1.07-1.02 (m, 2H), 0.99-0.91 (m, 1H), 0.82-0.78 (m, 2H). [α]_{D}²⁰ = +40.9 °, c = 0.455, Chloroform. |
| | 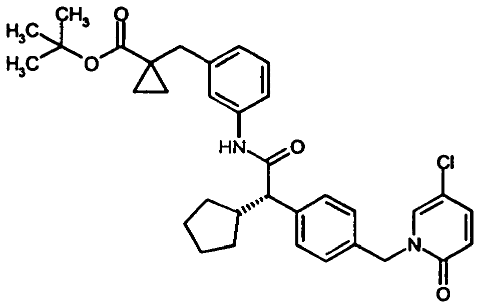 | |
| **411A** | (+)-3-(3-{[(2*S*)-2-{4-[(5-Chlor-2-oxopyridin-1(2*H*)-yl)-methyl]phenyl}-2-cyclopentylacetyl]amino}phenyl)-propansäure-*tert.*-butylester | LC-MS (Methode 15): Rₜ=1.31 min; m/z = 549 (M+H)⁺. ¹H-NMR (400 MHz, DMSO-d₆): δ = 9.96 (s, 1H), 8.09 (d, 1H), 7.47 (dd, 1H), 7.45 (s, 1H), 7.37 (d, 2H), 7.34 (d, 1H), 7.25 (d, 2H), 7.15 (t, 1H), 6.86 (d, 1H), 6.44 (d, 1H), 5.03 (s, 2H), 3.36 (d, 1H), 2.74 (t, 2H), 2.63-2.55 (m, 1H), 2.48-2.42 (m, 2H), 1.81-1.32 (m, 6H), 1.31 (s, 9H), 1.27-1.20 (m, 1H), 1.02-0.90 (m, 1H). [α]_{D}²⁰ = +44.4°, c = 0.490, Chloroform. |
| | 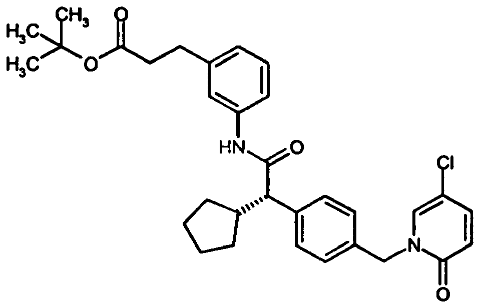 | |
| **412A** | (+)-3-(3-{[(2*S*,3*R*)-4,4,4-Trifluor-3-methyl-2-(4-{[2-oxo-5-(trifluormethyl)pyridin-1(2*H*)-yl]methyl}-phenyl)butanoyl]amino}phenyl)propansäure-*tert.*-butylester | LC-MS (Methode 11): Rₜ = 1.51 min; m/z = 555 (M-C₄H₈)⁺. ¹H-NMR (400 MHz, CDCl₃): δ = 7.66 (br. s, 1H), 7.45 (dd, 1H), 7.39 (d, 2H), 7.35-7.28 (m, 3H), 7.24-7.16 (m, 2H), 7.11 (s, 1H), 6.94 (d, 1H), 6.67 (d, 1H), 5.14 (s, 2H), 3.57 (d, 1H), 3.45-3.30 (m, 1H), 2.85 (t, 2H), 2.50 (t, 2H), 1.40 (s, 9H), 0.90 (d, 3H). [α]_{D}²⁰ = +17.9 °, c = 0.360, Chloroform. |
| | 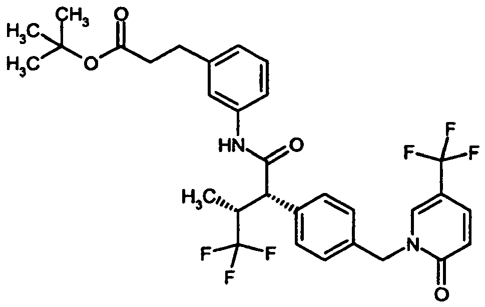 | |
| **413A** | (-)-3-(3-{[(2*R*,3*S*)-4,4,4-Trifluor-3-methyl-2-(4-([2-oxo-5-(trifluormethyl)pyridin-1(2*H*)-yl]methyl)-phenyl)butanoyl]amino}phenyl)propansäure-*tert*.-butylester | LC-MS (Methode 11): Rₜ = 1.51 min; m/z = 555 (M-C₄H₈)⁺. ¹H-NMR (400 MHz, CDCl₃): δ = 7.66 (br. s, 1H), 7.45 (dd, 1H), 7.39 (d, 2H), 7.34-7.29 (m, 3H), 7.24-7.16 (m, 2H), 7.10 (s, 1H), 6.94 (d, 1H), 6.68 (d, 1H), 5.14 (s, 2H), 3.57 (d, 1H), 3.44-3.32 (m, 1H), 2.85 (t, 2H), 2.56-2.45 (m, 2H), 1.40 (s, 9H), 0.90 (d, 3H). [α]_{D}²⁰ = -74°, c = 0.220, Chloroform. |
| | 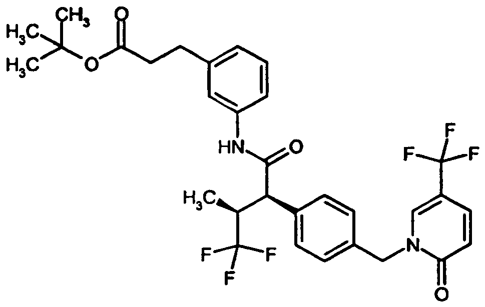 | |
| **414A** | (+)-1-(3-{[(2*S*)-2-Cyclopentyl-2-(4-{[2-oxo-5-(trifluor-methyl)pyridin-1(2*H*)-yl]methyl}phenyl)acetyl]-amino}benzyl)cyclopropancarbonsäure-*tert*.-butylester | LC-MS (Methode 15): Rₜ = 1.40 min; m/z = 553 (M-C₄H₈)⁺. ¹H-NMR (400 MHz, DMSO-d₆): δ = 9.94 (s, 1H), 8.54 (s, 1H), 7.67 (dd, 1H), 7.49 (s, 1H), 7.38 (d, 2H), 7.33 (d, 1H), 7.26 (d, 2H), 7.14 (t, 1H), 6.88 (d, 1H), 6.55 (d, 1H), 5.12 (s, 2H), 3.37 (d, 1H), 2.76 (d, 2H), 2.63-2.55 (m, 1H), 1.82-1.71 (m, 1H), 1.68-1.29 (m, 6H), 1.23 (s, 9H), 1.08-1.03 (m, 2H), 1.00-0.89 (m, 1H), 0.83-0.75 (m, 2H). [α]_{D}²⁰ = +22.1°, c = 0.505, Chloroform. |
| | 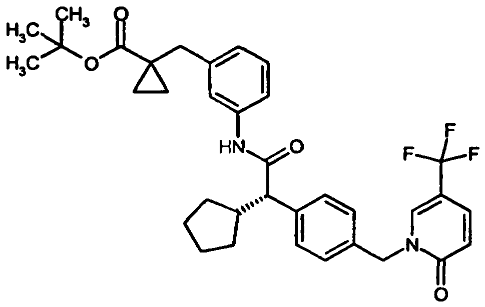 | |
| **415A** | (+)-3-(3-{[(2*S*)-2-Cyclopentyl-2-(4-{[2-oxo-5-(trifluor-methyl)pyridin-1(2*H*)-yl]methyl)phenyl)acetyl]-amino}phenyl)propansäure-*tert.*-butylester | LC-MS (Methode 15): Rₜ = 1.35 min; m/z = 527 (M-C₄H₈)⁺. ¹H-NMR (400 MHz, DMSO-d₆): δ = 9.97 (s, 1H), 8.54 (s, 1H), 7.67 (dd, 1H), 7.45 (s, 1H), 7.38 (d, 2H), 7.34 (d, 1H), 7.26 (d, 2H), 7.15 (t, 1H), 6.86 (d, 1H), 6.55 (d, 1H), 5.12 (s, 2H), 3.36 (d, 1H), 2.78-2.70 (m, 2H), 2.64-2.55 (m, 110, 2.48-2.40 (m, 2H),1.81-1.71 (m, 1H), 1.69-1.32 (m, 5H), 1.30 (s, 9H), 1.24 (dd, 1H), 0.88-0.99 (m, 1H). [α]_{D}²⁰ = +28.9°, c = 0.525, Chloroform. |
| | 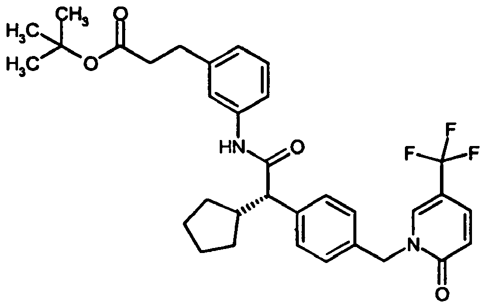 | |

### Beispiel 416A

### (+/-)-1-{3-[(4,4,4-Trifluor-3-methyl-2-(4-[(1-oxo-1,3-dihydro-2H-isoindol-2-yl)methyl]phenyl)-butanoyl)amino]benzyl}cyclocarbonsäure-tert.-butylester

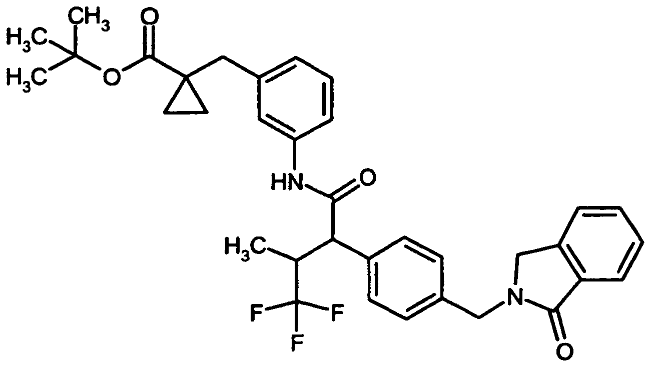

Zu einer Lösung von 206 mg (0.55 mmol) 4,4,4-Trifluor-3-methyl-2-{4-[(1-oxo-1,3-dihydro-2*H-*isoindol-2-yl)methyl]phenyl}butansäure, 100.3 mg (0.66 mmol) 1-Hydroxy-1*H*-benzotriazol-Hydrat und 285 µl (1.64 mmol) DIEA in 1 ml DMF wurden 202.5 mg (0.82 mmol) 1-(3-Aminobenzyl)cyclopropancarbonsäure-tert.-butylester gegeben. Die resultierende Mischung wurde bei 0°C portionsweise mit 249.1 mg (0.66 mmol) HATU versetzt. Das Reaktionsgemisch wurde dann langsam auf RT erwärmt und 2 h bei RT gerührt. Die Mischung wurde auf Wasser gegeben und dreimal mit Ethylacetat extrahiert. Die vereinigten organischen Phasen wurden über Magnesiumsulfat getrocknet und im Vakuum eingeengt. Das Rohprodukt wurde durch Chromatographie an Silicagel gereinigt (Laufmittel Cyclohexan/Ethylacetat 3:1). Es wurden 287 mg (87% d. Th.) der Titelverbindung erhalten.
LC-MS (Methode 12): Rₜ = 2.72 min; m/z = 551 (M-C₄H₈)⁺.

Das oben erhaltene Isomerengemisch (größtenteils Racemat eines Hauptdiastereomers) wurde durch präparative HPLC an chiraler Phase in die Enantiomere aufgetrennt [Säule: Daicel Chiralpak AD-H, 5 µm, 250 mm x 20 mm; Fluss: 18 ml/min; UV-Detektion: 230 nm; Injektionsvolumen: 0.5 ml; Temperatur: RT; Eluent: 60% Isohexan / 40% Ethanol]. Ausgehend von 250 mg Isomerengemisch wurden 115 mg von Enantiomer 1 und 99 mg von Enantiomer 2 isoliert (*siehe Beispiele 417A und 418A*)*.*

### Beispiel 417A

### (+)-1-{3-[(4,4,4-Trifluor-3-methyl-2-{4-[(1-oxo-1,3-dihydro-2H-isoindol-2-yl)methyl]phenyl}-butanoyl)amino]benzyl}cyclocarbonsäure-tert.-butylester (Enantiomer 1)

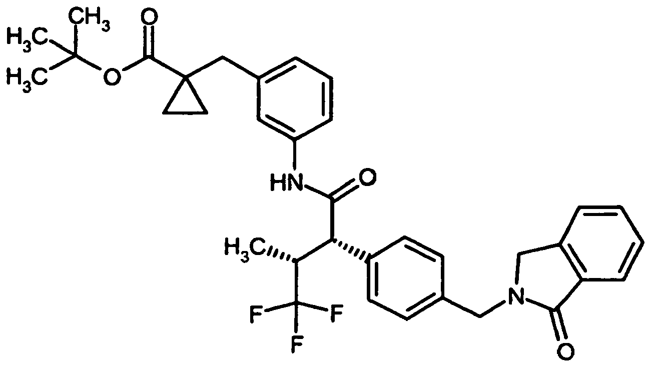

LC-MS (Methode 7): Rₜ = 2.94 min; m/z = 551 (M-C₄H₈)⁺.
¹H-NMR (500 MHz, DMSO-*d*₆): δ = 10.11 (s, 1 H), 7.71 (d, 1 H), 7.60-7.44 (m, 4 H), 7.40 (d, 2 H), 7.31 (d, 1 H), 7.26 (d, 2 H), 7.14 (t, 1 H), 6.89 (d, 1 H), 4.71 (s, 2 H), 4.35 (s, 2 H), 3.81 (d, 1 H), 2.76 (s, 2 H), 1.20 (s, 10 H), 1.09 (s, 1 H), 1.04 (br. s, 3 H), 0.90-0.82 (m, 1 H), 0.78 (br. s, 2 H).
[α]_{D}²⁰ = +61°, c = 0.265, Chloroform.

### Beispiel 418A

### (-)-1-{3-[(4,4,4-Trifluor-3-methyl-2-{4-[(1-oxo-1,3-dihydro-2H-isoindol-2-yl)methyl]phenyl}-butanoyl)amino]benzyl} cyclocarbonsäure-tert.-butylester (Enantiomer 2)

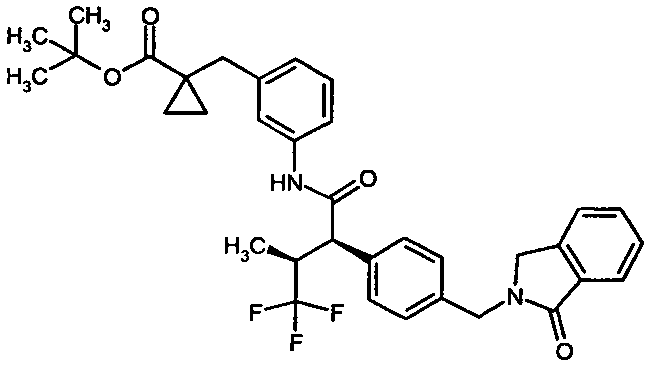

LC-MS (Methode 7): Rₜ = 2.95 min; m/z = 551 (M-C₄H₈)⁺.
¹H-NMR (500 MHz, DMSO-*d*₆): δ = 10.11 (s, 1 H), 7.71 (d, 1 H), 7.57 (d, 1 H), 7.54-7.44 (m, 3 H), 7.40 (d, 2 H), 7.31 (d, 1 H), 7.26 (d, 2 H), 7.14 (t, 1 H), 6.89 (d, 1 H), 4.71 (s, 2 H), 4.35 (s, 2 H), 3.81 (d, 1 H), 2.76 (s, 2 H), 1.47-1.40 (m, 1 H), 1.34-1.26 (m, 1 H), 1.20 (s, 9 H), 1.09 (s, 2 H), 1.04 (br. s, 3 H), 0.90-0.82 (m, 1 H).
[α]_{D}²⁰ = -61°, c = 0.285, Chloroform.

### Beispiel 419A

### (+/-)-1-{3-[(Cyclopentyl (4-[(4-fluor-1-oxo-1,3-dihydro-2H-isoindol-2-yl)methyl]phenyl)acetyl)-amino]benzyl}cyclopropancarbonsäure-tert.-butylester

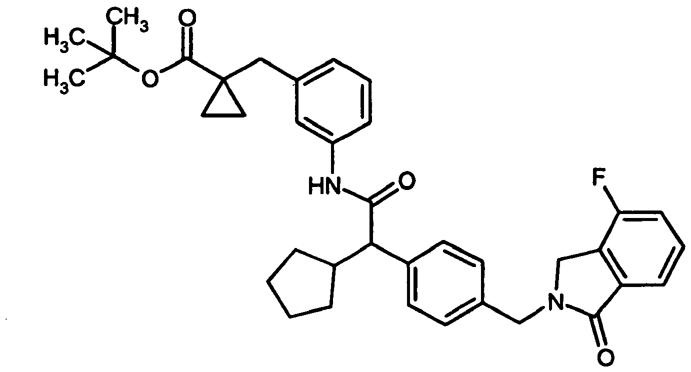

Zu einer Lösung von 96 mg (0.26 mmol) Cyclopentyl{4-[(4-fluor-1-oxo-1,3-dihydro-2*H*-isoindol-2-yl)methyl]phenyl}essigsäure in 1 ml DMF wurden 77.5 mg (0.31 mmol) 1-(3-Aminobenzyl)-cyclopropancarbonsäure-*tert*.-butylester gegeben. Die resultierende Mischung wurde bei 0°C mit 136 µl (0.78 mmol) DIEA und danach portionsweise mit 119.2 mg (0.31 mmol) HATU versetzt. Das Reaktionsgemisch wurde dann langsam auf RT erwärmt und 1 h bei RT gerührt. Der Ansatz wurde auf Wasser gegeben und dreimal mit Ethylacetat extrahiert. Die vereinigten organischen Phasen wurden über Magnesiumsulfat getrocknet und im Vakuum eingeengt. Es wurden 146 mg (93% d. Th.) der Titelverbindung erhalten.
LC-MS (Methode 15): Rₜ = 1.44 min; m/z = 541 (M-C₄H₈)⁺.
¹H-NMR (400 MHz, DMSO-*d*₆): δ = 9.95 (s, 1 H), 7.62-7.53 (m, 2 H), 7.50 (s, 1 H), 7.47-7.42 (m, 1 H), 7.39 (d, 2 H), 7.34 (d, 1 H), 7.24 (d, 2 H), 7.14 (t, 1 H), 6.88 (d, 1 H), 4.69 (s, 2 H), 4.46 (s, 2 H), 3.37 (d, 1 H), 2.76 (s, 2 H), 1.82-1.72 (m, 1 H), 1.68-1.24 (m, 7 H), 1.22 (s, 9 H), 1.09 (s, 1 H), 1.07-1.02 (m, 2 H), 0.81-0.76 (m, 2 H).

Das oben erhaltene Racemat wurde durch präparative HPLC an chiraler Phase in die Enantiomere aufgetrennt [Säule: Daicel Chiralpak AD-H, 5 µm, 250 mm x 20 mm; Fluss: 15 ml/min; UV-Detektion: 230 nm; Injektionsvolumen: 0.5 ml; Temperatur: 30°C; Eluent: 65% Isohexan / 35% Ethanol]. Ausgehend von 109 mg Racemat wurden 53 mg von Enantiomer 1 und 35 mg von Enantiomer 2 isoliert (*siehe Beispiele 420A und 421A*).

### Beispiel 420A

### (+)-1-{3-[(Cyclopentyl{4-[(4-fluor-1-oxo-1,3-dihydro-2H-isoindol-2-yl)methyl]phenyl}acetyl)-amino]benzyl}cyclopropancarbonsäure-tert.-butylester (Enantiomer 1)

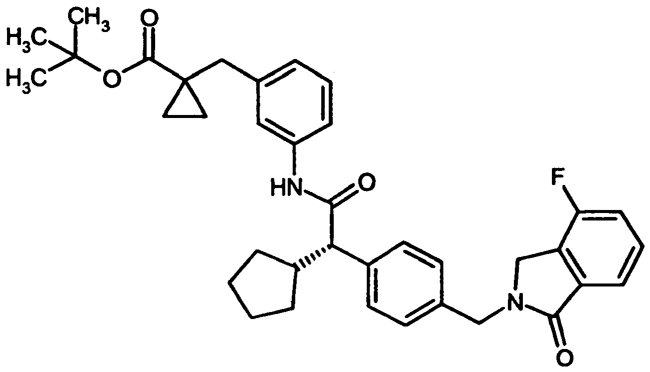

LC-MS (Methode 12): Rₜ = 2.84 min; m/z = 541 (M-C₄H₈)⁺.
¹H-NMR (400 MHz, CDCl₃): δ = 7.68 (d, 1 H), 7.50-7.42 (m, 1 H), 7.38 (d, 2 H), 7.32 (br. s, 2 H), 7.27 (br. s, 2 H), 7.17 (q, 2 H), 7.09 (s, 1 H), 6.96 (d, 1 H), 4.77 (s, 2 H), 4.31 (s, 2 H), 3.11 (d, 1 H), 2.87 (s, 2 H), 2.66 (d, 1 H), 2.02 (dd, 1 H), 1.69-1.61 (m, 2 H), 1.48 (td, 2 H), 1.31 (s, 9 H), 1.21-1.16 (m, 2 H), 1.03 (d, 1 H), 0.76-0.68 (m, 2 H).
[α]_{D}²⁰ = +33°, c = 0.205, Chloroform.

### Beispiel 421A

### (-)-1-{3-[(Cyclopentyl{4-[(4-fluor-1-oxo-1,3-dihydro-2H-isoindol1-2-yl)methyl]phenyl}acetyl)-amino]benzyl}cyclopropancarbonsäure-tert.-butylester (Enantiomer 2)

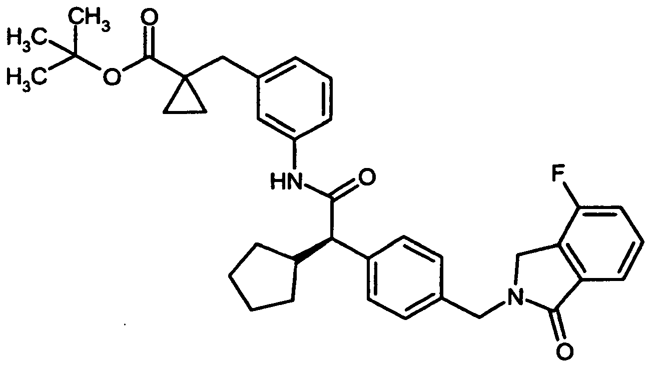

LC-MS (Methode 12): Rₜ = 2.84 min; m/z = 541 (M-C₄H₈)⁺.
¹H-NMR (400 MHz, CDCl₃): δ = 7.68 (d, 1 H), 7.45 (td, 1 H), 7.38 (d, 2 H), 7.32 (br. s, 2 H), 7.27 (br. s, 2 H), 7.17 (q, 2 H), 7.08 (s, 1 H), 6.96 (d, 1 H), 4.77 (s, 2 H), 4.31 (s, 2 H), 3.11 (d, 1 H), 2.87 (s, 2 H), 2.72-2.59 (m, 1 H), 2.07-1.94 (m, 1H), 1.71-1.57 (m, 3 H), 1.48 (td, 2 H), 1.32 (s, 9 H), 1.21-1.16 (m, 2 H), 1.07-0.82 (m, 1 H), 0.74-0.68 (m, 1 H).
[α]_{D}²⁰ = -37°, c = 0.270, Chloroform.

### Beispiel 422A

### 1-(3-{[4,4,4-Trifluor-2-{4-[(4-fluor-1-oxo-1,3-dihydro-2H-isoindol-2-yl)methyl]phenyl}-3-methyl-butanoyl]amino}benzyl)cyclopropancarbonsäure-tert.-butylester

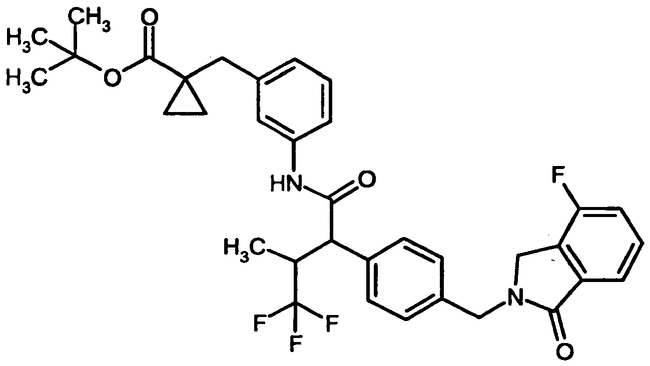

Zu einer Lösung von 129.0 mg (0.33 mmol) 4,4,4-Trifluor-2-{4-[(4-fluor-1-oxo-1,3-dihydro-2*H-*isoindol-2-yl)methyl]phenyl}-3-methylbutansäure und 52.9 mg (0.39 mmol) 1-Hydroxy-1*H*-benzotriazol-Hydrat in 1 ml DMF wurden 110.8 mg (0.41 mmol) 1-(3-Aminobenzyl)cyclopropancarbonsäure-*tert.*-butylester gegeben. Die resultierende Mischung wurde bei 0°C mit 142 µl (0.82 mmol) DIEA und dann portionsweise mit 136.5 mg (0.36 mmol) HATU versetzt. Das Reaktionsgemisch wurde danach langsam auf RT erwärmt und 2.5 h bei RT gerührt. Die Mischung wurde auf Wasser gegeben und dreimal mit Ethylacetat extrahiert. Die vereinigten organischen Phasen wurden über Magnesiumsulfat getrocknet und im Vakuum eingeengt. Das Rohprodukt wurde durch präparative RP-HPLC gereinigt (Eluent: Acetonitril/Wasser-Gradient). Es wurden 97.4 mg (47.6% d. Th.) der Titelverbindung erhalten.
LC-MS (Methode 15): Rₜ = 1.39 min; m/z = 623 (M-H)⁻.

Das oben erhaltene Isomerengemisch (größtenteils Racemat eines Hauptdiastereomers) wurde durch präparative HPLC an chiraler Phase in die Enantiomere aufgetrennt [Säule: Daicel Chiralpak AD-H, 5 µm, 250 mm x 20 mm; Fluss: 15 ml/min; UV-Detektion: 220 nm; Injektionsvolumen: 0.5 ml; Temperatur: 30°C; Eluent: 75% Isohexan / 25% Isopropanol]. Ausgehend von 94 mg Isomerengemisch wurden 31 mg von Enantiomer 1 und 32 mg von Enantiomer 2 isoliert (*siehe Beispiele 423A und 424A*)*.*

### Beispiel 423A

### (+)-1-(3-{[(2S,3R)-4,4,4-Trifluor-2-{4-[(4-fluor-l-oxo-1,3-dihydro-2H-isoindol-2-yl)methyl]-phenyl}-3-methylbutanoyl]amino}benzyl)cyclopropancarbonsäure-tert.-butylester (Enantiomer 1)

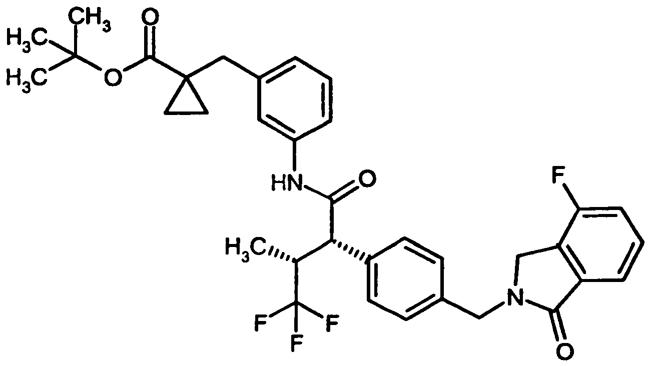

LC-MS (Methode 15): Rₜ = 1.38 min; m/z = 623 (M-H)⁻.
¹H-NMR (400 MHz, CDCl₃): δ = 7.69 (d, 1 H), 7.46 (td, 1 H), 7.39-7.33 (m, 2 H), 7.30 (d, 4 H), 7.23-7.11 (m, 3 H), 6.98 (d, 1 H), 4.79 (s, 2 H), 4.33 (s, 2 H), 3.57 (d, 1 H), 3.44-3.34 (m, 1 H), 2.87 (s, 2 H), 1.31 (s, 9 H), 1.22-1.16 (m, 2 H), 0.92-0.87 (m, 3 H), 0.71 (d, 2 H).
[α]_{D}²⁰ = +34°, c = 0.230, Chloroform.

### Beispiel 424A

### (-)-1-(3-{[(2R,3S)-4,4,4-Trifluor-2-{4-[(4-fluor-1-oxo-1,3-dihydro-2H-isoindol-2-yl)methyl]-phenyl}-3-methylbutanoyl]amino} benzyl)cyclopropancarbonsäure-tert.-butylester (Enantiomer 2)

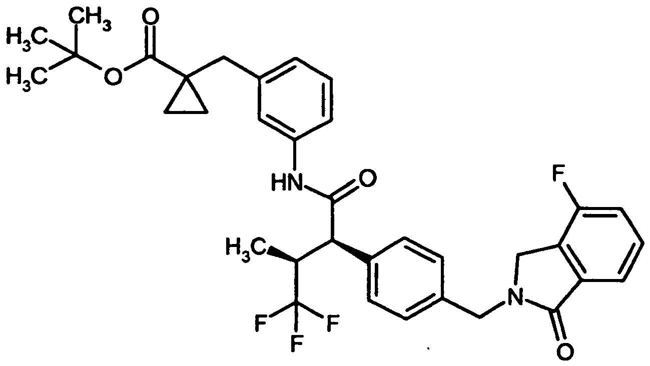

LC-MS (Methode 15): Rₜ = 1.38 min; m/z = 623 (M-H)⁻.
¹H-NMR (400 MHz, CDCl₃): δ = 7.69 (d, 1 H), 7.46 (td, 1 H), 7.40-7.34 (m, 2 H), 7.30 (d, 4 H), 7.23-7.08 (m, 3 H), 6.98 (d, 1 H), 4.79 (s, 2 H), 4.33 (s, 2 H), 3.57 (d, 1 H), 3.45-3.33 (m, 1 H), 2.87 (s, 2 H), 1.31 (s, 9 H), 1.19 (d, 2 H), 0.89 (d, 3 H), 0.71 (d, 2 H).
[α]_{D}²⁰ = -85°, c = 0.235, Chloroform.

### Beispiel 425A

### (+)-3-(3-{[(2S)-2-Cyclopentyl-2-{4-[(5-oxo-2-phenyl-5,6-dihydro-4H-1,3,4-oxadiazin-4-yl)-methyl]phenyl}acetyl]amino}-4-fluorphenyl)propansäure-tert.-butylester

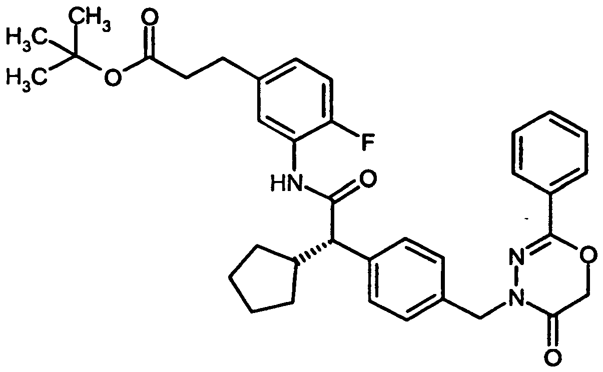

Zu einer Lösung von 150.0 mg (0.38 mmol) (2S)-Cyclopentyl{4-[(5-oxo-2-phenyl-5,6-dihydro-4*H-*1,3,4-oxadiazin-4-yl)methyl]phenyl}ethansäure und 70.2 mg (0.46 mmol) 1-Hydroxy-1*H-*benzotriazol-Hydrat in 1 ml DMF wurden 109.75 mg (0.46 mmol) 3-(3-Amino-4-fluorphenyl)propansäure-*tert.*-butylester gegeben. Die resultierende Mischung wurde bei 0°C mit 200 µl (1.15 mmol) DIEA und dann portionsweise mit 174.4 mg (0.46 mmol) HATU versetzt. Das Reaktionsgemisch wurde danach langsam auf RT erwärmt und anschließend 4 h bei 60°C gerührt. Die Mischung wurde auf Wasser gegeben und dreimal mit Ethylacetat extrahiert. Die vereinigten organischen Phasen wurden über Magnesiumsulfat getrocknet und im Vakuum eingeengt. Das Rohprodukt wurde durch präparative RP-HPLC gereinigt (Eluent: Acetonitril/Wasser-Gradient). Es wurden 104 mg (44.3% d. Th.) der Titelverbindung erhalten.
LC-MS (Methode 11): Rₜ = 1.68 min; m/z = 612 (M-H)⁻.
¹H-NMR (400 MHz, DMSO-*d*₆): δ = 9.77 (s, 1 H), 7.76 (d, 2 H), 7.65 (dd, 1 H), 7.52-7.43 (m, 3 H), 7.40 (d, 2 H), 7.31 (d, 2 H), 7.09 (dd, 1 H), 6.98-6.90 (m, 1 H), 4.94 (d, 1 H), 4.90 (s, 2 H), 4.85 (s, 2 H), 3.60 (d, 1 H), 2.72 (t, 2 H), 2.43 (t, 2 H), 1.82-1.31 (m, 7 H), 1.29 (s, 9 H), 1.01-0.92 (m, 1H).
[α]_{D}²⁰ = +61°, c = 0.480, Chloroform.

### Beispiel 426A

### 3-{3-[(4,4,4-Trifluor-2-{4-[(4-fluor-1-oxo-1,3-dihydro-2H-isoindol-2-yl)methyl]phenyl}-3-methyl-butanoyl)amino]phenyl}propansäure-tert.-butylester

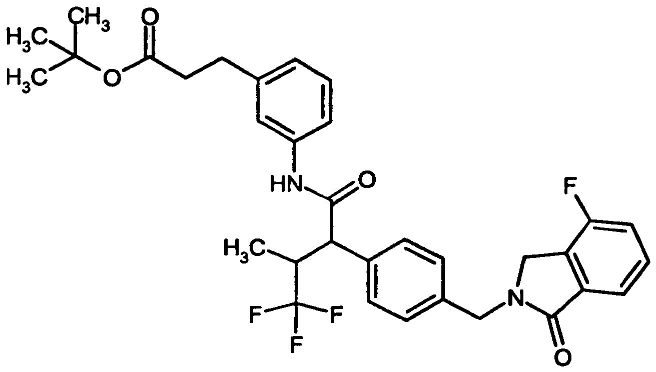

Zu einer Lösung von 280.0 mg (0.71 mmol) 4,4,4-Trifluor-2-(4-[(4-fluor-1-oxo-1,3-Mydro-2*H-*isoindol-2-yl)methyl]phenyl)-3-methylbutansäure und 114.8 mg (0.85 mmol) 1-Hydroxy-1*H-*benzotriazol-Hydrat in 1.4 ml DMF wurden 215.3 mg (0.89 mmol) 3-(3-Aminophenyl)propansäure-*tert.*-butylester gegeben. Die resultierende Mischung wurde bei 0°C mit 308 µl (1.77 mmol) DIEA und dann portionsweise mit 296.2 mg (0.78 mmol) HATU versetzt. Das Reaktionsgemisch wurde danach langsam auf RT erwärmt und anschließend 2.5 h bei RT gerührt. Die Mischung wurde auf Wasser gegeben und dreimal mit Ethylacetat extrahiert. Die vereinigten organischen Phasen wurden über Magnesiumsulfat getrocknet und im Vakuum eingeengt. Das Rohprodukt wurde durch präparative RP-HPLC gereinigt (Eluent: Acetonitril/Wasser-Gradient). Es wurden 333 mg (78.6% d. Th.) der Titelverbindung erhalten.
LC-MS (Methode 15): Rₜ = 1.33 min; m/z = 597 (M-H)⁻.
¹H-NMR (400 MHz, DMSO-*d*₆): δ = 10.15 (s, 1 H), 7.61-7.53 (m, 2 H), 7.46-7.37 (m, 4 H), 7.33 (d, 1 H), 7.29 (d, 2 H), 7.16 (t, 1 H), 6.87 (d, 1 H), 4.71 (s, 2 H), 4.47 (s, 2 H), 3.81 (d, 1 H), 2.73 (t, 2 H), 2.44 (t, 2 H), 1.40-1.36 (m, 1 H), 1.28 (s, 9 H), 0.78 (d, 3 H).

Das oben erhaltene Isomerengemisch (größtenteils Racemat eines Hauptdiastereomers) wurde durch präparative HPLC an chiraler Phase in die Enantiomere aufgetrennt [Säule: Daicel Chiralpak AD-H, 5 µm, 250 mm x 20 mm; Fluss: 15 ml/min; UV-Detektion: 220 nm; Injektionsvolumen: 0.25 ml; Temperatur: 40°C; Eluent: 70% Isohexan / 30% Ethanol]. Ausgehend von 333 mg Isomerengemisch wurden 138 mg von Enantiomer 1 und 134 mg von Enantiomer 2 isoliert (*siehe Beispiele 427A und 428A).*

### Beispiel 427A

### (+)-3-{3-[(4,4,4-Trifluor-2-(4-[(4-fluor-1 -oxo-1,3-dihydro-2H-isoindol-2-yl)methyl]phenyl)-3-methylbutanoyl)amino]phenyl}propansäure-tert.-butylester (Enantiomer 1)

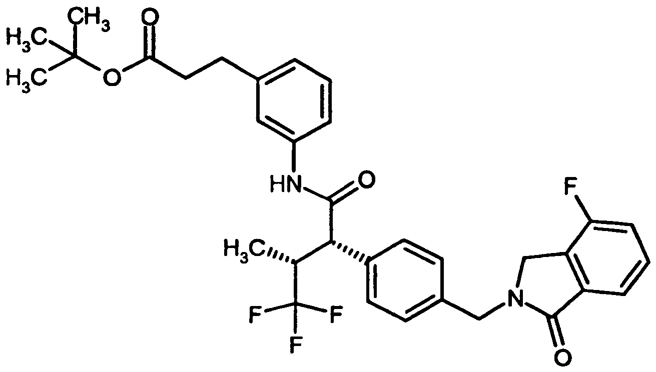

LC-MS (Methode 15): Rₜ = 1.34 min; m/z = 597 (M-H)⁻.
¹H-NMR (400 MHz, DMSO-*d*₆): δ = 10.15 (s, 1 H), 7.61-7.53 (m, 2 H), 7.46-7.37 (m, 4 H), 7.33 (d, 1 H), 7.29 (d, 2 H), 7.16 (t, 1 H), 6.87 (d, 1 H), 4.71 (s, 2 H), 4.47 (s, 2 H), 3.81 (d, 1 H), 3.40-3.30 (m, 1 H), 2.73 (t, 2 H), 2.44 (t, 2 H), 1.40-1.36 (m, 1 H), 1.28 (s, 9 H), 0.78 (d, 3 H).
[α]_{D}²⁰ = +68.4°, c = 0.605, Chloroform.

### Beispiel 428A

### (-)-3-{3-[(4,4,4-Trifluor-2-{4-[(4-fluor-1-oxo-1,3-dihydro-2H-isoindol-2-yl)methyl]phenyl}-3-methylbutanoyl)amino]phenyl}propansäure-tert.-butylester (Enantiomer 2)

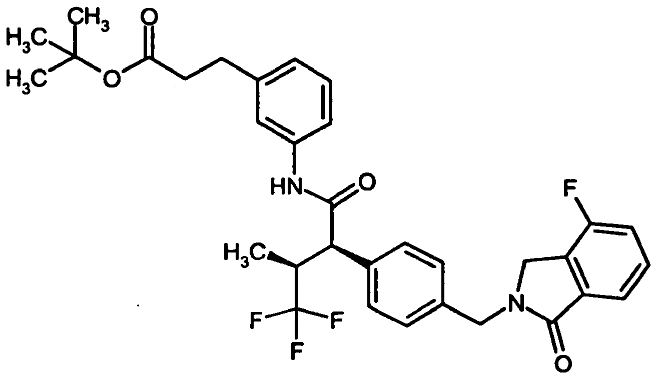

LC-MS (Methode 15): Rₜ = 1.34 min; m/z = 597 (M-H)⁻.
¹H-NMR (400 MHz, DMSO-*d*₆): δ = 10.15 (s, 1 H), 7.61-7.53 (m, 2 H), 7.46-7.37 (m, 4 H), 7.33 (d, 1 H), 7.29 (d, 2 H), 7.16 (t, 1 H), 6.87 (d, 1 H), 4.71 (s, 2 H), 4.47 (s, 2 H), 3.81 (d, 1 H), 3.40-3.30 (m, 1 H), 2.73 (t, 2 H), 2.44 (t, 2 H), 1.40-1.36 (m, 1 H), 1.28 (s, 9 H), 0.78 (d, 3 H).
[α]_{D}²⁰ = -76.8°, c = 0.485, Chloroform.

### Beispiel 429A

### (+)-3-(3-{[(2S)-2-Cyclopentyl-2-{4-[(5-oxo-2-phenyl-5,6-dihydro-4H-1,3,4-oxadiazin-4-yl)-methyl]phenyl} acetyl]amino}-2-fluorphenyl)propansäure-tert.-butylester

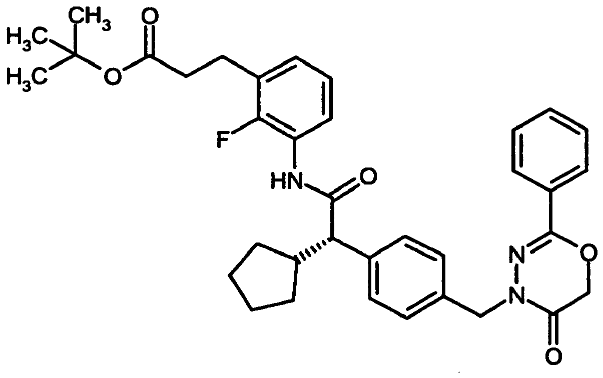

Zu einer Lösung von 155.8 mg (0.40 mmol) (2S)-Cyclopentyl{4-[(5-oxo-2-phenyl-5,6-dihydro-4*H-*1,3,4-oxadiazin-4-yl)methyl]phenyl}ethansäure in 1 ml DMF wurden 114.0 mg (0.77 mmol) 3-(3-Amino-2-fluorphenyl)propansäure-tert.-butylester und 193 µl (0.99 mmol) DIEA gegeben. Die resultierende Mischung wurde bei 0°C portionsweise mit 181.1 mg (0.47 mmol) HATU versetzt. Das Reaktionsgemisch wurde 30 min bei 0°C gerührt, dann auf RT erwärmt und anschließend 7 h bei 60°C gerührt. Der Ansatz wurde danach auf gesättigte Natriumcarbonat-Lösung gegeben und dreimal mit Ethylacetat extrahiert. Die vereinigten organischen Phasen wurden über Magnesiumsulfat getrocknet und im Vakuum eingeengt. Das Reaktionsprodukt wurde durch präparative RP-HPLC gereinigt (Eluent: Acetonitril/Wasser-Gradient). Es wurden 125.6 mg (51.6% d. Th.) der Titelverbindung erhalten.
LC-MS (Methode 7): Rₜ = 3.19 min; m/z = 612 (M-H)⁻.
¹H-NMR (400 MHz, DMSO-d₆): δ = 9.78 (s, 1 H), 7.78 (d, 1 H), 7.62 (m, 1 H), 7.52-7.49 (m, 5 H), 7.30 (d, 2 H), 7.02-6.98 (m, 2 H), 4.91 (s, 2 H), 4.85 (s, 2H), 3.61 (d, 1 H), 2.81 (t, 2 H), 1.82-1.75 (m, 1 H), 1.70-1.25 (m, 5 H), 1.35 (s, 9 H), 1.01-0.91 (m, 1 H).
[α]_{D}²⁰ = +6.5°, c = 0.34, Chloroform.

### Allgemeine Vorschrift 7: Kupplung von Anilinen mit aliphatischen Carbonsäuren

Methode 7A: Eine 0.35 M Lösung der betreffenden Carbonsäure in DMF wurde mit 1.3 eq. HATU und 3 eq. *N,N*-Diisopropylethylamin versetzt und 30 min bei RT gerührt. Anschließend wurden 1.1 eq. des betreffenden Anilins zugegeben. Das Reaktionsgemisch wurde über Nacht bei RT gerührt und dann direkt über präparative RP-HPLC aufgereinigt.

Methode 7B: Eine 0.15 M Lösung der betreffenden Carbonsäure in DMF/Pyridin (3:1 v/v) wurde mit 1.3 eq. TCTU versetzt und 30 min bei RT gerührt. Anschließend wurde das betreffende Anilin zugegeben und das Reaktionsgemisch über Nacht bei RT gerührt. Der Ansatz wurde dann unter vermindertem Druck eingeengt und der Rückstand über präparative RP-HPLC gereinigt.

Nach der Allgemeinen Vorschrift 7A bzw. 7B wurden die folgenden Verbindungen hergestellt:

| **Beispiel** | **Struktur** | **Methode** | **Analytische Daten** |
|---|---|---|---|
| **430A** | 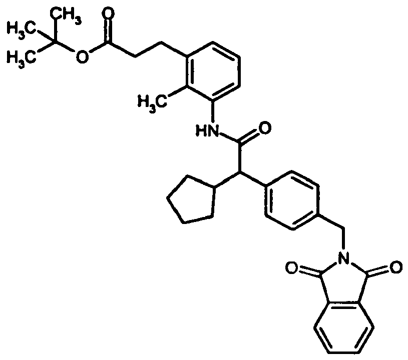 | 7B | LC-MS (Methode 7): Rₜ = 3.08 min; m/z = 599 [M+H]⁺. ¹H-NMR (400 MHz, DMSO-d₆): δ = 9.46 (s, 1H), 7.79-7.97 (m, 4H), 7.38 (d, 2H), 7.26 (d, 2H), 6.90-7.06 (m, 3H), 4.75 (s, 2H), 2.78 (t, 2H), 2.38-2.46 (m, 2H), 1.98 (s, 3H), 1.77-1.87 (m, 1H), 1.18-1.73 (m, 17H), 0.95 (dd, 1H). |
| **431A** | 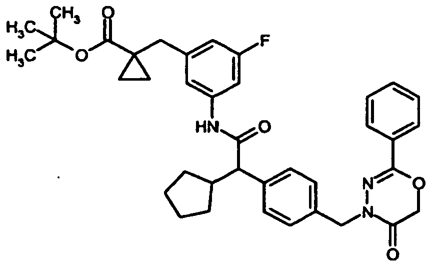 | 7A | LC-MS (Methode 15): Rₜ = 1.50 min; m/z = 657 [M+NH₄]⁺. ¹H-NMR (400 MHz, DMSO-d₆): δ = 10.16 (s, 1H), 7.68-7.85 (m, 2H), 7.41-7.55 (m, 3H), 7.28-7.40 (m, 5H), 7.21 (s, 1H), 6.70 (d, 1H), 4.78-4.96 (m, 4H), 3.34-3.42 (m, 2H), 2.74-2.79 (m, 2H), 1.77 (m, 1H), 1.15-1.68 (m, 15H), 1.04-1.13 (m, 2H), 0.88-1.03 (m, 1H), 0.77-0.87 (m, 2H). |
| **432A** | 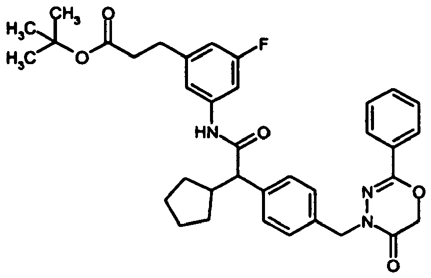 | 7A | LC-MS (Methode 15): Rₜ = 1.46 min; m/z = 631 [M+NH₄]⁺. ¹H-NMR (400 MHz, DMSO-d₆): δ = 10.18 (s, 1H), 7.69-7.84 (m, 2H), 7.41-7.54 (m, 3H), 7.28-7.40 (m, 5H), 7.15 (s, 1 H), 6.72 (d, 1 H), 4.80-4.96 (m, 4H), 3.36 (d, 1H), 2.73 (t, 2H), 2.43-2.48 (m, 2H), 1.71-1.84 (m, 1H), 1.13-1.69 (m, 16H), 0.90-1.02 (m, 1H). |
| **433A** | 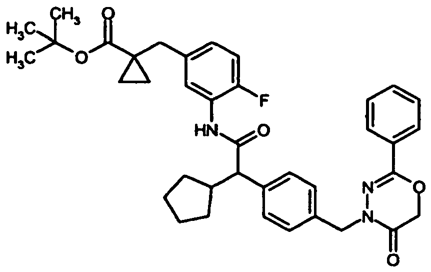 | 7A | LC-MS (Methode 11): Rₜ = 1.50 min; m/z = 584 [M-C₄H₉]⁺. ¹H-NMR (400 MHz, DMSO-d₆): δ = 9.75 (s, 1H), 7.74-7.82 (m, 2H), 7.68 (dd, 1H), 7.35-7.53 (m, 5H), 7.25-7.34 (m, 2H), 7.09 (dd, 1H), 6.89-7.01 (m, 1H), 4.80-4.95 (m, 4H), 3.58 (d, 1H), 2.74 (d, 2H), 1.78 (dd, 1H), 1.16-1.71 (m, 16H), 0.99-1.05 (m, 2H), 0.97 (d, 1H), 0.71-0.82 (m, 2H). |
| **434A** | 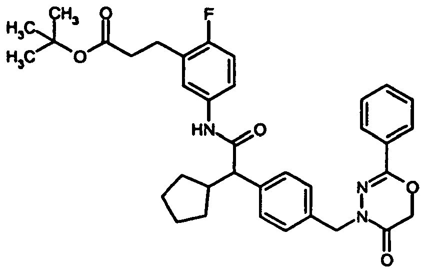 | 7A | LC-MS (Methode 11): Rₜ = 1.68 min; m/z = 632 [M+NH₄]⁺. ¹H-NMR (400 MHz, DMSO-d₆): δ = 10.02 (s, 1H), 7.76 (d, 2H), 7.41-7.60 (m, 4H), 7.29-7.41 (m, 5H), 7.02 (t, 1H), 4.80-4.92 (m, 4H), 2.70-2.80 (m, 2H), 2.40-2.47 (m, 2H), 1.71-1.81 (m, 1H), 1.14-1.70 (m, 16H), 0.86-1.04 (m, 1H). |
| **435A** | 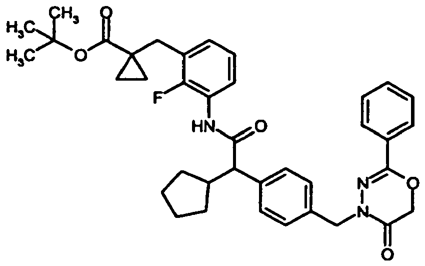 | 7A | LC-MS (Methode 12): Rₜ = 3.13 min; m/z = 657 [M+NH₄]⁺. ¹H-NMR (400 MHz, DMSO-d₆): δ = 9.76 (s, 1H), 7.72-7.86 (m, 2H), 7.53-7.66 (m, 1H), 7.38-7.53 (m, 5H), 7.31 (d, 2H), 6.95-7.14 (m, 2H), 4.71-4.99 (m, 4H), 3.54-3.65 (m, 1H), 2.87 (br. s, 2H), 1.74-1.83 (m, 1H), 1.20-1.71 (m, 15H), 1.06-1.10 (m, 2H), 0.91-1.02 (m, 1M), 0.77-0.81 (m, 2H). |
| **436A** | 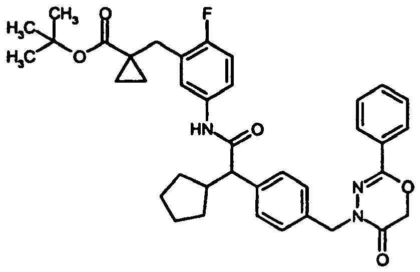 | 7A | LC-MS (Methode 12): Rₜ = 3.13 min; m/z = 657 [M+NH₄]⁺. ¹H-NMR (400 MHz, DMSO-d₆): δ = 10.01 (s, 1H), 7.73-7.79 (m, 2H), 7.58-7.64 (m, 1H), 7.34-7.53 (m, 7H), 7.28-7.33 (m, 2H), 6.97-7.04 (m, 1H), 4.81-4.94 (m, 4H), 2.78-2.84 (m, 2H), 1.70-1.84 (m, 1H), 1.17-1.68 (m, 15H), 1.05-1.12 (m, 2H), 0.90-1.03 (m, 1H), 0.73-0.84 (m, 2H). |

### Ausführungsbeispiele:

### Beispiele 1

### 4-({[(4-{[4-(4-Chlorphenyl)-1-oxophthalazin-2(1H)-yl]methyl}phenyl)(cyclopentyl)acetyl]-amino}methyl)benzolcarbonsäure (Racemat)

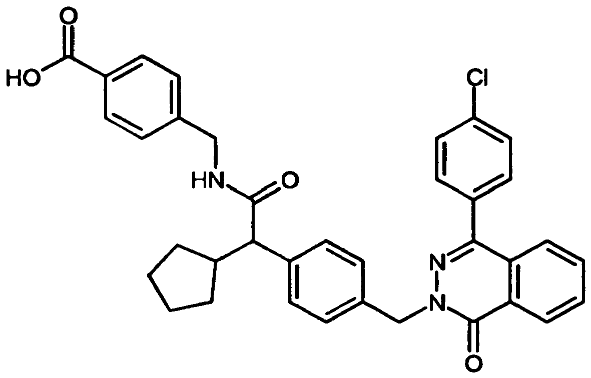

### Darstellungsmethode 1

270 mg (0.435 mmol) Methyl-4-({[(4-{[4-(4-chlorphenyl)-1-oxophthalazin-2(1*H*)-yl]methyl}-phenyl)(cyclopentyl)acetyl]amino}methyl)benzolcarboxylat (Beispiel 112A) wurden in 10 ml Dioxan/Wasser (3:1 v/v) gelöst und mit 0.652 ml (0.652 mmol) 1 N Natronlauge versetzt. Es wurde über Nacht bei Raumtemperatur gerührt. Das Reaktionsgemisch wurde dann mit 1 N Salzsäure angesäuert und mehrmals mit Essigsäureethylester extrahiert. Die vereinigten organischen Phasen wurden über Natriumsulfat getrocknet und im Vakuum eingeengt. Es wurden 269 mg der Zielverbindung erhalten.
LC-MS (Methode 7): Rₜ = 3.03 min; m/z = 606 (M+H)⁺.

### Beispiel 2

### 2-Methyl-4-[(3-methyl-2-(4-[(5-oxo-2-phenyl-5,6-dihydro-4H-1,3,4-oxadiazin-4-yl)methyl]-phenyl)butanoyl)amino]-2,3-dihydro-1H-inden-2-carbonsäure (Diastereomerengemisch)

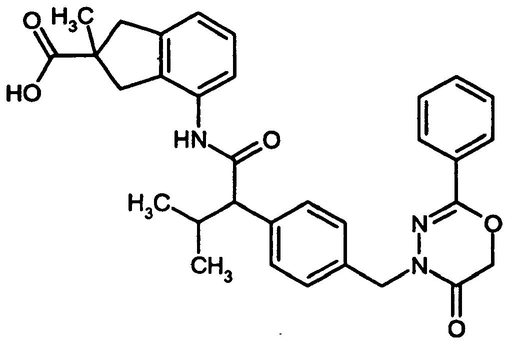

### Darstellungsmethode 2

Eine Lösung von 60 mg (0.11 mmol) Methyl-2-methyl-4-[(3-methyl-2-{4-[(5-oxo-2-phenyl-5,6-dihydro-4*H*-1,3,4-oxadiazin-4-yl)methyl]phenyl}butanoyl)amino]-2,3-dihydro-1*H*-inden-2-carboxylat (Beispiel 136A) in 2 ml THF, und 2 ml Wasser wurde mit 9.1 mg (0.22 mmol) Lithiumhydroxid-Monohydrat versetzt und 8 h bei 60°C gerührt. Der Ansatz wurde dann mit 1 M Salzsäure auf pH 4 gestellt und im Vakuum bis zur Trockene eingeengt. Das erhaltene Rohprodukt wurde durch Flash-Chromatographie an Kieselgel gereinigt (Laufmittel Cyclohexan/Essigsäureethylester 10:1 → 4:1 → 1:1 oder Dichlormethan/Methanol 10:1). Es wurden 43 mg (0.08 mmol, 74% d. Th.) der Titelverbindung erhalten.
¹H-NMR (400 MHz, DMSO-d₆, δ/ppm): 12.45-12.24 (1H, breites s), 9.46 (1H, d), 7.76 (2H, d), 7.52-7.26 (8H, m), 7.04 (1H, t), 6.94 (1H, d), 4.91 (2H, s), 4.86 (2H, s), 3.43-3.18 (3H, m), 2.79-2.57 (2H, m), 2.38-2.21 (1H, m), 1.23 (3H, s), 1.02 (3H, d), 0.65 (3H, d).
LC-MS (Methode 11): Rₜ = 1.34 min; m/z = 540 (M+H)⁺.

### Beispiel 3

### 3-{3-[(4-Methoxy-3-methyl-2-{4-[(5-oxo-2-phenyl-5,6-dihydro-4H-1,3,4-oxadinin-4-yl)methyl]-phenyl}butanoyl)amino]-2-methylphenyl}propansäure (Diastereomerengemisch)

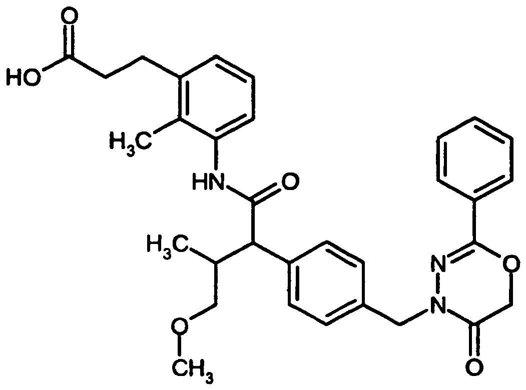

### Darstellungsmethode 3

Zu einer Lösung von 106 mg (0.17 mmol) *tert*.-Butyl-3-{3-[(4-methoxy-3-methyl-2-{4-[(5-oxo-2-phenyl-5,6-dihydro-4*H*-1,3,4-oxadiazin-4-yl)methyl]phenyl}butanoyl)amino]-2-methylphenyl}propanoat (Beispiel 170A) in 2 ml Dichlormethan wurden 0.27 ml (3.45 mmol) Trifluoressigsäure getropft und die Mischung 3 h bei Raumtemperatur gerührt. Anschließend wurde die Reaktionslösung im Vakuum bis zur Trockene eingeengt. Der erhaltene Rückstand wurde über präparative HPLC gereinigt. Es wurden 66 mg (0.12 mmol, 68% d. Th.) der Titelverbindung als Diastereomerengemisch erhalten.
LC-MS (Methode 11): Diastereomer 1: Rₜ = 1.23 min (28%), m/z = 558 (M+H)⁺; Diastereomer 2: Rₜ = 1.24 min (72%); m/z = 558 (M+H)⁺.

Auf analoge Weise wurden die in der folgenden Tabelle aufgeführten Verbindungen erhalten:

| **Beispiel** | **Name / Struktur Darstellungsmethode / Edukt** | **Analytische Daten** |
|---|---|---|
| **4** | 4-{[(Cyclopentyl{4-[(2-oxo-5-phenyl-1,3,4-oxadiazol-3(2*H*)-yl)methyl]phenyl}acetyl)amino]methyl}-benzolcarbonsäure (*Racemat*) | LC-MS (Methode 2): Rₜ = 2.38 min; m/z = 512 (M+H)⁺. |
| | 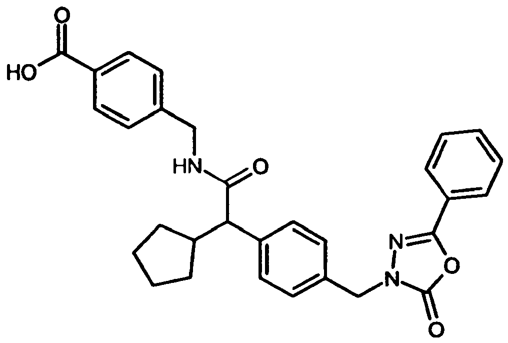 | |
| | Darstellungsmethode 1 (aus Bsp. 114A) | |
| **5** | 4-{[(Cyclopentyl{4-[(3,5-dioxo-6-phenyl-2,5-dihydro-1,2,4-triazin-4(3*H*)-yl)methyl]phenyl}acetyl)amino]-methyl}benzolcarbonsäure (*Racemat*) | LC-MS (Methode 2): Rₜ = 2.22 min; m/z = 539 (M+H)⁺. |
| | 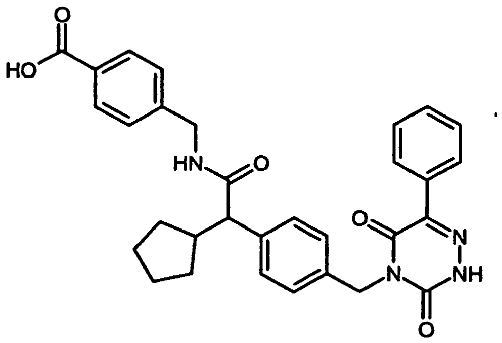 | |
| | Darstellungsmethode 1 (aus Bsp. 117A) | |
| **6** | 4-({[Cyclopentyl(4-{[(4*R*,5*S*)-4-methyl-2-oxo-5-phenyl-1,3-oxazolidin-3-yl]methyl}phenyl)acetyl]amino}-methyl)benzolcarbonsäure (*Racemat*) | LC-MS (Methode 7): Rₜ = 2.55 min; m/z = 527 (M+H)⁺. |
| | 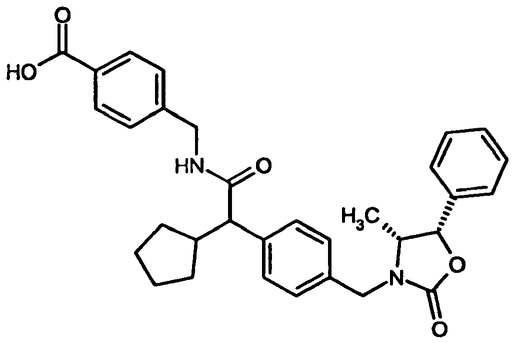 | |
| | Darstellungsmethode 1 (aus Bsp. 115A) | |
| **7** | 4-({[Cyclopentyl(4-{[(4*S*,5*R*)-4-methyl-2-oxo-5-phenyl-1,3-oxazolidin-3-yl]methyl}phenyl)acetyl]amino}-methyl)benzolcarbonsäure (*Racemat*) | LC-MS (Methode 2): Rₜ = 2.31 min; m/z = 527 (M+H)⁺. |
| | 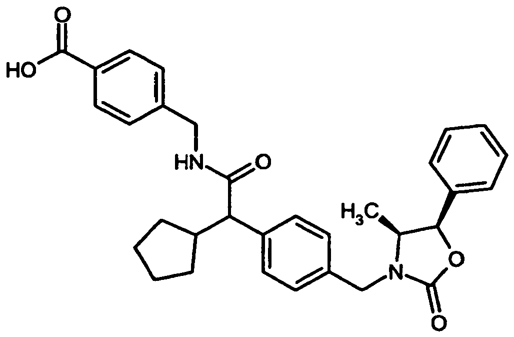 | |
| | Darstellungsmethode 1 (aus Bsp. 116A) | |
| **8** | 4-{[(Cyclopentyl{4-[(5-methyl-2-oxo-5-phenyl-piperidin-1-yl)methyl]phenyl}acetyl)amino]-methyl}benzolcarbonsäure (*Diastereomerengemisch*) | LC-MS (Methode 7): Rₜ = 2.50 min; m/z = 539 (M+H)⁺ |
| | 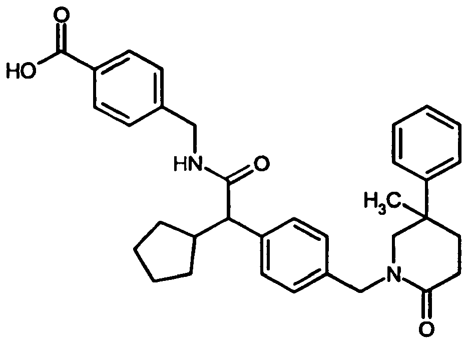 | |
| | Darstellungsmethode 1 (aus Bsp. 118A) | |
| **9** | 4-{[(Cyclopentyl{4-[(5-oxo-2-phenyl-5,6-dihydro-4*H*-1,3,4-oxadiazin-4-yl)methyl]phenyl}acetyl)amino]-methyl}phenyl)essigsäure (*Racemat*) | LC-MS (Methode 7): Rₜ = 2.53 min; m/z = 540 (M+H)⁺. |
| | 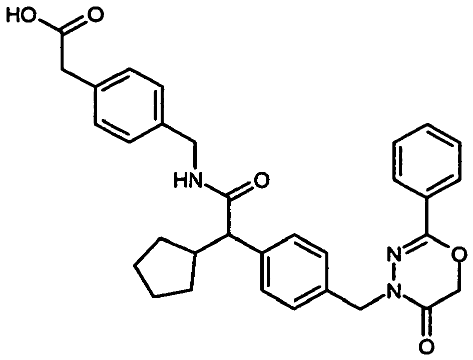 | |
| | Darstellungsmethode 1 (aus Bsp. 119A) | |
| **10** | 4-({[(4-{[2-(3-Chlorphenyl)-5-oxo-5,6-dihydro-4*H*-1,3,4-oxadiazin-4-yl]methyl}phenyl)(cyclopentyl)-acetyl]amino}methyl)benzolcarbonsäure (*Racemat*) | LC-MS (Methode 2): Rₜ = 2.57 min; m/z = 560 (M+H)⁺. |
| | 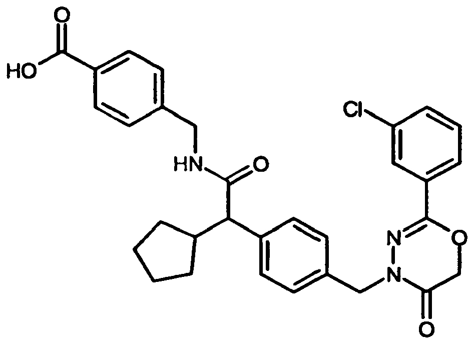 | |
| | Darstellungsmethode 1 (aus Bsp. 120A) | |
| **11** | 6-({[(4-{[4-(4-Chlorphenyl)-1-oxophthalazin-2(1*H*)-yl]-methyl}phenyl)(cyclopentyl)acetyl]amino}methyl)-pyridin-3-carbonsäure (*Racemat*) | LC-MS (Methode 2): Rₜ = 2.60 min; m/z = 607 (M+H)⁺. |
| | 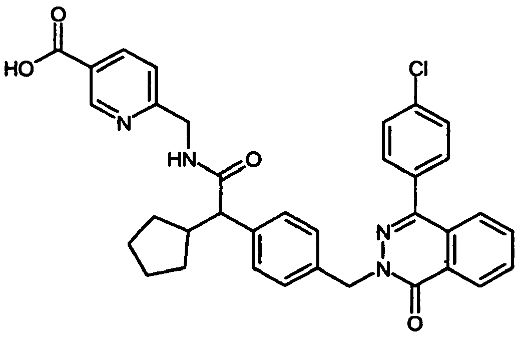 | |
| | Darstellungsmethode 1 (aus Bsp. 121A) | |
| **12** | 4-({[(4-{[2-(2-Chlorphenyl)-5-oxo-5,6-dihydro-4*H*-1,3,4-oxadiazin-4-yl]methyl}phenyl)(cyclopentyl)-acetyl]amino}methyl)benzolcarbonsäure (*Racemat*) | LC-MS (Methode 2): Rₜ = 2.42 min; m/z = 560 (M+H)⁺. |
| | 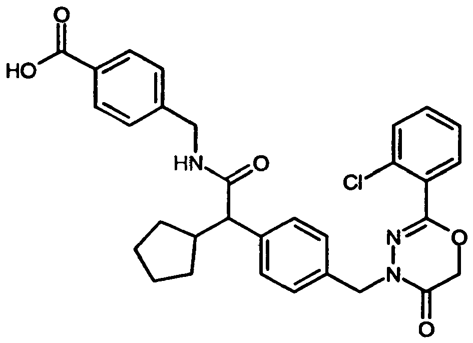 | |
| | Darstellungsmethode 1 (aus Bsp. 122A) | |
| **13** | 5-({[(4-{[4-(4-Chlorphenyl)-1-oxophthalazin-2(1*H*)-yl]-methyl}phenyl)(cyclopentyl)acetyl]amino}methyl)-furan-2-carbonsäure (*Racemat*) | LC-MS (Methode 9): Rₜ = 4.09 min; m/z = 596 (M+H)⁺. |
| | 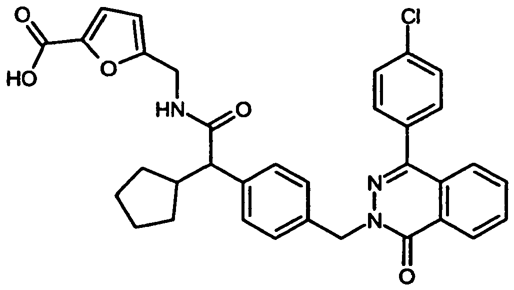 | |
| | Darstellungsmethode 1 (aus Bsp. 123A) | |
| **14** | 4-({[(4-{[2-(4-Chlorphenyl)-5-oxo-5,6-dihydro-4H-1,3,4-oxadiazin-4-yl]methyl}phenyl)(cyclopentyl)-acetyl]amino}methyl)benzolcarbonsäure *(Racemat)* | LC-MS (Methode 7): Rₜ= 2.90 min; m/z = 560 (M+H)⁺. |
| | 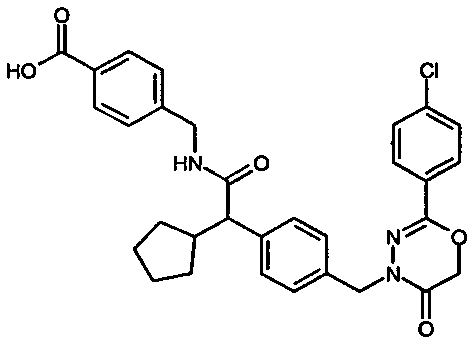 | |
| | Darstellungsmethode 1 (aus Bsp. 124A) | |
| **15** | 4-({[Cyclopentyl(4-{[2-(2-methoxyphenyl)-5-oxo-5,6-dihydro-4H-1,3,4-oxadiazin-4-yl]methyl}phenyl)-acetyl]amino}methyl)benzolcarbonsäure *(Racemat)* | LC-MS (Methode 8): Rₜ = 3.57 min; m/z = 566 (M+H)⁺. |
| | 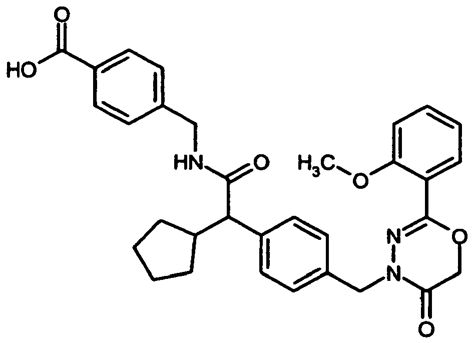 | |
| | Darstellungsmethode 1 (aus Bsp. 125A) | |
| **16** | 4-{[Cyclopentyl(4-{[2-(2-methylpropyl)-5-oxo-5,6-dihydro-4*H*-1,3,4-oxadiazin-4-yl]methyl}phenyl)-acetyl]amino}-2,3-dihydro-1*H*-inden-2-carbonsäure (*Diastereomerengemisch*) | LC-MS (Methode 11): Rₜ = 1.38 min; m/z = 532 (M+H)⁺. |
| | 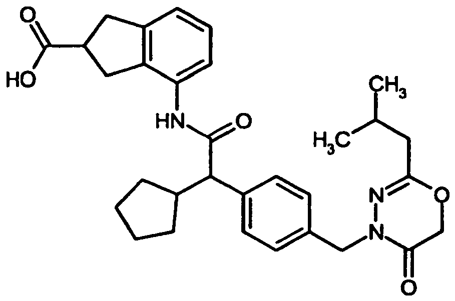 | |
| | Darstellungsmethode 1 (aus Bsp. 185A) | |
| **17** | 4-{[(4-{[2-(4-Chlorphenyl)-5-oxo-5,6-dihydro-4*H*-1,3,4-oxadiazin-4-yl]methyl}phenyl)(cyclopentyl)acetyl]-amino}-2,3-dihydro-1*H*-inden-2-carbonsäure (*Diastereomerengemisch*) | LC-MS (Methode 7): Rₜ = 3.01 min; m/z = 586 (M+H)⁺. |
| | 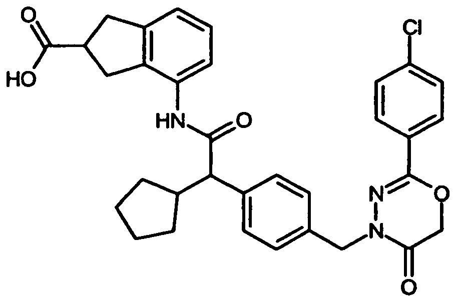 | |
| | Darstellungsmethode 2 (aus Bsp. 132A) | |
| **18** | 4-{[(4-{[2-(4-Fluorphenyl)-5-oxo-5,6-dihydro-4*H*-1,3,4-oxadiazin-4-yl]methyl}phenyl)(cyclopentyl)acetyl]-amino}-2,3-dihydro-1*H*-inden-2-carbonsäure (*Diastereomerengemisch*) | ¹H-NMR (400 MHz, DMSO-d₆, δ/ppm): 12.35-12.18 (1H, breites s), 9.47 (1H, d), 7.85-7.75 (2H, m), 7.41 (2H, d), 7.36-7.22 (5H, m), 7.04 (1H, t), 6.96 (1H, d), 4.91 (2H, s), 4.85 (2H, s), 3.52 (1H, d), 3.28-3.15 (1H, m), 3.14-2.97 (4H, m), 2.61-2.46 (1H, m), 1.87-1.74 (1H, m), 1.73-1.21 (5H, m), 1.04-0.90 (1H, m). LC-MS (Methode 7): Rₜ = 2.86 min; m/z = 570 (M+H)⁺. |
| | 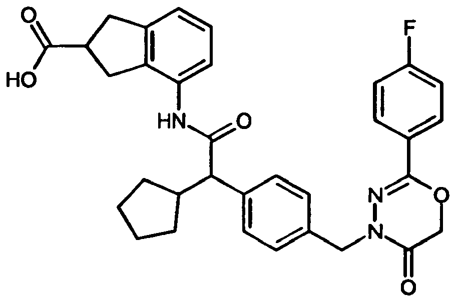 | |
| | Darstellungsmethode 2 (aus Bsp. 133A) | |
| **19** | 4-{[Cyclopentyl(4-{[4-methyl-5-(2-methylpropyl)-2-oxo-1,3-thiazol-3(2*H*)-yl]methyl}phenyl)acetyl]amino}-2,3-dihydro-1*H*-inden-2-carbonsäure (*Diastereomerengemisch*) | LC-MS (Methode 7): R, = 3.02 min; m/z = 547 (m+H)⁺_{.} |
| | 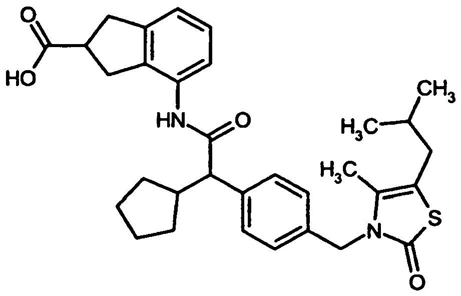 | |
| | Darstellungsmethode 1 (aus Bsp. 106A) | |
| **20** | 4-{[Cyclopentyl(4-{[5-oxo-2-(propan-2-yl)-5,6-dihydro-4*H*-1,3,4-oxadiazin-4-yl]methyl}phenyl)acetyl]amino}-2,3-dihydro-1*H*-inden-2-carbonsäure (*Diastereomerengemisch*) | LC-MS (Methode 10): Rₜ = 2.14 min; m/z = 518 (M+H)⁺. |
| | 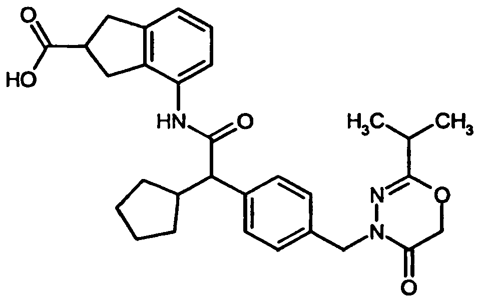 | |
| | Darstellungsmethode 1 (aus Bsp. 107A) | |
| **21** | 3-{3-[(Cyclopentyl{4-[(4-oxo-1,2,3-benzotriazin-3(4*H*)-yl)methyl]phenyl}acetyl)amino]phenyl}propansäure (*Racemat*) | ¹H-NMR (400 MHz, DMSO-d₆, δ/ppm): 12.2-11.8 (1H, breites s), 9.98 (1H, d), 8.30- |
| | 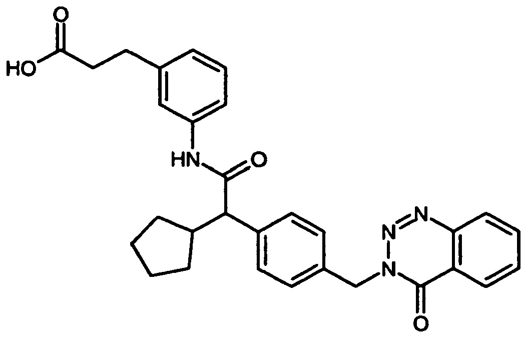 | 8.18 (2H, m), 8.09 (1H, t), 7.98-7.87 (1H, m), 7.43-7.28 (6H, m), 7.14 (1H, t), 6.86 (1H, d), 5.55 (2H, s), 3.38 (1H, d), 2.73 (2H, t), 2.61-2.46 (1H, m), 2.45 (2H, t), 1.83-1.70 (1H, m), 1.70-1.19 (6H, m), 1.01-0.88 (1H, m). LC-MS (Methode 11): |
| | Darstellungsmethode 3 (aus Bsp. 156A) | Rₜ = 1.28 min; m/z = 511 (M+H)⁺. |
| **22** | 3-{3-[(Cyclopentyl{4-[(2-oxo-5-phenyl-1,3,4-oxadiazol-3(2*H*)-yl)methyl]phenyl}acetyl)amino]phenyl}-propansäure (*Racemat*) | ¹H-NMR (400 MHz, DMSO-d₆, δ/ppm): 12.25-11.95 (1H, breites s), 10.00 (1H, d), 7.79 (2H, d), 7.62-7.48 (3H, m), 7.48-7.35 (4H, m), 7.32 (2H, d), 7.15 (1H, t), 6.88 (1H, d), 4.94 (2H, s), 3.39 (1H, d), 2.74 (2H, t), 2.65-2.42 (1H, m), 2.48 (2H, t), 1.88-1.71 (1H, m), 1.70-1.19 (6H, m), 1.07-0.89 (1H, m). LC-MS (Methode 11): Rₜ = 1.36 min; m/z = 526 (M+H)⁺. |
| | 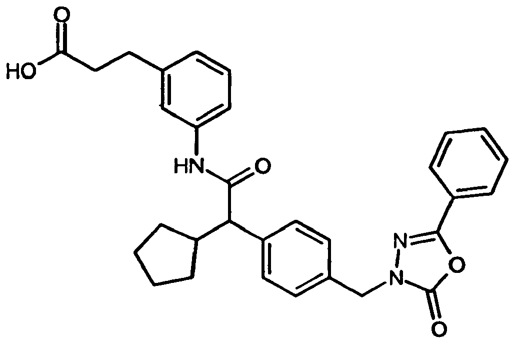 | |
| | Darstellungsmethode 3 (aus Bsp. 157A) | |
| **23** | 3-{3-[(3-Methyl-2-{4-[(5-oxo-2-phenyl-5,6-dihydro-4*H*-1,3,4-oxadiazin-4-yl)methyl]phenyl}hexanoyl)amino]-phenyl}propansäure (*Diastereomerengemisch*) | LC-MS (Methode 7): Rₜ = 2.70 min; m/z = 542 (M+H)⁺. |
| | 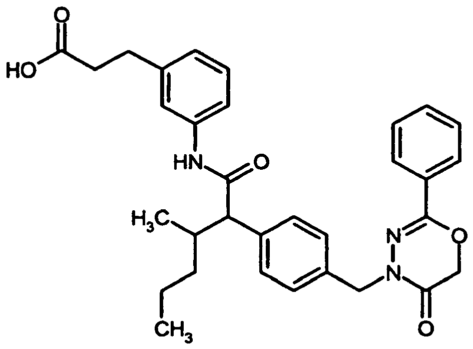 | |
| | Darstellungsmethode 2 (aus Bsp. 164A) | |
| **24** | 4-[(3-Methyl-2-{4-[(5-oxo-2-phenyl-5,6-dihydro-4*H-*1,3,4-oxadiazin-4-yl)methyl]phenyl}hexanoyl)amino]-2,3-dihydro-1*H*-inden-2-carbonsäure (*Diastereomerengemisch*) | LC-MS (Methode 11): Rₜ = 1.42 min; m/z = 554 (M+H)⁺. |
| | 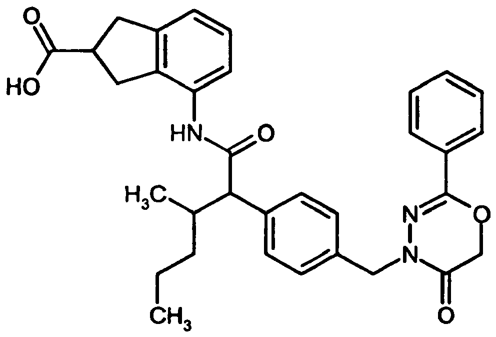 | |
| | Darstellungsmethode 2 (aus Bsp. 165A) | |
| **25** | 3-{3-[(Cyclopentyl{4-[(3-methyl-2-oxoimidazolidin-1-yl)methyl]phenyl}acetyl)amino]-2-methylphenyl}-propansäure (*Racemat*) | ¹H-NMR (400 MHz, DMSO-d₆, δ/ppm): 12.3-11.9 (1H, breites s), 9.46 (1H, d), 7.38 (2H, d), 7.17 (2H, d), 7.08-6.95 (3H, m), 4.23 (2H, s), 3.45 (1H, d), 3.22 (2H, t), 3.11 (2H, t), 2.79 (2H, t), 2.66 (3H, s), 2.64-2.42 (1H, m), 2.43 (2H, t), 1.99 (3H, s), 1.90-1.79 (1H, m), 1.75-1.29 (6H, m), 1.05-0.91 (1H, m). LC-MS (Methode 11): Rₜ = 1.12 min; m/z = 478 (M+H)⁺. |
| | 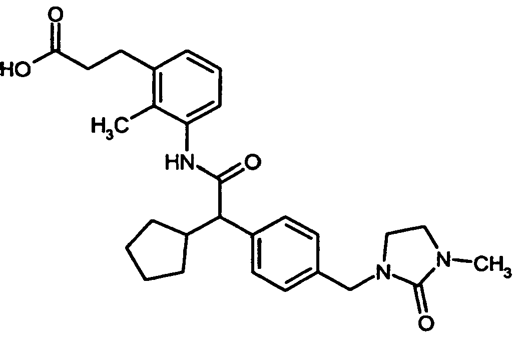 | |
| | Darstellungsmethode 3 (aus Bsp. 162A) | |
| **26** | 3-(3-{[(4-{[3-(3-Chlorphenyl)-2-oxoimidazolidin-1-yl]-methyl}phenyl)(cyclopentyl)acetyl]amino}-2-methylphenyl)propansäure (*Racemat*) | ¹H-NMR (400 MHz, DMSO-d₆, δ/ppm): 12.3-11.9 (1H, breites s), 9.47 (1H, d), 7.82 (1H, s), 7.49-7.39 (3H, m), 7.34 (1H, t), 7.26 (2H, d), 7.08-6.94 (4H, m), 4.38 (2H, s), 3.82 (2H, t), 3.47 (1H, d), 3.37 (2H, t), 2.80 (2H, t), 2.65-2.48 (1H, m), 2.43 (2H, t), 2.00 (3H, s), 1.91-1.78 (1H, m), 1.75-1.28 (6H, m), 1.05-0.91 (1H, m). LC-MS (Methode 10): Rₜ = 2.28 min; m/z = 574 (M+H)⁺. |
| | 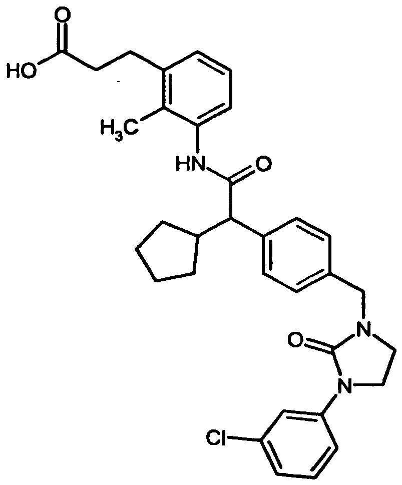 | |
| | Darstellungsmethode 3 (aus Bsp. 161A) | |
| **27** | 3-(3-{[(4-{[3-(3-Chlorphenyl)-2-oxoimidazolidin-1-yl]-methyl}phenyl)(cyclopentyl)acetyl]amino}phenyl)-propansäure *(Racemat)* | ¹H-NMR (400 MHz, DMSO-d₆, δ/ppm): 12.2-12.0 (1H, breites s), 10.00 (1H, d), 7.81 (1H, s), 7.49-7.38 (5H, m), 7.32 (1H, t), 7.24 (2H, d), 7.16 (1H, t), 7.03 (1H, d), 6.88 (1H, d), 4.36 (2H, s), 3.80 (2H, t), 3.41-3.29 (3H, m), 2.75 (2H, t), 2.69-2.52 (1H, m), 2.65-2.48 (2H, t), 1.86-1.71 (1H, m), 1.69-1.20 (6H, m), 1.03-0.90 (1H, m). LC-MS (Methode 10): Rₜ = 2.33 min; m/z = 560 (M+H)⁺. |
| | 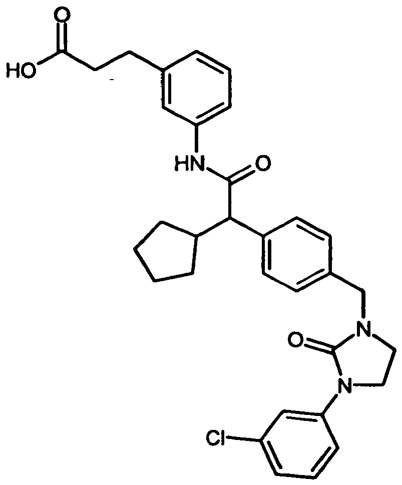 | |
| | Darstellungsmethode 3 (aus Bsp. 168A) | |
| **28** | 3-{3-[(Cyclopentyl{4-[(3-methyl-2-oxoimidazolidin-1-yl)methyl]phenyl}acetyl)amino]phenyl}propansäure (*Racemat*) | LC-MS (Methode 10): Rₜ = 1.77 min; m/z = 464 (M+H)⁺. |
| | 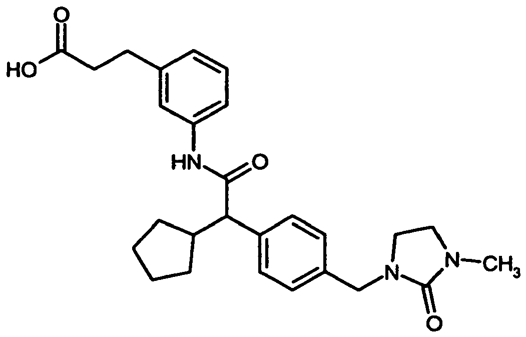 | |
| | Darstellungsmethode 3 (aus Bsp. 169A) | |
| **29** | 3-{3-[(Cyclopentyl{4-[(4-oxo-1,2,3-benzotriazin-3(4*H*)-yl)methyl]phenyl}acetyl)amino]-2-methylphenyl}-propansäure (*Racemat*) | ¹H-NMR (400 MHz, DMSO-d₆, δ/ppm): 12.13 (1H, s), 9.46 (1H, d), 8.31-8.19 (2H, m), 8.10 (1H, t), 7.95 (1H, t), 7.40 (2H, d), 7.33 (2H, d), 7.07-6.94 (3H, m), 5.57 (2H, s), 3.45 (1H, d), 2.78 (2H, t), 2.59-2.46 (1H, m), 2.42 (2H, t), 1.97 (3H, s), 1.88-1.75 (1H, m), 1.71-1.25 (6H, m), 1.01-0.89 (1H, m). LC-MS (Methode 10): Rₜ = 2.05 min; m/z = 525 (M+H)⁺. |
| | 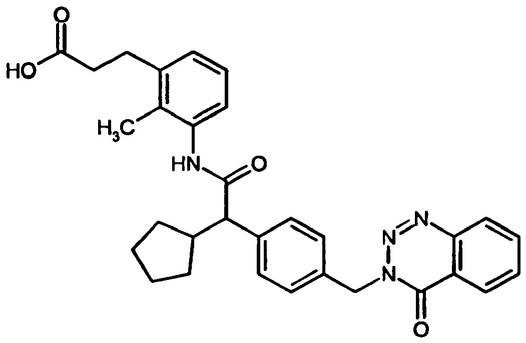 | |
| | Darstellungsmethode 3 (aus Bsp. 166A) | |
| **30** | 3-{3-[(Cyclopentyl{4-[(2-oxo-5-phenyl-1,3,4-oxadiazol-3(2*H*)-yl)methyl]phenyl}acetyl)amino]-2-methyl-phenyl}propansäure (*Racemat*) | ¹H-NMR (400 MHz, DMSO-d₆, δ/ppm): 12.3-11.8 (1H, breites s), 9.48 (1H, d), 7.80 (2H, m), 7.65-7.50 (3H, m), 7.44 (2H, d), 7.32 (2H, d), 7.09-6.92 (3H, m), 4.96 (2H, s), 3.48 (1H, d), 2.78 (2H, t), 2.62-2.48 (1H, m), 2.42 (2H, t), 1.99 (3H, s), 1.90-1.78 (1H, m), 1.72-1.28 (6H, m), 1.05-0.91 (1H, m). LC-MS (Methode 7): Rₜ = 2.61 min; m/z = 540 (M+H)⁺. |
| | 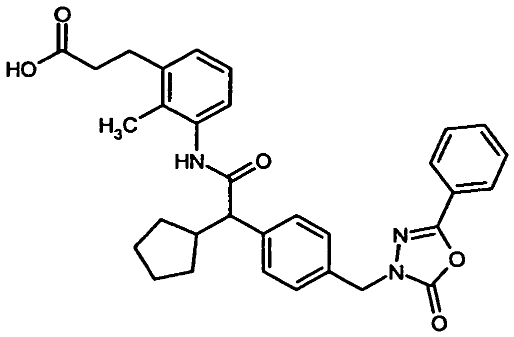 | |
| | Darstellungsmethode 3 (aus Bsp. 167A) | |
| **31** | 3-{3-[(4-Cyano-3-methyl-2-{4-[(5-oxo-2-phenyl-5,6-dihydro-4*H*-1,3,4-oxadiazin-4-yl)methyl]phenyl}-butanoyl)amino]-2-methylphenyl}propansäure (*Diastereomerengemisch*) | ¹H-NMR (400 MHz, DMSO-d₆, δ/ppm): 12.14 (1H, s), 9.68 (1H, d), 7.76 (2H, d), 7.54-7.31 (7H, m), 7.08-6.92 (3H, m), 4.92 (2H, s), 4.89 (2H, s), 3.63 (1H, d), 2.76 (2H, t), 2.65-2.48 (1H, m), 2.48-2.35 (3H, m), 2.11-1.98 (1H, m), 1.92 (3H, s), 1.18 und 0.82 (zus. 3H, 2d). LC-MS (Methode 11): Rₜ = 1.18 min; m/z = 553 (M+H)⁺. |
| | 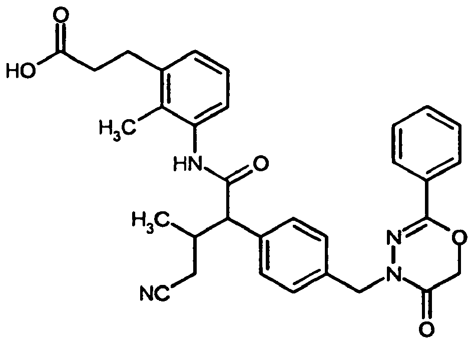 | |
| | Darstellungsmethode 3 (aus Bsp. 171A) | |
| **32** | 3-{3-[(4-Cyano-3-methyl-2-{4-[(5-oxo-2-phenyl-5,6-dihydro-4*H*-1,3,4-oxadiazin-4-yl)methyl]phenyl}-butanoyl)amino]phenyl}propansäure (*Diastereomerengemisch*) | LC-MS (Methode 7): Rₜ = 2.35 min; m/z = 539 (M+H)⁺. |
| | 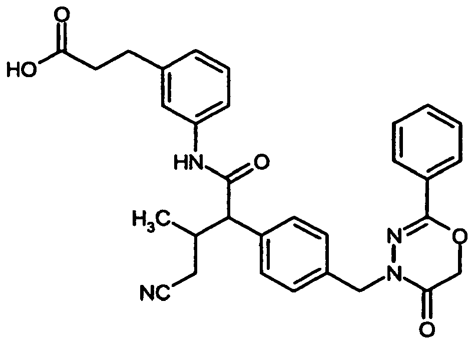 | |
| | Darstellungsmethode 2 (aus Bsp. 172A) | |
| **33** | 4-[(4-Cyano-3-methyl-2-{4-[(5-oxo-2-phenyl-5,6-dihydro-4*H*-1,3,4-oxadiazin-4-yl)methyl]phenyl}-butanoyl)amino]-2,3-dihydro-1*H*-inden-2-carbonsäure (*Diastereomerengemisch*) | LC-MS (Methode 7): Rₜ = 2.36 min; m/z = 551 (M+H)⁺. |
| | 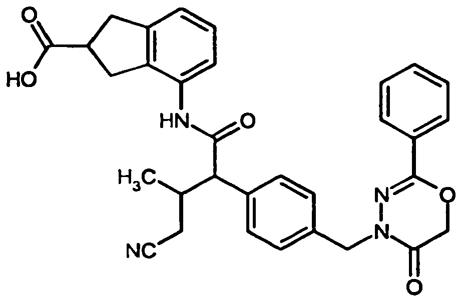 | |
| | Darstellungsmethode 2 (aus Bsp. 173A) | |

### Beispiel 34

### 3-{2-[(Cyclopentyl{4-[(5-oxo-2-phenyl-5,6-dihydro-4H-1,3,4-oxadiazin-4-yl)methyl]phenyl}-acetyl)amino]-1,3-thiazol-4-yl}propansäure (Racemat)

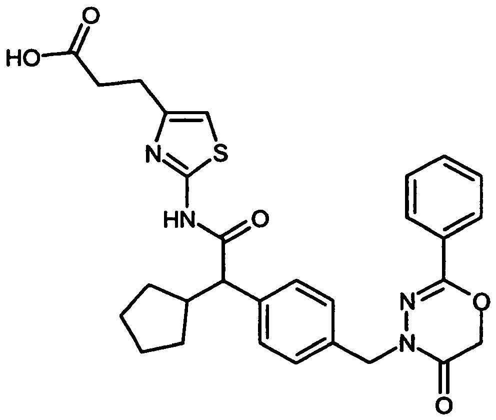

### Darstellungsmethode 4

Eine Lösung von 39.2 mg (0.1 mmol) Cyclopentyl{4-[(5-oxo-2-phenyl-5,6-dihydro-4*H*-1,3,4-oxadiazin-4-yl)methyl]phenyl}essigsäure (Beispiel 51A) in 0.5 ml DMSO wurde mit 20 mg (0.1 mmol) Ethyl-3-(2-amino-1,3-thiazol-4-yl)propanoat [Beilstein Reg.-Nr. 9762098], 41.7 mg (0.13 mmol) TBTU und 25.8 mg Ethyldiisopropylamin versetzt. Es wurde über Nacht bei Raumtemperatur verrührt. Dann wurden 0.3 ml 2 M Natronlauge zugegeben und die Mischung erneut über Nacht gerührt. Das Lösungsmittel wurde danach abgedampft und der erhaltene Rückstand direkt über präparative HPLC/MS gereinigt. Es wurden 7.1 mg (0.013 mmol, 13% d. Th.) der Titelverbindung erhalten.
LC-MS (Methode 14): Rₜ = 2.20 min; m/z = 547 (M+H)⁺.

Auf analoge Weise wurde die in der folgenden Tabelle aufgeführte Verbindung erhalten:

| **Beispiel** | **Name / Struktur Darstellungsmethode / Edukte** | **Analytische Daten** |
|---|---|---|
| **35** | {2-[(Cyclopentyl{4-[(5-oxo-2-phenyl-5,6-dihydro-4*H*-1,3,4-oxadiazin-4-yl)methyl]phenyl}acetyl)amino]-1,3-thiazol-4-yl}essigsäure (*Racemat*) | LC-MS (Methode 14): Rₜ = 2.23 min; m/z = 533 (M+H)⁺. |
| | 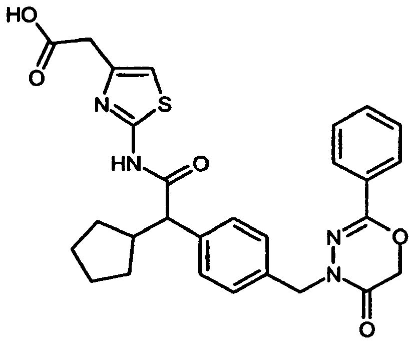 | |
| | Darstellungsmethode 4 (aus Bsp. 51A und Methyl-(2-amino-1,3-thiazol-4-yl)-acetat [CAS Reg.-Nr. 64987-16-2]) | |

### Beispiel 36

### 4-{[(Cyclopentyl{4-[(5-oxo-2-phenyl-5,6-dihydro-4H-1,3,4-oxadiazin-4-yl)methyl]phenyl}acetyl)-amino]methyl}benzolcarbonsäure (Racemat)

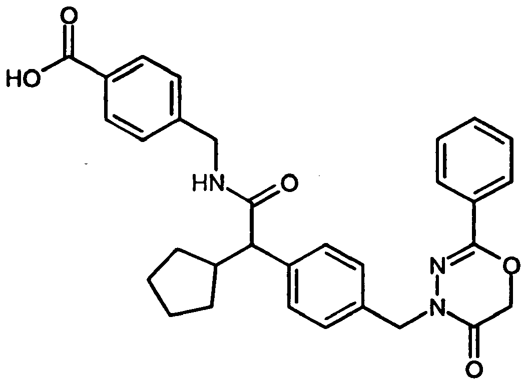

969 mg (1.757 mmol) Methyl-4-{[(cyclopentyl{4-[(5-oxo-2-phenyl-5,6-dihydro-4*H*-1,3,4-oxa-diazin-4-yl)methyl]phenyl}acetyl)amino]methyl}benzolcarboxylat (Beispiel 113A) wurden in 30 ml Dioxan/Wasser (3:1 v/v) gelöst und mit 3.87 ml (3.865 mmol) 1 N Natronlauge versetzt. Es wurde über Nacht bei Raumtemperatur gerührt. Danach wurde gesättigte wässrige Ammoniumchlorid-Lösung zugesetzt und das Gemisch mehrmals mit Dichlormethan extrahiert. Die vereinigten organischen Phasen wurden über Natriumsulfat getrocknet und am Rotationsverdampfer eingeengt. Es wurden 803 mg (1.53 mmol, 87% d. Th.) der Titelverbindung erhalten.

### Beispiel 37 und Beispiel 38

### ent-4-{[(Cyclopentyl{4-[(5-oxo-2-phenyl-5,6-dihydro-4H-1,3,4-oxadiazin-4-yl)methyl]phenyl}-acetyl)amino]methyl} benzolcarbonsäure (Enantiomer 1 und 2)

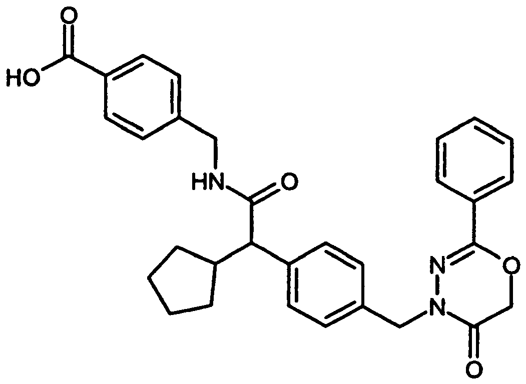

500 mg der erhaltenen racemischen 4-{[(Cyclopentyl{4-[(5-oxo-2-phenyl-5,6-dihydro-4*H*-1,3,4-oxadiazin-4-yl)methyl]phenyl}acetyl)amino]methyl}benzolcarbonsäure (Beispiel 36) wurden mittels präparativer HPLC an chiraler Phase in die Enantiomere aufgetrennt [Säule: Daicel Chiralpak OJ-H, 5 µm, 250 mm x 20 mm; Elutionsmittel: Isohexan/(Ethanol + 0.2% Trifluoressigsäure + 1% Wasser) 60:40 (v/v); Fluss: 15 ml/min; UV-Detektion: 220 nm; Temperatur: 40°C]:

### Beispiel 37 (Enantiomer 1):

Rₜ 5.84 min; Reinheit >96%; >99% ee (Säule s.o.)
Ausbeute: 257 mg
LC-MS (Methode 2): Rₜ = 2.38 min; MS (ESIpos): m/z = 526 (M+H)⁺.

### Beispiel 38 (Enantiomer 2):

Rₜ 7.61 min; Reinheit >97.5%; >98% ee (Säule s.o.)
Ausbeute: 214 mg
LC-MS (Methode 2): Rₜ = 2.38 min; MS (ESIpos): m/z = 526 (M+H)⁺.

### Beispiel 39

### 4-[(3-Methyl-2-{4-[(5-oxo-2-phenyl-5,6-dihydro-4H-1,3,4-oxadiazb-4-yl)methyl]phenyl}-butanoyl)amino]-2,3-dihydro-1H-inden-2-carbonsäure (Isomerengemisch)

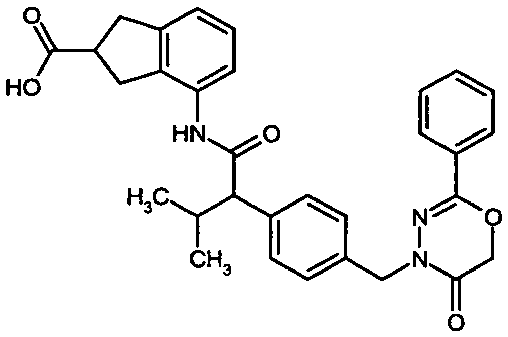

Eine Lösung von 170 mg (0.32 mmol) Methyl-4-[(3-methyl-2-{4-[(5-oxo-2-phenyl-5,6-dihydro-4*H*-1,3,4-oxadiazin-4-yl)methyl]phenyl}butanoyl)amino]-2,3-dihydro-1*H-*inden-2-carboxylat(Beispiel 134A) in 11 ml THF und 11 ml Wasser wurde mit 26 mg (0.63 mmol) Lithiumhydroxid-Monohydrat versetzt und 12 h bei 60°C gerührt. Der Ansatz wurde dann mit 1 M Salzsäure auf pH 2 gestellt und zweimal mit Essigsäureethylester extrahiert. Die vereinigten organischen Phasen wurden über Magnesiumsulfat getrocknet und anschließend im Vakuum bis zur Trockene eingeengt. Es wurden 141 mg (Gehalt 98%, 0.26 mmol, 84% d. Th.) der Titelverbindung als Isomerengemisch erhalten.
LC-MS (Methode 11): Rₜ = 1.30 min; m/z = 526 (M+H)⁺.

### Beispiele 40 - 43

### 4-[(3-Methyl-2-{4-[(5-oxo-2-phenyl-5,6-dihydro-4H-1,3,4-oxadiazin-4-yl)methyl]phenyl}-butanoyl)amino]-2,3-dihydro-1H-inden-2-carbonsäure (Isomer 1-4)

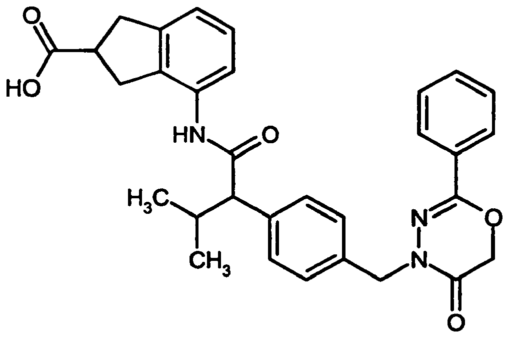

141 mg (Gehalt 98%, 0.26 mmol) des erhaltenen Isomerengemisches von 4-[(3-Methyl-2-{4-[(5-oxo-2-phenyl-5,6-dihydro-4*H*-1,3,4-oxadiazin-4-yl)methyl]phenyl}butanoyl)amino]-2,3-dihydro-1*H*-inden-2-carbonsäure (Beispiel 39) wurden mittels präparativer HPLC an chiraler Phase in die einzelnen Isomere aufgetrennt [Säule: Daicel Chiralpak AD-H, 5 µm, 250 mm x 20 mm; Elutionsmittel: Isohexan/(Isopropanol + 0.2% Trifluoressigsäure + 1 % Wasser) 70:30 (v/v); Fluss: 15 ml/ min; UV-Detektion: 220 nm; Temperatur: 25°C]:

### Beispiel 40 (Isomer 1):

Rₜ 7.79 min; Reinheit >99%; >99% ee (Säule s.o.)
Ausbeute: 36 mg
LC-MS (Methode 11): Rₜ = 1.30 min; m/z = 526 (M+H)⁺.

### Beispiel 41 (Isomer 2):

Rₜ 8.37 min; Reinheit 93%; >99% ee (Säule s.o.)
Ausbeute: 13 mg
LC-MS (Methode 11): Rₜ = 1.30 min; m/z = 526 (M+H)⁺.

### Beispiel 42 (Isomer 3):

Rₜ 10.70 min; Reinheit >99%; >98.5% ee (Säule s.o.)
Ausbeute: 41.4 mg
¹H-NMR (400 MHz, DMSO-d₆, δ/ppm): 12.5-12.1 (1H, breites s), 9.49 (1H, s), 7.76 (2H, d), 7.53-7.25 (8H, m), 7.04 (1H, t), 6.95 (1H, d), 4.91 (2H, s), 4.86 (2H, s), 3.38 (1H, d), 3.28-2.95 (6H, m), 2.35-2.21 (1H, m), 1.02 (3H, d), 0.66 (3H, d).
LC-MS (Methode 11): Rₜ = 1.30 min; m/z = 526 (M+H)⁺.

### Beispiel 43 (Isomer 4):

Rₜ12.10 min; Reinheit >94%; >99% ee (Säule s.o.)
Ausbeute: 14 mg
LC-MS (Methode 11): Rₜ = 1.30 min; m/z = 526 (M+H)⁺.

### Beispiel 44

### 4-[(Cyclopentyl{4-[(5-oxo-2-phenyl-5,6-dihydro-4H-1,3,4-oxadiazin-4-yl)methyl]phenyl}acetyl)-amino]-2-methyl-2,3-dihydro-1H-inden-2-carbonsäure (Diastereomerengemisch)

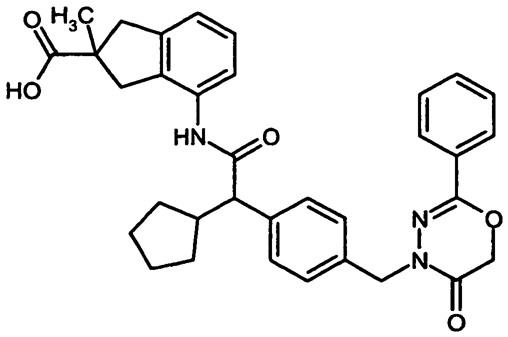

Eine Lösung von 74 mg (0.13 mmol) Methyl-4-[(cyclopentyl{4-[(5-oxo-2-phenyl-5,6dihydro-4*H-*1,3,4-oxadiazin-4-yl)methyl]phenyl}acetyl)amino]-2-methyl-2,3-dihydro-1*H*-inden-2-carboxylat (Beispiel 131A) in 2 ml THF und 1 ml Wasser wurde mit 11 mg (0.26 mmol) Lithiumhydroxid-Monohydrat versetzt und 12 h bei Raumtemperatur gerührt. Der Ansatz wurde dann mit 1 M Salzsäure auf pH 2 gestellt und zweimal mit Essigsäureethylester extrahiert. Die vereinigten organischen Phasen wurden über Magnesiumsulfat getrocknet, filtriert und anschließend im Vakuum bis zur Trockene eingeengt. Es wurden 64 mg (0.11 mmol, 88% d. Th.) der Titelverbindung als Diastereomerengemisch erhalten.
LC-MS (Methode 11): Rₜ = 1.40 min; m/z = 566 (M+H)⁺.

### Beispiel 45 und Beispiel 46

### 4-[(Cyclopentyl{4-[(5-oxo-2-phenyl-5,6-dihydro-4H-1,3,4-oxadiazin-4-yl)methyl]phenyl}acetyl)-amino]-2-methyl-2,3-dihydro-1H-inden-2-carbonsäure (Diastereomer 1 und 2)

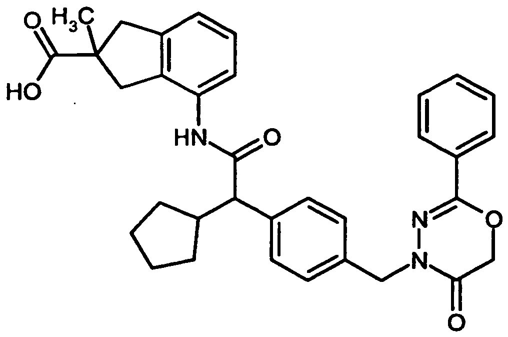

60 mg (0.11 mmol) des erhaltenen Diastereomerengemisches von 4-[(Cyclopentyl{4-[(5-oxo-2-phenyl-5,6-dihydro-4*H*-1,3,4-oxadiazin-4-yl)methyl]phenyl}acetyl)amino]-2-methyl-2,3-dihydro-1*H*-inden-2-carbonsäure (Beispiel 44) wurden mittels präparativer HPLC an chiraler Phase weiter aufgetrennt [Säule: Daicel Chiralpak AD-H, 5 µm, 250 mm x 20 mm; Elutionsmittel: Isohexan/ (Ethanol + 0.2% Trifluoressigsäure + 1% Wasser) 70:30 (v/v); Fluss: 15 ml/min; UV-Detektion: 220 nm; Temperatur: 35°C]:

### Beispiel 45 (Diastereomer 1):

Rₜ 6.2 min; Reinheit >99%; >99% ee (Säule s.o.)
Ausbeute: 19 mg
LC-MS (Methode 7): Rₜ = 2.74 min; m/z = 566 (M+H)⁺.

### Beispiel 46 (Diastereomer 2):

Rₜ 8.8 min; Reinheit >95.5%; >99% ee (Säule s.o.)
Ausbeute: 37 mg
¹H-NMR (400 MHz, DMSO-d₆, δ/ppm): 12.33 (1H, s), 9.48 (1H, s), 7.76 (2H, d), 7.55-7.38 (5H, m), 7.30 (3H, t), 7.04 (1H, t), 6.94 (1H, d), 4.91 (2H, s), 4.85 (2H, s), 3.53 (1H, d), 3.38-3.28 (1H, m), 3.23 (1H, d), 2.78-2.61 (2H, m), 2.61-2.46 (1H, m), 1.87-1.72 (1H, m), 1.72-1.20 (6H, m), 1.24 (3H, s), 1.04-0.90 (1H, m).
LC-MS (Methode 7): Rₜ = 2.76 min; m/z = 566 (M+H)⁺.

### Beispiel 47

### 4-{[{4-[(2-tert.-Butyl-5-oxo-5,6-dihydro-4H-1,3,4-oxadiazin-4-yl)methyl]phenyl}(cyclopentyl)-acetyl]amino}-2,3-dihydro-1H-inden-2-carbonsäure (Diastereomerengemisch)

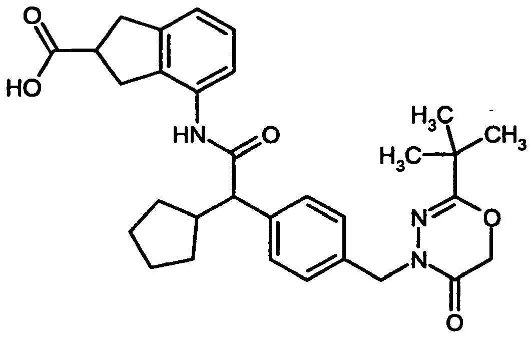

167 mg (0.306 mmol) Methyl-4-{[{4-[(2-*tert*.-butyl-5-oxo-5,6-dihydro-4*H*-1,3,4-oxadiazin-4-yl)-methyl]phenyl}(cyclopentyl)acetyl]amino}-2,3-dihydro-1*H*-inden-2-carboxylat (Beispiel 108A) wurden in 14 ml Dioxan/Wasser (3:1 v/v) gelöst und mit 0.46 ml 1 N Natronlauge versetzt. Es wurde über Nacht bei Raumtemperatur gerührt. Danach wurde mit 1 N Salzsäure angesäuert, das Gemisch mehrfach mit Dichlormethan extrahiert und die vereinigten organischen Phasen mit gesättigter Natriumchlorid-Lösung gewaschen und über Natriumsulfat getrocknet. Das Lösungsmittel wurde im Vakuum entfernt und der Rückstand mittels präparativer HPLC gereinigt. Es wurden 68 mg (42% d. Th.) der Titelverbindung als Diastereomerengemisch erhalten.
LC-MS (Methode 10): Rₜ = 2.28 min; m/z = 532 (M+H)⁺.

### Beispiel 48 und Beispiel 49

### 4-{[{4-[(2-tert.-Butyl-5-oxo-5,6-dihydro-4H-1,3,4-oxadiazin-4-yl)methyl]phenyl}(cyclopentyl)-acetyl]amino}-2,3-dihydro-1H-inden-2-carbonsäure (Diastereomer 1 und 2)

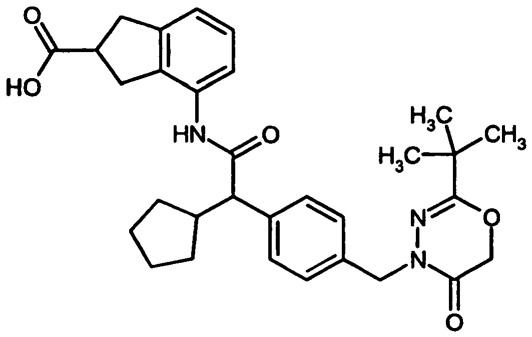

62 mg (0.117 mmol) des erhaltenen Diastereomerengemisches von 4-{[{4-[(2-*tert*.-Butyl-5-oxo-5,6-dihydro-4*H*-1,3,4-oxadiazin-4-yl)methyl]phenyl}(cyclopentyl)acetyl]amino}-2,3-dihydro-1*H-*inden-2-carbonsäure (Beispiel 47) wurden mittels präparativer HPLC an chiraler Phase weiter aufgetrennt [Säule: chirale Kieselgel-Phase basierend auf dem Selektor Poly(*N*-methacryloyl-L-leucin-*tert*.-butylamid, 670 mm x 40 mm; Elutionsmittel: Essigsäureethylester; Fluss: 80 ml/min; UV-Detektion: 260 nm; Temperatur: 24°C]:

### Beispiel 48 (Diastereomer 1):

Rₜ 2.63 min; >97.5% ee (Säule s.o.)
Ausbeute: 23 mg

### Beispiel 49 (Diastereomer 2):

Rₜ 4.04 min; >98% ee (Säule s.o.)
Ausbeute: 27 mg.

### Beispiel 50

### 4-[(5,5,5-Trifluor-2-{4-[(5-oxo-2-phenyl-5,6-dihydro-4H-1,3,4-oxadiazin-4-yl)methyl]phenyl}-pentanoyl)amino]-2,3-dihydro-1H-inden-2-carbonsäure (Diastereomerengemisch)

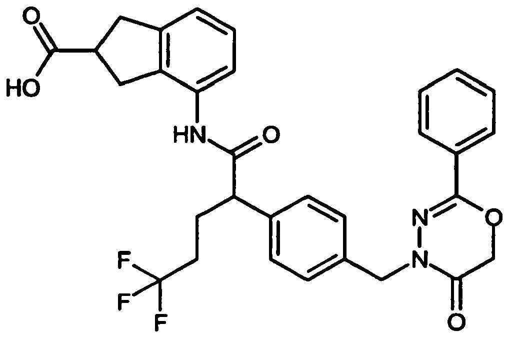

Eine Lösung von 112 mg (0.19 mmol) Methyl-4-[(5,5,5-trifluor-2-{4-[(5-oxo-2-phenyl-5,6-dihydro-4*H*-1,3,4-oxadiazin-4-yl)methyl]phenyl}pentanoyl)amino]-2,3-dihydro-1*H*-inden-2-carboxylat (Beispiel 143A) in 2 ml THF und 1 ml Wasser wurde mit 32 mg (0.76 mmol) Lithiumhydroxid-Monohydrat versetzt und über Nacht bei Raumtemperatur gerührt. Der Ansatz wurde dann mit 1 M Salzsäure auf pH 2 gestellt und zweimal mit Essigsäureethylester extrahiert. Die vereinigten organischen Phasen wurden über Magnesiumsulfat getrocknet, filtriert und anschließend im Vakuum bis zur Trockene eingeengt. Der erhaltene Rückstand wurde über präparative HPLC gereinigt. Es wurden 74 mg (0.13 mmol, 68% d. Th.) der Titelverbindung als Diastereomerengemisch erhalten.
LC-MS (Methode 10): Rₜ = 2.19 min; m/z = 580 (M+H)⁺.

### Beispiel 51 und Beispiel 52

### 4-[(5,5,5-Trifluor-2-{4-[(5-oxo-2-phenyl-5,6-dihydro-4H-1,3,4-oxadiazin-4-yl)methyl]phenyl}-pentanoyl)amino]-2,3-dihydro-1H-inden-2-carbonsäure (Diastereomer 1 und 2)

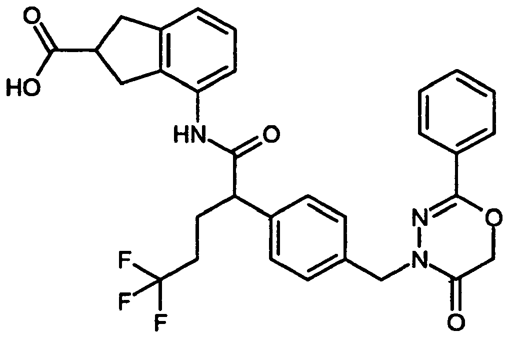

74 mg (0.13 mmol) des erhaltenen Diastereomerengemisches von 4-[(5,5,5-Trifluor-2-{4-[(5-oxo-2-phenyl-5,6-dihydro-4*H*-1,3,4-oxadiazin-4-yl)methyl]phenyl}pentanoyl)amino]-2,3-dihydro-1*H-*inden-2-carbonsäure (Beispiel 50) wurden mittels präparativer HPLC an chiraler Phase weiter aufgetrennt [Säule: Daicel Chiralpak AD-H, 5 µm, 250 mm x 20 mm; Elutionsmittel: Isohexan/ (Ethanol + 0.2% Trifluoressigsäure + 1% Wasser) 50:50 (v/v); Fluss: 15 ml/min; UV-Detektion: 220 nm; Temperatur: 30°C]:

### Beispiel 51 (Diastereomer 1):

Rₜ 5.61 min; Reinheit >99%; >99% ee (Säule s.o.)
Ausbeute: 20 mg
LC-MS (Methode 11): Rₜ = 1.34 min; m/z = 580 (M+H)⁺.

### Beispiel 52 (Diastereomer 2):

Rₜ 9.21 min; Reinheit >99%; >99% ee (Säule s.o.)
Ausbeute: 39 mg
¹H-NMR (400 MHz, DMSO-d₆, δ/ppm): 12.45-12.10 (1H, s), 9.57 (1H, s), 7.76 (2H, d), 7.53-7.31 (8H, m), 7.07 (1H, t), 6.97 (1H, d), 4.91 (2H, s), 4.86 (2H, s), 3.99 (1H, t), 3.24-3.12 (1H, m), 3.08 (2H, d), 2.98 (2H, t), 2.29-2.10 (3H, m), 1.97-1.82 (1H, m).
LC-MS (Methode 11): Rₜ = 1.34 min; m/z = 580 (M+H)⁺.

### Beispiel 53

### 3-{3-[(3-Methyl-2-{4-[(5-oxo-2-phenyl-5,6-dihydro-4H-1,3,4-oxadiazin-4-yl)methyl]phenyl}-butanoyl)amino]cyclohexyl}propansäure (Isomerengemisch)

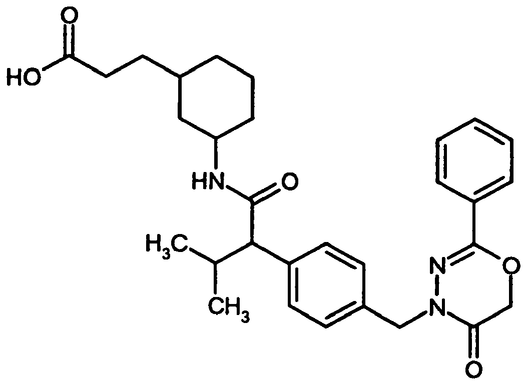

Eine Lösung von 600 mg (1.12 mmol) Methyl-3-{3-[(3-methyl-2-{4-[(5-oxo-2-phenyl-5,6-dihydro-4*H*-1,3,4-oxadiazin-4-yl)methyl]phenyl}butanoyl)amino]cyclohexyl}propanoat (Beispiel 142A) in 15 ml THF und 15 ml Wasser wurde mit 94 mg (2.25 mmol) Lithiumhydroxid-Monohydrat versetzt und über Nacht bei Raumtemperatur gerührt. Der Ansatz wurde dann mit 1 M Salzsäure auf pH 2 gestellt und zweimal mit Essigsäureethylester extrahiert. Die vereinigten organischen Phasen wurden über Magnesiumsulfat getrocknet, filtriert und anschließend im Vakuum bis zur Trockene eingeengt. Es wurden 573 mg (1.10 mmol, 98% d. Th.) der Titelverbindung als Isomerengemisch erhalten.
LC-MS (Methode 7): Rₜ = 2.48 min, m/z = 520 (M+H)⁺ (Diastereomer 1); Rₜ = 2.52 min, m/z = 520 (M+H)⁺ (Diastereomer 2).

### Beispiele 54 - 57

### 3-{3-[(3-Methyl-2-{4-[(5-oxo-2-phenyl-5,6-dihydro-4H-1,3,4-oxadiazin-4-yl)methyl]phenyl}-butanoyl)amino]cyclohexyl}propansäure (Isomer 1 - 4)

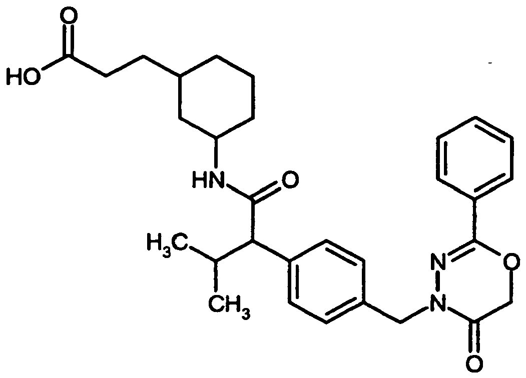

573 mg (1.10 mmol) des erhaltenen Isomerengemisches von 3-{3-[(3-Methyl-2-{4-[(5-oxo-2-phenyl-5,6-dihydro-4*H*-1,3,4-oxadiazin-4-yl)methyl]phenyl}butanoyl)amino]cyclohexyl}propansäure (Beispiel 53) wurden zunächst mittels präparativer HPLC in die beiden Diastereomere aufgetrennt [Säule: Kromasil 100 C18, 5 µm, 250 mm x 20 mm; Elutionsmittel: Wasser/Acetonitril/ 1% Trifluoressigsäure 40:50:10 (v/v); Fluss: 25 ml/min; UV-Detektion: 210 nm; Temperatur: 40°C; Ausbeute: 209 mg Diastereomer 1 Rₜ = 7.37 min, 243 mg Diastereomer 2 Rₜ = 7.77 min].

209 mg des so erhaltenen Diastereomers 1 wurden dann mittels präparativer HPLC an chiraler Phase weiter in die Enantiomere aufgetrennt [Daicel Chiralpak AS-H, 5 µm 250 mm x 20 mm; Elutionsmittel: Isohexan/(Ethanol + 0.2% Trifluoressigsäure + 1% Wasser) 80:20 (v/v); Fluss: 15 ml/min; UV-Detektion: 220 nm; Temperatur: 30°C]:

### Beispiel 54 (Diastereomer 1 / Enantiomer 1):

Rₜ 6.24 min; Reinheit >99%; >99% ee (Daicel-Säule s.o.)
Ausbeute: 19 mg
¹H-NMR (400 MHz, DMSO-d₆, δ/ppm): 11.98 (1H, s), 7.84 (1H, s), 7.76 (2H, d), 7.52-7.41 (3H, m), 7.33-7.22 (4H, m), 4.91 (2H, s), 4.84 (2H, s), 3.48-3.35 (1H, m), 2.93 (1H, d), 2.27-2.12 (3H, m), 1.82 (1H, d), 1.60 (2H, d), 1.52 (1H, d), 1.46-1.34 (2H, m), 1.31-1.05 (2H, m), 1.00-0.87 (1H, m), 0.92 (3H, d), 0.80-0.64 (2H, m), 0.59 (3H, d).
LC-MS (Methode 11): Rₜ = 1.27 min; m/z = 520 (M+H)⁺.

### Beispiel 55 (Diastereomer 1 / Enantiomer 2):

Rₜ 14.28 min; Reinheit >99%; >99% ee (Daicel-Säule s.o.)
Ausbeute: 99 mg
LC-MS (Methode 11): Rₜ = 1.27 min; m/z = 520 (M+H)⁺.

Ebenso wurden 243 mg des oben erhaltenen Diastereomers 2 mittels präparativer HPLC an chira-1er Phase weiter in die Enantiomere aufgetrennt [Daicel Chiralpak AD-H, 5 µm, 250 mm x 20 mm; Elutionsmittel: Isohexan/(Ethanol + 0.2% Trifluoressigsäure + 1% Wasser) 80:20 (v/v); Fluss: 15 ml/min; UV-Detektion: 220 nm; Temperatur: 30°C]:

### Beispiel 56 (Diastereomer 2 /Enantiomer 1):

Rₜ 5.82 min; Reinheit >99%; >99% ee (Daicel-Säule s.o.)
Ausbeute: 17 mg
¹H-NMR (400 MHz, DMSO-d₆, δ/ppm): 12.1-11.8 (1H, breites s), 7.85 (1H, s), 7.76 (2H, d), 7.53-7.41 (3H, m), 7.32-7.22 (4H, m), 4.91 (2H, s), 4.84 (2H, s), 3.48-3.35 (1H, m), 2.93 (1H, d), 2.25-2.15 (1H, m), 2.12 (2H, t), 1.79 (1H, d), 1.67 (1H, d), 1.57 (2H, t), 1.41-1.28 (2H, m), 1.28-1.11 (2H, m), 1.07-0.96 (1H, m), 0.91 (3H, d), 0.79-0.62 (2H, m), 0.59 (3H, d).
LC-MS (Methode 7): Rₜ = 2.53 min; m/z = 520 (M+H)⁺.

### Beispiel 57 (Diastereomer 2 / Enantiomer_2):

Rₜ 9.89 min; Reinheit >99%; >99% ee (Daicel-Säule s.o.)
Ausbeute: 100 mg
LC-MS (Methode 7): Rₜ = 2.53 min; m/z = 520 (M+H)⁺.

### Beispiel 58

### 3-{3-[(3-Methyl-2-{4-[(5-oxo-2-phenyl-5,6-dihydro-4H-1,3,4-oxadiazin-4-yl)methyl]phenyl}-pentanoyl)amino]phenyl}propansäure (Isomerengemisch)

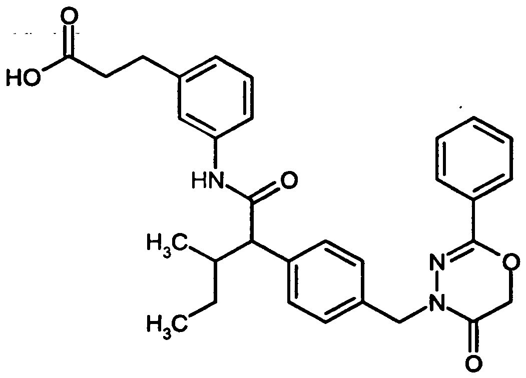

Eine Lösung von 167 mg (0.30 mmol) Ethyl-3-{3-[(3-methyl-2-{4-[(5-oxo-2-phenyl-5,6-dihydro-4*H*-1,3,4-oxadiazin-4-yl)methyl]phenyl}pentanoyl)amino]phenyl}propanoat (Beispiel 146A) in 4 ml THF wurde mit 1.2 ml (1.20 mmol) 1 N Natronlauge versetzt und über Nacht bei Raumtemperatur gerührt. Nach vollständiger Umsetzung wurde das THF im Vakuum abgezogen, die Reaktionslösung mit Wasser verdünnt und anschließend mit 1 M Salzsäure auf pH 2 gestellt. Der ausgefallene Feststoff wurde abfiltriert, mit Wasser gewaschen und über Nacht im Vakuum bei 45°C getrocknet. Es wurden 155 mg (0.29 mmol, 97% d. Th.) der Titelverbindung als Isomerengemisch erhalten.
LC-MS (Methode 7): Rₜ = 2.64 min; m/z = 528 (M+H)⁺.

### Beispiele 59 - 62

### 3-{3-[(3-Methyl-2-{4-[(5-oxo-2-phenyl-5,6-dihydro-4H-1,3,4-oxadiazin-4-yl)methyl]phenyl}-pentanoyl)amino]phenyl}propansäure (Isomer 1 - 4)

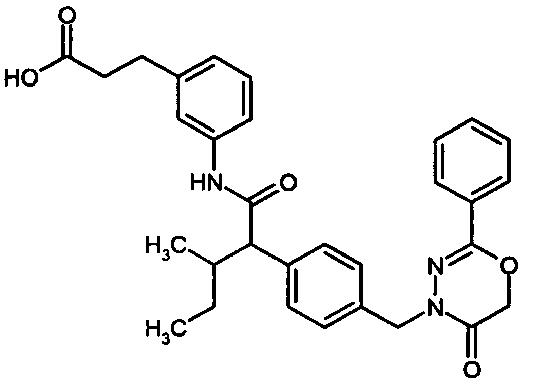

155 mg (0.29 mmol) des erhaltenen Isomerengemisches von 3-{3-[(3-Methyl-2-{4-[(5-oxo-2-phenyl-5,6-dihydro-4*H*-1,3,4-oxadiazin-4-yl)methyl]phenyl}pentanoyl)amino]phenyl}propansäure (Beispiel 58) wurden mittels präparativer HPLC an chiraler Phase weiter aufgetrennt [Säule: Daicel Chiralpak AS-H, 5 µm, 250 mm x 20 mm; Elutionsmittel: Isohexan/(Ethanol + 0.2% Trifluoressigsäure + 1% Wasser) 75:25 (v/v); Fluss: 15 ml/min; UV-Detektion: 220 nm; Temperatur: 25°C]. Es wurden zwei Fraktionen erhalten, die jeweils durch erneute HPLC-Chromatographie an chiraler Phase nachgetrennt wurden [Säule: Daicel Chiralpak AD-H, 5 µm, 250 mm x 20 mm; Elutionsmittel: Isohexan/(Ethanol + 0.2% Trifluoressigsäure + 1% Wasser) 75:25 (v/v); Fluss: 15 ml/min; UV-Detektion: 220 nm; Temperatur: 25°C]:

### Beispiel 59 (Isomer 1):

Rₜ 6.81 min; Reinheit >99%; >99% ee (Daicel AD-H-Säule s.o.)
Ausbeute: 49 mg
LC-MS (Methode 11): Rₜ = 1.36 min; m/z = 528 (M+H)⁺.

### Beispiel 60 (Isomer 2):

Rₜ 7.51 min; Reinheit >99%; >99% ee (Daicel AD-H-Säule s.o.)
Ausbeute: 24 mg
¹H-NMR (400 MHz, DMSO-d₆, δ/ppm): 12.09 (1H, s), 10.02 (1H, s), 7.76 (2H, d), 7.52-7.35 (7H, m), 7.31 (2H, d), 7.14 (1H, t), 6.86 (1H, d), 4.90 (2H, s), 4.84 (2H, s), 3.36 (1H, d), 2.74 (2H, t), 2.47 (2H, t), 2.23-2.10 (1H, m), 1.21-1.07 (1H, m), 0.96 (3H, d), 0.94-0.79 (1H, m), 0.74 (3H, t).
LC-MS (Methode 11): Rₜ = 1.35 min; m/z = 528 (M+H)⁺.

### Beispiel 61 (Isomer 3):

Rₜ 8.20 min; Reinheit >99%; >99% ee (Daicel AD-H-Säule s.o.)
Ausbeute: 44 mg
¹H-NMR (400 MHz, DMSO-d₆, δ/ppm): 12.09 (1H, s), 10.02 (1H, s), 7.76 (2H, d), 7.53-7.35 (7H, m), 7.31 (2H, d), 7.14 (1H, t), 6.86 (1H, d), 4.90 (2H, s), 4.84 (2H, s), 3.33 (1H, d), 2.73 (2H, t), 2.47 (2H, t), 2.24-2.10 (1H, m), 1.59-1.46 (1H, m), 1.27-1.10 (1H, m), 0.90 (3H, t), 0.61 (3H, d).
LC-MS (Methode 11): Rₜ = 1.36 min; m/z = 528 (M+H)⁺.

### Beispiel 62 (Isomer 4):

Rₜ 9.42 min; Reinheit >99%; >99% ee (Daicel AD-H-Säule s.o.)
Ausbeute: 17 mg
LC-MS (Methode 11): Rₜ = 1.35 min; m/z = 528 (M+H)⁺.

### Beispiel 63

### 4-[(3-Methyl-2-{4-[(5-oxo-2-phenyl-5,6-dihydro-4H-1,3,4-oxadiazin-4-yl)methyl]phenyl}-pentanoyl)amino]-2,3-dihydro-1H-inden-2-carbonsäure (Isomerengemisch)

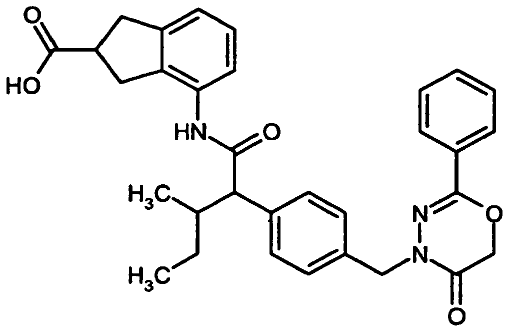

Eine Lösung von 180 mg (0.31 mmol) Methyl-4-[(3-methyl-2-{4-[(5-oxo-2-phenyl-5,6-dihydro-4*H*-1,3,4-oxadiazin-4-yl)methyl]phenyl}pentanoyl)amino]-2,3-dihydro-1*H*-inden-2-carboxylat (Beispiel 147A) in 3.6 ml THF und 1.3 ml (1.3 mmol) 1 N Natronlauge wurde über Nacht bei Raumtemperatur gerührt. Nach vollständiger Umsetzung wurde das THF im Vakuum abgezogen, die Reaktionslösung mit Wasser verdünnt und anschließend mit 1 M Salzsäure auf pH 2 gestellt. Der ausgefallene Feststoff wurde abfiltriert, mit Wasser gewaschen und über Nacht im Vakuum bei 45°C getrocknet. Es wurden 159 mg (0.29 mmol, 91% d. Th.) der Titelverbindung als Isomerengemisch erhalten.
LC-MS (Methode 10): Rₜ = 2.18 min; m/z = 540 (M+H)⁺.

### Beispiele 64 - 67

### 4-[(3-Methyl-2-{4-[(5-oxo-2-phenyl-5,6-dihydro-4H-1,3,4-oxadiazin-4-yl)methyl]phenyl}-pentanoyl)amino]-2,3-dihydro-1H-inden-2-carbonsäure (Isomer 1 - 4)

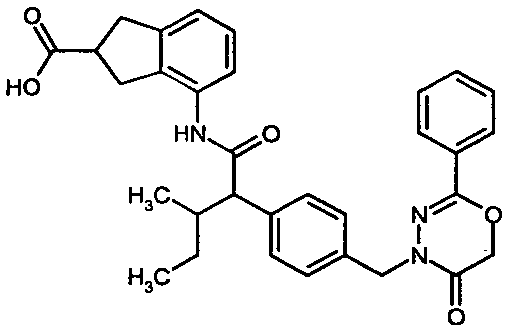

159 mg (0.29 mmol) des erhaltenen Isomerengemisches von 4-[(3-Methyl-2-{4-[(5-oxo-2-phenyl-5,6-dihydro-4*H*-1,3,4-oxadiazin-4-yl)methyl]phenyl}pentanoyl)amino]-2,3-dihydro-1*H*-inden-2-carbonsäure (Beispiel 63) wurden mittels präparativer HPLC an chiraler Phase weiter aufgetrennt [Säule: Daicel Chiralpak AD-H, 5 µm, 250 mm x 20 mm; Elutionsmittel: Isohexan/(Isopropanol + 0.2% Trifluoressigsäure + 1% Wasser) 70:30 (v/v); Fluss: 15 ml/min; UV-Detektion: 220 nm; Temperatur: 30°C]:

### Beispiel 64 (Isomer 1):

Rₜ 9.91 min; Reinheit >99%; >97.5% ee (Säule s.o.)
Ausbeute: 17 mg
LC-MS (Methode 7): Rₜ = 2.65 min; m/z = 540 (M+H)⁺.

### Beispiel 65 (Isomer 2):

Rₜ 11.26 min; Reinheit >99%; >98% ee (Säule s.o.)
Ausbeute: 14 mg
LC-MS (Methode 7): Rₜ = 2.60 min; m/z = 540 (M+H)⁺.

### Beispiel 66 (Isomer 3):

Rₜ 12.27 min; Reinheit >99%; >98% ee (Säule s.o.)
Ausbeute: 34 mg
LC-MS (Methode 7): Rₜ = 2.65 min; m/z = 540 (M+H)⁺.

### Beispiel 67 (Isomer 4):

Rₜ 12.94 min; Reinheit >99%; >98% ee (Säule s.o.)
Ausbeute: 35 mg
¹H-NMR (400 MHz, DMSO-d₆, δ/ppm): 12.28 (1H, s), 9.49 (1H, s), 7.76 (2H, d), 7.53-7.36 (5H, m), 7.35-7.25 (3H, m), 7.04 (1H, t), 6.95 (1H, d), 4.91 (2H, s), 4.86 (2H, s), 3.49 (1H, d), 3.25-3.18 (1H, m), 3.09 (2H, t), 3.00 (2H, d), 2.22-2.08 (1H, m), 1.62-1.48 (1H, m), 1.29-1.15 (1H, m), 0.92 (3H, t), 0.61 (3H, d).
LC-MS (Methode 7): Rₜ = 2.66 min; m/z = 540 (M+H)⁺.

### Beispiel 68

### 4-{[(3-Methyl-2-{4-[(5-oxo-2-phenyl-5,6-dihydro-4H-1,3,4-oxadiazin-4-yl)methyl]phenyl}-pentanoyl)amino]methyl}cyclohexancarbonsäure (Isomerengemisch)

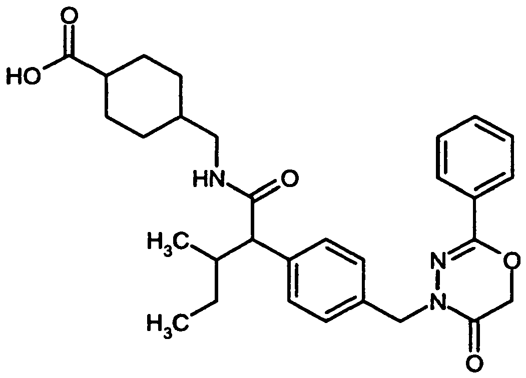

Eine Lösung von 492 mg (0.90 mmol) Ethyl-4-{[(3-methyl-2-{4-[(5-oxo-2-phenyl-5,6-dihydro-4*H-*1,3,4-oxadiazin-4-yl)methyl]phenyl}pentanoyl)amino]methyl}cyclohexancarboxylat (Beispiel 150A) in 8 ml THF und 4.6 ml (4.6 mmol) 1 N Natronlauge wurde vier Tage bei 60°C gerührt. Nach vollständiger Umsetzung wurde das THF im Vakuum abgezogen, die Reaktionslösung mit Wasser verdünnt und anschließend mit 1 M Salzsäure auf pH 2 gestellt. Der ausgefallene Feststoff wurde abfiltriert, mit Wasser gewaschen und über Nacht im Vakuum bei 45°C getrocknet. Es wurden 343 mg (0.66 mmol, 73% d. Th.) der Titelverbindung als Isomerengemisch erhalten.
LC-MS (Methode 7): Rₜ = 2.48 min und 2.52 min; m/z = 520 (M+H)⁺.

### Beispiele 69 - 75

### 4-{[(3-Methyl-2-{4-[(5-oxo-2-phenyl-5,6-dihydro-4H-1,3,4-oxadiazin-4-yl)methyl]phenyl}-pentanoyl)amino]methyl}cyclohexancarbonsäure (Isomer 1 - 7)

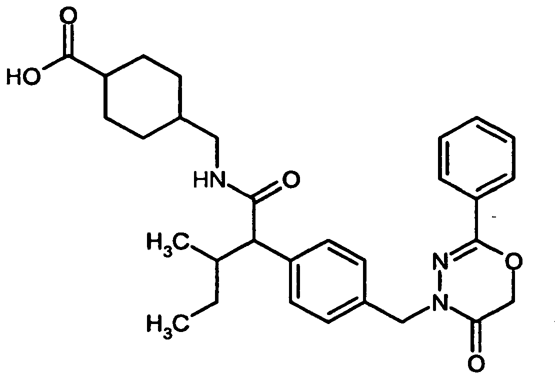

343 mg (0.66 mmol) des erhaltenen Isomerengemisches von 4-{[(3-Methyl-2-{4-[(5-oxo-2-phenyl-5,6-dihydro-4*H*-1,3,4-oxadiazin-4-yl)methyl]phenyl}pentanoyl)amino]methyl}cyclohexancarbonsäure (Beispiel 68) wurden mittels präparativer HPLC an chiraler Phase in die einzelnen Isomere aufgetrennt [Säule: Daicel Chiralpak AD-H, 5 µm, 250 mm x 20 mm; Elutionsmittel: Isohexan/ (Ethanol + 0.2% Trifluoressigsäure + 1% Wasser) 75:25 (v/v); Fluss: 15 ml/min; UV-Detektion: 220 nm; Temperatur: 30°C]. Es wurden 18 Fraktionen erhalten, die mit einer Eluentenzusammensetzung von Isohexan/(Isopropanol + 0.2% Trifluoressigsäure + 1% Wasser) 75:25 (v/v) unter ansonsten gleichen Bedingungen nochmals nachchromatographiert wurden.

### Beispiel 69 (Isomer 1):

Rₜ 4.71 min; Reinheit >95% (Säule s.o.)
Ausbeute: 19 mg
LC-MS (Methode 10): Rₜ = 2.03 min; m/z = 520 (M+H)⁺.

### Beispiel 70 (Isomer 2):

Rₜ 5.18 min; Reinheit >88% (Säule s.o.)
Ausbeute: 5 mg
LC-MS (Methode 7): Rₜ = 2.08 min; m/z = 520 (M+H)⁺.

### Beispiel 71 (Isomer 3):

Rₜ 5.79 min; Reinheit >93% (Säule s.o.)
Ausbeute: 5 mg
LC-MS (Methode 7): Rₜ = 2.08 min; m/z = 520 (M+H)⁺.

### Beispiel 72 (Isomer 4):

Rₜ 6.92 min; Reinheit >99% (Säule s.o.)
Ausbeute: 55 mg
¹H-NMR (400 MHz, DMSO-d₆, δ/ppm): 12.4-11.6 (1H, breites s), 7.95 (1H, t), 7.76 (2H, d), 7.53-7.39 (3H, m), 7.32-7.21 (4H, m), 4.91 (2H, s), 4.84 (2H, s), 3.09 (1H, d), 3.04-2.91 (1H, m), 2.78-2.64 (1H, m), 2.40-2.28 (1H, m), 2.12-2.00 (1H, m), 1.82-1.69 (2H, m), 1.54-1.29 (6H, m), 1.18-0.98 (3H, m), 0.86 (3H, t), 0.55 (3H, d).
LC-MS (Methode 7): Rₜ = 2.08 min; m/z = 520 (M+H)⁺.

### Beispiel 73 (Isomer 5):

Rₜ 7.92 min; Reinheit >97.5% (Säule s.o.)
Ausbeute: 41 mg
LC-MS (Methode 7): Rₜ = 2.08 min; m/z = 520 (M+H)⁺.

### Beispiel 74 (Isomer 6):

Rₜ 9.33 min; Reinheit >99% (Säule s.o.)
Ausbeute: 88 mg
LC-MS (Methode 7): Rₜ = 2.07 min; m/z = 520 (M+H)⁺.

### Beispiel 75 (Isomer 7):

Rₜ 9.62 min; Reinheit >93% (Säule s.o.)
Ausbeute: 14 mg
LC-MS (Methode 7): Rₜ = 2.08 min; m/z = 520 (M+H)⁺.

### Beispiel 76

### 3-{2-Methyl-3-[(3-methyl-2-{4-[(5-oxo-2-phenyl-5,6-dihydro-4H-1,3,4-oxadiazin-4-yl)methyl]-phenyl}pentanoyl)amino]phenyl}propansäure (Isomerengemisch)

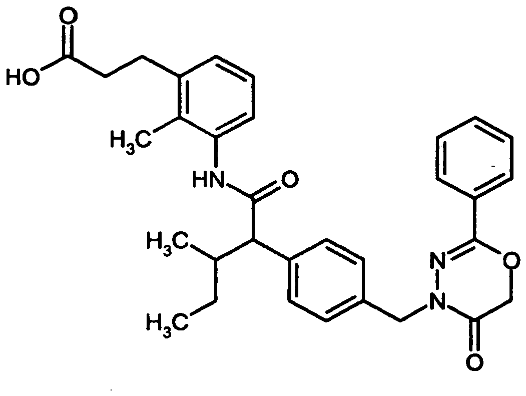

Eine Lösung von 175 mg (0.31 mmol) Ethyl-3-{2-methyl-3-[(3-methyl-2-{4-[(5-oxo-2-phenyl-5,6-dihydro-4*H*-1,3,4-oxadiazin-4-yl)methyl]phenyl}pentanoyl)amino]phenyl}propanoat (Beispiel 151A) in 5.5 ml THF und 1.2 ml (1.2 mmol) 1 N Natronlauge wurde über Nacht bei 60°C gerührt. Nach vollständiger Umsetzung wurde das THF im Vakuum abgezogen, die Reaktionslösung mit Wasser verdünnt und anschließend mit 1 M Salzsäure auf pH 2 gestellt. Der ausgefallene Feststoff wurde abfiltriert, mit Wasser gewaschen und über Nacht im Vakuum bei 45°C getrocknet. Es wurden 165 mg (0.30 mmol, 99% d. Th.) der Titelverbindung als Isomerengemisch erhalten.
LC-MS (Methode 10): Rₜ = 2.13 min; m/z = 542 (M+H)⁺.

### Beispiele 77 - 80

### 3-{2-Methyl-3-[(3-methyl-2-{4-[(5-oxo-2-phenyl-5,6-dihydro-4H-1,3,4-oxadiazin-4-yl)methyl]-phenyl}pentanoyl)amino]phenyl}propansäure (Isomer 1 - 4)

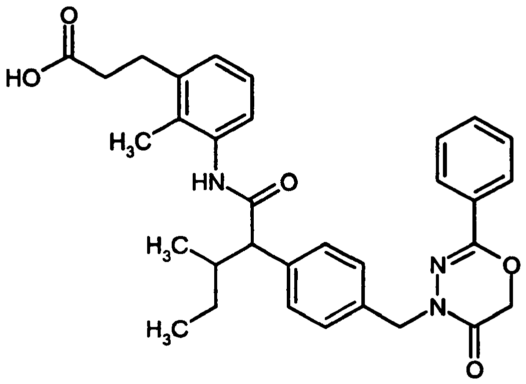

240 mg (0.44 mmol) des erhaltenen Isomerengemisches von 4-{[(3-Methyl-2-{4-[(5-oxo-2-phenyl-5,6-dihydro-4*H*-1,3,4-oxadiazin-4-yl)methyl]phenyl}pentanoyl)amino]methyl}cyclohexancarbonsäure (Beispiel 76) wurden mittels präparativer HPLC an chiraler Phase weiter aufgetrennt [Säule: Daicel Chiralpak IC, 5 µm 250 mm x 20 mm; Elutionsmittel: (tert.-Butylmethylether + 0.2% Eisessig)/Methanol 90:10 (v/v); Fluss: 15 ml/min; UV-Detektion: 220 nm; Temperatur: 25°C]:

### Beispiel 77 (Isomer 1):

Rₜ 7.72 min; Reinheit >99%; >98% ee
[Säule: Daicel Chiralpak AD-H, 5 µm, 250 mm x 4.6 mm; Elutionsmittel: Isohexan/(Isopropanol + 0.2% Trifluoressigsäure + 1% Wasser) 75:25 (v/v); Fluss: 2 ml/min; UV-Detektion: 220 nm; Temperatur: 45°C]
Ausbeute: 69 mg
¹H-NMR (400 MHz, DMSO-d₆, δ/ppm): 12.4-11.8 (1H, breites s), 9.48 (1H, s), 7.76 (2H, d), 7.53-7.42 (3H, m), 7.39 (2H, d), 7.31 (2H, d), 7.06-6.91 (3H, m), 4.92 (2H, s), 4.87 (2H, s), 3.45 (1H, d), 2.76 (2H, t), 2.40 (2H, t), 2.22-2.09 (1H, m), 1.94 (3H, s), 1.30-1.09 (1H, m), 1.01 (3H, d), 0.96-0.81 (1H, m), 0.76 (3H, t).
LC-MS (Methode 7): Rₜ = 2.62 min; m/z = 542 (M+H)⁺.

### Beispiel 78 (Isomer 2):

Rₜ 8.53 min; Reinheit >99%; >98% ee (analytische Säule s.o.)
Ausbeute: 66 mg
LC-MS (Methode 7): Rₜ = 2.62 min; m/z = 542 (M+H)⁺.

### Beispiel 79 (Isomer 3):

Rₜ 8.84 min; Reinheit >99%; >98% ee (analytische Säule s.o.)
Ausbeute: 37 mg
LC-MS (Methode 7): Rₜ = 2.62 min; m/z = 542 (M+H)⁺.

### Beispiel 80 (Isomer 4):

Rₜ 8.43 min; Reinheit >99%; >98% ee (analytische Säule s.o.)
Ausbeute: 37 mg
LC-MS (Methode 7): Rₜ = 2.62 min; m/z = 542 (M+H)⁺.

### Beispiel 81

### 2-Methyl-4-[(3-methyl-2-{4-[(5-oxo-2-phenyl-5,6-dihydro-4H-1,3,4-oxadiazin-4-yl)methyl]-phenyl}pentanoyl)amino]-2,3-dihydro-1H-inden-2-carbonsäure (Isomerengemisch)

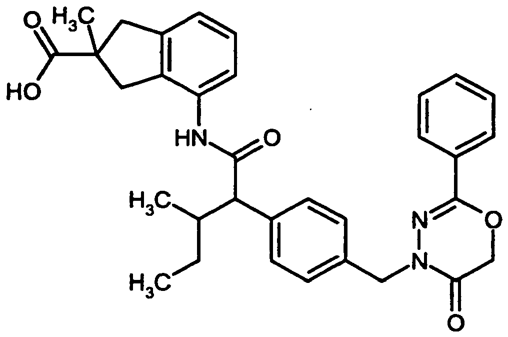

Eine Lösung von 339 mg (0.60 mmol) Methyl-2-methyl-4-[(3-methyl-2-{4-[(5-oxo-2-phenyl-5,6-dihydro-4*H*-1,3,4-oxadiazin-4-yl)methyl]phenyl}pentanoyl)amino]-2,3-dihydro-1*H*-inden-2-carboxylat (Beispiel 160A) in 12 ml THF und 6 ml Wasser wurde mit 100 mg (2.39 mmol) Lithiumhydroxid-Monohydrat versetzt und über Nacht bei Raumtemperatur gerührt. Nach vollständiger Umsetzung wurde das THF im Vakuum abgezogen, die Reaktionslösung mit Wasser verdünnt und anschließend mit 1 M Salzsäure auf pH 2 gestellt. Der ausgefallene Feststoff wurde abfiltriert, mit Wasser gewaschen und über Nacht im Vakuum bei 45°C getrocknet. Es wurden 305 mg (0.55 mmol, 92% d. Th.) der Titelverbindung als Isomerengemisch erhalten.
LC-MS (Methode 10): Rₜ = 2.13 min; m/z = 542 (M+H)⁺.

### Beispiele 82 - 88

### 2-Methyl-4-[(3-methyl-2-{4-[(5-oxo-2-phenyl-5,6-dihydro-4H-1,3,4-oxadiazin-4-yl)methyl]-phenyl}pentanoyl)amino]-2,3-dihydro-1H-inden-2-carbonsäure (Isomer 1 - 7)

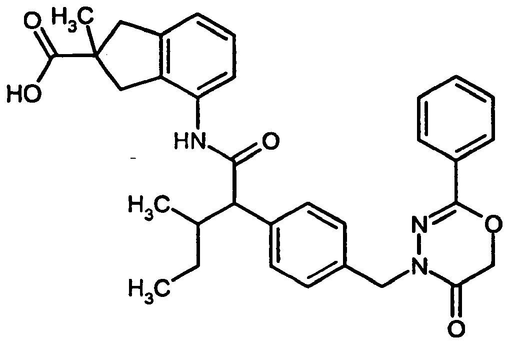

300 mg (0.54 mmol) des erhaltenen Isomerengemisches von 2-Methyl-4-[(3-methyl-2-{4-[(5-oxo-2-phenyl-5,6-dihydro-4*H*-1,3,4-oxadiazin-4-yl)methyl]phenyl}pentanoyl)amino]-2,3-dihydro-1H-inden-2-carbonsäure (Beispiel 81) wurden mittels präparativer HPLC an chiraler Phase weiter aufgetrennt [Säule: Daicel Chiralpak AD-H, 5 µm, 250 mm x 20 mm; Elutionsmittel: Isohexan/(Isopropanol + 0.2% Trifluoressigsäure +1% Wasser) 70:30 (v/v); Fluss: 15 ml/min; UV-Detektion: 220 nm; Temperatur: 30°C]. Es wurden 7 Fraktionen erhalten, die unter gleichen Bedingungen nochmals nachchromatographiert wurden.

### Beispiel 82 (Isomer 1):

Rₜ 7.89 min; Reinheit >94% (Säule s.o.)
Ausbeute: 16 mg
LC-MS (Methode 10): Rₜ = 2.32 min; m/z = 554 (M+H)⁺.

### Beispiel 83 (Isomer 2):

Rₜ 8.42 min; Reinheit >98.5% (Säule s.o.)
Ausbeute: 84 mg
LC-MS (Methode 10): Rₜ = 2.32 min; m/z = 554 (M+H)⁺.

### Beispiel 84 (Isomer 3):

Rₜ 9.22 min; Reinheit >98% (Säule s.o.)
Ausbeute: 14 mg
LC-MS (Methode 10): Rₜ = 2.32 min; m/z = 554 (M+H)⁺.

### Beispiel 85 (Isomer 4):

Rₜ 11.61 min; Reinheit >96.5% (Säule s.o.)
Ausbeute: 25 mg
LC-MS (Methode 10): Rₜ = 2.32 min; m/z = 554 (M+H)⁺.

### Beispiel 86 (Isomer 5):

Rₜ 13.40 min; Reinheit >97.3% (Säule s.o.)
Ausbeute: 66 mg
¹H-NMR (400 MHz, DMSO-d₆, δ/ppm): 12.6-11.9 (1H, breites s), 9.46 (1H, s), 7.76 (2H, d), 7.53-7.45 (3H, m), 7.39 (2H, d), 7.32 (3H, d), 7.04 (1H, m), 6.93 (1H, d), 4.91 (2H, s), 4.86 (2H, s), 3.49 (1H, d), 3.34-3.20 (2H, m), 2.75-2.61 (2H, m), 2.22-2.07 (1H, m), 1.63-1.49 (1H, m), 1.31-1.15 (1H, m), 1.23 (3H, s), 0.92 (3H, t), 0.62 (3H, d).
LC-MS (Methode 10): Rₜ = 2.32 min; m/z = 554 (M+H)⁺.

### Beispiel 87 (Isomer 6):

Rₜ 14.78 min; Reinheit >97.2% (Säule s.o.)
Ausbeute: 14 mg
LC-MS (Methode 10): Rₜ = 2.32 min; m/z = 554 (M+H)⁺.

### Beispiel 88 (Isomer 7):

Rₜ 16.60 min; Reinheit >95.5% (Säule s.o.)
Ausbeute: 52 mg
LC-MS (Methode 10): Rₜ = 2.32 min; m/z = 554 (M+H)⁺.

### Beispiel 89

### 3-{2-Methyl-3-[(3-methyl-2-{4-[(5-oxo-2-phenyl-5,6-dihydro-4H-1,3,4-oxadiazin-4-yl)methyl]-phenyl}hexanoyl)amino]phenyl}propansäure (Isomerengemisch)

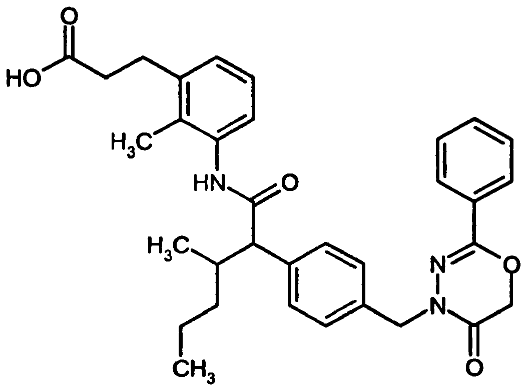

Eine Lösung von 460 mg (0.75 mmol) *tert*.-Butyl-3-{2-methyl-3-[(3-methyl-2-{4-[(5-oxo-2-phenyl-5,6-dihydro-4*H*-1,3,4-oxadiazin-4-yl)methyl]phenyl}hexanoyl)amino]phenyl}propanoat (Beispiel 163A) in 10 ml Dichlormethan wurde mit 1.74 ml (22.56 mmol) Trifluoressigsäure versetzt und 2 h bei Raumtemperatur gerührt. Der Ansatz wurde dann im Vakuum bis zur Trockene eingeengt. Es wurden 410 mg (0.74 mmol, 98% d. Th.) der Titelverbindung als Isomerengemisch erhalten.
LC-MS (Methode 11): Rₜ = 1.40 min; m/z = 556 (M+H)⁺.

### Beispiele 90 - 93

### 3-{2-Methyl-3-[(3-methyl-2-{4-[(5-oxo-2-phenyl-5,6-dihydro-4H-1,3,4-oxadiazin-4-yl)methyl]-phenyl}hexanoyl)amino]phenyl}propansäure (Isomer 1 - 4)

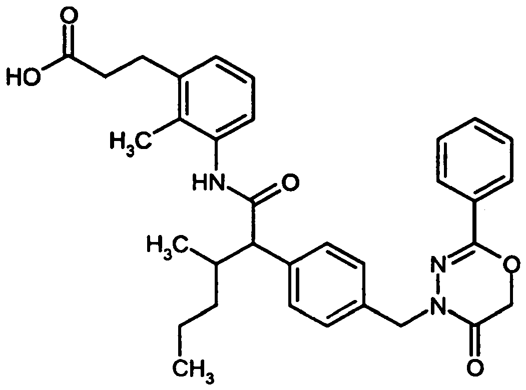

400 mg (0.72 mmol) des erhaltenen Isomerengemisches von 3-{2-Methyl-3-[(3-methyl-2-{4-[(5-oxo-2-phenyl-5,6-dihydro-4*H*-1,3,4-oxadiazin-4-yl)methyl]phenyl}hexanoyl)amino]phenyl}propansäure (Beispiel 89) wurden mittels präparativer HPLC an chiraler Phase weiter aufgetrennt [Säule: Daicel Chiralpak AD-H, 5 µm, 250 mm x 20 mm; Elutionsmittel: Isohexan/(Isopropanol + 0.2% Trifluoressigsäure + 1% Wasser) 70:30 (v/v); Fluss: 15 ml/min; UV-Detektion: 220 nm; Temperatur: 40°C]. Es wurden vier Fraktionen erhalten, die durch erneute HPLC-Chromatographie an chiraler Phase nachgereinigt wurden [Bedingungen wie oben, oder Säule: Daicel Chiralpak IA, 5 µm, 250 mm x 20 mm; Elutionsmittel: Methanol/(*tert*.-Butylmethylether + 0.2% Eisessig) 7:93 (v/v); Fluss: 15 ml/min; UV-Detektion: 220 nm; Temperatur: 40°C].

### Beispiel 90 (Isomer 1):

Rₜ 12.02 min; Reinheit >99%; >99% ee
[Säule: Daicel Chiralpak AD-H, 5 µm, 250 mm x 4.6 mm; Elutionsmittel: Isohexan/(Isopropanol + 0.2% Trifluoressigsäure + 1 % Wasser) 80:20 (v/v); Fluss: 2 ml/min; UV-Detektion: 220 nm; Temperatur: 30°C]
Ausbeute: 94 mg
¹H-NMR (400 MHz, DMSO-d₆, δ/ppm): 12.14 (1H, s), 9.48 (1H, s), 7.76 (2H, d), 7.53-7.41 (3H, m), 7.38 (2H, d), 7.31 (2H, d), 7.05-6.92 (3H, m), 4.92 (2H, s), 4.87 (2H, s), 3.40 (1H, d), 2.76 (2H, t), 2.41 (2H, t), 2.29-2:16 (1H, m), 1.94 (3H, s), 1.59-1.40 (2H, m), 1.34-1.18 (2H, m), 0.89 (3H, t), 0.63 (3H, d).
LC-MS (Methode 10): Rₜ = 2.29 min; m/z = 556 (M+H)⁺.

### Beispiel 91 (Isomer 2):

Rₜ 15.06 min; Reinheit >99%; >99% ee (analytische Säule s.o.)
Ausbeute: 95 mg
LC-MS (Methode 10): Rₜ = 2.29 min; m/z = 556 (M+H)⁺.

### Beispiel 92 (Isomer 3):

Rₜ 16.42 min; Reinheit >99%; >99% ee (analytische Säule s.o.)
Ausbeute: 32 mg
LC-MS (Methode 10): Rₜ = 2.29 min; m/z = 556 (M+H)⁺.

### Beispiel 93 (Isomer 4):

Rₜ 13.57 min; Reinheit >99%; >99% ee (analytische Säule s.o.)
Ausbeute: 40 mg
LC-MS (Methode 10): Rₜ = 2.29 min; m/z = 556 (M+H)⁺.

### Beispiel 94

### (2E)-3-{3-[(Cyclopentyl {4-[(5-oxo-2-phenyl-5,6-dihydro-4H-1,3,4-oxadiazin-4-yl)methyl]phenyl}-acetyl)amino]phenyl}prop-2-ensäure (Enantiomer 1)

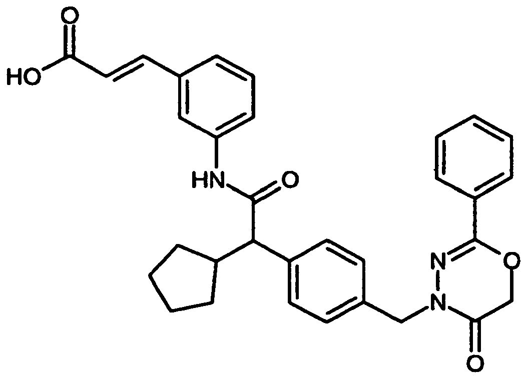

### Darstellungsmethode 5

827 mg (1.461 mmol) Ethyl-(2*E*)-3-{3-[(cyclopentyl{4-[(5-oxo-2-phenyl-5,6-dihydro-4*H*-1,3,4-oxadiazin-4-yl)methyl]phenyl}acetyl)amino]phenyl}prop-2-enoat (Beispiel 174A) wurden in 28 ml Dioxan/Wasser (3:1 v/v) gelöst und mit 2.2 ml 1 N Natronlauge versetzt. Der Ansatz wurde über Nacht bei Raumtemperatur gerührt. Das Reaktionsgemisch wurde dann mit 1 N Salzsäure auf pH 1 angesäuert und mehrmals mit Essigsäureethylester extrahiert. Die vereinigten organischen Phasen wurden mit gesättigter Natriumchlorid-Lösung gewaschen, über Natriumsulfat getrocknet und im Vakuum eingeengt. Es wurden 780 mg (95% d. Th.) der Zielverbindung erhalten.
LC-MS (Methode 10): Rₜ = 2.52 min; m/z = 538 (M+H)⁺.

Auf analoge Weise wurden die in der folgenden Tabelle aufgeführten Verbindungen erhalten:

| **Beispiel** | **Name / Struktur Darstellungsmethode / Edukt** | **Analytische Daten** |
|---|---|---|
| **95** | (2*E*)-3-{2-[(Cyclopentyl{4-[(5-oxo-2-phenyl-5,6-dihydro-4*H*-1,3,4-oxadiazin-4-yl)methyl]phenyl}-acetyl)amino]phenyl}prop-2-ensäure (*Enantiomer 1*) | LC-MS (Methode 11): Rₜ = 1.33 min; m/z = 538 (M+H)⁺. |
| | 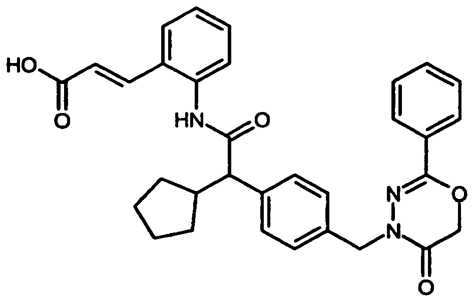 | |
| | Darstellungsmethode 5 (aus Bsp. 109A) | |
| **96** | (2*E*)-3-{4-[(Cyclopentyl{4-[(5-oxo-2-phenyl-5,6-dihydro-4*H*-1,3,4-oxadiazin-4-yl)methyl]phenyl}-acetyl)amino]phenyl}prop-2-ensäure (*Enantiomer 1*) | LC-MS (Methode 12): Rₜ = 2.56 min; m/z = 538 (M+H)⁺. |
| | 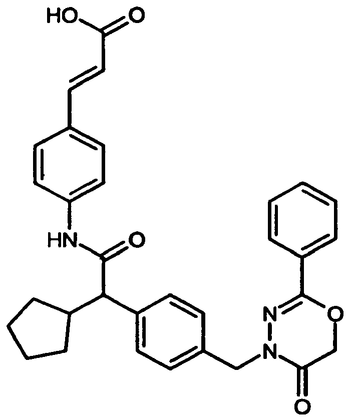 | |
| | Darstellungsmethode 5 (aus Bsp. 111A) | |
| **97** | (2*E*)-3-{2-[(3-Methyl-2-{4-[(5-oxo-2-phenyl-5,6-dihydro-4*H*-1,3,4-oxadiazin-4-yl)methyl]phenyl} -butanoyl)amino]phenyl}prop-2-ensäure (*Enantiomer 1*) | LC-MS (Methode 10): Rₜ = 2.04 min; m/z = 512 (M+H)⁺. |
| | 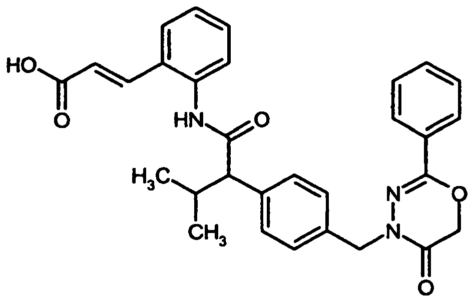 | |
| | Darstellungsmethode 5 (aus Bsp. 192A) | |
| **98** | (2*E*)-3-{4-[(3-Methyl-2-{4-[(5-oxo-2-phenyl-5,6-dihydro-4*H*-1,3,4-oxadiazin-4-yl)methyl]phenyl}-butanoyl)amino]phenyl}prop-2-ensäure (*Enantiomer 1*) | LC-MS (Methode 11): Rₜ = 1.29 min; m/z = 512 (M+H)⁺. |
| | 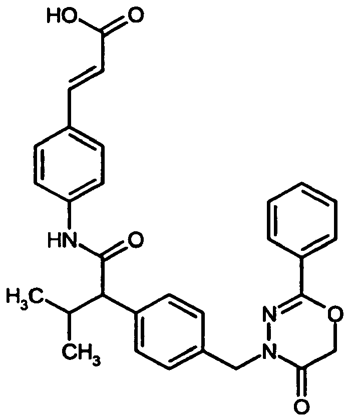 | |
| | Darstellungsmethode 5 (aus Bsp. 176A) | |
| **99** | (2*E*)-3-{3-[(2-{4-[(2-*tert*.-Butyl-5-oxo-5,6-dihydro-4H-1,3,4-oxadiazin-4-yl)methyl]phenyl}-3-methyl-butanoyl)amino]phenyl}prop-2-ensäure (*Racemat*) | LC-MS (Methode 7): Rₜ = 2.65 min; m/z = 492 (M+H)⁺. |
| | 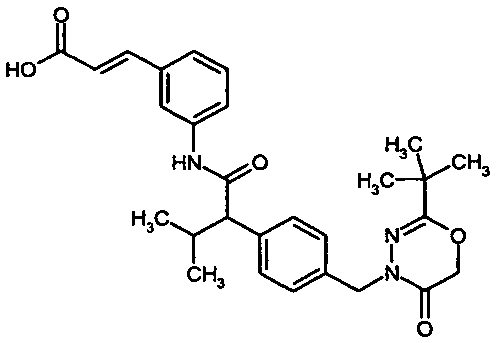 | |
| | Darstellungsmethode 5 (aus Bsp. 178A) | |

### Beispiel 100

### 3-{4-[(Cyclopentyl{4-[(5-oxo-2-phenyl-5,6-dihydro-4H-1,3,4-oxadiazin-4-yl)methyl]phenyl}-acetyl)amino]phenyl}propansäure (Enantiomer 1)

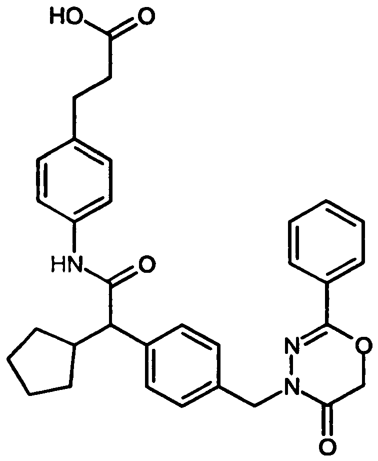

Eine Lösung von 46 mg (0.085 mmol) (2*E*)-3-{4-[(Cyclopentyl{4-[(5-oxo-2-phenyl-5,6-dihydro-4*H*-1,3,4-oxadiazin-4-yl)methyl]phenyl}acetyl)amino]phenyl}prop-2-ensäure (Beispiel 96) in 10 ml Ethanol wurde mit 50 mg Palladium auf Kohle (10%) versetzt. Unter einer Wasserstoffatmosphäre wurde bei Normaldruck für 2 h hydriert. Das Reaktionsgemisch wurde danach über Tonsil filtriert, der Filter-Rückstand mit Ethanol nachgewaschen und die vereinigten Filtrate am Rotationsverdampfer eingeengt. Das Rohprodukt wurde mittels präparativer HPLC gereinigt. Es wurden 29 mg (63% d. Th.) der Titelverbindung erhalten.
LC-MS (Methode 11): Rₜ = 1.35 min; m/z = 540 (M+H)⁺.

Auf analoge Weise wurden die in der folgenden Tabelle aufgeführten Verbindungen erhalten:

| **Beispiel** | **Name / Struktur / Edukt** | **Analytische Daten** |
|---|---|---|
| **101** | 3-{2-[(Cyclopentyl{4-[(5-oxo-2-phenyl-5,6-dihydro-4*H*-1,3,4-oxadiazin-4-yl)methyl]phenyl}acetyl)amino]-phenyl}propansäure (*Enantiomer 1*) | LC-MS (Methode 11): Rₜ = 1.33 min; m/z = 540 (M+H)⁺. |
| | 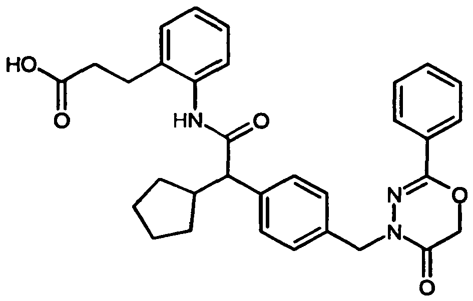 | |
| | (aus Bsp. 95) | |
| **102** | 3-{3-[(2-{4-[(2-*tert*.-Butyl-5-oxo-5,6-dihydro-4*H*-1,3,4-oxadiazin-4-yl)methyl]phenyl} -3-methylbutanoyl)-amino]phenyl}propansäure (*Racemat*) | LC-MS (Methode 11): Rₜ = 1.32 min; m/z = 494 (M+H)⁺. |
| | 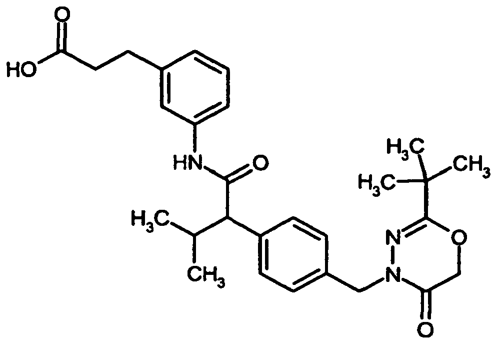 | |
| | (aus Bsp. 99) | |

### Beispiel 103

### 3-{3-[(Cyclopentyl{4-[(5-oxo-2-phenyl-5,6-dihydro-4H-1,3,4-oxadiazin-4-yl)methyl]phenyl}-acetyl)amino]phenyl}propansäure (Enantiomer 1)

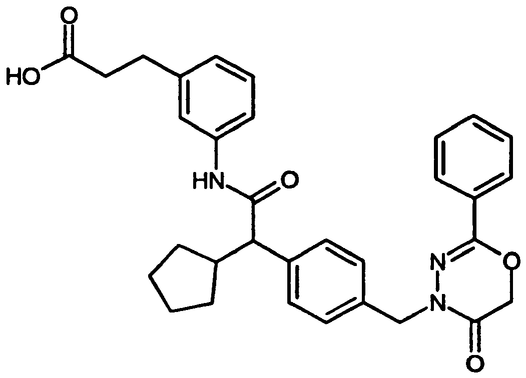

### Darstellungsmethode 6

Eine Lösung von 4.18 g (7.363 mmol) Ethyl-3-{3-[(cyclopentyl{4-[(5-oxo-2-phenyl-5,6-dihydro-4*H*-1,3,4-oxadiazin-4-yl)methyl]phenyl}acetyl)amino]phenyl}propanoat (Beispiel 180A) in 195 ml Dioxan/Wasser (4:1 v/v) wurde mit 11.05 ml 1 N Natronlauge versetzt. Das Gemisch wurde über Nacht bei Raumtemperatur gerührt, dann mit 1 N Salzsäure auf pH 1 angesäuert und mehrmals mit Essigsäureethylester extrahiert. Die vereinigten organischen Phasen wurden mit gesättigter Natriumchlorid-Lösung gewaschen, über Natriumsulfat getrocknet und am Rotationsverdampfer eingeengt. Das Rohprodukt wurde in Essigsäureethylester gelöst und über Kieselgel filtriert. Das Filtrat wurde mit Aktivkohle versetzt, auf Rückfluss erhitzt und über Tonsil filtriert. Nach dem Einengen im Vakuum wurden 3.20 g (81% d. Th.) der Titelverbindung erhalten.
LC-MS (Methode 11): Rₜ = 1.36 min; m/z = 540 (M+H)⁺.

Auf analoge Weise wurden die in der folgenden Tabelle aufgeführten Verbindungen erhalten:

| **Beispiel** | **Name / Struktur Darstellungsmethode / Edukt** | **Analytische Daten** |
|---|---|---|
| **104** | {3-[(Cyclopentyl {4-[(5-oxo-2-phenyl-5,6-dihydro-4*H* 1,3,4-oxadiazin-4-yl)methyl]phenyl}acetyl)-amino]phenyl}essigsäure (*Enantiomer 1*) | LC-MS (Methode 10): Rₜ = 2.16 min; m/z = 526 (M+H)⁺. |
| | 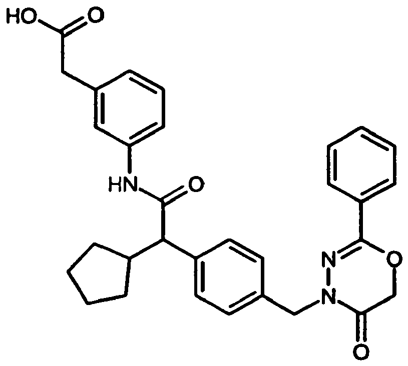 | |
| | Darstellungsmethode 6 (aus Bsp. 110A) | |
| **105** | 3-{3-[(3-Methyl-2-{4-[(5-oxo-2-phenyl-5,6-dihydro-4*H*-1,3,4-oxadiazin-4-yl)methyl]phenyl}butanoyl)-amino]phenyl}propansäure (*Enantiomer 1*) | ¹H-NMR (400 MHz, DMSO-d₆, δ/ppm): 12.25-11.95 (1H, breites s), 10.01 (1H, s), 7.76 (2H, d), 7.53-7.35 (7H, m), 7.32 (2H, d), 7.14 (1H, t), 6.86 (1H, d), 4.90 (2H, s), 4.84 (2H, s), 3.22 (1H, d), 2.74 (2H, t), 2.48 (2H, t), 2.40-2.22 (1H, m), 1.03 (3H, d), 0.65 (3H, d). LC-MS (Methode 11): Rₜ = 1.29 min; m/z = 514 (M+H)⁺. |
| | 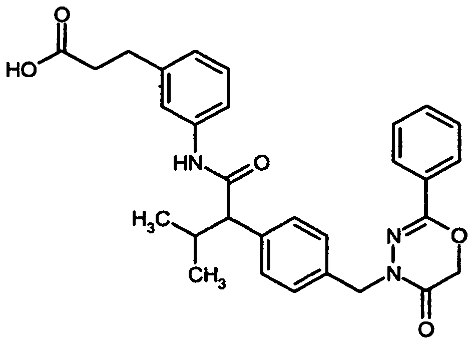 | |
| | Darstellungsmethode 6 (aus Bsp. 193A) | |
| **106** | 3-{4-[(3-Methyl-2-{4-[(5-oxo-2-phenyl-5,6-dihydro-4*H*-1,3,4-oxadiazin-4-yl)methyl]phenyl}butanoyl)-amino]phenyl}propansäure (*Enantiomer 1*) | LC-MS (Methode 10): Rₜ = 2.07 min; m/z = 514 (M+H)⁺. |
| | 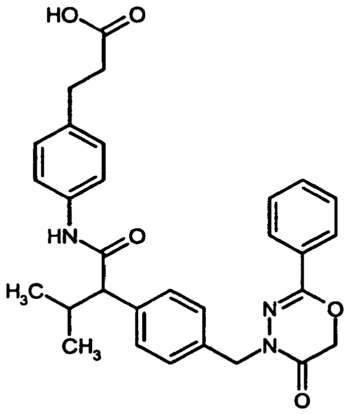 | |
| | Darstellungsmethode 6 (aus Bsp. 186A) | |
| **107** | ({3-[(3-Methyl-2-{4-[(5-oxo-2-phenyl-5,6-dihydro-4*H*-1,3,4-oxadiazin-4-yl)methyl]phenyl}butanoyl)-amino]phenyl}sulfanyl)essigsäure (*Enantiomer 1*) | ¹H-NMR (400 MHz, DMSO-d₆, δ/ppm): 12.85-12.65 (1H, breites s), 10.11 (1H, s), 7.77 (2H, d), 7.60 (1H, s), 7.53-7.40 (3H, m), 7.40-7.28 (5H, m), 7.19 (1H, t), 6.97 (1H, d), 4.90 (2H, s), 4.84 (2H, s), 3,74 (2H, s), 3.21 (1H, d), 2.39-2.25 (1H, m), 0.99 (3H, d), 0.66 (3H, d). LC-MS (Methode 11): Rₜ= 1.29 min; m/z = 532 (M+H)⁺. |
| | 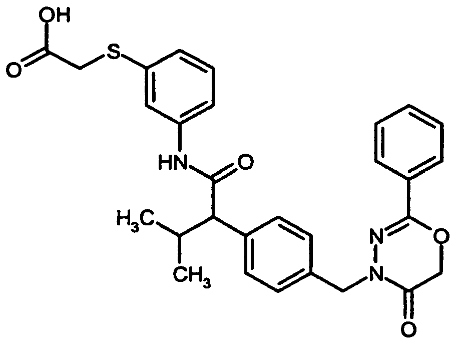 | |
| | Darstellungsmethode 2 (aus Bsp. 137A) | |
| **108** | ({3-[(Cyclopentyl{4-[(5-oxo-2-phenyl-5,6-dihydro-4*H*-1,3,4-oxadiazin-4-yl)methyl]phenyl}acetyl)-amino]phenyl}sulfanyl)essigsäure (*Enantiomer 1*) | ¹H-NMR (400 MHz, DMSO-d₆, δ/ppm): 12.85-12.60 (1H, breites s), 10.09 (1H, s), 7.76 (2H, d), 7.60 (1H, s), 7.53-7.28 (8H, m), 7.20 (1H, t), 6.97 (1H, d), 4.90 (2H, s), 4.84 (2H, s), 3.74 (2H, s), 3.36 (1H, d), 2.64-2.47 (1H, m), 1.86-1.71 (1H, m), 1.71-1.15 (6H, m), 1.03-0.90 (1H, m). LC-MS (Methode 11): Rₜ 1.36 min; m/z = 558 (M+H)⁺. |
| | 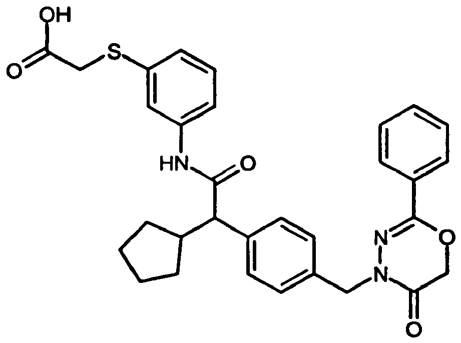 | |
| | Darstellungsmethode 2 (aus Bsp. 138A) | |
| **109** | 5-{[(Cyclopentyl {4-[(5-oxo-2-phenyl-5,6-dihydro-4*H* 1,3,4-oxadiazin-4-yl)methyl]phenyl}acetyl)amino]-methyl}isoxazol-3-carbonsäure (*Enantiomer 1*) | ¹H-NMR (400 MHz, DMSO-d₆, δ/ppm): 14.2-13.8 (1H, breites s), 8.72 (1H, t), 7.77 (2H, d), 7.53-7.40 (3H, m), 7.38-7.22 (4H, m), 6.43 (1H, s), 4.91 (2H, s), 4.84 (2H, s), 4.54-4.40 (1H, m), 4.38-4.22 (1H, m), 3.21 (1H, d), 2.60-2.41 (1H, m), 1.79-1.11 (7H, m), 1.01-0.84 (1H, m). LC-MS (Methode 7): Rₜ = 2.97 min; m/z = 517 (M+H)⁺. |
| | 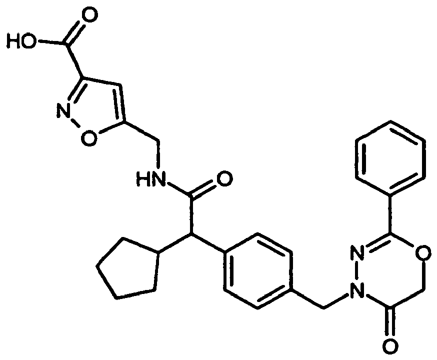 | |
| | Darstellungsmethode 2 (aus Bsp. 139A) | |
| **110** | 4-{3-[(Cyclopentyl{4-[(5-oxo-2-phenyl-5,6-dihydro-4*H*-1,3,4-oxadiazin-4-yl)methyl]phenyl}acetyl)-amino]phenyl}butansäure (*Enantiomer 1*) | LC-MS (Methode 11): Rₜ = 1.39 min; m/z = 554 (M+H)⁺. |
| | 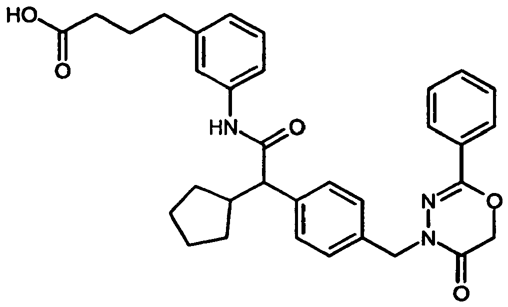 | |
| | Darstellungsmethode 1 (aus Bsp. 179A) | |
| **111** | 2-{3-[(Cyclopentyl{4-[(5-oxo-2-phenyl-5,6-dihydro-4*H*-1,3,4-oxadiazin-4-yl)methyl]phenyl}acetyl)-amino]-2-methylphenoxy}-2-methylpropansäure (*Enantiomer 1*) | LC-MS (Methode 11): Rₜ = 1.40 min; m/z = 584 (M+H)⁺. |
| | 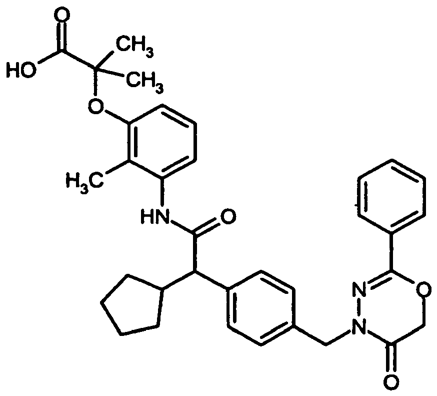 | |
| | Darstellungsmethode 1 (aus Bsp. 177A) | |
| **112** | 2-Methyl-2-({3-[(3-methyl-2-{4-[(5-oxo-2-phenyl-5,6-dihydro-4*H*-1,3,4-oxadiazin-4-yl)methyl]phenyl}-butanoyl)amino]phenyl} sulfanyl)propansäure (*Enantiomer 1*) | ¹H-NMR (400 MHz, DMSO-d₆, δ/ppm): 12.70-12.45 (1H, breites s), 10.17 (1H, s), 7.81-7.71 (3H, m), 7.59 (1H, d), 7.53-7.41 (3H, m), 7.38 (2H, d), 7.31 (2H, d), 7.26 (1H, t), 7.09 (1H, d), 4.90 (2H, s), 4.85 (2H, s), 3.22 (1H, d), 2.39-2.25 (1H, m), 1.36 (6H, s), 1.00 (3H, d), 0.66 (3H, d). LC-MS (Methode 10): Rₜ = 2.32 min; m/z = 560 (M+H)⁺. |
| | 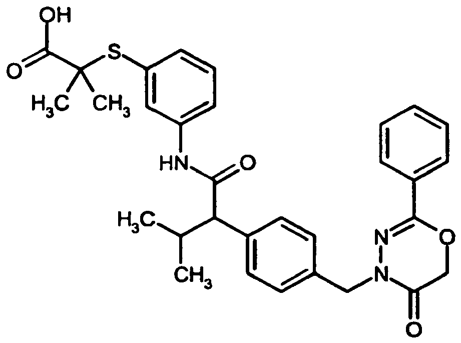 | |
| | Darstellungsmethode 2 (aus Bsp. 140A) | |
| **113** | 2-({3-[(Cyclopentyl {4-[(5-oxo-2-phenyl-5,6-dihydro-4*H*-1,3,4-oxadiazin-4-yl)methyl]phenyl}acetyl)-amino]phenyl} sulfanyl)-2-methylpropansäure (*Enantiomer 1*) | ¹H-NMR (400 MHz, DMSO-d₆, δ/ppm): 12.70-12.45 (1H, breites s), 10.15 (1H, s), 7.75 (3H, t), 7.58 (1H, d), 7.53-7.40 (3H, m), 7.39 (2H, d), 7.32 (2H, d), 7.26 (1H, t), 7.09 (1H, d), 4.90 (2H, s), 4.84 (2H, s), 3.45-3.29 (1H, d), 2.65-2.47 (1H, m), 1.84-1.71 (1H, m), 1.69-1.41 (4H, m), 1.40-1.30 (1H, m), 1.36 (6H, s), 1.29-1.19 (1H, m), 1.06-0.89 (1H, m). LC-MS (Methode 10): Rₜ = 2.43 min; m/z = 586 (M+H)⁺. |
| | 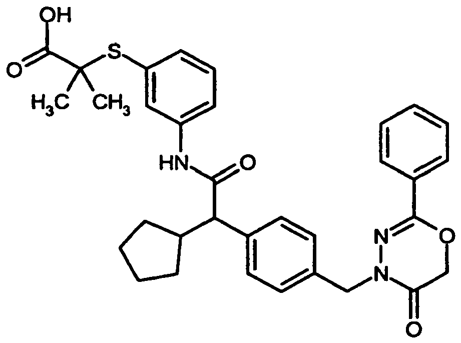 | |
| | Darstellungsmethode 2 (aus Bsp. 141A) | |
| **114** | 2-{3-[(Cyclopentyl{4-[(5-oxo-2-phenyl-5,6-dihydro-4*H*-1,3,4-oxadiazin-4-yl)methyl]phenyl}acetyl)-amino]phenyl}-2-methylpropansäure *(Enantiomer 1)* | LC-MS (Methode 10): Rₜ = 2.30 min; m/z = 554 (M+H)⁺. |
| | 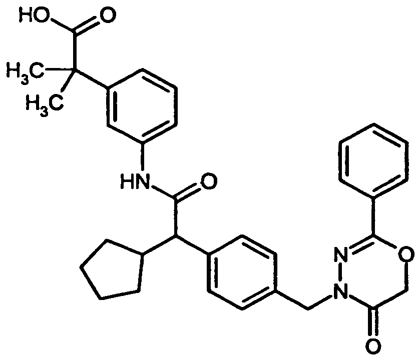 | |
| | Darstellungsmethode 1 (aus Bsp. 181A) | |
| **115** | 3-{3-[(Cyclopentyl{4-[(5-oxo-2-phenyl-5,6-dihydro-4*H*-1,3,4-oxadiazin-4-yl)methyl]phenyl}acetyl)-amino]-2-methylphenyl}propansäure (*Enantiomer 1*) | ¹H-NMR (400 MHz, DMSO-d₆, δ/ppm): 12.14 (1H, s), 9.46 (1H, s), 7.77 (2H, d), 7.53-7.38 (5H, m), 7.31 (2H, d), 7.05-6.93 (3H, m), 4.92 (2H, s), 4.87 (2H, s), 3.45 (1H, d), 2.77 (2H, t), 2.65-2.47 (1H, m), 2.41 (2H, t), 1.96 (3H, s), 1.89-1.77 (1H, m), 1.74-1.27 (6H, m), 1.05-0.91 (1H, m). LC-MS (Methode 11): Rₜ = 1.36 min; m/z = 554 (M+H)⁺. |
| | 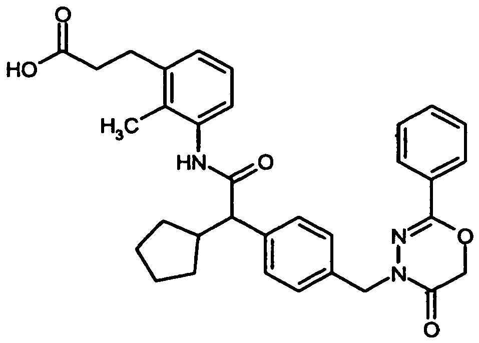 | |
| | Darstellungsmethode 3 (aus Bsp. 175A) | |
| **116** | 3-{*trans*-4-[(3-Methyl-2-{4-[(5-oxo-2-phenyl-5,6-dihydro-4*H*-1,3,4-oxadiazin-4-yl)methyl]phenyl}-butanoyl)amino]cyclohexyl}propansäure (*Enantiomer 1*) | ¹H-NMR (400 MHz, DMSO-d₆, δ/ppm): 12.05-11.87 (1H, breites s), 7.83 (1H, d), 7.76 (2H, d), 7.53-7.40 (3H, m), 7.32-7.22 (4H, m), 4.91 (2H, s), 4.84 (2H, s), 3.42-3.28 (1H, m), 2.93 (1H, d), 2.26-2.12 (3H, m), 1.87-1.76 (1H, m), 1.72-1.49 (3H, m), 1.41-1.32 (2H, m), 1.18-0.96 (3H, m), 0.96-0.77 (2H, m), 0.91 (3H, d), 0.59 (3H, d). LC-MS (Methode 10): Rₜ = 2.03 min; m/z = 520 (M+H)⁺. |
| | 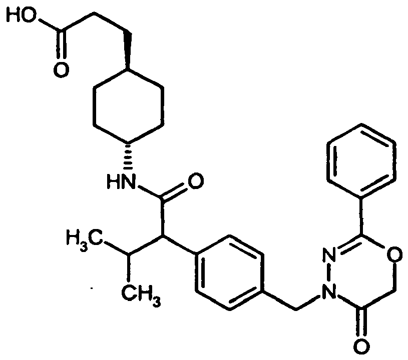 | |
| | Darstellungsmethode 2 (aus Bsp. 144A) | |
| **117** | 3-{cis-4-[(3-Methyl-2-{4-[(5-oxo-2-phenyl-5,6-dihydro-4*H*-1,3,4-oxadiazin-4-yl)methyl]phenyl}-butanoyl)amino]cyclohexyl}propansäure (*Enantiomer 1*) | ¹H-NMR (400 MHz, DMSO-d₆, δ/ppm): 12.05-11.90 (1H, breites s), 7.82-7.72 (3H, m), 7.53-7.41 (3H, m), 7.35-7.22 (4H, m), 4.91 (2H, s), 4.84 (2H, s), 3.73-3.62 (1H, m), 3.11 (1H, d), 2.27-2.12 (3H, m), 1.63-1.50 (1H, m), 1.50-1.20 (10H, m), 0.92 (3H, d), 0.61 (3H, d). LC-MS (Methode 10): Rₜ = 2.04 min; m/z = 520 (M+H)⁺. |
| | 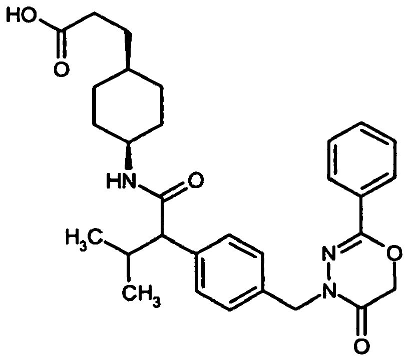 | |
| | Darstellungsmethode 2 (aus Bsp. 145A) | |
| **118** | 3- {*trans*-4-[(Cyclopentyl {4-[(5-oxo-2-phenyl-5,6-dihydro-4*H*-1,3,4-oxadiazin-4-yl)methyl]phenyl}-acetyl)amino]cyclohexyl}propansäure (*Enantiomer 1*) | ¹H-NMR (400 MHz, DMSO-d₆, δ/ppm): 12.2-11.8 (1H, breites s), 7.81 (1H, d), 7.77 (2H, d), 7.54-7.40 (3H, m), 7.28 (4H, q), 4.91 (2H, s), 4.83 (2H, s), 3.44-3.24 (1H, m), 3.08 (1H, d), 2.57-2.38 (1H, m), 2.17 (2H, t), 1.85-1.74 (1H, m), 1.74-0.96 (15H, m), 0.96-0.77 (3H, m). LC-MS (Methode 7): Rₜ = 2.61 min; m/z = 546 (M+H)⁺. |
| | 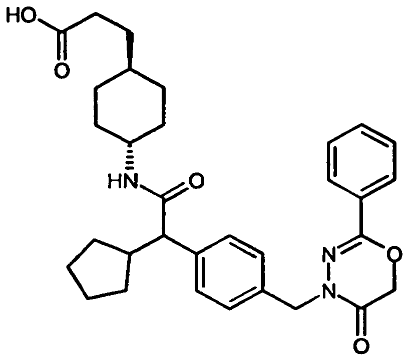 | |
| | Darstellungsmethode 2 (aus Bsp. 148A) | |
| **119** | 3- {*cis*-4-[(Cyclopentyl{4-[(5-oxo-2-phenyl-5,6-dihydro-4*H*-1,3,4-oxadiazin-4-yl)methyl]phenyl}-acetyl)amino]cyclohexyl}propansäure (*Enantiomer 1*) | ¹H-NMR (400 MHz, DMSO-d₆, δ/ppm): 11.98 (1H, s), 7.77 (3H, d), 7.54-7.41 (3H, m), 7.33 (2H, d), 7.26 (2H, d), 4.91 (2H, s), 4.84 (2H, s), 3.74-3.62 (1H, m), 3.26 (1H, d), 2.57-2.39 (1H, m), 2.19 (2H, t), 1.72-1.20 (17H, m), 0.99-0.79 (2H, m). LC-MS (Methode 7): Rₜ = 2.60 min; m/z = 546 (M+H)⁺. |
| | 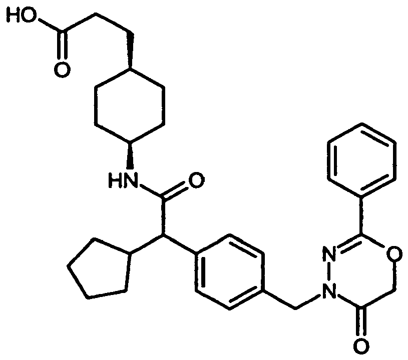 | |
| | Darstellungsmethode 2 (aus Bsp. 149A) | |
| **120** | (1*S*,3*S*)-3-[(Cyclopentyl {4-[(5-oxo-2-phenyl-5,6-dihydro-4*H*-1,3,4-oxadiazin-4-yl)methyl]phenyl}-acetyl)amino]cyclopentancarbonsäure (*Enantiomer 1*) | ¹H-NMR (400 MHz, DMSO-d₆, δ/ppm): 12.05 (1H, s), 7.98 (1H, d), 7.77 (2H, d), 7.53-7.41 (3H, m), 7.29 (4H, q), 4.91 (2H, s), 4.84 (2H, s), 4.01-3.92 (1H, m), 3.09 (1H, d), 2.84-2.72 (1H, m), 2.58-2.39 (1H, m), 1.96-1.79 (3H, m), 1.74-1.11 (10H, m), 0.95-0.81 (1H, m). LC-MS (Methode 7): Rₜ = 2.45 min; m/z = 504 (M+H)⁺. |
| | 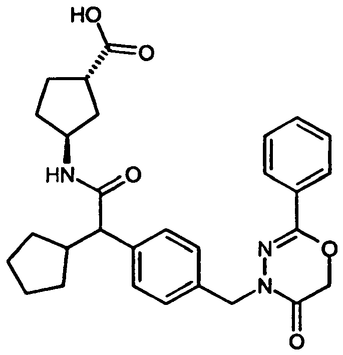 | |
| | Darstellungsmethode 2 (aus Bsp. 152A) | |
| **121** | (1*S*,3*R*)-3-[(Cyclopentyl{4-[(5-oxo-2-phenyl-5,6-dihydro-4*H*-1,3,4-oxadiazin-4-yl)methyl]phenyl}-acetyl)amino]cyclopentancarbonsäure (*Enantiomer 1*) | ¹H-NMR (400 MHz, DMSO-d₆, δ/ppm): 12.06 (1H, s), 8.00 (1H, d), 7.77 (2H, d), 7.53-7.40 (3H, m), 7.28 (4H, q), 4.91 (2H, s), 4.84 (2H, s), 3.97-3.83 (1H, m), 3.10 (1H, d), 2.72-2.60 (1H, m), 2.57-2.38 (1H, m), 2.21-1.98 (1H, m), 1.82-1.25 (11H, m), 1.25-1.12 (1H, m), 0.96-0.81 (1H, m). LC-MS (Methode 7): Rₜ = 2.42 min; m/z = 504 (M+H)⁺. |
| | 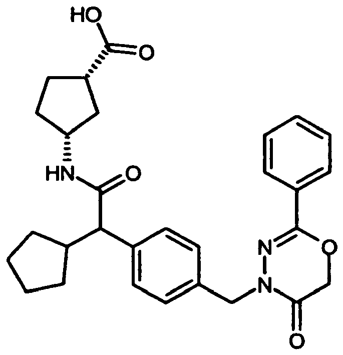 | |
| | Darstellungsmethode 2 (aus Bsp. 153A) | |
| **122** | (1*R*,3*R*)-3-[(Cyclopentyl{4-[(5-oxo-2-phenyl-5,6-dihydro-4*H*-1,3,4-oxadiazin-4-yl)methyl]phenyl}-acetyl)amino]cyclopentancarbonsäure (*Enantiomer 1*) | ¹H-NMR (400 MHz, DMSO-d₆, δ/ppm): 12.06 (1H, s), 7.97 (1H, d), 7.77 (2H, d), 7.53-7.41 (3H, m), 7.28 (4H, q), 4.91 (2H, s), 4.84 (2H, s), 4.03-3.92 (1H, m), 3.09 (1H, d), 2.84-2.72 (1H, m), 2.58-2.39 (1H, m), 1.99-1.11 (13H, m), 0.95-0.81 (1H, m). LC-MS (Methode 7): Rₜ = 2.42 min; m/z = 504 (M+H)⁺. |
| | 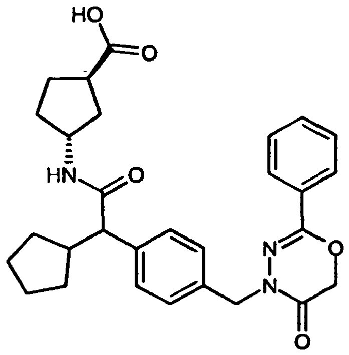 | |
| | Darstellungsmethode 2 (aus Bsp. 154A) | |
| **123** | (1*R*,3*S*)-3-[(Cyclopentyl {4-[(5-oxo-2-phenyl-5,6-dihydro-4*H*-1,3,4-oxadizin-4-yl)methyl]phenyl} -acetyl)amino]cyclopentancarbonsäure (*Enantiomer 1*) | ¹H-NMR (400 MHz, DMSO-d₆, δ/ppm): 12.05 (1H, s), 8.00 (1H, d), 7.77 (2H, d), 7.53-7.40 (3H, m), 7.28 (4H, q), 4.91 (2H, s), 4.84 (2H, s), 3.97-3.83 (1H, m), 3.10 (1H, d), 2.72-2.60 (1H, m), 2.57-2.38 (1H, m), 2.21-1.98 (1H, m), 1.82-1.25 (11H, m), 1.25-1.12 (1H, m), 0.96-0.81 (1H, m). LC-MS (Methode 11): Rₜ = 1.24 min; m/z = 504 (M+H)⁺. |
| | 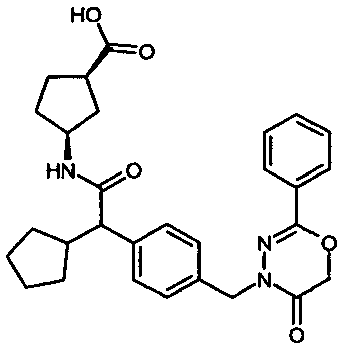 | |
| | Darstellungsmethode 2 (aus Bsp. 155A) | |
| **124** | *cis*-4-[(Cyclopentyl {4-[(5-oxo-2-phenyl-5,6-dihydro-4*H*-1,3,4-oxadiazin-4-yl)methyl]phenyl}acetyl)-amino]cyclohexancarbonsäure (*Enantiomer 1*) | ¹H-NMR (400 MHz, DMSO-d₆, δ/ppm): 12.15-11.92 (1H, breites s), 7.85 (1H, d), 7.77 (2H, d), 7.53-7.40 (3H, m), 7.28 (4H, q), 4.91 (2H, s), 4.83 (2H, s), 3.45-3.29 (1H, m), 3.09 (1H, d), 2.56-2.39 (1H, m), 2.25-2.04 (1H, m), 1.92-1.77 (3H, m), 1.75-1.63 (1H, m), 1.63-1.01 (10H, m), 0.95-0.81 (1H, m). LC-MS (Methode 11): Rₜ = 1.28 min; m/z = 518 (M+H)⁺. |
| | 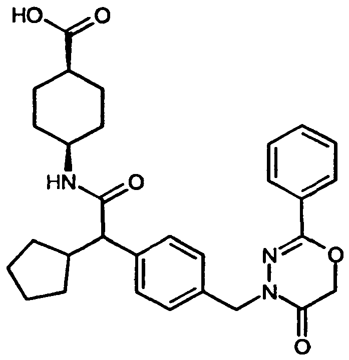 | |
| | Darstellungsmethode 2 (aus Bsp. 159A) | |
| **125** | *trans*-4-[(Cyclopentyl {4-[(5-oxo-2-phenyl-5,6-di-hydro-4*H*-1,3,4-oxadiazin-4-yl)methyl]phenyl}-acetyl)amino]cyclohexancarbonsäure (*Enantiomer 1*) | ¹H-NMR (400 MHz, DMSO-d₆, δ/ppm): 12.3-11.9 (1H, breites s), 7.85 (1H, d), 7.77 (2H, d), 7.53-7.40 (3H, m), 7.29 (4H, q), 4.91 (2H, s), 4.83 (2H, s), 3.67-3.53 (1H, m), 3.21 (1H, d), 2.57-2.39 (1H, m), 2.38-2.27 (1H, m), 1.87-1.72 (2H, m), 1.72-1.36 (10H, m), 1.36-1.24 (2H, m), 1.24-1.13 (1H, m), 0.95-0.81 (1H, m). LC-MS (Methode 11): Rₜ = 1.27 min; m/z = 518 (M+H)⁺. |
| | 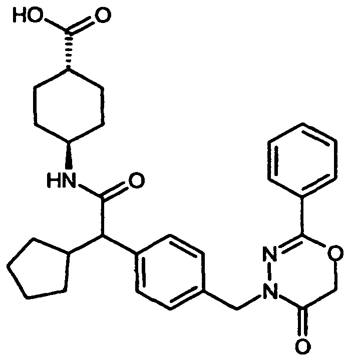 | |
| | Darstellungsmethode 2 (aus Bsp. 158A) | |
| **126** | 4-[(Cyclopentyl {4-[(5-oxo-2-phenyl-5,6-dihydro-4*H* 1,3,4-oxadiazin-4-yl)methyl]phenyl}acetyl)amino]-2,3-dihydro-1*H*-inden-2-carbonsäure (*Enantiomer 1*) | ¹H-NMR (400 MHz, DMSO-d₆, δ/ppm): 12.4-12.2 (1H, breites s), 9.48 (1H, s), 7.77 (2H, d), 7.53-7.38 (5H, m), 7.31 (2H, d), 7.27 (1H, d), 7.04 (1H, t), 6.96 (1H, d), 4.91 (2H, s), 4.85 (2H, s), 3.5 2 (1H, d), 3.28-3.19 (1H, m), 3.14-3.06 (2H, m), 3.06-2.98 (2H, m), 2.63-2.45 (1H, m), 1.85-1.72 (1H, m), 1.72-1.20 (6H, m), 1.02-0.90 (1H, m). LC-MS (Methode 10): Rₜ = 2.23 min; m/z = 552 (M+H)⁺. |
| | 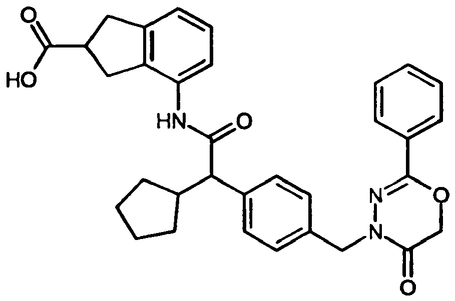 | |
| | Darstellungsmethode 2 (aus Bsp. 105A) | |
| **127** | 3-{3-[(Cyclopentyl {4-[(5-oxo-2-phenyl-5,6-dihydro-4*H*-1,3,4-oxadiazin-4-yl)methyl]phenyl}acetyl)-amino]cyclohexyl}propansäure (*Isomer 1*) | ¹H-NMR (400 MHz, DMSO-d₆, δ/ppm): 12.05-11.85 (1H, breites s), 7.81-7.71 (3H, m), 7.53-7.41 (3H, m), 7.33 (2H, d), 7.27 (2H, d), 4.91 (2H, s), 4.83 (2H, s), 3.82-3.72 (1H, m), 3.26 (1H, d), 2.57-2.39 (1H, m), 2.08 (2H, t), 1.72-1.57 (2H, m), 1.57-1.28 (12H, m), 1.28-1.17 (2H, m), 1.17-1.06 (1H, m), 1.02-0.82 (2H, m). LC-MS (Methode 7): Rₜ = 2.66 min; m/z = 546 (M+H)⁺. |
| | 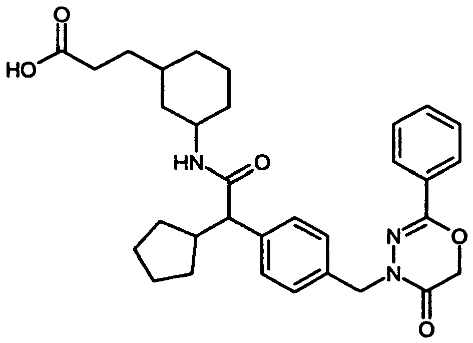 | |
| | Darstellungsmethode 2 (aus Bsp. 188A) | |
| **128** | 3-{3-[(Cyclopentyl {4-[(5-oxo-2-phenyl-5,6-dihydro-4*H*-1,3,4-oxadiazin-4-yl)methyl]phenyl}acetyl)-amino]cyclohexyl}propansäure (*Isomer 2*) | LC-MS (Methode 7): Rₜ = 2.60 min; m/z = 546 (M+H)⁺. |
| | 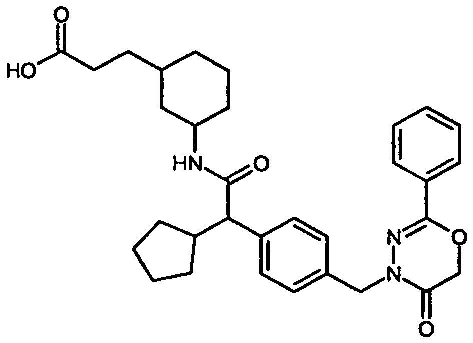 | |
| | Darstellungsmethode 2 (aus Bsp. 189A) | |
| **129** | 3-{3-[(Cyclopentyl{4-[(5-oxo-2-phenyl-5,6-dihydro-4*H*-1,3,4-oxadiazin-4-yl)methyl]phenyl}acetyl)-amino]cyclohexyl}propansäure (*Isomer 3*) | LC-MS (Methode 7): Rₜ = 2.61 min; m/z = 546 (M+H)⁺. |
| | 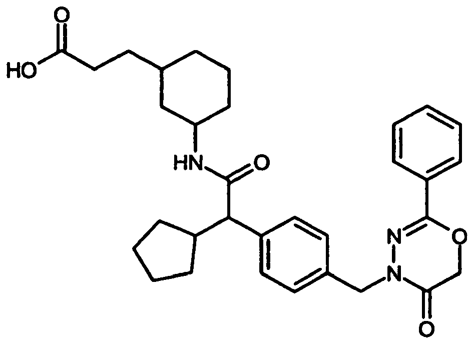 | |
| | Darstellungsmethode 2 (aus Bsp. 190A) | |
| **130** | 3- {3-[(Cyclopentyl {4-[(5-oxo-2-phenyl-5,6-dihydro-4*H*-1,3,4-oxadiazin-4-yl)methyl]phenyl}acetyl)-amino]cyclohexyl}propansäure (*Isomer 4*) | ¹H-NMR (400 MHz, DMSO-d₆, δ/ppm): 11.94 (1H, s), 7.82 (1H, d), 7.77 (2H, d), 7.53-7.40 (3H, m), 7.29 (4H, q), 4.91 (2H, s), 4.84 (2H, s), 3.48-3.35 (1H, m), 3.07 (1H, d), 2.57-2.38 (1H, m), 2.13 (2H, t), 1.82-1.72 (1H, m), 1.72-1.11 (13H, m), 1.08-0.94 (2H, m), 0.94-0.79 (1H, m), 0.79-0.62 (2H, m). LC-MS (Methode 7): Rₜ = 2.66 min; m/z = 546 (M+H)⁺. |
| | 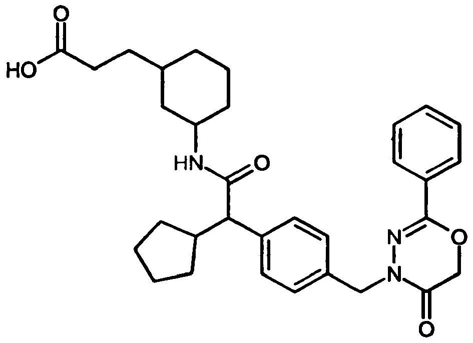 | |
| | Darstellungsmethode 2 (aus Bsp. 191A) | |

### Beispiel 131

### 4-{[(Cyclopentyl{4-[(5-oxo-2-phenyl-5,6-dihydro-4H-1,3,4-oxadiazin-4-yl)methyl]phenyl}acetyl)-amino]methyl}cyclohexancarbonsäure (Isomer 1)

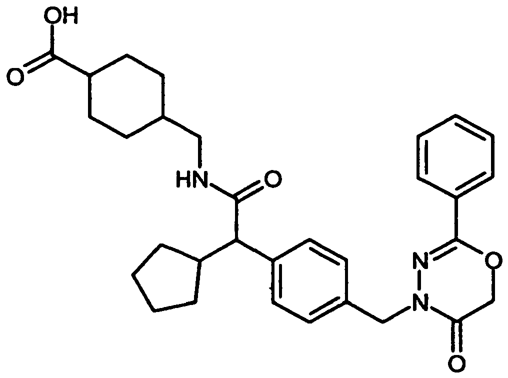

### Darstellungsmethode 7

Eine Lösung von 31 mg (55 µmol) Ethyl-4-{[(cyclopentyl{4-[(5-oxo-2-phenyl-5,6-dihydro-4*H-*1,3,4-oxadiazin-4-yl)methyl]phenyl}acetyl)amino]methyl}cyclohexancarboxylat (Beispiel 127A) in 3 ml Dioxan wurde mit 0.55 ml (1.11 mmol) 2 M Natronlauge versetzt und über Nacht bei Raumtemperatur gerührt. Der Ansatz wurde dann einmal mit *tert.*-Butylmethylether extrahiert. Anschließend wurde die wässrige Phase mit 1 M Salzsäure auf pH 2 eingestellt und zweimal mit Essigsäureethylester extrahiert. Die Essigester-Phasen wurden vereinigt und über Natriumsulfat getrocknet. Nach Filtration wurde das Lösungsmittel bis zur Trockene entfernt. Es wurden 25 mg (47 µmol, 85% d. Th.) der Titelverbindung als farbloser Feststoff erhalten.
¹H-NMR (400 MHz, DMSO-d₆, δ/ppm): 11.97 (1H, breites s), 7.93-7.90 (1H, t), 7.77-7.75 (2H, m), 7.52-7.43 (3H, m), 7.32-7.25 (4H, m), 4.90 (2H, s), 4.83 (2H, s), 3.62-3.59 (1H, m), 3.15-3.13 (1H, d), 2.97-2.90 (1H, m), 2.72-2.66 (1H, m), 2.08-2.02 (1H, m), 1.84-0.78 (17H, m).
LC-MS (Methode 10): Rₜ = 2.09 min; m/z = 531 (M+H)⁺.

Auf analoge Weise wurden die in der folgenden Tabelle aufgeführten Verbindungen erhalten:

| **Beispiel** | **Name / Struktur Darstellungsmethode / Edukt** | **Analytische Daten** |
|---|---|---|
| **132** | 4-{[(Cyclopentyl{4-[(5-oxo-2-phenyl-5,6-dihydro-4*H*-1,3,4-oxadiazin-4-yl)methyl]phenyl}acetyl)amino]-methyl}cyclohexancarbonsäure (*Isomer 2*) | ¹H-NMR (400 MHz, DMSO-d₆, δ/ppm): 11.97 (1H, breites s), 7.93-7.90 (1H, t), 7.77-7.75 (2H, m), 7.52-7.43 (3H, m), 7.32-7.25 (4H, m), 4.90 (2H, s), 4.83 (2H, s), 3.62-3.58 (1H, m), 3.15-3.13 (1H, d), 2.97-2.91 (1H, m), 2.72-2.63 (1H, m), 2.08-2.02 (1H, m), 1.84-0.78 (17H, m). LC-MS (Methode 11): Rₜ = 1.31 min; m/z = 532 {M+H)⁺. |
| | 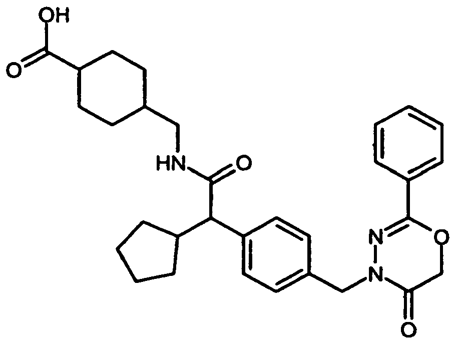 | |
| | Darstellungsmethode 7 (aus Bsp. 128A) | |
| **133** | 4-{[(Cyclopentyl{4-[(5-oxo-2-phenyl-5,6-dihydro-4*H*-1,3,4-oxadiazin-4-yl)methyl]phenyl}acetyl)amino]-methyl}cyclohexancarbonsäure (*Isomer 3*) | ¹H-NMR (400 MHz, DMSO-d₆, δ/ppm): 12.00 (1H, breites s), 7.93-7.90 (1H, t), 7.78-7.75 (2H, m), 7.52-7.43 (3H, m), 7.31-7.23 (4H, m), 4.90 (2H, s), 4.83 (2H, s), 3.13-3.11 (1H, d), 3.00-2.95 (1H, m), 2.73-2.67 (1H, m), 2.37-2.33 (1H, m), 1.76-0.86 (18H, m). LC-MS (Methode 11): Rₜ = 1.31 min; m/z = 532 (M+H)⁺. |
| | 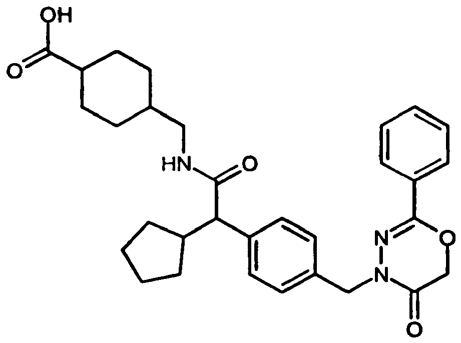 | |
| | Darstellungsmethode 7 (aus Bsp. 129A) | |
| **134** | 4-{[(Cyclopentyl{4-[(5-oxo-2-phenyl-5,6-dihydro-4*H*-1,3,4-oxadiazin-4-yl)methyl]phenyl}acetyl)amino]-methyl}cyclohexancarbonsäure (*Isomer 4*) | ¹H-NMR (400 MHz, DMSO-d₆, δ/ppm): 12.01 (1H, breites s), 7.93-7.90 (1H, t), 7.78-7.75 (2H, m), 7.51-7.43 (3H, m), 7.32-7.25 (4H, m), 4.90 (2H, s), 4.84 (2H, s), 3.14-3.11 (1H, d), 3.02-2.95 (1H, m), 2.73-2.69 (1H, m), 2.36-2.34 (1H, m), 1.80-0.80 (18H, m). LC-MS (Methode 11): Rₜ = 1.31 min; m/z = 532 (M+H)⁺. |
| | 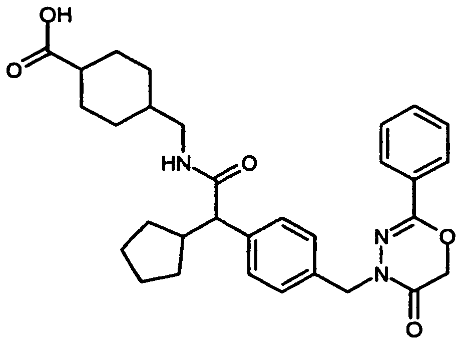 | |
| | Darstellungsmethode 7 (aus Bsp. 130A) | |

Analog zu Ausführungsbeispiel 1, 2 und 3 wurden die in der folgenden Tabelle aufgeführten Verbindungen hergestellt:

| **Beispiel** | **Name / Struktur / Edukt** | **Analytische Daten** |
|---|---|---|
| **135** | 3-{2-Methyl-3-[(3-methyl-2-{4-[(5-oxo-2-phenyl-5,6-dihydro-4*H*-1,3,4-oxadiazin-4-yl)methyl]phenyl}-butanoyl)amino]phenyl}propansäure | LC-MS (Methode 10): Rₜ = 2.08 min; m/z = 528 (M+H)⁺. ¹H-NMR (400 MHz, DMSO-d₆): δ = 9.48 (s, 1H), 7.76 (d, 2H), 7.42-7.54 (m, 3H), 7.39 (d, 2H), 7.32 (d, 2H), 6.94-7.04 (m, 3H), 4.92 (s, 2H), 4.87 (s, 2H), 3.31 (d, 1H), 2.76 (t, 2H), 2.41 (t, 2H), 2.25-2.37 (m, 1H), 1.94 (s, 3H), 1.05 (d, 3H), 0.67 (d, 3H). |
| | 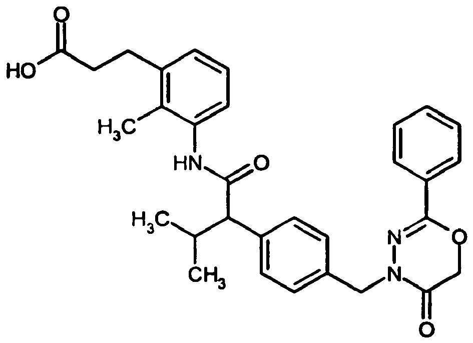 | |
| | (aus *tert*.-Butyl-3-{2-methyl-3-[(3-methyl-2-{4-[(5-oxo-2-phenyl-5,6-dihydro-4*H*-1,3,4-oxadiazin-4-yl)-methyl]phenyl}butanoyl)amino]phenyl}propanoat) | |
| **136** | 3-{3-[(Cyclopentyl{4-[(5-oxo-2-phenyl-5,6-dihydro-4*H*-1,3,4-oxadiazin-4-yl)methyl]phenyl}acetyl)-amino]phenyl}-2,2-dimethylpropansäure | LC-MS (Methode 7): Rₜ = 2.79 min; m/z = 568 (M+H)⁺. ¹H-NMR (400 MHz, DMSO-d₆): δ = 12.01-12.37 (br. s, 1H), 9.98 (s, 1H), 7.76 (d, 2H), 7.36-7.55 (m, 6H), 7.28-7.37 (m, 3H), 7.13 (t, 1H), 6.79 (d, 1H), 4.90 (s, 2H), 4.84 (s, 2H), 3.31 (d, 1H), 2.67-2.75 (s, 2H), 2.47-2.64 (m, 1H), 1.71-1.85 (m, 1H), 1.18-1.71 (m, 6H), 1.04 (s, 6H), 0.89-0.99 (m, 1H). |
| | 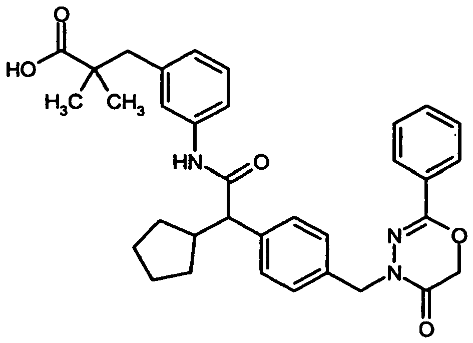 | |
| | (aus Methyl-3-{3-[(cyclopentyl{4-[(5-oxo-2-phenyl-5,6-dihydro-4*H*-1,3,4-oxadiazin-4-yl)methyl]phenyl}-acetyl)amino]phenyl}-2,2-dimethylpropanoat) | |
| **137** | 3-{3-[(Cyclopentyl{4-[(6-oxo-3-phenylpyridazin-1(6*H*)-yl)methyl]phenyl}acetyl)amino]phenyl}-2,2-dimethylpropansäure (*Enantiomer 1*) | LC-MS (Methode 7): Rₜ = 2.74 min; m/z = 564 (M+H)⁺. ¹H-NMR (400 MHz, DMSO-d₆): δ = 12.10 (br. s, 1H), 9.97 (s, 1H), 8.06 (d, 1H), 7.87 (d, 2H), 7.41-7.52 (m, 4H), 7.39 (d, 2H), 7.28-7.35 (m, 3H), 7.13 (t, 1H), 7.07 (d, 1H), 6.79 (d, 1H), 5.29 (s, 2H), 3.37 (d, 1H), 2.70 (s, 2H), 2.47-2.61 (m, 1H), 1.72-1.83 (m, 1H), 1.14-1.68 (m, 6H), 1.05 (s, 6H), 0.91-1.00 (m, 1H). |
| | 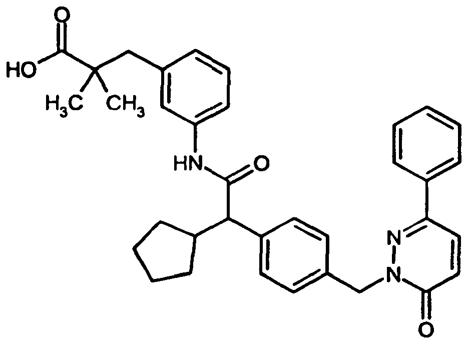 | |
| | (aus Methyl-3-{3-[(cyclopentyl{4-[(6-oxo-3-phenyl-pyridazin-1(6*H*)-yl)methyl]phenyl}acetyl)amino]-phenyl}-2,2-dimethylpropanoat (*Enantiomer 1*)) | |
| **138** | 3-{3-[(Cyclopentyl{4-[(6-oxo-3-phenylpyridazin-1(6*H*)-yl)methyl]phenyl}acetyl)amino]phenyl}-2,2-dimethylpropansäure (*Enantiomer 2*) | LC-MS (Methode 7): Rₜ = 2.74 min; m/z = 564 (M+H)⁺. ¹H-NMR (400 MHz, DMSO-d₆): δ= 12.10 (br. s, 1H), 9.97 (s, 1H), 8.06 (d, 1H), 7.87 (d, 2H), 7.41-7.52 (m, 4H), 7.39 (d, 2H), 7.28-7.35 (m, 3H), 7.13 (t, 1H), 7.07 (d, 1H), 6.79 (d, 1H), 5.29 (s, 2H), 3.37 (d, 1H), 2.70 (s, 2H), 2.47-2.61 (m, 1H), 1.72-1.83 (m, 1H), 1.14-1.68 (m, 6H), 1.05 (s, 6H), 0.91-1.00 (m, 1H). |
| | 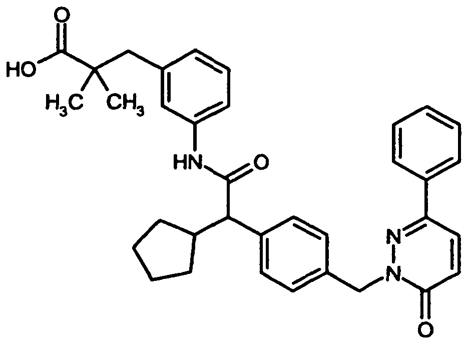 | |
| | (aus Methyl-3-{3-[(cyclopentyl{4-[(6-oxo-3-phenyl-pyridazin-1(6*H*)-yl)rnethyl]phenyl}acetyl)amino]-phenyl}-2,2-dimethylpropanoat (*Enantiomer 2*)) | |
| **139** | 2,2-Dimethyl-3-{3-[(3-methyl-2-{4-[(5-oxo-2-phenyl-5,6-dihydro-4*H*-1,3,4-oxadiazin-4-yl)methyl]phenyl}-butanoyl)amino]phenyl}propansäure | LC-MS (Methode 10): Rₜ = 2.31 min; m/z = 542 (M+H)⁺. ¹H-NMR (400 MHz, DMSO-d₆): δ = 12.22 (br. s, 1H), 10.00 (s, 1H), 7.76 (d, 2H), 7.40-7.52 (m, 4H), 7.37 (d, 2H), 7.27-7.35 (m, 3H), 7.13 (t, 1H), 6.79 (d, 1H), 4.90 (s, 2H), 4.84 (s, 2H), 3.23 (d, 1H), 2.70 (s, 2H), 2.24-2.39 (m, 1H), 1.04 (s, 6H), 0.99 (d, 3H), 0.65 (d, 3H). |
| | 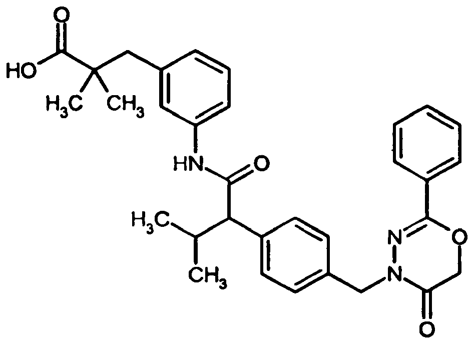 | |
| | (aus Methyl-2,2-dimethyl-3-{3-[(3-methyl-2-{4-[(5-oxo-2-phenyl-5,6-dihydro-4*H*-1,3,4-oxadiazin-4-yl)-methyl]phenyl}butanoyl)amino]phenyl}propanoat) | |
| **140** | 3-{3-[(Cyclopentyl{4-[(6-oxo-3-phenylpyridazin-1 (6*H*)-yl)methyl]phenyl}acetyl)amino]-2-methyl-phenyl}propansäure (*Enantiomer 1*) | LC-MS (Methode 10): Rₜ = 2.12 min; m/z = 550 (M+H)⁺. ¹H-NMR (400 MHz, DMSO-d₆): δ = 11.83-12.43 (br. s, 1H), 9.46 (s, 1H), 8.08 (d, 1H), 7.88 (d, 2H), 7.44-7.53 (m, 3H), 7.40 (d, 2H), 7.32 (d, 2H), 7.09 (d, 1H), 6.92-7.05 (m, 3H), 5.32 (s, 2H), 3.45 (d, 1H), 2.78 (t, 2H), 2.55-2.63 (m, 1H), 2.42 (t, 2H), 1.97 (s, 3H), 1.29-1.88 (m, 7H), 0.96 (dd, 1H). |
| | 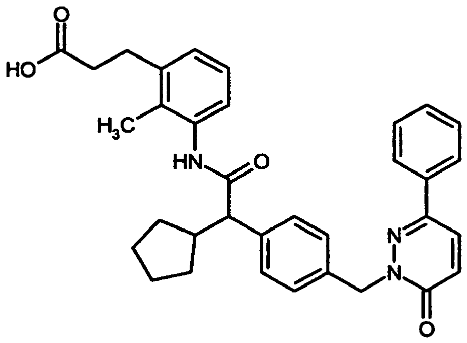 | |
| | (aus *tert*.-Butyl-3-{3-[(cyclopentyl{4-[(6-oxo-3-phenylpyridazin-1(6*H*)-yl)methyl]phenyl}acetyl)-amino]-2-methylphenyl}propanoat (*Enantiomer 1*)) | |
| **141** | 3-{3-[(Cyclopentyl{4-[(6-oxo-3-phenylpyridazin-1 (6*H*)-yl)methyl]phenyl}acetyl)amino]-2-methyl-phenyl}propansäure (*Enantiomer 2*) | LC-MS (Methode 10): Rₜ = 2.12 min; m/z = 550 (M+H)⁺. ¹H-NMR (400 MHz, DMSO-d₆): δ = 11.83-12.43 (br. s, 1H), 9.46 (s, 1H), 8.08 (d, 1H), 7.88 (d, 2H), 7.44-7.53 (m, 3H), 7.40 (d, 2H), 7.32 (d, 2H), 7.09 (d, 1H), 6.92-7.05 (m, 3H), 5.32 (s, 2H), 3.45 (d, 1H), 2.78 (t, 2H), 2.55-2.63 (m, 1H), 2.42 (t, 2H), 1.97 (s, 3H), 1.29-1.88 (m, 7H), 0.96 (dd, 1H). [α]_{D}²⁰ = +48.0°, c = 0.26, Methanol. |
| | 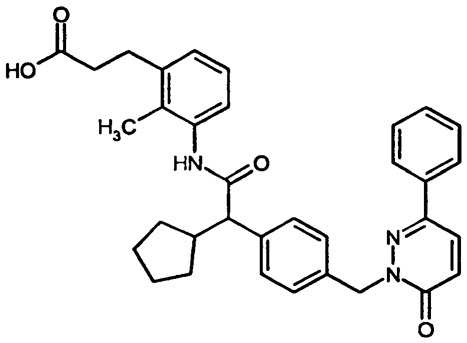 | |
| | (aus *tert*.-Butyl-3-{3-[(cyclopentyl{4-[(6-oxo-3-phenylpyridazin-1(6*H*)-yl)methyl]phenyl}acetyl)-amino]-2-methylphenyl}propanoat (*Enantiomer 2*)) | |
| **142** | 3-{2-Methyl-3-[(6,6,6-trifluor-4-methyl-2-{4-[(5-oxo-2-phenyl-5,6-dihydro-4*H*-1,3,4-oxadiazin-4-yl)-methyl]phenyl}hexanoyl)amino]phenyl}propansäure | LC-MS (Methode 10): Rₜ = 2.25 min; m/z = 610 (M+H)⁺. ¹H-NMR (400 MHz, DMSO-d₆): δ = 12.14 (s, 1H), 9.54 (d, 1H), 7.76 (d, 2H), 7.42-7.54 (m, 3H), 7.40 (d, 2H), 7.34 (d, 2H), 6.95-7.05 (m, 3H), 4.91 (s, 2H), 4.87 (s, 2H), 3.91 (m, 1H), 2.74-2.81 (m, 2H), 2.37-2.45 (m, 2H), 2.26-2.37 (m, 1H), 2.07-2.26 (m, 1H), 1.91-1.99 (m, 3H), 1.75-1.87 (m, 1H), 1.43-1.72 (m, 3H), 1.08 (d, 1H), 1.01 (d, 1H). |
| | 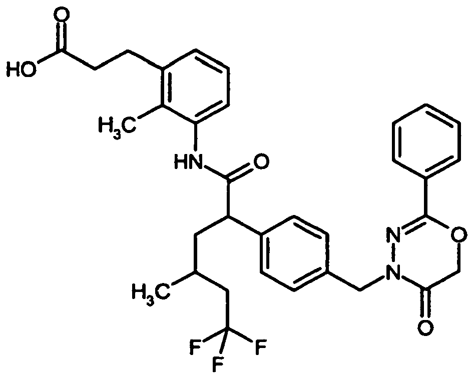 | |
| | (aus Methyl-3-{2-methyl-3-[(6,6,6-trifluor-4-methyl-2-{4-[(5-oxo-2-phenyl-5,6-dihydro-4*H*-1,3,4-oxa-diazin-4-yl)methyl]phenyl}hexanoyl)amino]phenyl}-propanoat) | |
| **143** | 2,2-Dimethyl-3-{3-[(3-methyl-2-(4-[(5-oxo-2-phenyl-5,6-dihydro-4*H*-1,3,4-oxadiazin-4-yl)methyl]phenyl)-pentanoyl)amino]phenyl}propansäure | LC-MS (Methode 11): Rₜ 1.44 min; m/z = 556 (M+H)⁺. ¹H-NMR (400 MHz, DMSO-d₆): δ = 10.01 (s, 1H), 7.76 (d, 2H), 7.41-7.52 (m, 4H), 7.35-7.40 (m, 2H), 7.28-7.35 (m, 3H), 7.13 (t, 1H), 6.79 (d, 1H), 4.90 (s, 2H), 4.84 (s, 2H), 2.70 (br. s, 2H), 2.16 (d, 1H), 1.52 (d, 1H), 1.11-1.25 (m, 2H), 1.04 (br. s, 6H), 0.90 (t, 3H), 0.61 (d, 3H). |
| | 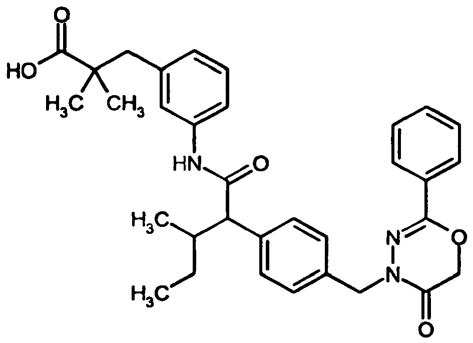 | |
| | (aus Methyl-2,2-dimethyl-3-{3-[(3-methyl-2-{4-[(5-oxo-2-phenyl-5,6-dihydro-4*H*-1,3,4-oxadiazin-4-yl)-methyl]phenyl}pentanoyl)amino]phenyl}propanoat) | |
| **144** | 3- {3-[(Cyclopentyl{4-[(2-oxo-5-phenyl-1,3,4-oxa-diazol-3(2*H*-yl)methyl]phenyl}acetyl)amino]-2-methylphenyl}propansäure (*Enantiomer 1*) | LC-MS (Methode 10): Rₜ = 2.22 min; m/z = 540 (M+H)⁺. ¹H-NMR (400 MHz, DMSO-d₆): δ = 11.95-12.34 (br. s, 1H), 9.48 (s, 1H), 7.80 (d, 2H), 7.50-7.62 (m, 3H), 7.44 (d, 2H), 7.33 (d, 2H), 6.94-7.06 (m, 3H), 4.96 (s, 2H), 3.48 (d, 1H), 2.79 (t, 2H), 2.43 (t, 2H), 1.99 (s, 3H), 1.78-1.90 (m, 1H), 1.30-1.71 (m, 6H), 0.94-1.03 (m, 2H). |
| | 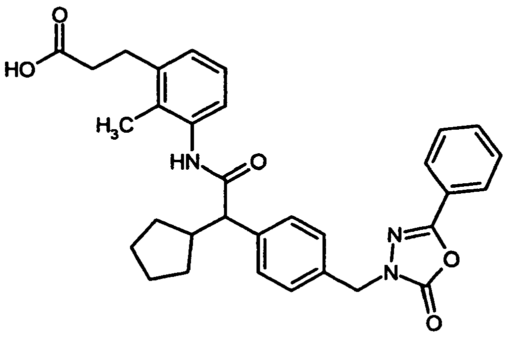 | |
| | (aus *tert*.-Butyl-3-{3-[(cyclopentyl{4-[(2-oxo-5-phenyl-1,3,4-oxadiazol-3(2*H*-yl)methyl]phenyl}-acetyl)amino]-2-methylphenyl}propanoat (*Enantiomer 1*)) | |
| **145** | 3-{3-[(Cyclopentyl{4-[(2-oxo-5-phenyl-1,3,4-oxa-diazol-3(2*H*)-yl)methyl]phenyl}-acetyl)amino]-2-methylphenyl}propansäure (*Enantiomer 2*) | LC-MS (Methode 10): Rₜ = 2.22 min; m/z = 540 (M+H)⁺. ¹H-NMR (400 MHz, DMSO-d₆): δ = 11.95-12.34 (br. s, 1H), 9.48 (s, 1H), 7.80 (d, 2H), 7.50-7.62 (m, 3H), 7.44 (d, 2H), 7.33 (d, 2H), 6.94-7.06 (m, 3H), 4.96 (s, 2H), 3.48 (d, 1H), 2.79 (t, 2H), 2.43 (t, 2H), 1.99 (s, 3H), 1.78-1.90 (m, 1H), 1.30-1.71 (m, 6H), 0.94-1.03 (m, 2H). |
| | 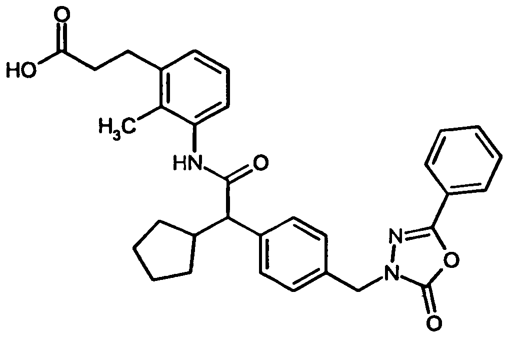 | |
| | (aus *tert*.-Butyl-3-{3-[(cyclopentyl{4-[(2-oxo-5-phenyl-1,3,4-oxadiazol-3(2*H*)-yl)methyl]phenyl}-acetyl)amino]-2-methylphenyl}propanoat (*Enantiomer2*)) | |
| **146** | 4-[(Cyclopentyl{4-[(6-oxo-3-phenylpyridazin-1(6*H*-yl)methyl]phenyl}acetyl)amino]-2,3-dihydro-1*H*-inden-2-carbonsäure | LC-MS (Methode 11): Rₜ= 1.34 min; m/z = 548 (M+H)⁺. ¹H-NMR (400 MHz, DMSO-d₆): δ = 12.01-12.32 (br. s, 1H), 9.47 (d, 1H), 8.07 (d, 1H), 7.88 (d, 2H), 7.45-7.51 (m, 3H), 7.40 (d, 2H), 7.32 (d, 2H), 7.26 (d, 1H), 7.08 (d, 1H), 7.04 (t, 1H), 6.95 (d, 1H), 5.31 (s, 2H), 3.46-3.57 (m, 4H), 3.07-3.13 (m, 1H), 3.02 (d, 1H), 1.71-1.85 (m, 1H), 1.21-1.71 (m, 6H), 1.04 (d, 1H), 0.91-0.99 (m, 1H). |
| | 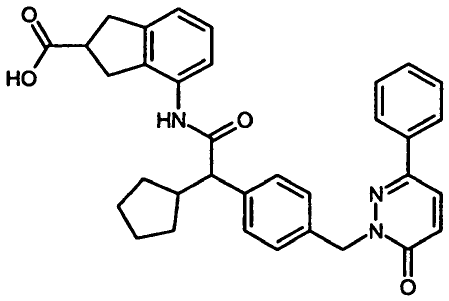 | |
| | (aus Methyl-4-[(cyclopentyl{4-[(6-oxo-3-phenyl-pyridazin-1(6*H*-yl)methyl]phenyl}acetyl)amino]-2,3-dihydro-1*H*-inden-2-carboxylat) | |
| **147** | 3-{2-Methyl-3-[(4,4,4-trifluor-3-methyl-2-{4-[(6-oxo-3-phenylpyridazin-1(6*H*)-yl)methyl]phenyl}butanoyl)-amino]phenyl}propansäure | LC-MS (Methode 11): Rₜ = 1.27 min; m/z = 578 (M+H)⁺. ¹H-NMR (400 MHz, DMSO-d₆): δ = 11.89-12.36 br. s, 1H), 9.62 (s, 1H), 8.08 (d, 1H), 7.87 (d, 2H), 7.40-7.52 (m, 5H), 7.36 (d, 2H), 7.10 (d, 1H), 6.92-7.04 (m, 3H), 5.34 (s, 2H), 3.91 (d, 1H), 3.28-3.41 (m, 1H), 2.75 (t, 2H), 2.40 (t, 2H), 1.88 (s, 3H), 0.78 (d, 3H). |
| | 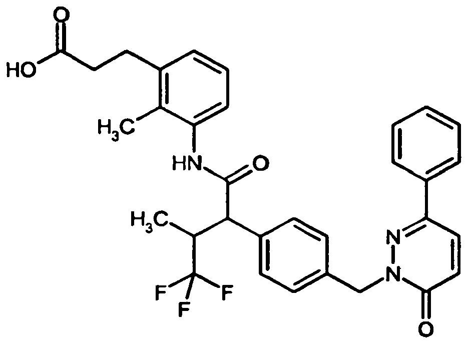 | |
| | (aus *tert*.-Butyl-3-{2-methyl-3-[(4,4,4-trifluor-3-methyt-2-{4-[(6-oxo-3-phenytpyridazin-1(6*H*)-yl)-methyl]phenyl}butanoyl)amino]phenyl}propanoat) | |
| **148** | 3-{3-[(Cyclopentyl{4-[(2-oxo-3,4-dihydrochinolin-1(2*H*-yl)methyl]phenyl}acetyl)amino]-2-methyl-phenyl}propansäure | LC-MS (Methode 11): Rₜ = 1.28 min; m/z = 525 (M+H)⁺. ¹H-NMR (400 MHz, DMSO-d₆): δ = 12.01-12.29 (br. s, 1H), 9.42 (s, 1H), 7.35 (d, 2H), 7.22 (d, 1H), 7.16 (d, 2H), 7.10 (d, 1H), 6.86-7.04 (m, 5H), 5.11 (s, 2H), 2.92-2.98 (m, 2H), 2.79 (t, 2H), 2.65-2.72 (m, 2H), 2.42 (t, 2H), 1.92 (s, 3H), 1.79-1.87 (m, 1H), 1.29-1.72 (m, 7H), 1.23 (s, 2H). |
| | 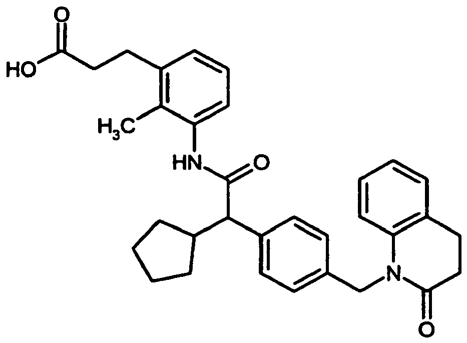 | |
| | (aus *tert*.-Butyl-3-{3-[(cyclopentyl{4-[(2-oxo-3,4-dihydrochinolin-1(2*H*)-yl)methyl]phenyl}-acetyl)amino]-2-methylphenyl}propanoat) | |
| **149** | 3-{3-[(Cyclopentyl{4-[(1-oxoisochinolin-2(1*H*)-yl)-methyl]phenyl}acetyl)amino]-2-methylphenyl}-propansäure | LC-MS (Methode 10): Rₜ = 2.06 min; m/z = 523 (M+H)⁺. |
| | 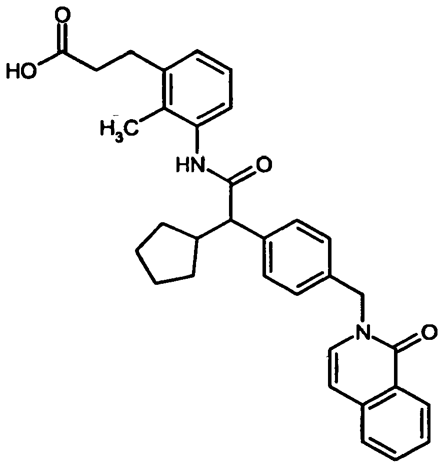 | |
| | (aus *tert*.-Butyl-3-{3-[(cyclopentyl{4-[(1-oxo-isochmotin-2(1*H*)-yl)methyl]phenyl}acetyl)amino]-2-methylphenyl}propanoat) | |
| **150** | 3-{3-[(Cyclopentyl{4-[(4-oxochinazolin-3(4*H*)-yl)-methyl]phenyl}acetyl)amino]-2-methylphenyl}-propansäure | LC-MS (Methode 7): Rₜ = 2.42 min; m/z = 524 (M+H)⁺. ¹H-NMR (400 MHz, DMSO-d₆): δ = 12.03-12.24 (br. s, 1H), 9.45 (s, 1H), 8.47 (s, 1H), 8.28 (d, 1H), 7.82-7.99 (m, 3H), 7.38 (d, 2H), 7.27 (d, 2H), 6.89-7.04 (m, 3H), 5.32 (s, 2H), 3.44 (d, 1H), 2.78 (t, 2H), 2.42 (t, 2H), 1.98 (s, 3H), 1.82 (dd, 1H), 1.61-1.72 (m, 1H), 1.29-1.59 (m, 5H), 0.95 (dd, 1H). |
| | 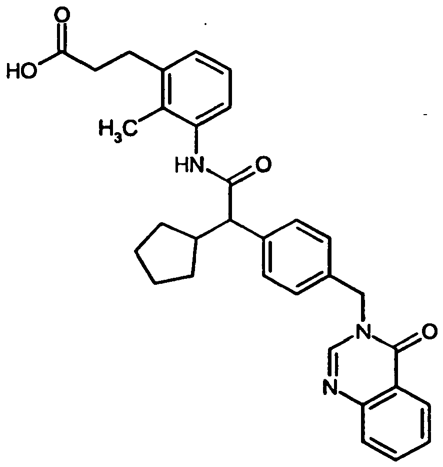 | |
| | (aus *tert*.-Butyl-3-{3-[(cyclopentyl{4-[(4-oxo-chinazolin-3(4*H*)-yl)metbyl]phenyl}acetyl)amino]-2-methylphenyl}propanoat) | |
| **151** | 3-{3-[(Cyclopentyl{4-[(1-oxophthalazin-2(1*H*)-yl)-methyl]phenyl}acetyl)amino]-2-methylphenyl}-propansäure | LC-MS (Methode 7): Rₜ = 2.30 min; m/z = 524 (M+H)⁺. ¹H-NMR (400 MHz, DMSO-d₆): δ = 12.13 (br. s, 1H), 9.45 (s, 1H), 8.57 (s, 1H), 8.16 (d, 1H), 7.84 (t, 1H), 7.70 (d, 1H), 7.56 (t, 1H), 7.40 (d, 2H), 7.31 (d, 2H), 6.90-7.08 (m, 3H), 5.19 (s, 2H), 3.45 (d, 1H), 2.78 (t, 2H), 2.42 (t, 2H), 1.97 (s, 3H), 1.78-1.90 (m, 1H), 1.29-1.71 (m, 6H), 0.88-1.01 (m, 1H). |
| | 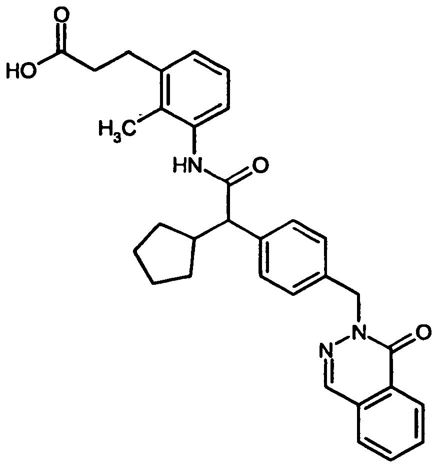 | |
| | (aus *tert.*-Butyl-3-{3-[(cyclopentyl{4-[(1-oxo-phthalazin-2(1*H*-yl)methyl]phenyl}acetyl)amino]-2-methylphenyl}propanoat) | |
| **152** | 3-(3-{[{4-[(6-Chlor-2-oxo-3,4-dihydrochinolin-1(2*H*)-yl)methyl]phenyl}(cyclopentyl)acetyl]amino}-2-methylphenyl)propansäure | LC-MS (Methode 10): Rₜ = 2.13 min; m/z = 560 (M+H)⁺. |
| | 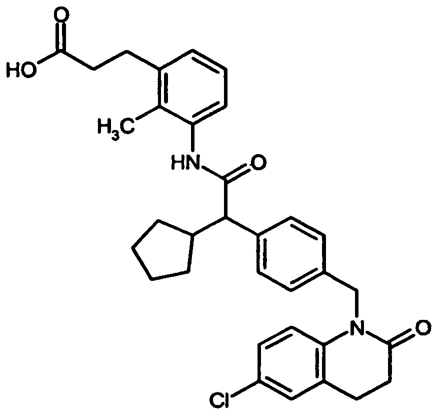 | |
| | (aus *tert*.-Butyl-3-(3-{[{4-[(6-chlor-2-oxo-3,4-dihydro-chinolin-1(2*H*)-yl)methyl]phenyl}(cyclopentyl)-acetyl]amino}-2-methylphenyl)propanoat) | |
| **153** | 3-(3-{[{4-[(3-Chlor-6-oxopyridazin-1(6H)-yl)methyl]-phenyl}(cyclopentyl)acetyl]amino}-2-methylphenyl)-propansäure | LC-MS (Methode 7): Rₜ = 2.30 min; m/z = 508 (M+H)⁺. ¹H-NMR (400 MHz, DMSO-d₆): δ = 12.13 (br. s, 1H), 9.46 (s, 1H), 7.57 (d, 1H), 7.40 (d, 2H), 7.24 (d, 2H), 7.09 (d, 1H), 6.95-7.04 (m, 3H), 5.18 (s, 2H), 3.57-3.64 (m, 1H), 3.46 (d, 1H), 2.79 (t, 2H), 2.43 (t, 2H), 1.99 (s, 3H), 1.80-1.88 (m, 1H), 1.76 (dt, 1H), 1.62-1.72 (m, 1H), 1.52-1.60 (m, 2H), 1.46 (d, 1H), 1.29-1.41 (m, 2H). |
| | 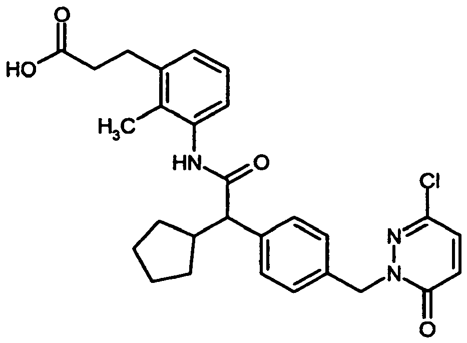 | |
| | (aus *tert*.-Butyl-3-(3-{[{4-[(3-chlor-6-oxopyridazin-1(6*H*)-yl)methyl]phenyl}(cyclopentyl)acetyl]amino}-2-methylphenyl)propanoat) | |
| **154** | 3-{3-[(Cyclopentyl{4-[(4-methyl-1-oxophthalazin-2(1*H*)-yl)methyl]phenyl}acetyl)amino]-2-methyl-phenyl}propansäure | LC-MS (Methode 7): Rₜ = 2.47 min; m/z = 538 (M+H)⁺. ¹H-NMR (400 MHz, DMSO-d₆): δ = 12.13 (s, 1H), 9.45 (s, 1H), 8.31 (d, 1H), 7.96 (d, 2H), 7.84-7.92 (m, 1H), 7.38 (d, 2H), 7.26 (d, 2H), 6.94-7.05 (m, 3H), 5.28 (s, 2H), 3.44 (d, 1H), 2.78 (t, 2H), 2.42 (t, 2H), 1.98 (s, 3H), 1.78-1.90 (m, 1H), 1.62-1.73 (m, 1H), 1.49-1.60 (m, 2H), 1.40-1.50 (m, 1H), 1.28-1.38 (m, 2H), 0.96 (dd, 1H). |
| | 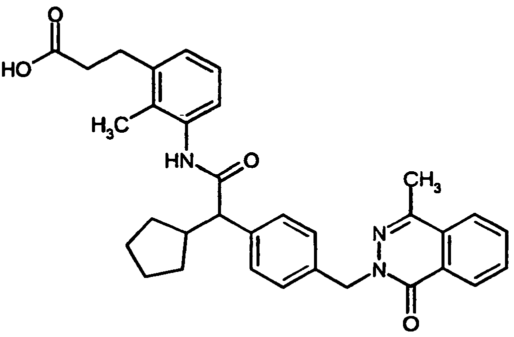 | |
| | (aus *tert*.-Butyl-3-{3-[(cyclopentyl{4-[(4-methyl-1-oxophthalazin-2(1*H*)-yl)methyl]phenyl}acetyl)amino]-2-methylphenyl}propanoat) | |
| **155** | 3-{3-[(Cyclopentyl{4-[(1,1-dioxido-3-oxo-1,2-benz-isothiazol-2(3*H*)-yl)methyl]phenyl}acetyl)amino]-2-methylphenyl}propansäure | LC-MS (Methode 10): Rₜ = 2.09 min; m/z = 561 (M+H)⁺. ¹H-NMR (400 MHz, DMSO-d₆): δ = 11.93-12.29 (br. s, 1H), 9.48 (s, 1H), 8.33 (d, 1H), 8.13 (d, 1H), 7.96-8.10 (m, 2H), 7.32-7.45 (m, 4H), 6.92-7.06 (m, 3H), 4.90 (s, 2H), 3.46 (d, 1H), 2.79 (t, 2H), 2.43 (t, 2H), 1.99 (s, 3H), 1.77-1.89 (m, 1H), 1.63-1.70 (m, 1H), 1.51-1.62 (m, 2H), 1.41-1.49 (m, 1H), 1.29-1.39 (m, 2H), 1.24 (s, 1H), 0.92-1.05 (m, 1H). |
| | 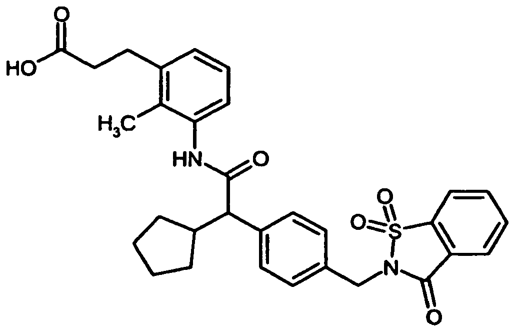 | |
| | (aus *tert.*-Butyl-3-{3-[(cyclopentyl{4-[(1,1-dioxido-3-oxo-1,2-benzisothiazol-2(3*H*)-yl)methyl]phenyl}-acetyl)amino]-2-methylphenyl}propanoat) | |
| **156** | 1-{3-[(Cyclopentyl{4-[(6-oxo-3-phenylpyridazin-1(6*H*)-yl)methyl]phenyl}acetyl)amino]benzyl}-cyclopropancarbonsäure | LC-MS (Methode 10): Rₜ = 2.29 min; m/z = 562 (M+H)⁺. ¹H-NMR (400 MHz, DMSO-d₆): δ = 12.07 (s, 1H), 9.97 (s, 1H), 8.06 (d, 1H), 7.87 (d, 2H), 7.35-7.55 (m, 7H), 7.31 (d, 2H), 7.12 (t, 1H), 7.07 (d, 1H), 6.89 (d, 1H), 5.29 (s, 2H), 3.37 (d, 1H), 2.81 (s, 2H), 2.56-2.64 (m, 1H), 1.72-1.85 (m, 1H), 1.58-1.69 (m, 1H), 1.49-1.58 (m, 2H), 1.38-1.48 (m, 1H), 1.28-1.38 (m, 1H), 1.20-1.28 (m, 1H), 1.10 (d, 2H), 0.91-1.00 (m, 1H), 0.78 (d, 2H). |
| | 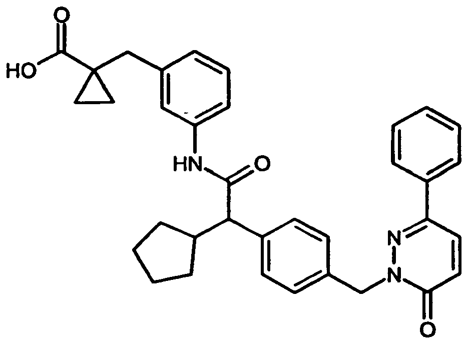 | |
| | (aus *tert*.-Butyl-1-{3-[(cyclopentyl{4-[(6-oxo-3-phenylpyridazin-1(6*H*)-yl)methyl]phenyl}acetyl)-amino]benzyl}cyclopropancarboxylat) | |
| **157** | 1-{3-[(4,4,4-Trifluor-3-methyl-2-{4-[(6-oxo-3-phenyl-pyridazin-1(6*H*-yl)methyl]phenyl}butanoyl)amino]-benzyl}cyclopropancarbonsäure (*Isomer 1*) | LC-MS (Methode 11): Rₜ = 1.36 min; m/z = 590 (M+H)⁺. ¹H-NMR (400 MHz, DMSO-d₆): δ = 12.07 (br. s, 1H), 10.15 (s, 1H), 8.06 (d, 1H), 7.87 (d, 2H), 7.42-7.51 (m, 3H), 7.40 (d, 3H), 7.35 (d, 3H), 7.13 (t, 1H), 7.07 (d, 1H), 6.90 (d, 1H), 5.31 (s, 2H), 3.81 (d, 1H), 2.80 (s, 2H), 1.17-1.23 (1H, m), 1.06-1.12 (m, 2H), 0.77 (d, 5H). |
| | 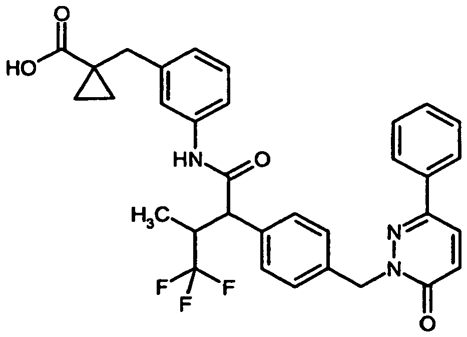 | |
| | (aus *tert*.-Butyl-1-(3-[(4,4,4-trifluor-3-methyl-2-{4-[(6-oxo-3-phenylpyridazin-1(6*H*-yl)methyl]phenyl}-butanoyl)amino]benzyl}cyclopropancarboxylat (*Isomer 1*)) | |
| **158** | 1-{3-[(4,4,4-Trifluor-3-methyl-2-{4-[(6-oxo-3-phenyl-pyridazin-1(6*H*-yl)methyl]phenyl butanoyl)amino]-benzyl}cyclopropancarbonsäure (*Isomer 2*) | LC-MS (Methode 11): Rₜ = 1.36 min; m/z = 590 (M+H)⁺. ¹H-NMR (400 MHz, DMSO-d₆): δ = 12.07 (br. s, 1H), 10.15 (s, 1H), 8.06 (d, 1H), 7.87 (d, 2H), 7.42-7.51 (m, 3H), 7.40 (d, 3H), 7.35 (d, 3H), 7.13 (t, 1H), 7.07 (d, 1H), 6.90 (d, 1H), 5.31 (s, 2H), 3.81 (d, 1H), 2.80 (s, 2H), 1.17-1.23 (1H, m), 1.06-1.12 (m, 2H), 0.77 (d, 5H). |
| | 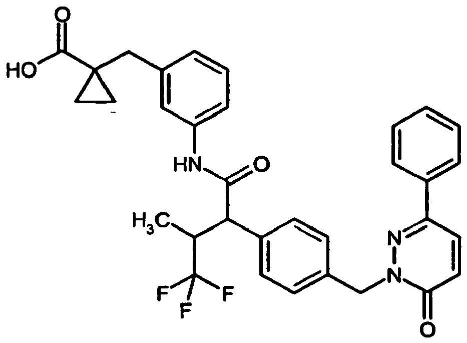 | |
| | (aus *tert.*-Butyl-1-{3-[(4,4,4-trifluor-3-methyl-2-{4-[(6-oxo-3-phenylpyridazin-1(6*H*)-yl)methyl]phenyl}-butanoyl)amino]benzyl}cyclopropancarboxylat (*Isomer 2*)) | |
| **159** | 3-{3-[(Cyclopentyl{4-[(3-methyl-6-oxopyridazin-1(6*H*)-yl)methyl]phenyl}acetyl)amino]-2-methyl-phenyl}propansäure | LC-MS (Methode 15): Rₜ = 1.01 min; m/z = 488 (M+H)⁺. ¹H-NMR (400 MHz, DMSO-d₆): δ =11.79-12.29 (br. s, 1H), 9.46 (s, 1H), 7.36 (dd, 3H), 7.22 (d, 2H), 6.95-7.04 (m, 3H), 6.91 (d, 1H), 5.16 (s, 2H), 3.45 (d, 1H), 2.79 (t, 2H), 2.52-2.61 (m, 1H), 2.43 (t, 2H), 2.26 (s, 3H), 1.99 (s, 3H), 1.77-1.89 (m, 1H), 1.62-1.71 (m, 1H), 1.51-1.61 (m, 2H), 1.42-1.50 (m, 1H), 1.30-1.41 (m, 2H), 0.92-1.02 (m, 1H). |
| | 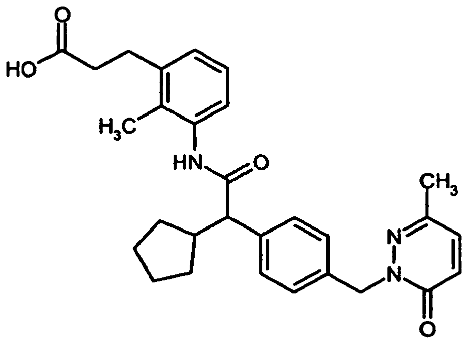 | |
| | (aus *tert*.-Butyl-3-{3-[(cyclopentyl{4-[(3-methyl-6-oxopyridazin-1(6*H*)-yl)methyl]phenyl}acetyl)amino]-2-methylphenyl}propanoat) | |
| **160** | 1-{3-[(5,5,5-Trifluor-3-methyl-2-(4-[(5-oxo-2-phenyl-5,6-dihydro-4*H*-1,3,4-oxadiazin-4-yl)methyl]phenyl)-pentanoyl)amino]benzyl}cyclopropancarbonsäure | LC-MS (Methode 11): Rₜ = 1.43 min; m/z = 608 (M+H)⁺ (*Diastereomer 1*); Rₜ = 1.44 min; m/z = 608 (M+H)⁺ (*Diastereomer 2*). ¹H-NMR (400 MHz, DMSO-d₆): δ = 12.07 (br. s, 1H), 10.14 (s, 0.5H), 10.09 (s, 0.5H) 7.75 (d, 2H), 7.31-7.51 (m, 9H), 7.14 (t, 1H), 6.90 (d, 1H), 4.91 (s, 2H), 4.86 (s, 2H), 3.49 (d, 1H), 2.80 (s, 2H), 2.49-2.64 (m, 1H), 2.24-2.36 (m, 1H), 2.20-2.37 (m, 1H), 1.95-2.10 (m, 0.5H), 1.76-1.95 (m, 0.5H), 1.07-1.15 (m, 4H), 0.72-0.81 (m, 3H). |
| | 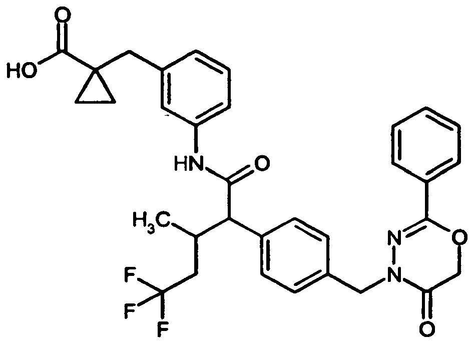 | |
| | (aus *tert*.-Butyl-1-{3-[(5,5,5-trifluor-3-methyl-2-{4-[(5-oxo-2-phenyl-5,6-dihydro-4*H*-1,3,4-oxadiazin-4-yl)methyl]phenyl}pentanoyl)amino]benzyl}-cyclopropancarboxylat) | |
| **161** | 1-(3-{[{4-[(3-Chlor-6-oxopyridazin-1(6*H*)-yl)methyl]-phenyl}(cyclopentyl)acetyl]amino}benzyl)-cyclopropancarbonsäure | LC-MS (Methode 11): Rₜ = 1.29 min; m/z = 520 (M+H)⁺. ¹H-NMR (400 MHz, DMSO-d₆): δ = 12.08 (s, 1H), 9.97 (s, 1H), 7.40-7.45 (m, 2H), 7.36 (d, 2H), 7.32 (d, 1H), 7.21 (d, 2H), 7.13 (t, 1H), 6.89 (d, 2H), 5.16 (s, 2H), 3.36 (d, 1H), 2.81 (s, 2H), 2.49-2.63 (m, 1H), 1.72-1.83 (m, 1H), 1.41-1.71 (m, 3H), 1.29-1.40 (m, 1H), 1.19-1.30 (m, 1H), 1.10 (d, 2H), 0.89-1.02 (m, 1H), 0.78 (d, 2H). |
| | 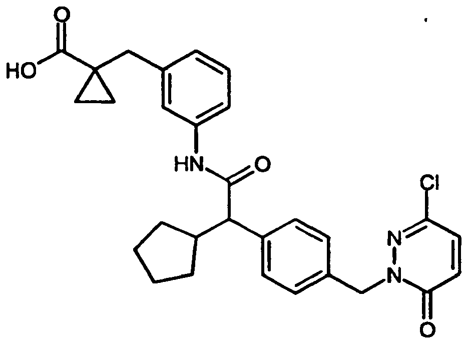 | |
| | (aus *tert.*-Butyl-1-(3-{[{4-[(3-chlor-6-oxopyridazin-1(6*H*)-yl)methyl]phenyl}(cyclopentyl)acetyl]-amino} benzyl)cyclopropancarboxylat) | |
| **162** | 3-{3-[(Cyclopentyl{4-[(5-methyl-2-oxopyridin-1(2*H*-yl)methyl]phenyl}acetyl)amino]-2-methyl-phenyl}propansäure | LC-MS (Methode 15): Rₜ = 1.00 min; m/z = 487 (M+H)⁺. ¹H-NMR (500 MHz, DMSO-d₆): δ = 11.90-12.30 (br. s, 1H), 9.47 (s, 1H), 7.56 (s, 1H), 7.38 (d, 2H), 7.30 (d, 1H), 7.22 (d, 2H), 6.93-7.06 (m, 3H), 6.37 (d, 1H), 5.02 (s, 2H), 3.45 (d, 1H), 2.79 (t, 2H), 2.53-2.63 (m, 1H), 2.43 (t, 2H), 2.00 (s, 3H), 1.99 (s, 3H), 1.80-1.90 (m, 1H), 1.64-1.73 (m, 1H), 1.50-1.60 (m, 2H), 1.46 (d, 1H), 1.30-1.39 (m, 2H), 0.96 (dd, 1H). |
| | 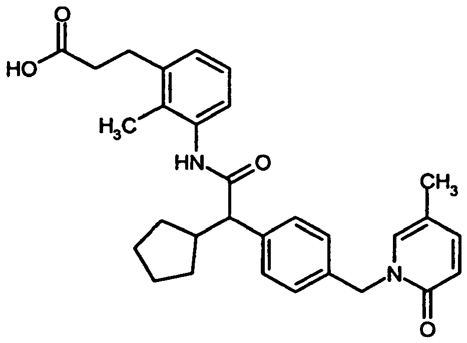 | |
| | (aus *tert*.-Butyl-3-{3-[(cyclopentyl{4-[(5-methyl-2-oxopyridin-1(2,*H*)-yl)methyl]phenyl}acetyl)amino]-2-methylphenyl}propanoat) | |
| **163** | 3-{3-[(Cyclopentyl{4-[(3-oxo-1,2-benzisoxazol-2(3*H*)-yl)methyl]phenyl}acetyl)amino]-2-methyl-phenyl}propansäure | LC-MS (Methode 11): Rₜ = 1.23 min; m/z = 513 (M+H)⁺. |
| | 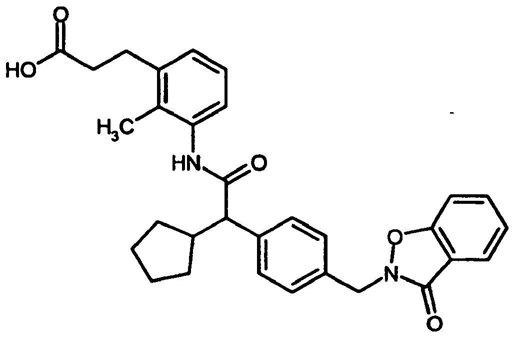 | |
| | (aus *tert*.-Butyl-3-{3-[(cyclopentyl{4-[(3-oxo-1,2-benzisoxazol-2(3*H*)-yl)methyl]phenyl}acetyl)amino]-2-methylphenyl}propanoat) | |
| **164** | 1-{3-[(4,4,4-Trifluor-3-methyl-2-{4-[(5-oxo-2-phenyl-5,6-dihydro-4*H* 1,3,4-oxadiazin-0-yl)methyl]phenyl}-butanoyl)amino]benzyl}cyclopropancarbonsäure *(Isomer 1*) | LC-MS (Methode 15): Rₜ = 1.23 min, m/z = 594 (M+H)⁺. ¹H-NMR (400 MHz, DMSO-d₆): δ = 12.08 (br. s, 1H), 10.24 (s, 1H), 7.75 (d, 2H), 7.36-7.51 (m, 7H), 7.32 (d, 2H), 7.12-7.18 (m, 1H), 6.91 (d, 1H), 4.90 (s, 2H), 4.83-4.87 (m, 2H), 3.87 (d, 1H), 3.15-3.29 (m, 1H), 2.80 (s, 2H), 1.20 (d, 3H), 1.07-1.12 (m, 2H), 0.75-0.81 (m, 2H). |
| | 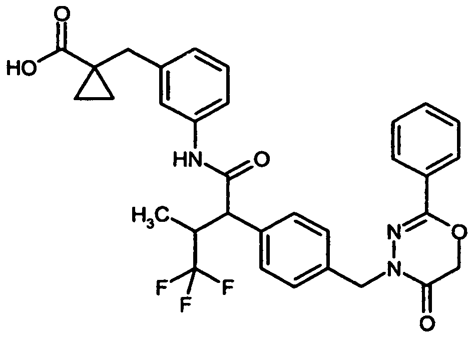 | |
| | (aus *tert*.-Butyl-1-{3-[(4,4,4-trifluor-3-methyl-2-{4-[(5-oxo-2-phenyl-5,6-dihydro-4*H*-1,3,4-oxadiazin-4-yl)methyl]phenyl}butanoyl)amino]benzyl}-cyclopropancarboxylat *(Isomer 1*)) | |
| **165** | 1-{3-[(4,4,4-Trifluor-3-methyl-2-{4-[(5-oxo-2-phenyl-5,6-dihydro-4*H*-1,3,4-oxadiazin-4-yl)methyl]phenyl}-butanoyl)amino]benzyl}cyclopropancarbonsäure *(Isomer 2*) | LC-MS (Methode 15): Rₜ = 1.23 min; m/z = 594 (M+H)⁺. ¹H-NMR (400 MHz, DMSO-d₆): δ = 12.08 (br. s, 1H), 10.24 (s, 1H), 7.75 (d, 2H), 7.36-7.51 (m, 7H), 7.32 (d, 2H), 7.12-7.18 (m, 1H), 6.91 (d, 1H), 4.90 (s, 2H), 4.83-4.87 (m, 2H), 3.87 (d, 1H), 3.15-3.29 (m, 1H), 2.80 (s, 2H), 1.20 (d, 3H), 1.07-1.12 (m, 2H), 0.75-0.81 (m, 2H). |
| | 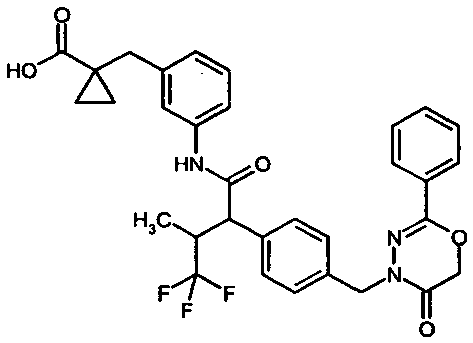 | |
| | (aus *tert*.-Butyl-1-{3-[(4,4,4-trifluor-3-methyl-2-{4-[(5-oxo-2-phenyl-5,6-dihydro-4*H*-1,3,4-oxadiazin-4-yl)methyl]phenyl}butanoyl)amino]benzyl}-cyclopropancarboxylat *(Isomer 2*)) | |
| **166** | 1-{3-[(4,4,4-Trifluor-3-methyl-2-{4-[(5-oxo-2-phenyl-5,6-dihydro-4*H*-1,3,4-oxadiazin-4-yl)methyl]phenyl} -butanoyl)amino]benzyl}cyclopropancarbonsäure *(Isomer 3*) | LC-MS (Methode 15): Rₜ = 1.22 min; m/z = 594 (M+H)⁺. ¹H-NMR (400 MHz, DMSO-d₆): δ = 11.50-12.50 (br. s, 1H), 10.15 (s, 1H), 7.75 (d, 2H), 7.39-7.51 (m, 7H), 7.36 (d, 2H), 7.13 (t, 1H), 6.90 (d, 1H), 4.90 (s, 2H), 4.86 (s, 2H), 3.82 (d, 1H), 3.30-3.44 (m, 1H), 2.80 (s, 2H), 1.09 (d, 2H), 0.78 (d, 5H). |
| | 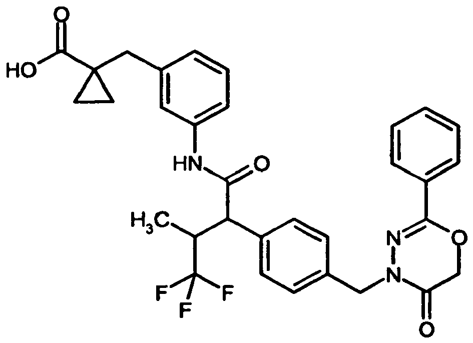 | |
| | (aus *tert*.-Butyl-1-{3-[(4,4,4-trifluor-3-methyl-2-{4-[(5-oxo-2-phenyl-5,6-dihydro-4*H*-1,3,4-oxadiazin-4-yl)methyl]phenyl}butanoyl)amino]benzyl}-cyclopropancarboxylat *(Isomer 3*)) | |
| **167** | 1-{3-[(4,4,4-Trifluor-3-methyl-2-{4-[(5-oxo-2-phenyl-5,6-dihydro-4*H*-1,3,4-oxadiazin-4-yl)methyl]phenyl}-butanoyl)amino]benzyl}cyclopropancarbonsäure *(Isomer 4)* | LC-MS (Methode 15): Rₜ = 1.23 min; m/z = 594 (M+H)⁺. ¹H-NMR (400 MHz, DMSO-d₆): δ = 11.50-12.50 (br. s, 1H), 10.15 (s, 1H), 7.75 (d, 2H), 7.39-7.51 (m, 7H), 7.36 (d, 2H), 7.13 (t, 1H), 6.90 (d, 1H), 4.90 (s, 2H), 4.86 (s, 2H), 3.82 (d, 1H), 3.30-3.44 (m, 1H), 2.80 (s, 2H), 1.09 (d, 2H), 0.78 (d, 5H). |
| | 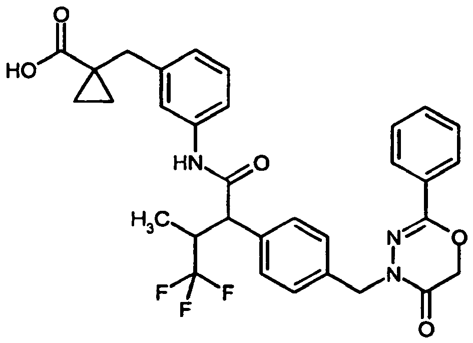 | |
| | (aus *tert*.-Butyl-1-{3-[(4,4,4-trifluor-3-methyl-2-{4-[(5-oxo-2-phenyl-5,6-dihydro-4*H*-1,3,4-oxadiazin-4-yl)methyl]phenyl}butanoyl)amino]benzyl}-cyclopropancarboxylat *(Isomer 4*)) | |
| **168** | 3-{2-Methyl-3-[(4,4,4-trifluor-3-methyl-2-{4-[(5-oxo-2-phenyl-5,6-dihydro-4*H*-1,3,4-oxadiazin-4-yl)-methyl]phenyl} butanoyl)amino]phenyl} propansäure | LC-MS (Methode 15): Rₜ = 1.14 min; m/z = 582 (M+H)⁺. |
| | 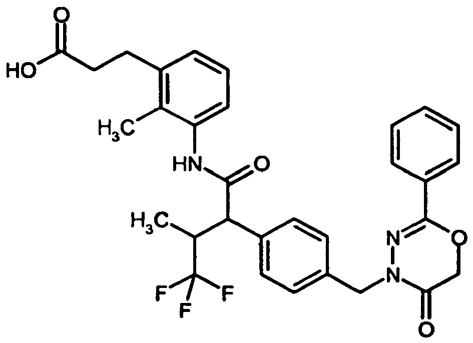 | |
| | (aus *tert*.-Butyl-3-{2-methyl-3-[(4,4,4-trifluor-3-methyl-2-{4-[(5-oxo-2-phenyl-5,6-dihydro-4*H*-,3,4-oxadiazin-4-yl)methyl]phenyl}butanoyl)amino]-phenyl}propanoat) | |
| **169** | 3-{3-[(4,4,4-Trifluor-3-methyl-2-{4-[(5-oxo-2-phenyl-5,6-dihydro-4*H*-1,3,4-oxadiazin-4-yl)methyl]phenyl}-butanoyl)amino]phenyl}propansäure | LC-MS (Methode 15): Rₜ = 1.17 min; m/z = 568 (M+H)⁺. |
| | 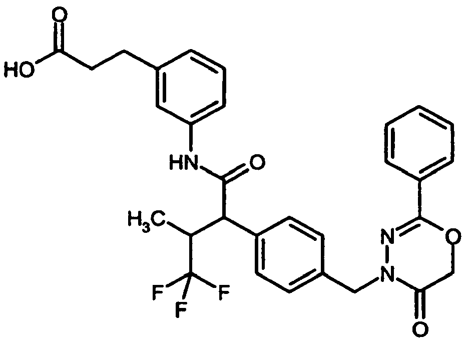 | |
| | (aus *tert*.-Butyl-3-{3-[(4,4,4-trifluor-3-methyl-2-{4-[(5-oxo-2-phenyl-5,6-dihydro-4*H*-1,3,4-oxadiazin-4-yl)methyl]phenyl}butanoyl)amino]phenyl}propanoat) | |
| **170** | 1-(3-{[2-{4-[(5-Oxo-2-phenyl-5,6-dihydro-4*H*-1,3,4-oxadiazin-4-yl)methyl]phenyl}-3-(trifluormethyl)-pentanoyl]amino}benzyl)cyclopropancarbonsäure | LC-MS (Methode 15): Rₜ = 1.26 min; m/z = 608 (M+H)⁺. ¹H-NMR (400 MHz, DMSO-d₆): δ = 12.10 (br. s, 1H), 10.16 (s, 1H), 7.75 (d, 2H), 7.28-7.60 (m, 9H), 7.13 (t, 1H), 6.89 (d, 1H), 4.90 (s, 2H), 4.86 (s, 2H), 3.94 (d, 1H), 3.20-3.31 (m, 1H), 2.80 (s, 2H), 1.20-1.43 (m, 2H), 1.09 (d, 2H), 0.71-0.81 (m, 5H). |
| | 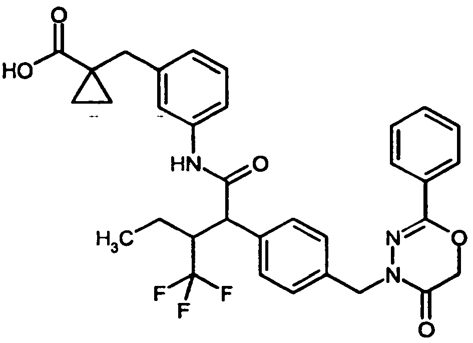 | |
| | (aus *tert*.-Butyl-1-(3-{[2-{4-[(5-oxo-2-phenyl-5,6-dihydro-4*H*-1,3,4-oxadiazin-4-yl)methyl]phenyl}-3-(trifluormethyl)pentanoyl]amino}benzyl)-cyclopropancarboxylat) | |
| **171** | 1-{3-[(Cyclopentyl{4-[(3-methyl-6-oxopyridazin-1 (6*H*)-yl)methyl]phenyl}acetyl)amino]benzyl} -cyclopropancarbonsäure | LC-MS (Methode 11): Rₜ = 1.23 min; m/z = 500 (M+H)⁺. |
| | 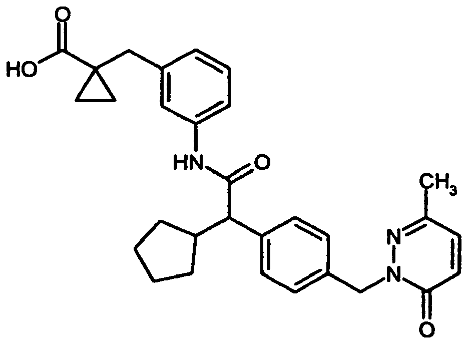 | |
| | (aus *tert*.-Butyl-1-{3-[(cyclopentyl{4-[(3-methyl-6-oxopyridazin-1(6*H*)-yl)methyl]phenyl}acetyl)amino]-benzyl}cyclopropancarboxylat) | |
| **172** | 3-(3-{[Cyclopentyl(4-{[6-oxo-3-(trifluormethyl)-pyridazin-1 (6*H*)-yl]methyl}phenyl)acetyl]amino}-2-methylphenyl)propansäure | LC-MS (Methode 15): Rₜ = 1.13 min; m/z = 542 (M+H)⁺. ¹H-NMR (400 MHz, DMSO-d₆): δ =12.13 (s, 1H), 9.47 (s, 1H), 7.87 (d, 1H), 7.41 (d, 2H), 7.26 (d, 2H), 7.21 (d, 1H), 6.95-7.06 (m, 3H), 5.29 (s, 2H), 3.46 (d, 1H), 2.79 (t, 2H), 2.56-2.63 (m, 1H), 2.43 (t, 2H), 1.98 (s, 3H), 1.77-1.88 (m, 1H), 1.30-1.72 (m, 6H), 0.96 (dd, 1H). |
| | 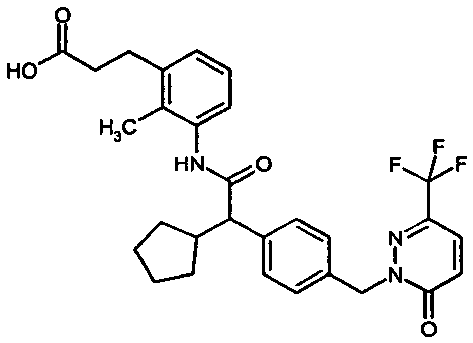 | |
| | (aus *tert*.-Butyl-3-(3-{[cyclopentyl(4-{[6-oxo-3-(trifluormethyl)pyridazin-1(6*H*)-yl]methyl}phenyl)-acetyl]amino}-2-methylphenyl)propanoat) | |
| **173** | 1-(3-{[Cyclopentyl(4-{[6-oxo-3-(trifluormethyl)-pyridazin-1(6*H*)-yl]methyl}phenyl)acetyl]amino}-benzyl)cyclopropancarbonsäure | LC-MS (Methode 15): Rₜ = 1.20 min; m/z = 554 (M+H)⁺. ¹H-NMR (400 MHz, DMSO-d₆): δ = 12.08 (br. s, 1H), 9.98 (s, 1H), 7.85 (d, 1H), 7.35-7.46 (m, 4H), 7.26 (d, 2H), 7.09-7.21 (m, 2H), 6.89 (d, 1H), 5.27 (s, 2H), 3.38 (d, 1H), 2.81 (s, 2H), 2.56-2.64 (m, 1H), 1.73-1.84 (m, 1H), 1.18-1.70 (m, 6H), 1.07-1.13 (m, 2H), 0.85-1.02 (m, 1H), 0.78 (d, 2H). |
| | 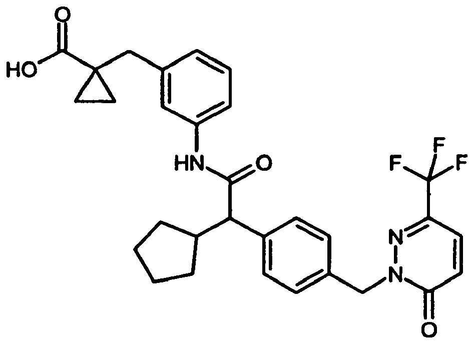 | |
| | (aus *tert*.-Butyl-1-(3-{[cyclopentyl(4-{[6-oxo-3-(trifluormethyl)pyridazin-1(6*H*)-yl]methyl}phenyl)-acetyl]amino}benzyl)cyclopropancarboxylat) | |
| **174** | 3-(3-{[Cyclopentyl(4-{[6-oxo-3-(trifluormethyl)-pyridazin-1(6*H*)-yl]methyl}phenyl)acetyl]amino}-phenyl)propansäure | LC-MS (Methode 11): Rₜ = 1.31 min; m/z = 528 (M+H)⁺. ¹H-NMR (400 MHz, DMSO-d₆): δ = 12.07 (br. s, 1H), 9.99 (s, 1H), 7.85 (d, 1H), 7.39 (d, 4H), 7.26 (d, 2H), 7.07-7.20 (m, 2H), 6.87 (d, 1H), 5.27 (s, 2H), 3.37 (d, 1H), 2.75 (t, 2H), 2.57-2.64 (m, 1H), 2.48 (t, 2H), 1.78 (dd, 1H), 1.59-1.71 (m, 1H), 1.41-1.59 (m, 3H), 1.34 (dd, 1H), 1.25 (dd, 1H), 0.95 (dd, 1H). |
| | 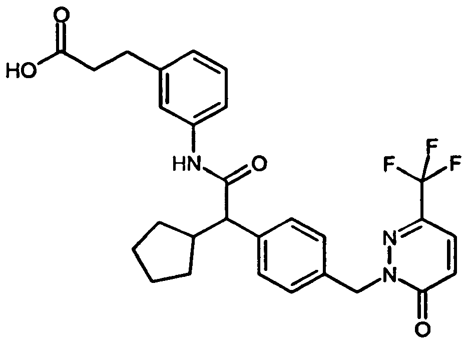 | |
| | (aus Methyl-3-(3-{[cyclopentyl(4-{[6-oxo-3-(trifluor-methyl)pyridazin-1(6*H*)-yl]methyl} phenyl)acetyl]-amino}phenyl)propanoat) | |
| **175** | 1-{3-[(Cyclopentyl{4-[(3-methyl-2-oxopyridin-1(2*H*)-yl)methyl]phenyl}acetyl)amino]benzyl}-cyclopropancarbonsäure | LC-MS (Methode 15): Rₜ = 1.11 min; m/z = 499 (M+H)⁺. ¹H-NMR (400 MHz, DMSO- (400 MHz, DMSO-d₆): δ = 11.5-12.5 (br. s, 1H), 9.97 (s, 1H), 7.62 (d, 1H), 7.39-7.47 (m, 2H), 7.36 (d, 2H), 7.28 (d, 1H), 7.22 (d, 2H), 7.13 (t, 1H), 6.89 (d, 1H), 6.13 (t, 1H), 5.06 (s, 2H), 3.38 (d, 1H), 2.81 (s, 2H), 2.58-2.64 (m, 1H), 1.99 (s, 3H), 1.70-1.82 (m, 1H), 1.58-1.68 (m, 1H), 1.49-1.58 (m, 2H), 1.40-1.49 (m, 1H), 1.29-1.38 (m, 1H), 1.24 (dd, 1H), 1.06-1.13 (m, 2H), 0.94 (dd, 1H), 0.74-0.82 (m, 2H). |
| | 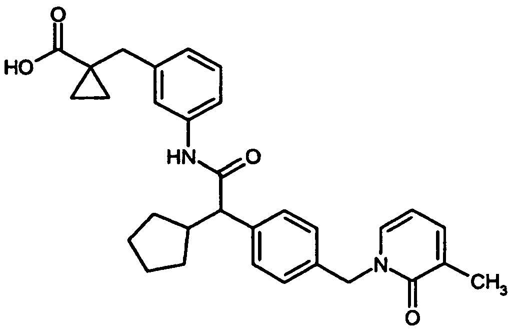 | |
| | (aus *tert*.-Butyl-1-{3-[(cyclopentyl{4-[(3-methyl-2-oxopyridin-1(2*H*)-yl)methyl]phenyl}acetyl)amino]-benzyl}cyclopropancarboxylat) | |
| **176** | 1-(3-{[Cyclopentyl(4-{[2-oxo-4-(trifluormethyl)-pyridin-1(2*H*)-yl]methyl}phenyl)acetyl]amino}-benzyl)cyclopropancarbonsäure | LC-MS (Methode 15): Rₜ = 1.17 min; m/z = 553 (M+H)⁺. ¹H-NMR (400 MHz, DMSO-d₆): δ = 12.08 (s, 1H), 9.97 (s, 1H), 8.06 (d, 1H), 7.36-7.46 (m, 4H), 7.25 (d, 2H), 7.13 (t, 1H), 6.89 (d, 1H), 6.80 (s, 1H), 6.49 (dd, 1H), 5.12 (s, 2H), 3.38 (d, 1H), 2.81 (s, 2H), 2.56-2.64 (m, 1H), 1.72-1.83 (m, 1H), 1.59-1.70 (m, 1H), 1.49-1.58 (m, 2H), 1.40-1.49 (m, 1H), 1.28-1.39 (m, 1H), 1.20-1.29 (m, 1H), 1.07-1.12 (m, 2H), 0.89-1.01 (m, 1H), 0.74-0.80 (m, 2H). |
| | 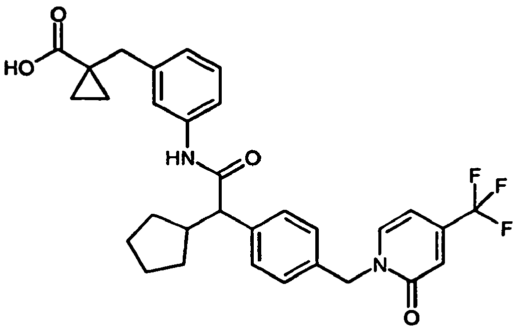 | |
| | (aus *tert*.-Butyl-1-(3-{[cyclopentyl(4-{[2-oxo-4-(trifluormethyl)pyridin-1(2*H*)-yl]methyl}phenyl)-acetyl]amino}benzyl)cyclopropancarboxylat) | |
| **177** | 3-(3-{[(3,3-Difluorcyclopentyl) {4-[(5-oxo-2-phenyl-5,6-dihydro-4*H*-1,3,4-oxadiazin-4-yl)methyl]phenyl}-acetyl]amino}phenyl)propansäure | LC-MS (Methode 11): Rₜ = 1.33 min; m/z = 576 (M+H)⁺. ¹H-NMR (400 MHz, DMSO-d₆): δ = 12.08 (br. s, 1H), 10.06 (d, 1H), 7.76 (d, 2H), 7.38-7.54 (m, 7H), 7.34 (d, 2H), 7.15 (t, 1H), 6.88 (d, 1H), 4.90 (s, 2H), 4.85 (s, 2H), 3.52 (d, 1H), 2.82-2.96 (m, 1H), 2.74 (t, 2H), 2.47 (t, 2H), 1.95-2.38 (m, 3H), 1.79-1.92 (m, 1H), 1.44-1.70 (m, 2H). |
| | 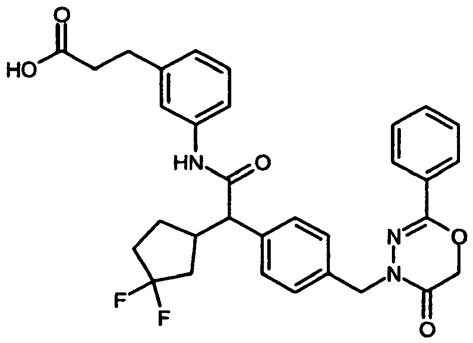 | |
| | (aus Methyl-3-(3-{[(3,3-difluorcyclopentyl){4-[(5-oxo-2-phenyl-5,6-dihydro-4*H*-1,3,4-oxadiazin-4-yl)-methyl]phenyl}acetyl]amino}phenyl)propanoat) | |
| **178** | 1-(3-{[(3,3-Difluorcyclopentyl){4-[(5-oxo-2-phenyl-5,6-dihydro-4*H*-1,3,4-oxadiazin-4-yl)methyl]phenyl}-acetyl]amino}benzyl)cyclopropancarbonsäure | LC-MS (Methode 11): Rₜ = 1.39 min; m/z = 602 (M+H)⁺. ¹H-NMR (400 MHz, DMSO-d₆): δ = 12.07 (br. s, 1H), 10.05 (d, 1H), 7.76 (d, 2H), 7.38-7.53 (m, 7H), 7.34 (d, 2H), 7.14 (t, 1H), 6.90 (d, 1H), 4.90 (s, 2H), 4.85 (s, 2H), 3.49-3.55 (m, 1H), 2.88 (dd, 1H), 2.80 (s, 2H), 1.79-2.39 (m, 4H), 1.46-1.65 (m, 1H), 1.21-1.37 (m, 1H), 1.09 (d, 2H), 0.78 (d, 2H). |
| | 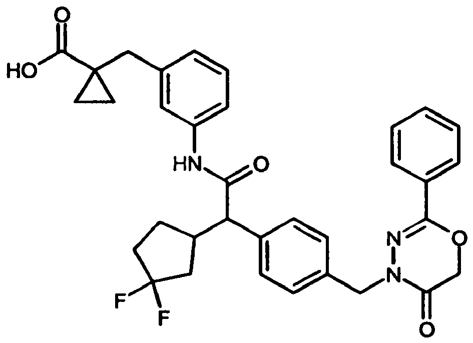 | |
| | (aus *tert*.-Butyl-1-(3-{[(3,3-difluorcyclopentyl){4-[(5-oxo-2-phenyl-5,6-dihydro-4*H*-1,3,4-oxadiazin-4-yl)-methyl]phenyl}acetyl]amino} benzyl)cyclopropan-carboxylat) | |
| **179** | 1-{3-[(Cyclopentyl{4-[(4-methyl-2-oxopyridin-1(2*H*)-yl)methyl]phenyl}acetyl)amino]benzyl}-cyclopropancarbonsäure | LC-MS (Methode 15): Rₜ = 1.06 min; m/z = 499 (M+H)⁺. ¹H-NMR (400 MHz, DMSO-d₆): δ = 12.10 (br. s, 1H), 9.96 (s, 1H), 7.62 (d, 1H), 7.39-7.47 (m, 2H), 7.36 (d, 2H), 7.20 (d, 2H), 7.13 (t, 1H), 6.89 (d, 1H), 6.21 (br. s, 1H), 6.07 (d, 1H), 5.01 (s, 2H), 3.38 (d, 1H), 2.81 (s, 2H), 2.10 (s, 3H), 1.71-1.83 (m, 2H), 1.19-1.68 (m, 6H), 1.10 (br. s, 2H), 0.90-1.01 (m, 1H), 0.78 (br. s, 2H). |
| | 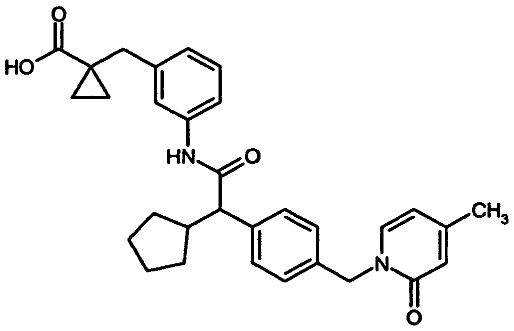 | |
| | (aus *tert*.-Butyl-1-{3-[(cyclopentyl{4-[(4-methyl-2-oxopyridin-1(2*H*)-yl)methyl]phenyl}acetyl)amino]-benzyl} cyclopropancarboxylat) | |
| **180** | 3-(3-{[4,4,4-Trifluor-3-methyl-2-(4-{[6-oxo-3-(trifluormethyl)pyridazin-1(6*H*)-yl]methyl}phenyl)-butanoyl]amino}phenyl)propansäure | LC-MS (Methode 15): Rₜ = 1.11 min; m/z = 556 (M+H)⁺. |
| | 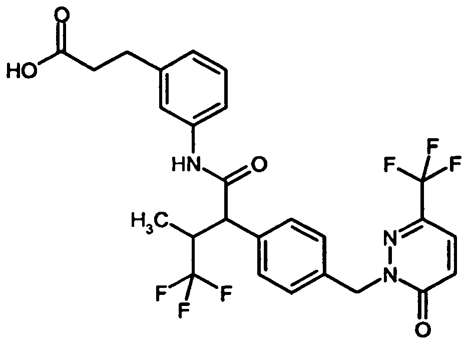 | |
| | (aus Methyl-3-(3-{[4,4,4-trifluor-3-methyl-2-(4-{[6-oxo-3-(trifluormethyl)pyridazin-1(6*H*)-yl]methyl}-phenyl)butanoyl]amino}phenyl)propanoat) | |
| **181** | 1-(3-{[4,4,4-Trifluor-3-methyl-2-(4-{[6-oxo-3-(trifluormethyl)pyridazin-1(6*H*)-yl]methyl}phenyl)-butanoyl]amino}benzyl)cyclopropancarbonsäure (*Enantiomer 1*) | LC-MS (Methode 15): Rₜ = 1.14 min; m/z = 582 (M+H)⁺. ¹H-NMR (400 MHz, DMSO-d₆): δ = 11.85-12.25 (br. s, 1H), 10.15 (s, 1H), 7.84 (d, 1H), 7.41 (d, 3H), 7.36 (s, 1H), 7.30 (d, 2H), 7.19 (d, 1H), 7.14 (t, 1H), 6.91 (d, 1H), 5.29 (s, 2H), 3.82 (d, 1H), 3.28-3.44 (m, 1H), 1.06-1.14 (m, 2H), 0.79 (d, 5H). |
| | 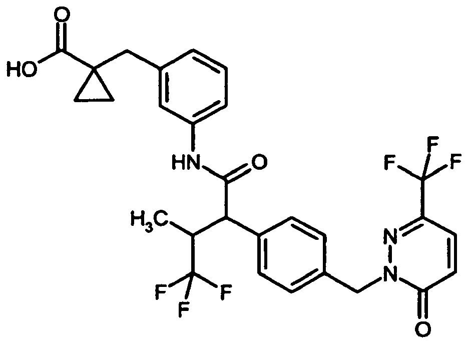 | |
| | (aus *tert*.-Butyl-1-(3-{[4,4,4-trifluor-3-methyl-2-(4-{[6-oxo-3-(trifluormethyl)pyridazin-1(6*H*)-yl]methyl}-phenyl)butanoyl]amino}benzyl)cyclopropancarboxylat (*Enantiomer 1*)) | |
| **182** | 1-(3-{[4,4,4-Trifluor-3-methyl-2-(4-{[6-oxo-3-(trifluormethyl)pyridazin-1(6*H*)-yl]methyl}phenyl)-butanoyl]amino}benzyl)cyclopropancarbonsäure *(Enantiomer 2*) | LC-MS (Methode 15): Rₜ = 1.14 min; m/z = 582 (M+H)⁺. ¹H-NMR (400 MHz, DMSO-d₆): δ = 11.85-12.25 (br. s, 1H), 10.15 (s, 1H), 7.84 (d, 1H), 7.41 (d, 3H), 7.36 (s, 1H), 7.30 (d, 2H), 7.19 (d, 1H), 7.14 (t, 1H), 6.91 (d, 1H), 5.29 (s, 2H), 3.82 (d, 1H), 3.28-3.44 (m, 1H), 1.06-1.14 (m, 2H), 0.79 (d, 5H). |
| | 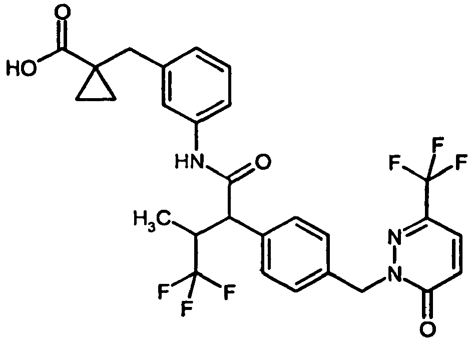 | |
| | (aus *tert*.-Butyl-1-(3-{[4,4,4-trifluor-3-methyl-2-(4-{[6-oxo-3-(trifluormethyl)pyridazin-1(6*H*)-yl]methyl}-phenyl)butanoyl]amino}benzyl)cyclopropancarboxylat (*Enantiomer 2*)) | |
| **183** | 1-(3- {[Cyclopentyl(4-{[2-oxo-3-(trifluormethyl)-pyridin-1(2*H*)-yl]methyl}phenyl)acetyl]amino}-benzyl)cyclopropancarbonsäure | LC-MS (Methode 15): Rₜ = 1.16 min; m/z = 553 (M+H)⁺. ¹H-NMR (400 MHz, DMSO-d₆): δ = 12.07 (s, 1H), 9.97 (s, 1H), 8.14 (d, 1H), 7.93 (d, 1H), 7.36-7.46 (m, 4H), 7.26 (d, 2H), 7.13 (t, 1H), 6.89 (d, 1H), 6.38 (t, 1H), 5.13 (s, 2H), 3.38 (d, 1H), 2.81 (s, 2H), 2.56-2.64 (m, 1H), 1.77 (dd, 1H), 1.41-1.68 (m, 4H), 1.28-1.38 (m, 1H), 1.23-1.29 (m, 1H), 1.10 (d, 2H), 0.95 (dd, 1H), 0.78 (d, 2H). |
| | 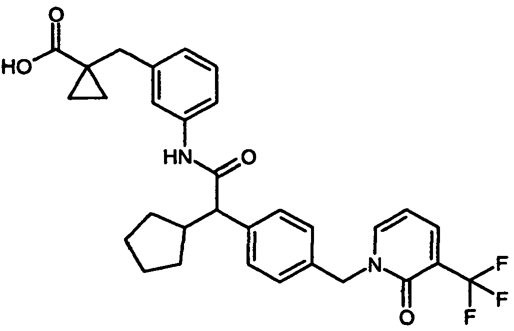 | |
| | (aus *tert*.-Butyl-1-(3-{[cyclopentyl(4-{[2-oxo-3-(trifluormethyl)pyridin-1(2*H*)-yl]methyl}phenyl)-acetyl]amino}benzyl)cyclopropancarboxylat) | |
| **184** | 3-{2-Chlor-3-[(4,4,4-trifluor-3-methyl-2-{4-[(5-oxo-2-phenyl-5,6-dihydro-4*H*-1,3,4-oxadiazin-4-yl)methyl]-phenyl}butanoyl)amino]phenyl}propansäure | LC-MS (Methode 15): Rₜ = 1.21 min; m/z = 602 (M+H)⁺. ¹H-NMR (400 MHz, DMSO-d₆): δ = 12.21 (br. s, 1H), 9.78 (s, 1H), 7.76 (d, 2H), 7.41-7.55 (m, 5H), 7.32-7.39 (m, 3H), 7.19 (t, 1H), 7.12 (d, 1H), 4.92 (s, 2H), 4.88 (s, 2H), 4.10 (d, 1H), 3.36-3.43 (m, 1H), 2.87 (t, 2H), 2.46 (t, 2H), 0.79 (d, 3H). |
| | 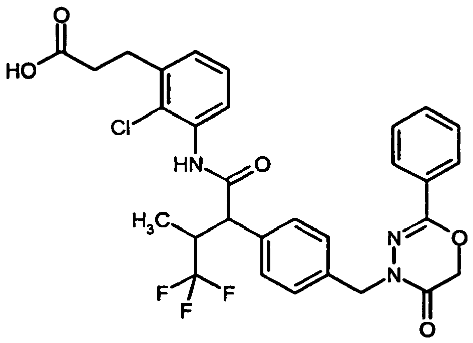 | |
| | (aus *tert*.-Butyl-3-{2-chlor-3-[(4,4,4-trifluor-3-methyl-2-{4-[(5-oxo-2-phenyl-5,6-dihydro-4*H*-1,3,4-oxa-diazin-4-yl)methyl]phenyl}butanoyl)amino]phenyl}-propanoat) | |
| **185** | 3-{4-Fluor-3-[(4,4,4-trifluor-3-methyl-2-{4-[(5-oxo-2-phenyl-5,6-dihydro-4*H*-1,3,4-oxadiazin-4-yl)methyl]-phenyl}butanoyl)amino]phenyl}propansäure | LC-MS (Methode 15): Rₜ = 1.15 min; m/z = 586 (M+H)⁺. ¹H-NMR (400 MHz, DMSO-d₆): δ = 12.09 (br. s, 1H), 9.99 (s, 1H), 7.76 (d, 2H), 7.66 (d, 1H), 7.40-7.53 (m, 5H), 7.36 (d, 2H), 7.09 (dd, 1H), 6.92-7.00 (m, 1H), 4.91 (s, 2H), 4.87 (s, 2H), 4.08 (d, 1H), 3.36-3.42 (m, 1H), 2.73 (t, 2H), 2.41-2.48 (m, 2H), 0.77 (d, 3H). |
| | 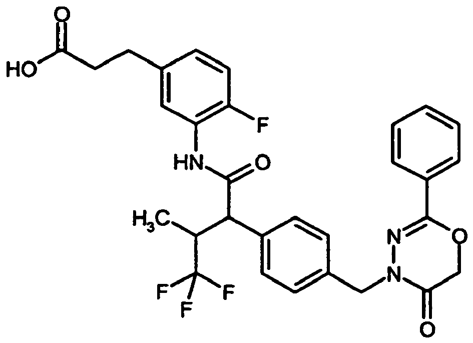 | |
| | (aus *tert*.-Butyl-3-{4-fluor-3-[(4,4,4-trifluor-3-methyl-2-{4-[(5-oxo-2-phenyl-5,6-dihydro-4*H*-1,3,4-oxa-diazin-4-yl)methyl]phenyl}butanoyl)amino]phenyl}-propanoat) | |
| **186** | 1-{4-Fluor-3-[(4,4,4-trifluor-3-methyl-2-{4-[(5-oxo-2-phenyl-5,6-dihydro-4*H*-1,3,4-oxadiazin-4-yl)methyl]-phenyl}butanoyl)amino]benzyl}cyclopropan-carbonsäure | LC-MS (Methode 15): Rₜ = 1.21 min; m/z = 612 (M+H)⁺. ¹H-NMR (400 MHz, DMSO-d₆): δ = 12.11 (br. s, 1 H), 9.97 (s, 1H), 7.76 (d, 2H), 7.67 (d, 1H), 7.40-7.53 (m, 5H), 7.36 (d, 2H), 7.08 (t, 1H), 6.95-7.02 (m, 1H), 4.91 (s, 2H), 4.87 (s, 2H), 4.07 (d, 1H), 3.29-3.40 (m, 1H), 2.79 (s, 2H), 1.07 (d, 2H), 0.77 (d, 5H). |
| | 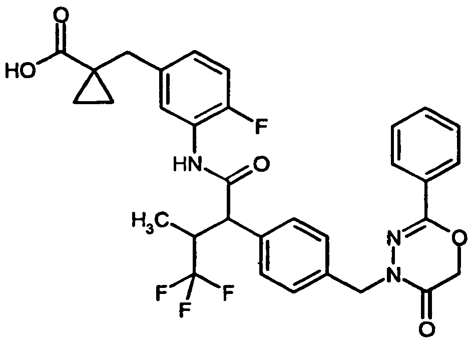 | |
| | (aus *tert*.-Butyl-1-{4-fluor-3-[(4,4,4-trifluor-3-methyl-2-{4-[(5-oxo-2-phenyl-5,6-dihydro-4*H*-1,3,4-oxa-diazin-4-yl)methyl]phenyl}butanoyl)amino]benzyl}-cyclopropancarboxylat) | |
| **187** | 3-{4-Chlor-3-[(cyclopentyl{4-[(5-oxo-2-phenyl-5,6-dihydro-4*H*-1,3,4-oxadiazin-4-yl)methyl]phenyl}-acetyl)amino]phenyl}propansäure | LC-MS (Methode 15): Rₜ = 1.25 min; m/z = 574 (M+H)⁺. ¹H-NMR (400 MHz, DMSO-d₆): δ = 12.01-12.25 (br. s, 1H), 9.60 (s, 1H), 7.79 (d, 2H), 7.38-7.55 (m, 6H), 7.33 (t, 3H), 7.02 (dd, 1H), 4.91 (s, 2H), 4.85 (s, 2H), 3.59 (d, 1H), 2.75 (t, 2H), 2.49-2.62 (m, 1H), 2.47 (t, 2H), 1.76-1.89 (m, 1H), 1.61-1.75 (m, 1H), 1.49-1.61 (m, 2H), 1.41-1.49 (m, 1H), 1.29-1.41 (m, 2H), 0.89-1.04 (m, 1H). |
| | 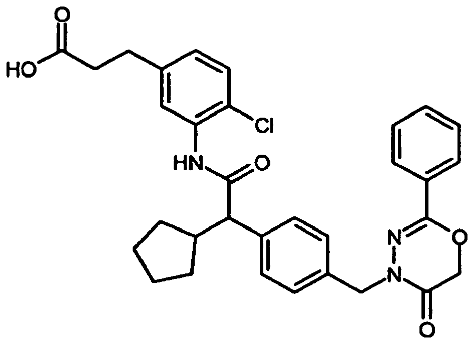 | |
| | (aus *tert*.-Butyl-3-{4-chlor-3-[(cyclopentyl{4-[(5-oxo-2-phenyl-5,6-dihydro-4*H*-1,3,4-oxadiazin-4-yl)-methyl]phenyl}acetyl)amino]phenyl}propanoat) | |

### Beispiel 188 und Beispiel 189

### 1-(3-{[{4-[(3-Chlor-6-oxopyridazin-1(6H)-yl)methyl]phenyl}(cyclopentyl)acetyl]amino}benzyl)-cyclopropancarbonsäure (Enantiomer 1 und 2)

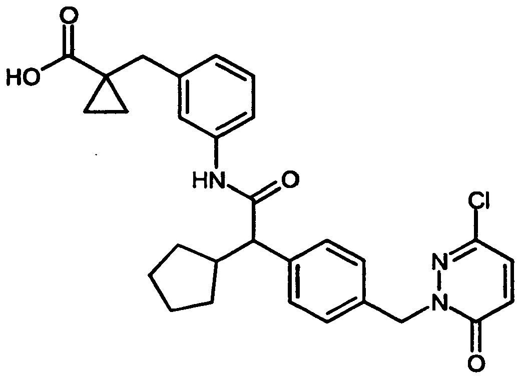

170 mg (0.33 mmol) der racemischen 1-(3-{[{4-[(3-Chlor-6-oxopyridazin-1(6*H*)-yl)methyl]-phenyl}(cyclopentyl)acetyl]amino}benzyl)cyclopropancarbonsäure wurden mittels präparativer HPLC an chiraler Phase in die Enantiomere aufgetrennt [Säule: Daicel Chiralpak AD-H, 5 µm, 250 mm x 20 mm; Elutionsmittel: Isohexan/(Isopropanol + 0.2% Trifluoressigsäure + 1% Wasser) 70:30 (v/v); Fluss: 15 ml/min; UV-Detektion: 220 nm; Temperatur: 30°C]:

### Beispiel 188 (Enantiomer 1):

Ausbeute: 93 mg
Rₜ 7.83 min; Reinheit >99%; >99% ee
[Säule: Daicel Chiralpak AD-H, 5 µm, 250 mm x 4.6 mm; Elutionsmittel: Isohexan/(Isopropanol + 0.2% Trifluoressigsäure + 1 % Wasser) 65:35 (v/v); Fluss: 1 ml/min; UV-Detektion: 220 nm; Temperatur: 25°C].
LC-MS (Methode 15): Rₜ = 1.15 min; m/z = 520 (M+H)⁺.
¹H-NMR (400 MHz, DMSO-d₆): δ = 9.98 (s, 1H), 7.55 (d, 1H), 7.35-7.46 (m, 4H), 7.25 (d, 2H), 7.14 (t, 1H), 7.07 (d, 1H), 6.89 (d, 1H), 5.15 (s, 2H), 3.38 (d, 1H), 2.81 (s, 2H), 2.55-2.62 (m, 1H), 1.77 (dd, 1H), 1.41-1.68 (m, 4H), 1.20-1.40 (m, 2H), 1.07-1.14 (m, 2H), 0.91-1.00 (m, 1H), 0.78 (d, 2H).

### Beispiel 189 (Enantiomer 2):

Ausbeute: 85 mg
Rₜ 9.17 min; Reinheit >99%; >97% ee
[Säule: Daicel Chiralpak AD-H, 5 µm, 250 mm x 4.6 mm; Elutionsmittel: Isohexan/(Isopropanol + 0.2% Trifluoressigsäure + 1% Wasser) 65:35 (v/v); Fluss: 1 ml/min; UV-Detektion: 220 nm; Temperatur: 25°C].
LC-MS (Methode 15): Rₜ = 1.15 min; m/z = 520 (M+H)⁺.
¹H-NMR (400 MHz, DMSO-d₆): δ = 9.98 (s, 1H), 7.55 (d, 1H), 7.35-7.47 (m, 4H), 7.25 (d, 2H), 7.14 (t, 1H), 7.07 (d, 1H), 6.89 (d, 1H), 5.15 (s, 2H), 3.38 (d, 1H), 2.81 (s, 2H), 2.56-2.64 (m, 1H), 1.69-1.84 (m, 1H), 1.40-1.67 (m, 4H), 1.19-1.38 (m, 2H), 1.10 (d, 2H), 0.90-1.01 (m, 1H), 0.78 (d, 2H).

### Beispiel 190 und Beispiel 191

### 3-{2-Methyl-3-[(4,4,4-trifluor-3-methyl-2-{4-[(5-oxo-2-phenyl-5,6-dihydro-4H-1,3,4-xadiazin-4-yl)methyl]phenyl}butanoyl)amino]phenyl}propansäure (Isomer 1 und 2)

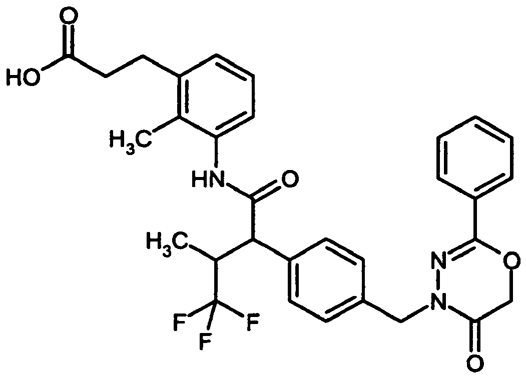

120 mg (0.21 mmol) des Isomerengemisches von 3-{2-Methyl-3-[(4,4,4-trifluor-3-methyl-2-{4-[(5-oxo-2-phenyl-5,6-dihydro-4*H*-1,3,4-oxadiazin-4-yl)methyl]phenyl}butanoyl)amino]phenyl}propansäure wurden mittels präparativer HPLC an chiraler Phase weiter aufgetrennt [Säule: Daicel Chiralpak AD-H, 5 µm, 250 mm x 20 mm; Elutionsmittel: Isohexan/(Isopropanol + 0.2% Trifluoressigsäure + 1% Wasser) 70:30 (v/v); Fluss: 15 ml/min; UV-Detektion: 220 nm; Temperatur: 30°C]:

### Beispiel 190 (Isomer 1):

Ausbeute: 37 mg
Rₜ 6.59 min; Reinheit >99%; >99% ee
[Säule: Daicel Chiralpak AD-H, 5 µm, 250 mm x 4.6 mm; Elutionsmittel: Isohexan/(Isopropanol + 0.2% Trifluoressigsäure + 1% Wasser) 65:35 (v/v); Fluss: 1 ml/min; UV-Detektion: 220 nm; Temperatur: 25°C].
LC-MS (Methode 15): Rₜ = 1.16 min; m/z = 582 (M+H)⁺.
¹H-NMR (400 MHz, DMSO-d₆): δ = 12.13 (br. s, 1H), 9.62 (s, 1H), 7.76 (d, 2H), 7.40-7.55 (m, 5H), 7.36 (d, 2H), 6.92-7.07 (m, 3H), 4.92 (s, 2H), 4.89 (s, 2H), 3.91 (d, 1H), 3.36-3.44 (m, 1H), 2.73 (t, 2H), 2.39 (t, 2H), 1.87 (s, 3H), 0.79 (d, 3H).
[α]_{D}²⁰ = +64.0°, c = 0.420, Methanol.

### Beispiel 191 (Isomer 2):

Ausbeute: 35 mg
Rₜ 7.25 min; Reinheit >99%; >96% ee
[Säule: Daicel Chiralpak AD-H, 5 µm, 250 mm x 4.6 mm; Elutionsmittel: Isohexan/(Isopropanol + 0.2% Trifluoressigsäure + 1 % Wasser) 65:35 (v/v); Fluss: 1 ml/min; UV-Detektion: 220 nm; Temperatur. 25°C].
LC-MS (Methode 15): Rₜ = 1.16 min; m/z = 582 (M+H)⁺.
¹H-NMR (400 MHz, DMSO-d₆): δ = 12.13 (br. s, 1H), 9.62 (s, 1H), 7.76 (d, 2H), 7.40-7.55 (m, 5H), 7.36 (d, 2H), 6.92-7.07 (m, 3H), 4.92 (s, 2H), 4.89 (s, 2H), 3.91 (d, 1H), 3.36-3.44 (m, 1H), 2.73 (t, 2H), 2.39 (t, 2H), 1.87 (s, 3H), 0.79 (d, 3H).
[α]_{D}²⁰ = -62.6°, c = 0.420, Methanol.

### Beispiele 192 -195

### 3-{3-[(4,4,4-Trifluor-3-methyl-2-{4-[(5-oxo-2-phenyl-5,6-dihydro-4H-1,3,4-oxadiazin-4-yl)-methyl]phenyl}butanoyl)amino]phenyl}propansäure (Isomer 1 - 4)

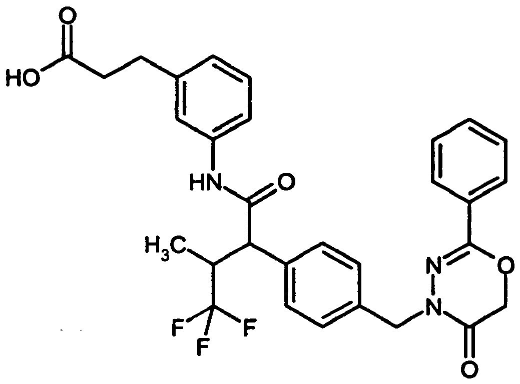

125 mg (0.22 mmol) des Isomerengemisches von 3-{3-[(4,4,4-Trifluor-3-methyl-2-{4-[(5-oxo-2-phenyl-5,6-dihydro-4*H*-1,3,4-oxadiazin-4-yl)methyl]phenyl}butanoyl)amino]phenyl}propansäure wurden mittels präparativer HPLC an chiraler Phase weiter aufgetrennt [Säule: Daicel Chiralpak AD-H, 5 µm, 250 mm x 20 mm; Elutionsmittel: Isohexan/(Isopropanol + 0.2% Trifluoressigsäure + 1% Wasser) 70:30 (v/v); Fluss: 15 ml/min; UV-Detektion: 220 nm; Temperatur: 30°C]. Zunächst wurden 22 mg von *Isomer 4* isoliert. Das gleichfalls erhaltene Gemisch der Isomeren 1 bis 3 wurde durch erneute präparative HPLC an chiraler Phase aufgetrennt [Säule: Daicel Chiralpak AS-H, 5 µm, 250 mm x 20 mm; Elutionsmittel: Isohexan/(Ethanol + 0.2% Trifluoressigsäure + 1% Wasser) 75:25 (v/v); Fluss: 15 ml/min; UV-Detektion: 220 nm; Temperatur: 30°C]. Es wurden 26 mg von *Isomer 1* isoliert. Das restliche Gemisch der Isomeren 2 und 3 wurde durch nochmalige präparative HPLC an chiraler Phase aufgetrennt [Säule: Daicel Chiralpak AD-H, 5 µm, 250 mm x 20 mm; Elutionsmittel: Isohexan/(Ethanol + 0.2% Trifluoressigsäure + 1% Wasser) 75:25 (v/v); Fluss: 15 ml/min; UV-Detektion: 220 nm; Temperatur: 30°C]. Es wurden 11 mg von *Isomer 2* und 19 mg von *Isomer 3* isoliert.

### Beispiel 192 (Isomer 1):

Ausbeute: 26 mg
Rₜ 6.63 min; Reinheit >98.5%; >97% ee
[Säule: Daicel Chiralpak AS-H, 5 µm, 250 mm x 4.6 mm; Elutionsmittel: Isohexan/(Ethanol + 0.2% Trifluoressigsäure + 1% Wasser) 75:25 (v/v); Fluss: 1 ml/min; UV-Detektion: 220 nm; Temperatur: 25°C].
LC-MS (Methode 15): Rₜ = 1.16 min; m/z = 568 (M+H)⁺.
¹H-NMR (400 MHz, DMSO-d₆): δ = 10.18 (s, 1H), 7.75 (d, 2H), 7.32-7.54 (m, 10H), 7.15 (t, 1H), 6.88 (d, 1H), 4.90 (s, 2H), 4.86 (s, 2H), 3.82 (d, 1H), 2.73 (t, 2H), 2.46 (t, 2H), 0.78 (d, 3H).

### Beispiel 193 (Isomer 2):

Ausbeute: 11 mg
Rₜ 6.36 min; Reinheit >95%; >97% ee
[Säule: Daicel Chiralpak AD-H, 5 µm, 250 mm x 4.6 mm; Elutionsmittel: Isohexan/(Ethanol + 0.2% Trifluoressigsäure + 1% Wasser) 75:25 (v/v); Fluss: 1 ml/min; UV-Detektion: 220 nm; Temperatur: 25°C].
LC-MS (Methode 11):Rₜ = 1.34 min; m/z = 568 (M+H)⁺.

### Beispiel 194 (Isomer 3):

Ausbeute: 19 mg
Rₜ 7.06 min; Reinheit >96%; >96% ee
[Säule: Daicel Chiralpak AD-H, 5 µm, 250 mm x 4.6 mm; Elutionsmittel: Isohexan/(Ethanol + 0.2% Trifluoressigsäure + 1% Wasser) 75:25 (v/v); Fluss: 1 ml/min; UV-Detektion: 220 nm; Temperatur: 25°C].
LC-MS (Methode 11): Rₜ = 1.34 min; m/z = 568 (M+H)⁺.

### Beispiel 195 (Isomer 4):

Ausbeute: 22 mg
Rₜ 7.82 min; Reinheit >90%; >98% ee
[Säule: Daicel Chiralpak AD-H, 5 µm, 250 mm x 4.6 mm; Elutionsmittel: Isohexan/(Ethanol + 0.2% Trifluoressigsäure + 1% Wasser) 75:25 (v/v); Fluss: 1 ml/min; UV-Detektion: 220 nm; Temperatur: 25°C].

### Beispiel 196 und Beispiel 197

### 1-{3-[(Cyclopentyl{4-[(3-methyl-6-oxopyridazin-1(6H)-yl)methyl]phenyl}acetyl)amino]benzyl}-cyclopropancarbonsäure (Enantiomer 1 und 2)

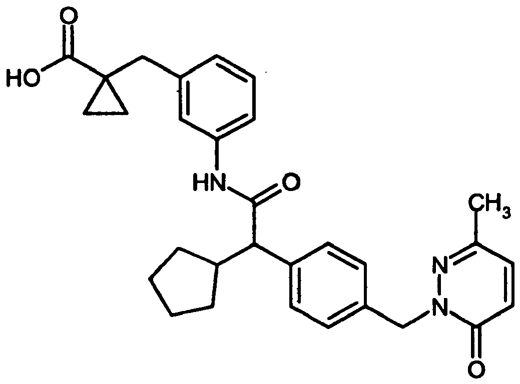

180 mg (0.36 mmol) der racemischen 1-{3-[(Cyclopentyl{4-[(3-methyl-6-oxopyridazin-1(6*H*)-yl)-methyl]phenyl}acetyl)amino]benzyl}cyclopropancarbonsäure wurden mittels präparativer HPLC an chiraler Phase in die Enantiomere aufgetrennt [Säule: Daicel Chiralpak AD-H, 5 µm, 250 mm x 20 mm; Elutionsmittel: Isohexan/(Isopropanol + 0.2% Trifluoressigsäure + 1% Wasser) 70:30 (v/v); Fluss: 15 ml/min; UV-Detektion: 220 nm; Temperatur: 30°C]:

### Beispiel 196 (Enantiomere 1):

Ausbeute: 94 mg
Rₜ 8.74 min; Reinheit >99%; >99% ee
[Säule: Daicel Chiralpak AD-H, 5 µm, 250 mm x 4.6 mm; Elutionsmittel: Isohexan/(Isopropanol + 0.2% Trifluoressigsäure + 1% Wasser) 65:35 (v/v); Fluss: 1 ml/min; UV-Detektion: 220 nm; Temperatur: 25°C].
LC-MS (Methode 15): Rₜ = 1.09 min; m/z = 500 (M+H)⁺.

### Beispiel 197 Enantiomer 2):

Ausbeute: 84 mg
Rₜ 10.46 min; Reinheit >99%; >98% ee
[Säule: Daicel Chiralpak AD-H, 5 µm, 250 mm x 4.6 mm; Elutionsmittel: Isohexan/(Isopropanol + 0.2% Trifluoressigsäure + 1% Wasser) 65:35 (v/v); Fluss: 1 ml/min; UV-Detektion: 220 nm; Temperatur: 25°C].
LC-MS (Methode 15): Rₜ = 1.09 min; m/z = 500 (M+H)⁺.

### Beispiel 198 und Beispiel 199

### 3-(3-{[Cyclopentyl(4-{[6-oxo-3-(trifluormethyl)pyridazin-1(6H)-yl]methyl}phenyl)acetyl]amino}-2-methylphenyl)propansäure (Enantiomer 1 und 2)

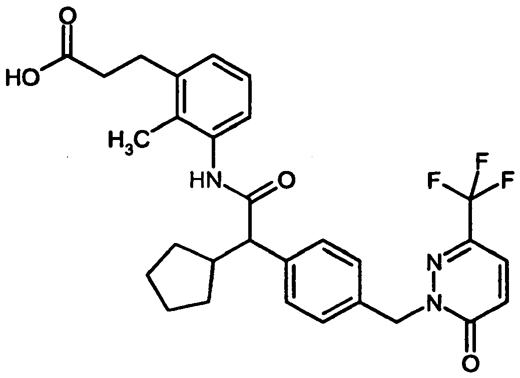

110 mg (0.20 mmol) der racemischen 3-(3-{[Cyclopentyl(4-{[6-oxo-3-(trifluormethyl)pyridazin-1(6*H*)-yl]methyl}phenyl)acetyl]amino}-2-methylphenyl)propansäure wurden mittels präparativer HPLC an chiraler Phase in die Enantiomere aufgetrennt [Säule: Daicel Chiralpak AD-H, 5 µm, 250 mm x 20 mm; Elutionsmittel: Isohexan/(Isopropanol + 0.2% Trifluoressigsäure + 1 % Wasser) 70:30 (v/v); Fluss: 15 ml/min; UV-Detektion: 220 nm; Temperatur: 30°C]:

### Beispiel 198 (Enantiomer 1):

Ausbeute: 94 mg
Rₜ 9.32 min; Reinheit >92.5%; >99% ee
[Säule: Chiralcel OD-H, 5 µm, 250 mm x 4.6 mm; Elutionsmittel: Isohexan/(Isopropanol + 0.2% Trifluoressigsäure + 1 % Wasser) 70:30 (v/v); Fluss: 1 ml/min; UV-Detektion: 220 nm; Temperatur: 25°C].
LC-MS (Methode 11): Rₜ = 1.29 min; m/z = 542 (M+H)⁺.

### Beispiel 199 (Enantiomer 2):

Ausbeute: 84 mg
Rₜ 7.84 min; Reinheit >98%; >96% ee
[Säule: Chiralcel OD-H, 5 µm, 250 mm x 4.6 mm; Elutionsmittel: Isohexan/(Isopropanol + 0.2% Trifluoressigsäure + 1% Wasser) 70:30 (v/v); Fluss: 1 ml/min; UV-Detektion: 220 nm; Temperatur: 25°C].
LC-MS (Methode 11): Rₜ = 1.29 min; m/z = 542 (M+H)⁺.

### Beispiel 200 und Beispiel 201

### 1-(3-{[Cyclopentyl(4-{[6-oxo-3-(trifluormethyl)pyridazin-1(6H)-yl]methyl}phenyl)acetyl]amino}-benzyl)cyclopropancarbonsäure (Enantiomer 1 und 2)

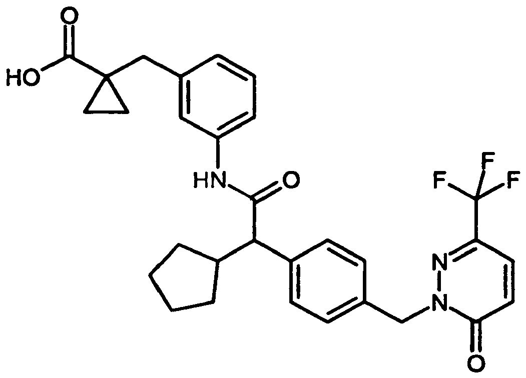

130 mg (0.24 mmol) der racemischen 1-(3-{[Cyclopentyl(4-{[6-oxo-3-(trifluormethyl)pyridazin-1(6*H*)-yl]methyl}phenyl)acetyl]amino}benzyl)cyclopropancarbonsäure wurden mittels präparativer HPLC an chiraler Phase in die Enantiomere aufgetrennt [Säule: Daicel Chiralpak AD-H, 5 µm, 250 mm x 20 mm; Elutionsmittel: Isohexan/(Isopropanol + 0.2% Trifluoressigsäure + 1% Wasser) 70:30 (v/v); Fluss: 15 ml/min; UV-Detektion: 220 nm; Temperatur: 30°C]:

### Beispiel 200 (Enantiomer 1):

Ausbeute: 67 mg
Rₜ 6.58 min; Reinheit >99%; >99% ee
[Säule: Daicel Chiralpak AD-H, 5 µm, 250 mm x 4.6 mm; Elutionsmittel: Isohexan/(Isopropanol + 0.2% Trifluoressigsäure + 1% Wasser) 70:30 (v/v); Fluss: 1 ml/min; UV-Detektion: 220 nm; Temperatur: 25°C].
LC-MS (Methode 11): Rₜ = 1.38 min; m/z = 554 (M+H)⁺.

### Beispiel 201 Enantiomer 2):

Ausbeute: 60 mg
Rₜ 8.10 min; Reinheit >99%; >97.5% ee
[Säule: Daicel Chiralpak AD-H, 5 µm, 250 mm x 4.6 mm; Elutionsmittel: Isohexan/(Isopropanol + 0.2% Trifluoressigsäure + 1% Wasser) 70:30 (v/v); Fluss: 1 ml/min; UV-Detektion: 220 nm; Temperatur: 25°C].
LC-MS (Methode 11): Rₜ = 1.38 min; m/z = 554 (M+H)⁺.

### Allgemeine Vorschrift 8: Spaltung von tert.-Butylestern zu den entsprechenden Carbonsäuren mit Chlorwasserstoff

Der betreffende *tert*.-Butylester wurde bei RT in einer 4 N Lösung von Chlorwasserstoff-Gas in 1,4-Dioxan gelöst (ca. 0.05 bis 0.2 mol/l) und 2-6 h bei RT gerührt. Das Reaktionsgemisch wurde dann eingefroren (ca. -76°C) und im Hochvakuum lyophilisiert. Falls erforderlich, wurde das Produkt durch präparative RP-HPLC gereinigt (Eluent: Acetonitril/Wasser-Gradient).

Die folgenden Beispiele wurden gemäß der Allgemeinen Vorschrift 8 hergestellt:

| **Beispiel** | **Name / Struktur** | **Analytische Daten** |
|---|---|---|
| **202** | 1-{3-[(Cyclopentyl{4-[(1-oxo-1,3-dihydro-2*H*-iso-indol-2-yl)methyl]phenyl}acetyl)amino]benzyl}-cyclopropancarbonsäure | LC-MS (Methode 12): Rₜ = 2.38 min; m/z = 523 (M+H)⁺. ¹H-NMR (400 MHz, DMSO-*d*₆): δ = 9.98 (s, 1H), 7.72 (d, 1H), 7.61-7.36 (m, 8H), 7.22 (d, 2H), 7.13 (t, 1H), 6.89 (d, 1H), 4.69 (s, 2H), 4.35 (s, 2H), 3.57 (s, 2H), 3.37 (d, 1H), 2.62-2.55 (m, 1H), 1.82-1.72 (m, 1H), 1.69-1.18 (m, 6H), 1.10 (q, 2H), 0.86-1.00 (m, 1H), 0.78 (q, 2H). |
| | 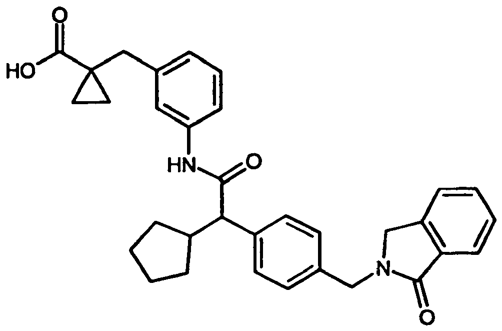 | |
| **203** | 1-(3-{[(2*S*)-2-Cyclopentyl-2-{4-[(1-oxo-1,3-dihydro-2*H*-isoindol-2-yl)methyl]phenyl}acetyl]amino}-benzyl)cyclopropancarbonsäure | LC-MS (Methode 11): Rₜ = 1.29 min; m/z = 523 (M+H)⁺. ¹H-NMR (400 MHz, DMSO-*d*₆): δ = 12.07 (br. s, 1H), 9.98 (s, 1H), 7.72 (d, 2H), 7.62-7.36 (m, 7H), 7.22 (d, 2H), 7.13 (t, 1H), 6.89 (d, 1H), 4.69 (s, 2H), 4.35 (s, 2H), 2.81 (s, 2H), 2.65-2.59 (m, 1H), 1.85-1.72 (m, 1H), 1.69-1.42 (m, 4H), 1.40-1.30 (m, 1H), 1.28-1.18 (m, 1H), 1.13-1.07 (m, 2H), 1.02-0.89 (m, 1H), 0.83-0.75 (m, 2H). |
| | 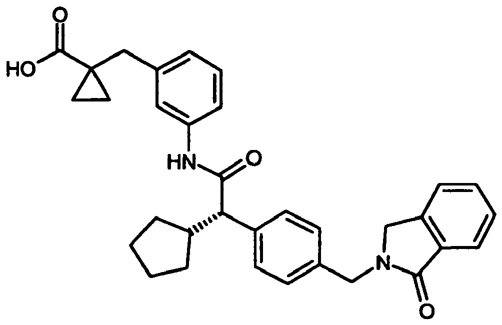 | |

### Beispiel 204

### (+)-1-(3-{[(2S)-2-Cyctopentyl-2-{4-[(5-oxo-2-phenyl-5,6-dihydro-4H-1,3,4-oxadiazin-4-yl)-methyl]phenyl}acetyl]amino}benzyl)cyclopropancarbonsäure

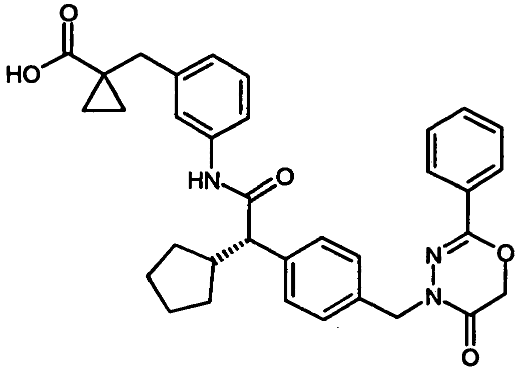

4.24 g (0.6.82 mmol) *tert*.-Butyl-1-(3-{[(2*S*)-2-cyclopentyl-2-{4-[(5-oxo-2-phenyl-5,6-dihydro-4*H-*1,3,4-oxadiazin-4-yl)methyl]phenyl}acetyl]amino}benzyl)cyclopropancarboxylat wurden in 20 ml Dichlormethan vorgelegt und mit 5.25 ml Trifluoressigsäure versetzt. Es wurde 2.5 h bei RT gerührt. Das Reaktionsgemisch wurde dann im Vakuum eingeengt, der Rückstand auf Wasser gegeben und die Mischung dreimal mit Ethylacetat extrahiert. Die vereinigten organischen Phasen wurden über Magnesiumsulfat getrocknet und im Vakuum eingeengt. Das Produkt wurde durch Chromatographie an Silicagel gereinigt (LaufiMittel Cyclohexan/Ethylacetat 2:1 + 0.1% Essigsäure). Es wurden 3.6 g (89% d. Th.) der Titelverbindung erhalten.
LC-MS (Methode 15): Rₜ = 1.27 min; m/z = 566 (M+H)⁺.
¹H-NMR (400 MHz, DMSO-*d*₆): δ = 12.05 (br. s, 1 H), 9.97 (s, 1 H), 7.76 (d, 2 H), 7.54-7.35 (m, 7 H), 7.31 (d, 2 H), 7.13 (t, 1 H), 6.88 (d, 1 H), 4.89 (s, 2 H), 4.84 (s, 2 H), 3.38 (d, 1 H), 2.80 (s, 2 H), 2.66-2.56 (m, 1 H), 1.85-1.21 (m, 6 H), 1.09 (q, 3 H), 1.02-0.93 (m, 1 H), 0.81-0.74 (m, 2 H).
[α]_{D}²⁰ = +28.0°, c = 0.570, Chloroform.

### Allgemeine Vorschrift 9A: Spaltung von tert.-Butylestem zu den entsprechenden Carbonsäuren mit Trifluoressigsäure

Zu einer Lösung des betreffenden *tert*.-Butylesters in Dichlormethan (Konzentration 0.1 bis 1.0 mol/l; zusätzlich optional ein Tropfen Wasser) wurde bei 0°C bis RT tropfenweise Trifluoressigsäure (TFA) hinzugefügt, bis ein DichlonnethanrTFA-Verhältnis von ca. 2:1 bis 1:2 (v/v) erreicht war. Die Mischung wurde 1-18 h bei RT gerührt (gegebenenfalls Erwärmung bis auf 40°C, bis vollständiger Umsatz erreicht war) und dann im Vakuum eingeengt. Das Rohprodukt wurde, falls erforderlich, durch Kristallisation aus Wasser/Acetonitril-Gemischen oder durch präparative RP-HPLC (Eluent: Acetonitril/Wasser-Gradient) gereinigt.

Die folgenden Beispiele wurden gemäß der Allgemeinen Vorschrift 9A hergestellt:

| **Beispiel** | **Name / Struktur** | **Analytische Daten** |
|---|---|---|
| **205** | (+)-1-(3-{[(2*S*)-2-Cyclopentyl-2-{4-[(5-oxo-2-phenyl-5,6-dihydro-4*H*-1,3,4-oxadiazin-4-yl)methyl]phenyl}-acetyl]amino}-2-methylbenzyl)cyclopropancarbonsäure | LC-MS (Methode 7): Rₜ = 2.71 min; m/z = 580 (M+H)⁺. ¹H-NMR (400 MHz, DMSO-*d₆):* δ = 12.28-12.06 (m, 1H), 9.48 (s, 1H), 7.77 (d, 2H), 7.55-7.35 (m, 5H), 7.31 (d, 2H), 7.06-6.91 (m, 3H), 4.92 (s, 2H), 4.87 (s, 2H), 3.49-3.41 (m, 1H), 2.89 (s, 2H), 2.65-2.57 (m, 1H), 1.89 (s, 3H), 1.87-1.78 (m, 1H), 1.73-1.61 (m, 1H), 1.62-1.52 (m, 2H), 1.50-1.42 (m, 1H), 1.40-1.29 (m, 2H), 1.13 (d, 2H), 1.03-0.91 (m, 1H), 0.59 (d, 2H). [α]_{D}²⁰ = +21.0°, c = 0.500, Chloroform. |
| | 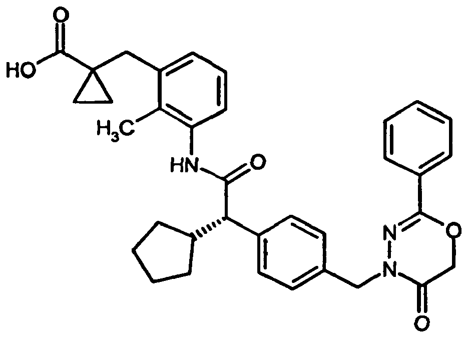 | |
| **206** | 1-{3-[(Cyclopentyl{4-[(3-oxo-1,3-dihydro-2*H* indazol-2-yl)methyl]phenyl}acetyl)amino]benzyl}-cyclopropancarbonsäure | LC-MS (Methode 11): Rₜ = 1.27 min; m/z = 524 (M+H)⁺. |
| | 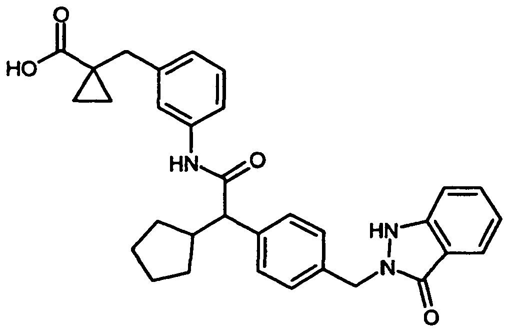 | |
| **207** | 1-(3-{[(2*S*,3*R*)-4,4,4-Trifluor-3-methyl-2-{4-[(1-oxo-1,3-dihydro-2*H*-isoindol-2-yl)methyl]phenyl}-butanoyl]amino}benzyl)cyclopropancarbonsäure | LC-MS (Methode 15): Rₜ = 1.11 min; m/z =551 (M+H)⁺. ¹H-NMR (400 MHz, DMSO-*d*₆): δ =10.15 (s, 1H), 7.72 (d, 1H), 7.61-7.46 (m, 3H), 7.447.39 (m, 3H), 7.36 (s, 1H), 7.27 (d, 2H), 7.14 (t, 1H), 6.90 (d, 1H), 4.71 (s, 2H), 4.36 (s, 2H), 3.82 (d, 1H), 2.81 (s, 2H), 1.09 (d, 2H), 0.81-0.73 (m, 5H). |
| | 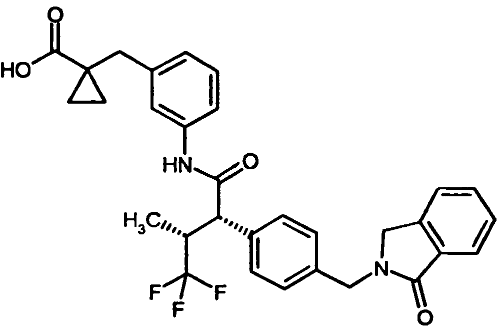 | |
| **208** | 1-(3-{[(2*R*,3*S*)-4,4,4-Trifluor-3-methyl-2-{4-[(1-oxo-1,3-dihydro-2*H*-isoindol-2-yl)methyl]phenyl}-butanoyl]amino}benzyl)cyclopropancarbonsäure | LC-MS (Methode 15): Rₜ = 1.11 min; m/z = 551 (M+H)⁺. ¹H-NMR (400 MHz, DMSO-*d*₆): δ = 10.15 (s, 1H), 7.72 (d, 1H), 7.62-7.46 (m, 3H), 7.45-7.39 (m, 3H), 7.36 (s, 1H), 7.27 (d, 2H), 7.14 (t, 1H), 6.90 (d, 1H), 4.71 (s, 2H), 4.36 (s, 2H), 3.82 (d, 1H), 2.81 (s, 2H), 1.13-1.07 (m, 2H), 0.77 (d, 5H). |
| | 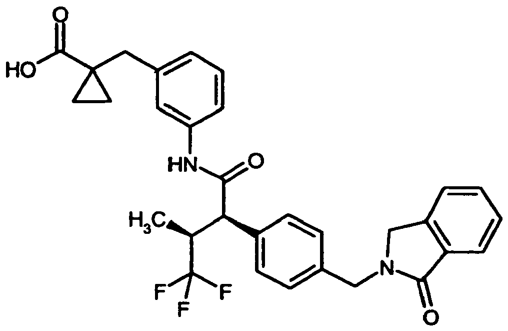 | |
| **209** | 1-{3-[(Cyclopentyl{4-[(5,6-difluor-1-oxo-1,3-dihydro-2*H*-isoindol-2-yl)methyl]phenyl}acetyl)amino]-benzyl}cyclopropancarbonsäure | LC-MS (Methode 11): Rₜ = 1.35 min; m/z = 559 (M+H)⁺. ¹H-NMR (400 MHz, DMSO-*d*₆): δ = 9.98 (s, 1H), 7.76 (t, 1H), 7.65 (dd, 1H), 7.47-7.36 (m, 4H), 7.22 (d, 2H), 7.14 (t, 1H), 6.89 (d, 1H), 4.68 (s, 2H), 4.33 (s, 2H), 3.38 (d, 1H), 3.17 (d, 1H), 2.81 (s, 2H), 2.64-2.56 (m, 1H), 1.38-1.18 (m, 6H), 1.14-1.07 (m, 2H), 0.99-0.91 (m, 1H), 0.81-0.74 (m, 2H). |
| | 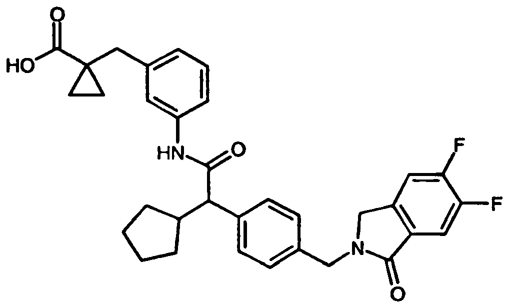 | |
| **210** | 1-{3-[(Cyclopentyl{4-[(4,7-difluor-1-oxo-1,3-dihydro-2*H*-isoindol-2-yl)methyl]phenyl}acetyl)amino]-benzyl}cyclopropancarbonsäure | LC-MS (Methode 11): Rₜ = 1.34 min; m/z = 559 (M+H)⁺. ¹H-NMR (400 MHz, DMSO-*d*₆): δ = 9.98 (s, 1H), 7.51-7.37 (m, 5H), 7.33 (dt, 1H), 7.25 (d, 2H), 7.13 (t, 1H), 6.89 (d, 1H), 4.65 (s, 2H), 4.47 (s, 2H), 3.38 (d, 1H), 2.81 (s, 2H), 2.63-2.56 (m, 1H), 1.84-1.20 (m, 8H), 1.13-1.08 (m, 2H), 1.02-0.91 (m, 1H), 0.81-0.74 (m, 2H). |
| | 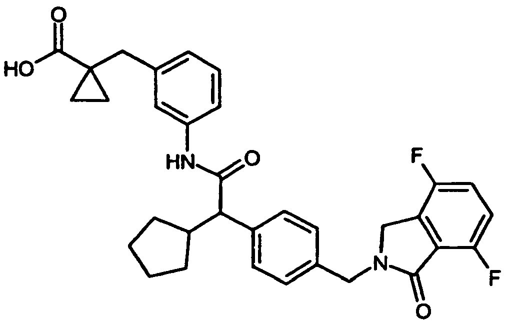 | |
| **211** | 1-(3-{[{4-[(5-Chlor-2-oxopyridin-1(2*H*)-yl)methyl]-phenyl}(cyclopentyl)acetyl]amino}benzyl)-cyclopropancarbonsäure | LC-MS (Methode 15): Rₜ = 1.12 min; m/z = 519 (M+H)⁺. ¹H-NMR (400 MHz, DMSO-*d*₆): δ = 9.97 (s, 1H), 8.09 (s, 1H), 7.48 (dd, 1H), 7.42 (s, 2H), 7.38 (d, 2H), 7.25 (d, 2H), 7.13 (t, 1H), 6.89 (d, 1H), 6.45 (d, 1H), 5.03 (s, 2H), 3.39 (d, 1H), 2.81 (s, 2H), 2.63-2.56 (m, 1H), 1.84-1.72 (m, 1H), 1.69-1.19 (m, 6H), 1.10 (q, 2H), 1.00-0.89 (m, 1H), 0.81-0.75 (m, 2H). |
| | 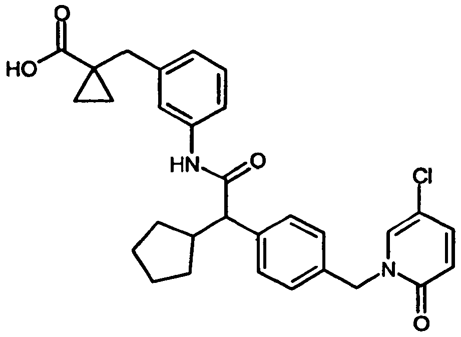 | |
| **212** | 1-(3-{[Cyclopentyl(4-{[2-oxo-5-(trifluormethyl)-pyridin-1(2*H*)-yl]methyl}phenyl)acetyl]amino}-benzyl)cyclopropancarbonsäure | LC-MS (Methode 15): Rₜ = 1.17 min; m/z = 553 (M+H)⁺. ¹H-NMR (400 MHz, DMSO-*d*₆): δ = 9.97 (s, 1H), 8.54 (s, 1H), 7.67 (dd, 1H), 7.47-7.35 (m, 4H), 7.25 (d, 2H), 7.13 (t, 1H), 6.89 (d, 1H), 6.55 (d, 1H), 5.12 (s, 2H), 3.57 (s, 3H), 3.37 (d, 1H), 2.81 (s, 2H), 2.65-2.56 (m, 1H), 1.84-1.19 (m, 4H), 1.13-1.07 (m, 2H), 1.03-0.88 (m, 1H), 0.81-0.74 (m, 2H). |
| | 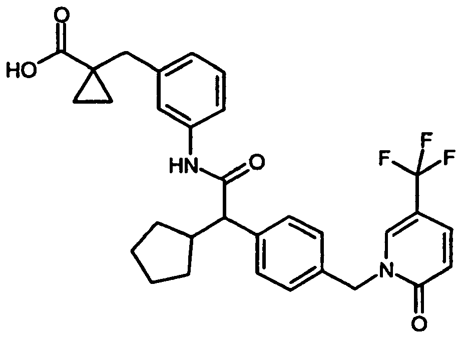 | |
| **213** | 1-{3-[(Cyclopentyl{4-[(4-fluor-1-oxo-1,3-dihydro-2*H-*isoindol-2-yl)methyl]phenyl}acetyl)amino]benzyl}-cyclopropancarbonsäure | LC-MS (Methode 15): Rₜ = 1.18 min; m/z = 541 (M+H)⁺. ¹H-NMR (400 MHz, DMSO-*d*₆): δ = 9.98 (s, 1H), 7.61-7.53 (m, 2H), 7.46-7.36 (m, 5H), 7.24 (d, 2H), 7.13 (t, 1H), 6.89 (d, 1H), 4.69 (s, 2H), 4.48 (s, 2H), 3.38 (d, 1H), 2.81 (s, 2H), 2.63-2.55 (m, 1H), 1.83-1.21 (m, 7H), 1.10 (q, 2H), 1.00-0.91 (m, 1H), 0.78 (q, 2H). |
| | 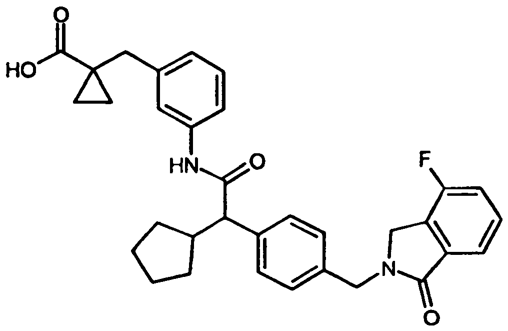 | |
| **214** | (+)-1-(3-{[(2*S*)-2-Cyclopentyl-2-{4-[(4-fluor-1-oxo-1,3-dihydro-2*H*-isoindol-2-yl)methyl]phenyl}acetyl]-amino}benzyl)cyclopropancarbonsäure | LC-MS (Methode 15): Rₜ = 1.16 min; m/z = 541 (M+H)⁺. ¹H-NMR (400 MHz, DMSO-*d*₆): δ = 12.08 (br. s, 1H), 9.98 (s, 1H), 7.61-7.51 (m, 2H), 7.47-7.36 (m, 5H), 7.24 (d, 2H), 7.13 (t, 1H), 6.89 (d, 1H), 4.69 (s, 2H), 4.48 (s, 2H), 3.38 (d, 1H), 2.81 (s, 2H), 2.63-2.55 (m, 1H), 1.85-1.20 (m, 7H), 1.10 (q, 2H), 1.01-0.91 (m, 1H), 0.78 (q, 2H). [α]_{D}²⁰ = +45°, c = 0.112, Chloroform. |
| | 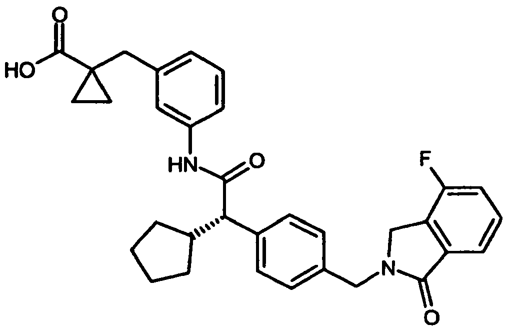 | |
| **215** | (-)-1-(3-{[(2*R*)-2-Cyclopentyl-2-{4-[(4-fluor-1-oxo-1,3-dihydro-2*H*-isoindol-2-yl)methyl]phenyl}acetyl]-amino}benzyl)cyclopropancarbonsäure | LC-MS (Methode 15): Rₜ = 1.16 min; m/z = 541 (M+H)⁺. ¹H-NMR (400 MHz, DMSO-*d*₆): δ = 12.08 (br. s, 1H), 9.98 (s, 1H), 7.63-7.52 (m, 2H), 7.47-7.36 (m, 5H), 7.24 (d, 2H), 7.13 (t, 1H), 6.89 (d, 1H), 4.69 (s, 2H), 4.47 (s, 2H), 3.38 (d, 1H), 2.81 (s, 2H), 2.63-2.55 (m, 1H), 1.83-1.18 (m, 7H), 1.10 (q, 2H), 1.01-0.92 (m, 1H), 0.78 (q, 2H). [α]_{D}²⁰ = -34°, c = 0.210, Chloroform. |
| | 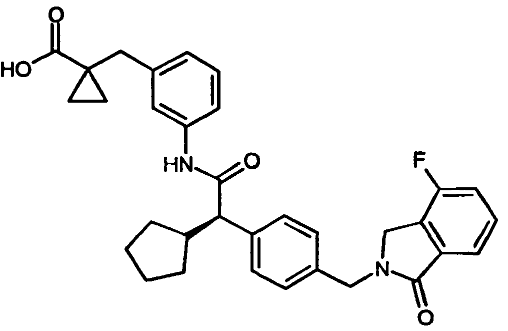 | |
| **216** | (+)-1-(3-{[(2*S*)-2-{4-[(5-Chlor-2-oxopyridin-1(2*H*)-yl)methyl]phenyl}-2-cyclopentylacetyl]-amino}benzyl)cyclopropancarbonsäure | LC-MS (Methode 15): Rₜ = 1.12 min; m/z = 519 (M+H)⁺. ¹H-NMR (400 MHz, DMSO-*d*₆): δ = 9.97 (s, 1H), 8.09 (d, 1H), 7.48 (dd, 1H), 7.44-7.33 (m, 4H), 7.25 (d, 2H), 7.13 (t, 1H), 6.89 (d, 1H), 6.45 (d, 1H), 5.03 (s, 2H), 3.37 (d, 1H), 2.81 (s, 2H), 2.66-2.55 (m, 1H), 1.85-1.18 (m, 7H), 1.13-1.08 (m, 2H), 0.94 (dd, 1H), 0.77 (q, 2H). [α]_{D}²⁰ = +40°, c = 0.550, Chloroform. |
| | 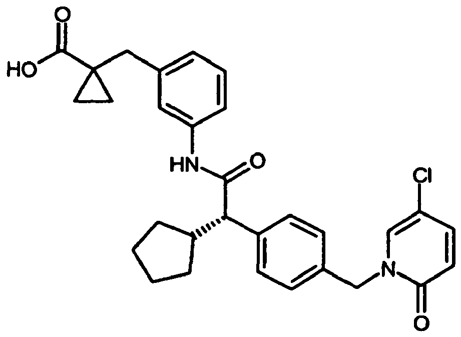 | |
| **217** | (+)-3-(3-{[(2*S*)-2-{4-[(5-Chlor-2-oxopyridin-1(2*H*)-yl)methyl]phenyl}-2-cyclopentylacetyl]-amino}phenyl)propansäure | LC-MS (Methode 15): Rₜ = 1.06 min; m/z = 493 (M+H)⁺. ¹H-NMR (400 MHz, DMSO-*d*₆): δ = 9.98 (s, 1H), 8.10 (d, 1H), 7.48 (dd, 1H), 7.42-7.35 (m, 4H), 7.25 (d, 2H), 7.15 (t, 1H), 6.87 (d, 1H), 6.45 (d, 1H), 5.03 (s, 2H), 4.03 (q, 1H), 3.37 (d, 1H), 2.79-2.70 (m, 2H), 2.67-2.55 (m, 1H), 1.84-1.20 (m, 7H), 1.21-1.14 (m, 1H), 1.17 (t, 1H). [α]_{D}²⁰ = +27.6°, c = 0.485, Chloroform. |
| | 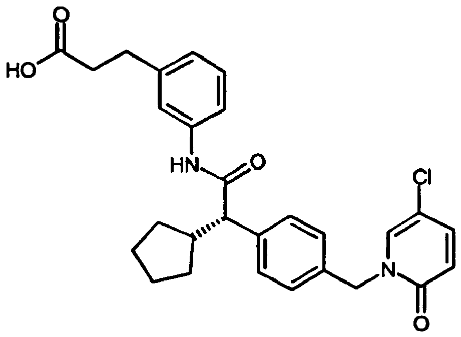 | |
| **218** | 3-(3-{[(2*S*)-2-Cyclopentyl-2-(4-{[2-oxo-5-(trifluor-methyl)pyridin-1(2*H*)-yl]methyl}phenyl)acetyl]-amino}phenyl)propansäure | LC-MS (Methode 15): Rₜ = 1.10 min; m/z = 527 (M+H)⁺. ¹H-NMR (400 MHz, DMSO-*d*₆): δ = 12.09 (s, 1H), 9.98 (s, 1H), 8.54 (s, 1H), 7.67 (dd, 1H), 7.45-7.36 (m, 4H), 7.26 (d, 2H), 7.15 (t, 1H), 6.87 (d, 1H), 6.55 (d, 1H), 5.12 (s, 2H), 3.37 (d, 1H), 2.80-2.71 (m, 2H), 2.64-2.55 (m, 1H), 1.84-1.18 (m, 7H), 0.94 (dd, 1H). |
| | 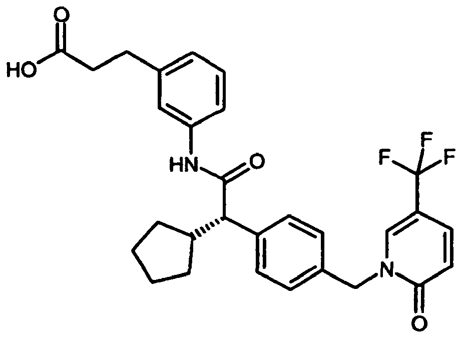 | |
| **219** | (+)-1-(3-{[(2*S*,3*R*)-4,4,4-Trifluor-2-{4-[(4-fluor-1-oxo-1,3-dihydro-2*H*-isoindol-2-yl)methyl]phenyl}-3-methylbutanoyl]amino}benzyl)cyclopropan-carbonsäure | LC-MS (Methode 11): Rₜ = 1.29 min; m/z = 569 (M+H)⁺. ¹H-NMR (400 MHz, DMSO-*d*₆): δ = 10.14 (s, 1H), 7.52-7.61 (m, 2H), 7.38-7.47 (m, 4H), 7.36 (s, 1H), 7.29 (d, 2H), 7.14 (t, 1H), 6.90 (d, 1H), 4.71 (s, 2H), 4.48 (s, 2H), 3.82 (d, 1H), 3.37-3.48 (m, 1H), 2.81 (s, 2H), 1.04-1.13 (m, 2H), 0.78 (d, 5H). [α]_{D}²⁰ = +45.1°, c = 0.575, Chloroform. |
| | 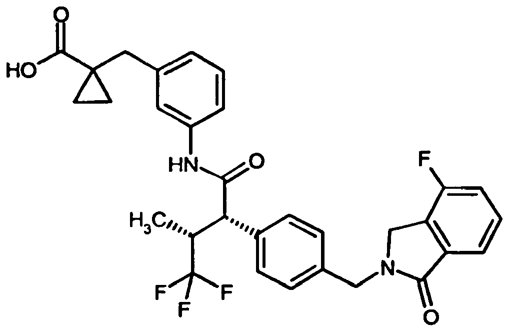 | |
| **220** | (-)-1-(3-{[(2*R*,3*S*)-4,4,4-Trifluor-2-{4-[(4-fluor-1-oxo-1,3-dihydro-2*H*-isoindol-2-yl)methyl]phenyl}-3-methylbutanoyl]amino}benzyl)cyclopropan-carbonsäure | LC-MS (Methode 11): Rₜ = 1.29 min; m/z = 569 (M+H)⁺. ¹H-NMR (400 MHz, DMSO-*d*₆): δ = 12.00 (br. s, 1H), 10.14 (s, 1H), 7.62-7.51 (m, 2H), 7.49-7.38 (m, 4H), 7.36 (s, 1H), 7.29 (d, 2H), 7.14 (t, 2H), 6.90 (d, 1H), 4.71 (s, 2H), 4.51-4.46 (m, 2H), 3.82 (d, 1H), 2.81 (s, 2H), 1.10 (d, 2H), 0.78 (d, 5H). [α]_{D}²⁰ = -32.9°, c = 0.555, Chloroform. |
| | 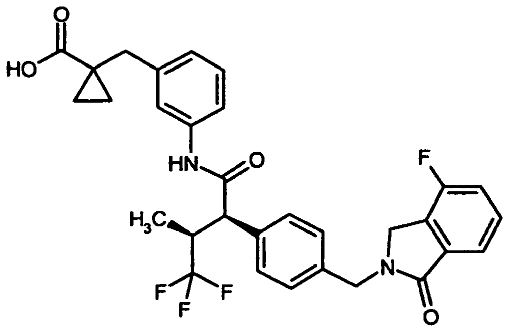 | |
| **221** | 1-(3-{[(2*S*)-2-Cyclopentyl-2-(4-{(2-oxo-5-(trifluor-methyl)pyridin-1(2*H*)-yl]methyl}phenyl)acetyl]-amino}benzyl)cyclopropancarbonsäure | LC-MS (Methode 15): Rₜ = 1.15 min; m/z = 553 (M+H)⁺. ¹H-NMR (400 MHz, DMSO-*d*₆): δ = 12.07 (s, 1H), 9.97 (s, 1H), 8.54 (s, 1H), 7.67 (dd, 1H), 7.45-7.36 (m, 4H), 7.25 (d, 2H), 7.13 (t, 1H), 6.89 (d, 1H), 6.55 (d, 1H), 5.12 (s, 2H), 3.37 (d, 1H), 2.81 (s, 2H), 2.67-2.55 (m, 1H), 1.84-1.19 (m, 7H), 1.10 (q, 2H), 1.02-0.88 (m, 1H), 0.78 (q, 2H). |
| | 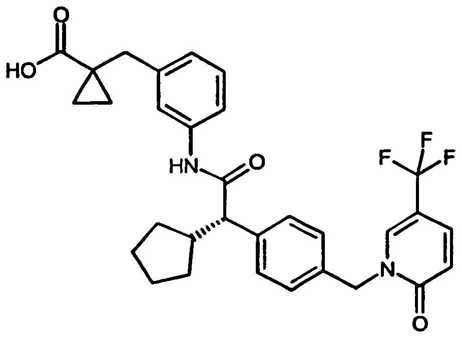 | |
| **222** | (-)-3-(3-{[(2*R*,3*S*)-4,4,4-Trifluor-3-methyl-2-(4-{[2-oxo-5-(trifluormethyl)pyridin-1(2*H*)-yl]methyl}-phenyl)butanoyl]amino}phenyl)propansäure | LC-MS (Methode 11): Rₜ = 1.23 min; m/z = 555 (M+H)⁺. ¹H-NMR (400 MHz, DMSO-*d*₆): δ = 10.15 (s, 1H), 8.54 (br. s, 1H), 7.67 (dd, 1H), 7.42-7.33 (m, 4H), 7.29 (d, 2H), 7.16 (t, 1H), 6.89 (d, 1H), 6.55 (d, 1H), 5.13 (s, 2H), 3.81 (d, 1H), 3.57 (s, 4H), 2.74 (t, 2H), 0.77 (d, 3H). [α]_{D}²⁰ = -45.4 °, c = 0.545, Chloroform. |
| | 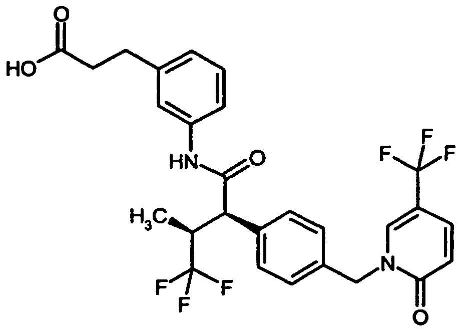 | |
| **223** | (+)-3-(3-{[(2*S*,3*R*)-4,4,4-Trifluor-3-methyl-2-(4-{[2-oxo-5-(trifluormethyl)pyridin-1(2*H*)-yl]methyl}-phenyl)butanoyl]amino}phenyl)propansäure | LC-MS (Methode 11): Rₜ = 1.23 min; m/z = 555 (M+H)⁺. ¹H-NMR (400 MHz, DMSO-*d*₆): δ = 10.15 (s, 1H), 8.54 (br. s, 1H), 7.67 (dd, 1H), 7.43-7.33 (m, 4H), 7.29 (d, 2H), 7.15 (t, 1H), 6.88 (d, 1H), 6.55 (d, 1H), 5.13 (s, 2H), 3.81 (d, 1H), 3.57 (s, 3H), 2.77-2.72 (m, 2H), 1.35 (s, 1H), 0.77 (d, 3H). [α]_{D}²⁰ = +66°, c = 0.205, Chloroform. |
| | 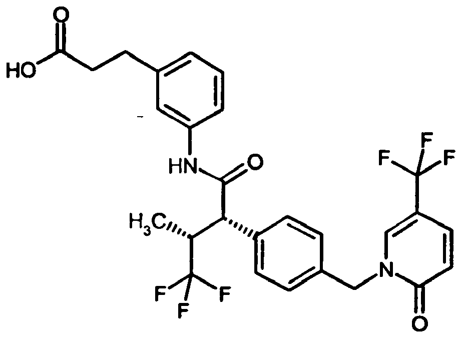 | |
| **224** | (+)-3-(3-{[(2*S*)-2-Cyclopentyl-2-{4-[(5-oxo-2-phenyl-5,6-dihydro-4*H*-1,3,4-oxadiazin-4-yl)methyl]phenyl}-acetyl]amino}-4-fluorphenyl)propansäure | LC-MS (Methode 15): Rₜ = 1.22 min; m/z = 558 (M+H)⁺. ¹H-NMR (400 MHz, DMSO-*d*₆): δ = 9.78 (s, 1H), 7.81-7.74 (m, 2H), 7.64 (dd, 1H), 7.50-7.42 (m, 3H), 7.40 (d, 2H), 7.35-7.27 (m, 3H), 7.09 (dd, 1H), 7.00-6.92 (m, 1H), 4.91 (s, 2H), 4.85 (s, 2H), 3.59 (d, 1H), 2.73 (t, 2H), 2.46 (t, 2H), 0.86-1.87 (m, 9H). [α]_{D}²⁰ = +52.6°, c = 0.515, Chloroform. |
| | 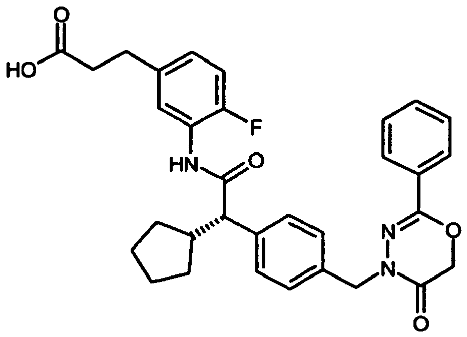 | |
| **225** | (+)-3-(3-{[(2*S*)-2-Cyclopentyl-2-{4-[(5-oxo-2-phenyl-5,6-dihydro-4*H*-1,3,4-oxadiazin-4-yl)methyl]phenyl}-acetyl]amino}-2-fluorphenyl)propansäure | LC-MS (Methode 7): Rₜ = 2.70 min; m/z = 558 (M+H)⁺. ¹H-NMR (400 MHz, DMSO-*d*₆): δ = 12.19 (br. s, 1H), 9.78 (s, 1H), 7.78 (d, 1H), 7.62 (m, 1H), 7.52-7.48 (m, 5H), 7.31 (d, 2H), 7.03-6.98 (m, 2H), 4.91 (s, 2H), 4.85 (s, 2H), 3.61 (d, 1H), 2.81 (t, 2H), 1.83-1.75 (m, 1H), 1.70-1.25 (m, 5H), 1.01-0.92 (m, 1H). [α]_{D}²⁰ = +9.5°, c = 0.455, Chloroform. |
| | 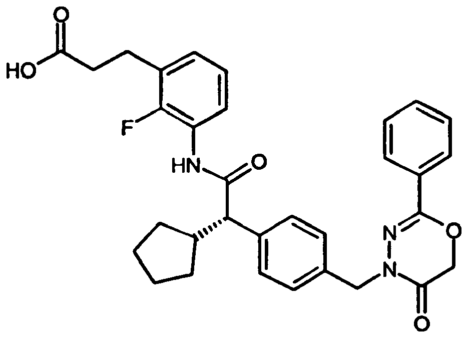 | |
| **226** | (+)-3-(3-{[(2*S*,3*R*)-4,4,4-Trifluor-2-{4-[(4-fluor-1-oxo-1,3-dihydro-2*H*-isoindol-2-yl)methyl]phenyl}-3-methylbutanoyl]amino}phenyl)propansäure | LC-MS (Methode 15): Rₜ = 1.09 min; m/z = 543 (M+H)⁺. ¹H-NMR (400 MHz, DMSO-*d*₆): δ = 12.11 (s, 1H), 10.18 (s, 1H), 7.60-7.53 (m, 1H), 7.48-7.35 (m, 5H), 7.30 (d, 2H), 7.17 (t, 1H), 6.89 (d, 1H), 4.72 (s, 2H), 4.49 (s, 2H), 3.72 (d, 1H), 3.42-3.30 (m, 1H), 2.79-2.70 (m, ca. 2H), 0.78 (d, 3H). [α]_{D}²⁰ = +85°, c = 0.300, Chloroform. |
| | 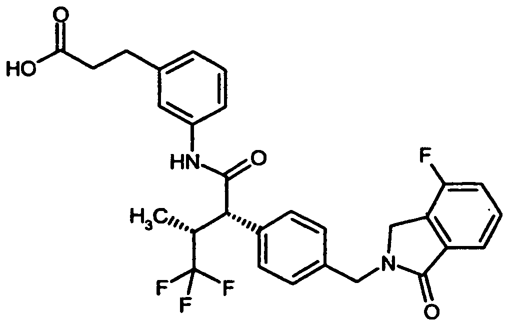 | |
| **227** | (-)-3-(3-{[(2*R*,3*S*)-4,4,4-Trifluor-2-{4-[(4-fluor-1-oxo-1,3-dihydro-2*H*-isoindol-2-yl)methyl]phenyl}-3-methylbutanoyl]amino}phenyl)propansäure | LC-MS (Methode 15): Rₜ = 1.08 min; m/z = 543 (M+H)⁺. ¹H-NMR (400 MHz, DMSO-*d*₆): δ = 12.12 (br. s, 1H), 10.18 (s, 1H), 7.60-7.53 (m, 1H), 7.48-7.35 (m, 5H), 7.30 (d, 2H), 7.17 (t, 1H), 6.89 (d, 1H), 4.72 (s, 2H), 4.49 (s, 2H), 3.72 (d, 1H), 3.40-3.30 (m, 1H), 2.72 (t, 2H), 2.49 (m, ca. 2H), 0.78 (d, 3H). [α]_{D}²⁰ = -73.6°, c = 0.375, Chloroform. |
| | 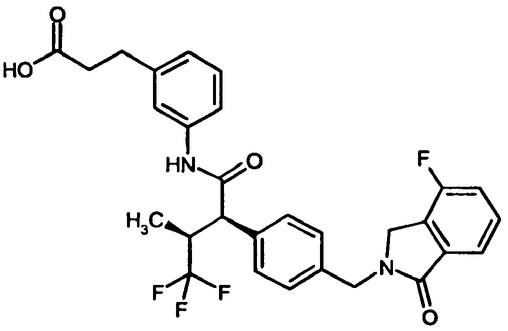 | |

### Allgemeine Vorschrift 9B: Spaltung von tert.-Butylestern zu den entsprechenden Carbonsäuren mit Trifluoressigsäure

Zu einer Lösung des betreffenden *tert*.-Butylesters in Dichlormethan (ca. 0.1 mol/l) wurden 20 eq. Trifluoressigsäure gegeben. Die Reaktionslösung wurde über Nacht bei Raumtemperatur gerührt und anschließend in ein Gemisch aus Wasser und Essigsäureethylester (1:1 v/v) gegeben. Die organische Phase wurde dreimal mit Wasser und einmal mit gesättigter Natriumchlorid-Lösung gewaschen, über Magnesiumsulfat getrocknet und eingeengt. Gegebenenfalls wurde das erhaltene Produkt durch präparative RP-HPLC gereinigt.

Entsprechend der Allgemeinen Vorschrift 9B wurden die folgenden Verbindungen hergestellt:

| **Beispiel** | **Name / Struktur** | **Analytische Daten** |
|---|---|---|
| **228** | (+/-)-3-{3-[(Cyclopentyl{4-[(1,3-dioxo-1,3-dihydro-2*H*-isoindol-2-yl)methyl]phenyl}acetyl)amino]-2-methylphenyl}propansäure | LC-MS (Methode 7): Rₜ = 2.50 min; m/z = 525 [M+H]⁺. ¹H-NMR (400 MHz, DMSO-d₆): δ = 12.18 (s, 1H), 9.45 (s, 1H), 7.91-7.85 (m, 4H), 7.38 (d, 2H), 7.26 (d, 2H), 7.04-6.96 (m, 3H), 4.75 (s, 2H), 3.44 (d, 1H), 2.78 (t, 2H), 2.42 (t, 2H), 1.98 (s, 3H), 1.87-1.29 (m, 8H), 1.0-0.9 (m, 1H). |
| | 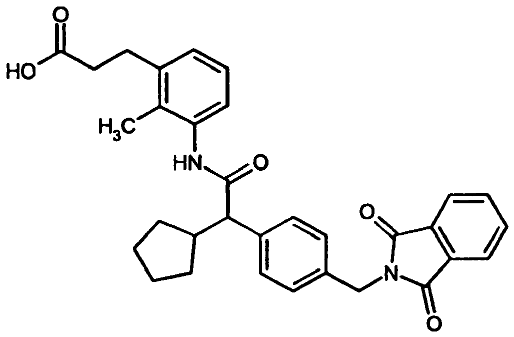 | |
| **229** | (+/-)-3-{3-[(Cyclopentyl{4-[(1-oxo-1,3-dihydro-2*H*-isoindol-2-yl)methyl]phenyl}acetyl)amino]-2-methylphenyl}propansäure | LC-MS (Methode 11): Rₜ = 2.50 min; m/z = 511 [M+H]⁺. ¹H-NMR (400 MHz, DMSO-*d*₆): δ = 9.46 (s, 1H), 7.73 (d, 1H), 7.54-7.63 (m, 2H), 7.46-7.53 (m, 1H), 7.41 (d, 2H), 7.23 (d, 2H), 6.94-7.07 (m, 3H), 4.71 (s, 2H), 4.37 (s, 2H), 3.45 (d, 1H), 2.79 (t, 2H), 2.43 (t, 2H), 1.98 (d, 4H), 1.26-1.90 (m, 7H), 0.97 (dq, 1H). |
| | 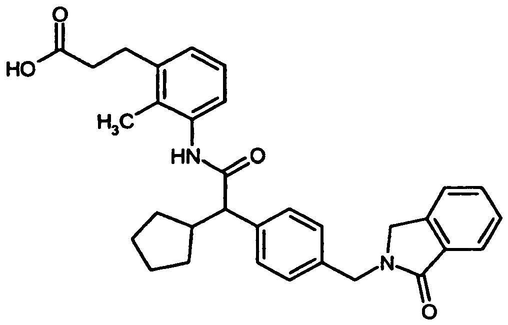 | |
| **230** | (+/-)-3-{3-[(Cyclopentyl{4-[(5-oxo-2-phenyl-5,6-dihydro-4*H*-1,3,4-oxadiazin-4-yl)methyl]phenyl}-acetyl)amino]-5-fluorphenyl}propansäure | LC-MS (Methode 11): Rₜ = 1.43 min; m/z = 558 [M+H]⁺. ¹H-NMR (400 MHz, DMSO-*d*₆): δ = 12.13 (br. s, 1H), 10.20 (s, 1H), 7.66-7.83 (m, 2H), 7.41-7.53 (m, 3H), 7.38 (d, 2H), 7.28-7.35 (m, 2H), 7.11 (s, 1H), 6.73 (d, 1H), 4.76-4.95 (m, 4H), 3.27-3.43 (m, 3H), 2.70-2.79 (m, 2H), 1.70-1.85 (m, 1H), 1.17-1.69 (m, 6H), 1.09 (t, 1H), 0.90-1.02 (m, 1H). |
| | 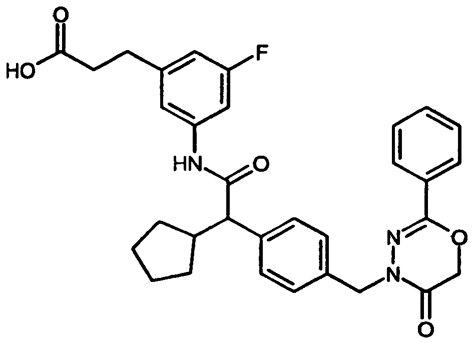 | |
| **231** | (+/-)-1-{3-[(Cyclopentyl{4-[(5-oxo-2-phenyl-5,6-dihydro-4*H*-1,3,4-oxadiazin-4-yl)methyl]phenyl}-acetyl)amino]-4-fluorbenzyl}cyclopropancarbonsäure | LC-MS (Methode 11): Rₜ = 1.45 min; m/z = 584 [M+H]⁺. ¹H-NMR (400 MHz, DMSO-*d*₆): δ = 12.10 (s, 1H), 9.76 (s, 1H), 7.73-7.82 (m, 2H), 7.65 (dd, 1H), 7.42-7.53 (m, 3H), 7.37-7.42 (m, 2H), 7.28-7.34 (m, 2H), 7.08 (dd, 1H), 6.96-7.03 (m, 1H), 4.81-4.95 (m, 4H), 3.58 (d, 1H), 2.79 (s, 2H), 1.20-1.86 (m, 8H), 1.07 (q, 2H), 0.91-1.02 (m, 1H), 0.73-0.82 (m, 2H). |
| | 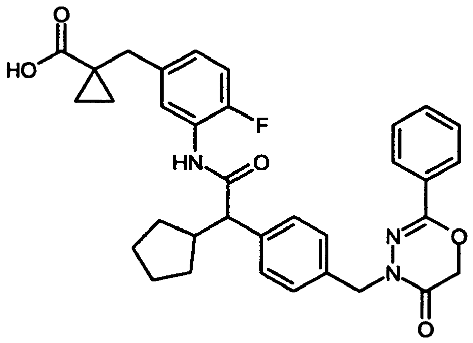 | |
| **232** | (+/-)-3-(5-{2-Cyclopentyl-2-{4-[(5-oxo-2-phenyl-5,6-dihydro-4*H*-1,3,4-oxadiazin-4-yl)methyl]phenyl}-acetyl]amino}-2-fluorphenyl)propansäure | LC-MS (Methode 11): Rₜ = 1.41 min; m/z = 558 [M+H]⁺. ¹H-NMR (400 MHz, DMSO-*d*₆): δ = 12.05-12.34 (m, 1H), 10.04 (s, 1H), 7.69-7.83 (m, 2H), 7.40-7.56 (m, 5H), 7.35-7.40 (m, 2H), 7.27-7.34 (m, 2H), 7.02 (t, 1H), 4.69-4.96 (m, 4H), 3.17 (d, 1H), 2.72-2.81 (m, 2H), 2.42-2.48 (m, 2H), 1.72-1.87 (m, 1H), 1.16-1.72 (m, 7H), 0.90-1.04 (m, 1H). |
| | 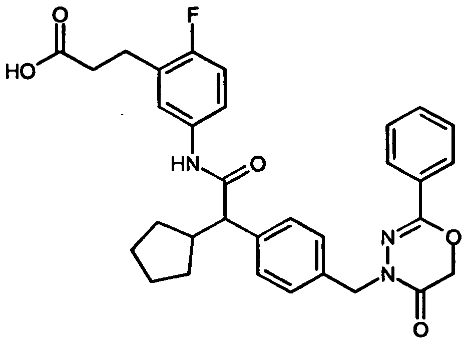 | |
| **233** | 1-{3-[(Cyclopentyl{4-[(5-oxo-2-phenyl-5,6-dihydro-4*H*-1,3,4-oxadiazin-4-yl)methyl]phenyl}acetyl)-amino]-2-fluorbenzyl}cyclopropancarbonsäure | LC-MS (Methode 11): Rₜ = 1.45 min; m/z = 584 [M+H]⁺ . ¹H-NMR (400 MHz, DMSO-*d*₆): δ = 12.19 (br. s, 1H), 9.77 (s, 1H), 7.73-7.82 (m, 2H), 7.55-7.65 (m, 1H), 7.36-7.53 (m, 5H), 7.29-7.35 (m, 2H), 7.05-7.13 (m, 1H), 6.96-7.05 (m, 1H), 4.77-4.97 (m, 4H), 3.60 (d, 1H), 2.90 (s, 2H), 1.72-1.86 (m, 1H), 1.21-1.72 (m, 6H), 1.09-1.16 (m, 2H), 0.90-1.02 (m, 1H), 0.70-0.83 (m, 2H). |
| | 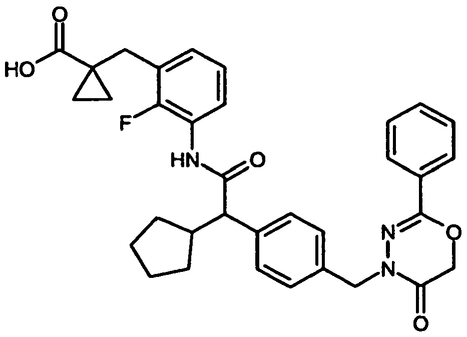 | |
| **234** | (+/-)-1-{5-[(Cyclopentyl{4-[(5-oxo-2-phenyl-5,6-dihydro-4*H*-1,3,4-oxadiazin-4-yl)methyl]phenyl}-acetyl)amino]-2-fluorbenzyl}cyclopropancarbonsäure | LC-MS (Methode 15): Rₜ = 1.29 min; m/z = 584 [M+H]⁺. ¹H-NMR (400 MHz, DMSO-*d*₆): δ = 12.16 (br. s, 1H), 10.05 (s, 1H), 7.71-7.82 (m, 2H), 7.41-7.56 (m, 5H), 7.36-7.41 (m, 2H), 7.28-7.36 (m, 2H), 6.95-7.06 (m, 1H), 4.79-4.96 (m, 4H), 2.85 (s, 2H), 1.71-1.83 (m, 1H), 1.18-1.68 (m, 6H), 1.09-1.15 (m, 2H), 0.91-1.02 (m, 1H), 0.71-0.81 (m, 2H). |
| | 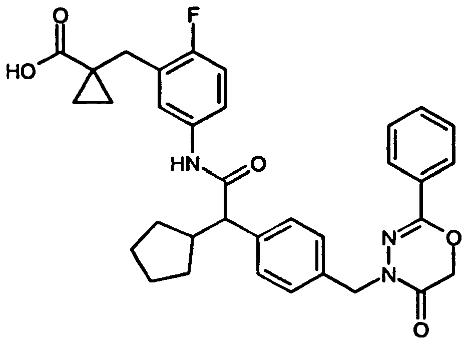 | |
| **235** | (+/-)-1-{3-[(Cyclopentyl{4-[(5-oxo-2-phenyl-5,6-dihydro-4*H*-1,3,4-oxadiazin-4-yl)methyl]phenyl}-acetyl)amino]-5-fluorbenzyl}cyclopropancarbonsäure | LC-MS (Methode 15): Rₜ = 1.30 min; m/z = 584 [M+H]⁺. ¹H-NMR (400 MHz, DMSO-*d*₆): δ = 12.13 (br. s, 1H), 10.20 (s, 1H), 7.67-7.83 (m, 2H), 7.41-7.54 (m, 5H), 7.36-7.40 (m, 2H), 7.28-7.35 (m, 2H), 7.14 (s, 1H), 6.71 (d, 1H), 4.78-4.94 (m, 4H), 2.80 (s, 2H), 1.71-1.84 (m, 1H), 1.17-1.70 (m, 6H), 1.09-1.16 (m, 2H), 0.96 (dd, 1H), 0.77-0.87 (m, 2H). |
| | 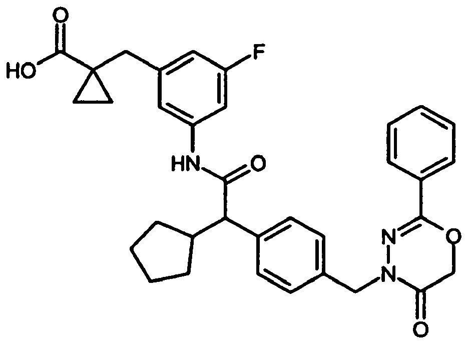 | |

### Beispiel 236 und Beispiel 237

### 1-{3-[(Cyclopentyl{4-[(5-oxo-2-phenyl-5,6-dyhydro-4H-1,3,4-oxadiazin-4-yl)methyl]phenyl}-acetyl)amino]-5-fluorbenzyl}cyclopropancarbonsäure (Enantiomer 1 und 2)

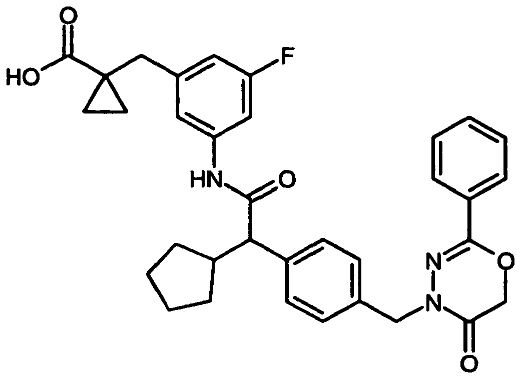

57 mg der racemischen 1-{3-[(Cyclopentyl{4-[(5-oxo-2-phenyl-5,6-dihydro-4*H*-1,3,4-oxadiazin-4-yl)methyl]phenyl}acetyl)amino]-5-fluorbenzyl}cyclopropancarbonsäure wurden durch präparative HPLC an chiraler Phase in die Enantiomere getrennt [Säule: Daicel Chiralpak AD-H, 5 µm, 250 mm x 20 mm; Fluss: 15 ml/min; UV-Detektion: 220 nm; Injektionsvolumen: 0.2 ml; Temperatur: 40°C; Eluent: 30% Isopropanol / 70% Isohexan]:

### Beispiel 236 (Enantiomer 1):

Ausbeute: 30 mg
LC-MS (Methode 15): Rₜ = 1.30 min; m/z = 584 [M+H]⁺.
¹H-NMR (400 MHz, DMSO-*d*₆): δ = 12.13 (br. s, 1 H), 10.20 (s, 1 H), 7.67-7.83 (m, 2 H), 7.41-7.54 (m, 5 H), 7.36-7.40 (m, 2 H), 7.28-7.35 (m, 2 H), 7.14 (s, 1 H), 6.71 (d, 1 H), 4.78-4.94 (m, 4 H), 2.80 (s, 2 H), 1.71-1.84 (m, 1 H), 1.17-1.70 (m, 6 H), 1.09-1.16 (m, 2 H), 0.96 (dd, 1 H), 0.77-0.87 (m, 2 H).
[α]_{D}²⁰ = -26°, c = 0.28, Chloroform.

### Beispiel 237 (Enantiomer 2):

Ausbeute: 32 mg (leicht verunreinigt)
LC-MS (Methode 15): Rₜ = 1.30 min; m/z = 584 [M+H]⁺.
¹H-NMR (400 MHz, DMSO-*d*₆): δ = 12.13 (br. s, 1 H), 10.20 (s, 1 H), 7.67-7.83 (m, 2 H), 7.41-7.54 (m, 5 H), 7.36-7.40 (m, 2 H), 7.28-7.35 (m, 2 H), 7.14 (s, 1 H), 6.71 (d, 1 H), 4.78-4.94 (m, 4 H), 2.80 (s, 2 H), 1.71-1.84 (m, 1 H), 1.17-1.70 (m, 6 H), 1.09-1.16 (m, 2 H), 0.96 (dd, 1 H), 0.77-0.87 (m, 2 H).
[α]_{D}²⁰ = +23°, c = 0.26, Chloroform.

### Beispiele 238 - 241

### 1-(3-{[(3,3-Difluorcyclopentyl){4-[(5-oxo-2-phenyl-5,6-dihydro-4H-1,3,4-oxadiazin-4-yl)methyl]-phenyl}acetyl]amino}benzyl)cyclopropancarbonsäure (Isomer 1 - 4)

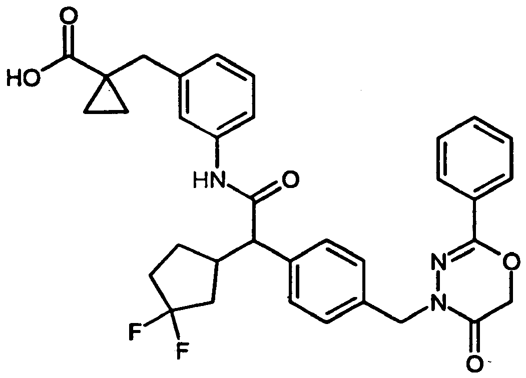

90 mg (0.15 mmol) des Isomerengemisches von 1-(3-{[(3,3-Difluorcyclopentyl){4-[(5-oxo-2-phenyl-5,6-dihydro-4*H*-1,3,4-oxadiazin-4-yl)methyl]phenyl}acetyl]amino}benzyl)cyclopropancarbonsäure wurden mittels präparativer HPLC an chiraler Phase weiter aufgetrennt [Säule: Daicel Chiralcel OD-H, 5 µm, 250 mm x 20 mm; Elutionsmittel: Isohexan/(Ethanol + 0.2% Trifluoressigsäure + 1% Wasser) 75:25 (v/v); Fluss: 15 ml/min; UV-Detektion: 220 nm; Temperatur: 40°C]. Es wurden drei Fraktionen isoliert, wobei Fraktion 2 aus zwei Isomeren bestand. Diese Mischfraktion wurde durch nochmalige präparative HPLC an chiraler Phase nachgetrennt [Säule: Daicel Chiralpak AD-H, 5 µm, 250 mm x 20 mm; Elutionsmittel: Isohexan/(Ethanol + 0.2% Trifluoressigsäure + 1% Wasser) 70:30 (v/v); Fluss: 15 ml/min; UV-Detektion: 220 nm; Temperatur: 30°C].

### Beispiel 238 (Isomer 1):

Ausbeute: 15 mg
Rₜ 9.61 min; chem. Reinheit >99%; Isomeren-Reinheit: verunreinigt mit 1.2% Isomer 2
[Säule: Daicel Chiralpak AD-H, 5 µm, 250 mm x 4.6 mm; Elutionsmittel: Isohexan/(Ethanol + 0.2% Trifluoressigsäure + 1% Wasser) 70:30 (v/v); Fluss: 1 ml/min; UV-Detektion: 220 nm; Temperatur: 40°C].
LC-MS (Methode 15): Rₜ = 1.22 min; m/z = 602 (M+H)⁺.

### Beispiel 239 (Isomer 2):

Ausbeute: 12 mg
Rₜ 8.68 min; Reinheit >99%; >99% ee
[Säule: Daicel Chiralpak AD-H, 5 µm, 250 mm x 4.6 mm; Elutionsmittel: Isohexan/(Ethanol + 0.2% Trifluoressigsäure + 1% Wasser) 70:30 (v/v); Fluss: 1 ml/min; UV-Detektion: 220 nm; Temperatur: 40°C].
LC-MS (Methode 15): Rₜ = 1.22 min; m/z = 602 (M+H)⁺.

### Beispiel 240 (Isomer 3):

Ausbeute: 15 mg
Rₜ 10.28 min; chem. Reinheit >99%; Isomeren-Reinheit: verunreinigt mit 10.9% Isomer 1 und 3.8% Isomer 2
[Säule: Daicel Chiralpak AD-H, 5 µm, 250 mm x 4.6 mm; Elutionsmittel: Isohexan/(Ethanol + 0.2% Trifluoressigsäure + 1% Wasser) 70:30 (v/v); Fluss: 1 ml/min; UV-Detektion: 220 nm; Temperatur: 40°C].
LC-MS (Methode 15): Rₜ =1.22 min; m/z = 602 (M+H)⁺.

### Beispiel 241 (Isomer 4):

Ausbeute: 19 mg
Rₜ 7.96 min; Reinheit >99%; >99% ee
[Säule: Daicel Chiralpak AD-H, 5 µm, 250 mm x 4.6 mm; Elutionsmittel: Isohexan/(Ethanol + 0.2% Trifluoressigsäure + 1% Wasser) 70:30 (v/v); Fluss: 1 ml/min; UV-Detektion: 220 nm; Temperatur: 40°C].
LC-MS (Methode 15): Rₜ = 1.22 min; m/z = 602 (M+H)⁺.

### B. Bewertung der pharmakologischen Wirksamkeit

Die pharmakologische Wirkung der erfindungsgemäßen Verbindungen kann in folgenden Assays gezeigt werden:

### B-1. Gefäßrelaxierende Wirkung in vitro:

Kaninchen werden durch intravenöse Injektion von Thiopental-Natrium narkotisiert bzw. getötet (ca. 50 mg/kg) und entblutet. Die Arteria Saphena wird entnommen und in 3 mm breite Ringe geteilt. Die Ringe werden einzeln auf je einem triangelförmigen, am Ende offenen Häkchenpaar aus 0.3 mm starkem Spezialdraht (Remanium^{®}) montiert. Jeder Ring wird unter Vorspannung in 5 ml-Organbäder mit 37°C warmer, carbogenbegaster Krebs-Henseleit-Lösung folgender Zusammensetzung gebracht: NaCl 119 mM; KC1 4.8 mM; CaCl₂ x 2 H₂O 1 mM; MgSO₄ x 7 H₂O 1.4 mM; KH₂PO₄ 1.2 mM; NaHCO₃ 25 mM; Glucose 10 mM; Rinderserumalbumin 0.001%. Die Kontraktionskraft wird mit Statham UC2-Zellen erfasst, verstärkt und über A/D-Wandler (DAS-1802 HC, Keithley Instruments, München) digitalisiert sowie parallel auf Linienschreibern registriert. Kontraktionen werden durch Zugabe von Phenylephrin induziert.

Nach mehreren (allgemein 4) Kontrollzyklen wird die zu untersuchende Substanz in jedem weiteren Durchgang in steigender Dosierung zugesetzt und die Höhe der unter dem Einfluss der Testsubstanz erzielten Kontraktion mit der Höhe der im letzten Vordurchgang erreichten Kontraktion verglichen. Daraus wird die Konzentration errechnet, die erforderlich ist, um die in der Vorkontrolle erreichte Kontraktion auf 50% zu reduzieren (IC₅₀-Wert). Das Standard-Applikationsvolumen beträgt 5 µl. Der DMSO-Anteil in der Badlösung entspricht 0.1%.

Repräsentative Ergebnisse zu den erfindungsgemäßen Verbindungen sind in Tabelle 1 aufgeführt:

**Tabelle 1: Gefäßrelaxierende Wirkung in vitro**

| **Beispiel Nr.** | **IC₅₀ [nM]** |
|---|---|
| 46 | 110 |
| 77 | 625 |
| 86 | 180 |
| 103 | 120 |
| 115 | 163 |
| 126 | 145 |
| 138 | 52 |
| 143 | 345 |
| 156 | 45 |
| 164 | 26 |
| 166 | 4.9 |
| 185 | 64 |
| 186 | 37 |
| 192 | 356 |
| 204 | 86 |
| 214 | 11 |
| 224 | 490 |
| 237 | 158 |

### B-2. Stimulation der rekombinanten löslichen Guanylatcyclase sGC) in vitro:

Die Untersuchungen zur Stimulation der rekombinanten löslichen Guanylatcyclase (sGC) durch die erfindungsgemäßen Verbindungen mit und ohne Natriumnitroprussid sowie mit und ohne den Häm-abhängigen sGC-Inhibitor 1*H*-1,2,4-Oxadiazolo[4,3-a]chinoxalin-1-on (ODQ) werden nach der in folgender Literaturstelle im Detail beschriebenen Methode durchgeführt: M. Hoenicka, E.M. Becker, H. Apeler, T. Sirichoke, H. Schroeder, R. Gerzer und J.-P. Stasch, "Purified soluble guanylyl cyclase expressed in a baculovirus/Sf9 system: Stimulation by YC-1, nitric oxide, and carbon oxide", J. Mol. Med. 77 (1999), 14-23. Die Häm-freie Guanylatcyclase wird durch Zugabe von Tween 20 zum Probenpuffer (0.5% in der Endkonzentration) erhalten.

Die Aktivierung der sGC durch eine Prüfsubstanz wird als n-fache Stimulation der Basalaktivität angegeben. Das Ergebnis für Beispiel 103 ist in Tabelle 2 gezeigt:

**Tabelle 2: Stimulation (n-fach) der rekombinanten löslichen Guanylatcyclase (sGC) in vitro durch Beispiel 103**

| **Konzentration Beispiel 103 [µM]** | **Häm-haltige sGC** | | | **Häm-freie sGC Basal** |
|---|---|---|---|---|
| | **Basal** | **+ 0.1 µM DEA/NO** | **+10 µM ODQ** | |
| 0 | 1.0 | 6.5 | 5.7 | 1.0 |
| 0.1 | 1.2 | 6.9 | 5.8 | 1.1 |
| 1 | 1.4 | 7.8 | 8.5 | 2.4 |
| 10 | 6.2 | 13.5 | 36.8 | 13.9 |
| 100 | 22.3 | 35.6 | 91.8 | 56.1 |

| | | | | |
|---|---|---|---|---|
| [DEA/NO = *2-*(*N,N-*Diethylamino)diazenolat-2-oxid; ODQ = 1*H*-1,2,4-Oxadiazolo[4,3-α]chinoxalin-1-on]. | | | | |

Aus Tabelle 2 ist ersichtlich, dass eine Stimulation sowohl des Häm-haltigen als auch des Hämfreien Enzyms erreicht wird. Weiterhin zeigt die Kombination aus Beispiel 103 und 2-(*N,N-*Diethylamino)diazenolat-2-oxid (DEA/NO), einem NO-Donor, keinen synergistischen Effekt, d.h. die Wirkung von DEA/NO wird nicht potenziert, wie dies bei einem über einen Häm-abhängigen Mechanismus wirkenden sGC-Aktivator zu erwarten wäre. Darüber hinaus wird die Wirkung des erfindungsgemäßen sGC-Aktivators durch den Häm-abhängigen Inhibitor der löslichen Guanylatcyclase ODQ nicht blockiert, sondern sogar gesteigert. Die Ergebnisse aus Tabelle 2 belegen somit den Wirkmechanismus der erfindungsgemäßen Verbindungen als Aktivatoren der löslichen Guanylatcyclase.

### B-3. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative Ergebnisse zu den erfmdungsgemäßen Verbindungen sind in Tabelle 3 aufgeführt:

**Tabelle 3: sGC-aktivierende Wirkung in der CHO-Reporterzelle in vitro**

| **Beispiel Nr.** | **MEC [nM]** |
|---|---|
| 1 | 10 |
| 3 | 300 |
| 11 | 300 |
| 21 | 30 |
| 22 | 300 |
| 26 | 300 |
| 29 | 30 |
| 30 | 30 |
| 38 | 30 |
| 46 | 3 |
| 62 | 3 |
| 77 | 1 |
| 86 | 1 |
| 103 | 10 |
| 105 | 10 |
| 106 | 10 |
| 115 | 3 |
| 118 | 30 |
| 126 | 30 |
| 138 | 10 |
| 143 | 3 |
| 156 | 3 |
| 160 | 30 |
| 164 | 0.3 |
| 166 | 0.3 |
| 170 | 3 |
| 171 | 30 |
| 176 | 30 |
| 181 | 0.7 |
| 184 | 3 |
| 185 | 0.3 |
| 186 | 0.2 |
| 187 | 1 |
| 192 | 3 |
| 204 | 1 |
| 207 | 3 |
| 213 | 3 |
| 214 | 0.1 |
| 219 | 0.2 |
| 220 | 0.3 |
| 224 | 1 |
| 230 | 30 |
| 231 | 0.2 |
| 232 | 20 |
| 233 | 0.3 |
| 234 | 1 |
| 237 | 1 |
| 241 | 3 |

| | |
|---|---|
| (MEC = minimale effektive Konzentration). | |

### B-4. Stimulation der sGC-Enzymaktivität

Lösliche Guanylatcyclase (sGC) setzt unter Stimulation GTP zu cGMP und Pyrophosphat (PPi) um. PPi wird mit Hilfe des nachfolgend beschriebenen Tests nachgewiesen. Das im Test entstehende Signal nimmt mit fortschreitender Umsetzung zu und dient als Maß für die sGC-Enzymaktivität unter der gegebenen Stimulation.

Zur Durchführung des Tests werden 29 µl Enzymlösung [0-10 nM lösliche Guanylatcyclase (hergestellt nach Hönicka et al., J. Mol. Med. 77, 14-23 (1999)) in 50 mM TEA, 2 mM MgCl₂, 0.1% BSA (Fraktion V), 0.005% Brij^{®}, pH 7.5] in eine Mikroplatte vorgelegt und 1 µl der zu testenden Substanz (als seriell verdünnte Lösung in DMSO) hinzugegeben. Der Ansatz wird 10 min bei Raumtemperatur inkubiert. Anschließend werden 20 µl Detektionsmix [1.2 nM Firefly-Luciferase (*Photinus* pyralis-Luciferase, Fa. Promega), 29 µM Dehydro-Luziferin (hergestellt nach Bitler & McElroy, Arch. Biochem. Biophys. 72, 358 (1957)), 122 µM Luziferin (Fa. Promega), 153 µM ATP (Fa. Sigma) und 0.4 mM DTT (Fa. Sigma) in 50 mM TEA, 2 mM MgCl₂, 0.1% BSA (Fraktion V), 0.005% Brij^{®}, pH 7.5] zugegeben. Die Enzymreaktion wird durch Zugabe von 20 µl Substratlösung [1.25 mM Guanosin-5'-triphosphat (Fa. Sigma) in 50 mM TEA, 2 mM MgCl₂, 0.1% BSA (Fraktion V), 0.005% Brij^{®}, pH 7.5] gestartet und kontinuierlich luminometrisch vermessen. Das Maß der Stimulation durch die zu testende Substanz kann relativ zum Signal der nicht stimulierten Reaktion bestimmt werden.

Durch Zugabe von 25 µM 1*H*-1,2,4-Oxadiazolo[4,3-α]chinoxalin-1-on (ODQ) zur Enzymlösung und anschließende 30-minütige Inkubation wird die Aktivierung der Häm-freien Guanylatcyclase untersucht und mit der Stimulation des nativen Enzyms verglichen.

Repräsentative Ergebnisse zu den erfindungsgemäßen Verbindungen sind in Tabelle 4 aufgeführt:

**Tabelle 4: Aktivierende Wirkung am sGC-Enzym in vitro**

| **Beispiel Nr.** | **MEC [nM]** | **EC₅₀ [nM]** |
|---|---|---|
| 46 | 4.1 | 130 |
| 77 | 0.4 | 24 |
| 86 | 1.4 | 73 |
| 103 | 20 | 381 |
| 115 | 1.6 | 40 |
| 126 | 10 | 320 |
| 138 | 5.1 | 330 |
| 156 | 0.2 | 5.4 |
| 160 | 3.4 | 35 |
| 164 | 0.5 | 6.1 |
| 166 | 0.4 | 2.4 |
| 170 | 1 | 11 |
| 171 | 3.7 | 59 |
| 176 | 10 | 320 |
| 181 | 1.4 | 69 |
| 184 | 7 | 240 |
| 186 | 0.1 | 1.5 |
| 192 | 2.5 | 30 |
| 204 | 0.5 | 18 |
| 207 | 0.5 | 11 |
| 213 | 1.2 | 24 |
| 214 | 0.4 | 19 |
| 219 | 0.2 | 3.6 |
| 220 | 2 | 75 |
| 224 | 1 | 20 |
| 230 | 8.1 | 420 |
| 231 | 0.4 | 5.2 |
| 232 | 93 | 1100 |
| 233 | 3.3 | 78 |
| 234 | 4.1 | 220 |
| 237 | 0.9 | 21 |
| 241 | 1.2 | 17 |

| | | |
|---|---|---|
| (MEC = minimale effektive Konzentration; EC₅₀ = Konzentration bei 50% der maximalen Wirksamkeit). | | |

### B-5. Radiotelemetrische Messung von Blutdruck und Herzfrequenz an wachen SH-Ratten

Für die im Folgenden beschriebenen Messungen an wachen SH-Ratten wird ein im Handel erhältliches Telemetriesystem der Firma Data Sciences International DSI, USA, eingesetzt.

Das System besteht aus 3 Hauptkomponenten: (*1*) Implantierbare Sender, (*2*) Empfänger, die über einen Multiplexer mit einem (*3*) Datenakquisitionscomputer verbunden sind. Die Telemetrieanlage ermöglicht eine kontinuierliche Erfassung von Blutdruck und Herzfrequenz an wachen Tieren in ihrem gewohnten Lebensraum.

Die Untersuchungen werden an ausgewachsenen weiblichen, spontan-hypertensiven Ratten (SH-Ratten) mit einem Körpergewicht von >200 g durchgeführt. Die Versuchstiere werden nach Senderimplantation einzeln in Makrolon-Käfigen Typ 3 gehalten. Sie haben freien Zugang zu Standardfutter und Wasser. Der Tag/Nacht-Rhythmus im Versuchslabor wird per Raumbeleuchtung um 6:00 Uhr morgens und um 19:00 Uhr abends gewechselt.

Die eingesetzten Telemetriesender (TAM PA-C40, DSI) werden den Versuchstieren mindestens 14 Tage vor dem ersten Versuchseinsatz unter aseptischen Bedingungen chirurgisch implantiert.

Die so instrumentierten Tiere sind nach Abheilen der Wunde und Einwachsen des Implantats wiederholt einsetzbar.

Zur Implantation werden die nüchternen Tiere mit Pentobarbital (Nembutal, Sanofi, 50 mg/kg i.p.) narkotisiert und an der Bauchseite weiträumig rasiert und desinfiziert. Nach Eröffnung des Bauchraumes entlang der Linea alba wird der flüssigkeitsgefüllte Messkatheter des Systems oberhalb der Bifurcation nach cranial in die Aorta descendens eingesetzt und mit Gewebekleber (VetBonD™, 3M) befestigt. Das Sendergehäuse wird intraperitoneal an der Bauchwandmuskulatur fixiert und die Wunde schichtweise verschlossen. Postoperativ wird zur Infektionsprophylaxe ein Antibiotikum verabreicht (Tardomyocel COMP, Bayer AG, 1 ml/kg s.c.).

### Versuchsablauf:

Die zu untersuchenden Substanzen werden jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert. Eine Lösungsmittel-behandelte Gruppe von Tieren wird als Kontrolle eingesetzt.

Die Telemetrie-Messeinrichtung ist für 24 Tiere konfiguriert. Jeder Versuch wird unter einer Versuchsnummer registriert.

Den in der Anlage lebenden instrumentierten Ratten ist jeweils eine eigene Empfangsantenne zugeordnet (1010 Receiver, DSI). Die implantierten Sender sind über einen eingebauten Magnetschalter von außen aktivierbar und werden bei Versuchsvorlauf auf Sendung geschaltet. Die ausgestrahlten Signale können durch ein Datenakquisitionssystem (Dataquest™ A.R.T. for Windows, DSI) online erfasst und entsprechend aufgearbeitet werden. Die Ablage der Daten erfolgt jeweils in einem hierfür eröffneten Ordner, der die Versuchsnummer trägt.

Im Standardablauf werden über je 10 Sekunden Dauer gemessen: (*1*) systolischer Blutdruck (SBP), (*2*) diastolischer Blutdruck (DBP), (*3*) arterieller Mitteldruck (MAP) und (*4*) Herzfrequenz (HR).

Die Messwerterfassung wird rechnergesteuert in 5 Minuten-Abständen wiederholt. Die als Absolutwert erhobenen Quelldaten werden im Diagramm mit dem aktuell gemessenen Barometerdruck korrigiert und in Einzeldaten abgelegt. Weitere technische Details sind in der Dokumentation der Herstellerfirma (DSI) aufgeführt.

Die Verabreichung der Prüfsubstanzen erfolgt am Versuchstag um 9:00 Uhr. Im Anschluss an die Applikation werden die oben beschriebenen Parameter über 24 Stunden gemessen. Nach Versuchsende werden die erhobenen Einzeldaten mit der Analysis-Software (Dataquest™ A.R.T. Analysis) sortiert. Als Leerwert wird der Zeitpunkt 2 Stunden vor Substanz-Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten-Mittelwert, 30 Minuten-Mittelwert) und als Textdatei auf einen Datenträger übertragen. Die so vorsortierten und komprimierten Messwerte werden in Excel-Vorlagen übertragen und tabellarisch dargestellt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 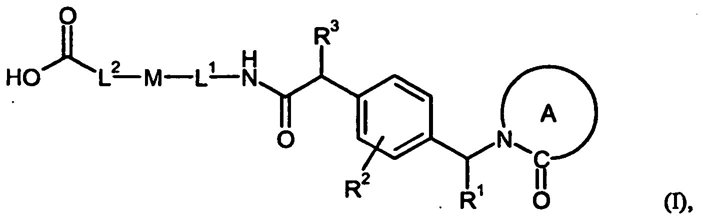 in welcher
der Ring A für einen N-verknüpften 5- bis 7-gliedrigen, gesättigten oder partiell ungesättigten, Oxo-substituierten Azaheterocyclus steht,
der (*i*) ein oder zwei weitere Heteroatome aus der Reihe N, O und/oder S als Ringglieder enthalten kann,
der (*ii*) mit einem Rest ausgewählt aus der Reihe Fluor, Chlor, (C₁-C₆)-Alkyl, Trifluormethyl, (C₃-C₇)-Cycloalkyl, 4- bis 7-gliedriges Heterocyclyl und Phenyl substituiert ist oder der benzo-anelliert ist,
wobei der Phenyl-Substituent und der anellierte Phenylring ihrerseits bis zu zweifach, gleich oder verschieden, mit einem Rest ausgewählt aus der Reihe Halogen, Cyano, (C₁-C₄)-Alkyl, (C₂-C₄)-Alkenyl, Trifluormethyl, (C₁-C₄)-Alkoxy und Trifluormethoxy substituiert sein können,
und
der (*iii*) darüber hinaus bis zu zweifach, gleich oder verschieden, mit weiteren Resten ausgewählt aus der Reihe Fluor, (C₁-C₆)-Alkyl, Trifluormethyl, Oxo, (C₃-C₇)-Cycloalkyl, 4- bis 7-gliedriges Heterocyclyl und Phenyl substituiert sein kann,
wobei Phenyl seinerseits bis zu zweifach, gleich oder verschieden, mit einem Rest ausgewählt aus der Reihe Halogen, Cyano, (C₁-C₄)-Alkyl, (C₂-C₄)-Alkenyl, Trifluormethyl, (C₁-C₄)-Alkoxy und Trifluormethoxy substituiert sein kann,
R¹ für Wasserstoff, (C₁-C₄)-Alkyl oder Cyclopropyl steht,
R² für Wasserstoff, Halogen, Cyano, (C₁-C₄)-Alkyl oder Trifluormethyl steht,
R³ für (C₃-C₆)-Alkyl oder (C₃-C₆)-Alkenyl, welche jeweils mit Cyano, (C₁-C₄)-Alkoxy oder Trifluormethoxy und bis zu sechsfach mit Fluor substituiert sein können,
oder
für (C₃-C₇)-Cycloalkyl oder (C₃-C₇)-Cycloalkenyl, welche jeweils bis zu zweifach, gleich oder verschieden, mit (C₁-C₄)-Alkyl, Trifluormethyl und (C₁-C₄)-Alkoxy sowie bis zu vierfach mit Fluor substituiert sein können,
oder
für Oxetanyl, Tetrahydrofuranyl oder Tetrahydropyranyl steht,
L¹ für eine Bindung oder für Methylen, Ethan-1,2-diyl oder Propan-1,3-diyl, welche jeweils bis zu zweifach, gleich oder verschieden, mit (C₁-C₄)-Alkyl substituiert sein können, steht,
L² für eine Bindung oder für Methylen, Ethan-1,2-diyl, Propan-1,3-diyl, Ethen-1,2-diyl oder Propen-1,3-diyl, welche jeweils bis zu zweifach, gleich oder verschieden, mit (C₁-C₄)-Alkyl substituiert sein können, steht
oder
für eine Gruppe der Formel 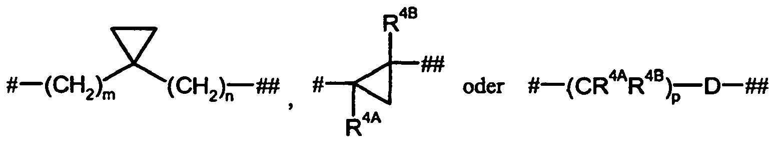 steht, worin
# die Verknüpfungsposition mit der Carbonsäure-Gruppierung kennzeichnet,
## die Verknüpfungsposition mit der Gruppe M kennzeichnet,
m die Zahl 0 oder 1 bedeutet,
n die Zahl 0, 1 oder 2 bedeutet,
p die Zahl 1 oder 2 bedeutet,
D O oder S bedeutet
und R^{4A} und R^{4B} voneinander Wasserstoff oder (C₁-C₄)-Alkyl bedeuten, wobei im Falle, dass die Gruppe -CR^{4A}R^{4B}- zweifach auftritt, die einzelnen Bedeutungen von R^{4A} und R^{4B} jeweils gleich oder verschieden sein können,
M für Phenylen oder 5- oder 6-gliedriges Heteroarylen mit bis zu zwei Ring-Heteroatomen aus der Reihe N, O und/oder S steht, wobei Phenylen und Heteroarylen jeweils bis zu zweifach, gleich oder verschieden, mit einem Rest ausgewählt aus der Reihe Halogen, Cyano, (C₁-C₄)-Alkyl, Trifluormethyl, Hydroxy, (C₁-C₄)-Alkoxy und Trifluormethoxy substituiert sein können,
oder
für Cyclopropan-1,2-diyl, Cyclobutan-1,2-diyl, Cyclobutan-1,3-diyl, Cyclopentan-1,2-diyl, Cyclopentan-1,3-diyl, Cyclohexan-1,2-diyl, Cyclohexan-1,3-diyl oder Cyclohexan-1,4-diyl steht, welche jeweils bis zu zweifach, gleich oder verschieden, mit einem Rest ausgewählt aus der Reihe Fluor, (C₁-C₄)-Alkyl, Trifluormethyl und (C₁-C₄)-Alkoxy substituiert sein können,
oder L² und M miteinander verknüpft sind und zusammen eine Gruppe der Formel 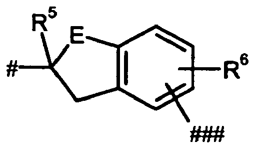 bilden, worin
# die Verknüpfungsposition mit der Carbonsäure-Gruppierung kennzeichnet,
### die Verknüpfungsposition mit der Gruppe L¹ kennzeichnet,
E O, S, CH₂ oder CH₂CH₂ bedeutet,
R⁵ und Wasserstoff, (C₁-C₄)-Alkyl oder Trifluormethyl bedeutet
R⁶ Wasserstoff, Halogen, (C₁-C₄)-Alkyl, Trifluormethyl, (C₁-C₄)-Alkoxy oder Trifluormethoxy bedeutet,
sowie ihre Salze, Solvate und Solvate der Salze.

2. Verbindung der Formel (I) nach Anspruch 1, in welcher
der Ring A für einen Oxo-substituierten Azaheterocyclus der Formel 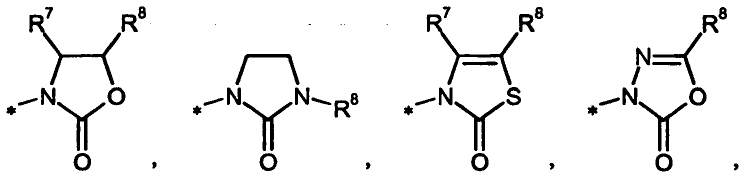 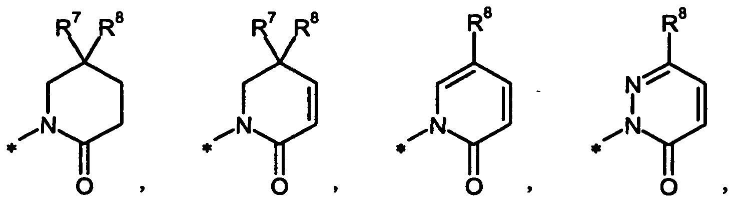 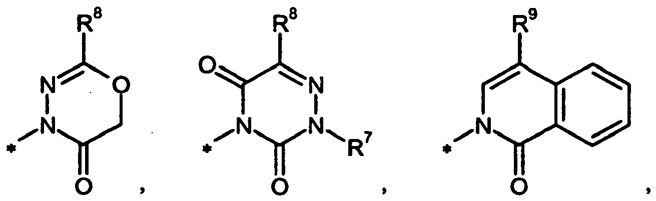 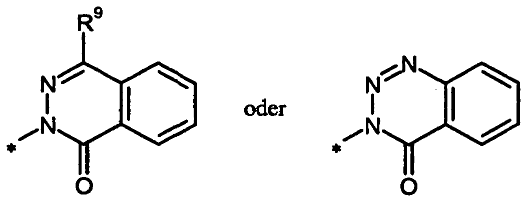 steht, worin
* die Verknüpfungsposition mit dem restlichen Teil des Moleküls kennzeichnet,
R⁷ Wasserstoff, (C₁-C₆)-Alkyl oder (C₃-C₆)-Cycloalkyl bedeutet,
R⁸ (C₁-C₆)-Alkyl, Trifluormethyl, (C₃-C₆)-Cycloalkyl, 4- bis 6-gliedriges Heterocyclyl oder Phenyl bedeutet, wobei Phenyl seinerseits bis zu zweifach, gleich oder verschieden, mit einem Rest ausgewählt aus der Reihe Fluor, Chlor, Brom, Cyano, (C₁-C₄)-Alkyl, Vinyl, Trifluormethyl, (C₁-C₄)-Alkoxy und Trifluormethoxy substituiert sein kann,
und R⁹ Wasserstoff bedeutet oder die oben genannte Bedeutung von R⁸ hat,
sowie ihre Salze, Solvate und Solvate der Salze.

3. Verbindung der Formel (I) nach Anspruch 1, in welcher
der Ring A für einen Oxo-substituierten Azaheterocyclus der Formel 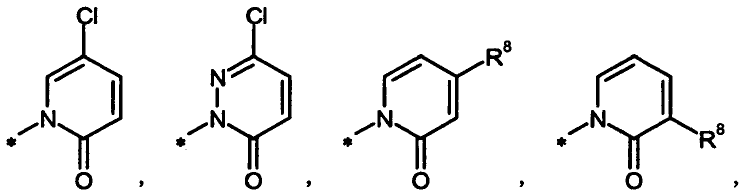 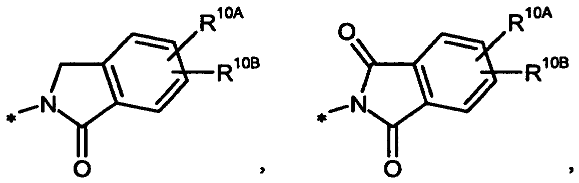 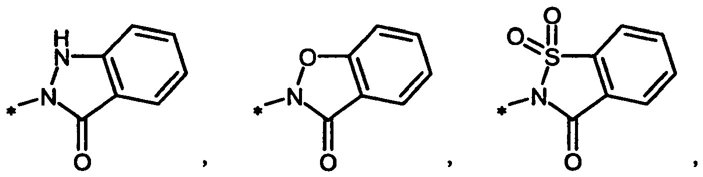 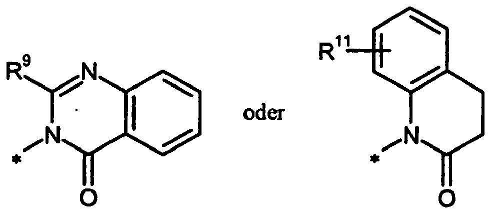 steht, worin
* die Verknüpfungsposition mit dem restlichen Teil des Moleküls kennzeichnet,
R⁸ (C₁-C₆)-Alkyl, Trifluormethyl, (C₃-C₆)-Cycloalkyl, 4- bis 6-gliedriges Heterocyclyl oder Phenyl bedeutet, wobei Phenyl seinerseits bis zu zweifach, gleich oder verschieden, mit einem Rest ausgewählt aus der Reihe Fluor, Chlor, Brom, Cyano, (C₁-C₄)-Alkyl, Vinyl, Trifluormethyl, (C₁-C₄)-Alkoxy und Trifluormethoxy substituiert sein kann,
R⁹ Wasserstoff bedeutet oder die oben genannte Bedeutung von R⁸ hat,
R^{10A} und R^{10B} unabhängig voneinander Wasserstoff, Fluor oder Chlor bedeuten
und R¹¹ Wasserstoff, Fluor oder Chlor bedeutet,
sowie ihre Salze, Solvate und Solvate der Salze.

4. Verbindung der Formel (I) nach Anspruch 1 oder 2, in welcher
der Ring A für einen Oxo-substituierten Azaheterocyclus der Formel 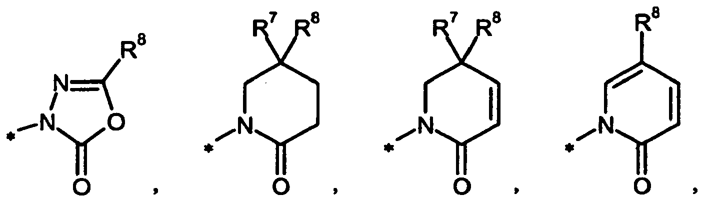 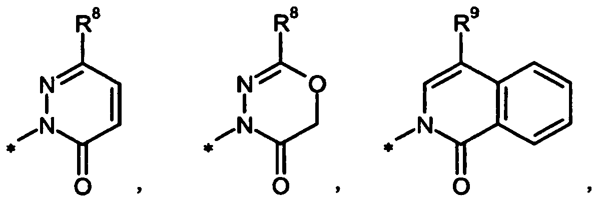 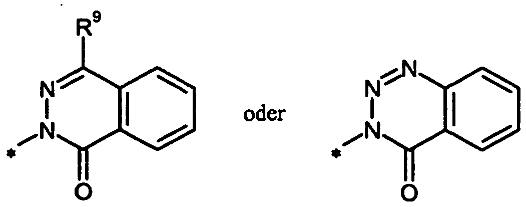 steht, worin
* die Verknüpfungsposition mit dem restlichen Teil des Moleküls kennzeichnet,
R⁷ Wasserstoff oder (C₁-C₄)-Alkyl bedeutet,
R⁸ (C₁-C₆)-Alkyl, Trifluormethyl, (C₃-C₆)-Cycloalkyl oder Phenyl bedeutet, wobei Phenyl seinerseits bis zu zweifach, gleich oder verschieden, mit einem Rest ausgewählt aus der Reihe Fluor, Chlor, Cyano, (C₁-C₄)-Alkyl, Trifluormethyl, (C₁-C₄)-Alkoxy und Trifluormethoxy substituiert sein kann,
und
R⁹ Wasserstoff bedeutet oder die zuvor genannte Bedeutung von R⁸ hat,
R¹ für Wasserstoff oder (C₁-C₄)-Alkyl steht,
R² für Wasserstoff, Fluor, Chlor oder Trifluormethyl steht,
R³ für (C₃-C₆)-Alkyl oder (C₃-C₆)-Alkenyl, welche jeweils mit Cyano, Methoxy, Ethoxy oder Trifluormethoxy und bis zu sechsfach mit Fluor substituiert sein können,
oder
für (C₃-C₆)-Cycloalkyl oder (C₄-C₆)-Cycloalkenyl, welche jeweils bis zu zweifach, gleich oder verschieden, mit (C₁-C₄)-Alkyl und Trifluormethyl sowie bis zu vierfach mit Fluor substituiert sein können,
oder
für Oxetanyl steht,
L¹ für eine Bindung oder für Methylen, Ethan-1,2-diyl oder Propan-1,3-diyl, welche jeweils bis zu zweifach mit Methyl substituiert sein können, steht,
L² für eine Bindung oder für Methylen, Ethan-1,2-diyl, Propan-1,3-diyl oder Ethen-1,2-diyl, welche jeweils bis zu zweifach mit Methyl substituiert sein können, steht
oder für eine Gruppe der Formel 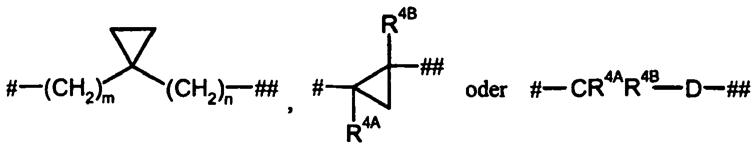 steht, worin
# die Verknüpfungsposition mit der Carbonsäure-Gruppierung kennzeichnet,
## die Verknüpfungsposition mit der Gruppe M kennzeichnet,
m die Zahl 0 oder 1 bedeutet,
n die Zahl 0, 1 oder 2 bedeutet,
D O oder S bedeutet
und R^{4A} und R^{4B} unabhängig voneinander Wasserstoff oder Methyl bedeuten,
M für Phenylen oder 5- oder 6-gliedriges Heteroarylen mit bis zu zwei Ring-Heteroatomen aus der Reihe N, O und/oder S steht, wobei Phenylen und Heteroarylen jeweils bis zu zweifach, gleich oder verschieden, mit einem Rest ausgewählt aus der Reihe Fluor, Chlor, (C₁-C₄)-Alkyl, Trifluormethyl, Methoxy und Trifluormethoxy substituiert sein können,
oder
für Cyclopentan-1,3-diyl, Cyclohexan-1,3-diyl oder Cyclohexan-1,4-diyl steht, welche jeweils bis zu zweifach, gleich oder verschieden, mit einem Rest ausgewählt aus der Reihe Fluor, Methyl und Trifluormethyl substituiert sein können,
oder L² und M miteinander verknüpft sind und zusammen eine Gruppe der Formel 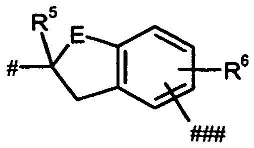 bilden, worin
# die Verknüpfungsposition mit der Carbonsäure-Gruppierung kennzeichnet,
### die Verknüpfungsposition mit der Gruppe L¹ kennzeichnet,
E CH₂ oder CH₂CH₂ bedeutet,
R⁵ Wasserstoff, Methyl oder Trifluormethyl bedeutet
und R⁶ Wasserstoff, Fluor, Chlor, Methyl, Trifluormethyl, Methoxy oder Trifluormethoxy bedeutet,
sowie ihre Salze, Solvate und Solvate der Salze.

5. Verbindung der Formel (I) nach Anspruch 1 oder 3, in welcher der Ring A für einen Oxo-substituierten Azaheterocyclus der Formel 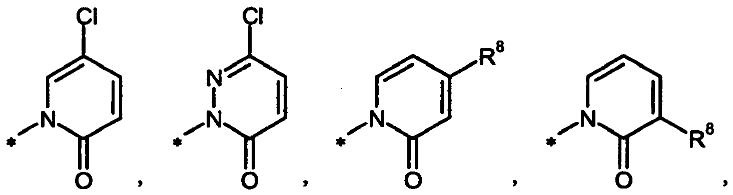 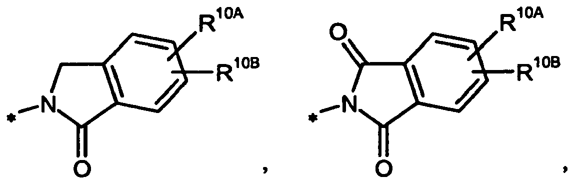 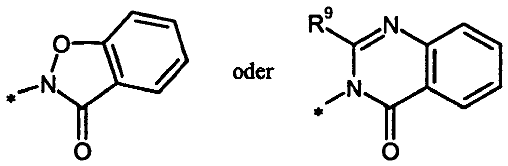 steht, worin
* die Verknüpfungsposition mit dem restlichen Teil des Moleküls kennzeichnet,
R⁸ (C₁-C₆)-Alkyl, Trifluormethyl, (C₃-C₆)-Cycloalkyl oder Phenyl bedeutet, wobei Phenyl seinerseits bis zu zweifach, gleich oder verschieden, mit einem Rest ausgewählt aus der Reihe Fluor, Chlor, Cyano, (C₁-C₄)-Alkyl, Trifluormethyl, (C₁-C₄)-Alkoxy und Trifluormethoxy substituiert sein kann,
R⁹ Wasserstoff bedeutet oder die zuvor genannte Bedeutung von R⁸ hat
und R^{10A} und R^{10B} unabhängig voneinander Wasserstoff, Fluor oder Chlor bedeuten,
R¹ für Wasserstoff oder (C₁-C₄)-Alkyl steht,
R² für Wasserstoff, Fluor, Chlor oder Trifluormethyl steht,
R³ für (C₃-C₆)-Alkyl oder (C₃-C₆)-Alkenyl, welche jeweils mit Cyano, Methoxy, Ethoxy oder Trifluormethoxy und bis zu sechsfach mit Fluor substituiert sein können,
oder
für (C₃-C₆)-Cycloalkyl oder (C₄-C₆)-Cycloalkenyl, welche jeweils bis zu zweifach, gleich oder verschieden, mit (C₁-C₄)-AlKyl und Trifluormethyl sowie bis zu vierfach mit Fluor substituiert sein können,
oder
für Oxetanyl steht,
L¹ für eine Bindung oder für Methylen, Ethan-1,2-diyl oder Propan-1,3-diyl, welche jeweils bis zu zweifach mit Methyl substituiert sein können, steht,
L² für eine Bindung oder für Methylen, Ethan-1,2-diyl, Propan-1,3-diyl oder Ethen-1,2-diyl, welche jeweils bis zu zweifach mit Methyl substituiert sein können, steht
oder
für eine Gruppe der Formel 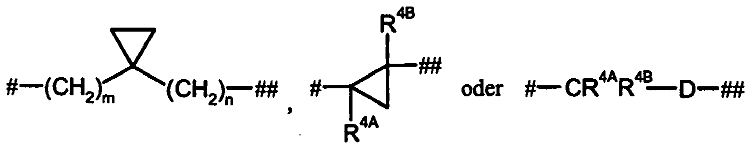 steht, worin
# die Verknüpfungsposition mit der Carbonsäure-Gruppierung kennzeichnet,
## die Verknüpfungsposition mit der Gruppe M kennzeichnet,
m die Zahl 0 oder 1 bedeutet,
n die Zahl 0, 1 oder 2 bedeutet,
D O oder S bedeutet
und R^{4A} und R^{4B} unabhängig voneinander Wasserstoff oder Methyl bedeuten,
M für Phenylen oder 5- oder 6-gliedriges Heteroarylen mit bis zu zwei Ring-Heteroatomen aus der Reihe N, O und/oder S steht, wobei Phenylen und Heteroarylen jeweils bis zu zweifach, gleich oder verschieden, mit einem Rest ausgewählt aus der Reihe Fluor, Chlor, (C₁-C₄)-Alkyl, Trifluormethyl, Methoxy und Trifluormethoxy substituiert sein können,
oder
für Cyclopentan-1,3-diyl, Cyclohexan-1,3-diyl oder Cyclohexan-1,4-diyl steht, welche jeweils bis zu zweifach, gleich oder verschieden, mit einem Rest ausgewählt aus der Reihe Fluor, Methyl und Trifluormethyl substituiert sein können,
oder L² und M miteinander verknüpft sind und zusammen eine Gruppe der Formel 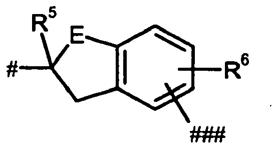 bilden, worin
# die Verknüpfungsposition mit der Carbonsäure-Gruppierung kennzeichnet,
### die Verknüpfungsposition mit der Gruppe L¹ kennzeichnet,
E CH₂ oder CH₂CH₂ bedeutet,
R⁵ Wasserstoff, Methyl oder Trifluormethyl bedeutet
und R⁶ Wasserstoff, Fluor, Chlor, Methyl, Trifluormethyl, Methoxy oder Trifluormethoxy bedeutet,
sowie ihre Salze, Solvate und Solvate der Salze.

6. Verbindung der Formel (I) nach Anspruch 1, 2 oder 4, in welcher
der Ring A für einen Oxo-substituierten Azaheterocyclus der Formel 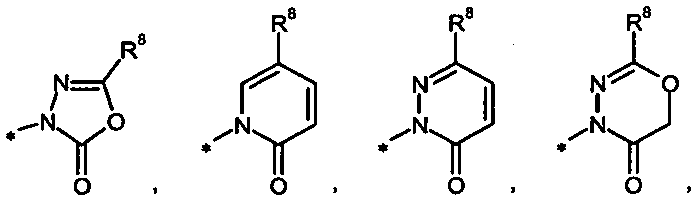 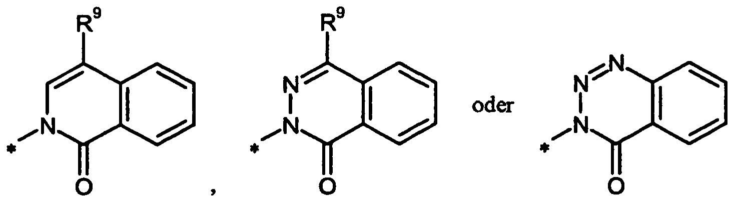 steht, worin
* die Verknüpfungsposition mit dem restlichen Teil des Moleküls kennzeichnet,
R⁸ Trifluormethyl, (C₃-C₆)-Cycloalkyl oder Phenyl bedeutet, wobei Phenyl seinerseits bis zu zweifach, gleich oder verschieden, mit einem Rest ausgewählt aus der Reihe Fluor, Chlor, Cyano, Methyl, Trifluormethyl, Methoxy und Trifluormethoxy substituiert sein kann,
und
R⁹ Wasserstoff bedeutet oder die zuvor genannte Bedeutung von R⁸ hat,
R¹ für Wasserstoff oder Methyl steht,
R² für Wasserstoff, Fluor oder Chlor steht,
R³ für (C₃-C₆)-Alkyl oder (C₃-C₆)-Alkenyl, welche jeweils bis zu sechsfach mit Fluor substituiert sein können, steht
oder
für (C₃-C₆)-Cycloalkyl, Cyclopentenyl oder Cyclohexenyl, welche jeweils bis zu zweifach, gleich oder verschieden, mit Methyl, Ethyl und Trifluormethyl sowie bis zu vierfach mit Fluor substituiert sein können, steht,
L¹ für eine Bindung oder für Methylen oder Ethan-1,2-diyl steht,
L² für eine Bindung oder für Methylen, Ethan-1,2-diyl, Propan-1,3-diyl oder Ethen-1,2-diyl, welche jeweils bis zu zweifach mit Methyl substituiert sein können, steht
oder
für eine Gruppe der Formel 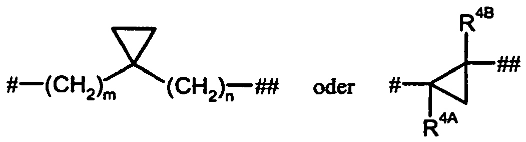 steht,
worin
# die Verknüpfungsposition mit der Carbonsäure-Gruppierung kennzeichnet,
## die Verknüpfungsposition mit der Gruppe M kennzeichnet,
m die Zahl 0 oder 1 bedeutet,
n die Zahl 1 oder 2 bedeutet,
und R^{4A} und R^{4B} unabhängig voneinander Wasserstoff oder Methyl bedeuten,
M für Phenylen, Pyridylen, Furylen, Thienylen, Thiazolylen oder Isoxazolylen, welche jeweils bis zu zweifach, gleich oder verschieden, mit einem Rest ausgewählt aus der Reihe Fluor, Chlor, Methyl und Trifluormethyl substituiert sein können, steht
oder
für Cyclopentan-1,3-diyl, Cyclohexan-1,3-diyl oder Cyclohexan-1,4-diyl, welche jeweils bis zu zweifach, gleich oder verschieden, mit Fluor und Methyl substituiert sein können, steht,
oder L² und M miteinander verknüpft sind und zusammen eine Gruppe der Formel 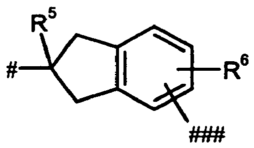 bilden, worin
# die Verknüpfungsposition mit der Carbonsäure-Gruppierung kennzeichnet,
### die Verknüpfungsposition mit der Gruppe L¹ kennzeichnet,
R⁵ Wasserstoff, Methyl oder Trifluormethyl bedeutet
und R⁶ Wasserstoff, Fluor oder Chlor bedeutet,
sowie ihre Salze, Solvate und Solvate der Salze.

7. Verbindung der Formel (I) nach Anspruch 1, 3 oder 5, in welcher
der Ring A für einen Oxo-substituierten Azaheterocyclus der Formel 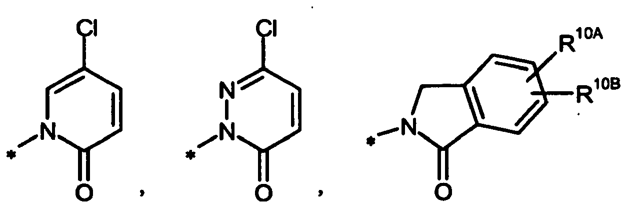 oder 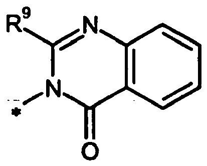 steht, worin
* die Verknüpfungsposition mit dem restlichen Teil des Moleküls kennzeichnet,
R⁹ Wasserstoff, Methyl oder Trifluormethyl bedeutet
und R^{10A} und R^{10B} unabhängig voneinander Wasserstoff oder Fluor bedeuten,
R¹ für Wasserstoff oder Methyl steht,
R² für Wasserstoff, Fluor oder Chlor steht,
R³ für (C₃-C₆)-Alkyl oder (C₃-C₆)-Alkenyl, welche jeweils bis zu sechsfach mit Fluor substituiert sein können, steht
oder
für (C₃-C₆)-Cycloalkyl, Cyclopentenyl oder Cyclohexenyl, welche jeweils bis zu zweifach, gleich oder verschieden, mit Methyl, Ethyl und Trifluormethyl sowie bis zu vierfach mit Fluor substituiert sein können, steht,
L¹ für eine Bindung oder für Methylen oder Ethan-1,2-diyl steht,
L² für eine Bindung oder für Methylen, Ethan-1,2-diyl, Propan-1,3-diyl oder Ethen-1,2-diyl, welche jeweils bis zu zweifach mit Methyl substituiert sein können, steht
oder
für eine Gruppe der Formel 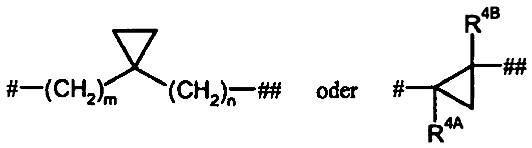 steht,
worin
# die Verknüpfungsposition mit der Carbonsäure-Gruppierung kennzeichnet,
## die Verknüpfungsposition mit der Gruppe M kennzeichnet,
m die Zahl 0 oder 1 bedeutet,
n die Zahl 1 oder 2 bedeutet,
und R^{4A} und R^{4B} unabhängig voneinander Wasserstoff oder Methyl bedeuten,
M für Phenylen, Pyridylen, Furylen, Thienylen, Thiazolylen oder Isoxazolylen, welche jeweils bis zu zweifach, gleich oder verschieden, mit einem Rest ausgewählt aus der Reihe Fluor, Chlor, Methyl und Trifluormethyl substituiert sein können, steht
oder
für Cyclopentan-1,3-diyl, Cyclohexan-1,3-diyl oder Cyclohexan-1,4-diyl, welche jeweils bis zu zweifach, gleich oder verschieden, mit Fluor und Methyl substituiert sein können, steht,
oder L² und M miteinander verknüpft sind und zusammen eine Gruppe der Formel 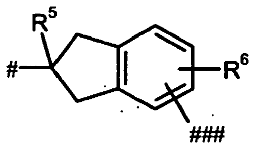 bilden, worin
# die Verknüpfungsposition mit der Carbonsäure-Gruppierung kennzeichnet,
### die Verknüpfungsposition mit der Gruppe L¹ kennzeichnet,
R⁵ Wasserstoff, Methyl oder Trifluormethyl bedeutet
und R⁶ Wasserstoff, Fluor oder Chlor bedeutet,
sowie ihre Salze, Solvate und Solvate der Salze.

8. Verbindung der Formel (I) nach Anspruch 1, 2, 4 oder 6, in welcher
der Ring A für einen Oxo-substituierten Azaheterocyclus der Formel 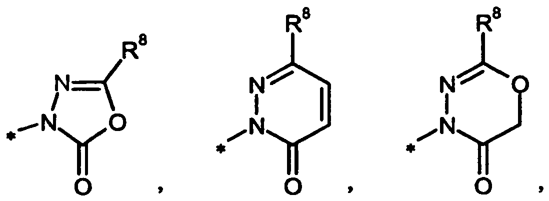 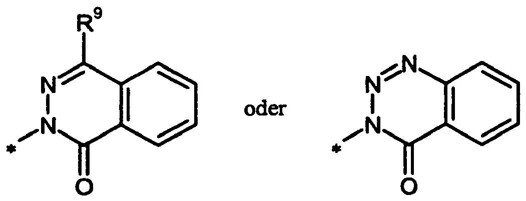 steht, worin
* die Verknüpfungsposition mit dem restlichen Teil des Moleküls kennzeichnet,
R⁸ Trifluormethyl oder Phenyl, welches bis zu zweifach, gleich oder verschieden, mit einem Rest ausgewählt aus der Reihe Fluor, Chlor, Methyl und Trifluormethyl substituiert sein kann, bedeutet
und R⁹ Wasserstoff bedeutet oder die zuvor genannte Bedeutung von R⁸ hat,
R¹ für Wasserstoff steht,
R² für Wasserstoff steht,
R³ für Propan-2-yl, Butan-2-yl, Pentan-2-yl, 1,1,1-Trifluorpropan-2-yl, 1,1,1-Trifluorbutan-2-yl, 4,4,4-Trifluorbutan-2-yl, 4,4,4-Trifluor-2-methylbutan-1-yl, Cyclopentyl oder 3,3-Difluorcyclopentyl steht,
L¹ für eine Bindung oder für Methylen steht,
L² für eine Bindung, für Methylen oder Ethan-1,2-diyl, welche jeweils bis zu zweifach mit Methyl substituiert sein können, oder für Ethen-1,2-diyl steht
oder
für eine Gruppe der Formel 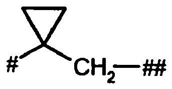 steht, worin
# die Verknüpfungsposition mit der Carbonsäure-Gruppierung
und ## die Verknüpfungsposition mit der Gruppe M kennzeichnen,
M für 1,3-Phenylen oder 1,4-Phenylen, welche bis zu zweifach, gleich oder verschieden, mit Fluor, Chlor, Methyl und Trifluormethyl substituiert sein können, oder für Cyclohexan-1,3-diyl oder Cyclohexan-1,4-diyl steht,
oder L² und M miteinander verknüpft sind und zusammen eine Gruppe der Formel 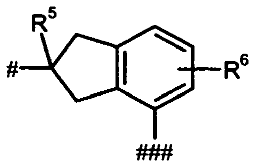 bilden, worin
# die Verknüpfungsposition mit der Carbonsäure-Gruppierung kennzeichnet,
### die Verknüpfungsposition mit der Gruppe L¹ kennzeichnet,
R⁵ Wasserstoff oder Methyl bedeutet
und R⁶ Wasserstoff oder Fluor bedeutet,
sowie ihre Salze, Solvate und Solvate der Salze.

9. Verbindung der Formel (I) nach Anspruch 1, 3, 5 oder 7, in welcher
der Ring A für einen Oxo-substituierten Azaheterocyclus der Formel 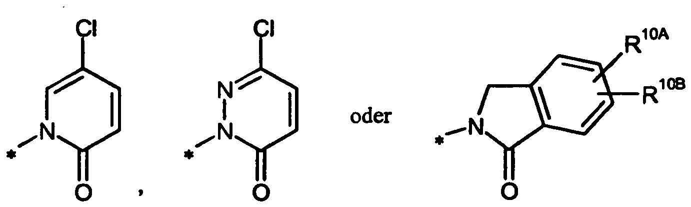 steht, worin
* die Verknüpfungsposition mit dem restlichen Teil des Moleküls kennzeichnet
und R^{10A} und R^{10B} unabhängig voneinander Wasserstoff oder Fluor bedeuten,
R¹ für Wasserstoff steht,
R² für Wasserstoff steht,
R³ für Propan-2-yl, Butan-2-yl, Pentan-2-yl, 1,1,1-Trifluorpropan-2-yl, 1,1,1-Trifluorbutan-2-yl, 4,4,4-Trifluorbutan-2-yl, 4,4,4-Trifluor-2-methylbutan-1-yl, Cyclopentyl oder 3,3-Difluorcyclopentyl steht,
L¹ für eine Bindung steht,
L² für Methylen oder Ethan-1,2-diyl, welche jeweils bis zu zweifach mit Methyl substituiert sein können, oder für Ethen-1,2-diyl steht
oder
für eine Gruppe der Formel 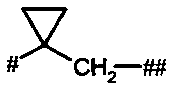 steht, worin
# und die Verknüpfungsposition mit der Carbonsäure-Gruppierung
## die Verknüpfungsposition mit der Gruppe M kennzeichnen,
M für 1,3-Phenylen oder 1,4-Phenylen, welche bis zu zweifach, gleich oder verschieden, mit Fluor, Chlor, Methyl und Trifluormethyl substituiert sein können, steht,
oder L² und M miteinander verknüpft sind und zusammen eine Gruppe der Formel 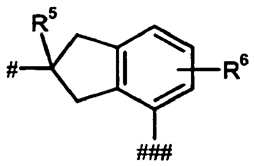 bilden, worin
# die Verknüpfungsposition mit der Carbonsäure-Gruppierung kennzeichnet,
### die Verknüpfungsposition mit der Gruppe L¹ kennzeichnet,
R⁵ Wasserstoff oder Methyl bedeutet
und R⁶ Wasserstoff oder Fluor bedeutet,
sowie ihre Salze, Solvate und Solvate der Salze.

10. Verfahren zur Herstellung einer Verbindung der Formel (I), wie in den Ansprüchen 1 bis 9 definiert, **dadurch gekennzeichnet, dass** man zunächst eine Verbindung der Formel (II) 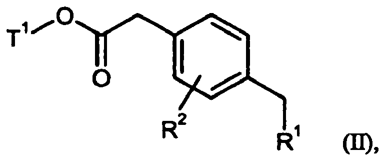 in welcher R¹ und R² die in den Ansprüchen 1 bis 9 angegebenen Bedeutungen haben
und
T¹ für (C₁-C₄)-Alkyl steht,
in einem inerten Lösungsmittel in Gegenwart einer Base mit einer Verbindung der Formel (III) 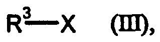 in welcher R³ die in den Ansprüchen 1 bis 9 angegebene Bedeutung hat
und
X für eine Abgangsgruppe wie beispielsweise Halogen, Mesylat, Tosylat oder Triflat steht,
in eine Verbindung der Formel (IV) 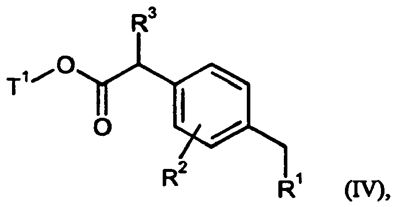 in welcher R¹, R², R³ und T¹ jeweils die oben angegebenen Bedeutungen haben,
überführt, diese danach in einem inerten Lösungsmittel mit elementarem Brom oder mit N-Bromsuccinimid zu einer Verbindung der Formel (V) 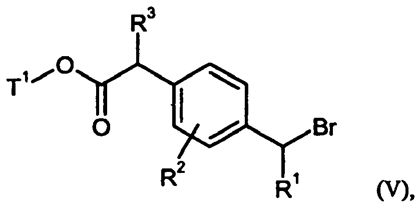 in welcher R¹, R², R³ und T¹ jeweils die oben angegebenen Bedeutungen haben,
bromiert, anschließend in einem inerten Lösungsmittel in Gegenwart einer Base mit einer Verbindung der Formel (VI) 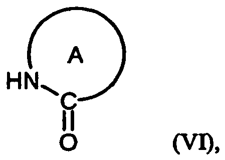 in welcher der Ring A für einen Oxo-substituierten Azaheterocyclus, wie in den Ansprüchen 1 bis 9 definiert, steht,
zu einer Verbindung der Formel (VII) 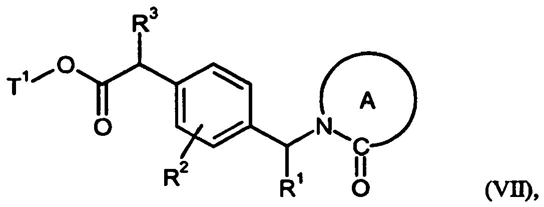 in welcher der Ring A, R¹, R², R³ und T¹ jeweils die oben angegebenen Bedeutungen haben,
umsetzt, nachfolgend den Ester-Rest T¹ in (VII) unter basischen oder sauren Bedingungen abspaltet, die resultierende Carbonsäure der Formel (VIII) 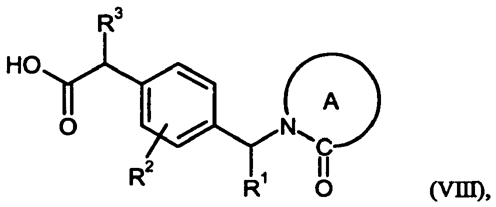 in welcher der Ring A, R¹, R² und R³ jeweils die oben angegebenen Bedeutungen haben,
dann in einem inerten Lösungsmittel in Gegenwart eines Kondensationsmittels oder über die Zwischenstufe des entsprechenden Carbonsäurechlorids in Gegenwart einer Base mit einem Amin der Formel (IX) 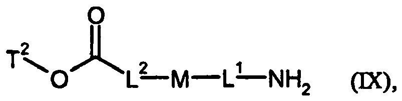 in welcher L¹, L² und M die in den Ansprüchen 1 bis 9 angegebenen Bedeutungen haben
und
T² für (C₁-C₄)-Alkyl steht,
zu einer Verbindung der Formel (X) 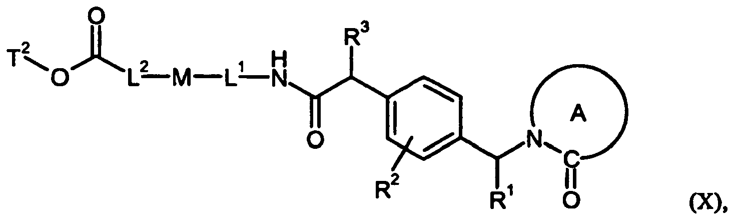 in welcher der Ring A, R¹, R², R³, L¹, L², M und T² jeweils die oben angegebenen Bedeutungen haben,
kuppelt und abschließend den Ester-Rest T² in (X) durch erneute basische oder saure Solvolyse zur Carbonsäure der Formel (I) abspaltet
und die Verbindungen der Formel (I) gegebenenfalls nach dem Fachmann bekannten Methoden in ihre Enantiomere und/oder Diastereomere trennt und/oder gegebenenfalls mit den entsprechenden (i) Lösungsmitteln und/oder (ii) Basen oder Säuren zu ihren Solvaten, Salzen und/oder Solvaten der Salze umsetzt.

11. Verbindung, wie in einem der Ansprüche 1 bis 9 definiert, zur Behandlung und/oder Prävention von Krankheiten.

12. Verbindung, wie in einem der Ansprüche 1 bis 9 definiert, zur Verwendung in einem Verfahren zur Behandlung und/oder Prävention von Herzinsuffizienz, Angina pectoris, Hypertonie, pulmonaler Hypertonie, Ischämien, Gefäßerhankungen, thromboembolischen Erkrankungen und Arteriosklerose.

13. Verwendung einer Verbindung, wie in einem der Ansprüche 1 bis 9 definiert, zur Herstellung eines Arzneimittels zur Behandlung und/oder Prävention von Herzinsuffizienz, Angina pectoris, Hypertonie, pulmonaler Hypertonie, Ischämien, Gefäßerkrankungen, thromboembolischen Erkrankungen und Arteriosklerose.

14. Arzneimittel enthaltend eine Verbindung, wie in einem der Ansprüche 1 bis 9 definiert, in Kombination mit einem oder mehreren inerten, nicht-toxischen, pharmazeutisch geeigneten Hilfsstoffen.

15. Arzneimittel enthaltend eine Verbindung, wie in einem der Ansprüche 1 bis 9 definiert, in Kombination mit einem oder mehreren weiteren Wirkstoffen ausgewählt aus der Gruppe bestehend aus organischen Nitraten, NO-Donatoren, cGMP-PDE-Inhibitoren, Stimulatoren der Guanylatcyclase, antithrombotisch wirkenden Mitteln, den Blutdruck senkenden Mitteln sowie den Fettstoffwechsel verändernden Mitteln.

16. Arzneimittel nach Anspruch 14 oder 15 zur Behandlung und/oder Prävention von Herzinsuffizienz, Angina pectoris, Hypertonie, pulmonaler Hypertonie, Ischämien, Gefäßerkrankungen, thromboembolischen Erkrankungen und Arteriosklerose.

## Claims

1. Compound of the formula (I) 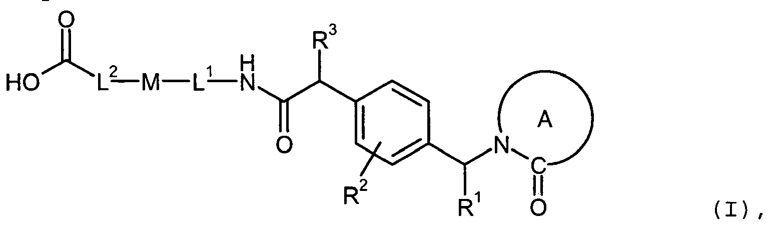 in which
ring A represents a 5- to 7-membered saturated or partially unsaturated oxo-substituted azaheterocycle attached via nitrogen, which (*i*) may contain one or two further heteroatoms from the group consisting of N, O and S as ring members, which (*ii*) is substituted by a radical selected from the group consisting of fluorine, chlorine, (C₁-C₆)-alkyl, trifluoromethyl, (C₃-C₇)-cycloalkyl, 4- to 7-membered heterocyclyl and phenyl or is benzo-fused,
where the phenyl substituent and the fused phenyl ring for their part may be substituted up to two times by identical or different radicals selected from the group consisting of halogen, cyano, (C₁-C₄)-alkyl , (C₂-C₄)-alkenyl, trifluoromethyl, (C₁-C₄)-alkoxy and trifluoromethoxy,
and
which (*iii*) may additionally be substituted up to two times by identical or different further radicals selected from the group consisting of fluorine, (C₁-C₆)-alkyl, trifluoromethyl, oxo, (C₃-C₇)-cycloalkyl, 4-to 7-membered heterocyclyl and phenyl,
where phenyl for its part may be substituted up to two times by identical or different radicals selected from the group consisting of halogen, cyano, (C₁-C₄)-alkyl, (C₂-C₄)-alkenyl, trifluoromethyl, (C₁-C₄)-alkoxy and trifluoromethoxy,
R¹ represents hydrogen, (C₁-C₄)-alkyl or cyclopropyl,
R² represents hydrogen, halogen, cyano, (C₁-C₄)-alkyl or trifluoromethyl,
R³ represents (C₃-C₆)-alkyl or (C₃-C₆)-alkenyl, each of which may be substituted by cyano, (C₁-C₄)-alkoxy or trifluoromethoxy and up to six times by fluorine,
or
represents (C₃-C₇-cycloalkyl or (C₃-C₇)-cycloalkenyl, each of which may be substituted up to two times by identical or different radicals from the group consisting of (C₁-C₄)-alkyl, trifluoromethyl and (C₁-C₄)-alkoxy and also up to four times by fluorine,
or
represents oxetanyl, tetrahydrofuranyl or tetrahydropyranyl,
L¹ represents a bond or represents methylene, ethane-1,2-diyl or propane-1,3-diyl, each of which may be substituted up to two times by identical or different radicals from the group consisting of (C₁-C₄)-alkyl,
L² represents a bond or represents methylene, ethane-1,2-diyl, propane-1,3-diyl, ethene- 1,2-diyl or propene-1,3-diyl, each of which may be substituted up to two times by identical or different radicals from the group consisting of (C₁-C₄)-alkyl, or represents a group of the formula 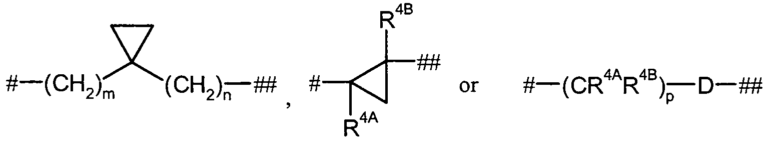 in which
# denotes the point of attachment to the carboxylic acid grouping,
## denotes the point of attachment to group
M, m represents the number 0 or 1,
n represents the number 0, 1 or 2,
p represents the number 1 or 2,
D represents O or S
and R^{4A} and R^{4B} independently of one another represent hydrogen or (C₁-C₄)-alkyl, where in the case that the group -CR^{4A}R^{4B}-appears twice, the individual meanings of R^{4A} and R^{4B} may in each case be identical or different,
M represents phenylene or 5- or 6-membered heteroarylene having up to two ring heteroatoms from the group consisting of N, O and *S*, where phenylene and heteroarylene may each be substituted up to two times by identical or different radicals selected from the group consisting of halogen, cyano, (C₁-C₄)-alkyl, trifluoromethyl , hydroxyl, (C₁-C₄)-alkoxy and trifluoromethoxy,
or
represents cyclopropane-1,2-diyl, cyclobutane-1,2-diyl, cyclobutane-1,3-diyl, cyclopentane-1,2-diyl, cyclopentane-1,3-diyl, cyclohexane-1,2-diyl, cyclohexane-1,3-diyl or cyclohexane-1,4-diyl, each of which may be substituted up to two times by identical or different radicals selected from the group consisting of fluorine, (C₁-C₄)-alkyl, trifluoromethyl and (C₁-C₄)-alkoxy,
or
L² and M are attached to one another and together form a group of the formula 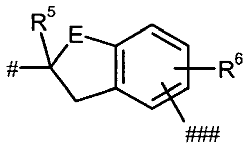 in which
# denotes the point of attachment to the carboxylic acid grouping,
### denotes the point of attachment to group
L¹, E represents O, S, CH₂ or CH₂CH₂,
R⁵ represents hydrogen, (C₁-C₄)-alkyl or trifluoromethyl
and R⁶ represents hydrogen, halogen, (C₁-C₄)-alkyl, trifluoromethyl, (C₁-C₄)-alkoxy or trifluoromethoxy,
or a salt, solvate or solvate of a salt thereof.

2. Compound of the formula (I) according to Claim 1 in which
ring A represents an oxo-substituted azaheterocycle of the formula 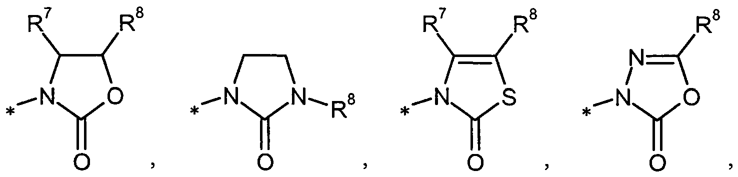 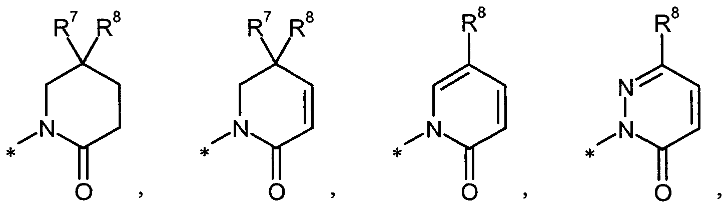 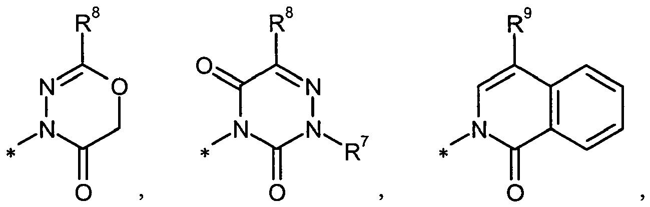 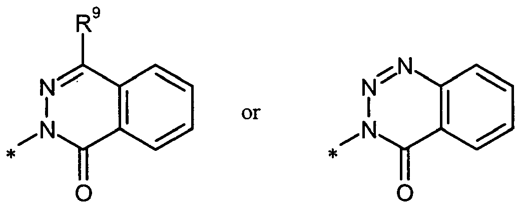 in which
* denotes the point of attachment to the remainder of the molecule,
R⁷ represents hydrogen, (C₁-C₆)-alkyl or (C₃-C₆)-cycloalkyl,
R⁸ represents (C₁-C₆)-alkyl, trifluoromethyl, (C₃-C₆)-cycloalkyl, 4-to 6-membered heterocyclyl or phenyl, where phenyl for its part may be substituted up to two times by identical or different radicals selected from the group consisting of fluorine, chlorine, bromine, cyano, (C₁-C₄)-alkyl, vinyl, trifluoromethyl, (C₁-C₄)-alkoxy and trifluoromethoxy, and R⁹ represents hydrogen or has the meaning of R⁸ given above,
or a salt, solvate or solvate of a salt thereof.

3. Compound of the formula (I) according to Claim 1 in which
ring A represents an oxo-substituted
azaheterocycle of the formula 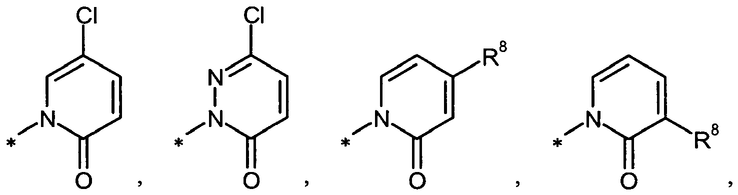 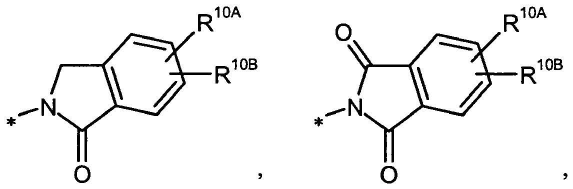 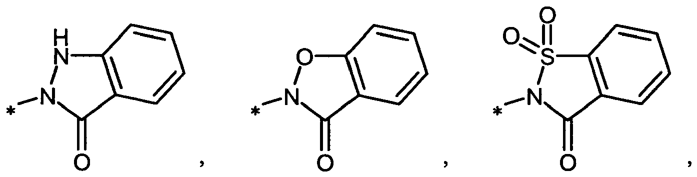 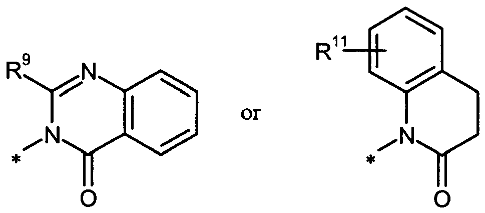 in which
* denotes the point of attachment to the remainder of the molecule,
R⁸ represents (C₁-C₆)-alkyl, trifluoromethyl, (C₃-C₆)-cycloalkyl, 4-to 6-membered heterocyclyl or phenyl, where phenyl for its part may be substituted up to two times by identical or different radicals selected from the group consisting of fluorine, chlorine, bromine, cyano, (C₁-C₄)-alkyl, vinyl, trifluoromethyl, (C₁-C₄)-alkoxy and trifluoromethoxy,
R⁹ of R⁸ represents hydrogen or has the meaning given above,
R^{10A} and R^{10B} independently of one another represent hydrogen, fluorine or chlorine
and R¹¹ represents hydrogen, fluorine or chlorine,
or a salt, solvate or solvate of a salt thereof.

4. Compound of the formula (I) according to Claim 1 or 2 in which
ring A represents an oxo-substituted azaheterocycle of the formula 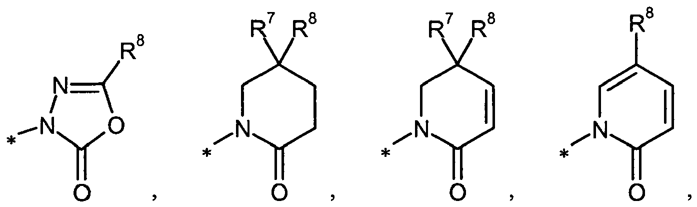 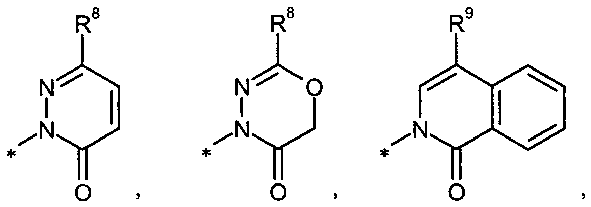 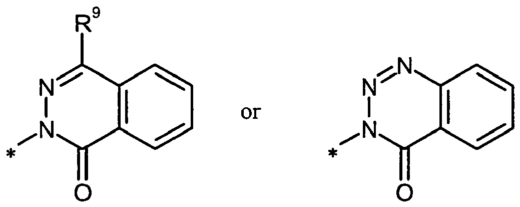 in which
* denotes the point of attachment to the remainder of the molecule,
R⁷ represents hydrogen or (C₁-C₄)-alkyl,
R⁸ represents (C₁-C₆)-alkyl, trifluoromethyl, (C₃-C₆)-cycloalkyl or phenyl, where phenyl for its part may be substituted up to two times by identical or different radicals selected from the group consisting of fluorine, chlorine, cyano, (C₁-C₄)-alkyl, trifluoromethyl, (C₁-C₄)-alkoxy and trifluoromethoxy,
and R⁹ represents hydrogen or has the meaning of R⁸ given above,
R¹ represents hydrogen or (C₁-C₄)-alkyl,
R² represents hydrogen, fluorine, chlorine or trifluoromethyl,
R³ represents (C₃-C₆)-alkyl or (C₃-C₆)-alkenyl, each of which may be substituted by cyano, methoxy, ethoxy or trifluoromethoxy and up to six times by fluorine,
or
represents (C₃-C₆)-cycloalkyl or (C₄-C₆)-cycloalkenyl, each of which may be substituted up to two times by identical or different radicals from the group consisting of (C₁-C₄)-alkyl and trifluoromethyl and also up to four times by fluorine,
or
represents oxetanyl,
L¹ represents a bond or represents methylene, ethane-1,2-diyl or propane-1,3-diyl, each of which may be substituted up to two times by methyl,
L² represents a bond or represents methylene, ethane-1,2-diyl, propane-1,3-diyl or ethene-1,2-diyl, each of which may be substituted up to two times by methyl, or represents a group of the formula 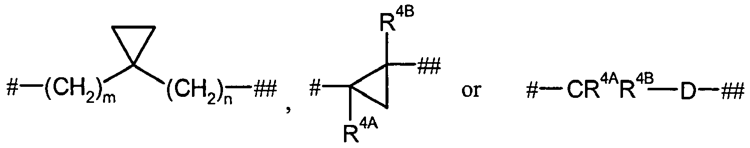 in which
# denotes the point of attachment to the carboxylic acid grouping,
## denotes the point of attachment to group
M, m represents the number 0 or 1,
n represents the number 0, 1 or 2,
D represents O or S
and
R^{4A} and R^{4B} independently of one another represent hydrogen or methyl,
M represents phenylene or 5- or 6-membered heteroarylene having up to two ring heteroatoms from the group consisting of N, O and S, where phenylene and heteroarylene may each be substituted up to two times by identical or different radicals selected from the group consisting of fluorine, chlorine, (C₁-C₄)-alkyl, trifluoromethyl, methoxy and trifluoromethoxy,
or
represents cyclopentane-1,3-diyl, cyclohexane-1,3-diyl or cyclohexane-1,4-diyl, each of which may be substituted up to two times by identical or different radicals selected from the group consisting of fluorine, methyl and trifluoromethyl,
or
L² and M are attached to one another and together form a group of the formula 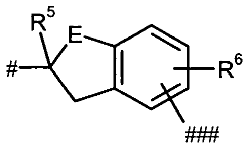 in which
# denotes the point of attachment to the carboxylic acid grouping,
### denotes the point of attachment to group
L¹, E represents CH₂ or CH₂CH₂,
R⁵ represents hydrogen, methyl or trifluoromethyl
and R⁶ represents hydrogen, fluorine, chlorine, methyl, trifluoromethyl, methoxy or trifluoromethoxy,
or a salt, solvate or solvate of a salt thereof.

5. Compound of the formula (I) according to Claim 1 or 3 in which
ring A represents an oxo-substituted azaheterocycle of the formula 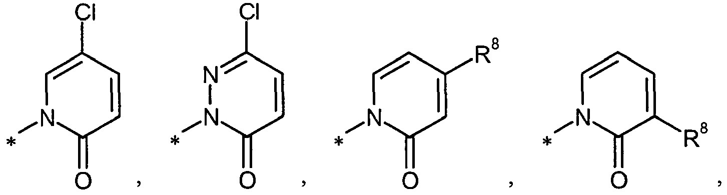 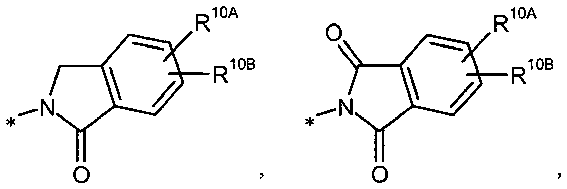 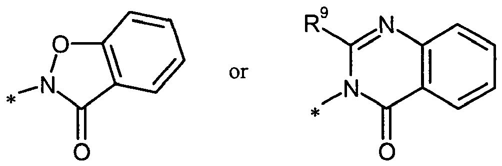 in which
* denotes the point of attachment to the remainder of the molecule,
R⁸ represents (C₁-C₆)-alkyl, trifluoromethyl, (C₃-C₆)-cycloalkyl or phenyl, where phenyl for its part may be substituted up to two times by identical or different radicals selected from the group consisting of fluorine, chlorine, cyano, (C₁-C₄)-alkyl, trifluoromethyl, (C₁-C₄)-alkoxy and trifluoromethoxy,
R⁹ represents hydrogen or has the meaning of R⁸ given above
and R^{10A} and R^{10B} independently of one another represent hydrogen, fluorine or chlorine,
R¹ represents hydrogen or (C₁-C₄)-alkyl,
R² represents hydrogen, fluorine, chlorine or trifluoromethyl,
R³ represents (C₃-C₆)-alkyl or (C₃-C₆)-alkenyl, each of which may be substituted by cyano, methoxy, ethoxy or trifluoromethoxy and up to six times by fluorine,
or
represents (C₃-C₆)-cycloalkyl or (C₄-C₆)-cycloalkenyl, each of which may be substituted up to two times by identical or different radicals from the group consisting of (C₁-C₄)-alkyl and trifluoromethyl and also up to four times by fluorine,
or
represents oxetanyl,
L¹ represents a bond or represents methylene, ethane-1,2-diyl or propane-1,3-diyl, each of which may be substituted up to two times by methyl,
L² represents a bond or represents methylene, ethane-1,2-diyl, propane-1,3-diyl or ethene-1,2-diyl, each of which may be substituted up to two times by methyl, or represents a group of the formula 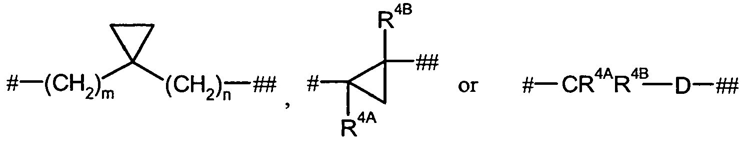 in which
# denotes the point of attachment to the carboxylic acid grouping,
## denotes the point of attachment to group
M, m represents the number 0 or 1,
n represents the number 0, 1 or 2,
D represents O or S and
R^{4A} and R^{4B} independently of one another represent hydrogen or methyl,
M represents phenylene or 5- or 6-membered heteroarylene having up to two ring heteroatoms from the group consisting of N, O and S, where phenylene and heteroarylene may each be substituted up to two times by identical or different radicals selected from the group consisting of fluorine, chlorine, (C₁-C₄)-alkyl, trifluoromethyl, methoxy and trifluoromethoxy,
or
represents cyclopentane-1,3-diyl, cyclohexane-1,3-diyl or cyclohexane-1,4-diyl, each of which may be substituted up to two times by identical or different radicals selected from the group consisting of fluorine, methyl and trifluoromethyl, or
L² and M are attached to one another and together form a group of the formula 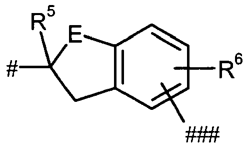 in which
# denotes the point of attachment to the carboxylic acid grouping,
### denotes the point of attachment to group
L¹, E represents CH₂ or CH₂CH₂,
R⁵ represents hydrogen, methyl or trifluoromethyl
and R⁶ represents hydrogen, fluorine, chlorine, methyl, trifluoromethyl, methoxy or trifluoromethoxy,
or a salt, solvate or solvate of a salt thereof.

6. Compound of the formula (I) according to Claim 1, 2 or 4 in which
ring A represents an oxo-substituted azaheterocycle of the formula 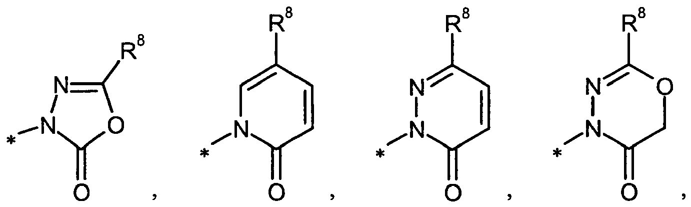 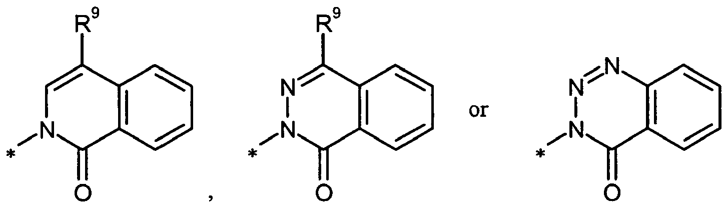 in which
* denotes the point of attachment to the remainder of the molecule,
R⁸ represents trifluoromethyl, (₋C₃-C₆)-cycloalkyl or phenyl, where phenyl for its part may be substituted up to two times by identical or different radicals selected from the group consisting of fluorine, chlorine, cyano, methyl, trifluoromethyl, methoxy and trifluoromethoxy, and
R⁹ represents hydrogen or has the meaning of R⁸ given above,
R¹ represents hydrogen or methyl,
R² represents hydrogen, fluorine or chlorine,
R³ represents (C₃-C₆)-alkyl or (C₃-C₆)-alkenyl, each of which may be substituted up to six times by fluorine,
or
represents (C₃-C₆)-cycloalkyl, cyclopentenyl or cyclohexenyl, each of which may be substituted up to two times by identical or different radicals from the group consisting of methyl, ethyl and trifluoromethyl and also up to four times by fluorine,
L¹ represents a bond or represents methylene or ethane-1,2-diyl,
L² represents a bond or represents methylene, ethane-1,2-diyl, propane-1,3-diyl or ethene-1,2-diyl, each of which may be substituted up to two times by methyl,
or
represents a group of the formula 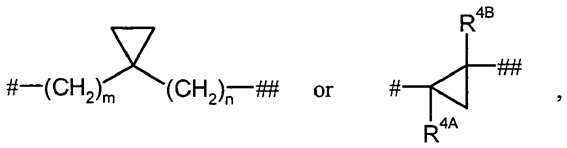 in which
# denotes the point of attachment to the carboxylic acid grouping,
## denotes the point of attachment to group
M, m represents the number 0 or 1,
n represents the number 1 or 2,
and R^{4A} and R^{4B} independently of one another represent hydrogen or methyl,
M represents phenylene, pyridylene, furylene, thienylene, thiazolylene or isoxazolylene, each of which may be substituted up to two times by identical or different radicals selected from the group consisting of fluorine, chlorine, methyl and trifluoromethyl,
or
represents cyclopentane-1,3-diyl, cyclohexane-1,3-diyl or cyclohexane-1,4-diyl, each of which may be substituted up to two times by identical or different radicals from the group consisting of fluorine and methyl, or
L² and M are attached to one another and together form a group of the formula 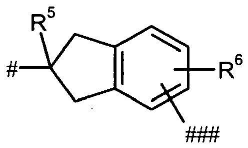 in which
### # denotes the point of attachment to the carboxylic acid grouping, denotes the point of attachment to group
L¹, R⁵ represents hydrogen, methyl or trifluoromethyl
and R⁶ represents hydrogen, fluorine or chlorine,
or a salt, solvate or solvate of a salt thereof.

7. Compound of the formula (I) according to any of Claims 1, 3 and 5 in which
ring A represents an oxo-substituted azaheterocycle of the formula 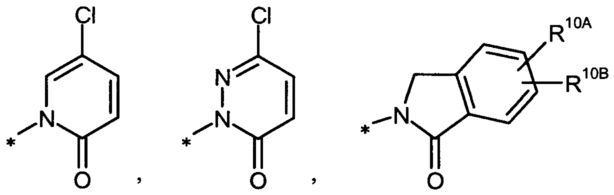 or 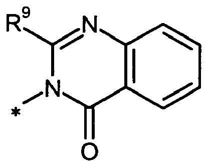 in which
* denotes the point of attachment to the remainder of the molecule,
R⁹ represents hydrogen, methyl or trifluoromethyl and
R^{10A} and R^{10B} independently of one another represent hydrogen or fluorine,
R¹ represents hydrogen or methyl,
R² represents hydrogen, fluorine or chlorine,
R³ represents (C₃-C₆)-alkyl or (C₃-C₆)-alkenyl, each of which may be substituted up to six times by fluorine,
or
represents (C₃-C₆)-cycloalkyl, cyclopentenyl or cyclohexenyl, each of which may be substituted up to two times by identical or different radicals from the group consisting of methyl, ethyl and trifluoromethyl and also up to four times by fluorine,
L¹ represents a bond or represents methylene or ethane-1,2-diyl,
L² represents a bond or represents methylene, ethane-1,2-diyl, propane-1,3-diyl or ethene-1,2-diyl, each of which may be substituted up to two times by methyl, or represents a group of the formula 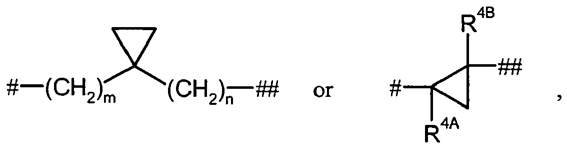 in which
# denotes the point of attachment to the carboxylic acid grouping,
## denotes the point of attachment to group
M, m represents the number 0 or 1,
n represents the number 1 or 2,
and R^{4A} and R^{4B} independently of one another represent hydrogen or methyl,
M represents phenylene, pyridylene, furylene, thienylene, thiazolylene or isoxazolylene, each of which may be substituted up to two times by identical or different radicals selected from the group consisting of fluorine, chlorine, methyl and trifluoromethyl,
or
represents cyclopentane-1,3-diyl, cyclohexane-1,3-diyl or cyclohexane-1,4-diyl, each of which may be substituted up to two times by identical or different radicals from the group consisting of fluorine and methyl,
or L² and M are attached to one another and together form a group of the formula 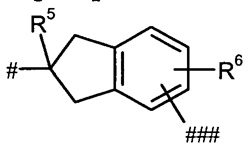 in which
# denotes the point of attachment to the carboxylic acid grouping,
### denotes the point of attachment to group
L¹, R⁵ represents hydrogen, methyl or trifluoromethyl
and R⁶ represents hydrogen, fluorine or chlorine,
or a salt, solvate or solvate of a salt thereof.

8. Compound of the formula (I) according to any of Claims 1, 2, 4 and 6 in which
ring A represents an oxo-substituted azaheterocycle of the formula 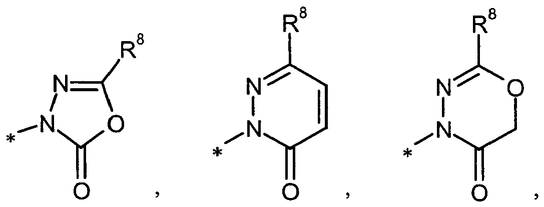 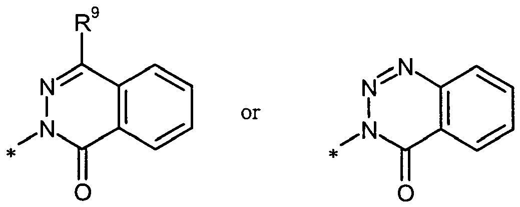 in which
* denotes the point of attachment to the remainder of the molecule,
R⁸ represents trifluoromethyl or phenyl which may be substituted up to two times by identical or different radicals selected from the group consisting of fluorine, chlorine, methyl and trifluoromethyl
and R⁹ of R⁸ represents hydrogen or has the meaning given above,
R¹ represents hydrogen,
R² represents hydrogen,
R³ represents propan-2-yl, butan-2-yl, pentan-2-yl, 1,1,1-trifluoropropan-2-yl, 1,1,1-trifluorobutan-2-yl, 4,4,4-trifluorobutan-2-yl, 4,4,4-trifluoro-2-methylbutan-1-yl, cyclopentyl or 3,3-difluorocyclopentyl,
L¹ represents a bond or represents methylene,
L² represents a bond, represents methylene or ethane-1,2-diyl, each of which may be substituted up to two times by methyl, or represents ethene-1,2-diyl
or
represents a group of the formula 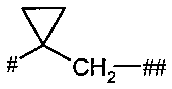 in which
# denotes the point of attachment to the carboxylic acid grouping
and ## denotes the point of attachment to group M,
M represents 1,3-phenylene or 1,4-phenylene, each of which may be substituted up to two times by identical or different radicals from the group consisting of fluorine, chlorine, methyl and trifluoromethyl, or represents cyclohexane-1,3-diyl or cyclohexane-1,4-diyl, or
L² and M are attached to one another and together form a group of the formula 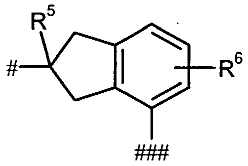 in which
# denotes the point of attachment to the carboxylic acid grouping,
### denotes the point of attachment to group
L¹, R⁵ represents hydrogen or methyl
and R⁶ represents hydrogen or fluorine,
or a salt, solvate or solvate of a salt thereof.

9. Compound of the formula (I) according to any of Claims 1, 3, 5 and 7 in which
ring A represents an oxo-substituted azaheterocycle of the formula 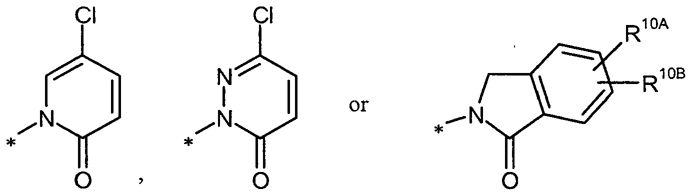 in which
* denotes the point of attachment to the remainder of the molecule
and R^{10A} and R^{10B} independently of one another represent hydrogen or fluorine,
R¹ represents hydrogen,
R² represents hydrogen,
R³ represents propan-2-yl, butan-2-yl, pentan-2-yl, 1,1,1-trifluoropropan-2-yl, 1,1,1-trifluorobutan-2-yl, 4,4,4-trifluorobutan-2-yl, 4,4,4-trifluoro-2-methylbutan-1-yl, cyclopentyl or 3,3-difluorocyclopentyl,
L¹ represents a bond,
L² represents methylene or ethane-1,2-diyl, each of which may be substituted up to two times by methyl, or represents ethene-1,2-diyl or represents a group of the formula 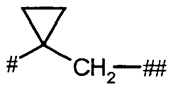 in which
# denotes the point of attachment to the carboxylic acid grouping
and ## M, denotes the point of attachment to group
M represents 1,3-phenylene or 1,4-phenylene, each of which may be substituted up to two times by identical or different radicals from the group consisting of fluorine, chlorine, methyl and trifluoromethyl,
or
L² and M are attached to one another and together form a group of the formula R⁵ ### in which
# denotes the point of attachment to the carboxylic acid grouping,
### denotes the point of attachment to group
L¹, R⁵ represents hydrogen or methyl
and R⁶ represents hydrogen or fluorine,
or a salt, solvate or solvate of a salt thereof.

10. Process for preparing a compound of the formula (I) as defined in any of Claims 1 to 9, **characterized in that** initially a compound of the formula (II) 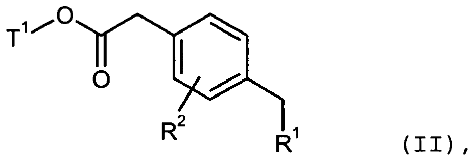 in which R¹ and R² have the meanings given in any of Claims 1 to 9
and
T¹ represents (C₁-C₄)-alkyl,
is converted in an inert solvent in the presence of a base with a compound of the formula (III) 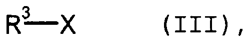 in which R³ has the meaning given in any of Claims 1 to 9
and
X represents a leaving group such as, for example, halogen, mesylate, tosylate or triflate,
into a compound of the formula (IV) 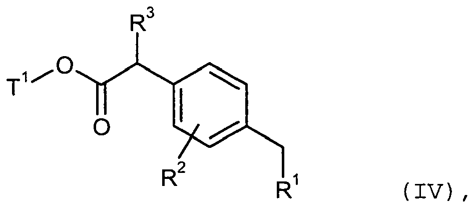 in which R¹, R², R³ and T¹ each have the meanings given above,
this is then brominated in an inert solvent with elemental bromine or with *N*-bromosuccinimide to give a compound of the formula (V) 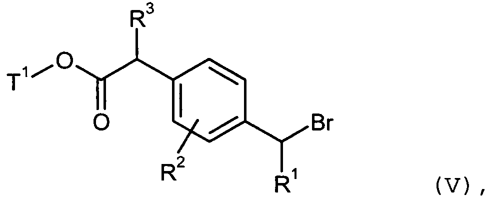 in which R¹, R², R³ and T¹ each have the meanings given above,
then reacted in an inert solvent in the presence of a base with a compound of the formula (VI) 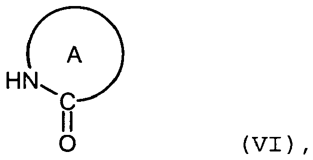 in which ring A represents an oxo-substituted azaheterocycle, as defined in any of Claims 1 to 9,
to give a compound of the formula (VII) 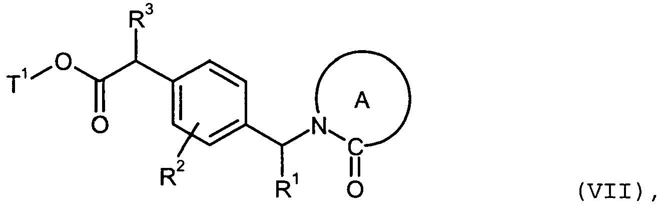 in which ring A, R¹, R², R³ and T¹ each have the meanings given above,
the ester radical T¹ in (VII) is then removed under basic or acidic conditions, the resulting carboxylic acid of the formula (VIII) 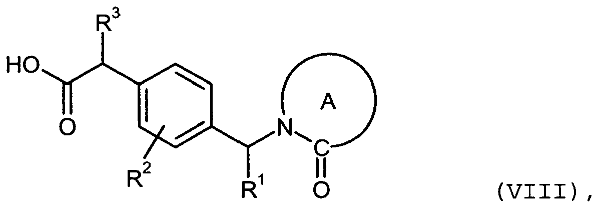 in which ring A, R¹, R² and R³ each have the meanings given above,
is then coupled in an inert solvent in the presence of a condensing agent or via the intermediate of the corresponding carbonyl chloride in the presence of a base with an amine of the formula (IX) 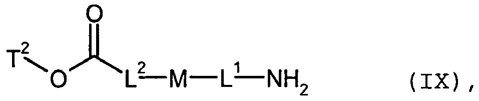 in which L¹, L² and M have the meanings given in any of Claims 1 to 9
and
T² represents (C₁-C₄)-alkyl ,
to give a compound of the formula (X) 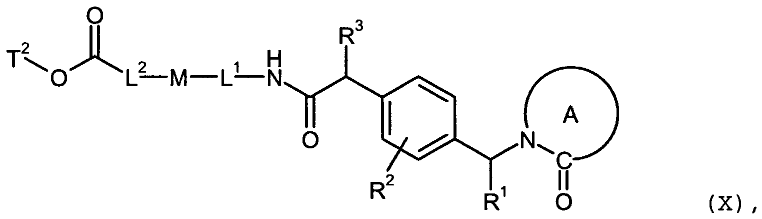 in which ring A, R¹, R², R³, L¹, L², M and T² each have the meanings given above,
and the ester radical T² in (X) is then removed by further basic or acidic solvolysis to give the carboxylic acid of the formula (I)
and the compounds of the formula (I) are separated where appropriate by methods known to the skilled person into their enantiomers and/or diastereomers, and/or where appropriate reacted with the appropriate (i) solvents and/or (ii) bases or acids to give the solvates, salts and/or solvates of the salts thereof.

11. Compound as defined in any of Claims 1 to 9 for the treatment and/or prevention of diseases.

12. Compound as defined in any of Claims 1 to 9 for use in a method for the treatment and/or prevention of heart failure, angina pectoris, hypertension, pulmonary hypertension, ischemias, vascular disorders, thromboembolic disorders and arteriosclerosis.

13. Use of a compound as defined in any of Claims 1 to 9 for producing a medicament for the treatment and/or prevention of heart failure, angina pectoris, hypertension, pulmonary hypertension, ischemias, vascular disorders, thromboembolic disorders and arteriosclerosis.

14. Medicament comprising a compound as defined in any of Claims 1 to 9 in combination with one or more inert, non-toxic, pharmaceutically suitable excipients.

15. Medicament comprising a compound as defined in any of Claims 1 to 9 in combination with one or more further active ingredients selected from the group consisting of organic nitrates, NO donors, cGMP-PDE inhibitors, stimulators of guanylate cyclase, agents having antithrombotic activity, agents lowering blood pressure, and agents altering lipid metabolism.

16. Medicament according to Claim 14 or 15 for the treatment and/or prevention of heart failure, angina pectoris, hypertension, pulmonary hypertension, ischemias, vascular disorders, thromboembolic disorders and arteriosclerosis.

## Revendications

1. Composé de formule (I) 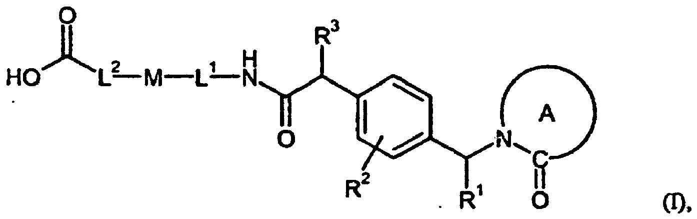 dans laquelle
le cycle A représente un hétérocycle aza à substitution oxo saturé ou partiellement insaturé, de 5 à 7 éléments, à liaison N,
qui (i) peut contenir un ou deux hétéroatomes supplémentaires de la série N, O et/ou S en tant qu'éléments de cycle,
qui (ii) est substitué avec un radical choisi dans la série fluor, chlore, alkyle en C₁-C₆, trifluorométhyle, cycloalkyle en C₃-C₇, hétérocyclyle de 4 à 7 éléments et phényle ou qui est benzo-annelé,
le substituant phényle et le cycle phényle annelé pouvant de leur côté être substitués jusqu'à deux fois, de manière identique ou différente, avec un radical choisi dans la série halogène, cyano, alkyle en C₁-C₄, alcényle en C₂-C₄, trifluorométhyle, alcoxy en C₁-C₄ et trifluorométhoxy,
et
qui (iii) peut également être substitué jusqu'à deux fois, de manière identique ou différente, avec d'autres radicaux choisis dans la série fluor, alkyle en C₁-C₆, trifluorométhyle, oxo, cycloalkyle en C₃-C₇, hétérocyclyle de 4 à 7 éléments et phényle,
phényle pouvant de son côté être substitué jusqu'à deux fois, de manière identique ou différente, avec un radical choisi dans la série halogène, cyano, alkyle en C₁-C₄, alcényle en C₂-C₄, trifluorométhyle, alcoxy en C₁-C₄ et trifluorométhoxy,
R¹ représente hydrogène, alkyle en C₁-C₄ ou cyclopropyle,
R² représente hydrogène, halogène, cyano, alkyle en C₁-C₄ ou trifluorométhyle,
R³ représente alkyle en C₃-C₆ ou alcényle en C₃-C₆, qui peuvent chacun être substitués avec cyano, alcoxy en C₁-C₄ ou trifluorométhoxy et jusqu'à six fois avec fluor,
ou
cycloalkyle en C₃-C₇ ou cycloalcényle en C₃-C₇, qui peuvent chacun être substitués jusqu'à deux fois, de manière identique ou différente, avec alkyle en C₁-C₄, trifluorométhyle et alcoxy en C₁-C₄ et jusqu'à quatre fois avec fluor,
ou
oxétanyle, tétrahydrofuranyle ou tétrahydropyranyle,
L¹ représente une liaison ou méthylène, éthane-1,2-diyle ou propane-1,3-diyle, qui peuvent chacun être substitués jusqu'à deux fois, de manière identique ou différente, avec alkyle en C₁-C₄,
L² représente une liaison ou méthylène, éthane-1,2-diyle, propane-1,3-diyle, éthène-1,2-diyle ou propène-1,3-diyle, qui peuvent chacun être substitués jusqu'à deux fois, de manière identique ou différente, avec alkyle en C₁-C₄,
ou
un groupe de formule 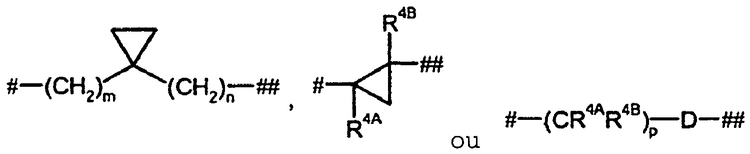 dans lesquelles
# caractérise la position de liaison avec le groupe acide carboxylique,
## caractérise la position de liaison avec le groupe M,
m signifie le nombre 0 ou 1,
n signifie le nombre 0, 1 ou 2,
p signifie le nombre 1 ou 2,
D signifie O ou S,
et
R^{4A} et R^{4B} signifient indépendamment l'un de l'autre hydrogène ou alkyle en C₁-C₄,
si le groupe -CR^{4A}R^{4B} apparaît deux fois, les significations individuelles de R^{4A} et R^{4B} pouvant à chaque fois être identiques ou différentes,
M représente phénylène ou hétéroarylène de 5 ou 6 éléments contenant jusqu'à deux hétéroatomes de cycle de la série N, O et/ou S, phénylène et hétéroarylène pouvant chacun être substitués jusqu'à deux fois, de manière identique ou différente, avec un radical choisi dans la série halogène, cyano, alkyle en C₁-C₄, trifluorométhyle, hydroxy, alcoxy en C₁-C₄ et trifluorométhoxy,
ou
cyclopropane-1,2-diyle, cyclobutane-1,2-diyle, cyclobutane-1,3-diyle, cyclopentane-1,2-diyle, cyclopentane-1,3-diyle, cyclohexane-1,2-diyle, cyclohexane-1,3-diyle ou cyclohexane-1,4-diyle, qui peuvent chacun être substitués jusqu'à deux fois, de manière identique ou différente, avec un radical choisi dans la série fluor, alkyle en C₁-C₄, trifluorométhyle et alcoxy en C₁-C₄,
ou
L² et M sont reliés l'un avec l'autre et forment ensemble un groupe de formule 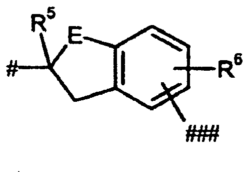 dans laquelle
# caractérise la position de liaison avec le groupe acide carboxylique,
### caractérise la position de liaison avec le groupe L¹,
E signifie O, S, CH₂ ou CH₂CH₂,
R⁵ signifie hydrogène, alkyle en C₁-C₄ ou trifluorométhyle,
et
R⁶ signifie hydrogène, halogène, alkyle en C₁-C₄, trifluorométhyle, alcoxy en C₁-C₄ ou trifluorométhoxy, ainsi que leurs sels, solvates et solvates des sels.

2. Composé de formule (I) selon la revendication 1, dans lequel
le cycle A représente un hétérocyle aza à substitution oxo de formule 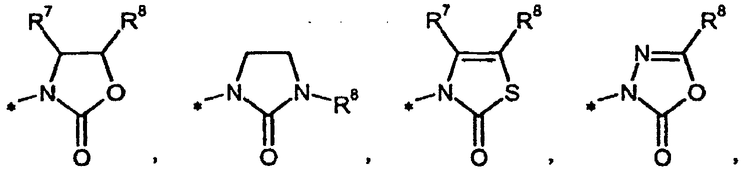 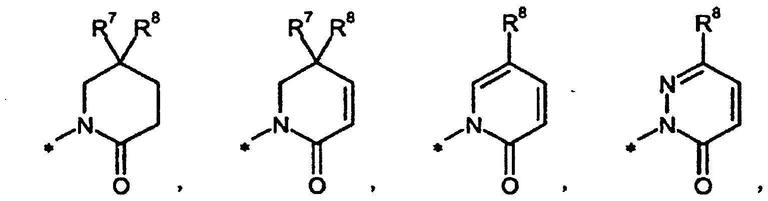 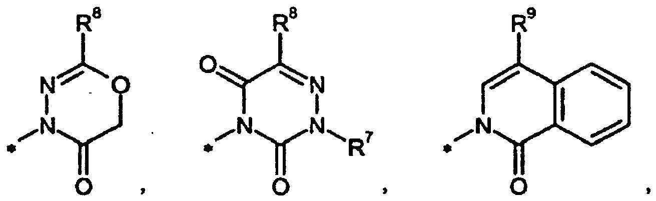 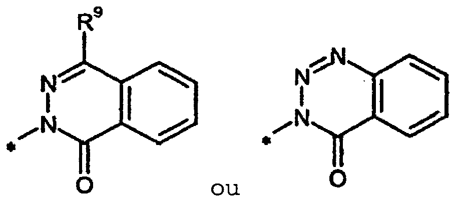 dans lesquelles
* caractérise la position de liaison avec la partie restante de la molécule,
R⁷ signifie hydrogène, alkyle en C₁-C₆ ou cycloalkyle en C₃-C₆,
R⁸ signifie alkyle en C₁-C₆, trifluorométhyle, cycloalkyle en C₃-C₆, hétérocyclyle de 4 à 6 éléments ou phényle, phényle pouvant de son côté être substitué jusqu'à deux fois, de manière identique ou différente, avec un radical choisi dans la série fluor, chlore, brome, cyano, alkyle en C₁-C₄, vinyle, trifluorométhyle, alcoxy en C₁-C₄ et trifluorométhoxy,
et
R⁹ signifie hydrogène ou a la signification donnée précédemment pour R⁸,
ainsi que leurs sels, solvates et solvates des sels.

3. Composé de formule (I) selon la revendication 1, dans lequel
le cycle A représente un hétérocycle aza à substitution oxo de formule 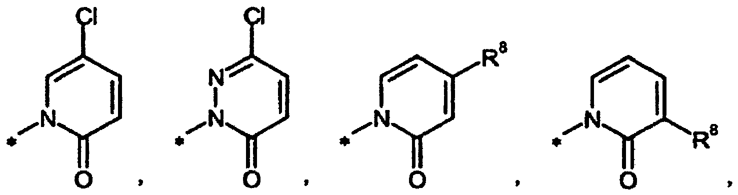 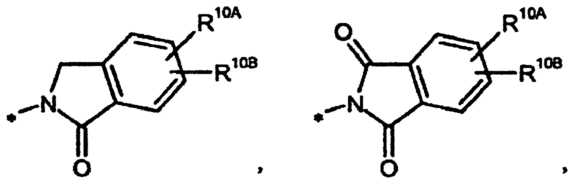 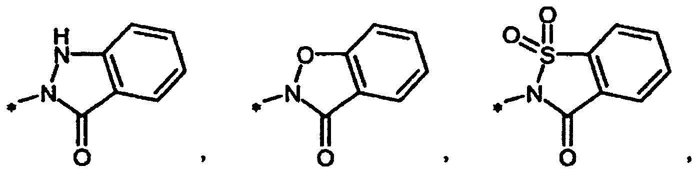 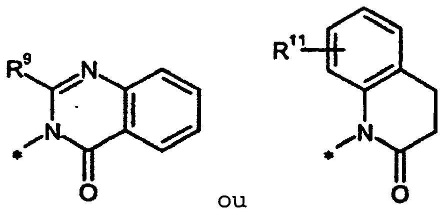 dans lesquelles
* caractérise la position de liaison avec la partie restante de la molécule,
R⁸ signifie alkyle en C₁-C₆, trifluorométhyle, cycloalkyle en C₃-C₆, hétérocyclyle de 4 à 6 éléments ou phényle, phényle pouvant de son côté être substitué jusqu'à deux fois, de manière identique ou différente, avec un radical choisi dans la série fluor, chlore, brome, cyano, alkyle en C₁-C₄, vinyle, trifluorométhyle, alcoxy en C₁-C₄ et trifluorométhoxy,
R⁹ signifie hydrogène ou a la signification donnée précédemment pour R⁸,
R^{10A} et R^{10B} signifient indépendamment l'un de l'autre hydrogène, fluor ou chlore,
et
R¹¹ signifie hydrogène, fluor ou chlore,
ainsi que leurs sels, solvates et solvates des sels.

4. Composé de formule (I) selon la revendication 1 ou 2, dans lequel
le cycle A représente un hétérocycle aza à substitution oxo de formule 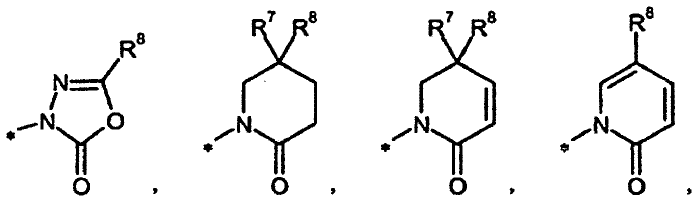 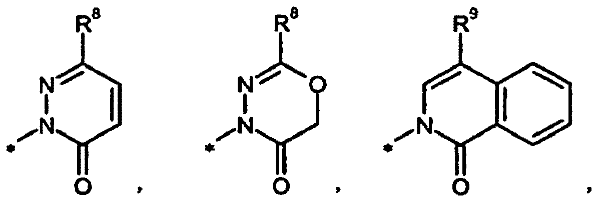 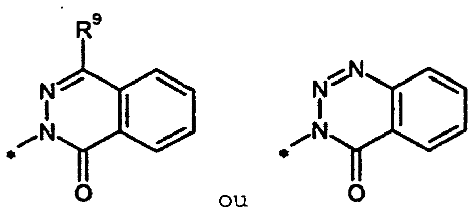 dans lesquelles
* caractérise la position de liaison avec la partie restante de la molécule,
R⁷ signifie hydrogène ou alkyle en C₁-C₄,
R⁸ signifie alkyle en C₁-C₆, trifluorométhyle, cycloalkyle en C₃-C₆ ou phényle, phényle pouvant de son côté être substitué jusqu'à deux fois, de manière identique ou différente, avec un radical choisi dans la série fluor, chlore, cyano, alkyle en C₁-C₄, trifluorométhyle, alcoxy en C₁-C₄ et trifluorométhoxy,
et
R⁹ signifie hydrogène ou a la signification donnée précédemment pour R⁸,
R¹ représente hydrogène ou alkyle en C₁-C₄,
R² représente hydrogène, fluor, chlore ou trifluorométhyle,
R³ représente alkyle en C₃-C₆ ou alcényle en C₃-C₆, qui peuvent chacun être substitués avec cyano, méthoxy, éthoxy ou trifluorométhoxy et jusqu'à six fois avec fluor,
ou
cycloalkyle en C₃-C₆ ou cycloalcényle en C₄-C₆, qui peuvent chacun être substitués jusqu'à deux fois, de manière identique ou différente, avec alkyle en C₁-C₄ et trifluorométhyle et jusqu'à quatre fois avec fluor,
ou
oxétanyle,
L¹ représente une liaison ou méthylène, éthane-1,2-diyle ou propane-1,3-diyle, qui peuvent chacun être substitués jusqu'à deux fois avec méthyle,
L² représente une liaison ou méthylène, éthane-1,2-diyle, propane-1,3-diyle ou éthène-1,2-diyle, qui peuvent chacun être substitués jusqu'à deux fois avec méthyle,
ou
un groupe de formule 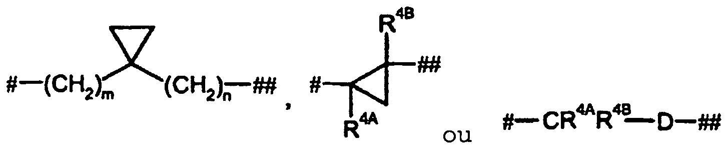 dans lesquelles
# caractérise la position de liaison avec le groupe acide carboxylique,
## caractérise la position de liaison avec le groupe M,
m signifie le nombre 0 ou 1,
n signifie le nombre 0, 1 ou 2,
D signifie O ou S,
et
R^{4A} et R^{4B} signifient indépendamment l'un de l'autre hydrogène ou méthyle,
M représente phénylène ou hétéroarylène de 5 ou 6 éléments contenant jusqu'à deux hétéroatomes de cycle de la série N, O et/ou S, phénylène et hétéroarylène pouvant chacun être substitués jusqu'à deux fois, de manière identique ou différente, avec un radical choisi dans la série fluor, chlore, alkyle en C₁-C₄, trifluorométhyle, méthoxy et trifluorométhoxy,
ou
cyclopentane-1,3-diyle, cyclohexane-1,3-diyle ou cyclohexane-1,4-diyle, qui peuvent chacun être substitués jusqu'à deux fois, de manière identique ou différente, avec un radical choisi dans la série fluor, méthyle et trifluorométhyle,
ou
L² et M sont reliés l'un avec l'autre et forment ensemble un groupe de formule 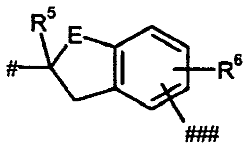 dans laquelle
# caractérise la position de liaison avec le groupe acide carboxylique,
### caractérise la position de liaison avec le groupe L¹,
E signifie CH₂ ou CH₂CH₂,
R⁵ signifie hydrogène, méthyle ou trifluorométhyle, et
R⁶ signifie hydrogène, fluor, chlore, méthyle, trifluorométhyle, méthoxy ou trifluorométhoxy,
ainsi que leurs sels, solvates et solvates des sels.

5. Composé de formule (I) selon la revendication 1 ou 3, dans lequel
le cycle A représente un hétérocycle aza à substitution oxo de formule 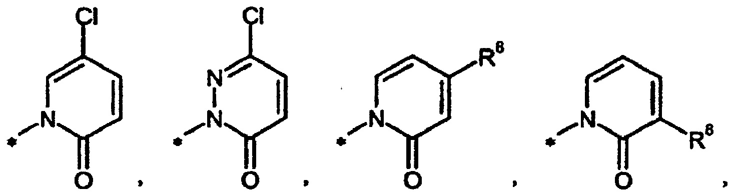 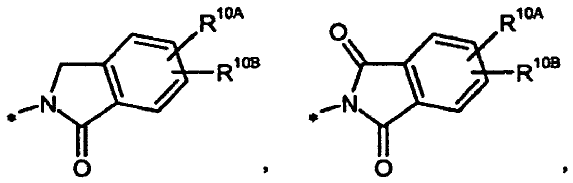 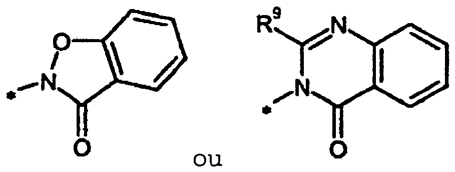 dans lesquelles
* caractérise la position de liaison avec la partie restante de la molécule,
R⁸ signifie alkyle en C₁-C₆, trifluorométhyle, cycloalkyle en C₃-C₆ ou phényle, phényle pouvant de son côté être substitué jusqu'à deux fois, de manière identique ou différente, avec un radical choisi dans la série fluor, chlore, cyano, alkyle en C₁-C₄, trifluorométhyle, alcoxy en C₁-C₄ et trifluorométhoxy,
R⁹ signifie hydrogène ou a la signification donnée précédemment pour R⁸,
et
R^{10A} et R^{10B} signifient indépendamment l'un de l'autre hydrogène, fluor ou chlore,
R¹ représente hydrogène ou alkyle en C₁-C₄,
R² représente hydrogène, fluor, chlore ou trifluorométhyle,
R³ représente alkyle en C₃-C₆ ou alcényle en C₃-C₆, qui peuvent chacun être substitués avec cyano, méthoxy, éthoxy ou trifluorométhoxy et jusqu'à six fois avec fluor,
ou
cycloalkyle en C₃-C₆ ou cycloalcényle en C₄-C₆, qui peuvent chacun être substitués jusqu'à deux fois, de manière identique ou différente, avec alkyle en C₁-C₄ et trifluorométhyle et jusqu'à quatre fois avec fluor,
ou
oxétanyle,
L¹ représente une liaison ou méthylène, éthane-1,2-diyle ou propane-1,3-diyle, qui peuvent chacun être substitués jusqu'à deux fois avec méthyle,
L² représente une liaison ou méthylène, éthane-1,2-diyle, propane-1,3-diyle ou éthène-1,2-diyle, qui peuvent chacun être substitués jusqu'à deux fois avec méthyle,
ou
un groupe de formule 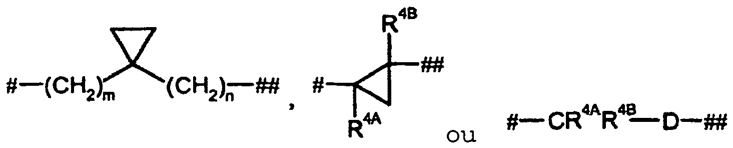 dans lesquelles
# caractérise la position de liaison avec le groupe acide carboxylique,
## caractérise la position de liaison avec le groupe M,
m signifie le nombre 0 ou 1,
n signifie le nombre 0, 1 ou 2,
D signifie O ou S,
et
R^{4A} et R^{4B} signifient indépendamment l'un de l'autre hydrogène ou méthyle,
M représente phénylène ou hétéroarylène de 5 ou 6 éléments contenant jusqu'à deux hétéroatomes de cycle de la série N, O et/ou S, phénylène et hétéroarylène pouvant chacun être substitués jusqu'à deux fois, de manière identique ou différente, avec un radical choisi dans la série fluor, chlore, alkyle en C₁-C₄, trifluorométhyle, méthoxy et trifluorométhoxy,
ou
cyclopentane-1,3-diyle, cyclohexane-1,3-diyle ou cyclohexane-1,4-diyle, qui peuvent chacun être substitué jusqu'à deux fois, de manière identique ou différente, avec un radical choisi dans la série fluor, méthyle et trifluorométhyle,
ou
L² et M sont reliés l'un avec l'autre et forment ensemble un groupe de formule 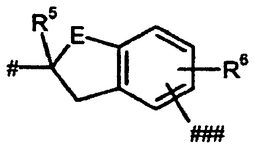 dans laquelle
# caractérise la position de liaison avec le groupe acide carboxylique,
### caractérise la position de liaison avec le groupe L¹,
E signifie CH₂ ou CH₂CH₂,
R⁵ signifie hydrogène, méthyle ou trifluorométhyle, et
R⁶ signifie hydrogène, fluor, chlore, méthyle, trifluorométhyle, méthoxy ou trifluorométhoxy,
ainsi que leurs sels, solvates et solvates des sels.

6. Composé de formule (I) selon la revendication 1, 2 ou 4, dans lequel
le cycle A représente un hétérocycle aza à substitution oxo de formule 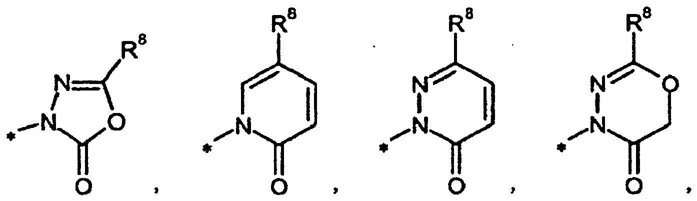 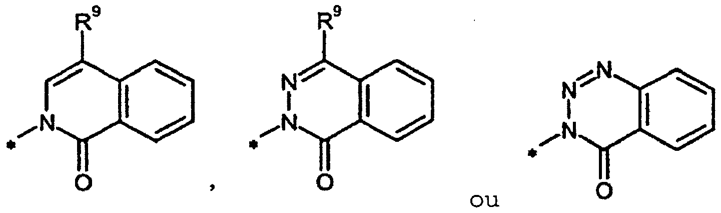 dans lesquelles
* caractérise la position de liaison avec la partie restante de la molécule,
R⁸ signifie trifluorométhyle, cycloalkyle en C₃-C₆ ou phényle, phényle pouvant de son côté être substitué jusqu'à deux fois, de manière identique ou différente, avec un radical choisi dans la série fluor, chlore, cyano, méthyle, trifluorométhyle, méthoxy et trifluorométhoxy
et
R⁹ signifie hydrogène ou a la signification donnée précédemment pour R⁸,
R¹ représente hydrogène ou méthyle,
R² représente hydrogène, fluor ou chlore,
R³ représente alkyle en C₃-C₆ ou alcényle en C₃-C₆, qui peuvent chacun être substitués jusqu'à six fois avec fluor,
ou
cycloalkyle en C₃-C₆, cyclopentényle ou cyclohexényle, qui peuvent chacun être substitués jusqu'à deux fois, de manière identique ou différente, avec méthyle, éthyle et trifluorométhyle et jusqu'à quatre fois avec fluor,
L¹ représente une liaison ou méthylène ou éthane-1,2-diyle,
L² représente une liaison ou méthylène, éthane-1,2-diyle, propane-1,3-diyle ou éthène-1,2-diyle, qui peuvent chacun être substitués jusqu'à deux fois avec méthyle,
ou
un groupe de formule 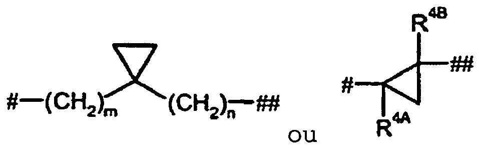 dans lesquelles
# caractérise la position de liaison avec le groupe acide carboxylique,
## caractérise la position de liaison avec le groupe M,
m signifie le nombre 0 ou 1,
n signifie le nombre 1 ou 2,
et
R^{4A} et R^{4B} signifient indépendamment l'un de l'autre hydrogène ou méthyle,
M représente phénylène, pyridylène, furylène, thiénylène, thiazolylène ou isoxazolylène, qui peuvent chacun être substitués jusqu'à deux fois, de manière identique ou différente, avec un radical choisi dans la série fluor, chlore, méthyle et trifluorométhyle,
ou
cyclopentane-1,3-diyle, cyclohexane-1,3-diyle ou cyclohexane-1,4-diyle, qui peuvent chacun être substitués jusqu'à deux fois, de manière identique ou différente, avec fluor et méthyle,
ou
L² et M sont reliés l'un avec l'autre et forment ensemble un groupe de formule 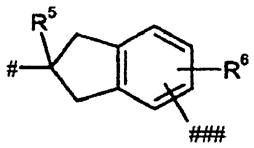 dans laquelle
# caractérise la position de liaison avec le groupe acide carboxylique,
### caractérise la position de liaison avec le groupe L¹,
R⁵ signifie hydrogène, méthyle ou trifluorométhyle, et
R⁶ signifie hydrogène, fluor ou chlore,
ainsi que leurs sels, solvates et solvates des sels.

7. Composé de formule (I) selon la revendication 1, 3 ou 5, dans lequel
le cycle A représente un hétérocycle aza à substitution oxo de formule 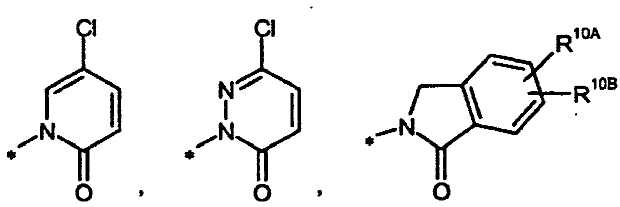 ou 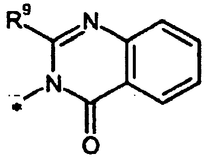 dans lesquelles
* caractérise la position de liaison avec la partie restante de la molécule,
R⁹ signifie hydrogène, méthyle ou trifluorométhyle
et
R^{10A} et R^{10B} signifient indépendamment l'un de l'autre hydrogène ou fluor,
R¹ représente hydrogène ou méthyle,
R² représente hydrogène, fluor ou chlore,
R³ représente alkyle en C₃-C₆ ou alcényle en C₃-C₆, qui peuvent chacun être substitués jusqu'à six fois avec fluor,
ou
cycloalkyle en C₃-C₆, cyclopentényle ou cyclohexényle, qui peuvent chacun être substitués jusqu'à deux fois, de manière identique ou différente, avec méthyle, éthyle et trifluorométhyle et jusqu'à quatre fois avec fluor,
L¹ représente une liaison ou méthylène ou éthane-1,2-diyle,
L² représente une liaison ou méthylène, éthane-1,2-diyle, propane-1,3-diyle ou éthène-1,2-diyle, qui peuvent chacun être substitués jusqu'à deux fois avec méthyle,
ou
un groupe de formule 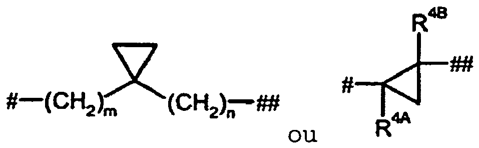 dans lesquelles
# caractérise la position de liaison avec le groupe acide carboxylique,
## caractérise la position de liaison avec le groupe M,
m signifie le nombre 0 ou 1,
n signifie le nombre 1 ou 2,
et
R^{4A} et R^{4B} signifient indépendamment l'un de l'autre hydrogène ou méthyle,
M représente phénylène, pyridylène, furylène, thiénylène, thiazolylène ou isoxazolylène, qui peuvent chacun être substitués jusqu'à deux fois, de manière identique ou différente, avec un radical choisi dans la série fluor, chlore, méthyle et trifluorométhyle,
ou
cyclopentane-1,3-diyle, cyclohexane-1,3-diyle ou cyclohexane-1,4-diyle, chacun pouvant être substitué jusqu'à deux fois, de manière identique ou différente, avec fluor et méthyle,
ou
L² et M sont reliés l'un avec l'autre et forment ensemble un groupe de formule 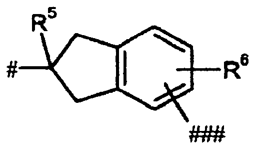 dans laquelle
# caractérise la position de liaison avec le groupe acide carboxylique,
### caractérise la position de liaison avec le groupe L¹,
R⁵ signifie hydrogène, méthyle ou trifluorométhyle, et
R⁶ signifie hydrogène, fluor ou chlore,
ainsi que leurs sels, solvates et solvates des sels.

8. Composé de formule (I) selon la revendication 1, 2, 4 ou 6, dans lequel
le cycle A représente un hétérocycle aza à substitution oxo de formule 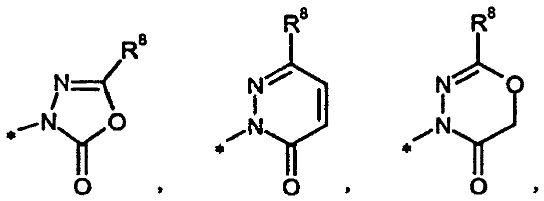 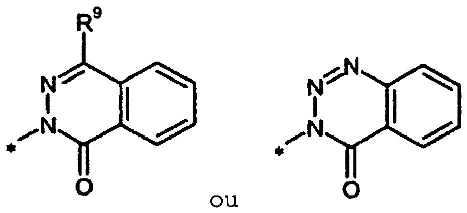 dans lesquelles
* caractérise la position de liaison avec la partie restante de la molécule,
R⁸ signifie trifluorométhyle ou phényle, qui peut être substitué jusqu'à deux fois, de manière identique ou différente, avec un radical choisi dans la série fluor, chlore, méthyle et trifluorométhyle,
et
R⁹ signifie hydrogène ou a la signification donnée précédemment pour R⁸,
R¹ représente hydrogène,
R² représente hydrogène,
R³ représente propan-2-yle, butan-2-yle, pentan-2-yle, 1,1,1-trifluoropropan-2-yle, 1,1,1-trifluorobutan-2-yle, 4,4,4-trifluorobutan-2-yle, 4,4,4-trifluoro-2-méthylbutan-1-yle, cyclopentyle ou 3,3-difluorocyclopentyle,
L¹ représente une liaison ou méthylène,
L² représente une liaison, méthylène ou éthane-1,2-diyle, qui peuvent chacun être substitués jusqu'à deux fois avec méthyle, ou éthène-1,2-diyle,
ou
un groupe de formule 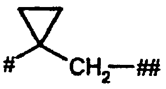 dans laquelle
# caractérise la position de liaison avec le groupe acide carboxylique,
et
## caractérise la position de liaison avec le groupe M,
M représente 1,3-phénylène ou 1,4-phénylène, qui peuvent chacun être substitués jusqu'à deux fois, de manière identique ou différente, avec fluor, chlore, méthyle et trifluorométhyle, ou cyclohexane-1,3-diyle ou cyclohexane-1,4-diyle,
ou
L² et M sont reliés l'un avec l'autre et forment ensemble un groupe de formule 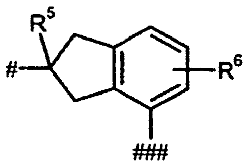 dans laquelle
# caractérise la position de liaison avec le groupe acide carboxylique,
### caractérise la position de liaison avec le groupe L¹,
R⁵ signifie hydrogène ou méthyle,
et
R⁶ signifie hydrogène ou fluor,
ainsi que leurs sels, solvates et solvates des sels.

9. Composé de formule (I) selon la revendication 1, 3, 5 ou 7, dans lequel
le cycle A représente un hétérocyle aza à substitution oxo de formule 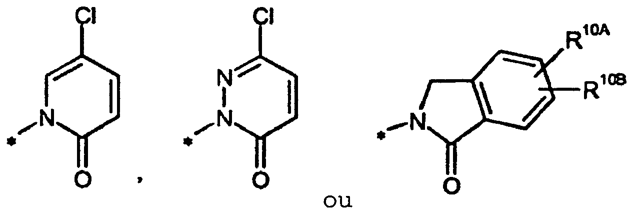 dans lesquelles
* caractérise la position de liaison avec la partie restante de la molécule,
et
R^{10A} et R^{10B} signifient indépendamment l'un de l'autre hydrogène ou fluor,
R¹ représente hydrogène,
R² représente hydrogène,
R³ représente propan-2-yle, butan-2-yle, pentan-2-yle, 1,1,1-trifluoropropan-2-yle, 1,1,1-trifluorobutan-2-yle, 4,4,4-trifluorobutan-2-yle, 4,4,4-trifluoro-2-méthylbutan-1-yle, cyclopentyle ou 3,3-difluorocyclopentyle,
L¹ représente une liaison,
L² représente méthylène ou éthane-1,2-diyle, qui peuvent chacun être substitués jusqu'à deux fois avec méthyle, ou éthène-1,2-diyle,
ou
un groupe de formule 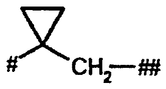 dans laquelle
# caractérise la position de liaison avec le groupe acide carboxylique,
et
## caractérise la position de liaison avec le groupe M,
M représente 1,3-phénylène ou 1,4-phénylène, qui peuvent chacun être substitués jusqu'à deux fois, de manière identique ou différente, avec fluor, chlore, méthyle et trifluorométhyle,
ou
L² et M sont reliés l'un avec l'autre et forment ensemble un groupe de formule 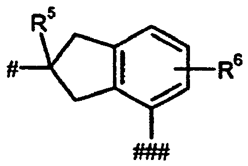 dans laquelle
# caractérise la position de liaison avec le groupe acide carboxylique,
### caractérise la position de liaison avec le groupe L¹,
R⁵ signifie hydrogène ou méthyle,
et
R⁶ signifie hydrogène ou fluor,
ainsi que leurs sels, solvates et solvates des sels.

10. Procédé de fabrication d'un composé de formule (I), tel que défini dans les revendications 1 à 9, **caractérisé en ce qu'**un composé de formule (II) 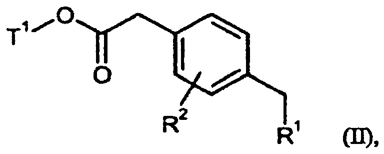 dans laquelle R¹ et R² ont les significations données dans les revendications 1 à 9,
et
T¹ représente alkyle en C₁-C₄,
est tout d'abord transformé dans un solvant inerte en présence d'une base avec un composé de formule (III) 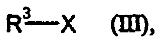 dans laquelle R³ a la signification donnée dans les revendications 1 à 9,
et
X représente un groupe partant tel que par exemple halogène, mésylate, tosylate ou triflate,
en un composé de formule (IV) 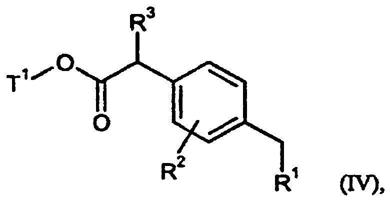 dans laquelle R¹, R², R³ et T¹ ont chacun les significations données précédemment,
puis celui-ci est bromé dans un solvant inerte avec du brome élémentaire ou avec du N-bromosuccinimide en un composé de formule (V) 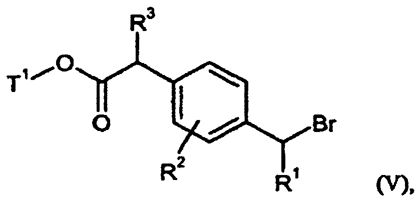 dans laquelle R¹, R², R³ et T¹ ont chacun les significations données précédemment,
puis mis en réaction dans un solvant inerte en présence d'une base avec un composé de formule (VI) 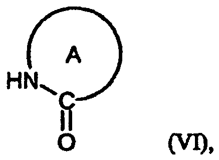 dans laquelle le cycle A représente un hétérocycle aza à substitution oxo tel que défini dans les revendications 1 à 9,
pour former un composé de formule (VII) 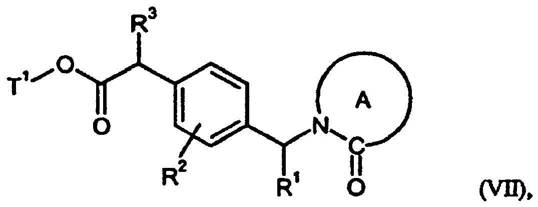 dans laquelle le cycle A, R¹, R², R³ et T¹ ont chacun les significations données précédemment,
puis le radical ester T¹ dans (VII) est clivé en conditions basiques ou acides, l'acide carboxylique résultant de formule (VIII) 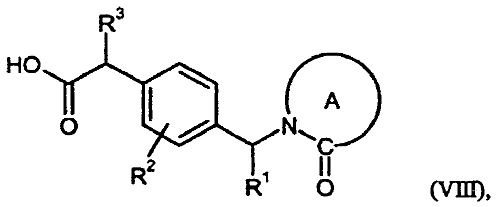 dans laquelle le cycle A, R¹, R² et R³ ont chacun les significations données précédemment,
est ensuite couplé dans un solvant inerte en présence d'un agent de condensation ou par l'intermédiaire du chlorure d'acide carboxylique correspondant en présence d'une base avec une amine de formule (IX) 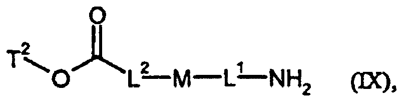 dans laquelle L¹, L² et M ont les significations données dans les revendications 1 à 9
et
T² représente alkyle en C₁-C₄,
pour former un composé de formule (X) 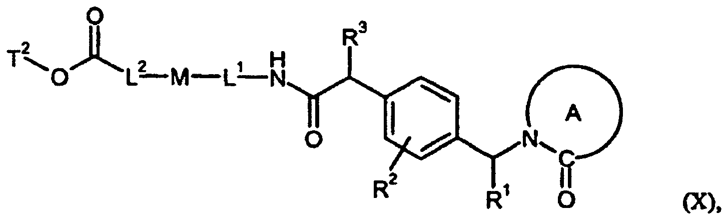 dans laquelle le cycle A, R¹, R², R³, L¹, L², M et T² ont chacun les significations données précédemment,
puis le radical ester T² dans (X) est clivé par une nouvelle solvolyse basique ou acide pour former l'acide carboxylique de formule (I),
et les composés de formule (I) sont éventuellement séparés selon des procédés connus de l'homme du métier en leurs énantiomères et/ou diastéréomères et/ou éventuellement transformés avec les (i) solvants et/ou (ii) bases ou acides correspondants en leurs solvates, sels et/ou solvates des sels.

11. Composé, tel que défini dans l'une quelconque des revendications 1 à 9, pour le traitement et/ou la prévention de maladies.

12. Composé, tel que défini dans l'une quelconque des revendications 1 à 9, pour une utilisation dans un procédé de traitement et/ou de prévention de l'insuffisance cardiaque, de l'angine de poitrine, de l'hypertension, de l'hypertension pulmonaire, des ischémies, des angiopathies, des maladies thromboemboliques et de l'artériosclérose.

13. Utilisation d'un composé, tel que défini dans l'une quelconque des revendications 1 à 9, pour la fabrication d'un médicament pour le traitement et/ou la prévention de l'insuffisance cardiaque, de l'angine de poitrine, de l'hypertension, de l'hypertension pulmonaire, des ischémies, des angiopathies, des maladies thromboemboliques et de l'artériosclérose.

14. Médicament contenant un composé tel que défini dans l'une quelconque des revendications 1 à 9, en combinaison avec un ou plusieurs adjuvants inertes, non toxiques, pharmaceutiquement appropriés.

15. Médicament contenant un composé tel que défini dans l'une quelconque des revendications 1 à 9, en combinaison avec une ou plusieurs substances actives supplémentaires choisies dans le groupe constitué par les nitrates organiques, les donneurs de NO, les inhibiteurs de cGMP-PDE, les stimulateurs de guanylate cyclase, les agents à action antithrombotique, les agents abaissant la tension artérielle, ainsi que les agents modifiant le métabolisme des lipides.

16. Médicament selon la revendication 14 ou 15 pour le traitement et/ou la prévention de l'insuffisance cardiaque, de l'angine de poitrine, de l'hypertension, de l'hypertension pulmonaire, des ischémies, des angiopathies, des maladies thromboemboliques et de l'artériosclérose.
